(19) Europäisches Patentamt
European Patent Office
Office européen des brevets

(11) EP 2 154 245 B1

(12) **EUROPEAN PATENT SPECIFICATION**

(45) Date of publication and mention of the grant of the patent:
**11.11.2015 Bulletin 2015/46**

(51) Int Cl.:
***C12N 15/00** (2006.01)* ***C12N 15/09** (2006.01)*
***C12Q 1/68** (2006.01)* ***G01N 33/53** (2006.01)*
***G01N 33/574** (2006.01)*

(21) Application number: **09177359.8**

(22) Date of filing: **01.09.2006**

(54) **Composition and method for diagnosing kidney cancer and for predicting prognosis for kidney cancer patient**

Zusammensetzung und Verfahren zur Diagnose von Nierenkrebs und zur Vorhersage einer Prognose für einen Nierenkrebspatienten

Composition et procédé pour le diagnostic du cancer du rein et pour prédire le pronostic d'un patient atteint de cancer du rein

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.09.2005 JP 2005255499**
**01.11.2005 JP 2005318589**

(43) Date of publication of application:
**17.02.2010 Bulletin 2010/07**

(60) Divisional application:
**11175092.3 / 2 404 998**

(62) Document number(s) of the earlier application(s) in accordance with Art. 76 EPC:
**06821799.1 / 1 930 426**

(73) Proprietors:
- **Toray Industries, Inc.**
  **Tokyo 103-8666 (JP)**
- **Kyoto University**
  **Kyoto-shi**
  **Kyoto 606-8501 (JP)**

(72) Inventors:
- **Kozono, Satoko**
  **Kamakura-shi, Kanagawa 248-8555 (JP)**
- **Akiyama, Hideo**
  **Kamakura-shi, Kanagawa 248-8555 (JP)**
- **Myomoto, Akira**
  **Kamakura-shi, Kanagawa 248-8555 (JP)**
- **Nobumasa, Hitoshi**
  **Kamakura-shi, Kanagawa 248-8555 (JP)**
- **Nomura, Osamu**
  **Kamakura-shi, Kanagawa 248-8555 (JP)**
- **Ogawa, Osamu**
  **Kyoto-shi Kyoto 606-8501 (JP)**
- **Nakamura, Eijiro**
  **Kyoto-shi Kyoto 606-8501 (JP)**
- **Tsujimoto, Gozoh**
  **Kyoto-shi Kyoto 606-8501 (JP)**

(74) Representative: **Prüfer & Partner GbR**
**European Patent Attorneys**
**Sohnckestraße 12**
**81479 München (DE)**

(56) References cited:
**WO-A-98/45432** **WO-A-2004/032842**
**WO-A-2005/003776** **WO-A-2005/036176**
**US-A1- 2003 092 616**

- **VASSELLI JAMES R ET AL: "Predicting survival in patients with metastatic kidney cancer by gene-expression profiling in the primary tumor." PROCEEDINGS OF THE NATIONAL ACADEMY OF SCIENCES OF THE UNITED STATES OF AMERICA 10 JUN 2003, vol. 100, no. 12, 10 June 2003 (2003-06-10), pages 6958-6963, XP002520165 ISSN: 0027-8424**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- TAKAHASHI MASAYUKI ET AL: "Gene expression profiling of clear cell renal cell carcinoma: Gene identification and prognostic classification" PROCEEDINGS OF THE NATIONAL ACADEMY OF SCIENCES OF USA, NATIONAL ACADEMY OF SCIENCE, WASHINGTON, DC.; US, vol. 98, no. 17, 14 August 2001 (2001-08-14), pages 9754-9759, XP002318256 ISSN: 0027-8424
- BOER JUDITH M ET AL: "Identification and classification of differentially expressed genes in renal cell carcinoma by expression profiling on a global human 31,500-element cDNA array" GENOME RESEARCH, COLD SPRING HARBOR LABORATORY PRESS, WOODBURY, NY, US, vol. 11, no. 11, 1 November 2001 (2001-11-01), pages 1861-1870, XP002215718 ISSN: 1088-9051
- MIYAKE H ET AL: "Introduction of basic fibroblast growth factor gene into mouse renal cell carcinoma cell line enhances its metastatic potential." CANCER RESEARCH 15 MAY 1996, vol. 56, no. 10, 15 May 1996 (1996-05-15), pages 2440-2445, XP002520166 ISSN: 0008-5472
- KITAGAWA ET AL: "EXPRESSION OF MESSENGER RNAS FOR MEMBRANE-TYPE 1, 2, AND 3 MATRIX METALLOPROTEINASES IN HUMAN RENAL CELL CARCINOMAS" JOURNAL OF UROLOGY, BALTIMORE, MD, US, vol. 162, no. 3, 1 September 1999 (1999-09-01), pages 905-909, XP005557829 ISSN: 0022-5347
- FREEMAN M R ET AL: "Aberrant expression of epidermal growth factor receptor and HER-2 (erbB-2) messenger RNAs in human renal cancers." CANCER RESEARCH 15 NOV 1989, vol. 49, no. 22, 15 November 1989 (1989-11-15), pages 6221-6225, XP002520167 ISSN: 0008-5472

SESENCE LISTING

<110> TORAY Industries, Inc. Kyoto University

<120> Composition and methods for diagnosing kidney cancer and for predicting prognosis for kidney cancer patient

<130> PH-2870-PCT

<140> PCT/JP2006/317380
<141> 2006- 09- 01

<150> JP 2005-255499
<151> 2005-09-02

<150> JP 2005-318589
<151> 2005-11-01

<160> 197

<170> PatentIn version 3. 1

<210> 1
<211> 3072
<212> DNA
<213> Homo sapiens

<400> 1

aatgaaggtg gacacccaaa tagccccaat acaaatgcct gttcaatcaa ccaaacatct 60
aagcagcaca tctatgtggt agcatattgc caggccgtga gactgcgaat ataaatagga 120
accgcccctc atctgcaggc gctcacaacc tagttagcaa acagtaaaac aattaagcgc 180
gccgtggaca taggcccact tgtcctggga aatgagggga agctggggtt tgcagtggtt 240
tgattgaagg gggactacat gttagaggca cagactgggt gcaggtacac ccaaaggaac 300
gagaagagtg gaaggaaaca acatccacaa agtaaccaca tgctggcgta tcgaaggccg 360
tgatttacgg ttttgagact ttacctcgcc agcaaagggg ggccagtctg ttagcggtgc 420
agattggagg ggtgacattg gaagctgtcc aggaaaaaga aaatggaact ggggagcaga 480
aggcctacgc aagagggcgg gacagacagg acttgtgact agtagctctg gactgaggaa 540
tcctccctgc tttctggtgc gggagagcta gtggatgatg gtgccaataa cctggatggg 600
gaaagtaagc tccctcctgg aatgcttcat tcacaacctc cattttcagc aacatcccat 660
ctactggtgc ttcctggtcg agatacaagt ttcctgaaac tgctgctctg ttttgggcct 720
cacccggcca acagctcact agctggcaag cagtagtatc aagatggcgg ccccctagga 780
ctggctagtc atgtgacctc gggtttccca agtttgaagc ccggcagtcc tttcgggggc 840
aaggttcacc tgtcacgaaa cgagtgtcac cccttcgact ctcgcaagcc aatcggcatc 900
tgagactggg ccactgcggt gaggcgatcg gaagattggt cctttccagt cgcctagcta 960

gggccaat ca cggagcgt cc cat act t cgc gggcccgccc gt aggccggg gagaagcagg 1020
aat at cgt ca cagcgt ggcg gt at t at t ac ct aaggact c gat aggaggt gggacgcgt g 1080
t t gat t gaca ggcagat t t c cct accggga t t t gagaat t t ggcgcagt g cccgcct t ag 1140
aggt gcgct t at t t gat t gc caagt aat at t ccccaat gg agt act agct cat ggt gacg 1200
ggcaggcagc t t gagct aat gagt cct ccg t ggccggcgc agct ct ccac at gccgggcg 1260
gcgggcccca gt ct gagcgg cgat ggcggc ggcggcggcg gcggcagcag cagcgggggc 1320
t gcgggcggt cggggct ccg ggccggggcg gcggcgccat ct t gt gcccg gggccggt gg 1380
ggaggccggg gagggggccc cggggggcgc aggggact ac gggaacggcc t ggagt ct ga 1440
ggaact ggag cct gaggagc t gct gct gga gcccgagccg gagcccgagc ccgaagagga 1500
gccgccccgg ccccgcgccc ccccgggagc t ccgggccct gggcct ggt t cgggagcccc 1560
cggcagccaa gaggaggagg aggagccggg act ggt cgag ggt gacccgg gggacggcgc 1620
cat t gaggac ccggagct gg aagct at caa agct cgagt c agggagat gg aggaagaagc 1680
t gagaagct a aaggagct ac agaacgaggt agagaagcag at gaat at ga gt ccacct cc 1740
aggcaat gct ggcccggt ga t cat gt ccat t gaggagaag at ggaggct g at gcccgt t c 1800
cat ct at gt t ggcaat gt gg act at ggt gc aacagcagaa gagct ggaag ct cact t t ca 1860
t ggct gt ggt t cagt caacc gt gt t accat act gt gt gac aaat t t agt g gccat cccaa 1920
agggt t t gcg t at at agagt t ct cagacaa agagt cagt g aggact t cct t ggcct t aga 1980
t gagt ccct a t t t agaggaa ggcaaat caa ggt gat ccca aaacgaacca acagaccagg 2040
cat cagcaca acagaccggg gt t t t ccacg agcccgct ac cgcgcccgga ccaccaact a 2100
caacagct cc cgct ct cgat t ct acagt gg t t t t aacagc aggccccggg gt cgcgt ct a 2160
caggggccgg gct agagcga cat cat ggt a t t cccct t ac t aaaaaaagt gt gt at t agg 2220
aggagagaga ggaaaaaaag aggaaagaag gaaaaaaaaa agaat t aaaa aaaaaaaaaa 2280
gaaaaacaga agat gacct t gat ggaaaaa aaat at t t t t t aaaaaaaag at at act gt g 2340
gaagggggga gaat cccat a act aact gct gaggagggac ct gct t t ggg gagt agggga 2400
aggcccaggg agt ggggcag ggggct gct t at t cact ct g gggat t cgcc at ggacacgt 2460
ct caact gcg caagct gct t gcccat gt t t ccct gccccc t t cacccccct t gggcct gct 2520
caagggt agg t gggcgt ggg t ggt aggagg gt t t t t t t t a cccagggct c t ggaaggaca 2580
ccaaact gt t ct gct t gt t a cct t ccct cc cgt ct t ct cc t cgcct t t ca cagt cccct c 2640
ct gcct gct c ct gt ccagcc aggt ct acca cccaccccac ccct ct t t ct ccggct ccct 2700
gcccct ccag at t gcct ggt gat ct at t t t gt t t cct t t t gt gt t t ct t t t t ct gt t t t g 2760
agt gt ct t t c t t t gcaggt t t ct gt agccg gaagat ct cc gt t ccgct cc cagcggct cc 2820
agt gt aaat t cccct t cccc ct ggggaaat gcact acct t gt t t t ggggg gt t t aggggt 2880
gt t t t t gt t t t t cagt t gt t t t gt t t t t t t gt t t t t t t t t t t t cct t t gc ct t t t t t ccc 2940
t t t t at t t gg agggaat ggg aggaagt ggg aacagggagg t gggaggt gg at t t t gt t t a 3000

t t t t t t t agc t cat t t ccag gggt gggaat t t t t t t t t aa t at gt gt cat gaat aaagt t 3060

gt t t t t gaaa at 3072

<210> 2
<211> 2658
<212> DNA
<213> Homo sapiens

<400> 2

cgcagaggca ccgccccaag t t t gt t gt ga ccggcggggg acgccggt gg t ggcggcagc 60

ggcggct gcg ggggcaccgg gccgcggcgc caccat ggcg gt gcgacagg cgct gggccg 120

cggcct gcag ct gggt cgag cgct gct gct gcgct t cacg ggcaagcccg gccgggcct a 180

cggct t gggg cggccgggcc cggcggcggg ct gt gt ccgc ggggagcgt c caggct gggc 240

cgcaggaccg ggcgcggagc ct cgcagggt cgggct cggg ct ccct aacc gt ct ccgct t 300

ct t ccgccag t cggt ggccg ggct ggcggc gcggt t gcag cggcagt t cg t ggt gcgggc 360

ct ggggct gc gcgggccct t gcggccgggc agt ct t t ct g gcct t cgggc t agggct ggg 420

cct cat cgag gaaaaacagg cggagagccg gcgggcggt c t cggcct gt c aggagat cca 480

ggcaat t t t t acccagaaaa gcaagccggg gcct gacccg t t ggacacga gacgct t gca 540

gggct t t cgg ct ggaggagt at ct gat agg gcagt ccat t ggt aagggct gcagt gct gc 600

t gt gt at gaa gccaccat gc ct acat t gcc ccagaacct g gaggt gacaa agagcaccgg 660

gt t gct t cca gggagaggcc caggt accag t gcaccagga gaagggcagg agcgagct cc 720

gggggcccct gcct t cccct t ggccat caa gat gat gt gg aacat ct cgg caggt t cct c 780

cagcgaagcc at ct t gaaca caat gagcca ggagct ggt c ccagcgagcc gagt ggcct t 840

ggct ggggag t at ggagcag t cact t acag aaaat ccaag agaggt ccca agcaact agc 900

ccct caccc aacat cat cc gggt t ct ccg cgcct t cacc t ct t ccgt gc cgct gct gcc 960

aggggccct g gt cgact acc ct gat gt gct gccct cacgc ct ccaccct g aaggcct ggg 1020

ccat ggccgg acgct gt t cc t cgt t at gaa gaact at ccc t gt accct gc gccagt acct 1080

t t gt gt gaac acacccagcc cccgcct cgc cgccat gat g ct gct gcagc t gct ggaagg 1140

cgt ggaccat ct ggt t caac agggcat cgc gcacagagac ct gaaat ccg acaacat cct 1200

t gt ggagct g gacccagacg gct gcccct g gct ggt gat c gcagat t t t g gct gct gcct 1260

ggct gat gag agcat cggcc t gcagt t gcc ct t cagcagc t ggt acgt gg at cggggcgg 1320

aaacggct gt ct gat ggccc cagaggt gt c cacggcccgt cct ggcccca gggcagt gat 1380

t gact acagc aaggct gat g cct gggcagt gggagccat c gcct at gaaa t ct t cggct 1440

t gt caat ccc t t ct acggcc agggcaaggc ccacct t gaa agccgcagct accaagaggc 1500

t cagct acct gcact gcccg agt cagt gcc t ccagacgt g agacagt t gg t gagggcact 1560

gct ccagcga gaggccagca agagaccat c t gcccgagt a gccgcaaat g t gct t cat ct 1620

aagcct ct gg ggt gaacat a t t ct agccct gaagaat ct g aagt t agaca agat ggt t gg 1680
ct ggct cct c caacaat cgg ccgccact t t gt t ggccaac aggct cacag agaagt gt t g 1740
t gt ggaaaca aaaat gaaga t gct ct t t ct ggct aacct g gagt gt gaaa cgct ct gcca 1800
ggcagccct c ct cct ct gct cat ggagggc agccct gt ga t gt ccct gca t ggagct ggt 1860
gaat t act aa aagaact t gg cat cct ct gt gt cgt gat gg t ct gt gaat g gt gagggt gg 1920
gagt caggag acaagacagc gcagagaggg ct ggt t agcc ggaaaaggcc t cgggct t gg 1980
caaat ggaag aact t gagt g agagt t cagt ct gcagt cct gt gct cacag acat ct gaaa 2040
agt gaat ggc caagct ggt c t agt agat ga ggct ggact g aggaggggt a ggcct gcat c 2100
cacagagagg at ccaggcca aggcact ggc t gt cagt ggc agagt t t ggc t gt gacct t t 2160
gcccct aaca cgaggaact c gt t t gaaggg ggcagcgt ag cat gt ct gat t t gccacct g 2220
gat gaaggca gacat caaca t gggt cagca cgt t cagt t a cgggagt ggg aaat t acat g 2280
aggcct gggc ct ct gcgt t c ccaagct gt g cgt t ct ggac cagct act ga at t at t aat c 2340
t cact t agcg aaagt gacgg at gagcagt a agt aagt aag t gt ggggat t t aaact t gag 2400
ggt t t ccct c ct gact agcc t ct ct t acag gaat t gt gaa at at t aaat g caaat t t aca 2460
act gcagat g acgt at gt gc ct t gaact ga at at t t ggct t t aagaat ga t t ct t at act 2520
ct gaaggt ga gaat at t t t g t gggcaggt a t caacat t gg ggaagagat t t cat gt ct aa 2580
ct aact aact t t at acat ga t t t t t aggaa gct at gcct a aat cagcgt c aacat gcagt 2640
aaaggt t gt c t t caact g 2658

<210> 3
<211> 604
<212> DNA
<213> Homo sapiens

<400> 3

at gcat t ct g caagccaccc t ggggt gcag ct gagct aga cat gggacgg cgagacgccc 60
agct cct ggc agcgct cct c gt cct ggggc t at gt gccct ggcggggagt gagaaaccct 120
ccccct gcca gt gct ccagg ct gagccccc at aacaggac gaact gcggc t t ccct ggaa 180
t caccagt ga ccagt gt t t t gacaat ggat gct gt t t cga ct ccagt gt c act ggggt cc 240
cct ggt gt t t ccaccccct c ccaaagcaag agt cggat ca gt gcgt cat g gaggt ct cag 300
accgaagaaa ct gt ggct ac ccgggcat ca gccccgagga at gcgcct ct cggaagt gct 360
gct t ct ccaa ct t cat ct t t gaagt gccct ggt gct t ct t cccgaagt ct gt ggaagact 420
gccat t act a agagaggct g gt t ccagagg at gcat ct gg ct caccgggt gt t ccgaaac 480
caaagaagaa act t cgcct t at cagct t ca t act t cat ga aat cct gggt t t t ct t aacc 540
at ct t t t cct cat t t t caat ggt t t aacat at aat t t ct t t aaat aaaac cct t aaaat c 600
t gct 604

<210> 4
<211> 2977
<212> DNA
<213> Homo sapiens

<400> 4

```
gggagagtcg gaagcgcggc ggccgcggag ccctgcgagt aggcagcgtt gggcccatgc 60
aggacgcgga gaacgtggcg gtgcccgagg cggccgagga gcgcgccgag cccggccagc 120
agcagccggc cgccgagccg ccgccagccg aggggctgct gcggcccgcg gggcccggcg 180
ctccggaggc cgcggggacc gaggcctcca gtgaggaggt ggggatcgcg gaggccgggc 240
cggagtccga ggtgaggacc gagccggcgg ccgaggcaga ggcggcctcc ggcccgtccg 300
agtcgccctc gccgccggcc gccgaggagc tgcccgggtc gcatgctgag ccccctgtcc 360
cggcacaggg cgaggcccca ggagagcagg ctcgggacga gcgctccgac agccgggccc 420
aggcggtgtc cgaggacgcg ggaggaaacg agggcagagc ggccgaggcc gaaccccggg 480
cgctggagaa cggcgacgcg gacgagccct ccttcagcga ccccgaggac ttcgtggacg 540
acgtgagcga ggaagaatta ctgggagatg tactcaaaga tcggccccag gaagcagatg 600
gaatcgattc ggtgattgta gtggacaatg tccctcaggt gggacccgac cgacttgaga 660
aactcaaaaa tgtcatccac aagatctttt ccaagtttgg gaaaatcaca aatgattttt 720
atcctgaaga ggatgggaag acaaaagggt atattttcct ggagtacgcg tcccctgccc 780
acgctgtgga tgctgtgaag aacgccgacg gctacaagct tgacaagcag cacacattcc 840
gggtcaacct ctttacggat tttgacaagt atatgacgat cagtgacgag tgggatattc 900
cagagaaaca gcctttcaaa gacctgggga acttacgtta ctggcttgaa gaggcagaat 960
gcagagatca gtacagtgtg atttttgaga gtggagaccg cacttccata ttctggaatg 1020
acgtaaaaga ccctgtctca attgaagaaa gagcgagatg gacagagacg tatgtgcgtt 1080
ggtctcctaa gggcacctac ctggctacct ttcatcaaag aggcattgct ctatgggggg 1140
gagagaaatt caagcaaatt cagagattca gccaccaagg ggttcagctt attgacttct 1200
caccttgtga aaggtacctg gtgaccttta gccccctgat ggacacgcag gatgaccctc 1260
aggccataat catctgggac atccttacgg ggcacaagaa gaggggtttt cactgtgaga 1320
gctcagccca ttggcctatt tttaagtgga gccatgatgg caaattcttt gccagaatga 1380
ccctggatac gcttagcatc tatgaaactc cttctatggg tcttttggac aagaagagtt 1440
tgaagatctc tgggataaaa gacttttctt ggtctcctgg tggtaacata atcgccttct 1500
gggtgcctga agacaaagat attccagcca gggtaaccct gatgcagctc cctaccaggc 1560
aagagatccg agtgaggaac ctgttcaatg tggtggactg caagctccat tggcagaaga 1620
acggagacta cttgtgtgtg aaagtagata ggactccgaa aggcacccag ggtgttgtca 1680
caaattttga aattttccga atgagggaga aacaggtacc tgtggatgtg gtcgagatga 1740
```

aagaaaccat catagccttt gcctgggaac caaatggaag taagtttgct gtgctgcacg 1800
gagaggctcc gcggatatct gtgtctttct accacgtcaa aaacaacggg aagattgaac 1860
tcatcaagat gttcgacaag cagcaggcga acaccatctt ctggagcccc caaggacagt 1920
tcgtggtgtt ggcgggcctg aggagtatga acggtgcctt agcgtttgtg gacacttcgg 1980
actgcacggt catgaacatc gcagagcact acatggcttc cgacgtcgaa tgggatccta 2040
ctgggcgcta cgtcgtcacc tctgtgtcct ggtggagcca taaggtggac aacgcgtact 2100
ggctgtggac tttccaggga cgcctcctgc agaagaacaa caaggaccgc ttctgccagc 2160
tgctgtggcg gccccggcct cccacactcc tgagccagga acagatcaag caaattaaaa 2220
aggatctgaa gaaatactct aagatctttg aacagaagga tcgtttgagt cagtccaaag 2280
cctcaaagga attggtggag agaaggcgca ccatgatgga agatttccgg aagtaccgga 2340
aaatggccca ggagctctat atggagcaga aaaacgagcg cctggagttg cgaggagggg 2400
tggacactga cgagctggac agcaacgtgg acgactggga agaggagacc attgagttct 2460
tcgtcactga agaaatcatt cccctcggga atcaggagtg acctggagca ctgtgcgcag 2520
ccgtgtgtgc tgtggagccg aggccgtcct gcaggaagcc gcgtgactcc cgcctcctcc 2580
ctgtgctctc tggctctgga ctgtgactgc gcctggattc tgccattgcg acacattttt 2640
gtgcctttca gcccctggtg tctgcagtgg gggatttaag gcacccgctt ccacttcttt 2700
cttgtttgga gttttctgtt ggaaccgccg gcgttggctc cgaagactta gcgacgccac 2760
tggcggcacc ttctcctgcg cccagtgatg tttccacggt gcctgtacac agccgagcag 2820
catttccgtt gaaggacttg catccccatt gcgggcagtg ctggacgtgt cccggagacc 2880
caccgggagg gcgccgccat gccttgtacc cccaccgtgc aggttgtggc cggttttctc 2940
cgcaggttga acatggaaat aaaagcaaac ttgtatg 2977

<210> 5
<211> 2002
<212> DNA
<213> Homo sapiens

<400> 5

ggtggttctt gcccgtgttg tgtgtgtgtg tgagtgagag agcgagtgag tgagtgagtg 60
agtgtgtgtg tgggggggac tcggcttgtt gttgtcggtg acttccccct ccccttcacc 120
ccttcccctc cccgccgccg ctgcagtggc cgctccctgg gccgtaggaa atgagcgata 180
acgatgacat cgaggtggag agcgacgaag agcaaccgag gtttcaatct gcggctgaca 240
aacgggctca tcataatgca ctggaacgaa aacgtaggga ccacatcaaa gacagctttc 300
acagtttgcg ggactcagtc ccatcactcc aaggagagaa ggcatcccgg gcccaaatcc 360
tagacaaagc cacagaatat atccagtata tgcgaaggaa aaaccacaca caccagcaag 420
atattgacga cctcaagcgg cagaatgctc ttctggagca gcaagtccgt gcactggaga 480

aggcgaggtc aagtgcccaa ctgcagacca actacccctc ctcagacaac agcctctaca 540
ccaacgccaa gggcagcacc atctctgcct tcgatggggg ctcggactcc agctcggagt 600
ctgagcctga agagccccaa agcaggaaga agctccggat ggaggccagc taagccactc 660
ggggcaggcc agcaataaaa actgtctgtc tccatcgtct catcctcctt tcagttcgtt 720
ggtagagccc tcagaaccat ttaagagact ctttattttt ctctttctcc cttttttttt 780
taaattttta tttttacgta gaagctcttg gacaacagct ctcgttctcc ttccccattt 840
ccactgtata ttttttaatg tattcccttc agggattccc tgtccccaac aggaattttt 900
aaaccaaaac accccaactt ggcagctttt tctgtggagg acagacggcc ggccggacct 960
ctgagcacat agtgtcctgc ccaccctacc agctcctcca gccctgccgg gcacatgccc 1020
gggggacgcc tgccctgccc aggtggcctc ctggcccgcc ctcacctctg atagactttg 1080
tgaatctgaa ctgctctact ttgagaagat gaccggtttg gagtaatcag aatgaaccct 1140
cctccttttt aagggttttt tttttttcct ttttctaaaa agctatgtat cgctcctatt 1200
gaaagaccag atccttagag aagtttgtgg tataaaaagg aagtggggac agattcgcag 1260
cacagagtcg ctggcatgtt tcactcctgc ttctctcagc cagctgttta agcctgcggc 1320
gccagcctca cggagggccg tgtgacactc tcgtggtatg tatgggagat ggcagcagtg 1380
aagcagcagc caccagggag tggccatttg gggttgggac agggagggtg ttttgggtgg 1440
catagaggtt ttgtattgag ggccagtgat gatgttttga tatttatttc ctgctactta 1500
aatttgaatc tgagtgaatt gtacctattt ctgatgatgt cggtcttgca aagcgacaga 1560
ttcataaagt aatgatgaaa tctttctttc ttcccgtgtg tatttctaag aaatagagcc 1620
aactgatttt gtatgtaaat accaagagca atttacctgg tactaaaccc gcaccccagt 1680
gcggaccctt cccagccctc atcccacttc ctttcctact gtcctggaac ctgtctccat 1740
tgtgtgatcc agccctggtt ctggctgtgg tcagcagatg ccagtgaagg gttttgtgtg 1800
tttaggcctc atttctttgt ctttttccta ctccgttcct ggcatttgct gatttctagt 1860
gtatactctg tagtctcagt tcgtgtttga ttccattcca tggaaataaa aagtatgttg 1920
tacatactgc cgaagaattg tcttgcaagt taaggcttcc ccctttacta taagactata 1980
aataaaaact tattttatcc tt 2002

<210> 6
<211> 8469
<212> DNA
<213> Homo sapiens

<400> 6

atggcggcgg cggcgggcgg cggcagttgc cccgggcctg gctccgcgcg gggccgcttc 60
ccgggccggc cgcggggcgc cggcgggggc gggggccgcg gcggacgggg caacggggcc 120
gaaagagtgc gggtagctct gcggcgcggc ggtggcgcga cggggccggg cggagccgag 180

cccggggagg acacggccct gctccgtttg ctggggctcc gccggggcct gcgccggctc 240
cgccgcctgt gggccggccc gcgggtccag cggggccggg gacggggtcg gggccggggc 300
tggggcccga gtcgaggctg cgtgccggag gaggagagca gtgacgggga atccgacgag 360
gaggagtttc agggttttca ttcagatgaa gatgtggccc ccagttccct gcgctctgcg 420
ctccgatccc agcgaggtcg agcgccccga ggtcggggtc gcaagcataa gacgaccccc 480
cttcctcctc ctcgcctagc agatgtggct cctacccccc caaagacccc tgcccggaaa 540
cggggtgagg aaggcacaga acggatggtg caggcactga ctgaacttct ccggcgggcc 600
caggcacccc aagcaccccg gagccgggca tgtgagccct ccacccccg gcggtctcgg 660
ggacggcccc caggacggcc agcaggcccc tgcaggagga agcagcaagc agtagtggtg 720
gcagaagcag ctgtgacaat ccccaaacct gagcccccac ctcctgtggt tccagtgaaa 780
catcagactg gcagctggaa atgcaaggag gggcccggtc caggacctgg gacccccagg 840
cgtggaggac agtcaagccg tggaggccgt ggaggcaggg gccgcggccg aggtggtggg 900
ctcccctttg tgatcaagtt tgtttcaagg gccaaaaaag taaagatggg acaattgtcc 960
ttgggactcg aatcaggtca aggtcaaggt caacatgagg aaagttggca ggatgtcccc 1020
caaagaagag ttggatctgg acagggaggg agcccttgct ggaaaaagca ggaacagaag 1080
ctggatgacg aggaagaaga gaagaaagaa gaagaagaaa aagacaagga gggagaagag 1140
aaggaagaaa gagctgtagc tgaggagatg atgccagctg cggaaaagga agaggcaaag 1200
ctgccaccac cgcctctgac tcctccagcc ccttcacctc ctccacccct cccaccccct 1260
tcgacatctc ctccaccccc actctgccct ccaccaccac ccccagtgtc cccaccacct 1320
ctaccatccc ctccaccgcc tcctgcccaa gaggagcagg aggaatcccc tcctcctgtg 1380
gtcccagcta cgtgctccag gaagaggggc cggcctcccc tgactcccag ccagcgggcg 1440
gagcgggaag ctgctcgggc agggccagag ggcacctctc ctcccactcc aacccccagc 1500
accgccacgg gaggccctcc ggaagacagt cccaccgtgg cccccaaaag caccaccttc 1560
ctgaagaata tccggcagtt tattatgcct gtggtgagtg cccgctcctc ccgtgtcatc 1620
aagacacccc ggcgatttat ggatgaagac ccccccaaac ccccaaaggt ggaggtctca 1680
cctgtcctgc gacctcccat taccacctcc ccacctgttc cccaggagcc agcaccagtc 1740
ccctctccac cacgtgcccc aactcctcca tctaccccag ttccactccc tgagaagaga 1800
cggtccatcc taagggaacc cacatttcgc tggacctcac tgacccggga gctgcccccct 1860
cctcccccag cccctccacc tcccccggcc ccctccccac cccctgctcc tgccacctcc 1920
tcccggaggc ccctactcct tcgggcccct cagtttaccc caagcgaagc ccacctgaag 1980
atctacgaat cggtgcttac tcctcctcct cttggggctc ctgaagcccc tgagccagag 2040
cctcctcctg ccgatgactc tccagctgag cctgagcctc gggcagtggg ccgcaccaac 2100
cacctcagcc tgcctcgatt cgcccctgtg gtcaccactc ctgttaaggc cgaggtgtcc 2160
cctcacgggg ctccagctct gagcaacggg ccacagacac aggctcagct actgcagccc 2220

ctgcaggcct tgcaaaccca gctcctgccc caggcactac cgccaccaca gccacagctg 2280
cagccaccgc cgtcaccaca gcagatgcct cccctggaaa aagcccggat tgcgggcgtg 2340
ggttccttgc cgctgtctgg ggtagaggag aagatgttca gcctcctcaa gagagccaaa 2400
gtgcagctat tcaagatcga tcagcagcag cagcagaagg tggcagcttc catgccgctg 2460
agccctggag ggcagatgga ggaggtggcc ggggctgtca agcagatctc cgacagaggc 2520
cctgtccggt ctgaagatga gtcggtggaa gctaagagag agcggccctc aggtcccgag 2580
tcccctgtgc aaggtccccg catcaaacat gtctgccgtc atgctgctgt ggccctgggt 2640
caggcccggg ccatggtgcc tgaagatgtc cctcgcctca gtgccctccc tctccgggat 2700
cggcaggacc tcgccacaga ggatacatca tcggcgtccg agactgagag tgtcccgtca 2760
cggtcccggc ggggaaaggt ggaggcagca ggccctgggg gagaatcaga gcccacaggt 2820
tctggaggga ccctggccca cacaccccgg cgctcactgc cctcccatca cggcaagaag 2880
atgcgcatgg ctcgatgtgg acactgtcgg ggctgcctac gtgtgcagga ctgtgggtcc 2940
tgtgtcaact gcctagacaa gcccaagttt gggggcccta acaccaagaa gcagtgctgt 3000
gtataccgga agtgtgacaa aatagaggct cggaagatgg aacgactggc taaaaaaggc 3060
cggacgatag tgaagacgct gttgccctgg gattccgatg aatctcctga ggcctcccct 3120
ggtcctccag gcccacgccg gggggcggga gctggggggc cccgggagga ggtggtggcc 3180
cacccagggc ccgaggagca ggactccctc ctgcagcgca agtcagctcg gcgctgcgtc 3240
aaacagcgac cctcctatga tatcttcgag gattcggatg actcggagcc cgggggcccc 3300
cctgctcctc ggcgtcggac cccccgagaa aatgagctgc cactgccaga acctgaggag 3360
cagagccggc cccgcaaacc taccctgcag cctgtgttgc agctcaaggc ccgaaggcgc 3420
ctggacaagg atgctttggc cctggccccc tttgcttctt ttcccaatgg ctggactgga 3480
aagcagaagt ctcccgatgg tgtgcaccgc gtccgtgtgg attttaagga ggattgtgat 3540
ttagagaacg tgtggctgat ggggggcctg agtgtgctca cctctgtgcc agggggcccc 3600
ccgatggtgt gcttgctgtg tgccagcaaa ggactccacg agctggtgtt ctgtcaagtc 3660
tgctgtgacc cattccaccc attctgcctg gaggaggccg agcggcccct gccccagcat 3720
cacgacacct ggtgctgccg tcgctgcaaa ttctgccacg tctgtggacg caaaggtcgt 3780
ggatccaagc acctcctgga gtgcgagcgc tgccgccatg cataccaccc ggcctgtctg 3840
gggcccagct atccaacccg ggccacgcgc aaacggcgcc actggatctg ttcagcctgt 3900
gtgcgctgta agagctgtgg ggcaactcca ggcaagaact gggacgtcga gtggtctgga 3960
gattacagcc tctgccccag gtgcacccag ctatatgaga aaggaaacta ctgcccgatc 4020
tgtacacgct gctatgaaga caacgactat gagagcaaga tgatgcagtg cgcacagtgc 4080
gatcactggg tgcatgccaa gtgcgagggg ctctcagatg aagactacga gatcctttca 4140
ggactgccag actcggtgct gtacacctgc ggaccgtgtg ctggggcagc gcagccccgc 4200
tggcgagagg ccctgagcgg ggccctccag ggggggcctgc gccaggtgct ccagggcctg 4260

ct gagct cca aggt ggt ggg cccact gct g ct ct gcaccc agt gt gggcc agat gggaag 4320
caact gcacc caggaccct g cggcct gcaa gct gt gagt c agcgct t cga ggat ggccac 4380
t acaagt ct g t gcacagct t cat ggaggac at ggt gggca t cct cat gcg gcact cggag 4440
gagggagaga ccccggaccg ccgggct gga ggccagat ga aggggct cct gct gaagct g 4500
ct agaat ct g cgt t cggct g gt t cgacgcc cacgacccca agt act ggcg acggagt acc 4560
cggct gccaa acggagt cct t cccaat gcg gt gt t gcccc cat ccct gga t cat gt ct at 4620
gcgcagt gga gacagcagga accagagacc ccagaat cag ggcagcct cc aggggat ccc 4680
t cagcagcat t ccagggcaa ggat ccggct gcct t ct cac acct ggagga cccccgt cag 4740
t gt gcact ct gcct caaat a cggggat gca gact ccaagg aggcggggcg gct ct t gt ac 4800
at cgggcaga acgagt ggac acacgt caac t gt gccat ct ggt cggcgga agt ct t cgag 4860
gagaacgacg gct ccct caa gaat gt gcat gct gct gt gg cccgagggag gcagat gcgc 4920
t gcgagct ct gcct gaagcc t ggcgccacg gt gggct gct gcct gt cct c ct gcct cagc 4980
aact t ccact t cat gt gt gc ccgggccagc t act gcat ct t ccaggat ga caagaaagt c 5040
t t ct gccaga aacacact ga t ct cct ggat ggcaaggaaa t t gt gaaccc cgat ggt t t t 5100
gat gt t ct cc gccgagt ct a t gt ggact t c gagggcat ca act t caagcg gaagt t ct t g 5160
acggggct t g aacccgat gc cat caacgt g ct cat t ggt t ccat ccgcat t gact ccct g 5220
ggt act ct gt ct gat ct ct c ggact gcgag ggacggct ct t ccccat t gg ct accagt gc 5280
t cccgt ct gt act ggagcac agt ggat gct cggaggcgct gct ggt at cg gt gccgaat t 5340
ct ggagt at c ggccat gggg gccgagggaa gagccagct c acct ggaggc t gcagaggag 5400
aaccagacca t t gt gcacag ccccgcccct t cct cagagc ccccaggt gg t gaggacccc 5460
ccact ggaca cagat gt t ct t gt ccct gga gct cct gagc gccact cgcc cat t cagaac 5520
ct ggaccct c cact gcggcc agat t caggc agcgcccct c ct ccagcccc ccgt t ct t t t 5580
t cgggggct c gaat caaagt gcccaact ac t cgccat ccc ggaggccct t ggggggt gt c 5640
t cct t t ggcc ccct gccct c ccct ggaagt ccat ct t cac t gacccacca cat ccccaca 5700
gt gggagacc cggact t ccc agct cccccc agacgt t ccc gt cgt cccag ccct t t ggct 5760
cccaggccgc ct ccat cacg gt gggcct cc cct cct ct aa aaacct cccc t cagct cagg 5820
gt gccccct c ct acct cagt cgt cacagcc ct cacacct a cct cagggga gct ggct ccc 5880
cct ggcccgg ccccat ct cc accacccccct gaagacct gg gcccagact t cgaggacat g 5940
gaggt ggt gt caggact gag t gct gct gac ct ggact t cg cggccagcct gct ggggact 6000
gagccct t cc aggaagagat t gt agccgct ggggccat gg ggagcagcca cgggggcccg 6060
ggggacagct ccgaggagga gt ccagcccc acct cccgct acat ccact t ccct gt gact 6120
gt ggt gt ccg cccct ggt ct ggcccccagc gct acccct g gagcccccg cat t gaacag 6180
ct ggacggcg t ggacgacgg cact gacagt gaggct gagg cggt gcagca gcct cggggc 6240
cagggcacgc ct cct t cggg gccaggagt a gt ccgggcag gggt cct t gg ggct gcaggg 6300

gacagggccc ggcct cct ga ggacct gcca t cggaaat t g t ggat t t t gt gt t gaagaac 6360
ct agggggt c ct ggggat gg aggt gct ggc cct agagagg agt cact ccc cccggcgcct 6420
cccct ggct a at ggcagcca gccct cccaa ggcct gaccg ccagcccagc t gaccccacc 6480
cgcacat t t g cct ggct ccc aggggcccca ggggt ccggg t gt t aagcct t ggccct gcc 6540
cct gagcccc ccaaacccgc cacat ccaaa at cat act t g t caacaagct ggggcaagt a 6600
t t t gt gaaga t ggct gggga gggt gaacct gt cccacccc cagt gaagca gccacct t t g 6660
ccccccacca t t t cccccac ggct cccacc t cct ggact c t gcccccagg cccccct cct c 6720
ggcgt gct gc ccgt ggt cgg agt ggt ccgc cct gccccgc ccccgccacc ccct cccct g 6780
acgct ggt gc t gagcagt gg gccagccagc ccgccccgcc aggccat ccg cgt caagagg 6840
gt gt ccact t t ct ccggccg gt ccccgcca gcacct cccc cat acaaagc cccccggct g 6900
gat gaagat g gagaggcct c agaggat acc cct caggt t c cagggct t gg cagt ggcggg 6960
t t t agccgt g t gaggat gaa aacccccaca gt gcgt gggg t cct t gacct ggat cggcct 7020
ggggagcccg ct ggggaaga aagt cct ggg cccct ccagg aacggt cccc t t t gct gcca 7080
ct t ccggaag at ggt cct cc ccaggt cccc gat ggt cccc cagacct gct gct t gagt cc 7140
cagt ggcacc act at t cagg t gaggct t cg agct ct gagg aagagcct cc at ccccagat 7200
gat aaagaga accaggcccc aaaacggact ggcccacat c t gcgct t cga gat cagcagt 7260
gaggat gggt t cagcgt t ga ggcagagagc t t ggaggggg cgt ggagaac t ct gat cgag 7320
aaagt gcaag aggcccgagg gcat gcccga ct cagacat c t ct cct t t ag t ggaat gagt 7380
ggggcgagac t cct gggcat ccaccat gat gct gt cat ct t cct ggccga gcagct cccc 7440
ggagcccagc gt t gccagca ct at aagt t c cgt t accacc agcagggaga gggccaggag 7500
gagccgcccc t gaat cccca t ggggct gct cgggcagagg t ct at ct ccg gaagt gcacc 7560
t t t gacat gt t caact t cct ggcct cccag caccgggt gc t ccct gaggg ggccacct gt 7620
gat gaggaag aggat gaggt gcagct cagg t caaccagac gt gccaccag cct ggagct g 7680
cccat ggcca t gcgt t t t cg t cacct t aag aagacgt cca aagaagct gt gggt gt ct ac 7740
agat cagcca t ccacgggcg aggcct gt t c t gt aagcgca acat cgacgc gggggagat g 7800
gt cat cgagt act ct ggcat t gt cat ccgc t cggt gt t ga ct gacaagcg ggagaagt t c 7860
t acgat ggga agggcat cgg gt gct at at g t t ccgcat gg at gact t t ga t gt agt ggac 7920
gccacgat gc at ggcaat gc cgcccgct t c at caaccact cct gt gagcc caact gct t c 7980
t ct cgggt ca t ccacgt gga gggccagaaa cacat t gt t a t ct t cgccct gcgccgcat c 8040
ct gcgt ggt g aggagct cac ct acgact ac aagt t cccca t cgaggat gc cagcaacaag 8100
ct gccct gca act gt ggcgc caagcgct gc cgt cggt t cc t t aact gagg ccgt ggct gc 8160
ccaccacgac ccct cacacc t cct gct gcc gt cgct gcca t ct t gcccct agcct ggggg 8220
ct ccct agcc cct cccagag cat ct caccc ccacccct cat gt t cagggt g gat gt gggca 8280
t gcaggt gac aagggccct g cct ccacccc t ccagcccat ccagcaat cg cccccct t t ct 8340

gccct ggggg cccaggat gt agat at t gt a caaaggt t t c t aaat ccct t ct t t t ct at g 8400
cact t t t t t a t t t aagaggt ggggt cccag gt gggaaccc ccccacaat a aagt ct gt ca 8460
at gt t t gga 8469

<210> 7
<211> 3704
<212> DNA
<213> Homo sapiens

<400> 7

cagcagcgt g ct aagaagca gt cacat aaa cagcagcagg agt aggcct c ct gct t t t ca 60
aaagcagagt act gcagggt cgcgaaat gc aagacact ca gat gt t t gaa aat ct cccga 120
gt t gagaat g gct act gt aa aagcgt cacc aagaaact ct gacgat ct gg acagt cct aa 180
ct ct gt gt t a gcaat act t a ct t ccggaaa at t aat gct a ct t ct t gt ag at t t t t gcaa 240
at aggaaacc ccct t gaaga agat ct caaa t t acgccccc cacccccaaa aaaagacaaa 300
caggggagaa caaagt t t t g gcat gcct gc aggaacggt g gct t t t t t ag aaact acct a 360
ggaggcagaa gct aagt gat t t gct cat gc ct ct t acct g ggagt agaag gt gggaagaa 420
at ggaccgag gct gt gacga gaagacaagg cacagt gcag ct t ggt gaag ccacacgct g 480
act gcgt t ct gccccct ct t cat gcagt gc t gcgggct gg t gcat cgccg gcgagt acgg 540
gt gt cct at g gt t cggcaga ct cct acact agccgt ccat ccgat t ccga t gt at ct ct g 600
gaggaggacc gggaggcagt gcgcagagaa gcggagcggc aggcccaggc acagt t ggaa 660
aaagcaaaga caaagcccgt t gcat t t gcg gt t cggacaa at gt cagct a cagt gcggcc 720
cat gaagat g at gt t ccagt gcct ggcat g gccat ct cat t cgaagcaaa agat t t t ct g 780
cat gt t aagg aaaaat t t aa caat gact gg t ggat agggc gat t ggt aaa agaaggct gt 840
gaaat cggat t cat t ccaag cccagt caaa ct agaaaaca t gaggct gca gcat gaacag 900
agagccaagc aagggaaat t ct act ccagt aaat caggag gaaat t cat c at ccagt t t g 960
ggt gacat ag t acct agt t c cagaaaat ca acacct ccat cat ct gct at agacat agat 1020
gct act ggct t agat gcaga agaaaat gat at t ccagcaa accaccgct c ccct aaaccc 1080
agt gcaaaca gt gt aacgt c accccact cc aaagagaaaa gaat gccct t ct t t aagaag 1140
acagagcaca ct cct ccgt a t gat gt ggt a cct t ccat gc gaccagt ggt cct agt gggc 1200
cct t ct ct ga agggct acga ggt cacagat at gat gcaaa aagcgct gt t t gat t t t t t a 1260
aaacacagat t t gaagggcg gat at ccat c acaagggt ca ccgct gacat ct cgct t gcc 1320
aaacgct cgg t at t aaacaa t cccagt aag cacgcaat aa t agaaagat c caacacaagg 1380
t caagct t ag cggaagt t ca gagt gaaat c gaaaggat t t t t gaact t gc aagaacat t g 1440
cagt t ggt gg t cct t gacgc ggat acaat t aat cat ccag ct caact cag t aaaacct cc 1500
t t ggcccct a t t at agt at a t gt aaagat t t ct t ct cct a aggt t t t aca aaggt t aat a 1560

aaat ct cgag ggaaat ct ca agct aaacac ct caacgt cc agat ggt agc agct gat aaa 1620
ct ggct cagt gt cct ccaga gct gt t cgat gt gat ct t gg at gagaacca gct t gaggat 1680
gcct gt gagc acct t gccga ct at ct ggag gcct act gga aggccaccca t cct cccagc 1740
agt agcct cc ccaaccct ct cct t agccgt acat t agcca ct t caagt ct gcct ct t agc 1800
cccaccct ag cct ct aat t c acagggt t ct caaggt gat c agaggact ga t cgct ccgct 1860
cct at ccgt t ct gct t ccca agct gaagaa gaacct agt g t ggaaccagt caagaaat cc 1920
cagcaccgct ct t cct cct c agccccacac cacaaccat c gcagt gggac aagt cgcggc 1980
ct ct ccaggc aagagacat t t gact cggaa acccaggaga gt cgagact c t gcct acgt a 2040
gagccaaagg aagat t at t c ccat gaccac gt ggaccact at gcct caca ccgt gaccac 2100
aaccacagag acgagaccca cgggagcagt gaccacagac acagggagt c ccggcaccgt 2160
t cccgggacg t ggat cgaga gcaggaccac aacgagt gca acaagcagcg cagccgt cat 2220
aaat ccaagg at cgct act g t gaaaaggat ggagaagt ga t at caaaaaa acggaat gag 2280
gct ggggagt ggaacaggga t gt t t acat c ccccaat gag t t t t gccct t t t gt gt t t t t 2340
t t t t t t t t t t t t t t gaagt c t t gt at aact aacagcat cc ccaaaacaaa gt ct t t gggg 2400
t ct acact gc aat cat at gt gat ct gt ct t gt aat at t t t gt at t at t gc t gt t gct t ga 2460
at agcaat ag cat ggat aga gt at t gagat act t t t t ct t t t gt aagt gc t acat aaat t 2520
ggcct ggt at ggct gcagt c ct ccggt t gc at act ggact ct t caaaaac t gt t t t gggt 2580
agct gccact t gaacaaaat ct gt t gccac ccaggt gat g t t agt gt t t t aagaaat gt a 2640
gt t gat gt at ccaacaagcc agaat cagca cagat aaaaa gt ggaat t t c t t gt t t ct cc 2700
agat t t t t aa t acgt t aat a cgcaggcat c t gat t t gcat at t cat t cat ggaccact gt 2760
t t ct t gct t g t acct ct ggc t gact aaat t t ggggacaga t t cagt ct t g cct t acacaa 2820
aggggat cat aaagt t agaa t ct at t t t ct at gt act agt act gt gt act gt at agacag 2880
t t t gt aaat g t t at t t ct gc aaacaaacac ct cct t at t a t at at aat at at at at at at 2940
at cagt t t ga t cacact at t t t agagt ct t aat gccaagt cagcagat t t gct t t at gaa 3000
t t acagggac t agaaat gcc cacat t cagg aaat t t gt aa t aacat t gt c t agacacct a 3060
t cct cat t ct agt agaaagt gt gt acat ac t gt aaat at g t gt gat t gct t gact t gaaa 3120
aggt t t gaat t ct gaat gt t at accat cct t gt aagt aag t t t gt aat t t ccaccat aaa 3180
t t at ggt aaa t at aaaact c cagaggt t gt t ct act ccat acagt t caca ct gat t gt ga 3240
cacat t ct t a gt agct agt g t ct gt t ct ag t cact gcact ggagt ct acg agccggaact 3300
cgct at at gc acgt gt gt gt gt ccgt at gt aagaaagt gt gcaccgagt g act gaat ggt 3360
t gagat gaat t ggaat gct g aagact aacg aagaaact ag agact gat at cgagcat t ct 3420
gcccacct cg ct ct gt at t t aat t aat t gt gct at at gt t gct t t aacaa cccat t gagc 3480
agt cagggaa t gt gagt aag ct t gct gcca aaggt aact a ggaaagcat t cat ct gct gc 3540
ct cct t gt t t t t gct cct ag agagt gaaaa t acaggcaat t t t act gt ga gt gt t t cact 3600

ggaaatgtac aatctttgtg tgttagagta tttgttttag taagaaatgt ttacacagct 3660
tgtggaatta tttcgtggga aataaatttt ttataacttc tccc 3704

<210> 8
<211> 4184
<212> DNA
<213> Homo sapiens

<400> 8

ccggggctga gctgcggcgg cgcctgacaa tgccccctcg cgccccggcc tcggcgcccg 60
ctcccggctc cagcgcgcct gcacgtacgt agccttcacg tgtgtgtgga tacggagtgc 120
atgtgtggag acagaggaat atgctccaac aaatttcgga tagaggcgcc gaagacagga 180
cgtggggaag ctaaagaagt aaacaggtca tggcacgttt aacaaaaaga cgacaggcgg 240
atacaaaagc tatccagcat ctttgggcag ccattgagat tatacggaac cagaagcaga 300
ttgccaacat tgaccgtatt acaaagtata tgtctcgagt ccacggtatg caccctaaag 360
agaccacccg tcagctgagc ttagctgtga aagatggtct tattgtcgaa actctaacag 420
tgggctgcaa aggttcaaaa gctggtattg aacaagaagg atattggttg ccaggagatg 480
agattgactg ggaaacagaa aatcatgact ggtattgttt tgaatgccat ttgcctggag 540
aggtgttgat atgtgacctg tgttttcgtg tgtatcattc caagtgtttg tctgatgagt 600
tcaggcttag agacagcagt agtccctggc agtgcccagt ttgcaggagc attaagaaga 660
agaatacaaa caaacaggag atgggcacat acctcagatt cattgtctcc cgcatgaagg 720
agagggctat agatcttaat aaaaagggga aggacaataa acacccgatg tacaggaggc 780
tggtgcactc agctgtggac gttcccacca ttcaagagaa agtgaatgaa gggaaatacc 840
gaagttatga agagttcaaa gctgatgccc aattgcttct ccacaatacc gtgattttct 900
atggagcaga cagtgagcaa gctgacattg cgaggatgct atataaagac acatgtcatg 960
agctggatga actgcagctt tgcaagaatt gcttttactt gtcaaatgct cgtcctgaca 1020
actggttctg ttatccttgt atacctaatc atgagctggt ttgggctaaa atgaaaggtt 1080
ttgggttttg gccagccaaa gtcatgcaga aagaagacaa tcaagtcgac gttcgcttct 1140
ttggccacca ccaccagagg gcctggattc cttctgaaaa cattcaagat atcacagtca 1200
acattcatcg gctgcacgtg aagcgcagta tgggttggaa aaaggcctgt gatgagctgg 1260
agctgcatca gcgtttccta cgagaaggga gattttggaa atctaagaat gaggaccgag 1320
gtgaggaaga ggcagaatcc agtatctcct ccaccagtaa tgagcagcta aaggtcactc 1380
aagaaccaag agcaaagaaa ggacgacgta atcaaagtgt ggagcccaaa aaggaagaac 1440
cagagcctga aacagaagca gtaagttcta gccaggaaat acccacgatg cctcagccca 1500
tcgaaaaagt ctccgtgtca actcagacaa agaagttaag tgcctcttca ccaagaatgc 1560
tgcatcggag cacccagacc acaaacgacg gcgtgtgtca gagcatgtgc catgacaaat 1620

acaccaagat cttcaatgac ttcaaagacc ggatgaagtc ggaccacaag cgggagacag 1680
agcgtgttgt ccgagaagct ctggagaagc tgcgttctga aatggaagaa gaaaagagac 1740
aagctgtaaa taaagctgta gccaacatgc agggtgagat ggacagaaaa tgtaagcaag 1800
taaaggaaaa gtgtaaggaa gaatttgtag aagaaatcaa gaagctggca acacagcaca 1860
agcaactgat ttctcagacc aagaagaagc agtggtgcta caactgtgag gaggaggcca 1920
tgtaccactg ctgctggaac acatcctact gctccatcaa gtgccagcag gagcactggc 1980
acgcggagca caagcgcacc tgccgccgga aaagatgaag ctggcccttc ccggagtcac 2040
cccgatgatt actcttttca gacacagcgg tttttgtttc caagaagcca aaattgttta 2100
gaatttgctt cccattttgc accagccttt aaacactttt cgtgaagaaa ttttgcacag 2160
tagtttaaat cttttgttaa tgctcctccg aagtttttca gggggtaaaa gtaacatcag 2220
tggagggtat tattttaaat aaattttaat tgagaatttg ttgcattttc agcaaatttt 2280
aaaacatttt taggttttac agagatttta acctttaaac aacagatctt taaaaaacag 2340
gtgaatacaa gtgagtttaa caaagaaaca tttagaatag atctgaatgt aagaactaca 2400
gaactgtttc agaaataaaa catactacct tgatgtgaca tttttttctt aaccttgttg 2460
agctggtttt gttcagctta atttactgtt caaaggcatt atctgttggt cacaccagtg 2520
ggtatatgat tgaatttagg gaacagggtt gacacagcag ggctagtcct gcatattttt 2580
tcttaaatat ttcccaattg tgtttttcat tatttctttt caatatataa cttttataac 2640
aaattattag ctttgatctt gtagtttaaa attgcaggga actggggtaa tcttttactg 2700
agctggatct tagagaaaat gaatatttaa attttaaagt ttgcacattt catctttgtc 2760
ctaacatgag tgcttgtaac aaaataaaca acaaaaacaa agccaaaaac tacctttatc 2820
catatgtgaa attatagatg aggcatacga atttgtttaa tgcttccctt cccttcccac 2880
atatcatctc actgcctatt atctggtgtc acctcatgta tcgtaagtta atactaaaag 2940
aagagaaagc acttaagttt cacagaagcc gttatgtttg tagtaatggg tcattgccta 3000
ctaatgaact ccatcactgt acacagaatg aagaataatg catgttaatt ttcttgtatt 3060
aaagatgccg tgatttgtaa aaagtctgta ttttgcggaa tgtctggatt aagaagcatt 3120
accaatagga atggatcgat agttgaataa tgatttttta tacatagata tataaaatac 3180
agccaggaaa acttaaatta cttttctttt aaaatatctc acaatttatg tggtattttt 3240
aaagactgat cttagaccaa gtcaaattcc catttatcat ttagttattt tttgaggtta 3300
gagaataatt tgtacatatt tatttagacc tttgatattt attaaagcaa ttactcacaa 3360
tggaagtgaa agaatcagga gaaaagcctt ttaaaaagta ttctccaggt ttgtcagggt 3420
cactctaaag ataaaaatgt aactaagtct tctgtgaaat atcatccatc taatcttgat 3480
gctgttgcag atggtggtga cacaagttaa ttgacaaact actgccaaat ggtgcacaat 3540
attttgtaaa aagtacccag tagccccatt tcatacaatg tacctaaatt atgcagtaac 3600
ttggcatcat cgttccctcc ttgttgctgt gtaattagtc agtgttgcca cagtgtgtgg 3660

cgctgatgga gatgtcagaa ccgagaacac ttaaccttct ttgattgttt ttcaagtttt 3720
aagacttcga tccaccccta tgagagcaag taattgtgga aatatttttg gtgtaaaatc 3780
attccagagt atgtaatatt taactgatag ctgcatgaaa gtgagattcg tgttactttg 3840
gcttttctgt ctctgttgac acggttgcac atttccaagt tactacagcg tgaaaactgt 3900
gtgtttaaaa aaaaacaaaa ttaaaaaaag agattgtggc ctggttttgt aaaagtttta 3960
ggtaaaatta caattgattg ttttaggaat cattattaaa aattttctgc aaatcataaa 4020
gctatatcga ggtgttgagc ttagccacta tgcacattgt aattattcat tgtggctgtc 4080
cagttctatg atgcattctg gcattttgta agctgctctg actagcaata tatagattca 4140
tttaatgtgt taacattggt taataaatgt atttaaaaaa aact 4184

<210> 9
<211> 6714
<212> DNA
<213> Homo sapiens

<400> 9

ccctaggccc gggtcccgga tccccgcgca cccggccagg ctctggcacg ttttggggga 60
ggtgcctgca ggacccaaca tactcaatga gcttccagcg caatgtccga tcgctcgggg 120
ccgactgcca agggaaagga tggaaagaag tattcctcgc tcaacctgtt tgatacgtat 180
aagggcaagt ccttagagat ccagaaaccc gctgttgccc ctcgccatgg cctgcagagt 240
ctcgggaaag ttgccattgc ccggcgtatg ccacctccag ccaaccttcc aagcctgaaa 300
gccgagaaca aaggcaatga ccccaatgtc tcactagtgc caaaagacgg aacaggatgg 360
gcaagcaaac aggagcagtc cgaccccaag agttccgatg cctcaaccgc tcagccgccg 420
gaatcgcagc cactgccggc ttcacagacg cctgcctcca accagccgaa acgaccccca 480
gcagcccccg agaacactcc tttggttcca agcggggtaa agtcctgggc acaagccagc 540
gtcacccatg gagcacatgg agatggtgga agggcatcaa gcctactgtc acgattctct 600
cgagaggaat ttccgaccct gcaggcggct ggcgaccagg acaaggctgc caaggaaagg 660
gagtctgccg aacagtcgtc tgggcccgga ccaagcctcc gcccccaaaa ttctacaact 720
tggagggacg gaggtgggcg tggccctgat gagctggagg gcccggactc caaacttcat 780
catggtcatg atccccgggg tgggctacag ccttcaggcc caccccagtt ccctccctac 840
cgcggaatga tgccgccttt catgtatccc ccatatctcc cgttccctcc gccctatgga 900
ccccaggggc cttaccgata ccccactcct gatgggccca gccgttttcc ccgtgtggcg 960
ggcccccgag gctcagggcc accaatgcgc ttagtagagc ctgtgggtcg tccctctatt 1020
ctcaaagagg ataatctcaa agagtttgat cagttggatc aggagaatga tgatggttgg 1080
gcaggggccc atgaagaggt tgactacact gaaaagctca agttcagcga tgaggaagat 1140
gggcgagact ctgatgagga gggagctgag ggccacaggg attcccaatc agcttctggt 1200

gaggaacggc cccct gaagc agat ggcaaa aagggcaact cccccaacag cgaaccgccc 1260
act cct aaga cggcct gggc agaaacct ct cggcct ccag agacagagcc gggacct cct 1320
gccccaaagc ct cccct acc ccct ggggac t acccagat c gt gggggt cc t ccct gcaag 1380
cccccagcac ct gaagat ga ggat gaggca t ggcggcagc gacgaaagca gt cgt cat ct 1440
gagat t t ccc t ggcagt gga gcgggcccgg cgacggcgag aagaagagga gcggcgcat g 1500
caagaagagc gccgggcagc ct gt gct gag aagct caagc gact cgat ga aaagt t t ggg 1560
gcacct gaca agcggct caa agcagagcct gct gccccac ct gct gcccc t t ct acccca 1620
gct ccaccac ct gcagt ccc t aaagaact c cct gcacct c cagct ccacc t ccagcat ca 1680
gccccaacac cagagaaaga acct gaagag ccagcacagg cccct cct gc ccaat ct act 1740
cct act ccag gt gt ggct gc ggct cccact ct ggt gagt g gt ggt ggcag t accagt agc 1800
accagcagt g gcagct t cga agccagccca gt ggaaccac aact gccct c aaaagagggt 1860
cct gaaccac cagaagaggt t cct cct cct accacacccc cagt t ccaaa ggt ggaaccc 1920
aagggt gat g ggat t ggt cc cacccgccag ccccct agt c agggct t ggg ct accccaaa 1980
t at cagaagt cgt t gcct cc t cgt t t ccag cggcagcagc aggagcagct cct gaagcag 2040
cagcagcagc accagt ggca gcagcat caa cagggct ct g cccct cct ac cccagt gccc 2100
ccat caccac cacagcct gt gaccct gggg gct gt gccag ct ccacaggc t ccacccccg 2160
ccccccaagg ccct gt accc aggt gct ct g ggccggcccc cacccat gcc cccaat gaac 2220
t t t gat cccc gat ggat gat gat t cct cct t at gt ggacc cccggct cct ccagggt cgt 2280
ccccct ct ag act t ct accc t cct ggt gt g cat ccct ct g gcct agt t cc ccgagagcgt 2340
t cagacagt g ggggct caag ct cagagcca t t t gaccgt c at gcacct gc t at gt t acgg 2400
gaacggggca ct ccaccggt ggat ccaaag t t ggcct ggg t aggagat gt ct t caccgcc 2460
acacccgct g aaccccgccc act t acct ca cct ct gcgcc aggct gcgga t gaggat gac 2520
aaggggat ga ggagcgagac t cct ccagt a cct cccccac caccct at ct ggccagt t at 2580
ccaggct t t c ct gagaat gg agcccct ggg cccccaat ct ct cgct t t cc t ct ggaggaa 2640
ccagggcccc gt ccact ccc ct ggcccccca ggcagt gat g aagt ggccaa gat acaaact 2700
ccaccaccca agaaggagcc ccct aaggag gagact gcac agct gacggg gccagaagca 2760
ggccgaaagc ct gcccgcgg agt cgggagt ggaggccagg gcccccccacc accacgcaga 2820
gagagt cgca cagagacccg ct ggggccct cgt ccaggga gcagt cgt cg t ggaat ccct 2880
ccagaggagc caggggcccc accccgccgg gct gggcct a t aaagaaacc t ccaccacct 2940
acaaaagt ag aagagct gcc t cccaagccc ct cgaacagg gggat gaaac ccccaaaccc 3000
ccaaagccag acccact caa gat aaccaag gggaagct ag ggggccccaa ggagacccca 3060
cccaat ggaa at ct t t cccc t gccccaagg ct t cggaggg act at t cgt a t gaaagagt g 3120
ggt cct acct ct t gccgggg t cggggccga ggcgagt at t t t gccagagg gaggggt t t t 3180
cgggggacct at gggggacg agggcgggga gcccgaagcc gggaat t ccg cagt t accga 3240

gagt t t cgag gagat gat gg gcgt ggaggt gggacagggg gaccaaacca ccct cct gct 3300
ccccgaggcc gcact gccag cgagacacgg agcgagggt t cagagt at ga ggaaat cccc 3360
aagcggcgcc ggcagcgggg ct cagaaaca ggcagcgaga cccat gagag t gat ct ggct 3420
cct t cagaca aggaggct cc cacacccaag gagggaacac t cacccaggt ccct ct cgct 3480
cccccaccac caggagcccc acct t cacca gccccagccc gct t cact gc ccggggt ggg 3540
cgagt ct t ca ct cccagagg ggt gccat ct cgccggggcc gaggaggagg gaggcccccct 3600
cct caagt t t gcccaggct g gagccct cca gccaagt ct c t ggct cccaa gaaacct ccc 3660
acaggccct t t gccaccaag t aaggagcct t t gaaagaga agt t gat ccc agggcct ct g 3720
t cccct gt gg cgcgcggagg cagcaat gga ggt agcaat g t gggcat gga agat ggggag 3780
cgaccccgaa ggaggcgaca t gggagggct cagcagcagg at aaaccgcc t cgt t t ccgg 3840
aggct gaagc aggaacggga gaat gccgca agggggt ct g agggcaagcc ct ccct aacc 3900
ct t ccagcct ccgct cct gc acct gaggag gccct cacaa cagt cacagt ggccccagca 3960
cct cgccggg cagct gccaa gt ct cct gat ct gt caaacc agaact caga ccaagccaat 4020
gaggaat ggg agact gcat c agagagcagt gact t cacca gt gagcgccg aggggacaaa 4080
gaggcacccc caccagt act gct gacaccc aaggct gt gg gaact cct gg gggaggt gga 4140
ggt ggagccg t accaggt at t t cagccat g t cccgcggag at ct gagcca gagagccaag 4200
gat t t gagt a aacggagct t ct caagt cag cggccaggca t ggaacggca gaat cggcgc 4260
cct ggcccag ggggcaaggc t ggcagcagt ggcagcagca gt ggaggagg cggt gggggt 4320
cct ggaggaa ggaccgggcc aggacgaggc gacaagagga gct ggccct c t cccaagaac 4380
cgaagt cgt c ct ccagagga gcgt cccccg gggct t cccc t gcct ccccc acct cccagc 4440
agt t ct gct g t ct t ccgcct ggaccaagt t at ccacagca accct gct gg cat ccaacag 4500
gct ct ggccc agct t agt ag ccgt caaggg agt gt aact g caccaggggg t cat ccaagg 4560
cacaagcct g ggcct cccca agcccct cag ggcccct ct c ct aggccccc aacccgat ac 4620
gagccccaga gggt caacag cggcct cagt t ct gaccccc act t t gagga gccggggcca 4680
at ggt gagag gggt gggt gg gact cct cgg gact ct gccg gggt t agt cc ct t t ccccct 4740
aaacgt cggg agcggcct cc cagaaaacca gagct gct ac aggaggaat c t t t gccacct 4800
cct cat agct ct ggat t ct t gggct ct aag cct gagggcc caggccct ca ggcagagt cc 4860
agagat acag gcacagaggc cct gacccct cacat ct gga accgt t t aca t act gccact 4920
agccgaaaga gt t accggcc cagct ccat g gagcct t gga t ggagcccct gagt cct t t t 4980
gaggat gt gg ct ggcacaga aat gagt cag t ct gacagt g gggt ggacct gagt ggggat 5040
t ct caggt gt cat caggt cc ct gcagccag cgaagt t ccc ct gat ggagg act caagggg 5100
gcagcagagg gacccccaa gaggcct gga ggct cct cac ccct gaat gc t gt t cct t gt 5160
gagggt ccac ct ggct ct ga acct cct agg agaccaccac ct gcccccca cgat ggggac 5220
agaaaggagc t gccccggga gcagcct ct g ccccct ggcc ccat t ggcac agaacgat ca 5280

cagcat acag accgaggcac agagcct ggc cccat t cggc cat cccat cg acct ggt ccc 5340
ccagt ccagt t t ggcact ag t gacaaggac t cagact t ac gcct agt ggt aggagacagc 5400
t t gaaagcag agaaggagct aacagcat ca gt cact gagg ccat t cct gt at cacgagac 5460
t gggagct gc t t cccagt gc t gct gcct ct gct gagccac aat ccaagaa cct ggat t ct 5520
gggcact gt g t cccggagcc cagct cct ca ggccagcgcc t gt at cct ga ggt t t t ct at 5580
ggcagt gct g ggcct t ccag t t ct cagat c t ct gggggag ccat ggact c t caat t acat 5640
ccaaacagt g gaggct t ccg ccct gggaca ccct cact gc accct t acag at cacagccc 5700
ct at acct ac cccccggccc agcccct ccc t cagcact gc t ct ct ggggt agct ct caag 5760
ggccagt t t c t ggat t t ct c cacaat gcaa gct acagagc t ggggaagt t gccggct gga 5820
ggagt t ct ct accct ccacc t t cct t cct c t act ct ccgg ct t t ct gccc cagt cct t t g 5880
cct gacacat cgt t gct t ca ggt acgccag gat ct gccat cccct t cgga t t t t t at t ct 5940
act cct ct gc agcct ggt gg ccaaagt ggc t t t ct ccct t caggggct cc t gcccagcag 6000
at gct t ct ac ccat ggt aga ct cacagct g cct gt ggt ga act t t ggct c cct gccgcca 6060
gcaccacct c ct gccccacc t cccct t t ct ct gt t acct g t gggccct gc t ct gcagccc 6120
cccagcct gg ct gt gcggcc cccacct gct cct gct act c gggt gct gcc t t cacct gcc 6180
aggcccct t cc ccgct agct t ggggcgagca gagct gcat c cagt ggaact aaagccgt t c 6240
caggat t at c aaaaact gag cagcaacct t gggggacct g gat cat cacg gact ccccca 6300
act ggaaggt cct t ct ct gg cct caat t cc cgt ct caagg ccacgcct t c cacct acagt 6360
ggagt ct t cc gcacccagcg cgt cgacct t t accagcagg cct ccccacc agat gccct g 6420
cgct ggat ac ct aagcct t g ggagcggaca gggct gccac ct cgagaagg gccct cccga 6480
cgggcagagg agcct gggt c ccgaggggac aaggagcct g ggt t gccccc accccgct ga 6540
gggagt t cct ct t gccccct acccccgggg ct t gt at at a gat t at aaat at at aagggg 6600
gaaaggggt g ggcggggagg ggt t gt gggg ct ggggcct c act t cccct c ct cccccct t c 6660
ccct ggt ccc ct gt ccct gg ggct gt t t gt t aaaaaagag t aat aaaagg at t t 6714

<210> 10
<211> 2197
<212> DNA
<213> Homo sapiens

<400> 10

gccgcgaggg cggggcccgc agt t cggt t g cgct gcggag cgcagct gt g agggagt cgc 60
t gt gat ccgg ggccccggaa cccgagct gg agct gaagcg caggct gcgg ggcgcggagt 120
cgggaggcct gagt gt t cct t ccagcat gt cggaggggga gt cccagaca gt act t agca 180
gt ggct caga cccaaaggt a gaat cct cat ct t cagct cc t ggcct gaca t cagt gt cac 240
ct cct gt gac ct ccacaacc t cagct gct t ccccagagga agaagaagaa agt gaagat g 300

```
agtctgagat tttggaagag tcgccctgtg ggcgctggca gaagaggcga gaagaggtga 360
atcaacggaa tgtaccaggt attgacagtg catacctggc catggataca gaggaaggtg 420
tagaggttgt gtggaatgag gtacagttct ctgaacgcaa gaactacaag ctgcaggagg 480
aaaaggttcg tgctgtgttt gataatctga ttcaattgga gcatcttaac attgttaagt 540
ttcacaaata ttgggctgac attaaagaga acaaggccag ggtcattttt atcacagaat 600
acatgtcatc tgggagtctg aagcaatttc tgaagaagac caaaaagaac cacaagacga 660
tgaatgaaaa ggcatggaag cgttggtgca cacaaatcct ctctgcccta agctacctgc 720
actcctgtga cccccccatc atccatggga acctgacctg tgacaccatc ttcatccagc 780
acaacggact catcaagatt ggctctgtgg ctcctgacac tatcaacaat catgtgaaga 840
cttgtcgaga agagcagaag aatctacact tctttgcacc agagtatgga gaagtcacta 900
atgtgacaac agcagtggac atctactcct ttggcatgtg tgcactggag atggcagtgc 960
tggagattca gggcaatgga gagtcctcat atgtgccaca ggaagccatc agcagtgcca 1020
tccagcttct agaagaccca ttacagaggg agttcattca aaagtgcctg cagtctgagc 1080
ctgctcgcag accaacagcc agagaacttc tgttccaccc agcattgttt gaagtgccct 1140
cgctcaaact ccttgcggcc cactgcattg tgggacacca acacatgatc ccagagaacg 1200
ctctagagga gatcaccaaa aacatggata ctagtgccgt actggctgaa atccctgcag 1260
gaccaggaag agaaccagtt cagactttgt actctcagtc accagctctg gaattagata 1320
aattccttga agatgtcagg aatgggatct atcctctgac agcctttggg ctgcctcggc 1380
cccagcagcc acagcaggag gaggtgacat cacctgtcgt gcccccctct gtcaagactc 1440
cgacacctga accagctgag gtggagactc gcaaggtggt gctgatgcag tgcaacattg 1500
agtcggtgga ggagggagtc aaacaccacc tgacacttct gctgaagttg gaggacaaac 1560
tgaaccggca cctgagctgt gacctgatgc caaatgagaa tatccccgag ttggcggctg 1620
agctggtgca gctgggcttc attagtgagg ctgaccagag ccggttgact tctctgctag 1680
aagagacctt gaacaagttc aattttgcca ggaacagtac cctcaactca gccgctgtca 1740
ccgtctcctc ttagagctca ctcgggccag gccctgatct gcgctgtggc tgtccctgga 1800
cgtgctgcag ccctcctgtc ccttcccccc agtcagtatt accctgtgaa gccccttccc 1860
tcctttatta ttcaggaggg ctgggggggc tccctggttc tgagcatcat cctttcccct 1920
cccctctctt cctcccctct gcactttgtt tacttgtttt gcacagacgt gggcctgggc 1980
cttctcagca gccgccttct agttgggggc tagtcgctga tctgccggct cccgcccagc 2040
ctgtgtggaa aggaggccca cgggcactag gggagccgaa ttctacaatc ccgctggggc 2100
ggccggggcg ggagagaaag gtggtgctgc agtggtggcc ctgggggccc attcgattcg 2160
cctcagttgc tgctgtaata aaagtctact ttttgct 2197
```

<210> 11
<211> 6113
<212> DNA

<213> Homo sapiens

<400> 11

```
taatacttaa ttaccttcta ataattggag cagaagatga acccaactaa tcctttcagt     60
gggcagcagc ctagtgcttt ttcggcgtct tctagtaatg taggaacact tccatctaag    120
ccgccatttc gatttggtca accttctctt tttggacaaa acagtacctt atctgggaag    180
agctcgggat tttcacaggt atccagcttt ccagcgtctt ctggagtaag tcattcctct    240
tcagtgcaaa cattagggtt cacccaaacc tcaagtgttg gacccttttc tggacttgag    300
cacacttcca cctttgtggc tacctctggg ccttcaagtt catctgtgct gggaaacaca    360
ggatttagtt ttaaatcacc caccagtgtt ggggctttcc caagcacttc tgcttttgga    420
caagaagctg gagaaatagt gaactctggt tttgggaaaa cagaattcag ctttaaacct    480
ctggaaaatg cagtgttcaa accaatactg gggctgaat ctgagccaga gaaaacccag    540
agccaaattg cttctgggtt ttttacattt tcccacccaa ttagtagtgc acctggaggc    600
ctggcccctt tctcttttcc tcaagtaaca agtagttcag ctaccacttc aaattttacc    660
ttttcaaaac ctgttagtag taataattca ttatctgcct ttacccctgc tttgtcaaac    720
caaaatgtag aggaagagaa gagaggacct aagtcaatat ttggaagttc taataatagc    780
ttcagtagct tccctgtatc atctgcggtt ttgggcgaac ctttccaggc tagcaaagca    840
ggtgtcaggc aggggtgtga agaagctgtt tcccaggtgg aaccacttcc cagcctaatg    900
aaaggactga aaggaaggga ggaccaggat cgctccccaa ggagacatgg ccacgagcca    960
gcagaagatt cggatcctct gtcccggggc gatcatcctc cagacaaacg acctgtccgc   1020
ctgaatcgac cccggggagg tactttattt ggtcggacga tacaggatgt tttcaaaagc   1080
aataaggaag taggtcgtct gggcaacaag gaggccaaaa aggaaactgg ctttgttgag   1140
tctgcagaaa gtgaccacat ggctatccca ggagggaatc agtctgtcct ggcaccttcc   1200
cggattccag gtgtgaataa agaggaagaa actgaaagta gagagaagaa agaagattct   1260
ctaagaggaa ctccggcgcg tcagagtaac agaagcgaga gcacagacag tcttgggggc   1320
ttgtctccct ctgaagtcac agccatccag tgcaagaaca tccctgacta cctcaacgac   1380
aggaccattc tggagaacca ttttggcaaa attgctaaag tgcagcgcat ctttaccagg   1440
cgcagcaaaa agcttgcagt ggtacatttc tttgatcatg catctgcagc cctggctaga   1500
aagaagggga aaagtttgca taaagacatg gctatctttt ggcacaggaa gaaaataagc   1560
cccaataaga aacccttttc cctgaaggag aagaaaccag gtgacggtga agtcagcccg   1620
agcacagagg atgcaccctt tcagcactct cctcttggca aggccgcagg gaggactggt   1680
gctagcagcc tcctgaataa aagctctcca gtgaagaagc caagtcttct aaaggcccac   1740
caattcgagg gagactcttt tgactcagcc tccgagggct ccgagggcct cgggccatgt   1800
gtgctctccc tcagtaccct gataggcact gtggctgaga catccaagga gaagtaccgc   1860
```

ctgcttgacc agagagacag gatcatgcgg caagctcggg tgaagagaac cgatctggac 1920
aaagcgagga cttttgttgg cacctgcctg gatatgtgtc ctgagaagga gaggtacatg 1980
cgggagaccc gtagccagct gagcgtgttc gaagtggtcc cagggactga ccaggtggac 2040
cacgcagcag ctgtgaaaga gtacagtcgg tcctcggcgg atcaggagga gcccctgccc 2100
cacgagctgc ggcccttgcc agtgctcagc aggaccatgg actacctggt gacccagatc 2160
atggaccaga aggagggcag cctgcgggat tggtatgact tcgtgtggaa ccgcacgcgt 2220
ggcatacgga aggatatcac gcagcagcac ctctgtgacc ccctgacggt gtccctgatt 2280
gagaagtgca cccggtttca catccactgt gcccacttca tgtgtgagga gcccatgtcc 2340
tcctttgatg ccaagatcaa taatgagaac atgaccaagt gcctgcagag cctgaaggag 2400
atgtaccagg acctgagaaa caagggtgtc ttctgtgcca gcgaagcgga gttccagggc 2460
tacaatgttc tgctcagtct caacaaggga gacatcctaa gagaagtaca acagttccat 2520
cctgctgtta gaaactcatc tgaggtgaaa tttgctgttc aggcttttgc tgcattgaac 2580
agtaataatt ttgtgagatt tttcaaactg gtccagtcag cttcttacct gaacgcttgt 2640
cttttacact gttacttcag tcagatccgc aaggatgctc tccgggcgct caactttgcg 2700
tacacggtga gcacacagcg atctaccatc tttcccctgg atggtgtggt gcgcatgctg 2760
ctgttcagag actgtgaaga ggccaccgac ttcctcacct gccacggcct caccgtttcc 2820
gacggctgtg tggagctgaa ccggtctgca ttcctggaac cagagggatt atccaagacc 2880
aggaagtcgg tgtttattac taggaagctg acggtgtcag tcggggaaat tgtgaacgga 2940
gggccattgc cccccgtccc tcgtcatacc cctgtgtgca gcttcaactc ccagaacaag 3000
tacatcgggg agagcctggc cgcggagctg cccgtcagca cccagagacc cggctccgac 3060
acagtgggcg gagggagagg agaggagtgt ggtgtagagc cggatgcacc cctgtccagt 3120
ctcccacagt ctctaccagc ccctgcgccc tcaccagtgc ctctgcctcc tgtcctggca 3180
ctgaccccgt ctgtggcgcc cagcctcttc cagctgtctg tgcagcctga accaccgcct 3240
ccagagcccg tgcccatgta ctctgacgag gacctggcgc aggtggtgga cgagctcatc 3300
caggaggccc tgcagaggga ctgtgaggaa gttggctctg cgggtgctgc ctacgcagct 3360
gccgccctgg gtgtttctaa tgctgctatg gaggatttgt taacagctgc aaccacgggc 3420
attttgaggc acattgcagc tgaagaagtg tctaaggaaa gagagcgaag ggagcaggag 3480
aggcagcggg ctgaagagga aaggttgaaa caagagagag agctggtgtt aagtgagctg 3540
agccagggcc tggccgtgga gctgatggaa cgcgtgatga tggagtttgt gagggaaacc 3600
tgctcccagg agttgaagaa tgcagtagag acagaccaga gggtccgtgt ggcccgttgc 3660
tgtgaggatg tctgtgccca cttagtggac ttgtttctcg tggaggaaat cttccagact 3720
gcaaaggaga ccctccagga gcttcagtgc ttctgcaagt atctacagcg gtggagggaa 3780
gctgtcacag cccgcaagaa actgaggcgc caaatgcggg ctttccctgc tgcgccctgc 3840
tgcgtggacg tgagcgaccg gctgagggcg ctggcgccca gcgcagagtg ccccattgct 3900

gaagagaacc tggccagggg cctcctggac ctgggccatg cagggagatt gggcatctcc 3960
tgcaccaggt taaggcggct cagaaacaag acagctcacc agatgaaggt tcagcacttc 4020
taccagcagc tgctgagtga tgtggcatgg gcgtctctgg acctgccatc cctcgtggct 4080
gagcacctcc ctgggaggca ggagcatgtg ttttggaagc tggtgctggt gttgccggat 4140
gtagaggagc agtccccaga gagttgtggc agaattctag caaattggtt aaaagtcaag 4200
ttcatgggag atgaaggctc agtggatgac acatccagcg atgctggtgg gattcagacg 4260
ctttcgcttt tcaactcact tagcagcaaa ggggatcaga tgatttctgt taacgtgtgt 4320
ataaaggtgg cccatggcgc cctcagtgat ggtgccattg atgctgtgga gacacagaag 4380
gacctcctgg gagccagtgg gctcatgctg ctgcttcccc ccaaaatgaa gagtgaggac 4440
atggcagagg aggacgtgta ctggctgtcg gccttgctgc agctcaagca gctcctgcag 4500
gctaagccct tccagcctgc gcttcctctg gtggttcttg tgcctagccc aggaggggac 4560
gccgttgaga aggaagtaga agatggtctg atgctacagg acttggtttc agctaagctg 4620
atttcagatt acactgttac cgagatccct gataccatta atgatctaca aggttcaact 4680
aaggttttgc aagcagtgca gtggctggtt tcccactgcc cccattccct tgacctctgc 4740
tgccagactc tcattcagta cgtcgaagac gggattggcc atgagtttag tggccgcttt 4800
ttccatgaca gaagagagag gcgtctgggc ggtcttgctt ctcaggagcc tggcgccatc 4860
attgagctgt ttaacagtgt gctgcagttc ctggcttctg tggtgtcctc tgaacagctg 4920
tgtgacctgt cctggcctgt cactgagttt gctgaggcag gggcagccgg gctgcttcct 4980
cacctgcact ggaatgcccc agagcacctg gcctggctga agcaggctgt gctcgggttc 5040
cagcttccgc agatggacct tccacccctg ggggccccct ggctccccgt gtgctccatg 5100
gttgtccagt acgcctccca gatccccagc tcacgccaga cacagcctgt cctccagtcc 5160
caggtggaga acctgctcca cagaacctac tgtaggtgga agagcaagag tccctcccca 5220
gtccatgggg caggcccctc ggtcatggag atcccatggg atgatcttat cgccttgtgt 5280
atcaaccaca agctgagaga ctggacgccc ccccggcttc ctgttacatc agaggcgctg 5340
agtgaagatg gtcagatatg tgtgtatttt tttaaaaacg atttgaaaaa atatgatgtt 5400
cctttgtcgt gggaacaagc caggttgcag acgcagaagg agctacagct gagagaggga 5460
cgtttggcaa taaagccttt tcatccttct gcaaacaatt ttcccatacc attgcttcac 5520
atgcaccgta actggaagag gagcacagag tgtgctcaag aggggaggat tcccagcaca 5580
gaggatctga tgcgaggagc ttctgctgag gagctcttgg cgcagtgttt gtcgagcagt 5640
ctgctgctgg agaaagaaga gaacaagagg tttgaagatc agcttcagca atggttgtct 5700
gaagactcag gagcatttac ggatttaact tcccttcccc tctatcttcc tcagactcta 5760
gtgtctcttt ctcacactat tgaacctgtg atgaaaacat ctgtaactac tagcccacag 5820
agtgacatga tgagggagca actgcagctg tcagaggcga caggaacgtg tctaggcgaa 5880
cgactaaagc acctggaaag gctgatccgg agttcaaggg aagaggaagt tgcctctgag 5940

ct ccat ct ct ct gcgct gct agacat ggt g gacat t t gag cagcct gacc t gt ggggagg 6000
gggt ct ct cc cgaagagt t t ct gt t t t t ac t caaaat aat gt t at t ct ca gat gct t gat 6060
gcact gt t gg aaat gt gat t aat t t aat ca t gcagat aaa ccat t t aaat gt c 6113

<210> 12
<211> 6493
<212> DNA
<213> Homo sapiens

<400> 12

gaagt ccggc ct t ccgagag ct agct gt cc gccgcggccc ccgcacgccg ggcagccgt c 60
cct cgccgcc t cgggcgcgc caccat gggg ccccggct ca gcgt ct ggct gct gct gct g 120
cccgccgccc t t ct gct cca cgaggagcac agccgggccg ct gcgaaggg t ggct gt gct 180
ggct ct ggct gt ggcaaat g t gact gccat ggagt gaagg gacaaaaggg t gaaagaggc 240
ct cccggggt t acaaggt gt cat t gggt t t cct ggaat gc aaggacct ga ggggccacag 300
ggaccaccag gacaaaaggg t gat act gga gaaccaggac t acct ggaac aaaagggaca 360
agaggacct c cgggagcat c t ggct accct ggaaacccag gact t cccgg aat t cct ggc 420
caagacggcc cgccaggccc cccaggt at t ccaggat gca at ggcacaaa gggggagaga 480
gggccgct cg ggcct cct gg ct t gcct ggt t t cgct ggaa at cccggacc accaggct t a 540
ccagggat ga agggt gat cc aggt gagat a ct t ggccat g t gcccgggat gct gt t gaaa 600
ggt gaaagag gat t t cccgg aat cccaggg act ccaggcc caccaggact gccagggct t 660
caaggt cct g t t gggcct cc aggat t t acc ggaccaccag gt cccccagg ccct cccggc 720
cct ccaggt g aaaagggaca aat gggct t a agt t t t caag gaccaaaagg t gacaagggt 780
gaccaagggg t cagt gggcc t ccaggagt a ccaggacaag ct caagt t ca agaaaaagga 840
gact t cgcca ccaagggaga aaagggccaa aaaggt gaac ct ggat t t ca ggggat gcca 900
ggggt cggag agaaaggt ga acccggaaaa ccaggaccca gaggcaaacc cggaaaagat 960
ggt gacaaag gggaaaaagg gagt cccggt t t t cct ggt g aacccgggt a cccaggact c 1020
at aggccgcc agggcccgca gggagaaaag ggt gaagcag gt cct cct gg cccacct gga 1080
at t gt t at ag gcacaggacc t t t gggagaa aaaggagaga ggggct accc t ggaact ccg 1140
gggccaagag gagagccagg cccaaaaggt t t cccaggac t accaggcca acccggacct 1200
ccaggcct cc ct gt acct gg gcaggct ggt gcccct ggct t ccct ggt ga aagaggagaa 1260
aaaggt gacc gaggat t t cc t ggt acat ct ct gccaggac caagt ggaag agat gggct c 1320
ccgggt cct c ct ggt t cccc t gggccccct gggcagcct g gct acacaaa t ggaat t gt g 1380
gaat gt cagc ccggacct cc aggt gaccag ggt cct cct g gaat t ccagg gcagccagga 1440
t t t at aggcg aaat t ggaga gaaaggt caa aaaggagaga gt t gcct cat ct gt gat at a 1500
gacggat at c gggggcct cc cgggccacag ggaccccccgg gagaaat agg t t t cccaggg 1560

cagccagggg ccaagggcga cagaggt t t g cct ggcagag at ggt gt t gc aggagt gcca 1620
ggccct caag gt acaccagg gct gat aggc cagccaggag ccaaggggga gcct ggt gag 1680
t t t t at t t cg act t gcggct caaaggt gac aaaggagacc caggct t t cc aggacagccc 1740
ggcat gccag ggagagcggg t t ct cct gga agagat ggcc at ccgggt ct t cct ggcccc 1800
aagggct cgc cgggt t ct gt aggat t gaaa ggagagcgt g gcccccct gg aggagt t gga 1860
t t cccaggca gt cgt ggt ga caccggcccc cct gggcct c caggat at gg t cct gct ggt 1920
cccat t ggt g acaaaggaca agcaggct t t cct ggaggcc ct ggat cccc aggcct gcca 1980
ggt ccaaagg gt gaaccagg aaaaat t gt t cct t t accag gcccccct gg agcagaagga 2040
ct gccggggt ccccaggct t cccaggt ccc caaggagacc gaggct t t cc cggaacccca 2100
ggaaggccag gcct gccagg agagaagggc gct gt gggcc agccaggcat t ggat t t cca 2160
gggccccccg gccccaaagg t gt t gacggc t t acct ggag acat ggggcc accggggact 2220
ccaggt cgcc cgggat t t aa t ggct t acct gggaacccag gt gt gcaggg ccagaaggga 2280
gagcct ggag t t ggt ct acc gggact caaa ggt t t gccag gt ct t cccgg cat t cct ggc 2340
acacccgggg agaaggggag cat t ggggt a ccaggcgt t c ct ggagaaca t ggagcgat c 2400
ggacccccct g ggct t caggg gat cagaggt gaaccgggac ct cct ggat t gccaggct cc 2460
gt ggggt ct c caggagt t cc aggaat aggc cccccct ggag ct aggggt cc ccct ggagga 2520
cagggaccac cggggt t gt c aggccct cct ggaat aaaag gagagaaggg t t t ccccgga 2580
t t ccct ggac t ggacat gcc gggccct aaa ggagat aaag gggct caagg act ccct ggc 2640
at aacgggac agt cggggct ccct ggcct t cct ggacagc agggggct cc t gggat t cct 2700
gggt t t ccag gt t ccaaggg agaaat gggc gt cat gggga cccccgggca gccgggct ca 2760
ccaggaccag t gggt gct cc t ggat t accg ggt gaaaaag gggaccat gg ct t t ccgggc 2820
t cct caggac ccaggggaga ccct ggct t g aaaggt gat a agggggat gt cggt ct ccct 2880
ggcaagcct g gct ccat gga t aaggt ggac at gggcagca t gaagggcca gaaaggagac 2940
caaggagaga aaggacaaat t ggaccaat t ggt gagaagg gat cccgagg agaccct ggg 3000
accccaggag t gcct ggaaa ggacgggcag gcaggacagc ct gggcagcc aggacct aaa 3060
ggt gat ccag gt at aagt gg aaccccaggt gct ccaggac t t ccgggacc aaaaggat ct 3120
gt t ggt ggaa t gggct t gcc aggaacacct ggagagaaag gt gt gcct gg cat ccct ggc 3180
ccacaaggt t cacct ggct t acct ggagac aaaggt gcaa aaggagagaa agggcaggca 3240
ggcccacct g gcat aggcat cccagggct g cgaggt gaaa agggagat ca agggat agcg 3300
ggt t t cccag gaagccct gg agagaaggga gaaaaaggaa gcat t gggat cccaggaat g 3360
ccagggt ccc caggcct t aa agggt ct ccc gggagt gt t g gct at ccagg aagt cct ggg 3420
ct acct ggag aaaaaggt ga caaaggcct c ccaggat t gg at ggcat ccc t ggt gt caaa 3480
ggagaagcag gt ct t cct gg gact cct ggc cccacaggcc cagct ggcca gaaaggggag 3540
ccaggcagt g at ggaat ccc ggggt cagca ggagagaagg gt gaaccagg t ct accagga 3600

agaggattcc cagggtttcc aggggccaaa ggagacaaag gttcaaaggg tgaggtgggt 3660
ttcccaggat tagccgggag cccaggaatt cctggatcca aggagagca aggattcatg 3720
ggtcctccgg ggccccaggg acagccgggg ttaccgggat ccccaggcca tgccacggag 3780
gggcccaaag gagaccgcgg acctcagggc cagcctggcc tgccaggact tccgggaccc 3840
atggggcctc cagggcttcc tgggattgat ggagttaaag gtgacaaagg aaatccaggc 3900
tggccaggag cacccggtgt cccagggccc aagggagacc ctggattcca gggcatgcct 3960
ggtattggtg gctctccagg aatcacaggc tctaagggtg atatggggcc tccaggagtt 4020
ccaggatttc aaggtccaaa aggtcttcct ggcctccagg gaattaaagg tgatcaaggc 4080
gatcaaggcg tcccgggagc taaaggtctc ccgggtcctc ctggcccccc aggtccttac 4140
gacatcatca aggggagccc gggctccct ggtcctgagg gcccccagg gctgaaaggg 4200
cttcagggac tgccaggccc gaaaggccag caaggtgtta caggattggt gggtatacct 4260
ggacctccag gtattcctgg gtttgacggt gcccctggcc agaaaggaga gatgggacct 4320
gccgggccta ctggtccaag aggatttcca ggtccaccag gccccgatgg gttgccagga 4380
tccatggggc cccaggcac cccatctgtt gatcacggct tccttgtgac caggcatagt 4440
caaacaatag atgacccaca gtgtccttct gggaccaaaa ttctttacca cgggtactct 4500
ttgctctacg tgcaaggcaa tgaacgggcc catggccagg acttgggcac ggccggcagc 4560
tgcctgcgca agttcagcac aatgcccttc ctgttctgca atattaacaa cgtgtgcaac 4620
tttgcatcac gaaatgacta ctcgtactgg ctgtccaccc ctgagcccat gcccatgtca 4680
atggcaccca tcacgggga aaacataaga ccatttatta gtaggtgtgc tgtgtgtgag 4740
gcgcctgcca tggtgatggc cgtgcacagc cagaccattc agatcccacc gtgccccagc 4800
gggtggtcct cgctgtggat cggctactct tttgtgatgc acaccagcgc tggtgcagaa 4860
ggctctggcc aagccctggc gtcccccggc tcctgcctgg aggagtttag aagtgcgcca 4920
ttcatcgagt gtcacggccg tgggacctgc aattactacg caaacgctta cagcttttgg 4980
ctcgccacca tagagaggag cgagatgttc aagaagccta cgccgtccac cttgaaggca 5040
ggggagctgc gcacgcacgt cagccgctgc caagtctgta tgagaagaac ataatgaagc 5100
ctgactcagc taatgtcaca acatggtgct acttcttctt cttttgtta acagcaacga 5160
accctagaaa tatatcctgt gtacctcact gtccaatatg aaaaccgtaa agtgccttat 5220
aggaatttgc gtaactaaca caccctgctt cattgacctc tacttgctga aggagaaaaa 5280
gacagcgata agctttcaat agtggcatac caaatggcac ttttgatgaa ataaaatatc 5340
aatattttct gcaatccaat gcactgatgt gtgaagtgag aactccatca gaaaaccaaa 5400
gggtgctagg aggtgtgggt gccttccata ctgtttgccc attttcattc ttgtattata 5460
attaattttc tacccccaga gataaatgtt tgtttatatc actgtctagc tgtttcaaaa 5520
tttaggtccc ttggtctgta caaataatag caatgtaaaa atggtttttt gaacctccaa 5580
atggaattac agactcagta gccatatctt ccaacccccc agtataaatt tctgtctttc 5640

t gct at gt gt ggt act t t gc agct gct t t t gcagaaat ca caat t t t cct gt ggaat aaa 5700
gat ggt ccaa aaat agt caa aaat t aaat a t at at at at a t t agt aat t t at at agat gt 5760
cagcaat t ag gcagat caag gt t t agt t t a act t ccact g t t aaaat aaa gct t acat ag 5820
t t t t ct t cct t t gaaagact gt gct gt cct t t aacat agg t t t t t aaaga ct aggat at t 5880
gaat gt gaaa cat ccgt t t t cat t gt t cac t t ct aaacca aaaat t at gt gt t gccaaaa 5940
ccaaacccag gt t cat gaat at ggt gt ct a t t at agt gaa acat gt act t t gagct t at t 6000
gt t t t t at t c t gt at t aaat at t t t caggg t t t t aaacac t aat cacaaa ct gaat gact 6060
t gact t caaa agcaacaacc t t aaaggccg t cat t t cat t agt at t cct c at t ct gcat c 6120
ct ggct t gaa aaacagct ct gt t gaat cac agt at cagt a t t t t cacacg t aagcacat t 6180
cgggccat t t ccgt ggt t t c t cat gagct g t gt t cacaga cct cagcagg gcat cgcat g 6240
gaccgcagga gggcagat t c ggaccact ag gcct gaaat g acat t t cact aaaagt ct cc 6300
aaaacat t t c t aagact act aaggcct t t t at gt aat t t c t t t aaat gt g t at t t ct t aa 6360
gaat t caaat t t gt aat aaa act at t t gt a t aaaaat t aa gct t t t at t a at t t gt t gct 6420
agt at t gcca cagacgcat t aaaagaaact t act gcacaa gct gct aat a aat t t gt aag 6480
ct t t gcat ac ct t 6493

<210> 13
<211> 2718
<212> DNA
<213> Homo sapiens

<400> 13

gcgaccgct c cccggcggga gccagcgaag gt t t ccat gt cagaggccga t ggagaact g 60
aagat t gcca cct acgcaca aaggccat t g agacact t cg t gt agct gga agacaccaac 120
t t cct gacag gagct t t at t t cat t t ggga t t t caagt t t acagat ggt a t ct t ct caaa 180
agt t ggaaaa acct at agag at gggcagt a gcgaacccct t cccat cgca gat ggt gaca 240
ggaggaggaa gaagaagcgg aggggccggg ccact gact c ct t gccagga aagt t t gaag 300
at at gt acaa gct gacct ct gaat t gct t g gagagggagc ct at gccaaa gt t caaggt g 360
ccgt gagcct acagaat ggc aaagagt at g ccgt caaaat cat cgagaaa caagcagggc 420
acagt cggag t agggt gt t t cgagaggt gg agacgct gt a t cagt gt cag ggaaacaaga 480
acat t t t gga gct gat t gag t t ct t t gaag at gacacaag gt t t t act t g gt ct t t gaga 540
aat t gcaagg aggt t ccat c t t agcccaca t ccagaagca aaagcact t c aat gagcgag 600
aagccagccg agt ggt gcgg gacgt t gct g ct gccct t ga ct t cct gcat accaaagaca 660
aagt ct ct ct ct gt cacct a ggct ggagt g ct at ggcgcc at cagggct c act gcagccc 720
caacct ccct gggct ccagt gat cct ccca cct cagcct c ccaagt agct gggact acag 780

gcat t gct ca t cgt gat ct g aaaccagaaa at at at t gt g t gaat ct cca gaaaaggt gt 840
ct ccagt gaa aat ct gt gac t t t gact t gg gcagt gggat gaaact gaac aact cct gt a 900
cccccat aac cacaccagag ct gaccaccc cat gt ggct c t gcagaat ac at ggcccct g 960
aggt agt gga ggt ct t cacg gaccaggcca cat t ct acga caagcgct gt gacct gt gga 1020
gcct gggcgt ggt cct ct ac at cat gct ga gt ggct accc accct t cgt g ggt cact gcg 1080
gggccgact g t ggct gggac cggggcgagg t ct gcagggt gt gccagaac aagct gt t t g 1140
aaagcat cca ggaaggcaag t at gagt t t c ct gacaagga ct gggcacac at ct ccagt g 1200
aagccaaaga cct cat ct cc aagct cct gg t gcgagat gc aaagcagaga ct t agcgccg 1260
cccaagt t ct gcagcaccca t gggt gcagg ggcaagct cc agaaaaggga ct ccccacgc 1320
cgcaagt cct ccagaggaac agcagcacaa t ggacct gac gct ct t cgca gct gaggcca 1380
t cgccct t aa ccgccagct a t ct cagcacg aagagaacga act agcagag gagccagagg 1440
cact agct ga t ggcct ct gc t ccat gaagc t t t cccct cc ct gcaagt ca cgcct ggccc 1500
ggagacgggc cct ggcccag gcaggccgt g gt gaagacag gagcccgccc acagcact ct 1560
gaaat gct cc agt cacacct t at aggccct aggcct ggcc aggcat t gt c ccct ggaaac 1620
ct gt gt ggct aaagt ct gct gagcaggcag cagcct ct gc t ct gt ggct c cat t caggct 1680
t t t t cat ct a cgaaggccct gaggt t ccca t caaccccca t t t ccct agg gt cct ggagg 1740
aaaaagct t t t t ccaaaggg gt t gt ct t t g aaaaggaaag caat cact t c t cact t t gca 1800
t aat t gcct g cagcaggaac at ct ct t cac t gggct ccac ct gct caccc gcct gcagat 1860
ct gggat cca gcct gct ct c accgct gt ag ct gt ggcggc t ggggct gca gcct gcaggg 1920
agaagcaaga agcat cagt t gacagaggct gccgacacgt gcct ct t ccc t ct ct t ct ct 1980
gt caccct cc t ct ggcggt c ct t ccacct t cct ct gt cct ccggat gt cc t ct t t gcccg 2040
t ct t ct ccct t ggct gagca aagccat ccc ct caat t cag ggaagggcaa ggagcct t cc 2100
t cat t cagga aat caaat ca gt ct t ccggt ct gcagcacg gaaaagcaca t aat ct t t ct 2160
t t gct gt gac t gaaat gt at ccct cgt t t a t cat cccct t t gt t t gt gat t gct gct aaa 2220
gt cagt agt a t cgt t t t t t t aaaaaaaaag t t t ggt gt t t t t aaccat gc t gt t ccagca 2280
aagat gat ac ct t aaact cc cact gcaagc ccat gaact t cccagagagt ggaacggct t 2340
gct ct t ct t t ct agaat gt c cat gcact t g ggt t t t aat c agcagt t ccc t at t at t ct g 2400
at t t t aagct gt t cct gt ga t gaact t aga gacagcat cg gt gt ct gct g ct gt gt cccc 2460
aggt ct t gt g t gggt ggcac agat ct gggc agt t agat ag t gct ct gt gc ct aaggt gaa 2520
gccacact ag ggt gaagcct cact t ccct g t t t gagcaat gcagt gcct g ct gcccgt gt 2580
gcat gaaggt acagccat t c agat aagt gg aact at t gag t t acat aaag aaaat agat t 2640
t gcat t t gt c aggcagacgt t t at acaaca ccacggt gct t t t at acat t gt gct t at t t 2700
t aat aaaact gaaat t ct 2718

<210> 14

<211> 1840
<212> DNA
<213> Homo sapiens

<400> 14

```
gcggcgcgag ccacatgcga gcgggcgcct ggcggcggcg gcggcggcac cagcgatccc     60
agcagcggcc acgacgcgga cgcgcctgcg gcccggggag cagcagcagc cacagccaca    120
gcagccgcca ctgcagttag agcggcagca gcagcgacag ccacagcagc agccgccgcg    180
gagagcggcg ctcggcgggc gcgccctcct gaaggaagcc gcccgccccc caccgccgcc    240
ccctccggcg tgttcatgcc cccggggccc cagggagcgc catggcccgc gcacgccagg    300
agggcagctc cccggagccc gtagagggcc tggcccgcga cggcccgcgc cccttcccgc    360
tcggccgcct ggtgccctcg gcagtgtcct gcggcctctg cgagcccggc ctggctgccg    420
cccccgccgc ccccaccctg ctgcccgctg cctacctctg cgcccccacc gccccacccg    480
ccgtcaccgc cgccctgggg ggttcccgct ggcctggggg tccccgcagc cggccccgag    540
gcccgcgccc ggacggtcct cagccctcgc tctcgctggc ggagcagcac ctggagtcgc    600
ccgtgcccag cgccccgggg gctctggcgg gcggtcccac ccaggcggcc ccgggagtcc    660
gcggggagga ggaacagtgg gcccgggaga tcggggccca gctgcggcgg atggcggacg    720
acctcaacgc acagtacgag cggcggagac aagaggagca gcagcggcac cgcccctcac    780
cctggagggt cctgtacaat ctcatcatgg gactcctgcc cttacccagg ggccacagag    840
cccccgagat ggagcccaat taggtgcctg cacccgcccg gtggacgtca gggactcggg    900
gggcaggccc ctcccacctc ctgacaccct ggccagcgcg gggacttttc tctgcaccat    960
gtagcatact ggactcccag ccctgcctgt cccgggggcg ggccggggca gccactccag   1020
ccccagccca gcctggggtg cactgacgga gatgcggact cctgggtccc tggccaagaa   1080
gccaggagag ggacggctga tggactcagc atcggaaggt ggcggtgacc gagggggtgg   1140
ggactgagcc gcccgcctct gccgcccacc accatctcag gaaaggctgt tgtgctggtg   1200
cccgttccag ctgcaggggt gacactgggg gggggggggc tctcctctcg gtgctccttc   1260
actctgggcc tggcctcagg cccctggtgc ttcccccccct cctcctggga gggggcccgt   1320
gaagagcaaa tgagccaaac gtgaccacta gcctcctgga gccagagagt ggggctcgtt   1380
tgccggttgc tccagcccgg cgcccagcca tcttccctga gccagccggc gggtggtggg   1440
catgcctgcc tcaccttcat caggggggtgg ccaggagggg cccagactgt gaatcctgtg   1500
ctctgcccgt gaccgccccc cgccccatca atcccattgc ataggtttag agagagcgac   1560
gtgtgaccac tggcattcat ttgggggtg ggagattttg gctgaagccg ccccagcctt   1620
agtccccagg gccaagcgct ggggggaaga cggggagtca gggaggggggg gaaatctcgg   1680
aagagggagg agtctgggag tggggaggga tggcccagcc tgtaagatac tgtatatgcg   1740
```

```
ctgctgtaga taccggaatg aatttttctgt acatgtttgg ttaatttttt ttgtacatga 1800
tttttgtatg tttcctttttc aataaaatca gattggaaca 1840
```

<210> 15
<211> 3093
<212> DNA
<213> Homo sapiens

<400> 15

```
tagaatctgt tcaggaagtt ggaggttctt actggcaaag agtaactctc atcctggaat 60
tactgcagca caaaaagaag ctcagaagtc ctcagatatt ggtgccaact cttttttaact 120
tgctatcaag atgtttagaa cccttgccac aagagcaggg aaatatggaa tacaccaaac 180
aattaattct tagttgtctg ctcaacatct gccaaaaact atctccagat ggtggcaaaa 240
tacccaaaga tattttagat gaggagaagt tcaacgtgga gttgatagtt cagtgcatcc 300
gccttttcgga gatgccgcag acccatcacc atgccctttt acttttgggc actgttgctg 360
gaatatttcc ggataaagtt ttacacaata tcatgtctat ttttacattt atgggagcca 420
atgtcatgcg cctagatgat acttacagtt ttcaagttat taacaagaca gtgaaaatgg 480
ttattcccgc acttattcag tctgatagtg gagattctat agaagtttca agaaacgttg 540
aagagattgt ggtaaaaatc attagtgtat ttgtggatgc gctgccacac gtcccggagc 600
acaggcgcct gcccatcctt gttcaacttg ttgatacact gggtgcagag aaattcctct 660
ggattctcct catcttgctt tttgaacagt atgtcacaaa aacagtgctg gcggctgcct 720
atggcgaaaa ggatgctatt ttagaagcag acactgaatt ttggttttca gtctgttgtg 780
agtttagtgt ccagcatcag atacaaagct tgatgaatat cctccagtac ttactaaagc 840
tgccagagga aaaagaagaa accattccca aagcagtgtc atttaataag agtgaatcac 900
aagaagaaat gctacaggtt tttaatgtag agactcacac tagcaagcaa ctgcggcatt 960
ttaaattttt gtcagtgtcc ttcatgtctc agctcctgtc ttccaatgat tttctgaaaa 1020
aggtagttga gagtggtggt cctgagattt taaaaggcct tgaagagagg ttgctggaga 1080
ccgttctcgg ctatatcagt gcagttgcac agtccatgga aaggaacgca gacaaactca 1140
ccgtgaagtt ctggcgcgcg ctccttagta aagcttacga cctgttagat aaggtcaatg 1200
ccttgctgcc cacagagaca ttcattcctg tgatcagagg gctggtgggc aatcccctgc 1260
catctgttcg ccgcaaagcg ctggaccttt tgaataacaa gctgcagcaa aatatatcct 1320
ggaagaagac aatagttacc cgtttcctaa aactggttcc agaccttttg gccattgtgc 1380
agcgtaagaa aaaggaaggg gaagaagaac aagcaatcaa cagacagaca gcgttgtata 1440
ccttaaagct tttatgcaag aattttggtg cagaaaatcc agatcctttt gtcccagtgc 1500
tgagcactgc tgtgaaactg attgctccag agagaaagga ggagaagaat gtcttgggaa 1560
```

gcgcgct gct gt gcat agca gaggt gacct ccaccct gga ggcgct ggcc at cccccagc 1620
t t cccagcct gat gccat cg t t gct gacaa caat gaagaa caccagcgag ct ggt ct cca 1680
gcgaggt ct a cct gct cagt gcct t ggct g ct ct gcagaa ggt t gt ggag act ct cccgc 1740
act t cat cag cccct at ct g gaaggcat t c t ct cccaggt gat t cat ct g gagaaaat ca 1800
ct agt gaaat gggt t ct gcg t cacgggct a at at ccgt ct cacat ct ct t aaaaagacac 1860
t ggct accac act t gcaccc cgagt cct gt t gcccgccat caaaaaaact t acaagcaga 1920
t t gagaagaa ct ggaagaat cacat gggt c cgt t t at gag cat ct t gcaa gagcat at t g 1980
gggcgat gaa gaaggaagag ct cacct ccc at cagt ct ca gct aaccgcc t t t t t cct gg 2040
aggccct gga ct t ccgagcc cagcact ct g agaacgat ct ggaggaagt t ggaaaaacgg 2100
aaaat t gt at cat t gact gt ct agt agcca t ggt t gt caa act t t ccgag gt cacat t ca 2160
ggcccct gt t ct t caagct g t t t gat t ggg ct aaaacaga agat gcccca aaggacaggt 2220
t gt t gacat t t t acaact t g gcagat t gca t t gct gaaaa gct gaaaggg ct t t t t act c 2280
t gt t t gccgg ccact t agt g aagcct t t t g ct gacacct t ggaccaggt g aacat ct cca 2340
aaacagat ga agcat t t t t t gact ct gaaa at gaccct ga aaagt gct gc t t gct gt t gc 2400
agt t t at t t t gaact gt t t a t acaaaat ct t cct t t t t ga t acccagcat t t t at aagt a 2460
aagagagagc aggagcct t g at gat gcct c t ggt ggat ca gct ggt aaac aggct t gggg 2520
gagaagagaa at t ccaggaa cgggt gacaa agcacct gat accat gcat c gcacagt t t t 2580
cagt ggccat ggcggat gac t ct ct t t gga aaccact gaa ct accagat t ct gct aaaga 2640
cgagagact c ct cgcct aag gt t cgat t t g ct gct t t gat t act gt gt t a gcact ggct g 2700
aaaaact aaa ggagaat t at at t gt ct t gc t accagaat c cat t cct t t c t t agcagagt 2760
t gat ggaaga t gaat gt gaa gaagt agaac at cagt gcca aaagact at t cagcaact gg 2820
aaact gt cct gggagagcca ct ccagagct at t t ct aaga ct t t ct gt gg t gt t t cat ac 2880
t ct act caga gt t cacact c at at t t cat a t t t t t at t t t t gggt gt t gg gt gccat gt t 2940
act t t t ggt g cct t aat aca cct act t gga t t act t acaa at gt t t t at c act t cgt t ac 3000
aaaat cccca cct ggct t gt gct gccacat aagcct ct cc t gcct at cgt at agagct gc 3060
agaaagagt a aat gat acac ggt at t t t t a t ac 3093

<210> 16
<211> 1702
<212> DNA
<213> Homo sapiens

<400> 16

caaaat ccca ggcagcat gg acct cagt ct t ct ct gggt a ct t ct gcccc t agt caccat 60
ggcct ggggc cagt at ggcg at t at ggat a cccat accag cagt at cat g act acagcga 120

t gat gggt gg gt gaat t t ga accggcaagg ct t cagct ac cagt gt cccc aggggcaggt 180
gat agt ggcc gt gaggagca t ct t cagcaa gaaggaaggt t ct gacagac aat ggaact a 240
cgcct gcat g cccacgccac agagcct cgg ggaacccacg gagt gct ggt gggaggagat 300
caacagggct ggcat ggaat ggt accagac gt gct ccaac aat gggct gg t ggcaggat t 360
ccagagccgc t act t cgagt cagt gct gga t cgggagt gg cagt t t t act gt t gt cgct a 420
cagcaagagg t gcccat at t cct gct ggct aacaacagaa t at ccaggt c act at ggt ga 480
ggaaat ggac at gat t t cct acaat t at ga t t act at at c cgaggagcaa caaccact t t 540
ct ct gcagt g gaaagggat c gccagt ggaa gt t cat aat g t gccggat ga ct gaat acga 600
ct gt gaat t t gcaaat gt t t agat t t gcca cat accaaat ct gggt gaaa ggaaaggggc 660
cggggacagg agggt gt cca cat at gt t aa cat cagt t gg at ct cct at a gaagt t t ct g 720
ct gct ct ct t t cct t ct ccc t gagct ggt a act gcaat gc caact t cct g ggcct t t ct g 780
act agt at ca cact t ct aat aaaat ccaca at t aaaccat gt t t ct cact t t t cacat gt 840
t t cat agcaa ct gct t t at a t gact gat ga t ggct t cct t gcacaccaca t at acagt gc 900
gcat gct t ac agccgggct t ct ggagcacc agct gcagcc t ggct act gc t t t t t act gc 960
agaat gaact gcaagt t cag cat agt ggag gggagaggca gaact ggagg agaggt gcag 1020
t gaaggt t ct ct acagct aa gcct gt t t ga at gat acgt a ggt t ccccac caaaagcagg 1080
ct t t ct gccc t gagggacat ct t cccact c ccct gct cca cat gagccat gcat gct t ag 1140
caat ccaagt gcagagct ct t t gct ccagg agt gaggaga ct gggaggt g aaat ggggaa 1200
at ggaagggt t t ggaggcag agct gaaaac agggt t ggaa ggat t t cct g aat t agaaga 1260
caaacgt t ag cat acccagt aaggaaaat g agt gcagggg ccaggggaac ccgt gaggat 1320
cact ct caaa t gagat t aaa aacaaggaag cagagaat gg t cagagaat g ggat t cagat 1380
t gggaact t g t ggggat gag agt gaccagg t t gaact ggg aagt ggaaaa aggagt t t ga 1440
gt cact ggca cct agaagcc t gcccacgat t cct aggaag gct ggcagac accct ggaac 1500
cct ggggagc t act ggcaaa ct ct cct gga t t gggcct ga t t t t t t t ggt gggaaaggct 1560
gccct gggga t caact t t cc t t ct gt gt gt ggct caggag t t ct t ct gca gagat ggcgc 1620
t at ct t t cct cct cct gt ga t gt cct gct c ccaaccat t t gt act ct t ca t t acaaaaga 1680
aat aaaaat a t t aacgt t ca ct 1702

<210> 17
<211> 2730
<212> DNA
<213> Homo sapiens

<400> 17

agaat gcct a t gagacacag gaagaaggca gcagacaaga at ct t ccct g ccgt cct t t a 60

gtatgtgcag tactggacct gatggtagag tttattgtaa cacacatgat gaaggagttt 120

cctatggatc tctatatacg ctgcatccag gtagtacaca aactgctctg ctaccagaag 180

aagtgtcggg tacgcctgca ttacacctgg cgggagctct ggtcagcctt gataaatttg 240

ctgaagttcc ttatgtcaaa tgagactgta cttttggcca aacacaacat ttttacatta 300

gcccttatga ttgtgaacct atttaatatg tttatcacat atggcgacac atttctgcca 360

acccccagca gctatgatga actttactat gagattatcc gcatgcacca gagctttgac 420

aacctctact ccatggtcct gaggctttct accaatgcag gccagtggaa ggaagcagct 480

agcaaggtga cccatgcatt ggttaatatc agagccatca tcaaccactt taaccccaaa 540

attgagtcct acgctgctgt gaatcacata tcccaactgt cagaggagca ggtgctggag 600

gtggtgagag ccaactatga cacgctcacg ctgaagctgc aggatggcct ggaccagtat 660

gagcgctact cagagcagca caaggaagct gccttcttca aagagctggt tcgatccatt 720

agcaccaacg tccggagaaa cctggtcttc cacacactca gccaagaagt cctgctcaag 780

gagttctcca ctatctcctg aggccacgcc tacctgagca gcctctgact gcccttaccc 840

atgaggatca tgggctggag gggagcgag gggagagggg gctgcccccg aggttggaga 900

gaaacacaga taccaagttc tcttggtgaa gaccgcactt caatggagct tgggcagcag 960

gggcaggagg gtcaagccag ggataatctt atttggggag gaatgggtgg gcacagtggg 1020

gagaggcctc caggaatgtg gggacttcca gagtgggctc cctaaacagc ccctcacatt 1080

tccaatcttg aggttacaag ctgtagctac tgactaattt tcgggagctg gtgaggcaag 1140

cctcagggta atgccagcac cctgaaagtc agtggctctg ctcggcttct gaaaacagat 1200

caactctcag atatagctgg agctacatcc tgcttccttt gggcctgggg ctgtgcttgg 1260

cttgaaatct gtgtcccctg cctgcactgg cactttgtcc agtcatcggg acctttggct 1320

cagatttagg gtaagatagg aatagggtac ctttttcctt ttttttctga gacggagtct 1380

cactctgtcg cccaggctgg aatgcagtgg cgtgatctcg gctttactgc agcctccgct 1440

tcctgggttc aagcgattct cctgcctcag cctcccgagt agctgggact acaggagcgt 1500

gtgccacgcc cggctaattt tttgtatttt tagtagagat ggagtttcac catgttaccc 1560

aggatggtct caatatcctg agttcatgat ccacccacct tggcctccca aagtgctggg 1620

attacaggcg tgagccacca cacccagcca gttttcctat tttctgaatt cagaattgac 1680

ttctctggga aaactggaga tgagaatctg cccagtgctc tgctgtccag tcaccgcctt 1740

ttgaatttta gttttggcac caggagtacc gttagctttc cccttcttct ggcccatttg 1800

actcatcagc acaatagggt caccacctta acttttaatt tctaatctgc tttactgtct 1860

ttagggtgtg tatgaaataa acattgacag gctttctgga gccctcaagg tgcctacttt 1920

gctggttccc tttccagcag ctcccccacc tcccttagcc ccccctcctc tggcagcctc 1980

tcctgcctct gctgagctcc cctccacgtg ttccaccccc ttaccctgct gttgtttaca 2040

tccaacctgc ctgagaattt cctctgggga ggaatctatt cctgtcatgg tctagtgcct 2100

gggagggaga gaacttcctg ggggtagggt gccctccatc tgaaacaggc caggtgagca 2160
tcatgcataa ggcctccatt ctgtccgctc agattctggg tggggccaca ggcaaatctc 2220
ctgacttatg gggagttggc ttgtggttcc tcccttggat agcctccatg gaaccactat 2280
aggctttccc aacagctgcc tctgaaatag ctgctgcttc gagatcctcc cttttaaag 2340
cactttctaa agccctcagg atggcgggag caaacagcac tggtatattc taggagtaag 2400
tgcaggaatt cagcagtgag agcatgtctg ggaccacctg gactgccatc catttaacct 2460
caaatctctt tgggatactc gccctccctg ggaaccagag ttctggctct aacattgagc 2520
agctatgcac tagttccaga gaagccacta acaggctgcc atgtgtagat gtaggttctt 2580
aagagatcac aggctgggtc atctgatcac tggatggata gctcagcctg gggcatttag 2640
tgttttccct ggtgataaat ccccaaggca gctggatttg gagctggtgg caagttgaaa 2700
ttattaaaaa ttgatttgtg tgggactgtc 2730

<210> 18
<211> 1972
<212> DNA
<213> Homo sapiens

<400> 18

aggggttggc cctggagtct gccagccaca ctgggaaaac attggagctt ggtctgccat 60
cctgaactgt ggttcccctg cctcccagcc cggctgtgag tgctcagcga ccctgacagg 120
ctgacagttg ggccatatgg tgctcttccc agtctggttc ctgtacagtc tgctcatgaa 180
gctgttccag cgctccacgc cagccatcac cctcgagagc ccggacatca agtacccgct 240
gcggctcatc gaccgggaga tcatcagcca tgacacccgg cgcttccgct ttgccctgcc 300
gtcaccccag cacatcctgg gcctccctgt cggccagcac atctacctct cggctcgaat 360
tgatggaaac ctggtcgtcc ggccctatac acccatctcc agcgatgatg acaagggctt 420
cgtggacctg gtcatcaagg tttacttcaa ggacacccat cccaagtttc ccgctggagg 480
gaagatgtct cagtacctgg agagcatgca gattggagac accattgagt tccggggccc 540
cagtgggctg ctggtctacc agggcaaagg gaagttcgcc atccgacctg acaaaaagtc 600
caaccctatc atcaggacag tgaagtctgt gggcatgatc gcgggaggga caggcatcac 660
cccgatgctg caggtgatcc gcgccatcat gaaggaccct gatgaccaca ctgtgtgcca 720
cctgctcttt gccaaccaga ccgagaagga catcctgctg cgacctgagc tggaggaact 780
caggaacaaa cattctgcac gcttcaagct ctggtacacg ctggacagag cccctgaagc 840
ctgggactac ggccagggct tcgtgaatga ggagatgatc cgggaccacc ttccaccccc 900
agaggaggag ccgctggtgc tgatgtgtgg ccccccaccc atgatccagt acgcctgcct 960
tcccaacctg gaccacgtgg gccaccccac ggagcgctgc ttcgtcttct gagggccggg 1020

cacggt caca cggccacccg ccccgcgcac cccacgccct gt t cacgct c acccagt cac 1080
ct ccccacat cgcacact gg ggccccgggt t cagcct ggc ct gcccgt gc cct ggt gaat 1140
cacct ggct g agcagt t ccc ct ggagcccc t t cgggagca gggct gt gt c ccagat gggc 1200
cacggct gag cct t cagagt acgt cct gcc t ggcact t ac t ggt cct t ac cagagacgcc 1260
cagccccat c cct gt cct ca t gacccct cg t ccacccccc acacacact a t aaggct gag 1320
ggct gccagc agccccgt ct gcccaccat t cccggccgt g gaccat agt c gggat gt cag 1380
cagacacaca t gggcagccc aaagct gcag gt gccagggc ccaccccagc ct cgcct gt c 1440
acccccact c ccgcct cagg gccaggccca ggcct cacca cct gacgct g cat gagacat 1500
t gacaccaga aagccct ct t gggggcact g ct ccct accc cagggccct g gccagccggg 1560
agct t ggct c t cct ct ggct agagt gggaa gagggggct g gccat ggggc cct cccagaa 1620
cct cagcat t t cct t ccagc ccat ccaaac act gaggcag cct t ggggaa ccccgagct g 1680
gggggt t ggc agcccact gc accgcct cag ggt t t t gggg t cct gggct g gggccaccat 1740
ccct gat ggc agaact ccca caaccacat g t at t t at t cc t ct gt cct aa accgt cccct 1800
cct t ccct ca cccccagcac agggggat t c t gagcagt gc ct ct t gt ct g agggacat at 1860
cagt gacct c gacgt t gcct t t agact aca gt t gt gt t ag cct ct t gcgt at t ggct t t t 1920
t cagagt cat t t at gagcag aaaaaaaaaa agt aaaact t t gct aat at t aa 1972

<210> 19
<211> 1852
<212> DNA
<213> Homo sapiens

<400> 19

agcgcgcgac t t t t t gaaag ccaggagggt t cgaat t gca acggcagct g ccgggcgt at 60
gt gt t ggt gc t agaggcagc t gcagggt ct cgct gggggc cgct cgggac caat t t t gaa 120
gaggt act t g gccacgact t at t t t cacct ccgacct t t c ct t ccaggcg gt gagact ct 180
ggact gagag t ggct t t cac aat ggaaggg at cagt aat t t caagacacc aagcaaat t a 240
t cagaaaaaa agaaat ct gt at t at gt t ca act ccaact a t aaat at ccc ggcct ct ccg 300
t t t at gcaga agct t ggct t t ggt act ggg gt aaat gt gt acct aat gaa aagat ct cca 360
agaggt t t gt ct cat t ct cc t t gggct gt a aaaaagat t a at cct at at g t aat gat cat 420
t at cgaagt g t gt at caaaa gagact aat g gat gaagct a agat t t t gaa aagcct t cat 480
cat ccaaaca t t gt t ggt t a t cgt gct t t t act gaagcca at gat ggcag t ct gt gt ct t 540
gct at ggaat at ggaggt ga aaagt ct ct a aat gact t aa t agaagaacg at at aaagcc 600
agccaagat c ct t t t ccagc agccat aat t t t aaaagt t g ct t t gaat at ggcaagaggg 660
t t aaagt at c t gcaccaaga aaagaaact g ct t cat ggag acat aaagt c t t caaat gt t 720

gt aat t aaag gcgat t t t ga aacaat t aaa at ct gt gat g t aggagt ct c t ct accact g 780

gat gaaaat a t gact gt gac t gaccct gag gct t gt t aca t t ggcacaga gccat ggaaa 840

cccaaagaag ct gt ggagga gaat ggt gt t at t act gaca aggcagacat at t t gcct t t 900

ggcct t act t t gt gggaaat gat gact t t a t cgat t ccac acat t aat ct t t caaat gat 960

gat gat gat g aagat aaaac t t t t gat gaa agt gat t t t g at gat gaagc at act at gca 1020

gcgt t gggaa ct aggccacc t at t aat at g gaagaact gg at gaat cat a ccagaaagt a 1080

at t gaact ct t ct ct gt at g cact aat gaa gaccct aaag at cgt cct t c t gct gcacac 1140

at t gt t gaag ct ct ggaaac agat gt ct ag t gat cat ct c agct gaagt g t ggct t gcgt 1200

aaat aact gt t t at t ccaaa at at t t acat agt t act at c agt agt t at t agact ct aaa 1260

at t ggcat at t t gaggacca t agt t t ct t g t t aacat at g gat aact at t t ct aat at ga 1320

aat at gct t a t at t ggct at aagcact t gg aat t gt act g ggt t t t ct gt aaagt t t t ag 1380

aaact agct a cat aagt act t t gat act gc t cat gct gac t t aaaacact agcagt aaaa 1440

cgct gt aaac t gt aacat t a aat t gaat ga ccat t act t t t at t aat gat ct t t ct t aaa 1500

t at t ct at at t t t aat ggat ct act gacat t agcact t t g t acagt acaa aat aaagt ct 1560

acat t t gt t t aaaacact ga acct t t t gct gat gt gt t t a t caaat gat a act ggaagct 1620

gaggagaat a t gcct caaaa agagt agct c ct t ggat act t cagact ct g gt t acagat t 1680

gt ct t gat ct ct t ggat ct c ct cagat ct t t ggt t t t t gc t t t aat t t at t aaat gt at t 1740

t t ccat act g agt t t aaaat t t at t aat t t gt acct t aag cat t t cccag ct gt gt aaaa 1800

acaat aaaac t caaat agga t gat aaagaa t aaaggacac t t t gggt acc ag 1852

<210> 20
<211> 2883
<212> DNA
<213> Homo sapiens

<400> 20

ccgggt gggc t ccaggcggc cggt ccccgg cct ccccccca t ggccaccgc cccct ct t at 60

cccgccgggc t ccct ggct c t cccgggccg gggt ct cct c cgcccccccgg cggcct agag 120

ct gcagt cgc cgccaccgct act gccccag at cccggccc cgggt t ccgg ggt ct cct t t 180

cacat ccaga t cgggct gac ccgcgagt t c gt gct gt t gc ccgccgcct c cgagct ggct 240

cat gt gaagc agct ggcct g t t ccat cgt g gaccagaagt t ccct gagt g t ggct t ct ac 300

ggcct t t acg acaagat cct gct t t t caaa cat gacccca cgt cggccaa cct cct gcag 360

ct ggt gcgct cgt ccggaga cat ccaggag ggcgacct gg t ggaggt ggt gct gt cggcc 420

t cggccacct t cgaggact t ccagat ccgc ccgcacgccc t cacggt gca ct cct at cgg 480

gcgcct gcct t ct gt gat ca ct gcggggag at gct ct t cg gcct agt gcg ccagggcct c 540

aagtgcgatg gctgcgggct gaactaccac aagcgctgtg ccttcagcat ccccaacaac 600
tgtagtgggg cccgcaaacg gcgcctgtca tccacgtctc tggccagtgg ccactcggtg 660
cgcctcggca cctccgagtc cctgccctgc acggctgaag agctgagccg tagcaccacc 720
gaactcctgc ctcgccgtcc cccgtcatcc tcttcctcct cttctgcctc atcgtatacg 780
ggccgcccca ttgagctgga caagatgctg ctctccaagg tcaaggtgcc gcacaccttc 840
ctcatccaca gctatacacg gcccaccgtt tgccaggctt gcaagaaact cctcaagggc 900
ctcttccggc agggcctgca atgcaaagac tgcaagttta actgtcacaa acgctgcgcc 960
acccgcgtcc ctaatgactg cctgggggag gcccttatca atggagatgt gccgatggag 1020
gaggccaccg atttcagcga ggctgacaag agcgccctca tggatgagtc agaggactcc 1080
ggtgtcatcc ctggctccca ctcagagaat gcgctccacg ccagtgagga ggaggaaggc 1140
gagggaggca aggcccagag ctccctgggg tacatccccc taatgagggt ggtgcaatcg 1200
gtgcgacaca cgacgcggaa atccagcacc acgctgcggg agggttgggt ggttcattac 1260
agcaacaagg acacgctgag aaagcggcac tattggcgcc tggactgcaa gtgtatcacg 1320
ctcttccaga acaacacgac caacagatac tataaggaaa ttccgctgtc agaaatcctc 1380
acggtggagt ccgcccagaa cttcagcctt gtgccgccgg gcaccaaccc acactgcttt 1440
gagatcgtca ctgccaatgc cacctacttc gtgggcgaga tgcctggcgg gactccgggt 1500
gggccaagtg ggcagggggc tgaggccgcc cggggctggg agacagccat ccgccaggcc 1560
ctgatgcccg tcatccttca ggacgcaccc agcgccccag gccacgcgcc ccacagacaa 1620
gcttctctga gcatctctgt gtccaacagt cagatccaag agaatgtgga cattgccact 1680
gtctaccaga tcttccctga cgaagtgctg ggctcagggc agtttggagt ggtctatgga 1740
ggaaaacacc ggaagacagg ccgggacgtg gcagttaagg tcattgacaa actgcgcttc 1800
cctaccaagc aggagagcca gctccggaat gaagtggcca ttctgcagag cctgcggcat 1860
cccgggatcg tgaacctgga gtgcatgttc gagacgcctg agaaagtgtt tgtggtgatg 1920
gagaagctgc atggggacat gttggagatg atcctgtcca gtgagaaggg ccggctgcct 1980
gagcgcctca ccaagttcct catcacccag atcctggtgg ctttgagaca ccttcacttc 2040
aagaacattg tccactgtga cttgaaacca gaaaacgtgt tgctggcatc agcagaccca 2100
tttcctcagg tgaagctgtg tgactttggc tttgctcgca tcatcggcga gaagtcgttc 2160
cgccgctcag tggtgggcac gccggcctac ctggcacccg aggtgctgct caaccagggc 2220
tacaaccgct cgctggacat gtggtcagtg ggcgtgatca tgtacgtcag cctcagcggc 2280
accttccctt tcaacgagga tgaggacatc aatgaccaga tccagaacgc cgccttcatg 2340
tacccggcca gcccctggag ccacatctca gctggagcca ttgacctcat caacaacctg 2400
ctgcaggtga agatgcgcaa acgctacagc gtggacaaat ctctcagcca cccctggtta 2460
caggagtacc agacgtggct ggacctccga gagctggagg ggaagatggg agagcgatac 2520
atcacgcatg agagtgacga cgcgcgctgg gagcagtttg cagcagagca tccgctgcct 2580

gggt ct gggc t gcccacgga cagggat ct c ggt ggggcct gt ccaccaca ggaccacgac 2640
at gcaggggc t ggcggagcg cat cagt gt t ct ct gaggt c ct gt gccct c gt ccagct gc 2700
t gccct ccac agcggt t ct t cacaggat cc cagcaat gaa ct gt t ct agg gaaagt ggct 2760
t cct gcccaa act ggat ggg acacgt gggg agt ggggt gg ggggagct at t t ccaaggcc 2820
cct ccct gt t t ccccagcaa t t aaaacgga ct cat ct ct g gccccat ggc ct t gat ct ca 2880
gca 2883

<210> 21
<211> 1463
<212> DNA
<213> Homo sapiens

<400> 21

cgcccct gac accat t cct c cct t cccccc t ccaccggcc gcgggcat aa aaggcgccag 60
gt gagggcct cgccgct cct cccgcgaat c gcagct t ct g agaccagggt t gct ccgt cc 120
gt gct ccgcc t cgccat gac t t cct acagc t at cgccagt cgt cggccac gt cgt cct t c 180
ggaggcct gg gcggcggct c cgt gcgt t t t gggccggggg t cgcct t t cg cgcgcccagc 240
at t cacgggg gct ccggcgg ccgcggcgt a t ccgt gt cct ccgcccgct t t gt gt cct cg 300
t cct cct cgg gggcct acgg cggcggct ac ggcggcgt cc t gaccgcgt c cgacgggct g 360
ct ggcgggca acgagaagct aaccat gcag aacct caacg accgcct ggc ct cct acct g 420
gacaaggt gc gcgccct gga ggcggccaac ggcgagct ag aggt gaagat ccgcgact gg 480
t accagaagc aggggcct gg gccct cccgc gact acagcc act act acac gaccat ccag 540
gacct gcggg acaagat t ct t ggt gccacc at t gagaact ccaggat t gt cct gcagat c 600
gacaat gccc gt ct ggct gc agat gact t c cgaaccaagt t t gagacgga acaggct ct g 660
cgcat gagcg t ggaggccga cat caacggc ct gcgcaggg t gct ggat ga gct gaccct g 720
gccaggaccg acct ggagat gcagat cgaa ggcct gaagg aagagct ggc ct acct gaag 780
aagaaccat g aggaggaaat cagt acgct g aggggccaag t gggaggcca ggt cagt gt g 840
gaggt ggat t ccgct ccggg caccgat ct c gccaagat cc t gagt gacat gcgaagccaa 900
t at gaggt ca t ggccgagca gaaccggaag gat gct gaag cct ggt t cac cagccggact 960
gaagaat t ga accgggaggt cgct ggccac acggagcagc t ccagat gag caggt ccgag 1020
gt t act gacc t gcggcgcac cct t cagggt ct t gagat t g agct gcagt c acagct gagc 1080
at gaaagct g cct t ggaaga cacact ggca gaaacggagg cgcgct t t gg agcccagct g 1140
gcgcat at cc aggcgct gat cagcggt at t gaagcccagc t gggcgat gt gcgagct gat 1200
agt gagcggc agaat cagga gt accagcgg ct cat ggaca t caagt cgcg gct ggagcag 1260
gagat t gcca cct accgcag cct gct cgag ggacaggaag at cact acaa caat t t gt ct 1320

gcctccaagg tcctctgagg cagcaggctc tggggcttct gctgtccttt ggagggtgtc 1380
ttctgggtag agggatggga aggaagggac ccttaccccc ggctcttctc ctgacctgcc 1440
aataaaaatt tatggtccaa ggg 1463

<210> 22
<211> 2309
<212> DNA
<213> Homo sapiens

<400> 22

cgcgctggag aggcgcgggg tcaagacgtc cggcctcggg ggcccgggct gggccgccga 60
gtcccctggg cggcgctgag cgggcgggag gcgggggccc gcggctcggg gcaaccgagg 120
ccgggtcgcg ggatcggccg ccatcgccgc cgccgctgca ctgggagccc gcggggatgg 180
agcgggaggc gttgccgtgg ggcctcgagc cccaggatgt gcagagttct gacgaaatga 240
ggagccccga agggtacctc agaggcaaca tgagtgagaa tgaggaagag gaaatttctc 300
agcaagaagg cagtggggac tatgaagtcg aagagatacc atttgggctt gaaccccaga 360
gccctgggtt tgagccacaa agcccagagt ttgaacccca aagccccaga tttgagcctg 420
aaagcccggg gtttgagtcc cgaagccctg ggcttgtgcc cccaagccct gagtttgcac 480
ccagaagccc tgaatcagat tctcagagcc ctgagtttga atcccagagc cctaggtatg 540
aaccccaaag ccctggctat gaacctcgga gccccgggta tgaaccccgg agccctggct 600
atgaatctga gagctctaga tatgaatccc agaacactga gctcaaaacc caaagcccag 660
aatttgaagc tcaaagttcc aaattccagg aaggtgcgga gatgcttctg aaccccgacg 720
aaaagagtcc tttgaatatc tccgtaggag ttcaccccct ggactccttc actcaggggt 780
ttggggagca gcccacaggg gacctgccca tagggccacc ttttgagatg cccacagggg 840
ccctgctgtc tacaccgcag tttgagatgc ttcagaatcc cctgggtctc acaggagccc 900
ttcgaggtcc aggtcggcgg ggtggccggg ccaggggtgg gcagggccct cggcctaaca 960
tctgcggcat ctgcgggaag agcttcgggc ggggctccac cctgatccag caccagcgca 1020
tccacaccgg tgagaagccc tacaaatgtg aggtctgcag caaggccttc tcccagagct 1080
ctgacctcat caaacaccag cgcacccaca ctggcgagcg gccctacaaa tgtccccgtt 1140
gcggcaaggc cttcgccgac agctcttacc tgcttcgcca ccagcgcact cactctggcc 1200
agaagcccta caagtgccca cattgtggca aggccttcgg cgacagctcc tacctcctgc 1260
gacaccagcg cacccacagc cacgagcggc cctacagctg caccgagtgc ggcaagtgct 1320
atagccagaa ctcgtccctg cgcagccatc agagggtgca caccggtcag aggcccttca 1380
gctgtggcat ctgcggcaag agcttctccc agcggtcggc ccttatcccc catgcccgca 1440
gccacgcccg ggagaagccc ttcaagtgcc ctgagtgcgg caagcgcttt ggccagagct 1500

cggt gct ggc cat ccacgcc cgcacccacc t gccaggccg cacct acagc t gccccgact 1560
gcggcaagac ct t caat cgc t cct ccact c t cat ccagca ccagcgct cc cacacgggcg 1620
agcggccct a caggt gcgcc gt gt gcggca agggct t ct g ccgct cct cc acgct t ct gc 1680
agcat caccg ggt ccacagt ggcgagcggc ct t acaagt g cgat gact gc ggaaaggcct 1740
t ct cccagag ct ccgacct c at ccgccacc agcggaccca cgcggcgggc cggcgct gac 1800
ct ggggcccc agcaggggt g ggaggt gt gg gcagaagat a aggggccagg gagct agagg 1860
aacct t t agg gaggat agt g gagaagccgc aggagaat gg aggagct gag gat gct ggaa 1920
gaagagaacc agggt ggagg aagt gacat g ccct ggagac t t gt gggaag t gggt t ggag 1980
ggaggccagg ccggcagcgg gaggccct gg gagaaat gga gagaggt cag cggagggct g 2040
gcct cagccg cggct cct t g gt gggt gcct ggct t ggcaa ct gct agagc aggga ggggg 2100
gagggcaggg gat t acct aa t t agagt ggg t t agct t aga t t ggt agct g ct gaaact ct 2160
cgt t gagt ca ggagcgggt a aaaggt aggt ggggt gggga gt ggggcccc ggggt ggggc 2220
ct gggcct ct gcgt gcaaac cccagcct cc ct ct t ct t cc t caggct t t g gagcccct ga 2280
gt t t gagt t c aat aaaaact t t at gt ggt 2309

<210> 23
<211> 1853
<212> DNA
<213> Homo sapiens

<400> 23

cacagggact gcgct ccct t gcccct agcg ct cccgcgct gct gct ccag ccgcccggca 60
gct ct gagga t ggagaggag ggcgcggagc t cct ccaggg agt cccgcgg gcgaggcggc 120
aggact ccgc acaaggagaa caagagggca aaggccgaga ggagcggcgg gggccgcggg 180
cgccaggagg ct gggcccga gccgt cgggc t ccggacggg cggggacccc gggggagccc 240
cgagcgcccg ccgccacggt ggt ggacgt g gat gaggt cc gaggct ccgg cgaggagggc 300
accgaggt gg t ggcgct gt t ggagagcgag cggcccgagg aaggt accaa at cct ccggc 360
t t aggggcct gt gagt ggct t ct t gt cct c at t t ccct gc t ct t cat cat cat gacct t c 420
cct t t t t cca t ct ggt t ct g cgt aaaggt t gt acaagagt at gaaagagt aat t at at t c 480
cgact gggac at ct gct t cc t ggaagagcc aaaggccct g gt ct t t t ct t t t t t t t gccc 540
t gcct ggat a cct accacaa ggt t gacct t cgt ct ccaaa ct ct ggagat acct t t t cat 600
gagat cgt ga ccaaagacat gt t t at aat g gagat agat g ccat t t gct a ct accgaat g 660
gaaaat gcct ct ct t ct cct aagcagt ct t gct cat gt at ct aaagct gt gcaat t cct t 720
gt gcaaacca ct at gaagcg t ct cct agca cat cgat ccc t cact gaaat t ct t ct agag 780
aggaagagca t cgcccaaga t gcaaaggt t gcct t ggat t cagt gacct g t at t t gggga 840

```
atcaaagtgg agagaataga aattaaagat gtgaggttgc cagctgggct tcagcactca 900
ctggctgtgg aggctgaagc gcaaagacaa gccaaagtgc ggatgattgc tgcagaagcg 960
gaaaaggctg cttctgagtc cctgaggatg gcagctgaga ttctgtcagg cacccctgct 1020
gctgttcagc ttcgatacct ccacaccctt cagtctctgt ccacagagaa gccttccact 1080
gtggttttac ctttgccatt tgacctactg aattgcctgt cttctcccag caacagaact 1140
cagggaagcc tccccttccc aagtccttcc aaacctgttg agccactaaa tcctaaaaag 1200
aaagactctc ccatgttata ggaaggatgg ggcataatgt gactgtaaag ggcctgcca 1260
tagaaaagtc acatccctga gggagacact ctgtcctcat tccctgccct tcctttggtt 1320
gccatatgga atggccatgg aatgcacgaa gtcacaatgc accatccatg agaagacagt 1380
gaaatgatgt aatgacagag aaggcagaca acatgtttcc gtgactcatc tagtcagagc 1440
aattatggga aacagctttg gtcaacattc tactttggaa agaattttga gtctagatgt 1500
ggttaaattt tgacttctgg gaacttggtt cagatgtccc tttcactgta tgtcctctga 1560
cccctttggc aaggttgcca cagctcccac agcccttcct acaagcacct atcattgggc 1620
ttgtcacact ctattgctct tctgtcccga agatgcagtc ttctctccaa tgatactacc 1680
aagtcttagt tttcctcaac cacactcaat ctttttgctc caccctgaat tcctcacacc 1740
taaccctgat agttacctaa agtgacactt aaatgtttca gagtgaatgc aaaaaagaga 1800
gatgtacttg gagtcggata tacaatttat ccctaattaa agcatttaaa agg 1853
```

<210> 24
<211> 765
<212> DNA
<213> Homo sapiens

<400> 24

```
ggcaagcact ttaacctttt aagcccaacc agatgagttg cctgcagttt tggaggcctt 60
cagagcattt cactagacct ctgtctgtgt cggtccagtg tctttagcca agctttgatt 120
aaagatgact tccttgtttg ctcaagaaat tcgcctttct aaaagacatg aagaaatagt 180
atcacaaaga ttaatgttac ttcaacaaat ggagaataaa ttgggtgatc aacacacaga 240
aaaggcatct caactccaaa ctgttgagac tgcttttaaa aggaacctta gtcttttaaa 300
ggatatagaa gcagcagaaa agtcactaca gaccaggatt cacccacttc cacggcctga 360
ggtggtttct cttgagactc gttactgggc atcagtagaa gaatatattc ccaaatggga 420
acagtttctt ttaggaagag caccatatcc ttttgctgtt gaaaatcaaa atgaagcaga 480
aaataccatt caaaatgagg cacagcgata acttcttcac atgctatttc aaaaagcctg 540
tttaataaag ctgaatgtta aggtgtatgt aggttattgc aggaacttta ggaattaaat 600
atgttcatat tcttcgatta tctcctaagt gacagtgaag atatgagaat ttactggcaa 660
```

gtcacatgtt atcacctact actatttcaa ggtcatgaat ttgctttcta ccaaacccat 720
agatgtgtta aacacgaata ttaaaaggtg gactctttat taatt 765

<210> 25
<211> 2448
<212> DNA
<213> Homo sapiens

<400> 25

acgtcccagc gtctgagaga acgagtaagc acagaattca aagcattctg cagcctgaat 60
tttgaaggag tgtgtttagg cactaagcaa gctgatttat gataactgct ttaaacttca 120
acaaccaaag gcataagaac taggagctgc tgacatttca atatgaaggg caactccacc 180
cttgccacta ctagcaaaaa cattaccagc ggtcttcact tcgggcttgt gaacatctct 240
ggcaacaatg agtctacctt gaactgttca cagaaaccat cagataagca tttagatgca 300
attcctattc tttactacat tatatttgta attggatttc tggtcaatat tgtcgtggtt 360
acactgtttt gttgtcaaaa gggtcctaaa aaggtttcta gcatatacat cttcaacctc 420
gctgtggctg atttactcct tttggctact cttcctctat gggcaaccta ttattcttat 480
agatatgact ggctctttgg acctgtgatg tgcaaagttt ttggttcttt tcttaccctg 540
aacatgtttg caagcatttt ttttatcacc tgcatgagtg ttgataggta ccaatctgtc 600
atctacccct ttctgtctca aagaagaaat ccctggcaag catcttatat agttcccctt 660
gtttggtgta tggcctgttt gtcctcattg ccaacatttt attttcgaga cgtcagaacc 720
attgaatact taggagtgaa tgcttgcatt atggctttcc cacctgagaa atatgcccaa 780
tggtcagctg ggattgcctt aatgaaaaat atccttggtt ttattatccc tttaatattc 840
atagcaacat gctattttgg aattagaaaa cacttactga agacgaatag ctatgggaag 900
aacaggataa cccgtgacca agtcctgaag atggcagctg ctgttgttct ggccttcatc 960
atttgctggc ttcccttcca tgttctgacc ttcctggatg ctctggcctg gatgggtgtc 1020
attaatagct gcgaagttat agcagtcatt gacctggcac ttccttttgc catcctcttg 1080
ggattcacca acagctgcgt taatccgttt ctgtattgtt ttgttggaaa ccggttccaa 1140
cagaagctcc gcagtgtgtt tagggttcca attacttggc tccaagggaa aagagagagt 1200
atgtcttgcc ggaaaagcag ttctcttaga gaaatggaga cctttgtgtc ttaaacgtga 1260
gagcaaaatg catgtaatca acatggctac ttgctttgag gctcaccaga attattttta 1320
agtggtttta ataaaataat aaaatttccc ctaatctttt ctgaatcttc tgaaaccaaa 1380
tgtaactatg ttttatcgtc cagtgacttt caggaattgc ccattgtttt tctgatatgt 1440
ttgtacaaga tttcattggt gagacatatt tacaacctag aagtaactgg tgatatatct 1500
caaattgtaa ttaataatag attgtgaata atgatttggg gattcagatt tctctttgaa 1560

acatgcttgt gtttcttagt ggggttttat atccattttt atcaggattt cctcttgaac 1620
cagaaccagt ctttcaactc attgcatcat ttacaagaca acattgtaag agagatgagc 1680
acttctaagt tgagtatatt ataatagatt agtactggat tattcaggct ttaggcatat 1740
gcttctttaa aaacgctata aattatattc ctcttgcatt tcacttgagt ggaggtttat 1800
agttaatcta taactacata ttgaataggg ctaggaatat agattaaatc atactcctat 1860
gctttagctt atttttacag ttatagaaag caagatgtac tataacatag aattgcaatc 1920
tataatattt gtgtgttcac taaactctga ataagcactt tttaaaaaac tttctactca 1980
ttttaatgat tgtttaaagg tttctatttt ctctgatact tttttgaaat cagtaaacac 2040
tgtgtattgt tgtaaaatgt aaaggtcact tttcacatcc ttgacttttt agatgtgctg 2100
ctttgatata taggacattg atttgatttt tattattaat gctttggttc tgggttgttt 2160
cctaaaatat ctgggtggct taaaaaaaac tctttaactt gtaataaacc cttaactggc 2220
ataggaaatg gtatccagaa tggaattttg ctacatgggg tctgggtggg ggcaaagaga 2280
cccagtcaat tacatgtttg gtaccaagaa aggaacctgt cagggcagta caatgtgact 2340
ttgaaaatat ataccgtggg ggtagtttta ccctatatct ataaacactg tttgttccag 2400
aatctgtatg attctatgga gctattttaa accaattgca ggtctaga 2448

<210> 26
<211> 1101
<212> DNA
<213> Homo sapiens

<400> 26

atggatttct taaattcatc tgatcaaaac ttgacctcag aggaactgtt aaacagaatg 60
ccatccaaaa ttctggtgtc cctcactctg tctgggctgg cactgatgac aacaactatc 120
aactcccttg tgatcgctgc aattattgtg acccggaagc tgcaccatcc agccaattat 180
ttaatttgtt cccttgcagt cacagatttt cttgtggctg tcctggtgat gccccttcagc 240
attgtgtata ttgtgagaga gagctggatt atggggcaag tggtctgtga catttggctg 300
agtgttgaca ttacctgctg cacgtgctcc atcttgcatc tctcagctat agctttggat 360
cggtatcgag caatcacaga tgctgttgag tatgccagga aaaggactcc aaagcatgct 420
ggcatttatga ttacaatagt ttggattata tctgttttta tctctatgcc tcctctattc 480
tggaggcacc aaggaactag cagagatgat gaatgcatca tcaagcacga ccacattgtt 540
tccaccattt actcaacatt tggagctttc tacatcccac tggcattgat tttgatcctt 600
tactacaaaa tatatagagc agcaaagaca ttataccaca agagacaagc aagtaggatt 660
gcaaaggagg aggtgaatgg ccaagtcctt ttggagagtg gtgagaaaag cactaaatca 720
gtttccacat cctatgtact agaaaagtct ttatctgacc catcaacaga ctttgataaa 780

at t cat agca cagt gagaag t ct caggt ct gaat t caagc at gagaaat c t t ggagaagg 840
caaaagat ct caggt acaag agaacggaaa gcagccact a ccct gggat t aat ct t gggt 900
gcat t t gt aa t at gt t ggct t cct t t t t t t gt aaaagaat t agt t gt t aa t gt ct gt gac 960
aaat gt aaaa t t t ct gaaga aat gt ccaat t t t t t ggcat ggct t gggt a t ct caat t cc 1020
ct t at aaat c cact gat t t a cacaat ct t t aat gaagact t caagaaagc at t ccaaaag 1080
ct t gt gcgat gt cgat gt t a g 1101

<210> 27
<211> 6103
<212> DNA
<213> Homo sapi ens

<400> 27

at ccggggca gcggggaat g gct gagccag gggt t cgccg cccccgccgc cgccgccgcc 60
gccgccgccg ccgccgccgc ccgct t t cgg ct cgggcct c aggaccgt ag cat cct gaga 120
cat t t t gaat t gacact t ct caagat t t ga ct ggat caga gt t cat cat g t caaagt t ga 180
aaagct caga gt cagt cagg gt ggt ggt t c gct gt cggcc cat gaat ggc aaggaaaagg 240
ct gct t cgt a t gacaaagt g gt ggat gt gg at gt t aagct ggggcaggt g t ct gt gaaga 300
accccaaagg gacggcccat gaaat gccca agacct t cac ct t t gat gcc gt ct at gact 360
ggaat gccaa gcagt t t gaa ct gt acgat g agacgt t ccg accact t gt t gact ct gt cc 420
t gcaaggt t t caat ggaacc at t t t t gcct at ggacaaac t gggacagga aaaacct aca 480
ccat ggaagg aat ccgt ggt gaccct gaaa aaagaggagt cat t cct aac t cat t t gacc 540
at at ct t cac ccacat ct ct cgat cccaga at caacaat a cct ggt cagg gct t ct t act 600
t agagat ct a ccaggaggag at ccgagat t t gct ct caaa ggat cagacc aaaaggct t g 660
agct caaaga gaggcct gac acaggagt gt at gt gaaaga cct gt ct t cc t t t gt cacca 720
agagt gt gaa ggagat agag cat gt gat ga at gt ggggaa ccagaaccgt t ct gt cggt g 780
ct accaacat gaacgagcac agct cgcgt t ct cat gcaat t t t cgt t at c act at t gagt 840
gcagcgaggt gggcct cgat ggt gaaaacc acat ccgt gt aggaaaat t g aacct t gt ag 900
at ct t gct gg cagcgaacgg caagccaaga ccggcgcaca aggggagaga t t aaaagaag 960
ct accaagat caacct ct cc ct t t ccgct t t gggt aat gt cat ct ct gct ct agt ggacg 1020
gcaaaagcac t cacat t cca t at cgggact caaagct t ac caggct cct c caagat t ccc 1080
t t ggt ggcaa t gccaagact gt gat ggt gg ccaacgt ggg gcct gcct ct t acaacgt ag 1140
aagagact ct gaccact ct g cgat at gcca accgt gccaa aaacat t aag aacaaaccaa 1200
gggt caat ga ggaccccaag gat gccct cc t t cgagaat t ccaggaagag at t gct cggc 1260
t caaggccca gct ggaaaaa cggt ccat t g gt aggaggaa gaggcgagag aagcggaggg 1320

aaggtggtgg cagtggtggg ggtggggaag aggaggagga ggagggagaa gagggtgagg 1380
aggaagggga tgataaggat gattactggc gggaacagca agaaaaactg gagattgaga 1440
agcgggccat tgtagaggat cacagcttgg ttgcagagga gaagatgagg ctgctgaagg 1500
agaaagagaa aaagatggag gacctgcggc gggagaagga tgctgccgag atgctgggcg 1560
ccaagatcaa ggccatggag agtaagttgc ttgttggagg aaaaaatata gtagatcata 1620
cgaatgaaca gcagaaaatc ctggagcaga aacgacagga aattgcagag cagaaacgtc 1680
gagaaagaga aatccagcaa cagatggaaa gtcgagatga ggagaccttg gaacttaaag 1740
agacatacag ctcattgcag caagaggtgg acatcaagac caaaaaactc aaaaagctct 1800
tctccaagct tcaggcagtg aaggctgaga tccatgacct ccaagaagaa cacatcaagg 1860
agcgccaaga gctagagcag actcagaatg agctcaccag ggagctgaaa ctcaagcatc 1920
ttattataga aaactttatc cctctggaag aaaaaagtaa aatttatgaat agagccttct 1980
ttgatgaaga ggaagatcat tggaaactac atcctataac cagactggag aaccagcaga 2040
tgatgaagcg gccagtctca gccgtgggat ataagagacc attgagccag cacgcaagaa 2100
tgtccatgat gattcgtcca gaggcccgat atagggcaga aaacattgtg ctgttagagc 2160
tggacatgcc cagccggacc accagagact atgagggtcc agccattgcc cccaaggtcc 2220
aggctgcatt ggatgcggct ctgcaggatg aagatgagat acaggtggat gcatcatcat 2280
ttgaaagcac tgcaaataag aaatccaagg ccaggcctaa aagtggaagg aagtcgggat 2340
cctcctcctc ttcctcagga acccctgcat ctcagcttta tccacagtct cggggggctgg 2400
ttccaaagta aagccagctt ctcctctccc agggcggaaa cagcatttgc cttctgagag 2460
aagagactag caaaaagctg cagagaggat tcggcccaaa ctcagaactg ttcccctgag 2520
gagaagcggt ggcctctttg cagatcaacc aacttaatct ggttgaacgt gctgttccta 2580
atctggcact cagcccctct gggaaacatc ttttaattag catctcagaa atgcatgggt 2640
aaggtaaagt gcgatagttc aagtggaaag caagagaatg accagtgacc ttgcttcctt 2700
ccccttgcc ttcttctccc ccttcccctg tgctcccttt ctctcctctc tcctttttcta 2760
gcctgttctt tacatggggc tcccttcttg ttgaacaata gggcagaatc aggagtcacc 2820
ttagcaggac cacatctttg gagcctcggg ataaaatgac agtgaggttg aaaagtgaaa 2880
accctagaac ttgaataggt gcctgttctt gtagggagaa atgagaaatc gcatttggat 2940
ccaggcccca ggtgggcacc atcagcagtc ttgcttccat gcacctcagt aagaagtgga 3000
tctgcctttg ggacctgctc agtgaggaaa tctcttccaa tttctgcttc tgaatgattc 3060
aatgttggga gcaatagaaa taacattccc tttgccttct ctgagtgttt agggaaatag 3120
cttctttaaa acctcaaaac catgaccatc ctgtcaaaga cctaagtctg taagctggtg 3180
ccatgtccat acaccatgtc actttactct tcatttgtca ccatcttttc ccatgcacgc 3240
atactctgaa catccttgtg tgggcccatc ctctgcatcc agagcatgct ctgcagtggg 3300
cctgttttgt ggaagaaagg aggctgtctc tgccttctct gatgggactg gagttgaggg 3360

aaggagct gt at t gt ggcac t t ct gaat t c cccgt t t t gt t ccat at t gg t at agagagc 3420
agaagagt ag ct aggcagat gcagagat gg agacat gaga ct cagt gcag t gggcaggga 3480
agacat aaca gat ggaagca aaggaat cct gcct gcct t c agcagagaat t caccgaat c 3540
ct agaact gt ggct ccct cc aggcagagcc t aagat gct g gt gaagaat a gct gt gt gat 3600
t gaat aggct caaaggagag t t cagaat t c ccat t t acat at t act agt t t ggt t t gt aa 3660
gt t t t agt t c ct t gt at t at t gagat t cag agct t cat t t t at gt t ggt c at t aggt gaa 3720
t at t act cat t t t ccct caa gagaagct ca t aagt gt gt g t gggt gt gag agcacgat gg 3780
t gcct gt gt t ct gt gaat gt gt ccat at gt gt ct gt aaga gagacagaga ccaagaact t 3840
gcccaat t t t agaaat acac t aat gt gcag t t gt t gcct t t t gt ct gt at t gaaggccca 3900
t t gaat gact aat ccaggct ggaagcat t c ccat gt gggt gt ct gagt cc at gagccaag 3960
cct gagggga cagt gagt ct ccaggt ct gc cacact ggt g cacct t gct g gcacggt gcc 4020
t caggaaggt ggcgact cag gt gggcct t g agt t at at t t t aact cagct gct cagt t cc 4080
cagggcacat t t ct ggat ca gaacccat gg gaaacaggag gt act aagt g caat gt ct t a 4140
gcat t ct gca aaat ggagat ct gt t gt cca gcggct t at c t cct t t t t ag t aaccct t ct 4200
t t ct gaaccc agggccct t t t cagcct t cc ct cat at t t t ct t gagat ca aact t t act t 4260
ct t t ct t at t t act aagaat t t gcct gt t t gaat aagaac aaaacgct aa ggt gggt agc 4320
ct aagct gat t t t ct gct gg t t acacgt gt ct ct cacacc acat t t cct c aaagct aat c 4380
t gaat t ct gt aggct aaaaa t at t cat gt a gcaaat ct ga gaat t gaaaa ct gcagat aa 4440
ccggccgggt at ggt gact c at gcct gt aa t cct agcact t t gggaggcc gaggt gggt g 4500
gaccacct ga ggt t aggact t caagaccag cct ggccaac at ggt gaaac cccat ct gt a 4560
ct aaaaat ac aaaaat t t gc ct ggt gt ggt ggt gcat gcc t ct agt cct a gct act cggg 4620
aggct gaggc acgagaat ca ct t gaacct g ggaggcggag gt t gcagt ga gt cgagat aa 4680
cact act gca t t ccagcct g ggt gacagag t gagact cca cct caaaaaa aaaaaaaaaa 4740
aaaaaaaaaa cagaaagaaa gaaaaagaaa act gcagat a accct at aca t t aat act gg 4800
t at ct cgagg t gact ct t ct gaccaagggt ggt t aagt ga cacat agaac t t t t ct aaga 4860
gaagacagac aagt t gacag gcat gcct t g t act cagct g t gt t cat gt g gt ggt ct gt g 4920
gaaagaaaag aagact cat t t ggaaat gaa gct gt ccct t t ccaagcagt ct ct ggt gct 4980
t t t ct t ct ct caaaat ggat ccgat aaat a t t t gaat aga gcagat t gt a gaat gt cgt g 5040
ct gt caccag aaagct gct g t t t t gggt t c t gcat t gagc caaat at gt a gaggacct ac 5100
caagcccact gagggact ag gt t t t cat gt ct ct agt cat acct agaat g t t ct gagccg 5160
t ct gagggcc t t cat gccgg cagcagct ag caaagccaga aagcaagt ct aacaggat ct 5220
aagat gacca t caggagaag gagt t t gaga ct gt gt at gc aaccccccaat agaccccct t 5280
t t act ct gat ct ggagaat g t at ct ggct t cat at t t t ca agt cacat gt ct ct cagacc 5340
cct ggat t ca gaacccaagg ccacaaat ca t aggcat gaa gcact t t ct t aagact gacc 5400

taacgctgga ttatttcccg tccaatgcct gcatgctgct tgaattgctc cacccacacc 5460
tccatgacca agggcgccag agtgctgcaa ctggggcgtg ggccgctctc tgcttttcct 5520
gtctgactct gacaagtcct ccctcactga atgtagaatc gttgccaagt ttctgagaag 5580
tgtcgattcc ctgttaacat ggatatcagt tctgcctcac atttcccact tgaggttgag 5640
gcgtactgga gacaacacct cagaccatct gaaccccatc agtggacgaa aatggggctg 5700
ttaatatact ctaaaagcca tactaaaaat gctctgaggg aactggctaa gaatagtggg 5760
cctggtgatt gtctatcacg caaggctttg ttttgtactg ttcagaaatc tgtcaccttt 5820
ctgcctgccc ttgtttcctg aatgaaatgc ttctggggtt atttatgaaa ggagtgatcc 5880
tggggcaggc aggaggcagt gggcttcatg gctccttgaa gttattactg atcttgacct 5940
tctctttggc tacctttaga caaagaatac gccaatcaat acttggggct ctaagtttta 6000
caattgatat ttatttgtat catctctttg tctaggaatg taaaagtgat tctaaactaa 6060
gatgtgtaat aaaaatcaat cagatttatt gtacctacaa aaa 6103

<210> 28
<211> 1318
<212> DNA
<213> Homo sapiens

<400> 28

atgaaggcca ctatcatcct ccttctgctt gcacaagttt cctgggctgg accgtttcaa 60
cagagaggct tatttgactt tatgctagaa gatgaggctt ctgggatagg cccagaagtt 120
cctgatgacc gcgacttcga gccctcccta ggcccagtgt gccccttccg ctgtcaatgc 180
catcttcgag tggtccagtg ttctgatttg ggtctggaca aagtgccaaa ggatcttccc 240
cctgacacaa ctctgctaga cctgcaaaac aacaaaataa ccgaaatcaa agatggagac 300
tttaagaacc tgaagaacct tcacgttgtc taccttcata acaacaatat ctctgtagtt 360
ggatcaagtg acttctgccc acctggacac aacaccaaaa aggcttctta ttcgggtgtg 420
agtcttttca gcaacccggt ccagtactgg gagatacagc catccacctt cagatgtgtc 480
tacgtgcgct ctgccattca actcggaaac tataagtaat tctcaagaaa gccctcattt 540
ttataacctg gcaaaatctt gttaatgtca ttgctaaaaa ataaataaaa gctagatact 600
ggaaacctaa ctgcaatgtg gatgttttac ccacatgact tattatgcat aaagccaaat 660
ttccagttta agtaattgcc tacaataaaa agaaattttg cctgccattt tcagaatcat 720
cttttgaagc tttctgttga tgttaactga gctactagag atattcttat ttcactaaat 780
gtaaaatttg gagtaaatat atatgtcaat atttagtaaa gcttttcttt tttaatttcc 840
aggaaaaaat aaaaagagta tgagtcttct gtaattcatt gagcagttag ctcatttgag 900
ataaagtcaa atgccaaaca ctagctctgt attaatcccc atcattactg gtaaagcctc 960

at t t gaat gt gt gaat t caa t acaggct at gt aaaat t t t t act aat gt c at t at t t t ga 1020
aaaaat aaat t t aaaaat ac at t caaaat t act at t gt at acaagct t aa t t gt t aat at 1080
t ccct aaaca caat t t t at g aagggagaag acat t ggt t t gt t gacaat a acagt acat c 1140
t t t t caagt t ct cagct at t t ct t ct acct ct ccct at ct t acat t t gag t at ggt aact 1200
t at gt cat ct at gt t gaat g t aagct t at a aagcacaaag cat acat t t c ct gact ggt c 1260
t agagaact g at gt t t caat t t acccct ct gct aaat aaa t at t aaaact at cat gt g 1318

<210> 29
<211> 1313
<212> DNA
<213> Homo sapiens

<400> 29

gggggcgca gcaggccagg gcaacccgcc gcgaggcggc gt ccacgccg aggagccgcg 60
cat t cccggt ccacacagac gcggcggagc gccct cccag gcgggact ac aact caat gc 120
ct ggcacggg ggcgggct cg gcggt gact t aat ccgggct gagt t t ggt g gt ggt agt ga 180
gggcaggt ag gggcact gcc cat t t t ggcc aagggt caca cagcat ct ac at cacaat t g 240
caccggagt t t aaaaat gt c t ggccct gt c cct ccccacc acccgccgct gt gt ccagac 300
agagaat gt t ct aacgct gg gggcggct gc ggat gaagt c ct t ggggaga aaaggagcag 360
gccaagggcg at ggt ggagt agagct gcct ct cagaggca gcat gagct g agagggt gat 420
aggaaggcgg cgct agacag cat ggaggac t t t ct gct ct ccaat gggt a ccagct gggc 480
aagaccat t g gggaagggac ct act caaaa gt caaagaag cat t t t ccaa aaaacaccaa 540
agaaaagt gg caat t aaagt t at agacaag at gggagggc cagaagagt t t at ccagaga 600
t t cct ccct c gggagct cca aat cgt ccgt accct ggacc acaagaacat cat ccaggt g 660
t at gagat gc t ggagt ct gc cgacgggaaa at ct gcct gg t gat ggagct cgct gaggga 720
ggggat gt ct t t gact gcgt gct gaat ggg gggccact gc ct gaaagccg ggccaaggcc 780
ct ct t ccgt c agat ggt t ga ggccat ccgc t act gccat g gct gt ggt gt ggcccaccgg 840
gacct caaat gt gagaacgc ct t gt t gcag ggct t caacc t gaagct gac t gact t t ggc 900
t t t gccaagg t gt t gcccaa gt cacaccgg gagct gagcc agacct t ct g cggcagt aca 960
gcct at gct g cccccgaggt gct gcagggc at t ccccacg at agcaaaaa aggt gat gt c 1020
t ggagcat gg gt gt ggt cct gt at gt cat g ct ct gt gcca gcct acct t t t gacgacaca 1080
gacat cccca agat gct gt g gcagcagcag aagggggt gt cct t ccccac t cat ct gagc 1140
at ct cggccg at t gccagga cct gct caag aggct cct gg aacccgat at gat cct ccgg 1200
cct t caat t g aagaagt t ag t t ggcat cca t ggct agcaa gcact t gat a aaagcaat gg 1260
caagt gct ct ccaat aaagt agggggagaa agcaaaccca aaaacccgct t ct 1313

<210> 30
<211> 3401

<212> DNA
<213> Homo sapiens

<400> 30

```
atgaggaggt cgctgagagc tgggaagagg cggcagacag cggggaaatc caaatctcct     60
cccaaagtgc ccattgtgat tcaggacgat agccttcccg cggggccccc tccacagatc    120
cgcatcctca agaggcccac cagcaacggt gtggtcagca gccccaactc caccagcagg    180
cccacccttc cagtcaagtc cctagcacag cgagaggccg agtacgccga ggcccggaag    240
cggatcctgg gcagcgccag ccccgaggag gagcaggaga aacccatcct cgacaggcca    300
accaggatct cccaacccga agacagcagg cagcccaata atgtgatcag acagcctttg    360
ggtcctgatg ggtctcaagg cttcaaacag cgcagataaa tgcaggcaag aaaagatgcc    420
gccgttgctg ccgtcaccgc ctcctgggtc gtccgccacg ggttgcactg ccgtggcaga    480
cagctggact tgagcagagg gaacgacctg acttacttgc actgtgatcc cccttgctcc    540
gcccactgtg accttgaacc ccatgcactg tgacctcccc ccttctcccc cttcccactg    600
tgattggcac atcgacaagg gctgtcccaa gtcaatggaa agggaaaggg tgggggttag    660
gggaaggttg gggggaccca gcaaggactc agagagtcag acagtgccac ttggccactt    720
ggggtaaagc cagtgccagc aataacagtt tatcatgctc attaatttgg gatttcaaaa    780
cacaaatgaa aactcacacc cacccacccc caagtgcatg tctccatcac ttaaaaagta    840
agttccattt gaaaatatcc tttctttttt ttttcttcct atttttgttt gtttatacaa    900
atatctgatt tgcaagaaaa agtgcatggg aggggtttta gtggtttaat gaatttttaa    960
ttaagaaagg gtagtttggt agtctactta aaaatgtttc tgggaaattc actagaaaca   1020
ttaaccaata ggattttggt gagcttagct tctgtattcc tactgccgcc cagaaaaggg   1080
gcagggctct gcagccgcca ggacagacga gcaccccatg cctatacctc cctccccgag   1140
ctaagtccca gggcatctgg gccttgcctg gagactgggc tagctctgta ggctcggaga   1200
gcctggggag ggtgccaacc ccacctctag tattttggga gatagggaaa gtgaaccgac   1260
ttccccttcc catacccctc agggtggttc cctaccagcc aggcttacta cttctagaag   1320
aaagcagagt gccagggagt gagattgcat ccctgggctt agaagtgacg gagagaagac   1380
ttgtttagta ttttgccatc agcacaagga aaaccaggag agagtctgcc tccaggactc   1440
tgagccttct gcctcatatg ttcagaaggt ggataggtct tcccactcca gcatggcttg   1500
aactcttagg ggtctgcagt gctccatctc cattggtggc cccagctcag taactatacc   1560
tggtacattt cctgtgtgca atcagtacct tgaaggcaga acattctgaa taaagttgga   1620
aaaagaacag ctttgctttg caaagattga tgacagactg gttcctcaga ggcctaggct   1680
```

acccgt cacc cct t t t t cca gagcgagggc ct ggaat gaa ggcagt t t at cct ct gt ccc 1740
t ggagcct gg ggt t t gct t t ggct cct t ga ggt ggaagag act aagaggg cagct gccca 1800
gagcagct gt gt gt acct gg ct cct ct cag gct t cct gat ccct t ccgt t gcact gcgcc 1860
t t at ccct ca gccagccaga cagcct ccct gct cct gacc agcagat acg t t t cggagt g 1920
gt t ggt gt gg t t t t t gt gat gagggcagca cgt ggt ggcc aaggt ggcaa gct gagt ct c 1980
acaggct cac t ccct cgt t g gt t ccct gt g ggaat ggt ag gccaggccca gt aagccat g 2040
ccccaacacg t cct ct cct c cggaggaagg gccagct gcc agct gagt ca gcagct agt c 2100
cat agcacag cct t at aact gt aaagccag gcat t gccca t gagcagagc t ggaaccaga 2160
gct t cagt ca gt aagaggga ggat t acct t caggagaagg caaggaagaa aact ggct gc 2220
t at ct t t at a gt t ccact gc cct aaccaag t gt ccacat t ct aaat gt gt agt gt ccat c 2280
cct t at gt aa t agt ggt t t c ccgcccaaag t gagact t t c ct t t t aat t g gagaagggt a 2340
t agaggt agt ccaggt ggga acgccagaag t gct gat t gc ccagccat t g ggaccacct g 2400
t t ct t gcccc act accct ct agt gggaggc caaagt aaag gct ggct ggt gggt gt ct gt 2460
ggat t gagga t gt ggcaggg act ggt cct c ccacct ccct ct ggccaaag at gggct t t g 2520
cccgct gt gt gcct gt cacc acccaccagc agt cat gccc t gggct t ccc aaat ggagag 2580
gt agcaggca acgt t t t t aa aaagaaagaa aacaggaaac t gt at t gt gt cgggggaggc 2640
gggagggaga t gaggaaacg gt t t ggat t t t gt gt gt ggg agggt at t t t t t ggggggt ag 2700
t t gt ct gt aa ct t t cct aag t gct t t t t t t cct t t t ct t t t t t aaagt aa gt t gcaggct 2760
t t ggct t gga aaaccccagg gggat ggggg gcagaaacct gaggct gct g cccct t t at c 2820
t gcct t cacg gt act gt ccc ct t cccccag ct cct ccct g accccat ggg ccaggcct ca 2880
gacct t ccag ct aaccgct t cccat gagcc act act ct ga t gt cagcct a t aaccaaagg 2940
agct gggggg t ccaggcct g gt gaccaacc t t t ct cagcc cact caat ca gggt gct ccc 3000
cacct gcagg caggaggcaa caccct at ct gct accat ca gcccct t cca gagcccat ct 3060
gccccgccca gccct gccct gcccagccat accct gct ct gccccat ct g ggggt gccct 3120
gct cagggat gggct ggcag ggct gt accc agcct ccct g gt aagcagag act caagaaa 3180
cct ct ggggt cct gt t t t ct ggt cgt gt ga t cccaggggt gcacat gggc ccct t gggt g 3240
t ct gaacaga agggcat ggg agggagggct gcacccct gc agt ct t act c t gct ggt gt a 3300
gcgggcagct gcccact ccc accccaccct gcaccgcggg ct cct gagt c ggcagat t aa 3360
gcat t t t at a aat t gt at t t t aaat acat g t t t t aaact t g 3401

<210> 31
<211> 5470
<212> DNA
<213> Homo sapi ens

<400> 31

gat t gccccc t gt gggt cac t t t ct cagt c at t t t gagct cagcct aat c aaagact gag 60
gt t at gaagt cgat cct aga t ggcct t gca gat accacct t ccgcaccat caccact gac 120
ct cct gt acg t gggct caaa t gacat t cag t acgaagaca t caaaggt ga cat ggcat cc 180
aaat t agggt act t cccaca gaaat t ccct t t aact t cct t t aggggaag t ccct t ccaa 240
gagaagat ga ct gcgggaga caaccccag ct agt cccag cagaccaggt gaacat t aca 300
gaat t t t aca acaagt ct ct ct cgt cct t c aaggagaat g aggagaacat ccagt gt ggg 360
gagaact t ca t ggacat aga gt gt t t cat g gt cct gaacc ccagccagca gct ggccat t 420
gcagt cct gt ccct cacgct gggcacct t c acggt cct gg agaacct cct ggt gct gt gc 480
gt cat cct cc act cccgcag cct ccgct gc aggcct t cct accact t cat cggcagcct g 540
gcggt ggcag acct cct ggg gagt gt cat t t t t gt ct aca gct t cat t ga ct t ccacgt g 600
t t ccaccgca aagat agccg caacgt gt t t ct gt t caaac t gggt ggggt cacggcct cc 660
t t cact gcct ccgt gggcag cct gt t cct c acagccat cg acaggt acat at ccat t cac 720
aggcccct gg cct at aagag gat t gt cacc aggcccaagg ccgt ggt ggc gt t t t gcct g 780
at gt ggacca t agccat t gt gat cgccgt g ct gcct ct cc t gggct ggaa ct gcgagaaa 840
ct gcaat ct g t t t gct caga cat t t t ccca cacat t gat g aaacct acct gat gt t ct gg 900
at cggggt ca ccagcgt act gct t ct gt t c at cgt gt at g cgt acat gt a t at t ct ct gg 960
aaggct caca gccacgccgt ccgcat gat t cagcgt ggca cccagaagag cat cat cat c 1020
cacacgt ct g aggat gggaa ggt acaggt g acccggccag accaagcccg cat ggacat t 1080
aggt t agcca agaccct ggt cct gat cct g gt ggt gt t ga t cat ct gct g gggccct ct g 1140
ct t gcaat ca t ggt gt at ga t gt ct t t ggg aagat gaaca agct cat t aa gacggt gt t t 1200
gcat t ct gca gt at gct ct g cct gct gaac t ccaccgt ga accccat cat ct at gct ct g 1260
aggagt aagg acct gcgaca cgct t t ccgg agcat gt t t c cct ct t gt ga aggcact gcg 1320
cagcct ct gg at aacagcat gggggact cg gact gcct gc acaaacacgc aaacaat gca 1380
gccagt gt t c acagggccgc agaaagct gc at caagagca cggt caagat t gccaaggt a 1440
accat gt ct g t gt ccacaga cacgt ct gcc gaggct ct gt gagcct gat g cct ccct ggc 1500
agcacaggaa aagaat t t t t t t t t t t aagc t caaaat ct a gaagagt ct a t t gt ct cct t 1560
ggt t at at t t t t t t aact t t accat gct ca at gaaaaggt gat t gt cacc at gat cact t 1620
at cagt t t gc t aat gt t t cc at agt t t agg t act caaact ccat t ct cca ggggt t t aca 1680
gt gaagaaag cct gt t gt t t aagt gact ga acgat cct t c aaagt ct caa t gaaat agga 1740
gggaaacct t t ggct acaca at t ggaagt c t aagaaccca t ggaaaaat g ccat caaat g 1800
aat aat gcct t t gt aaccac aact t t cact at aat gt gaa at gt aact gt ccgt agt at c 1860
agagat gt cc at t t t t acaa gt t at agt ac t agagat at t t t gt aaaat g t at t at gt cc 1920
t gt gagat gt gt at cagt gt t t at gt gct a t t aat at t t g t t t agt t cag caaaact gaa 1980

aggtagactt ttatgagaac aatggacaag cagtggatac gtgtcaatgt gtgcactttt 2040
tttctatatt attgcccatg atataacttt agaaataaac cttaatattt cttcaaatat 2100
ctctatttaa ttttgacact gaaataaccg taaaggttta tttttctgtt acctcaacaa 2160
gaagaatttg aagacttcaa aatattgagc agaattcatt catacttaaa aatttattag 2220
ccctgcattt tcataggaag acacattatc ttctggacta tagctgttct aatggattat 2280
aatcagaatg gaagagagaa agcatattga ctttttttga gcgacatctc tgactttctt 2340
tagtctttag ctattactgg atctcttaag acagcatgtg ttaatcttaa tgtatatcgt 2400
tatcactgtg cagttgctgt ttacttgaat agtattgtgt tcctatattc caggtttaag 2460
tagatttcat gcctgggtgg ccaaacaaca gtcttcattt tttttaattg aaaagaagta 2520
gtgtctggat cagtaaaatt atactgtgtg tgagtgtgaa tataaatgtg tgtatgtgtg 2580
tttctgtcct gtaactgtta cagtaatgtc ataaagtgag aaaactgtga ccaagtataa 2640
acttttacca cttgctgcac tcttgcacat ggattcagtt tctaaaattg agttcttcct 2700
gtaatcttgt tgataaaaat actgactcca accattcaaa aatttcaccc catccctcct 2760
taagagattg gatcaagtat tactaaattg acctttaggt attacacaag accagtgctt 2820
agcaaaaaat aatgacaggc atccaaggaa gggatgtatt tgtagtgtta ttgccaggaa 2880
aggagagtac tttggtttct gagcaccgaa tattgagcaa tatgtcagtc actaaaagga 2940
agacagttct acagaaaaac aatggtaaca tttttcaata gcgtgtgtag atagtatgca 3000
ctatatacat cacgttaaag taggactatc acacccagcc catgtggcta aaaaagctga 3060
atcagacagt ggatgagaca cacaacggca gtgaagaacc gatacacttg gcattgacgt 3120
ctagctatgc tgtatctgtg ctttgcccac atgcccttgg tgacagctga gcacccagct 3180
ctgtcttggt aggtttgggc taaggaacaa atctctcctt tgctcgtggt tagcaagata 3240
cactcaagca tgaagataaa cacagctgct ttcttcttac accccggtct catgctcctt 3300
aatggcgcca tgggtgcttg ttgggccttt ttccagtaag gaatgatatt gctgaagaat 3360
ctacttaacc ctgacaaatt ttaatttataa tctcttctta tacagataaa acatgactcc 3420
tacaaggccc caaggtttac atagtctgaa gtgaagtaca gagctggcat ctatctggtg 3480
atttctagct ctcgagatac ccaagcagcc tgatggggca gttccccttc ttacggttca 3540
cgctctaagg caggatgtgg cttatgagat actttgcatt gtctgtctgc acaccttgaa 3600
tctgcctgct ggctccctta ctttacctct ctgtcatgtg cagatgaagg ctcagggtgc 3660
tagaggatta gtaagatctc tttctaaaga caggagagat tatttacaag aagaactcac 3720
cagggtttag tttgcattta agaattgcca gtcttttgtc ctgcatcatc ttgaacatta 3780
atccacatgt ttcagagctc accaggcagt accaatgctc ttttcacagc tatgaagagc 3840
tagagaaatt cttgttatgg tagaaaaatt tcacgattca tttttgaaac tgcatttgtg 3900
cgtatgcagt gtagatttta tagtgtgttg tgctttcaag atctaaatca tatataataa 3960
attaagggac aatggggctg acagcactaa acttggtgct tattgatatt ctaagaaata 4020

t ct gt gaaat at cat cacgt at gt t at aca acct t cat t t aaaaaggt t t aaaact agt t 4080
agat t cact t t gacact t t t cat at cat t t ct t aacccaa gt gacgaaaa cat t gt cccc 4140
aat gaat at a ct cat t agaa t t accat t t g t t aat at cac t cat t aat t a accccat aat 4200
t agat ccat t aat t t aaat g at t t aaat t t aagt aagt t t t at aaggt ct gacat cagag 4260
gt at ct t act t t cct ct gag gat gat gt ac t t gccct gac cat gcat t t t accat cacac 4320
at gt t cagaa agggccaaat t cccaacct g ct cat t t t t t t t t t at caga gt cat gat ga 4380
at cagt cct a gaat gt t t ca t t t gcacaag t agggct gcc t ccaagagga acct ct gat t 4440
t at t t t gt at gaaat at at g t gaaaggat a t gaat ct gag agat gct gt a gacat ct gt c 4500
ct acact t ga gat gat t t cc aagcct ct ct ggcact t t ga gt t aagt ct a t ct ggt at t a 4560
aat gccaagg acct t t t gct gcct aaat cc act ct gcagg aaat aggccc aaccaccaga 4620
t gagaat t ag gccct ggat g agt agcgct a t agt t act gt cct gt t gat t aat t t ct gcc 4680
at t t cat gt c cat aaaagag accacccat a t cat gcacac aat t agat t t ct cacact ct 4740
aact gt at at t t gt at gat a t t t t aaaat c t cct aaat gc t gggcaat gg ct at t aacaa 4800
t t aat t gt ct t gcact ggcc t t ct gat gaa at gt t aacaa t gcct at t gt aat at agaaa 4860
aaaacat t ct at ct act gat t t gggct gaa t gt at gt aaa t aggt t t ct a aaaagt caga 4920
t gt t t gagca gt ggcct aca aat cagt aat t t t cggat gg gagagt t t ct t t acat t gcc 4980
gt ggcat ct t aaaagct at c t t cat gt aaa t t gact gt ac t aggcct act ggggat caga 5040
gt t cccaaga aaggaaacct t t t ct t gt at ct ggat t caa at t t at t t cc aat gt t t caa 5100
gcgggaaaca t gact ct t t a t t gt ct gt aa at ct aacat t at t act t t t c ct ct t agaag 5160
aat at t gt at t gt t agat gt t t gt t gagct ggt aacat cg t t gcaaccac t gcaat at ct 5220
t cgt t agt aa t ct gt at aat act t t gt at a caagt act gg t aagat t gt t at t aaat gt a 5280
gct t cagt ca t t aaat t act at agcaaagt agt act t ct t ct gt aat at t t acaat gt at 5340
t aagcccaca gt at at t t t a t t t caat gt a at t aaact gt t aact t at t c aaagagaaaa 5400
cat ct cat ca t gt ct at t gt ccaaagt t ac ct ggaat caa at aaaaat t c t agat t acca 5460
t gaagaacat 5470

<210> 32
<211> 1517
<212> DNA
<213> Homo sapi ens

<400> 32

gcggcgcgga gggcgcgggc ccgggagcca gggagcgagc ggggcgcccg gcagcgcgga 60
gt cagcgccg cgggggccgc acccgact cg cgcct ggaca ct cgcggggc gccgacct gg 120
caggggggcca aaccagt gct cct gccacct ct ct ggct gc cccct agagc ct gcccat cc 180

cagcctgacc aatgtccaca gccagggagc agccaatctt cagcacacgg gcgcacgtgt 240
tccaaattga cccagccacc aagcgaaact ggatcccagc gggcaagcac gcactcactg 300
tctcctattt ctacgatgcc acccgcaatg tgtaccgcat catcagcatc ggaggcgcca 360
aggccatcat caacagcact gtcactccca acatgacctt caccaaaact tcccagaagt 420
tcgggcagtg ggccgacagt cgcgccaaca cagtctacgg cttgggcttt gcctctgaac 480
agcatctgac acagtttgcc gagaagttcc aggaagtgaa ggaagcagcc aggctggcca 540
gggagaaatc tcaggatggc ggggagctca ccagtccagc cctggggctc gcctcccacc 600
aggtgccccc gagccctctc gtcagtgcca acggccccgg cgaggaaaaa ctgttccgca 660
gccagagcgc tgatgccccc ggccccacag agcgcgagcg gctaaagaag atgttgtctg 720
agggctccgt gggcgaggta cagtgggagg ccgagttttt cgcactgcag gacagcaaca 780
acaagctggc aggcgccctg cgagaggcca acgccgccgc agcccagtgg aggcagcagc 840
tggaggctca gcgtgcagag gccgagcggc tgcggcagcg ggtggctgag ctggaggctc 900
aggcagcttc agaggtgacc cccaccggtg agaaggaggg gctgggccag ggccagtcgc 960
tggaacagct ggaagctctg gtgcaaacca aggaccagga gattcagacc ctgaagagtc 1020
agactggggg gccccgcgag gccctggagg ctgccgagcg tgaggagact cagcagaagg 1080
tgcaggacct ggagacccgc aatgcggagt tggagcacca gctgcgggcg atggagcgca 1140
gcctggagga ggcacgggca gagcgggagc gggcgcgggc tgaggtgggc cgggcagcgc 1200
agctgctgga cgtcaggctg tttgagctga gtgagctgcg tgagggcctg gcccgcctgg 1260
ctgaggctgc gccctgagcc ggggctggtt ttctatgaac gattccggcc tgggatgcgg 1320
gccaggctgc aggcggcata gttgggccca ttcgtcctgg aaagggactg gggggtccca 1380
acttagccct gggtgggccg ggccgggctg ggctggggtg ggccccagtc ggctctggtt 1440
gttggcagct ttggggctgt ttttgagctt ctcattgtgt agaatttcta gatcccccga 1500
ttacatttct aagcgtg 1517

<210> 33
<211> 1228
<212> DNA
<213> Homo sapiens

<400> 33

agagatgggg acggaggcca cagagcaggt ttcctggggc cattactctg gggatgaaga 60
ggacgcatac tcggctgagc cactgccgga gctttgctac aaggccgatg tccaggcctt 120
cagccgggcc ttccaaccca gtgtctccct gaccgtggct gcgctgggtc tggccggcaa 180
tggcctggtc ctggccaccc acctggcagc ccgacgcgca gcgcgctcgc ccacctctgc 240
ccacctgctc cagctggccc tggccgacct cttgctggcc ctgactctgc ccttcgcggc 300

agcaggggct ct t cagggct ggagt ct ggg aagt gccacc t gccgcacca t ct ct ggcct 360
ct act cggcc t cct t ccacg ccggct t cct ct t cct ggcc t gt at cagcg ccgaccgct a 420
cgt ggccat c gcgcgagcgc t cccagccgg gccgcggccc t ccact cccg gccgcgcaca 480
ct t ggt ct cc gt cat cgt gt ggct gct gt c act gct cct g gcgct gcct g cgct gct ct t 540
cagccaggat gggcagcggg aaggccaacg acgct gt cgc ct cat ct t cc ccgagggcct 600
cacgcagacg gt gaaggggg cgagcgccgt ggcgcaggt g gccct gggct t cgcgct gcc 660
gct gggcgt c at ggt agcct gct acgcgct t ct gggccgc acgct gct gg ccgccagggg 720
gcccgagcgc cggcgt gcgc t gcgcgt cgt ggt ggct ct g gt ggcggcct t cgt ggt gct 780
gcagct gccc t acagcct cg ccct gct gct ggat act gcc gat ct act gg ct gcgcgcga 840
gcggagct gc cct gccagca aacgcaagga t gt cgcact g ct ggt gacca gcggct t ggc 900
cct cgcccgc t gt ggcct ca at cccgt t ct ct acgcct t c ct gggcct gc gct t ccgcca 960
ggacct gcgg aggct gct ac ggggt gggag ct cgccct ca gggcct caac cccgccgcgg 1020
ct gcccccgc cggccccgcc t t t ct t cct g ct cagct ccc acggagaccc acagt ct ct c 1080
ct gggacaac t agggct gcg aat ct agagg agggggcagg ct gagggt cg t gggaaaggg 1140
gagt aggt gg gggaacact g agaaagaggc agggacct aa agggact acc t ct gt gcct t 1200
gccacat t aa at t gat aaca t ggaaat g 1228

<210> 34
<211> 2057
<212> DNA
<213> Homo sapi ens

<400> 34

aaaaaaaaaa acacaggct t gat gct cgct cccgt t t ct a caggaggt t t t gacggct gg 60
at gct gcagg gcat cccct c gcct ggagga ggt t cact t g t ggccaggt t cccagcacac 120
ccact t gct c act ggggccc t ggggacacc gccacact cg agcccat cct acggcct caa 180
t cct t accag t ccccaggcc at ggact gcc acact gt agg t ct t caat ca aggt t t agt g 240
gagt t t cgaa t gt acacaca ccct gaaaga cagcgaggcc acct cagaaa acaacgcgcg 300
cccagct ggc t cccct ccga cagggcccac ggct act ggg t cggcct cct ccggaccct g 360
agcacagcct ggt at gcgt g cgggct cgga at acggat ct gagcagaaga aat gcgagt g 420
gagagcggt t ct gcgcagga aagaggaat t t t gt t ggaaa gcct gt cgac gt t gct agaa 480
aagaccacag cat ct cacga ggggcgt gcg ccgggaaacc gggagt t gac ggacct cct g 540
ccccccgagg t ct gcagt ct cct gaaccca gcagccat ct acgccaacaa cgagat cagc 600
ct gcgt gacg t t gaggt ct a cggct t t gac t acgact aca ccct ggccca gt at gcagac 660
gcact gcacc ccgagat ct t cagt accgcc cgt gacat cc t gat cgagca ct acaagt ac 720

ccagaaggga ttcggaagta tgactacaac cccagctttg ccatccgtgg cctccactat 780
gacattcaga agagccttct gatgaagatt gacgccttcc actacgtgca gctggggaca 840
gcctacaggg gcctccagcc tgtgccagac gaggaggtga ttgagctgta tgggggtacc 900
cagcacatcc cactatacca gatgagtggc ttctatggca agggtccctc cattaagcag 960
ttcatggaca tcttctcgct accggagatg gctctgctgt cctgtgtggt ggactacttt 1020
ctgggccaca gcctggagtt tgaccaagca catctctaca aggacgtgac ggacgccatc 1080
cgagacgtgc atgtgaaggg cctcatgtac cagtggatcg agcaggacat ggagaagtac 1140
atcctgagag gggatgagac gtttgctgtc ctgagccgcc tggtggccca tgggaaacag 1200
ctgttcctca tcaccaacag tcctttcagc ttcgtagaca aggggatgcg gcacatggtg 1260
ggtcccgatt ggcgccagct cttcgatgtg gtcattgtcc aggcagacaa gcccagcttc 1320
ttcactgacc ggcgcaagcc tttcagaaaa ctcgatgaga agggctcact tcagtgggac 1380
cggatcaccc gcttggaaaa gggcaagatc tatcggcagg gaaacctgtt tgacttctta 1440
cgcttgacgg aatggcgtgg cccccgcgtg ctctacttcg gggaccacct ctatagtgat 1500
ctggcggatc tcatgctgcg gcacggctgg cgcacaggcg ccatcatccc cgagctggag 1560
cgtgagatcc gcatcatcaa cacggagcag tacatgcact cgctgacgtg gcagcaggcg 1620
ctcacggggc tgctggagcg catgcagacc tatcaggacg cggagtcgag gcaggtgctg 1680
gctgcctgga tgaaagagcg gcaggagctg aggtgcatca ccaaggccct gttcaatgcg 1740
cagttcggca gcatcttccg caccttccac aaccccacct acttctcaag gcgcctcgtg 1800
cgctttctctg acctctacat ggcctccctc agctgcctgc tcaactaccg cgtggacttc 1860
accttctacc cacgccgtac gccgctgcag cacgaggcac ccctctggat ggaccagctc 1920
tgcaccggct gcatgaagac cccctttcctt ggtgacatgg cccacatccg ctgagggcac 1980
ctttattgtc tgggacaggc cctcagcccc tcctgcccca tccacccaga caagcaataa 2040
aagtggtctc ctccctg 2057

<210> 35
<211> 2413
<212> DNA
<213> Homo sapiens

<400> 35

attcatttcc cccagtgacc ttgacaagtc agaagcttga aagcagggaa atccggatgt 60
ctcggttatg aagtggagca gtgagtgtga gcctcaacat agttccagaa ctctccatcc 120
ggactagtta ttgagcatct gcctctcata tcaccagtgg ccatctgagg tgtttccctg 180
gctctgaagg ggtaggcacg atggccaggt gcttcagcct ggtgttgctt ctcacttcca 240
tctggaccac gaggctcctg gtccaaggct ctttgcgtgc agaagagctt tccatccagg 300

tgtcatgcag aattatgggg atcacccttg tgagcaaaaa ggcgaaccag cagctgaatt 360
tcacagaagc taaggaggcc tgtaggctgc tgggactaag tttggccggc aaggaccaag 420
ttgaaacagc cttgaaagct agctttgaaa cttgcagcta tggctgggtt ggagatggat 480
tcgtggtcat ctctaggatt agcccaaacc ccaagtgtgg gaaaaatggg gtgggtgtcc 540
tgatttggaa ggttccagtg agccgacagt ttgcagccta ttgttacaac tcatctgata 600
cttggactaa ctcgtgcatt ccagaaatta tcaccaccaa agatcccata ttcaacactc 660
aaactgcaac acaaacaaca gaatttattg tcagtgacag tacctactcg gtggcatccc 720
cttactctac aatacctgcc cctactacta ctcctcctgc tccagcttcc acttctattc 780
cacggagaaa aaaattgatt tgtgtcacag aagtttttat ggaaactagc accatgtcta 840
cagaaactga accatttgtt gaaaataaag cagcattcaa gaatgaagct gctgggtttg 900
gaggtgtccc cacggctctg ctagtgcttg ctctcctctt ctttggtgct gcagctggtc 960
ttggattttg ctatgtcaaa aggtatgtga aggccttccc ttttacaaac aagaatcagc 1020
agaaggaaat gatcgaaacc aaagtagtaa aggaggagaa ggccaatgat agcaacccta 1080
atgaggaatc aaagaaaact gataaaaacc cagaagagtc caagagtcca agcaaaacta 1140
ccgtgcgatg cctggaagct gaagtttaga tgagacagaa atgaggagac acacctgagg 1200
ctggtttctt tcatgctcct taccctgccc cagctgggga aatcaaaagg gccaaagaac 1260
caaagaagaa agtccacccc tggttcctaa ctggaatcag ctcaggactg ccattggact 1320
atggagtgca ccaaagagaa tgcccttctc cttattgtaa ccctgtctgg atcctatcct 1380
cctacctcca aagcttccca cggcctttct agcctggcta tgtcctaata atatcccact 1440
gggagaaagg agttttgcaa agtgcaagga cctaaaacat ctcatcagta tccagtggta 1500
aaaaggcctc ctggctgtct gaggctaggt gggttgaaag ccaaggagtc actgagacca 1560
aggctttctc tactgattcc gcagctcaga ccctttcttc agctctgaaa gagaaacacg 1620
tatcccacct gacatgtcct tctgagcccg gtaagagcaa aagaatggca gaaaagttta 1680
gcccctgaaa gccatggaga ttctcataac ttgagaccta atctctgtaa agctaaaata 1740
aagaaataga acaaggctga ggatacgaca gtacactgtc agcagggact gtaaacacag 1800
acagggtcaa agtgttttct ctgaacacat tgagttggaa tcactgttta gaacacacac 1860
acttactttt tctggtctct accactgctg atattttctc taggaaatat acttttacaa 1920
gtaacaaaaa taaaaactct tataaatttc tatttttatc tgagttacag aaatgattac 1980
taaggaagat tactcagtaa tttgtttaaa aagtaataaa attcaacaaa catttgctga 2040
atagctacta tatgtcaagt gctgtgcaag gtattacact ctgtaattga atattattcc 2100
tcaaaaaatt gcacatagta gaacgctatc tgggaagcta tttttttcag ttttgatatt 2160
tctagcttat ctacttccaa actaattttt atttttgctg agactaatct tattcatttt 2220
ctctaatatg gcaaccatta taaccttaat ttattattaa catacctaag aagtacattg 2280
ttacctctat ataccaaagc acattttaaa agtgccatta acaaatgtat cactagccct 2340

cct t t t t cca acaagaaggg act gagagat gcagaaat at t t gt gacaaa aaat t aaagc 2400

at t t agaaaa ct t 2413

<210> 36
<211> 4977
<212> DNA
<213> Homo sapi ens

<400> 36

gggaggggcg ggat t t cct g cgggaggcgc cgagccggcc t t ggaaaagg agaaggagaa 60

aggggggcag gggggagt gg gagccaccgt cct gagt gcc agggaccgag cagggccagc 120

cggaccccgg cgggcggccg ggagagagcc ct gagaccag t gcaccgct g ct gcct gagg 180

cccaagggaa t gct ggct gc t ct ct gggt g ggcact ggt g t t ccacagcc aagccgggaa 240

gat t ggt gt g cccagt ccca gcccat cct g gagct gt cgg aagccacaag ct gat at cca 300

t ccagat t t c cat gt agcct t cacccct c aaagat t agc t t t cct t cgt gt ggt ggcag 360

t t cacggagc acct t t ggga gt at ccct ca gt acct gacg aggagcccac t gcct gggac 420

accagact t g ct cccccgga accgggagcc ct ccaagggg ct gt ggcat g gcgccct gac 480

gccccagct t ggacccgt t g cct gcct t t g ccct ggact c ct gagt gt ag agct cagccc 540

aggagccagt at gggt cgt g aacaggacct gat cct cgcc gt caagaat g gagat gt gac 600

cggt gt gcag aaact ggt gg cgaaggt caa ggccacaaag acaaagct cc t gggct ccac 660

aaagaggct c aacgt gaact accaggat gc t gat ggat t c t ct gccct cc accacgct gc 720

t t t gggggc agcct ggagc t cat agcct t gct gct agag gct caggcca ct gt t gacat 780

caaggacagc aat ggcat gc gcccgct gca ct acgcagcc t ggcagggcc ggct ggagcc 840

t gt gaggct g ct gct gcgcg cct ct gcggc t gt caat gcc gcct cgct gg acggacagat 900

ccccct gcac ct ggct gcac agt at ggaca t t at gaggt g t cagaaat gc t cct ccagca 960

t cagt ccaac ccat gcct gg t caacaaggc caagaagacg cccct ggacc t ggcct gt ga 1020

at t t ggccga ct caaggt gg cccagct gct t ct gaacagc cact t at gt g t ggcact gct 1080

ggagggt gag gccaaagacc cgt gt gaccc caact acacc acgcccct gc act t ggct gc 1140

caagaat ggc cacagagaag t cat caggca gct cct gaga gct gggat cg agat caaccg 1200

ccagaccaag acgggt acgg cgct ccacga ggccgcact g t at ggcaaga ccgaggt ggt 1260

gcggct gct t ct ggagggag gt gt ggacgt gaacat ccgg aat acgt at a accagacggc 1320

gct ggacat a gt gaat cagt t caccacct c ccaggccagc cgggaaat ca agcagct act 1380

gcgggaggcc t cagggat cc t gaaggt ccg agcgct caag gat t t ct gga acct ccacga 1440

t cccact gct ct caat gt cc gggcagggga t gt cat cacg gt gct agaac agcat cccga 1500

cggccgct gg aagggccaca t ccacgagag ccagaggggc acagaccgca t aggct act t 1560

cccccccgggc at t gt cgagg t ggt cagcaa gcgggt gggc at ccct gcag cccgcct ccc 1620
ct ccgcaccc acccccct gc gcccaggct t ct cccggaca ccgcagcct c ct gccgaaga 1680
acccccgcac cct ct t acct acagccagct t cct cgggt g ggcct cagcc cagacagccc 1740
agcaggt gac aggaat agt g t gggcagt ga gggcagcgt g ggcagcat cc gcagt gccgg 1800
cagcgggcag agct ct gagg gcact aat gg ccat ggccct ggcct cct ga t t gagaacgc 1860
ccagccact g ccct ct gct g gagaggacca ggt gct gcca ggact ccacc cgccgt ccct 1920
ggcagacaac ct gagccacc gccct ct ggc caact gccgc t ct ggggagc agat ct t cac 1980
ccaggacgt g cggccagaac agct gct gga ggggaaggac gcgcaggcca t t cat aact g 2040
gct aagcgag t t ccagct gg agggct acac t gcccact t t ct gcaggccg gct at gat gt 2100
gcct accat c agccgcat ga cacct gagga cct gacggcc at cggggt ga ccaagcct gg 2160
gcacaggaag aagat cgcct cagagat cgc t cagct cagc at cgccgagt ggct gcccag 2220
ct acat ccca acggacct gc t ggagt ggct gt gt gcact g gggct gccac agt accacaa 2280
gcagct ggt g agcagcggct acgact ccat ggggct ggt g gccgacct ca cct gggagga 2340
gct gcaggag at t ggggt ca acaagct cgg gcat cagaag aagct cat gc t gggggt gaa 2400
gcggct ggcg gagct t cggc ggggcct gct gcagggggag gccct cagcg aaggcgggcg 2460
ccggct ggcc aagggt ccgg agct gat ggc cat cgaggga ct ggagaacg gagaaggccc 2520
agct acagct ggcccacggc t cct cacct t ccagggcagc gaact aagcc cagagct aca 2580
ggcggccat g gcagggggt g gccct gaacc act cccccct c ccacct gccc gct ct cccag 2640
ccaggagagc at cggggcac gct cacgggg gt ct ggccac t cacaggaac agcct gcccc 2700
acagcccagc ggt ggagat c ccagcccccc ccaggagagg aacct cccag agggcacaga 2760
gcggcccccct aagct t t gt t ct t cact t cc t ggccaagga cccccaccct at gt t t t t at 2820
gt accccccag ggct caccct ct agcccggc cccagggcca cct cct ggcg caccct gggc 2880
ct t ct cct ac t t ggccgggc cccct gccac t cccccagac ccgcct cgac ct aagcgccg 2940
gt cccacagc ct aagccgcc ct ggccccac agaggggggat gct gaggggg aggccgaagg 3000
gccagt gggc agcaccct ag gcagt t at gc t accct t acc cggcggccag gacgcagt gc 3060
cct t gt ccgg accagt cct a gcgt gacccc aaccccagct cgggggact c ct cgcagcca 3120
gt cct t t gcc ct gcgggccc ggcgcaaagg cccccccgccc ccgccccccca agcgcct cag 3180
ct ccgt ct ct ggccccagcc cggagccacc t ccact agat gggagcccag ggcccaagga 3240
aggggccaca gggccccgaa ggcgaacact gagt gaacct gct ggcccct cagagccccc 3300
t ggcccacct gccccggct g ggcccgcgt c agacacggag gaggaggagc caggccct ga 3360
ggggacgccc ccat ct cggg gcagct ct gg ggaagggct g ccgt t t gcag aggaagggaa 3420
cct gaccat c aaacagcgcc cgaagcccgc t ggccccccg ccccgagaga cacccgt gcc 3480
ccccggcct c gat t t caacc t cacggaat c agacact gt t aagcggaggc ccaagt gccg 3540
ggagagagag ccact gcaga ccgcact gct ggcct t cgga gt ggccagt g ccacgcct gg 3600

ccccgct gcc ccact gcct t ccccaact cc t ggcgagt ct cct ccagct t ct agcct t cc 3660
ccagcccgag cccagcagcc t t ccagccca aggagt t cca accoccct t g ct cccagccc 3720
cgccat gcag cct ccagt gc cgccct gccc agggccaggt ct ggaaagct cagcagct ag 3780
t cggt ggaat ggggagacag aaccccc ggc cgcccct gct gccct cct ca aggt gcccgg 3840
agcaggaaca gcccccaagc ct gt gt cggt ggcct gcacc cagct ggcat t t t ct ggccc 3900
t aagct agcg ccccggct cg gcccccgccc agt gcct cct ccacggcct g agagcact gg 3960
gact gt gggc ccaggccagg cccagcagag act ggagcag accagct cgt ccct ggcagc 4020
t gcact gaga gccgcagaga agagcat t gg caccaaggag caagagggca cccccagcgc 4080
ct ccaccaag cacat t ct gg at gacat cag caccat gt t c gacgccct gg ct gaccagct 4140
ggacgccat g ct ggact gag ccct ccagca gt gcccact g t gacct gccg aagt ccact g 4200
cct t t gcccc agcacagaag aggcccct gc caccct aggg acgggccaag ggct ggt cag 4260
gct gaagt gc ccct cct agc agggcccct t cccact cagc ccgcggct gt gggcaccaca 4320
gct ct t gt gg ggcagcccac ct t agaacct gact agcgag ggacct ccgc t gcat ct cag 4380
caaagcccct cccagggt t t gat cgat t ga gcaggacagc cct gct cct g gacagggacc 4440
ct ggt aagag ct ct ct cct c agggaggaag t aggggt ggg gct t t ggggg t gct t t ct ct 4500
gt accccca gcccat gt cc caagt t gt gc caagggaat g cct ct t gcca cccaccat cc 4560
t gcccaggcc ct gagaggct ggaggaggcg t gt gt ct ggc cgggccccct ct ccgt aggt 4620
gt acagagga cat gt aaat a cacagcggt g gt gcccaggc agct cct gcc cccgcct t cc 4680
ct cact gagg cagggct agc aggggt ggga ggcagt gggg t t aacagat c ccacaagt cc 4740
t aact t t t ca at t gt aaat g t t at t t t ct a agcagagaga aat gcat at a t t t t aaat gg 4800
aat t t at t ct at caact gcc t gagaggaca caat ggggga ggggct t cgg accacagcag 4860
gagccccgac t gcccacct g agggcaggga gagcct gacc ccat t ggccc aggccct ggc 4920
t ct gt aacca t t aacct ct t cccccaact a acaccaat ga aaacaccat t ccacgt g 4977

<210> 37
<211> 6202
<212> DNA
<213> Homo sapiens

<400> 37

cgcagact gt gct ggagct g gt gct gaaaa agggggt t t g cagaggct gc cct ggggct g 60
gt gct gaaag aagagcccac agct gact t c at ggt gct ac aat aacct ca gaat ct act t 120
t t cact ct ca ggagaaccca cagt ct aat a t t t agacat g at ggcaaact gggcggaagc 180
aagacct ct c ct cat t ct t a t t gt t t t at t agggcaat t t gt ct caat aa aagcccagga 240
agaagacgag gat gaaggat at ggt gaaga aat agcct gc act cagaat g gccagat gt a 300

ct t aaacagg gacat t t gga aacct gcccc t t gt cagat c t gt gt ct gt g acaat ggagc 360
cat t ct ct gt gacaagat ag aat gccagga t gt gct ggac t gt gccgacc ct gt aacgcc 420
ccct ggggaa t gct gt cct g t ct gt t caca aacacct gga ggt ggcaat a caaat t t t gg 480
t agaggaaga aagggacaaa agggagaacc aggat t agt g cct gt t gt aa caggcat acg 540
t ggt cgt cca ggaccggcag gacct ccagg at cacaggga ccaagaggag agcgagggcc 600
aaaaggaaga cct ggccct c gt ggacct ca gggaat t gat ggagaaccag gt gt t cct gg 660
t caacct ggt gct ccaggac ct cct ggaca t ccgt cccac ccaggacccg at ggct t gag 720
caggccgt t t t cagct caaa t ggct gggt t ggat gaaaaa t ct ggact t g ggagt caagt 780
aggact aat g cct ggct ct g t gggt cct gt t ggcccaagg ggaccacagg gt t t acaagg 840
acagcaaggt ggt gcaggac ct acaggacc t cct ggt gaa cct ggt gat c ct ggaccaat 900
gggt ccgat t ggt t cacgt g gaccagaggg ccct cct ggt aaacct gggg aagat ggt ga 960
acct ggcaga aat ggaaat c ct ggt gaagt gggat t t gca ggat ct ccgg gagct cgt gg 1020
at t t cct ggg gct cct ggt c t t ccaggt ct gaagggt cac cgaggacaca aaggt ct t ga 1080
aggccct aaa ggt gaagt t g gagcacct gg t t ccaagggt gaagct ggcc ccact ggt cc 1140
aat gggt gcc at gggt cct c t gggt ccgag gggaat gcca ggagagagag ggagact t gg 1200
gccacagggt gct cct ggac aacgaggt gc acat ggt at g cct ggaaaac ct ggaccaat 1260
gggt cct ct t gggat accag gct ct t ct gg t t t t ccagga aat cct ggaa t gaagggaga 1320
agcaggt cct acaggggcgc gaggccct ga aggt cct cag gggcagagag gt gaaact gg 1380
gcccccaggt ccagt t ggct ct ccaggt ct t cct ggt gca at aggaact g at ggt act cc 1440
t ggt cccaaa ggcccaacgg gct ct ccggg t acct ct ggt cct cct ggct cagcagggcc 1500
t cct ggat ct ccaggacct c agggt agcac t ggt cct cag gggaat t cgg gcct t ccggg 1560
t gat ccaggt t t caaaggag aagct ggccc aaaaggggaa ccagggccac at ggt at t ca 1620
gggt ccgat a ggcccacccg gt gaagaagg caaaagaggt cccagaggt g acccaggaac 1680
act t ggt cct ccagggccag t gggagaaag gggt gct cct ggcaat cgt g gt t t t ccagg 1740
ct ct gat ggt t t acct gggc caaagggt gc t caaggagaa cggggt cct g t aggt t ct t c 1800
aggacccaaa ggaagccagg gggat ccagg acgt ccaggg gaacct gggc t t ccaggt gc 1860
t cggggt t t g acaggaaat c ct ggt gt t ca aggt cct gaa ggaaaact t g gacct t t ggg 1920
t gcgccaggg gaagat ggcc gt ccaggt cc t ccaggct cc at aggaat ca aagggcagcc 1980
cgggaccat g ggcct t ccag gccccaaagg t agcaat ggt gaccct ggga aacct ggaga 2040
agcaggaaat cct ggagt t c ct gggcaaag gggagct cct ggaaaagat g gt aaagt t gg 2100
t cct t at ggt cct cct gggc cgccgggt ct acgt ggt gaa agaggagaac aaggacct cc 2160
agggcccaca ggt t t t cagg ggcat cct gg t cct ccaggt cct cct ggag aaggt ggaaa 2220
accaggt gat caaggt gt t c ct ggaggt cc cggagcagt t ggcccgt t ag gacct agagg 2280
agaacgagga aat cct gggg aaagaggaga acct gggat a act ggact cc ct ggt gagaa 2340

```
gggaatggct ggaggacatg gtcctgatgg cccaaaaggc agtccaggtc catctgggac 2400
ccctggagat acaggcccac caggtcttca aggtatgccg ggagaaagag gaattgcagg 2460
aactcctggc cccaagggtg acagaggtgg cataggagaa aaaggtgctg aaggcacagc 2520
tggaaatgat ggtgcaggag gtcttccagg tcctttgggc cctccaggtc cggcaggcct 2580
actgggagaa aagggtgaac ctggtcctcg aggtttagtt ggtcctcctg gctcccgggg 2640
caatcctggt tctcgaggtg aaaatgggcc aactggagct gttggttttg ccggacccca 2700
ggggtctgac ggacagcctg gagtaaaagg tgaacctgga gagccaggac agaagggaga 2760
tgctggttct cctggaccac aaggtttagc aggatcccct ggccctcatg gtcctaatgg 2820
tgttcctgga ctaaaaggtg gtcgaggaac ccaaggtccg cctggtgcta caggatttcc 2880
tggttctgcg ggcagagttg gacctccagg ccctgctgga gctccaggac ctgcgggacc 2940
cctaggggaa cccgggaagg agggacctcc aggtcctcgt ggggaccctg gctctcatgg 3000
gcgtgtggga gtccgaggac cagctggccc ccctggtggc ccaggagaca aaggggaccc 3060
aggagaagat gggcaacctg gtccagatgg cccccctggt ccagctggaa cgaccgggca 3120
gagaggaatt gttggcatgc ctgggcaacg tggagagaga ggcatgcccg gcctaccagg 3180
cccagcggga acaccaggaa aagtaggacc aactggtgca acaggagata aaggtccacc 3240
tggacctgtg gggcccccag gctccaatgg tcctgtaggg gaacctggac cagaaggtcc 3300
agctggcaat gatggtaccc caggacggga tggtgctgtt ggagaacgtg gtgatcgtgg 3360
agaccctggg cctgcaggtc tgccaggctc tcagggtgcc cctggaactc ctggccctgt 3420
gggtgctcca ggagatgcag gacaaagagg agatccgggt tctcggggtc ctataggaca 3480
cctgggtcga gctggaaaac gtggattacc tggaccccaa ggacctcgtg gtgacaaagg 3540
tgatcatgga gaccgaggcg acagaggtca gaagggccac agaggcttta ctggtcttca 3600
gggtcttcct ggccctcctg gtccaaatgg tgaacaagga agtgctggaa tccctggacc 3660
atttggccca agaggtcctc caggcccagt tggtccttca ggtaaagaag gaaaccctgg 3720
gccacttggg ccattgggac ctccaggtgt acgaggcagt gtaggagaag caggacctga 3780
gggccctcct ggtgagcctg gcccacctgg ccctccgggt ccccctggcc accttacagc 3840
tgctcttggg gatatcatgg ggcactatga tgaaagcatg ccagatccac ttcctgagtt 3900
tactgaagat caggcggctc ctgatgacaa aaacaaaacg gacccagggg ttcatgctac 3960
cctgaagtca ctcagtagtc agattgaaac catgcgcagc cccgatggct cgaaaaagca 4020
cccagcccgc acgtgtgatg acctaaagct ttgccattcc gcaaagcaga gtggtgaata 4080
ctggattgat cctaaccaag gatctgttga agatgccatc aaagtttact gcaacatgga 4140
aacaggagaa acatgtattt cagcaaaccc atccagtgta ccacgtaaaa cctggtgggc 4200
cagtaaatct cctgacaata aacctgtttg gtatggtctt gatatgaaca gagggtctca 4260
gttcgcttat ggagaccacc aatcacctaa tacagccatt actcagatga cttttttgcg 4320
ccttttatca aaagaagcct cccagaacat cacttacatc tgtaaaaaca gtgtaggata 4380
```

cat ggacgat caagct aaga acct caaaaa agct gt ggt t ct caaagggg caaat gact t 4440
agat at caaa gcagagggaa at at t agat t ccggt at at c gt t ct t caag acact t gct c 4500
t aagcggaat ggaaat gt gg gcaagact gt ct t t gaat at agaacacaga at gt ggcacg 4560
ct t gcccat c at agat ct t g ct cct gt gga t gt t ggcggc acagaccagg aat t cggcgt 4620
t gaaat t ggg ccagt t t gt t t t gt gt aaag t aagccaaga cacat cgaca at gagcacca 4680
ccat caat ga ccaccgccat t cacaagaac t t t gact gt t t gaagt t gat cct gagact c 4740
t t gaagt aat ggct gat cct gcat cagcat t gt at at at g gt ct t aagt g cct ggcct cc 4800
t t at cct t ca gaat at t t at t t t act t aca at cct caagt t t t aat t gat t t t aaat at t 4860
t t t caat aca acagt t t agg t t t aagat ga ccaat gacaa t gaccacct t t gcagaaagt 4920
aaact gat t g aat aaat aaa t ct ccgt t t t ct t caat t t a t t t cagt gt a at gaaaaagt 4980
t gct t agt at t t at gaggaa at t ct t ct t c ct ggcaggt a gct t aaagag t ggggt at at 5040
agagccacaa cacat gt t t a t t t t gct t gg ct gcagt t ga aaaat agaaa t t agt gccct 5100
t t t gt gacct ct cat t ccaa gat t gt caat t aaaaat gag t t t aaaat gt t t aact t gt g 5160
at cgagacct acat gcat gt ct t gat at t g t gt aact at a at agagact c t t t aaggaga 5220
at ct t aaaaa aaaaaaacgt t t ct cact gt ct t aaat aga at t t t t aaat agt at at at t 5280
cagt ggcat t t t ggagaaca aagt gaat t t act t cgact t ct t aaat t t t t gt aaaagac 5340
t at aagt t t a gacat ct t t c t cat t caaat t t aaagat at ct t t ct cct c t t gat caat c 5400
t at caat at t gat agaagt c acact agt at at accat t t a at acat t t ac act t t ct t at 5460
t t aagaagat at t gaat gca aaat aat t ga cat at agaac t t t acaaaca t at gt ccaag 5520
gact ct aaat t gagact ct t ccacat gt ac aat ct cat ca t cct gaagcc t at aat gaag 5580
aaaaagat ct agaaact gag t t gt ggagct gact ct aat c aaat gt gat g at t ggaat t a 5640
gaccat t t gg cct t t gaact t t cat aggaa aaat gaccca acat t t ct t a gcat gagct a 5700
cct cat ct ct agaagct ggg at ggact t ac t at t ct t gt t t at at t t t ag at act gaaag 5760
gt gct at gct t ct gt t at t a t t ccaagact ggagat aggc agggct aaaa aggt at t at t 5820
at t t t t cct t t aat gat ggt gct aaaat t c t t cct at aaa at t cct t aaa aat aaagat g 5880
gt t t aat cac t accat t gt g aaaacat aac t gt t agact t cccgt t t ct g aaagaaagag 5940
cat cgt t cca at gct t gt t c act gt t cct c t gt cat act g t at ct ggaat gct t t gt aat 6000
act t gcat gc t t ct t agacc agaacat gt a ggt cccct t g t gt ct caat a ct t t t t t t t 6060
ct t aat t gca t t t gt t ggct ct at t t t aat t t t t t t ct t t t aaaat aaac agct gggacc 6120
at cccaaaag acaagccat g cat acaact t t ggt cat gt a t ct ct gcaaa gcat caaat t 6180
aaat gcacgc t t t t gt cat g t c 6202

<210> 38
<211> 3085
<212> DNA
<213> Homo sapi ens

<400> 38

gacggccgcc ggagccgcga ggccaact gt cgcct ggt t g ggcccggaaa t gggacgt cg 60
cgct t t ct ca gggagcgt ag aagcagccag ggcct ct cca agccgct gct gt gacagaaa 120
gt gagt gagc t gccggagga t gt ccaccgc cacgacagt c gcccccgcgg ggat cccggc 180
gaccccgggc cct gt gaacc cacccccccc ggaggt ct cc aaccccagca agcccggccg 240
caagaccaac cagct gcagt acat gcagaa t gt ggt ggt g aagacgct ct ggaaacacca 300
gt t cgcct gg ccct t ct acc agcccgt gga cgcaat caaa t t gaacct gc cggat t at ca 360
t aaaat aat t aaaaacccaa t ggat at ggg gact at t aag aagagact ag aaaat aat t a 420
t t at t ggagt gcaagcgaat gt at gcagga ct t caacacc at gt t t acaa at t gt t acat 480
t t at aacaag cccacagat g acat agt gct aat ggcccaa gct t t agaga aaat t t t t ct 540
acaaaaagt g gcccagat gc cccaagagga agt t gaat t a t t acccccct g ct ccaaaggg 600
caaaggt cgg aagccggct g cgggagccca gagcgcaggt acacagcaag t ggcggccgt 660
gt cct ct gt c t ccccagcga ccccct t t ca gagcgt gccc cccaccgt ct cccagacgcc 720
cgt cat cgct gccacccct g t accaaccat cact gcaaac gt cacgt cgg t cccagt ccc 780
cccagct gcc gccccacct c ct cct gccac acccat cgt c cccgt ggt cc ct cct acgcc 840
gcct gt cgt c aagaaaaagg gcgt gaagcg gaaagcagac acaaccact c ccacgacgt c 900
ggccat cact gccagccgga gt gagt cgcc cccgccgt t g t cagacccca agcaggccaa 960
agt ggt ggcc cggcgggaga gt ggt ggccg ccccat caag cct cccaaga aggacct gga 1020
ggacggcgag gt gccccagc acgcaggcaa gaagggcaag ct gt cggagc acct gcgct a 1080
ct gcgacagc at cct caggg agat gct at c caagaagcac gcggcct acg cct ggccct t 1140
ct acaagcca gt ggat gccg aggccct gga gct gcacgac t accacgaca t cat caagca 1200
cccgat ggac ct cagcaccg t gaaaaggaa gat ggat ggc cgagagt acc cagacgcaca 1260
gggct t t gct gct gat gt cc ggct gat gt t ct cgaat t gc t acaaat aca at cccccaga 1320
ccacgaggt t gt ggccat gg cccggaagct ccaggacgt g t t t gagat ga ggt t t gccaa 1380
gat gccagat gagcccgt gg aggcaccggc gct gcct gcc cccgcggccc ccat ggt gag 1440
caagggcgct gagagcagcc gt agcagt ga ggagagct ct t cggact cag gcagct cgga 1500
ct cggaggag gagcgggcca ccaggct ggc ggagct gcag gagcagct ga aggccgt gca 1560
cgagcagct g gccgccct gt ct caggcccc agt aaacaaa ccaaagaaga agaaggagaa 1620
gaaggagaag gagaagaaga agaaggacaa ggagaaggag aaggagaagc acaaagt gaa 1680
ggccgaggaa gagaagaagg ccaaggt ggc t ccgcct gcc aagcaggct c agcagaagaa 1740
ggct cct gcc aagaaggcca acagcacgac cacggccggc agacagct ga agaaaggcgg 1800
caagcaggca t ct gcct cct acgact caga ggaagaggag gagggcct gc ccat gagct a 1860

cgat gaaaag cgccagct t a gcct ggacat caaccggct g cccggggaga agct gggccg 1920
ggt agt gcac at cat ccaat ct cgggagcc ct cgct cagg gact ccaacc ccgacgagat 1980
agaaat t gac t t t gagact c t gaaacccac cact t t gcgg gaact ggaga gat at gt caa 2040
gt ct t gt t t a cagaaaaagc aaaggaaacc gt t ct cagca agcgggaaga aacaggcagc 2100
caagt cgaaa gaggagct ag ct caggaaaa gaagaaggag ct ggaaaagc gt ct gcagga 2160
t gt cagcggg cagct gagca gcagcaagaa gcccgcccgg aaagagaagc ccggct cagc 2220
accct caggg ggcccgt cca ggct cagcag cagcagct cc t ccgagt ct g ggagcagcag 2280
ct ccagcggg t ccagct ct g acagcagt ga ct cagaat ga act ggct t cg gacagaacag 2340
gacagat gga t gt cgcacac gccgagact c t gccgt accc ct ct gt ggt t cat at t act a 2400
ct t ct gt t cc at ggt gt gca ggt ct gct t c t t aat t cagt gt t at gat at ct t ccagt t t 2460
t t gct t t cat aggt cagaga t ct at ct t gt gt gt gcgt t a gact t gat ga gaaggt gt ga 2520
act ct gcaga aagt ct ct t c t t cat cact g aat t cagt ca ct t ggagat g acaact t caa 2580
at gct aaccc gat gacccca gaaaaccgt g t gagat t cgt accgaagaac ct t gt ggaat 2640
ccct t t gct t aggcccaacc t ggt cgat ag ct cgagaaag aat t t t t t cc aaggaaat gt 2700
ct cggat at g ggt act gt at t t gaaagct g t t agct t t gt caacacgcat t gt cct t gt c 2760
at t t gggccc cgagct ct ga ccct cgt gt c t gacgcggcc acct ct t t ct ggaggggct g 2820
aggacagat g t gcct gct t g t ggagaccag gct gggcct a agcgaagggt cat cgcagcc 2880
ccagcccgga gcgt ggagcc ct t gggggt ggt cgggt gg gat gt gcgt t ct ccgct cgt 2940
ggt gat gt ca ggagct cct c ggagggaaca gagcggct gt gt at gcagcc t gcaggt t t c 3000
cat acact ga agct t t t acc t caact t t aa aaaaaaaaaa gaagaaaaga t t aaaaaaaa 3060
aaaaaaaaaa ct cgaggcat ct at g 3085

<210> 39
<211> 3120
<212> DNA
<213> Homo sapi ens

<400> 39

gagcaagccg agt ggaggga ggcaaaggcc aggct gagga t cagggt ggc ccgggt ggca 60
gcggggaggc gct gcat gct ggaggct gt g ct gagt gccc ggt gcaggt g agccggt cct 120
gcggagt t gt gccgagt gcc t gct gcagt c t cat t t ccag t t cct ccat g acgt ggcaag 180
t gaagacagg aat gaaagga at gt aaagca gct t t t ct ct gaagagaaga agagagagag 240
acacagccaa gaccgaggct gggccaagat ggcgt ct gt g t t t cgaagcg aggagat gt g 300
t t t gt cacaa ct gt t t ct cc aggt ggaagc t gcat at t gc t gt gt ggct g agct cggaga 360
gct cggat t g gt t cagt t ca aagat t t aaa t at gaat gt g aacagct t t c aaaggaaat t 420

t gt gaat gaa gt cagaaggt gt gaat cact ggagagaat c ct ccgt t t t c t ggaagacga 480
gat gcaaaat gagat t gt ag t t cagt t gct cgagaaaagc ccact gaccc cgct cccacg 540
ggaaat gat t accct ggaga ct gt t ct aga aaaact ggaa ggagagt t ac aggaagccaa 600
ccagaaccag caggcct t ga aacaaagct t cct agaact g acagaact ga aat acct cct 660
gaagaaaacc caagact t ct t t gagacgga aaccaat t t a gct gat gat t t ct t t act ga 720
ggacact t ct ggcct cct gg agt t gaaagc agt gcct gca t at at gaccg gaaagt t ggg 780
gt t cat agcc ggt gt gat ca acagggagag gat ggct t cc t t t gagcggt t act gt ggcg 840
aat ct gccga ggaaacgt gt act t gaagt t cagt gagat g gacgcccct c t ggaggat cc 900
t gt gacgaaa gaagaaat t c agaagaacat at t cat cat a t t t t accaag gagagcagct 960
caggcagaaa at caagaaga t ct gt gat gg gt t t cgagcc act gt ct acc ct t gcccaga 1020
gcct gcggt g gagcgcagag agat gt t gga gagcgt caat gt gaggct gg aagat t t aat 1080
caccgt cat a acacaaacag agt ct caccg ccagcgcct g ct gcaggaag ccgct gccaa 1140
ct ggcact cc t ggct cat ca aggt gcagaa gat gaaagct gt ct accaca t cct gaacat 1200
gt gcaacat c gacgt caccc agcagt gt gt cat cgccgag at ct ggt t cc cggt ggcaga 1260
t gccacacgt at caagaggg cact ggagca aggcat ggaa ct aagt ggct cct ccat ggc 1320
ccccat cat g accacagt gc aat ct aaaac agcccct ccc acat t t aaca ggaccaat aa 1380
at t cacagct ggct t ccaga at at t gt t ga t gcct at ggt gt cggcagct accgggagat 1440
aaacccagcc ccct acacca t cat cact t t cccct t cct g t t cgct gt ga t gt t t ggaga 1500
ct gt ggt cat ggaaccgt ga t gct cct ggc t gcact t t gg at gat t ct ga at gagagacg 1560
ct t gct ct cc cagaagacag acaat gagat t t ggaacacc t t ct t ccacg ggcgct at ct 1620
gat cct act t at gggcat ct t ct ccat ct a cacgggt t t g at ct acaat g act gct t ct c 1680
caagt cct t g aacat ct t t g gct ct t ct t g gagt gt ccaa cccat gt t ca gaaacggcac 1740
at ggaat act cat gt aat gg aggaaagt ct at at ct gcag ct ggacccag ccat accagg 1800
agt gt at t t t ggaaat ccat acccgt t t gg gat t gat ccg at t t ggaact t ggct t caaa 1860
caaact caca t t t ct gaact cgt at aaaat gaagat gt cg gt gat cct gg gaat t gt cca 1920
gat ggt t t t t ggt gt cat cc t cagcct t t t caat cacat a t act t cagaa gaact ct caa 1980
cat cat t ct g caat t t at cc ct gagat gat t t t t at cct g t gt ct gt t t g gat acct ggt 2040
t t t cat gat c at t t t caaat ggt gct gct t t gacgt ccac gt at ct cagc acgcccccag 2100
cat cct cat c cact t cat ca acat gt t t ct gt t t aact ac agt gact ct t ccaacgcacc 2160
cct ct acaaa cat cagcaag aagt ccaaag t t t ct t t gt g gt t at ggct t t gat t t ct gt 2220
gccgt ggat g ct t ct gat t a agccgt t t at t ct t agagcc agt cat cgga aat cccagct 2280
gcaggcat cc aggat ccaag aagat gccac t gagaacat t gaaggt gat a gct ccagccc 2340
t t ct agccgt t ct ggccaga ggact t ct gc agat acccac ggggct ct gg acgaccat gg 2400
agaagagt t c aact t t ggag acgt ct t t gt ccaccaagcc at ccacacca t cgagt act g 2460

cct gggct gc at t t caaaca cagcct cct a cct gcggct c t gggccct ca gcct ggct ca 2520
t gcacaact g t ct gaagt gc t ct ggact at ggt gat gaac agcggcct t c agacgcgagg 2580
ct ggggagga at cgt cgggg t t t t t at t at t t t t gccgt a t t t gct gt cc t gacagt agc 2640
cat cct t ct g at cat ggagg gcct ct ct gc t t t cct gcac gccct gcgac t gcact gggt 2700
t gagt t ccag aacaagt t ct at gt cgggga t ggt t acaag t t t t ct ccat t ct cct t t aa 2760
acacat cct g gat ggcacag ccgaggagt a ggct gagggc t gcacct ccc acggt ggt ca 2820
ccat gccaat gaaggaagt t cagt ct t gt c t t t gat at ca gcccct gcaa ggcgct caat 2880
gggaaggt t g t t ct t ggct c acct gaagca t gaaact gt g t at t at t t gg acgt cagcct 2940
gt ggat t t ga t acgact t aa ccacgt caga ggaaggact t t ggcaagt ga t at t gt ct t c 3000
at gt ggggt a t t aat t ct ca aat aat aaag t aat t gacaa at gaggggag aat gct aaac 3060
agat gt ct t c t t gcaat at t t t aaat at t g t at t t gagaa aat aaacat c t gagt cat t c 3120

<210> 40
<211> 2538
<212> DNA
<213> Homo sapi ens

<400> 40

ccct cggggc t gcccct gct gcct gt gct g t t cgct ct t g gggggct t ct gct cct ct cc 60
aat gcct cct gt gt cggggg ggt cct ct gg cagcggagac t caggcgt ct t gct gaggca 120
ct caact t cc ccccacacct t cat cct gga aggt cggaag aggaccgagt caggaacgaa 180
t at gaggaga gccagt ggac aggagagcgg gacact caga gct ccacggt cagcacaaca 240
gaggcagagc cgt at t accg ct ccct gagg gact t cagcc cccagct gcc cccgacgcag 300
gaggaggt gt ct t at t cccg aggt t t caca ggt gaagat g aggat at ggc ct t ccct ggg 360
cact t gt at g at gaggt aga aagaacgt ac cccccgt ct g gagcct gggg acccct ct ac 420
gat gaagt gc agat gggacc ct gggacct c cact ggcct g aagacacat a t caggat cca 480
agaggaat ct at gaccaggt ggccggagac t t ggacact c t ggaacccga t t ct ct gccc 540
t t cgagct ga ggggacat ct ggt at ggggt t t caaccat g t t agccaggc t ggt ct caaa 600
ct cct ggcct caagt gat cc acccgcct ca gcct cccaaa gt gct gagat t acagagt ct 660
cact ct gt ag t ccaggt t gg agt gcagt gg cgt t at t t cg gct cact gca t cct ct gcct 720
cct ggt t caa gagat t ct ct t gcct cagcc t cccgcat ag ct gggat t ac agcacct t gg 780
gaggcagagg t gagcagat c acct cagggc act caggat t ccccagt gac caggt ct ggt 840
ccccccct cca ggggct ggca gt ccct gagc t t t gat ggcg gggcct t cca cct t aagggc 900
acaggagagc t gacacgggc ct t gct ggt t ct ccggct gt gt gcct ggcc cccact cgt c 960
act cacgggc t gt t gct cca ggcct ggt ct cggcgact cc t gggct cccg gct ct caggc 1020

gcat t t ct cc gagcat ccgt ct at gggcag t t t gt ggct g gt gagacagc agaggaggt g 1080
aagggct gcg t gcagcagct gcggaccct c agcct ccgac cact gct ggc agt gcccact 1140
gaggaggagc cggact ct gc t gccaagagg at gaggct t c accat gt t gg ccaggct ggt 1200
ct t gaact cc t gaccccagc ggcat ct ggc t ct gt t gccc aggct ggagt gcagt ggaga 1260
caat cct ct g acaggggagg aggaaaccag gct gct gcct ccaggt ct t c cct t ct ccag 1320
gaggcagcat t ct ccccacc at gcggaagg t t gcagct t c cagcccaacc t gcct ccaga 1380
cacggt gct a ggggccgagg aagcat gaag gcaaaat ccc t gacct cgag gcact t act t 1440
gcaagt cagg ggcaagagac aat aat t aaa accaaagt ca gaat cccagc act t t ggaag 1500
gct gagcct g ggcaacacag caagaccccg t ct cagcaaa acaaaagt ca gt acgt aaca 1560
acact t t ggg aggccgat gt gggcagat ca ct t gagaacc t ccaggt ct c ct gcct caat 1620
gct gagcaga accagcacct ccgggcct cc ct cagccgcc t gcat cgggt ggcacaggt a 1680
acccct cct g ct ggaaccag cacct ccggg cct ccct ct g ccgcct gcat t gggt ggcac 1740
agccgcct gc at cgggt ggc acagt at gcc cgggcccagc acgt gcggct cct ggt ggat 1800
gcggagt aca cct cact gaa ccct gcgct c t cgct gct gg t ggct gccct ggct gt gcgc 1860
t ggaacagcc cgggt gaagg cgggccct gg gt gt ggaaca cct accaggc ct gt ct aaag 1920
gacacat t cg agcggct ggg gagggat gca gaggct gcgc acagggccgg cct ggcct t c 1980
ggagt gaagc t ggt acgagg t gcat at ct g gacaaggaga gagcggt ggc ccagct ccat 2040
gggat ggaag accccact ca gcct gact at gaggccacca gt gagct gaa cagagcat ct 2100
ccct t cagt t acagccgct g cct ggaact g at gct gacgc acgt ggcccg ccat ggcccc 2160
at gt gccacc t cat ggt ggc t t cccacaat gaggaat ct g t t cgccaggc aaccaagcgc 2220
at gt gggagc t gggcat t cc t ct ggat ggg act gt ct gt t t cggacaact t ct gggcat g 2280
t gt gaccacg t ct ct ct agc act ggggcag gccggct at g t agt gt at aa gt ccat t ccc 2340
t at ggct cct t ggaggaggt aat cccct ac ct gat ccgga gggcccagga gaaccggagc 2400
gt gct t cagg gt gcccgcag ggaacaggag ct gct cagcc aagaact gt g gcggcggct g 2460
ct gccaggat gccgaaggat accccact ag cacccct gag ggggt cat gt ggt caat aaa 2520
agt cct t agg t gct gcct 2538

<210> 41
<211> 4737
<212> DNA
<213> Homo sapi ens

<400> 41

gccccgggaa gcgcagccat ggct ct gcgg aggct ggggg ccgcgct gct gct gct gccg 60
ct gct cgccg ccgt ggaaga aacgct aat g gact ccact a cagcgact gc t gagct gggc 120

tggatggtgc atcctccatc agggtgggaa gaggtgagtg gctacgatga gaacatgaac 180
acgatccgca cgtaccaggt gtgcaacgtg tttgagtcaa gccagaacaa ctggctacgg 240
accaagttta tccggcgccg tggcgcccac cgcatccacg tggagatgaa gttttcggtg 300
cgtgactgca gcagcatccc cagcgtgcct ggctcctgca aggagacctt caacctctat 360
tactatgagg ctgactttga ctcggccacc aagaccttcc ccaactggat ggagaatcca 420
tgggtgaagg tggataccat tgcagccgac gagagcttct cccaggtgga cctgggtggc 480
cgcgtcatga aaatcaacac cgaggtgcgg agcttcggac ctgtgtcccg cagcggcttc 540
tacctggcct tccaggacta tggcggctgc atgtccctca tcgccgtgcg tgtcttctac 600
cgcaagtgcc cccgcatcat ccagaatggc gccatcttcc aggaaaccct gtcgggggct 660
gagagcacat cgctggtggc tgcccggggc agctgcatcg ccaatgcgga agaggtggat 720
gtacccatca agctctactg taacggggac ggcgagtggc tggtgcccat cgggcgctgc 780
atgtgcaaag caggcttcga ggccgttgag aatggcaccg tctgccgagg ttgtccatct 840
gggactttca aggccaacca aggggatgag gcctgtaccc actgtcccat caacagccgg 900
accacttctg aaggggccac caactgtgtc tgccgcaatg gctactacag agcagacctg 960
gaccccctgg acatgccctg cacaaccatc ccctccgcgc cccaggctgt gatttccagt 1020
gtcaatgaga cctcccctcat gctggagtgg acccctcccc gcgactccgg aggccgagag 1080
gacctcgtct acaacatcat ctgcaagagc tgtggctcgg gccggggtgc ctgcacccgc 1140
tgcggggaca atgtacagta cgcaccacgc cagctaggcc tgaccgagcc acgcatttac 1200
atcagtgacc tgctggccca cacccagtac accttcgaga tccaggctgt gaacggcgtt 1260
actgaccaga gccccttctc gcctcagttc gcctctgtga acatcaccac caaccaggca 1320
gctccatcgg cagtgtccat catgcatcag gtgagccgca ccgtggacag cattaccctg 1380
tcgtggtccc agccggacca gcccaatggc gtgatcctgg actatgagct gcagtactat 1440
gagaaggagc tcagtgagta caacgccaca gccataaaaa gccccaccaa cacggtcacc 1500
gtgcagggcc tcaaagccgg cgccatctat gtcttccagg tgcgggcacg caccgtggca 1560
ggctacgggc gctacagcgg caagatgtac ttccagacca tgacagaagc cgagtaccag 1620
acaagcatcc aggagaagtt gccactcatc atcggctcct cggccgctgg cctggtcttc 1680
ctcattgctg tggttgtcat cgccatcgtg tgtaacagaa gacgggggtt tgagcgtgct 1740
gactcggagt acacggacaa gctgcaacac tacaccagtg gccacatgac cccaggcatg 1800
aagatctaca tcgatccttt cacctacgag gaccccaacg aggcagtgcg ggagtttgcc 1860
aaggaaattg acatctcctg tgtcaaaatt gagcaggtga tcggagcagg ggagtttggc 1920
gaggtctgca gtggccacct gaagctgcca ggcaagagag agatctttgt ggccatcaag 1980
acgctcaagt cgggctacac ggagaagcag cgccgggact tcctgagcga agcctccatc 2040
atgggccagt tcgaccatcc caacgtcatc cacctggagg gtgtcgtgac caagagcaca 2100
cctgtgatga tcatcaccga gttcatggag aatggctccc tggactcctt tctccggcaa 2160

aacgat gggc agt t cacagt cat ccagct g gt gggcat gc t t cggggcat cgcagct ggc 2220
at gaagt acc t ggcagacat gaact at gt t caccgt gacc t ggct gcccg caacat cct c 2280
gt caacagca acct ggt ct g caaggt gt cg gact t t gggc t ct cacgct t t ct agaggac 2340
gat acct cag accccacct a caccagt gcc ct gggcggaa agat ccccat ccgct ggaca 2400
gccccggaag ccat ccagt a ccggaagt t c acct cggcca gt gat gt gt g gagct acggc 2460
at t gt cat gt gggaggt gat gt cct at ggg gagcggccct act gggacat gaccaaccag 2520
gat gt aat ca at gccat t ga gcaggact at cggct gccac cgcccat gga ct gcccgagc 2580
gccct gcacc aact cat gct ggact gt t gg cagaaggacc gcaaccaccg gcccaagt t c 2640
ggccaaat t g t caacacgct agacaagat g at ccgcaat c ccaacagcct caaagccat g 2700
gcgcccct ct cct ct ggcat caacct gccg ct gct ggacc gcacgat ccc cgact acacc 2760
agct t t aaca cggt ggacga gt ggct ggag gccat caaga t ggggcagt a caaggagagc 2820
t t cgccaat g ccggct t cac ct cct t t gac gt cgt gt ct c agat gat gat ggaggacat t 2880
ct ccgggt t g gggt cact t t ggct ggccac cagaaaaaaa t cct gaacag t at ccaggt g 2940
at gcgggcgc agat gaacca gat t cagt ct gt ggaggt t t gacat t cacc t gcct cggct 3000
cacct ct t cc t ccaagcccc gcccccct ct g ccccacgt gc cggccct cct ggt gct ct at 3060
ccact gcagg gccagccact cgccaggagg ccacgggcca cgggaagaac caagcggt gc 3120
cagccacgag acgt caccaa gaaaacat gc aact caaacg acggaaaaaa aaagggaat g 3180
ggaaaaaaga aaacagat cc t gggaggggg cgggaaat ac aaggaat at t t t t t aaagag 3240
gat t ct cat a aggaaagcaa t gact gt t ct t gcgggggat aaaaaagggc t t gggagat t 3300
cat gcgat gt gt ccaat cgg agacaaaagc agt t t ct ct c caact ccct c t gggaaggt g 3360
acct ggccag agccaagaaa cact t t caga aaaacaaat g t gaaggggag agacaggggc 3420
cgccct t ggc t cct gt ccct gct gct cct c t aggcct cac t caacaacca agcgcct gga 3480
ggacgggaca gat ggacaga cagccaccct gagaacccct ct gggaaaat ct at t cct gc 3540
caccact ggg caaacagaag aat t t t t ct g t ct t t ggaga gt at t t t aga aact ccaat g 3600
aaagacact g t t t ct cct gt t ggct cacag ggct gaaagg ggct t t t gt c ct cct gggt c 3660
agggagaacg cggggacccc agaaaggt ca gcct t cct ga ggat gggcaa cccccaggt c 3720
t gcagct cca ggt acat at c acgcgcacag cct ggcagcc t ggccct cct ggt gcccact 3780
cccgccagcc cct gcct cga ggact gat ac t gcagt gact gccgt cagct ccgact gccg 3840
ct gagaaggg t t gat cct gc at ct gggt t t gt t t acagca at t cct ggac t cgggggt at 3900
t t t ggt caca gggt ggt t t t ggt t t agggg gt t t gt t t gt t gggt t gt t t t t t gt t t t t t 3960
ggt t t t t t t t aat gacaat g aagt gacact t t gacat t t c ct acct t t t g aggact t gat 4020
cct t ct ccag gaagaaggt g ct t t ct gct t act gact t ag gcaat acacc aagggcgaga 4080
t t t t at at gc acat t t ct gg at t t t t t t at acggt t t t ca t t gacact ct t ccct cct cc 4140
cacct gccac caggcct cac caaagcccac t gccat gggg ccat ct gggc cat t cagaga 4200

ct ggagt gag at t t gggt gt ggagggggag gcgccaaggt ggaggagct t cccact ccag 4260
gact gt t gat gaaagggaca gat t gaggag gaagt gggct ct gaggct gc agggct ggaa 4320
gt cct t gccc act t cccact ct cct gcccc aat ct at ct a gt act t ccca ggcaaat agg 4380
cccct t t gag gct cct gagt gccct cagat ggt caaaacc cagt t t t ccc t ct gggagcc 4440
t aaaccaggc t gcat cggag gccaggaccc ggat cat t ca ct gt gat acc ct gccct cca 4500
gagggt gcgc t cagagacac gggcaagcat gcct ct t ccc t t ccct ggag agaaagt gt g 4560
t gat t t ct ct cccacct cct t cccccccacc agacct t t gc t gggcct aaa ggt ct t ggcc 4620
at ggggacgc cct cagt ct a gggat ct ggc cacagact cc ct cct gt gaa ccaacacaga 4680
cacccaagca gagcaat cag t t agt gaat t gaat ggaaat aaacgct t t a gt t at aa 4737

<210> 42
<211> 1505
<212> DNA
<213> Homo sapi ens

<400> 42

agct gcggcg ct gggct ggc ggcggcgagt ccacgt gct c cccgcggccg gt t gaaaccg 60
t t ggcgggcg ct ggct gaga ggcaat gt t t gct gt ct t cc at t ggagt ga ct gaat t t ct 120
acat gacggc t t t t t gacaa gact t aaaac ct gt ct t gga t agagaat at t t agccat t t 180
acct aaaaat ggt at t t t t t acat gcaat g cat gt ggt ga at cagt gaag aaaat acaag 240
t ggaaaagca t gt gt ct gt t t gcagaaact gt gaat gcct t t ct t gcat t gact gcggt a 300
aagat t t ct g gggcgat gac t at aaaaacc acgt gaaat g cat aagt gaa gat cagaagt 360
at ggt ggcaa aggct at gaa ggt aaaaccc acaaaggcga cat caaacag caggcgt gga 420
t t cagaaaat t agt gaat t a at aaagagac ccaat gt cag ccccaaagt g agagaact t t 480
t agagcaaat t agt gct t t t gacaacgt t c ccaggaaaaa ggcaaaat t t cagaat t gga 540
t gaagaacag t t t aaaagt t cat aat gaat ccat t ct gga ccaggt gt gg aat at ct t t t 600
ct gaagct t c caacagcgaa ccagt caat a aggaacagga t caacggcca ct ccacccag 660
t ggcaaat cc acat gcagaa at ct ccacca aggt t ccagc ct ccaaagt g aaagacgccg 720
t ggaacagca aggggaggt g aagaagaat a aaagagaaag aaaggaagaa cggcagaaga 780
aaaggaaaag agaaaagaaa gaact aaagt t agaaaacca ccaggaaaac t caaggaat c 840
agaagcct aa gaagcgcaaa aagggacagg aggct gacct t gaggct ggt ggggaggaag 900
t ccct gaggc caat ggct ct gcagggaaga ggagcaagaa gaagaagcag cgcaaggaca 960
gcgccagt ga ggaagaggca cgcgt gggcg cagggaagag gaagcggagg cact cggaag 1020
t t gaaacaga t t ct aagaag aaaaagat ga agct cccaga gcat cct gag ggcggagaac 1080
cagaagacga t gaggct cct gcaaaaggt a aat t caact g gaagggaact at t aaagcaa 1140

t t ct gaaaca ggccccagac aat gaaat aa ccat caaaaa gct aaggaaa aaggt t t t ag 1200
ct cagt act a cacagt gaca gat gagcat c acagat ccga agaggaact c ct ggt cat ct 1260
t t aacaagaa aat cagcaag aaccct acct t t aagt t at t aaaggacaaa gt caagct t g 1320
t gaaat gaac at t t gt gt at t t aaaaat t g aat ccat t ct gct gact t ct t cct t t cact 1380
gct gt t t at a aaat gt gt aa t gaat t ct aa caact caaat t t t gct t t t t gaagct gt at 1440
t t t t aagt t a agaaaat at a t t t t t ggt at aact t t t at g agaaaaat aa aat at at t ct 1500
ggt cc 1505

<210> 43
<211> 557
<212> DNA
<213> Homo sapiens

<400> 43

cct cct ggga gggagct gaa gccgct cgca agact cccgt agt ccccacc t ct ct cagct 60
t ccggct ggt agt agt t ccg ct t cct gt cc gact gt ggt g t ct t t gct ga gggt cacat t 120
gagct gcagg t t gaat ccgg ggt gcct t t a ggat t cagca ccat ggcgga agacat ggag 180
accaaaat ca agaact acaa gaccgcccct t t t gacagcc gct t ccccaa ccagaaccag 240
act agaaact gct ggcagaa ct acct ggac t t ccaccgct gt cagaaggc aat gaccgct 300
aaaggaggcg at at ct ct gt gt gcgaat gg t accagcgt g t gt accagt c cct ct gcccc 360
acat cct ggg t cacagact g ggat gagcaa cgggct gaag gcacgt t t cc cgggaagat c 420
t gaact ggct gcat ct ccct t t cct ct gt c ct ccat cct t ct cccaggat ggt gaagggg 480
gacct ggt ac ccagt gat cc ccaccccagg at cct aaat c at gact t acc t gct aat aaa 540
aact cat t gg aaaagt g 557

<210> 44
<211> 662
<212> DNA
<213> Homo sapiens

<400> 44

ggggggggga gcacaaagt t gggagt gaca ccagagcct c ct gcaagat g ct t ct gat t c 60
t gct gt cagt ggccct gct g gcct t cagct cagct cagga t t t aaat gaa gat gt cagcc 120
aggaagat gt t cccct cgt a at at cagat g gaggagact c t gagcagt t c at agat gagg 180
agcgt caggg accacct t t g ggaggacagc aat ct caacc ct ct gct ggt gat gggaacc 240
aggat gat gg ccct cagcag ggaccacccc aacaaggagg ccagcagcaa caaggt ccac 300

cacctcctca gggaaagcca caaggaccac cccaacaggg aggccatccc cctcctcctc 360
aaggaaggcc acaaggacca ccccaacagg gaggccatcc ccgtcctcct cgaggaaggc 420
cacaaggacc accccaacag ggaggccatc agcaaggtcc tcccccacct cctcctggaa 480
agccccaggg accacctccc caaggggccc gcccacaagg acctccacag gggcagtctc 540
ctcagtaatc caggattcaa tgacaggaag tgaataagaa gataacagtg tttcaaatgc 600
cgtgaaacat ggcatcatgc tctaacttca gtataccaat aaaacaatca gcttgcaatt 660
tc 662

<210> 45
<211> 2232
<212> DNA
<213> Homo sapiens

<400> 45

cttccccttc tctgccctgc tccaggcacc aggctctttc cccttcagtg tctcagagga 60
ggggacggca gcaccatgga cccccgcttg tccactgtcc gccagacctg ctgctgcttc 120
aatgtccgca tcgcaaccac cgccctggcc atctaccatg tgatcatgag cgtcttgttg 180
ttcatcgagc actcagtaga ggtggcccat ggcaaggcgt cctgcaagct ctcccagatg 240
ggctacctca ggatcgctga cctgatctcc agcttcctgc tcatcaccat gctcttcatc 300
atcagcctga gcctactgat cggcgtagtc aagaaccggg agaagtacct gctgcccttc 360
ctgtccctgc aaatcatgga ctatctcctg tgcctgctca ccctgctggg ctcctacatt 420
gagctgcccg cctacctcaa gttggcctcc cggagccgtg ctagctcctc caagttcccc 480
ctgatgacgc tgcagctgct ggacttctgc ctgagcatcc tgaccctctg cagctcctac 540
atggaagtgc ccacctatct caacttcaag tccatgaacc acatgaatta cctccccagc 600
caggaggata tgcctcataa ccagttcatc aagatgatga tcatcttttc catcgccttc 660
atcactgtcc ttatcttcaa ggtctacatg ttcaagtgcg tgtggcggtg ctacagattg 720
atcaagtgca tgaactcggt ggaggagaag agaaactcca agatgctcca gaaggtggtc 780
ctgccgtcct acgaggaagc cctgtctttg ccatcgaaga ccccagaggg gggcccagca 840
ccacccccat actcagaggt gtgaccctcg ccaggcccca gccccagtgc tgggaggggt 900
ggagctgcct cataatctgc tttttttgctt tggtggcccc tgtggcctgg gtgggccctc 960
ccgcccctcc ctggcaggac aatctgcttg tgtctccctc gctggcctgc tcctcctgca 1020
gggcctgtga gctgctcaca actgggtcaa cgctttaggc tgagtcactc ctcgggtctc 1080
tccataattc agcccaacaa tgcttggttt atttcaatca gctctgacac ttgtttagac 1140
gattggccat tctaaagttg gtgagtttgt caagcaacta tcgacttgat cagttcagcc 1200
aagcaactga caaatcaaaa acccacttgt cagttcagta aaataatttg gtcaaacaac 1260

agt ct at t gc at t gat t t at aaat agt t gt cagt t cacat agcaat t t aa t caagt aat c 1320
at t aat t agt t accccct at at at aaat at at gt aat caa t t t ct t caaa t agct t gct t 1380
acat gat aat caat t agcca accat gagt c at t t agaat a gt gat aaat a gaat acacag 1440
aat agt gat g aaat t caat t t aaaaaat ca cgt t agcct c caaaccat t t aat t caaat g 1500
aacccat caa ct ggat gcca act ct ggcga at gt aggacc t ct gagt ggc t gt at aat t g 1560
t t aat t caaa t gaaat t cat t t aaacagt t gacaaact gt cat t caacaa t t agct ccag 1620
gaaat aacag t t at t t cat c at aaaacagt ccct t caaac acacaat t gt t ct gct gaag 1680
agt t gt cat c aacaat ccaa t gct cacct a t t cagt t gct ct gt ggt cag t gt ggct gca 1740
t agcagt gga t t ccat gaaa ggagt cat t t t agt gat gag ct gccagt cc at t cccaggc 1800
caggct gt cg ct ggccat cc at t cagt cga t t cagt cat a ggcgaat ct g t t ct gcccga 1860
ggct t gt ggt caagcaaaaa t t cagccct g aaat caggca cat ct gt t cg t t ggact aaa 1920
cccacaggt t agt t cagt ca aagcaggcaa cccccct t gt g ggcact gacc ct gccact gg 1980
ggt cat ggcg gt t gt ggcag ct ggggaggt t t ggccccaa cagccct cct gt gcct gct t 2040
ccct gt gt gt cggggt cct c cagggagct g acccagaggt ggaggccacg gaggcagggt 2100
ct ct ggggac t gt cgggggg t acagaggga gaaggct ct g caagagct cc ct ggcaat ac 2160
cccct t gt gt aat t gct t t g t gt gcgacag ggaggaagt t t caat aaagc aacaacaagc 2220
t t caaggaat t c 2232

<210> 46
<211> 3136
<212> DNA
<213> Homo sapi ens

<400> 46

gaagcgcccg ccccggccgg agccgccat g t aaccggcgc cgcccggagc ccgagccgcg 60
cgggccccag cgacccgccc gccat ggggg acgaggacga cgat gagagc t gcgccgt gg 120
agct gcggat cacagaagcc aacct gaccg ggcacgagga gaaggt gagc gt ggagaact 180
t cgagct gct caaggt gct g ggcacgggag cct acggcaa ggt gt t cct g gt gcggaagg 240
cgggcgggca cgacgcgggg aagct gt acg ccat gaaggt gct gcgcaag gcggcgct gg 300
t gcagcgcgc caagacgcaa gagcacacgc gcaccgagcg ct cggt gct g gagct ggt gc 360
gccaggcgcc ct t cct ggt c acgct gcact acgct t t cca gacggat gcc aagct gcacc 420
t cat cct gga ct at gt gagc ggcggggaga t gt t caccca cct ct accag cgccagt act 480
t caaggaggc t gaggt gcgc gt gt at gggg gt gagat cgt gct ggccct g gaacacct gc 540
acaagct cgg cat cat t t ac cgagacct ga aact ggagaa t gt gct gct g gact ccgagg 600
gccacat t gt cct cacggac t t cgggct ga gcaaggagt t cct gacggag gagaaagagc 660

ggacct t ct c ct t ct gt ggc accat cgagt acat ggcccc cgaaat cat c cgt agcaaga 720
cggggcat gg caaggct gt g gact ggt gga gcct gggcat ct t gct ct t c gagct gct ga 780
cgggggcct c gccct t cacc ct ggagggcg agaggaacac gcaggct gag gt gt ct cgac 840
ggat cct gaa gt gct cccct ccct t ccccc ct cggat cgg gcccgt ggcg caggacct gc 900
t gcagcggct gct t t gt aag gat cct aaga agcgat t ggg cgcggggccc cagggggcac 960
aagaagt ccg gaaccat ccc t t ct t ccagg gcct cgat t g ggt ggct ct g gct gccagga 1020
agat t ccagc cccat t ccgg ccccaaat cc gct cagagct ggat gt gggc aact t t gcgg 1080
aggaat t cac t cggct ggag cct gt ct act cacccct gg cagccccca cct ggggacc 1140
cccgaat ct t t cagggat ac t cct t t gt gg caccct ccat t ct ct t t gac cacaacaacg 1200
cggt gat gac cgat gggct g gaagcgcct g gt gct ggaga ccggccaggt cgggcagcgg 1260
t ggccaggag cgct at gat g caggact cgc cct t ct t cca gcagt acgag ct ggacct gc 1320
gggagcct gc gct gggccag ggcagct t t ct gt gt gt cg ccgct gccgc cagcgccaga 1380
gcggccagga gt t cgcagt c aagat cct ca gt cgcaggct ggaggcgaac acgcagcgcg 1440
aagt ggct gc cct gcgcct g t gccagt cac accccaacgt ggt gaat ct g cacgaggt gc 1500
at cacgacca gct gcacacg t acct ggt cc t ggagct gct gcggggcggg gagct gct gg 1560
agcacat ccg caagaagcgg cact t cagcg agt cggaagc aagccagat c ct gcgcagcc 1620
t cgt gt cggc cgt gagct t c at gcacgagg aggcgggcgt ggt gcaccgc gacct caagc 1680
cggagaacat cct gt acgcc gacgacacgc ccggggcccc ggt gaaaat c at cgact t cg 1740
ggt t cgcgcg gt t gcggccg cagagt cccg gggt gcccat gcagacgccc t gct t cacgc 1800
t gcagt acgc t gcccccgag ct gct ggcgc agcagggct a cgacgagt cc t gcgacct ct 1860
ggagcct ggg cgt cat t ct g t acat gat gc t gt cggggca ggt cccct t c caggggcct 1920
ct ggccaggg cgggcagagc caggcggccg agat cat gt g caaaat ccgc gaggggcgct 1980
t ct ccct t ga cggggaggcc t ggcagggt g t at ccgagga agccaaggag ct ggt ccgag 2040
ggct cct gac cgt ggacccc gccaagcggc t gaagct cga gggact gcgg ggcagct cgt 2100
ggct gcagga cggcagcgcg cgct cct cgc ccccgct ccg gacgcccgac gt gct cgagt 2160
cct ct gggcc cgcagt gcgc t cgggt ct ca acgccacct t cat ggcat t c aaccggggca 2220
agcgggaggg ct t ct t cct g aagagcgt gg agaat gcacc cct ggccaag cggcggaagc 2280
agaagct gcg gagcgccacc gcct cccgcc ggggct cccc t gcaccagcc aacccgggcc 2340
gagcccccgt cgcct ccaaa ggggcccccc gccgagccaa cggcccccct g cccccct cct 2400
aat ccccacc act gt gaccc cct t ccct ca t aggggct gt gacct gggag cccggct cac 2460
t cccggaggc ct ct gcct gc ggct gacct g at ccccaagg gact gt cct t t cct ct cct a 2520
ccccacccca ct cccagaca gagcagaagt at t t t t at aa gcagagaat t t t t t at gt ct 2580
t accagat ag agt t gcaggg aagggggggc ct gct gggga gt ggggt t t g gggggccct c 2640
t cccaggaca ct gcct ct t c t gggcagaag gcccct ccag ggggact gct ccaacaggaa 2700

agagcccct c ccccact t ct aagcact gag t t aggagt gc t aact cct aa act gggaccc 2760
cct accct gt t ct cccct ga ggccccgt t c ct gggagggg cacccct caa ct gt cact t t 2820
at ggact gt c t gt gcaat t a cgt ccaccaa agacccgt gt t gggggt act gaaggagagg 2880
ccct ggggga ccct ct gaag cat t t ct gcc t cact t t at g t cat ct gct t ct cccct gt t 2940
ggggct aagg aaggagat ag gt ggct cct a aaagaggagg ccat ct t ct c acccacccct 3000
t cct ct t t gg cacagct act cct ggct ggg ggt ggggcct t gggggt ct g ggct gggcat 3060
ccat ggt cac t gcct cagcc cagccaggct gt gcct t t ga ct t t aaaat a aaagt ccacc 3120
cagt gct gt g t gt ggc 3136

<210> 47
<211> 6918
<212> DNA
<213> Homo sapiens

<400> 47

ct t t gacct g cagt agagcc t acgt cagag gct ggcgcaa acagaagt gc agcggt ggcg 60
gcggct ggt t gcgggccggc ggcgggct gg cggagat gga ggat ct t gt t caagat gggg 120
t ggct t cacc agct acccct gggaccggga aat ct aagct ggaaacat t g cccaaagaag 180
acct cat caa gt t t gccaag aaacagat ga t gct aat aca gaaagct aaa t caaggt gt a 240
cagaat t gga gaaagaaat t gaagaact ca gat caaaacc t gt t act gaa ggaact ggt g 300
at at t at t aa ggcat t aact gaacgt ct gg at gct ct t ct t ct ggaaaaa gcagagact g 360
agcaacagt g t ct t t ct ct g aaaaaggaaa at at aaaaat gaagcaagag gt t gaggat t 420
ct gt aacaaa gat gggagat gcacat aagg agt t ggaaca at cacat at a aact at gt ga 480
aagaaat t ga aaat t t gaaa aat gagt t ga t ggcagt acg t t ccaaat ac agt gaagaca 540
aagct aact t acaaaagcag ct ggaagaag caat gaat ac gcaat t agaa ct t t cagaac 600
aact t aaat t t cagaacaac t ct gaagat a at gt t aaaaa act acaagaa gagat t gaga 660
aaat t aggcc aggct t t gag gagcaaat t t t at at ct gca aaagcaat t a gacgct acca 720
ct gat gaaaa gaaggaaaca gt t act caac t ccaaaat at cat t gaggct aat t ct cagc 780
at t accaaaa aaat at t aat agt t t gcagg aagagct t t t acagt t gaaa gct at acacc 840
aagaagaggt gaaagagt t g at gt gccaga t t gaagcat c agct aaggaa cat gaagcag 900
agat aaat aa gt t gaacgag ct aaaagaga act t agt aaa acaat gt gag gcaagt gaaa 960
agaacat cca gaagaaat at gaat gt gagt t agaaaat t t aaggaaagcc acct caaat g 1020
caaaccaaga caat cagat a t gt t ct at t c t ct t gcaaga aaat acat t t gt agaacaag 1080
t agt aaat ga aaaagt caaa cact t agaag at acct t aaa agaact t gaa t ct caacaca 1140
gt at ct t aaa agat gaggt a act t at at ga at aat ct t aa gt t aaaact t gaaat ggat g 1200

```
ctcaacatat aaaggatgag ttttttcatg aacgggaaga cttagagttt aaaattaatg 1260
aattattact agctaaagaa gaacagggct gtgtaattga aaaattaaaa tctgagctag 1320
caggtttaaa taaacagttt tgctatactg tagaacagca taacagagaa gtacagagtc 1380
ttaaggaaca acatcaaaaa gaaatatcag aactaaatga gacattttttg tcagattcag 1440
aaaaagaaaa attaacatta atgtttgaaa tacagggtct taaggaacag tgtgaaaacc 1500
tacagcaaga aaagcaagaa gcaattttaa attatgagag tttacgagag attatggaaa 1560
ttttacaaac agaactgggg gaatctgctg gaaaaataag tcaagagttc gaatcaatga 1620
agcaacagca agcatctgat gttcatgaac tgcagcagaa gctcagaact gcttttactg 1680
aaaaagatgc ccttctcgaa actgtgaatc gcctccaggg agaaaatgaa aagttactat 1740
ctcaacaaga attggtacca gaacttgaaa ataccataaa gaaccttcaa gaaaagaatg 1800
gagtatactt acttagtctc agtcaaagag ataccatgtt aaaagaatta gaaggaaaga 1860
taaattctct tactgaggaa aaagatgatt ttataaataa actgaaaaat tcccatgaag 1920
aaatggataa tttccataag aaatgtgaaa gggaagaaag attgattctt gaacttggga 1980
agaaagtaga gcaaacaatc cagtacaaca gtgaactaga acaaaaggta aatgaattaa 2040
caggaggact agaggagact ttaaaagaaa aggatcaaaa tgaccaaaaa ctagaaaaac 2100
ttatggttca aatgaaagtt ctctctgaag acaaagaagt attgtcagct gaagtgaagt 2160
ctctttatga ggaaaacaat aaactcagtt cagaaaaaaa acagttgagt agggatttgg 2220
aggttttttt gtctcaaaaa gaagatgtta tccttaaaga acatattact caattagaaa 2280
agaaacttca gttaatggtt gaagagcaag ataatttaaa taaactgctt gaaaatgagc 2340
aagttcagaa gttatttgtt aaaactcagt tgtatggttt tcttaaagaa atgggatcag 2400
aagtttcaga agacagtgaa gagaaagatg ttgttaatgt cctacaggca gtcggtgaat 2460
ccttggcaaa aataaatgag gaaaaatgca acctggcttt tcagcgtgat gaaaaagtat 2520
tagagttaga aaaagagatt aagtgccttc aagaagagag tgtagttcag tgtgaagaac 2580
ttaagtcttt attgagagac tatgagcaag agaaagttct cttaaggaaa gagttagaag 2640
aaatacagtc agaaaaagag gccctgcagt ctgatcttct agaaatgaag aatgctaatg 2700
aaaaaacaag gcttgaaaat cagaatcttt taattcaagt tgaagaagta tctcaaacat 2760
gtagcaaaag tgaaatccat aatgaaaaag aaaaatgttt tataaaggaa catgaaaacc 2820
taaagccact actagaacaa aaagaattac gagataggag agcagagttg atactattaa 2880
aggattcctt agcaaaatca ccttctgtaa aaaatgatcc tctgtcttca gtaaaagagt 2940
tggaagaaaa aatagaaaat ctggaaaaag aatgcaaaga aaaggaggag aaaataaata 3000
agataaaatt agttgccgta aaggcaaaga aagaactaga ttccagcaga aaagagaccc 3060
agactgtgaa ggaagaactt gaatctcttc gatcagaaaa ggaccagtta tctgcttcca 3120
tgagagatct cattcaagga gcagaaagct ataagaatct tttattagaa tatgaaaagc 3180
agtcagagca actggatgtg gaaaaagaac gtgctaataa ttttgagcat cgtattgaag 3240
```

accttacaag acaattaaga aattcgactt tgcagtgtga aacaataaat tctgataatg 3300
aagatctcct ggctcgtatt gagacattac agtctaatgc caaattatta gaagtacaga 3360
ttttagaagt ccagagagcc aaagcaatgg tagacaaaga attagaagct gaaaaacttc 3420
agaaagaaca gaagataaag gaacatgcca ctactgtaaa tgaacttgaa gaacttcagg 3480
tacaacttca aaaggaaaag aaacagcttc agaaaaccat gcaagaatta gagctggtta 3540
aaaaggatgc ccaacaaacc acattgatga atatggaaat agctgattat gaacgtttga 3600
tgaaagaact aaatcaaaag ttaactaata aaaacaacaa gatagaagat ttggagcaag 3660
aaataaaaat tcaaaaacag aaacaagaaa ccctacaaga agaaataact tcattacagt 3720
cttcagtaca acaatatgaa gaaaaaaaca ccaaaatcaa gcaattgctt gtgaaaacca 3780
aaaaggaact ggcagattca aagcaagcag aaactgatca cttaatactt caagcatctt 3840
taaaaggtga gctggaggca agccagcagc aagtagaagt ctataaaata cagctggctg 3900
aaataacatc agagaagcac aaaatccacg agcacctgaa aacctctgcg gaacagcacc 3960
agcgtacgct aagtgcatac cagcagagag tgacagcact acaggaagag tgccgtgctg 4020
ccaaggcaga acaagctact gtaacctctg aattcgagag ctacaaagtc cgagttcata 4080
atgttctaaa acaacagaaa aataaatcta tgtctcaggc tgaaactgag ggcgctaaac 4140
aagaaaggga acatctggaa atgctgattg accagctaaa aatcaaatta caagatagcc 4200
aaaataactt acagattaat gtatctgaac ttcaaacatt gcagtctgaa catgatacac 4260
tgctagaaag gcacaacaag atgctgcagg aaactgtgtc caaagaggcg gaactccggg 4320
aaaaattgtg ttcaatacag tcagagaaca tgatgatgaa atctgaacat acacagactg 4380
tgagtcagct aacatcccag aacgaggtcc ttcgaaatag cttccgagat caagtgcgac 4440
atttgcagga agaacacaga aagacagtgg agacattaca gcagcagctc tccaagatgg 4500
aagcacagct cttccagctt aagaatgaac cgaccacaag aagcccagtt tcctctcaac 4560
aatctttgaa gaaccttcga gaaaggagaa acacagacct cccgcttcta gacatgcaca 4620
ctgtaacccg ggaagaggga gaaggcatgg agacaactga tacggagtct gtgtcttccg 4680
ccagcacata cacacagtct ttagagcagc tgcttaactc tcccgaaact aaacttgagc 4740
ctccattatg gcatgctgaa tttaccaaag aagaattggt tcagaagctc agttccacca 4800
caaaaagtgc agatcactta aacggcctgc ttcgggaaac agaagcaacc aatgcaattc 4860
ttatggagca aattaagctt ctcaaaagtg aaataagaag attggaaagg aatcaagagc 4920
gagagaagtc tgcagctaac ctggaatact tgaagaacgt cttgctgcag ttcattttct 4980
tgaaaccagg tagtgaaaga gagagacttc ttcctgttat aaatacgatg ttgcagctca 5040
gccctgaaga aaagggaaaa cttgctgcgg ttgctcaagg tgaggaagaa aatgcttccc 5100
gttcttctgg atgggcatcc tatcttcata gttggtctgg acttcgatag gttgatggaa 5160
ggaatatttt tattaaccaa atagaatcta tttacaaaaa tggttcacgt atattaccac 5220
aattcttttg tcaaaaagtg tgtatatatg tttgcatcta catatatttg tacatctata 5280

t gacagat gt at t t t aaaag t t t cat ct t g aagt aaaagt acaacagct t gaagt gt t ga 5340
t agcaggcca cagccct ct a act cat gt ga t t t cccat gc at gct gccag aat aaaacca 5400
ccaggaat ga at t cact ccc cact t ct ct g gaacct cagg acccgcccat t t ct cggcag 5460
t act gt gaat t t t gaagt t a aact aaat t t t ggt accat a ccaact ggaa t t t aggct t t 5520
aaaaat aat g t t t caaggcc aggt gt ggt g at t cat gcct gaaat cccac t act t t ggga 5580
ggct gaggct ggagaat t gc t t gaggct ag t gagct gt ga ct cccact gc act ccagct c 5640
ggggaacaga gcgagacct t gt ct ct aaaa at aat agt aa t aaaat aaaa at aacgt t t t 5700
at gact at t t at t gcaaggt cagagt t aca gat t gt t at a aat t gt t gag aaat t t t t gt 5760
gat t agaat a t gaaggaaaa agct t t gt t g gt aaaagt ga cat gt t aagg ggct at gaag 5820
t aaat at gct gcagt t aat t at gct aagt t aaaat acagt t t agt t at t t gct t t aaaat 5880
aaact ct t ct t t t t t t ct t t aaagt at act at ct caaaac t cat t at gt t gt cagagccc 5940
t agagct ggc t agt gt aaca ct gact at ga gt aggt gggc ccacact t ga gt t gaggt ga 6000
t t t cat ggt g t ct t t ccagg ct ct t gat ag ggt gt cact g cat gcaagcc at gaat ct gt 6060
t t t gagaat c ct ct ccat t t t cccaaat aa aaacct at ca caacagt gac t at at cact c 6120
agcat t ggat ct aaat at aa aagt ggt gct t t cagt gt t t t t ggcagat a gt gt t ccat a 6180
agct t t ccat cagaagggat t t t agacacc t t agaggt cc gt gct acat c gt cacagt t c 6240
ct ccgaat aa cct t aggt gg t agt gt t act t gcct t t gac acct ct gcat at gt t t t aat 6300
gact agat cc aaact gt gt t gt t ct t aaat caaaaat t gg at aat t t gt a at at t t at gt 6360
gt t aat caca cagt at gct c t ct gaagt t c t ct t aagcct t cagt t t at a ct ct t aat t t 6420
aat t t t ct t t ct gagct gga gaact ggct t t gcact t t gg t t acacagaa cat t ggt t t c 6480
caat t cagt t t aact gaaat t t gct gct ga t at gt t gagt t t gt t ct t t a aaaaat agct 6540
cat at at ct c at ct t t cct c ct gt ct t aga agaacagacc t aact agt ga at gt at t aat 6600
gaaaat gcat ct at t t caga gct gacat ga agagt t t agt t t t t t t act t t at aaact gt 6660
gaat at gagt at gccagct g cat acgat gt aact aat cat at t t aaat at at t t cact t t 6720
ct ct t t gact t t agacct t t t gaagt ct gt at aaact t gt t t t gaaat at agt ct ct gct 6780
t acgaat gt c at aacaaaat aat t t t t t gc at gat aaaaa at t act t t ga t t acaaaagg 6840
cat at t ct t t cat ggt t t ct gcaat gagag gaagt gt aat gat t at t t t a at at t t ct at 6900
t aaat at gt t t aact gt a 6918

<210> 48
<211> 6773
<212> DNA
<213> Homo sapi ens

<400> 48

cggccccaga aaacccgagc gagtaggggg cggcgcgcag gagggaggag aactgggggc 60
gcgggaggct ggtgggtgtc gggggtggag atgtagaaga tgtgacgccg cggcccggcg 120
ggtgccagat tagcggacgc gctgcccgcg gttgcaacgg gatcccgggc gctgcagctt 180
gggaggcggc tctccccagg cggcgtccgc ggagacaccc atccgtgaac cccaggtccc 240
gggccgccgg ctcgccgcgc accaggggcc ggcggacaga agagcggccg agcggctcga 300
ggctggggga ccgcgggcgc ggccgcgcgc tgccgggcgg gaggctgggg ggccggggcc 360
ggggccgtgc cccggagcgg gtcggaggcc ggggccgggg ccgggggacg gcggctcccc 420
gcgcggctcc agcggctcgg ggatcccggc cgggccccgc agggaccatg gcagccggga 480
gcatcaccac gctgcccgcc ttgcccgagg atggcggcag cggcgccttc ccgcccggcc 540
acttcaagga ccccaagcgg ctgtactgca aaaacggggg cttcttcctg cgcatccacc 600
ccgacggccg agttgacggg gtccgggaga agagcgaccc tcacatcaag ctacaacttc 660
aagcagaaga gagaggagtt gtgtctatca aaggagtgtg tgctaaccgt tacctggcta 720
tgaaggaaga tggaagatta ctggcttcta aatgtgttac ggatgagtgt ttcttttttg 780
aacgattgga atctaataac tacaatactt accggtcaag gaaatacacc agttggtatg 840
tggcactgaa acgaactggg cagtataaac ttggatccaa aacaggacct gggcagaaag 900
ctatactttt tcttccaatg tctgctaaga gctgatttta atggccacat ctaatctcat 960
ttcacatgaa agaagaagta tattttagaa atttgttaat gagagtaaaa gaaaataaat 1020
gtgtatagct cagtttggat aattggtcaa acaatttttt atccagtagt aaaatatgta 1080
accattgtcc cagtaaagaa aaataacaaa agttgtaaaa tgtatattct cccttttata 1140
ttgcatctgc tgttacccag tgaagcttac ctagagcaat gatctttttc acgcatttgc 1200
tttattcgaa aagaggcttt taaaatgtgc atgtttagaa acaaaatttc ttcatggaaa 1260
tcatatacat tagaaaatca cagtcagatg tttaatcaat ccaaaatgtc cactatttct 1320
tatgtcattc gttagtctac atgtttctaa acatataaat gtgaatttaa tcaattcctt 1380
tcatagtttt ataattctct ggcagttcct tatgatagag tttataaaac agtcctgtgt 1440
aaactgctgg aagttcttcc acagtcaggt caattttgtc aaacccttct ctgtacccat 1500
acagcagcag cctagcaact ctgctggtga tgggagttgt attttcagtc ttcgccaggt 1560
cattgagatc catccactca catcttaagc attcttcctg gcaaaaattt atggtgaatg 1620
aatatggctt taggcggcag atgatataca tatctgactt cccaaaagct ccaggatttg 1680
tgtgctgttg ccgaatactc aggacggacc tgaattctga ttttatacca gtctcttcaa 1740
aaacttctcg aaccgctgtg tctcctacgt aaaaaaagag atgtacaaat caataataat 1800
tacactttta gaaactgtat catcaaagat tttcagttaa agtagcatta tgtaaaggct 1860
caaaacatta ccctaacaaa gtaaagtttt caatacaaat tctttgcctt gtggatatca 1920
agaaatccca aaatattttc ttaccactgt aaattcaaga agcttttgaa atgctgaata 1980
tttctttggc tgctacttgg aggcttatct acctgtacat ttttggggtc agctcttttt 2040

aacttcttgc tgctcttttt cccaaaaggt aaaaatatag attgaaaagt taaaacattt 2100
tgcatggctg cagttccttt gtttcttgag ataagattcc aaagaactta gattcatttc 2160
ttcaacaccg aaatgctgga ggtgtttgat cagttttcaa gaaacttgga atataaataa 2220
ttttataatt caacaaaggt tttcacattt tataaggttg atttttcaat taaatgcaaa 2280
tttgtgtggc aggattttta ttgccattaa catattttg tggctgcttt ttctacacat 2340
ccagatggtc cctctaactg ggctttctct aattttgtga tgttctgtca ttgtctccca 2400
aagtatttag gagaagccct ttaaaaagct gccttcctct accactttgc tggaaagctt 2460
cacaattgtc acagacaaag atttttgttc caatactcgt tttgcctcta tttttcttgt 2520
ttgtcaaata gtaaatgata tttgcccttg cagtaattct actggtgaaa aacatgcaaa 2580
gaagaggaag tcacagaaac atgtctcaat tcccatgtgc tgtgactgta gactgtctta 2640
ccatagactg tcttacccat cccctggata tgctcttgtt ttttccctct aatagctatg 2700
gaaagatgca tagaaagagt ataatgtttt aaaacataag gcattcatct gccatttttc 2760
aattacatgc tgacttccct tacaattgag atttgcccat aggttaaaca tggttagaaa 2820
caactgaaag cataaaagaa aaatctaggc cgggtgcagt ggctcatgcc tatattccct 2880
gcactttggg aggccaaagc aggaggatcg cttgagccca ggagttcaag accaacctgg 2940
tgaaaccccg tctctacaaa aaaacacaaa aaatagccag gcatggtggc gtgtacatgt 3000
ggtctcagat acttgggagg ctgaggtggg agggttgatc acttgaggct gagaggtcaa 3060
ggttgcagtg agccataatc gtgccactgc agtccagcct aggcaacaga gtgagacttt 3120
gtctcaaaaa aagagaaatt ttccttaata agaaaagtaa tttttactct gatgtgcaat 3180
acatttgtta ttaaatttat tatttaagat ggtagcacta gtcttaaatt gtataaaata 3240
tcccctaaca tgtttaaatg tccattttta ttcattatgc tttgaaaaat aattatgggg 3300
aaatacatgt ttgttattaa atttattatt aaagatagta gcactagtct taaatttgat 3360
ataacatctc ctaacttgtt taaatgtcca tttttattct ttatgcttga aaataaatta 3420
tggggatcct atttagctct tagtaccact aatcaaaagt tcggcatgta gctcatgatc 3480
tatgctgttt ctatgtcgtg gaagcaccgg atgggggtag tgagcaaatc tgccctgctc 3540
agcagtcacc atagcagctg actgaaaatc agcactgcct gagtagtttt gatcagttta 3600
acttgaatca ctaactgact gaaaattgaa tgggcaaata agtgcttttg tctccagagt 3660
atgcgggaga cccttccacc tcaagatgga tatttcttcc ccaaggattt caagatgaat 3720
tgaaattttt aatcaagata gtgtgcttta ttctgttgta ttttttatta ttttaatata 3780
ctgtaagcca aactgaaata acatttgctg ttttataggt ttgaagaaca taggaaaaac 3840
taagaggttt tgtttttatt tttgctgatg aagagatatg tttaaatatg ttgtattgtt 3900
ttgtttagtt acaggacaat aatgaaatgg agtttatatt tgttatttct attttgttat 3960
atttaataat agaattagat tgaaataaaa tataatggga aataatctgc agaatgtggg 4020
tttcctggtg tttcctctga ctctagtgca ctgatgatct ctgataaggc tcagctgctt 4080

tatagttctc tggctaatgc agcagatact cttcctgcca gtggtaatac gattttttaa 4140
gaaggcagtt tgtcaatttt aatcttgtgg atacctttat actcttaggg tattatttta 4200
tacaaaagcc ttgaggattg cattctattt tctatatgac cctcttgata tttaaaaaac 4260
actatggata acaattcttc atttacctag tattatgaaa gaatgaagga gttcaaacaa 4320
atgtgtttcc cagttaacta gggtttactg tttgagccaa tataaatgtt taactgtttg 4380
tgatggcagt attcctaaag tacattgcat gttttcctaa atacagagtt taaataattt 4440
cagtaattct tagatgattc agcttcatca ttaagaatat cttttgtttt atgttgagtt 4500
agaaatgcct tcatatagac atagtctttc agacctctac tgtcagtttt catttctagc 4560
tgctttcagg gttttatgaa ttttcaggca aagctttaat ttatactaag cttaggaagt 4620
atggctaatg ccaacggcag tttttttctt cttaattcca catgactgag gcatatatga 4680
tctctgggta ggtgagttgt tgtgacaacc acaagcactt tttttttttt taaagaaaaa 4740
aaggtagtga atttttaatc atctggactt taagaaggat tctggagtat acttaggcct 4800
gaaattatat atatttggct tggaaatgtg tttttcttca attacatcta caagtaagta 4860
cagctgaaat tcagaggacc cataagagtt cacatgaaaa aaatcaattc atttgaaaag 4920
gcaagatgca ggagagagga agccttgcaa acctgcagac tgctttttgc ccaatataga 4980
ttgggtaagg ctgcaaaaca taagcttaat tagctcacat gctctgctct cacgtggcac 5040
cagtggatag tgtgagagaa ttaggctgta gaacaaatgg ccttctcttt cagcattcac 5100
accactacaa aatcatcttt tatatcaaca gaagaataag cataaactaa gcaaaaggtc 5160
aataagtacc tgaaaccaag attggctaga gatatatctt aatgcaatcc attttctgat 5220
ggattgttac gagttggcta tataatgtat gtatggtatt ttgatttgtg taaaagtttt 5280
aaaaatcaag ctttaagtac atggacattt ttaaataaaa tatttaaaga caatttagaa 5340
aattgcctta atatcattgt tggctaaata gaatagggga catgcatatt aaggaaaagg 5400
tcatggagaa ataatattgg tatcaaacaa atacattgat ttgtcatgat acacattgaa 5460
tttgatccaa tagtttaagg aataggtagg aaaatttggt ttctattttt cgatttcctg 5520
taaatcagtg acataaataa ttcttagctt attttatatt tccttgtctt aaatactgag 5580
ctcagtaagt tgtgttaggg gattatttct cagttgagac tttcttatat gacattttac 5640
tatgttttga cttcctgact attaaaaata aatagtagaa acaattttca taaagtgaag 5700
aattatataa tcactgcttt ataactgact ttattatatt tatttcaaag ttcatttaaa 5760
ggctactatt catcctctgt gatggaatgg tcaggaattt gttttctcat agtttaattc 5820
caacaacaat attagtcgta tccaaaataa cctttaatgc taaactttac tgatgtatat 5880
ccaaagcttc tccttttcag acagattaat ccagaagcag tcataaacag aagaataggt 5940
ggtatgttcc taatgatatt atttctacta atggaataaa ctgtaatatt agaaattatg 6000
ctgctaatta tatcagctct gaggtaattt ctgaaatgtt cagactcagt cggaacaaat 6060
tggaaaattt aaatttttat tcttagctat aaagcaagaa agtaaacaca ttaatttcct 6120

caacat t t t t aagccaat t a aaaat at aaa agat acacac caat at ct t c t t caggct ct 6180

gacaggcct c ct ggaaact t ccacat at t t t t caact gca gt at aaagt c agaaaat aaa 6240

gt t aacat aa ct t t cact aa cacacacat a t gt agat t t c acaaaat cca cct at aat t g 6300

gt caaagt gg t t gagaat at at t t t t t agt aat t gcat gc aaaat t t t t c t agct t ccat 6360

cct t t ct ccc t cgt t t ct t c t t t t t t t ggg ggagct ggt a act gat gaaa t ct t t t ccca 6420

cct t t t ct ct t caggaaat a t aagt ggt t t t gt t t ggt t a acgt gat aca t t ct gt at ga 6480

at gaaacat t ggagggaaac at ct act gaa t t t ct gt aat t t aaaat at t t t gct gct ag 6540

t t aact at ga acagat agaa gaat ct t aca gat gct gct a t aaat aagt a gaaaat at aa 6600

at t t cat cac t aaaat at gc t at t t t aaaa t ct at t t cct at at t gt at t t ct aat caga 6660

t gt at t act c t t at t at t t c t at t gt at gt gt t aat gat t t t at gt aaaa at gt aat t gc 6720

t t t t cat gag t agt at gaat aaaat t gat t agt t t gt gt t t t ct t gt ct c ccg 6773

<210> 49
<211> 3537
<212> DNA
<213> Homo sapiens

<400> 49

cagaccccag t t cgccgact aagcagaaga aagat caaaa accggaaaag aggagaagag 60

caaacaggca ct t t gaggaa caat cccct t t aact ccaag ccgacagcgg t ct aggaat t 120

caagt t cagt gcct accgaa gacaaaggcg ccccgaggga gt ggcggt gc gaccccaggg 180

cgt gggcccg gccgcggagc ccacact gcc cggct gaccc ggt ggt ct cg gaccat gt ct 240

cccgccccaa gacccccccg t t gt ct cct g ct cccccct gc t cacgct cgg caccgcgct c 300

gcct ccct cg gct cggccca aagcagcagc t t cagccccg aagcct ggct acagcaat at 360

ggct acct gc ct cccgggga cct acgt acc cacacacagc gct cacccca gt cact ct ca 420

gcggccat cg ct gccat gca gaagt t t t ac ggct t gcaag t aacaggcaa agct gat gca 480

gacaccat ga aggccat gag gcgcccccga t gt ggt gt t c cagacaagt t t ggggct gag 540

at caaggcca at gt t cgaag gaagcgct ac gccat ccagg gt ct caaat g gcaacat aat 600

gaaat cact t t ct gcat cca gaat t acacc cccaaggt gg gcgagt at gc cacat acgag 660

gccat t cgca aggcgt t ccg cgt gt gggag agt gccacac cact gcgct t ccgcgaggt g 720

ccct at gcct acat ccgt ga gggccat gag aagcaggccg acat cat gat ct t ct t t gcc 780

gagggct t cc at ggcgacag cacgccct t c gat ggt gagg gcggct t cct ggcccat gcc 840

t act t cccag gccccaacat t ggaggagac acccact t t g act ct gccga gcct t ggact 900

gt caggaat g aggat ct gaa t ggaaat gac at ct t cct gg t ggct gt gca cgagct gggc 960

cat gccct gg ggct cgagca t t ccagt gac ccct cggcca t cat ggcacc ct t t t accag 1020

t ggat ggaca cggagaat t t t gt gct gccc gat gat gacc gccggggcat ccagcaact t 1080
t at gggggt g agt cagggt t ccccaccaag at gccccct c aacccaggac t acct cccgg 1140
cct t ct gt t c ct gat aaacc caaaaacccc acct at gggc ccaacat ct g t gacgggaac 1200
t t t gacaccg t ggccat gct ccgaggggag at gt t t gt ct t caaggagcg ct ggt t ct gg 1260
cgggt gagga at aaccaagt gat ggat gga t acccaat gc ccat t ggcca gt t ct ggcgg 1320
ggcct gcct g cgt ccat caa cact gcct ac gagaggaagg at ggcaaat t cgt ct t ct t c 1380
aaaggagaca agcat t gggt gt t t gat gag gcgt ccct gg aacct ggct a ccccaagcac 1440
at t aaggagc t gggccgagg gct gcct acc gacaagat t g at gct gct ct ct t ct ggat g 1500
cccaat ggaa agacct act t ct t ccgt gga aacaagt act accgt t t caa cgaagagct c 1560
agggcagt gg at agcgagt a ccccaagaac at caaagt ct gggaagggat ccct gagt ct 1620
cccagagggt cat t cat ggg cagcgat gaa gt ct t cact t act t ct acaa ggggaacaaa 1680
t act ggaaat t caacaacca gaagct gaag gt agaaccgg gct accccaa gt cagccct g 1740
agggact gga t gggct gccc at cgggaggc cggccggat g aggggact ga ggaggagacg 1800
gaggt gat ca t cat t gaggt ggacgaggag ggcggcgggg cggt gagcgc ggct gccgt g 1860
gt gct gcccg t gct gct gct gct cct ggt g ct ggcggt gg gcct t gcagt ct t ct t ct t c 1920
agacgccat g ggacccccag gcgact gct c t act gccagc gt t ccct gct ggacaaggt c 1980
t gacgcccac cgccggcccg cccact cct a ccacaaggac t t t gcct ct g aaggccagt g 2040
gcagcaggt g gt ggt gggt g ggct gct ccc at cgt cccga gcccccct ccc cgcagcct cc 2100
t t gct t ct ct ct gt cccct g gct ggcct cc t t caccct ga ccgcct ccct ccct cct gcc 2160
ccggcat t gc at ct t ccct a gat aggt ccc ct gagggct g agt gggaggg cggccct t t c 2220
cagcct ct gc ccct caggggg aaccct gt ag ct t t gt gt ct gt ccagcccc at ct gaat gt 2280
gt t gggggct ct gcact t ga aggcaggacc ct cagacct c gct ggt aaag gt caaat ggg 2340
gt cat ct gct cct t t t ccat cccct gacat acct t aacct ct gaact ct g acct caggag 2400
gct ct gggca ct ccagccct gaaagcccca ggt gt accca at t ggcagcc t ct cact act 2460
ct t t ct ggct aaaaggaat c t aat ct t gt t gagggt agag accct gagac agt gt gaggg 2520
ggt ggggact gccaagccac cct aagacct t gggaggaaa act cagagag ggt ct t cgt t 2580
gct cagt cag t caagt t cct cggagat ct g cct ct gcct c acct acccca gggaact t cc 2640
aaggaaggag cct gagccac t ggggact aa gt gggcagaa gaaaccct t g gcagccct gt 2700
gcct ct cgaa t gt t agcct t ggat ggggct t t cacagt t a gaagagct ga aaccaggggt 2760
gcagct gt ca ggt agggt gg ggccggt ggg agaggcccgg gt cagagccc t gggggt gag 2820
cct gaaggcc acagagaaag aacct t gccc aaact caggc agct ggggct gaggcccaaa 2880
ggcagaacag ccagaggggg caggagggga ccaaaaagga aaat gaggac gt gcagcagc 2940
at t ggaaggc t ggggccggg caggccaggc caagccaagc agggggccac agggt gggct 3000
gt ggagct ct caggaagggc cct gaggaag gcacact t gc t cct gt t ggt ccct gt cct t 3060

gct gcccagg cagcgt ggag gggaagggt a gggcagccag agaaaggagc agagaaggca 3120
cacaaacgag gaat gagggg ct t cacgaga ggccacaggg cct ggct ggc cacgct gt cc 3180
cggcct gct c accat ct cag t gaggggcag gagct ggggc t cgct t aggc t gggt ccacg 3240
ct t ccct ggt gccagcaccc ct caagcct g t ct caccagt ggcct gccct ct cgct cccc 3300
cacccagccc acccat t gaa gt ct cct t gg gccaccaaag gt ggt ggcca t ggt accggg 3360
gact t gggag agt gagaccc agt ggaggga gcaagaggag agggat gt cg ggggggt ggg 3420
gcacggggt a ggggaaat gg ggt gaacggt gct ggcagt t cggct agat t t ct gt ct t gt 3480
t t gt t t t t t t gt t t t gt t t a at gt at at t t t t at t at aat t at t at at at gaat t cc 3537

<210> 50
<211> 4624
<212> DNA
<213> Homo sapi ens

<400> 50

ggaggaggt g gaggaggagg gct gct t gag gaagt at aag aat gaagt t g t gaagct gag 60
at t cccct cc at t gggaccg gagaaaccag gggagccccc cgggcagccg cgcgcccct t 120
cccacggggc cct t t act gc gccgcgcgcc cggcccccac ccct cgcagc accccgcgcc 180
ccgcgccct c ccagccgggt ccagccggag ccat ggggcc ggagccgcag t gagcaccat 240
ggagct ggcg gcct t gt gcc gct gggggct cct cct cgcc ct ct t gcccc ccggagccgc 300
gagcacccaa gt gt gcaccg gcacagacat gaagct gcgg ct ccct gcca gt cccgagac 360
ccacct ggac at gct ccgcc acct ct acca gggct gccag gt ggt gcagg gaaacct gga 420
act cacct ac ct gcccacca at gccagcct gt cct t cct g caggat at cc aggaggt gca 480
gggct acgt g ct cat cgct c acaaccaagt gaggcaggt c ccact gcaga ggct gcggat 540
t gt gcgaggc acccagct ct t t gaggacaa ct at gccct g gccgt gct ag acaat ggaga 600
cccgct gaac aat accaccc ct gt cacagg ggcct cccca ggaggcct gc gggagct gca 660
gct t cgaagc ct cacagaga t ct t gaaagg aggggt ct t g at ccagcgga acccccagct 720
ct gct accag gacacgat t t t gt ggaagga cat ct t ccac aagaacaacc agct ggct ct 780
cacact gat a gacaccaacc gct ct cgggc ct gccacccc t gt t ct ccga t gt gt aaggg 840
ct cccgct gc t ggggagaga gt t ct gagga t t gt cagagc ct gacgcgca ct gt ct gt gc 900
cggt ggct gt gcccgct gca aggggccact gcccact gac t gct gccat g agcagt gt gc 960
t gccggct gc acgggcccca agcact ct ga ct gcct ggcc t gcct ccact t caaccacag 1020
t ggcat ct gt gagct gcact gcccagccct ggt cacct ac aacacagaca cgt t t gagt c 1080
cat gcccaat cccgagggcc ggt at acat t cggcgccagc t gt gt gact g cct gt ccct a 1140
caact acct t t ct acggacg t gggat cct g caccct cgt c t gccccct gc acaaccaaga 1200

ggtgacagca gaggatggaa cacagcggtg tgagaagtgc agcaagccct gtgcccgagt 1260

gtgctatggt ctgggcatgg agcacttgcg agaggtgagg gcagttacca gtgccaatat 1320

ccaggagttt gctggctgca agaagatctt tgggagcctg gcatttctgc cggagagctt 1380

tgatggggac ccagcctcca acactgcccc gctccagcca gagcagctcc aagtgtttga 1440

gactctggaa gagatcacag gttacctata catctcagca tggccggaca gcctgcctga 1500

cctcagcgtc ttccagaacc tgcaagtaat ccggggacga attctgcaca atggcgccta 1560

ctcgctgacc ctgcaagggc tgggcatcag ctggctgggg ctgcgctcac tgagggaact 1620

gggcagtgga ctggccctca tccaccataa cacccacctc tgcttcgtgc acacggtgcc 1680

ctgggaccag ctctttcgga acccgcacca agctctgctc cacactgcca accggccaga 1740

ggacgagtgt gtgggcgagg gcctggcctg ccaccagctg tgcgcccgag ggcactgctg 1800

gggtccaggg cccacccagt gtgtcaactg cagccagttc cttcggggcc aggagtgcgt 1860

ggaggaatgc cgagtactgc aggggctccc cagggagtat gtgaatgcca ggcactgttt 1920

gccgtgccac cctgagtgtc agccccagaa tggctcagtg acctgttttg gaccggaggc 1980

tgaccagtgt gtggcctgtg cccactataa ggaccctccc ttctgcgtgg cccgctgccc 2040

cagcggtgtg aaacctgacc tctcctacat gcccatctgg aagtttccag atgaggaggg 2100

cgcatgccag ccttgcccca tcaactgcac ccactcctgt gtggacctgg atgacaaggg 2160

ctgccccgcc gagcagagag ccagccctct gacgtccatc atctctgcgg tggttggcat 2220

tctgctggtc gtggtcttgg gggtggtctt tgggatcctc atcaagcgac ggcagcagaa 2280

gatccggaag tacacgatgc ggagactgct gcaggaaacg gagctggtgg agccgctgac 2340

acctagcgga gcgatgccca accaggcgca gatgcggatc ctgaaagaga cggagctgag 2400

gaaggtgaag gtgcttggat ctggcgcttt tggcacagtc tacaagggca tctggatccc 2460

tgatggggag aatgtgaaaa ttccagtggc catcaaagtg ttgagggaaa acacatcccc 2520

caaagccaac aaagaaatct tagacgaagc atacgtgatg gctggtgtgg gctccccata 2580

tgtctcccgc cttctgggca tctgcctgac atccacggtg cagctggtga cacagcttat 2640

gccctatggc tgcctcttag accatgtccg ggaaaaccgc ggacgcctgg gctcccagga 2700

cctgctgaac tggtgtatgc agattgccaa ggggatgagc tacctggagg atgtgcggct 2760

cgtacacagg gacttggccg ctcggaacgt gctggtcaag agtcccaacc atgtcaaaat 2820

tacagacttc gggctggctc ggctgctgga cattgacgag acagagtacc atgcagatgg 2880

gggcaaggtg cccatcaagt ggatggcgct ggagtccatt ctccgccggc ggttcaccca 2940

ccagagtgat gtgtggagtt atggtgtgac tgtgtgggag ctgatgactt ttggggccaa 3000

accttacgat gggatcccag cccgggagat ccctgacctg ctggaaaagg gggagcggct 3060

gccccagccc cccatctgca ccattgatgt ctacatgatc atggtcaaat gttggatgat 3120

tgactctgaa tgtcggccaa gattccggga gttggtgtct gaattctccc gcatggccag 3180

ggacccccag cgctttgtgg tcatccagaa tgaggacttg ggcccagcca gtcccttgga 3240

cagcacct t c t accgct cac t gct ggagga cgat gacat g ggggacct gg t ggat gct ga 3300
ggagt at ct g gt accccagc agggct t ct t ct gt ccagac cct gccccgg gcgct ggggg 3360
cat ggt ccac cacaggcacc gcagct cat c t accaggagt ggcggt gggg acct gacact 3420
agggct ggag ccct ct gaag aggaggcccc caggt ct cca ct ggcaccct ccgaaggggc 3480
t ggct ccgat gt at t t gat g gt gacct ggg aat gggggca gccaaggggc t gcaaagcct 3540
ccccacacat gaccccagcc ct ct acagcg gt acagt gag gaccccacag t acccct gcc 3600
ct ct gagact gat ggct acg t t gcccccct gacct gcagc ccccagcct g aat at gt gaa 3660
ccagccagat gt t cggcccc agcccccct c gccccgagag ggccct ct gc ct gct gcccg 3720
acct gct ggt gccact ct gg aaaggcccaa gact ct ct cc ccagggaaga at ggggt cgt 3780
caaagacgt t t t t gcct t t g ggggt gccgt ggagaacccc gagt act t ga caccccaggg 3840
aggagct gcc cct cagcccc accct cct cc t gcct t cagc ccagcct t cg acaacct ct a 3900
t t act gggac caggacccac cagagcgggg ggct ccaccc agcacct t ca aagggacacc 3960
t acggcagag aacccagagt acct gggt ct ggacgt gcca gt gt gaacca gaaggccaag 4020
t ccgcagaag ccct gat gt g t cct cagcga gcagggaagg cct gact t ct gct ggcat ca 4080
agaggt ggga gggccct ccg accact t cca ggggaacct g ccat gccagg aacct gt cct 4140
aaggaacct t cct t cct gct t gagt t ccca gat ggct gga aggggt ccag cct cgt t gga 4200
agaggaacag cact ggggag t ct t t gt gga t t ct gaggcc ct gcccaat g agact ct agg 4260
gt ccagt gga t gccacagcc cagct t ggcc ct t t cct t cc agat cct ggg t act gaaagc 4320
ct t agggaag ct ggcct gag aggggaagcg gccct aaggg agt gt ct aag aacaaaagcg 4380
acccat t cag agact gt ccc t gaaacct ag t act gccccc cat gaggaag gaacagcaat 4440
ggt gt cagt a t ccaggct t t gt acagagt g ct t t t ct gt t t agt t t t t ac t t t t t t t gt t 4500
t t gt t t t t t t aaagat gaaa t aaagaccca gggggagaat gggt gt t gt a t ggggaggca 4560
agt gt ggggg gt cct t ct cc acacccact t t gt ccat t t g caaat at at t t t ggaaaaca 4620
gct a 4624

<210> 51
<211> 69
<212> DNA
<213> Homo sapi ens

<400> 51

gagt gt ct t t ct t t gcaggt t t ct gt agcc ggaagat ct c cgt t ccgct c ccagcggct c 60
cagt gt aaa 69

<210> 52
<211> 70
<212> DNA
<213> Homo sapiens

<400> 52

aagagaccat ct gcccgagt agccgcaaat gt gct t cat c t aagcct ct g gggt gaacat 60

at t ct agccc 70

<210> 53
<211> 69
<212> DNA
<213> Homo sapiens

<400> 53

accgaagaaa ct gt ggct ac ccgggcat ca gccccgagga at gcgcct ct cggaagt gct 60

gct t ct cca 69

<210> 54
<211> 69
<212> DNA
<213> Homo sapiens

<400> 54

acacagccga gcagcat t t c cgt t gaagga ct t gcat ccc cat t gcgggc agt gct ggac 60

gt gt cccgg 69

<210> 55
<211> 70
<212> DNA
<213> Homo sapiens

<400> 55

acaaacgggc t cat cat aat gcact ggaac gaaaacgt ag ggaccacat c aaagacagct 60

t t cacagt t t 70

<210> 56
<211> 69
<212> DNA
<213> Homo sapi ens

<400> 56

t ccagcccat ccagcaat cg ccccct t t ct gccct ggggg cccaggat gt agat at t gt a 60

caaaggt t t 69

<210> 57
<211> 70
<212> DNA
<213> Homo sapiens

<400> 57

ggt at ggct g cagt cct ccg gt t gcat act ggact ct t ca aaaact gt t t t gggt agct g 60

ccact t gaac 70

<210> 58
<211> 69
<212> DNA
<213> Homo sapi ens

<400> 58

ccggagt cac cccgat gat t act ct t t t ca gacacagcgg t t t t t gt t t c caagaagcca 60

aaat t gt t t 69

<210> 59
<211> 69
<212> DNA
<213> Homo sapiens

<400> 59

t ct ccacaat gcaagct aca gagct gggga agt t gccggc t ggaggagt t ct ct accct c 60

cacct t cct 69

<210> 60
<211> 69
<212> DNA
<213> Homo sapi ens

<400> 60

gct gggct t c at t agt gagg ct gaccagag ccggt t gact t ct ct gct ag aagagacct t 60

gaacaagt t 69

<210> 61
<211> 69
<212> DNA
<213> Homo sapi ens

<400> 61

ggcgacagga acgt gt ct ag gcgaacgact aaagcacct g gaaaggct ga t ccggagt t c 60

aagggaaga 69

<210> 62
<211> 69
<212> DNA
<213> Homo sapi ens

<400> 62

cat t cgggcc at t t ccgt gg t t t ct cat ga gct gt gt t ca cagacct cag cagggcat cg 60

cat ggaccg 69

<210> 63
<211> 69
<212> DNA

<213> Homo sapiens

<400> 63

agat agt gct ct gt gcct aa ggt gaagcca cact agggt g aagcct cact t ccct gt t t g 60

agcaat gca 69

<210> 64
<211> 70
<212> DNA
<213> Homo sapiens

<400> 64

t ggggaggga t ggcccagcc t gt aagat ac t gt at at gcg ct gct gt aga t accggaat g 60

aat t t t ct gt 70

<210> 65
<211> 69
<212> DNA
<213> Homo sapi ens

<400> 65

t cacat t cag gcccct gt t c t t caagct gt t t gat t gggc t aaaacagaa gat gccccaa 60

aggacaggt 69

<210> 66
<211> 69
<212> DNA
<213> Homo sapi ens

<400> 66

at t at gat t a ct at at ccga ggagcaacaa ccact t t ct c t gcagt ggaa agggat cgcc 60

agt ggaagt 69

<210> 67
<211> 69
<212> DNA
<213> Homo sapiens

<400> 67

caccaggagt accgt t agct t t cccct t ct t ct ggcccat t t gact cat c agcacaat ag 60

ggt caccac 69

<210> 68
<211> 69
<212> DNA
<213> Homo sapi ens

<400> 68

acccccagca cagggggat t ct gagcagt g cct ct t gt ct gagggacat a t cagt gacct 60

cgacgt t gc 69

<210> 69
<211> 69
<212> DNA
<213> Homo sapi ens

<400> 69

t ct gt gat gt aggagt ct ct ct accact gg at gaaaat at gact gt gact gaccct gagg 60

ct t gt t aca 69

<210> 70
<211> 69
<212> DNA
<213> Homo sapiens

<400> 70

ct ccacagcg gt t ct t caca ggat cccagc aat gaact gt t ct agggaaa gt ggct t cct 60

gcccaaact 69

<210> 71
<211> 69
<212> DNA
<213> Homo sapi ens

<400> 71

at gggaagga agggaccct t acccccggct ct t ct cct ga cct gccaat a aaaat t t at g 60

gt ccaaggg 69

<210> 72
<211> 69
<212> DNA
<213> Homo sapiens

<400> 72

ct t ggcaact gct agagcag ggagggggga gggcagggga t t acct aat t agagt gggt t 60

agct t agat 69

<210> 73
<211> 69
<212> DNA
<213> Homo sapi ens

<400> 73

gt cct cat t c cct gccct t c ct t t ggt t gc cat at ggaat ggccat ggaa t gcacgaagt 60

cacaat gca 69

<210> 74
<211> 69
<212> DNA
<213> Homo sapiens

<400> 74

t ct ct t gaga ct cgt t act g ggcat cagt a gaagaat at a t t cccaaat g ggaacagt t t 60

ct t t t agga 69

<210> 75
<211> 69
<212> DNA
<213> Homo sapi ens

<400> 75

t ggt at ccag aat ggaat t t t gct acat gg ggt ct gggt g ggggcaaaga gacccagt ca 60

at t acat gt 69

<210> 76
<211> 69
<212> DNA
<213> Homo sapiens

<400> 76

ct at t ct gga ggcaccaagg aact agcaga gat gat gaat gcat cat caa gcacgaccac 60

at t gt t t cc 69

<210> 77
<211> 69
<212> DNA
<213> Homo sapiens

<400> 77

t caggt gggc ct t gagt t at at t t t aact c agct gct cag t t cccagggc acat t t ct gg 60

at cagaacc 69

<210> 78
<211> 70
<212> DNA
<213> Homo sapi ens

<400> 78

gggagat aca gccat ccacc t t cagat gt g t ct acgt gcg ct ct gccat t caact cggaa 60

act at aagt a 70

<210> 79
<211> 69
<212> DNA

<213> Homo sapi ens

<400> 79

aat t aaagt t at agacaaga t gggagggcc agaagagt t t at ccagagat t cct ccct cg 60

ggagct cca 69

<210> 80
<211> 70
<212> DNA
<213> Homo sapiens

<400> 80

cagct t t gct t t gcaaagat t gat gacaga ct ggt t cct c agaggcct ag gct acccgt c 60

accccct t t t t 70

<210> 81
<211> 70
<212> DNA
<213> Homo sapi ens

<400> 81

cct cct gggg agt gt cat t t t t gt ct acag ct t cat t gac t t ccacgt gt t ccaccgcaa 60

agat agccgc 70

<210> 82
<211> 60
<212> DNA
<213> Homo sapi ens

<400> 82
gaaggagggg ctgggccagg gccagtcgct ggaacagctg gaagctctgg tgcaaaccaa 60

<210> 83
<211> 69
<212> DNA
<213> Homo sapi ens

<400> 83

ggt gggggaa cact gagaaa gaggcaggga cct aaaggga ct acct ct gt gcct t gccac 60

at t aaat t g 69

<210> 84
<211> 69
<212> DNA
<213> Homo sapiens

<400> 84

ggcaagat ct at cggcaggg aaacct gt t t gact t ct t ac gct t gacgga at ggcgt ggc 60

ccccgcgt g 69

<210> 85
<211> 69
<212> DNA
<213> Homo sapi ens

<400> 85

aggct aggt g ggt t gaaagc caaggagt ca ct gagaccaa ggct t t ct ct act gat t ccg 60

cagct caga 69

<210> 86
<211> 69
<212> DNA
<213> Homo sapiens

<400> 86

t gact agcga gggacct ccg ct gcat ct ca gcaaagcccc t cccagggt t t gat cgat t g 60

agcaggaca 69

<210> 87
<211> 69
<212> DNA
<213> Homo sapiens

<400> 87

t cct ct gt ca t act gt at ct ggaat gct t t gt aat act t g cat gct t ct t agaccagaac 60

at gt aggt c 69

<210> 88
<211> 69
<212> DNA
<213> Homo sapiens

<400> 88

agct cct cgg agggaacaga gcggct gt gt at gcagcct g caggt t t cca t acact gaag 60

ct t t t acct 69

<210> 89
<211> 69
<212> DNA
<213> Homo sapiens

<400> 89

gt aggct gag ggct gcacct cccacggt gg t caccat gcc aat gaaggaa gt t cagt ct t 60

gt ct t t gat 69

<210> 90
<211> 70
<212> DNA
<213> Homo sapi ens

<400> 90

acct cgaggc act t act t gc aagt cagggg caagagacaa t aat t aaaac caaagt caga 60

at cccagcac 70

<210> 91
<211> 69
<212> DNA
<213> Homo sapiens

<400> 91

at t cct gcca ccact gggca aacagaagaa t t t t t ct gt c t t t ggagagt at t t t agaaa 60

ct ccaat ga 69

<210> 92
<211> 70
<212> DNA
<213> Homo sapi ens

<400> 92

gagaaccaga agacgat gag gct cct gcaa aagct cagt a ct acacagt g acagat gagc 60

at cacagat c 70

<210> 93
<211> 69
<212> DNA
<213> Homo sapi ens

<400> 93

t t ct cccagg at ggt gaagg gggacct ggt acccagt gat ccccacccca ggat cct aaa 60

t cat gact t 69

<210> 94
<211> 70
<212> DNA
<213> Homo sapi ens

<400> 94

cagt gt t t ca aat gccgt ga aacat ggcat cat gct ct aa ct t cagt at a ccaat aaaac 60

aat cagct t g 70

<210> 95

<211> 69
<212> DNA
<213> Homo sapiens

<400> 95

tcgattcagt cataggcgaa tctgttctgc ccgaggcttg tggtcaagca aaattcagc 60

cctgaaatc 69

<210> 96
<211> 69
<212> DNA
<213> Homo sapiens

<400> 96

tgcaattacg tccaccaaag acccgtgttg ggggtactga aggagaggcc ctgggggacc 60

ctctgaagc 69

<210> 97
<211> 71
<212> DNA
<213> Homo sapi ens

<400> 97

gggaaaactt gctgcggttg ctcaaggtga ggaataaaat gcttcccgtt cttctggatg 60

ggcatcctat c 71

<210> 98
<211> 69
<212> DNA
<213> Homo sapiens

<400> 98

atataaaaga tacacaccaa tatcttcttc aggctctgac aggcctcctg gaaacttcca 60

catatttt 69

<210> 99
<211> 70
<212> DNA
<213> Homo sapi ens

<400> 99

agctcagggc agtggatagc gagtacccca agaacatcaa agtctgggaa gggatccctg 60

agtctcccag 70

<210> 100
<211> 69
<212> DNA
<213> Homo sapi ens

<400> 100

gact gt ccct gaaacct agt act gcccccc at gaggaagg aacagcaat g gt gt cagt at 60

ccaggct t t 69

<210> 101
<211> 306
<212> PRT
<213> Homo sapi ens

<400> 101

Met Al a Al a Al a Al a Al a Al a Al a Al a Al a Al a Gl y Al a Al a Gl y Gl y
1 5 10 15

Arg Gl y Ser Gl y Pro Gl y Arg Arg Arg Hi s Leu Val Pro Gl y Al a Gl y
20 25 30

Gl y Gl u Al a Gl y Gl u Gl y Al a Pro Gl y Gl y Al a Gl y Asp Tyr Gl y Asn
35 40 45

Gl y Leu Gl u Ser Gl u Gl u Leu Gl u Pro Gl u Gl u Leu Leu Leu Gl u Pro
50 55 60

Gl u Pro Gl u Pro Gl u Pro Gl u Gl u Gl u Pro Pro Arg Pro Arg Al a Pro
65 70 75 80

Pro Gl y Al a Pro Gl y Pro Gl y Pro Gl y Ser Gl y Al a Pro Gl y Ser Gl n
85 90 95

Glu Glu Glu Glu Glu Pro Gly Leu Val Glu Gly Asp Pro Gly Asp Gly
100 105 110

Ala Ile Glu Asp Pro Glu Leu Glu Ala Ile Lys Ala Arg Val Arg Glu
115 120 125

Met Glu Glu Glu Ala Glu Lys Leu Lys Glu Leu Gln Asn Glu Val Glu
130 135 140

Lys Gln Met Asn Met Ser Pro Pro Pro Gly Asn Ala Gly Pro Val Ile
145 150 155 160

Met Ser Ile Glu Glu Lys Met Glu Ala Asp Ala Arg Ser Ile Tyr Val
165 170 175

Gly Asn Val Asp Tyr Gly Ala Thr Ala Glu Glu Leu Glu Ala His Phe
180 185 190

His Gly Cys Gly Ser Val Asn Arg Val Thr Ile Leu Cys Asp Lys Phe
195 200 205

Ser Gly His Pro Lys Gly Phe Ala Tyr Ile Glu Phe Ser Asp Lys Glu
210 215 220

Ser Val Arg Thr Ser Leu Ala Leu Asp Glu Ser Leu Phe Arg Gly Arg
225 230 235 240

Gln Ile Lys Val Ile Pro Lys Arg Thr Asn Arg Pro Gly Ile Ser Thr
245 250 255

Thr Asp Arg Gly Phe Pro Arg Ala Arg Tyr Arg Ala Arg Thr Thr Asn
260 265 270

Tyr Asn Ser Ser Arg Ser Arg Phe Tyr Ser Gly Phe Asn Ser Arg Pro
275 280 285

Arg Gly Arg Val Tyr Arg Gly Arg Ala Arg Ala Thr Ser Trp Tyr Ser
290 295 300

Pro Tyr
305

<210> 102
<211> 581
<212> PRT
<213> Homo sapiens

<400> 102

Met Ala Val Arg Gln Ala Leu Gly Arg Gly Leu Gln Leu Gly Arg Ala
1 5 10 15

Leu Leu Leu Arg Phe Thr Gly Lys Pro Gly Arg Ala Tyr Gly Leu Gly
20 25 30

Arg Pro Gly Pro Ala Ala Gly Cys Val Arg Gly Glu Arg Pro Gly Trp
35 40 45

Ala Ala Gly Pro Gly Ala Glu Pro Arg Arg Val Gly Leu Gly Leu Pro
50 55 60

Asn Arg Leu Arg Phe Phe Arg Gln Ser Val Ala Gly Leu Ala Ala Arg
65 70 75 80

Leu Gln Arg Gln Phe Val Val Arg Ala Trp Gly Cys Ala Gly Pro Cys
85 90 95

Gly Arg Ala Val Phe Leu Ala Phe Gly Leu Gly Leu Gly Leu Ile Glu
100 105 110

Glu Lys Gln Ala Glu Ser Arg Arg Ala Val Ser Ala Cys Gln Glu Ile
115 120 125

Gln Ala Ile Phe Thr Gln Lys Ser Lys Pro Gly Pro Asp Pro Leu Asp
130 135 140

Thr Arg Arg Leu Gln Gly Phe Arg Leu Glu Glu Tyr Leu Ile Gly Gln
145 150 155 160

Ser Ile Gly Lys Gly Cys Ser Ala Ala Val Tyr Glu Ala Thr Met Pro
165 170 175

Thr Leu Pro Gln Asn Leu Glu Val Thr Lys Ser Thr Gly Leu Leu Pro
180 185 190

Gly Arg Gly Pro Gly Thr Ser Ala Pro Gly Glu Gly Gln Glu Arg Ala
195 200 205

Pro Gly Ala Pro Ala Phe Pro Leu Ala Ile Lys Met Met Trp Asn Ile
210 215 220

Ser Ala Gly Ser Ser Ser Glu Ala Ile Leu Asn Thr Met Ser Gln Glu
225 230 235 240

Leu Val Pro Ala Ser Arg Val Ala Leu Ala Gly Glu Tyr Gly Ala Val
245 250 255

Thr Tyr Arg Lys Ser Lys Arg Gly Pro Lys Gln Leu Ala Pro His Pro
260 265 270

Asn Ile Ile Arg Val Leu Arg Ala Phe Thr Ser Ser Val Pro Leu Leu
275 280 285

Pro Gly Ala Leu Val Asp Tyr Pro Asp Val Leu Pro Ser Arg Leu His
290 295 300

Pro Glu Gly Leu Gly His Gly Arg Thr Leu Phe Leu Val Met Lys Asn
305 310 315 320

Tyr Pro Cys Thr Leu Arg Gln Tyr Leu Cys Val Asn Thr Pro Ser Pro
325 330 335

Arg Leu Ala Ala Met Met Leu Leu Gln Leu Leu Glu Gly Val Asp His
340 345 350

Leu Val Gln Gln Gly Ile Ala His Arg Asp Leu Lys Ser Asp Asn Ile
355 360 365

Leu Val Glu Leu Asp Pro Asp Gly Cys Pro Trp Leu Val Ile Ala Asp
370 375 380

Phe Gly Cys Cys Leu Ala Asp Glu Ser Ile Gly Leu Gln Leu Pro Phe
385 390 395 400

Ser Ser Trp Tyr Val Asp Arg Gly Gly Asn Gly Cys Leu Met Ala Pro
405 410 415

Glu Val Ser Thr Ala Arg Pro Gly Pro Arg Ala Val Ile Asp Tyr Ser
420 425 430

Lys Ala Asp Ala Trp Ala Val Gly Ala Ile Ala Tyr Glu Ile Phe Gly
435 440 445

Leu Val Asn Pro Phe Tyr Gly Gln Gly Lys Ala His Leu Glu Ser Arg
450 455 460

Ser Tyr Gln Glu Ala Gln Leu Pro Ala Leu Pro Glu Ser Val Pro Pro
465 470 475 480

Asp Val Arg Gln Leu Val Arg Ala Leu Leu Gln Arg Glu Ala Ser Lys
485 490 495

Arg Pro Ser Ala Arg Val Ala Ala Asn Val Leu His Leu Ser Leu Trp
500 505 510

Gly Glu His Ile Leu Ala Leu Lys Asn Leu Lys Leu Asp Lys Met Val
515 520 525

Gly Trp Leu Leu Gln Gln Ser Ala Ala Thr Leu Leu Ala Asn Arg Leu
530 535 540

Thr Glu Lys Cys Cys Val Glu Thr Lys Met Lys Met Leu Phe Leu Ala
545 550 555 560

Asn Leu Glu Cys Glu Thr Leu Cys Gln Ala Ala Leu Leu Leu Cys Ser
565 570 575

Trp Arg Ala Ala Leu
580

<210> 103
<211> 129
<212> PRT
<213> Homo sapiens

<400> 103

Met Gly Arg Arg Asp Ala Gln Leu Leu Ala Ala Leu Leu Val Leu Gly
1 5 10 15

Leu Cys Ala Leu Ala Gly Ser Glu Lys Pro Ser Pro Cys Gln Cys Ser
20 25 30

Arg Leu Ser Pro His Asn Arg Thr Asn Cys Gly Phe Pro Gly Ile Thr
35 40 45

Ser Asp Gln Cys Phe Asp Asn Gly Cys Cys Phe Asp Ser Ser Val Thr
50 55 60

Gly Val Pro Trp Cys Phe His Pro Leu Pro Lys Gln Glu Ser Asp Gln
65 70 75 80

Cys Val Met Glu Val Ser Asp Arg Arg Asn Cys Gly Tyr Pro Gly Ile
85 90 95

Ser Pro Glu Glu Cys Ala Ser Arg Lys Cys Cys Phe Ser Asn Phe Ile
100 105 110

Phe Glu Val Pro Trp Cys Phe Phe Pro Lys Ser Val Glu Asp Cys His
115 120 125

Tyr

<210> 104
<211> 814
<212> PRT
<213> Homo sapiens

<400> 104

Met Gln Asp Ala Glu Asn Val Ala Val Pro Glu Ala Ala Glu Glu Arg
1 5 10 15

Ala Glu Pro Gly Gln Gln Gln Pro Ala Ala Glu Pro Pro Pro Ala Glu
20 25 30

Gly Leu Leu Arg Pro Ala Gly Pro Gly Ala Pro Glu Ala Ala Gly Thr
35 40 45

Glu Ala Ser Ser Glu Glu Val Gly Ile Ala Glu Ala Gly Pro Glu Ser
50 55 60

Glu Val Arg Thr Glu Pro Ala Ala Glu Ala Glu Ala Ala Ser Gly Pro
65 70 75 80

Ser Glu Ser Pro Ser Pro Pro Ala Ala Glu Glu Leu Pro Gly Ser His
85 90 95

Ala Glu Pro Pro Val Pro Ala Gln Gly Glu Ala Pro Gly Glu Gln Ala
100 105 110

Arg Asp Glu Arg Ser Asp Ser Arg Ala Gln Ala Val Ser Glu Asp Ala
115 120 125

Gly Gly Asn Glu Gly Arg Ala Ala Glu Ala Glu Pro Arg Ala Leu Glu
130 135 140

Asn Gly Asp Ala Asp Glu Pro Ser Phe Ser Asp Pro Glu Asp Phe Val
145 150 155 160

Asp Asp Val Ser Glu Glu Glu Leu Leu Gly Asp Val Leu Lys Asp Arg
165 170 175

Pro Gln Glu Ala Asp Gly Ile Asp Ser Val Ile Val Val Asp Asn Val
180 185 190

Pro Gln Val Gly Pro Asp Arg Leu Glu Lys Leu Lys Asn Val Ile His
195 200 205

Lys Ile Phe Ser Lys Phe Gly Lys Ile Thr Asn Asp Phe Tyr Pro Glu
210 215 220

Glu Asp Gly Lys Thr Lys Gly Tyr Ile Phe Leu Glu Tyr Ala Ser Pro
225 230 235 240

Ala His Ala Val Asp Ala Val Lys Asn Ala Asp Gly Tyr Lys Leu Asp
245 250 255

Lys Gln His Thr Phe Arg Val Asn Leu Phe Thr Asp Phe Asp Lys Tyr
260 265 270

Met Thr Ile Ser Asp Glu Trp Asp Ile Pro Glu Lys Gln Pro Phe Lys
275 280 285

Asp Leu Gly Asn Leu Arg Tyr Trp Leu Glu Glu Ala Glu Cys Arg Asp
290 295 300

Gln Tyr Ser Val Ile Phe Glu Ser Gly Asp Arg Thr Ser Ile Phe Trp
305 310 315 320

Asn Asp Val Lys Asp Pro Val Ser Ile Glu Glu Arg Ala Arg Trp Thr
325 330 335

Glu Thr Tyr Val Arg Trp Ser Pro Lys Gly Thr Tyr Leu Ala Thr Phe
340 345 350

His Gln Arg Gly Ile Ala Leu Trp Gly Gly Glu Lys Phe Lys Gln Ile
355 360 365

Gln Arg Phe Ser His Gln Gly Val Gln Leu Ile Asp Phe Ser Pro Cys
370 375 380

Glu Arg Tyr Leu Val Thr Phe Ser Pro Leu Met Asp Thr Gln Asp Asp
385 390 395 400

Pro Gln Ala Ile Ile Ile Trp Asp Ile Leu Thr Gly His Lys Lys Arg
405 410 415

Gly Phe His Cys Glu Ser Ser Ala His Trp Pro Ile Phe Lys Trp Ser
420 425 430

His Asp Gly Lys Phe Phe Ala Arg Met Thr Leu Asp Thr Leu Ser Ile
435 440 445

Tyr Glu Thr Pro Ser Met Gly Leu Leu Asp Lys Lys Ser Leu Lys Ile
450 455 460

Ser Gly Ile Lys Asp Phe Ser Trp Ser Pro Gly Gly Asn Ile Ile Ala
465 470 475 480

Phe Trp Val Pro Glu Asp Lys Asp Ile Pro Ala Arg Val Thr Leu Met
485 490 495

Gln Leu Pro Thr Arg Gln Glu Ile Arg Val Arg Asn Leu Phe Asn Val
500 505 510

Val Asp Cys Lys Leu His Trp Gln Lys Asn Gly Asp Tyr Leu Cys Val
515 520 525

Lys Val Asp Arg Thr Pro Lys Gly Thr Gln Gly Val Val Thr Asn Phe
530 535 540

Glu Ile Phe Arg Met Arg Glu Lys Gln Val Pro Val Asp Val Val Glu
545 550 555 560

Met Lys Glu Thr Ile Ile Ala Phe Ala Trp Glu Pro Asn Gly Ser Lys
565 570 575

Phe Ala Val Leu His Gly Glu Ala Pro Arg Ile Ser Val Ser Phe Tyr
580 585 590

His Val Lys Asn Asn Gly Lys Ile Glu Leu Ile Lys Met Phe Asp Lys
595 600 605

Gln Gln Ala Asn Thr Ile Phe Trp Ser Pro Gln Gly Gln Phe Val Val
610 615 620

Leu Ala Gly Leu Arg Ser Met Asn Gly Ala Leu Ala Phe Val Asp Thr
625 630 635 640

Ser Asp Cys Thr Val Met Asn Ile Ala Glu His Tyr Met Ala Ser Asp
645 650 655

Val Glu Trp Asp Pro Thr Gly Arg Tyr Val Val Thr Ser Val Ser Trp
660 665 670

Trp Ser His Lys Val Asp Asn Ala Tyr Trp Leu Trp Thr Phe Gln Gly
675 680 685

Arg Leu Leu Gln Lys Asn Asn Lys Asp Arg Phe Cys Gln Leu Leu Trp
690 695 700

Arg Pro Arg Pro Pro Thr Leu Leu Ser Gln Glu Gln Ile Lys Gln Ile
705 710 715 720

Lys Lys Asp Leu Lys Lys Tyr Ser Lys Ile Phe Glu Gln Lys Asp Arg
725 730 735

Leu Ser Gln Ser Lys Ala Ser Lys Glu Leu Val Glu Arg Arg Arg Thr
740 745 750

Met Met Glu Asp Phe Arg Lys Tyr Arg Lys Met Ala Gln Glu Leu Tyr
755 760 765

Met Glu Gln Lys Asn Glu Arg Leu Glu Leu Arg Gly Gly Val Asp Thr
770 775 780

Asp Glu Leu Asp Ser Asn Val Asp Asp Trp Glu Glu Glu Thr Ile Glu
785 790 795 800

Phe Phe Val Thr Glu Glu Ile Ile Pro Leu Gly Asn Gln Glu
805 810

<210> 105
<211> 160
<212> PRT
<213> Homo sapiens

<400> 105

Met Ser Asp Asn Asp Asp Ile Glu Val Glu Ser Asp Glu Glu Gln Pro
1 5 10 15

Arg Phe Gln Ser Ala Ala Asp Lys Arg Ala His His Asn Ala Leu Glu
20 25 30

Arg Lys Arg Arg Asp His Ile Lys Asp Ser Phe His Ser Leu Arg Asp
35 40 45

Ser Val Pro Ser Leu Gln Gly Glu Lys Ala Ser Arg Ala Gln Ile Leu
50 55 60

Asp Lys Ala Thr Glu Tyr Ile Gln Tyr Met Arg Arg Lys Asn His Thr
65 70 75 80

His Gln Gln Asp Ile Asp Asp Leu Lys Arg Gln Asn Ala Leu Leu Glu
85 90 95

Gln Gln Val Arg Ala Leu Glu Lys Ala Arg Ser Ser Ala Gln Leu Gln
100 105 110

Thr Asn Tyr Pro Ser Ser Asp Asn Ser Leu Tyr Thr Asn Ala Lys Gly
115 120 125

Ser Thr Ile Ser Ala Phe Asp Gly Gly Ser Asp Ser Ser Ser Glu Ser
130 135 140

Glu Pro Glu Glu Pro Gln Ser Arg Lys Lys Leu Arg Met Glu Ala Ser
145 150 155 160

<210> 106
<211> 2715
<212> PRT
<213> Homo sapiens

<400> 106

Met Ala Ala Ala Ala Gly Gly Gly Ser Cys Pro Gly Pro Gly Ser Ala
1 5 10 15

Arg Gly Arg Phe Pro Gly Arg Pro Arg Gly Ala Gly Gly Gly Gly Gly
20 25 30

Arg Gly Gly Arg Gly Asn Gly Ala Glu Arg Val Arg Val Ala Leu Arg
35 40 45

Arg Gly Gly Gly Ala Thr Gly Pro Gly Gly Ala Glu Pro Gly Glu Asp
50 55 60

Thr Ala Leu Leu Arg Leu Leu Gly Leu Arg Arg Gly Leu Arg Arg Leu
65 70 75 80

Arg Arg Leu Trp Ala Gly Pro Arg Val Gln Arg Gly Arg Gly Arg Gly
85 90 95

Arg Gly Arg Gly Trp Gly Pro Ser Arg Gly Cys Val Pro Glu Glu Glu
100 105 110

Ser Ser Asp Gly Glu Ser Asp Glu Glu Glu Phe Gln Gly Phe His Ser
115 120 125

Asp Glu Asp Val Ala Pro Ser Ser Leu Arg Ser Ala Leu Arg Ser Gln
130 135 140

Arg Gly Arg Ala Pro Arg Gly Arg Gly Arg Lys His Lys Thr Thr Pro
145 150 155 160

Leu Pro Pro Pro Arg Leu Ala Asp Val Ala Pro Thr Pro Pro Lys Thr
165 170 175

Pro Ala Arg Lys Arg Gly Glu Glu Gly Thr Glu Arg Met Val Gln Ala
180 185 190

Leu Thr Glu Leu Leu Arg Arg Ala Gln Ala Pro Gln Ala Pro Arg Ser
195 200 205

Arg Ala Cys Glu Pro Ser Thr Pro Arg Arg Ser Arg Gly Arg Pro Pro
210 215 220

Gly Arg Pro Ala Gly Pro Cys Arg Arg Lys Gln Gln Ala Val Val Val
225 230 235 240

Ala Glu Ala Ala Val Thr Ile Pro Lys Pro Glu Pro Pro Pro Pro Val
245 250 255

Val Pro Val Lys His Gln Thr Gly Ser Trp Lys Cys Lys Glu Gly Pro
260 265 270

Gly Pro Gly Pro Gly Thr Pro Arg Arg Gly Gly Gln Ser Ser Arg Gly
275 280 285

Gly Arg Gly Gly Arg Gly Arg Gly Arg Gly Gly Gly Leu Pro Phe Val
290 295 300

Ile Lys Phe Val Ser Arg Ala Lys Lys Val Lys Met Gly Gln Leu Ser
305 310 315 320

Leu Gly Leu Glu Ser Gly Gln Gly Gln Gly Gln His Glu Glu Ser Trp
325 330 335

Gln Asp Val Pro Gln Arg Arg Val Gly Ser Gly Gln Gly Gly Ser Pro
340 345 350

Cys Trp Lys Lys Gln Glu Gln Lys Leu Asp Asp Glu Glu Glu Glu Lys
355 360 365

Lys Glu Glu Glu Glu Lys Asp Lys Glu Gly Glu Glu Lys Glu Glu Arg
370 375 380

Ala Val Ala Glu Glu Met Met Pro Ala Ala Glu Lys Glu Glu Ala Lys
385 390 395 400

Leu Pro Pro Pro Pro Leu Thr Pro Pro Ala Pro Ser Pro Pro Pro Pro
405 410 415

Leu Pro Pro Pro Ser Thr Ser Pro Pro Pro Pro Leu Cys Pro Pro Pro
420 425 430

Pro Pro Pro Val Ser Pro Pro Pro Leu Pro Ser Pro Pro Pro Pro Pro
435 440 445

Ala Gln Glu Glu Gln Glu Glu Ser Pro Pro Pro Val Val Pro Ala Thr
450 455 460

Cys Ser Arg Lys Arg Gly Arg Pro Pro Leu Thr Pro Ser Gln Arg Ala
465 470 475 480

Glu Arg Glu Ala Ala Arg Ala Gly Pro Glu Gly Thr Ser Pro Pro Thr
485 490 495

Pro Thr Pro Ser Thr Ala Thr Gly Gly Pro Pro Glu Asp Ser Pro Thr
500 505 510

Val Ala Pro Lys Ser Thr Thr Phe Leu Lys Asn Ile Arg Gln Phe Ile
515 520 525

Met Pro Val Val Ser Ala Arg Ser Ser Arg Val Ile Lys Thr Pro Arg
530 535 540

Arg Phe Met Asp Glu Asp Pro Pro Lys Pro Pro Lys Val Glu Val Ser
545 550 555 560

Pro Val Leu Arg Pro Pro Ile Thr Thr Ser Pro Pro Val Pro Gln Glu
565 570 575

Pro Ala Pro Val Pro Ser Pro Pro Arg Ala Pro Thr Pro Pro Ser Thr
580 585 590

Pro Val Pro Leu Pro Glu Lys Arg Arg Ser Ile Leu Arg Glu Pro Thr
595 600 605

Phe Arg Trp Thr Ser Leu Thr Arg Glu Leu Pro Pro Pro Pro Pro Ala
610 615 620

Pro Pro Pro Pro Pro Ala Pro Ser Pro Pro Pro Ala Pro Ala Thr Ser
625 630 635 640

Ser Arg Arg Pro Leu Leu Leu Arg Ala Pro Gln Phe Thr Pro Ser Glu
645 650 655

Ala His Leu Lys Ile Tyr Glu Ser Val Leu Thr Pro Pro Pro Leu Gly
660 665 670

Ala Pro Glu Ala Pro Glu Pro Glu Pro Pro Pro Ala Asp Asp Ser Pro
675 680 685

Ala Glu Pro Glu Pro Arg Ala Val Gly Arg Thr Asn His Leu Ser Leu
690 695 700

Pro Arg Phe Ala Pro Val Val Thr Thr Pro Val Lys Ala Glu Val Ser
705 710 715 720

Pro His Gly Ala Pro Ala Leu Ser Asn Gly Pro Gln Thr Gln Ala Gln
725 730 735

Leu Leu Gln Pro Leu Gln Ala Leu Gln Thr Gln Leu Leu Pro Gln Ala
740 745 750

Leu Pro Pro Pro Gln Pro Gln Leu Gln Pro Pro Pro Ser Pro Gln Gln
755 760 765

Met Pro Pro Leu Glu Lys Ala Arg Ile Ala Gly Val Gly Ser Leu Pro
770 775 780

Leu Ser Gly Val Glu Glu Lys Met Phe Ser Leu Leu Lys Arg Ala Lys
785 790 795 800

Val Gln Leu Phe Lys Ile Asp Gln Gln Gln Gln Gln Lys Val Ala Ala
805 810 815

Ser Met Pro Leu Ser Pro Gly Gly Gln Met Glu Glu Val Ala Gly Ala
820 825 830

Val Lys Gln Ile Ser Asp Arg Gly Pro Val Arg Ser Glu Asp Glu Ser
835 840 845

Val Glu Ala Lys Arg Glu Arg Pro Ser Gly Pro Glu Ser Pro Val Gln
850 855 860

Gly Pro Arg Ile Lys His Val Cys Arg His Ala Ala Val Ala Leu Gly
865 870 875 880

Gln Ala Arg Ala Met Val Pro Glu Asp Val Pro Arg Leu Ser Ala Leu
885 890 895

Pro Leu Arg Asp Arg Gln Asp Leu Ala Thr Glu Asp Thr Ser Ser Ala
900 905 910

Ser Glu Thr Glu Ser Val Pro Ser Arg Ser Arg Arg Gly Lys Val Glu
915 920 925

Ala Ala Gly Pro Gly Gly Glu Ser Glu Pro Thr Gly Ser Gly Gly Thr
930 935 940

Leu Ala His Thr Pro Arg Arg Ser Leu Pro Ser His His Gly Lys Lys
945 950 955 960

Met Arg Met Ala Arg Cys Gly His Cys Arg Gly Cys Leu Arg Val Gln
965 970 975

Asp Cys Gly Ser Cys Val Asn Cys Leu Asp Lys Pro Lys Phe Gly Gly
980 985 990

Pro Asn Thr Lys Lys Gln Cys Cys Val Tyr Arg Lys Cys Asp Lys Ile
995 1000 1005

Glu Ala Arg Lys Met Glu Arg Leu Ala Lys Lys Gly Arg Thr Ile
1010 1015 1020

Val Lys Thr Leu Leu Pro Trp Asp Ser Asp Glu Ser Pro Glu Ala
1025 1030 1035

Ser Pro Gly Pro Pro Gly Pro Arg Arg Gly Ala Gly Ala Gly Gly
1040 1045 1050

Pro Arg Glu Glu Val Val Ala His Pro Gly Pro Glu Glu Gln Asp
1055 1060 1065

Ser Leu Leu Gln Arg Lys Ser Ala Arg Arg Cys Val Lys Gln Arg
1070 1075 1080

Pro Ser Tyr Asp Ile Phe Glu Asp Ser Asp Asp Ser Glu Pro Gly
1085 1090 1095

Gly Pro Pro Ala Pro Arg Arg Arg Thr Pro Arg Glu Asn Glu Leu
1100 1105 1110

Pro Leu Pro Glu Pro Glu Glu Gln Ser Arg Pro Arg Lys Pro Thr
1115 1120 1125

Leu Gln Pro Val Leu Gln Leu Lys Ala Arg Arg Arg Leu Asp Lys
1130 1135 1140

Asp Ala Leu Ala Pro Gly Pro Phe Ala Ser Phe Pro Asn Gly Trp
1145 1150 1155

Thr Gly Lys Gln Lys Ser Pro Asp Gly Val His Arg Val Arg Val
1160 1165 1170

Asp Phe Lys Glu Asp Cys Asp Leu Glu Asn Val Trp Leu Met Gly
1175 1180 1185

Gly Leu Ser Val Leu Thr Ser Val Pro Gly Gly Pro Pro Met Val
1190 1195 1200

Cys Leu Leu Cys Ala Ser Lys Gly Leu His Glu Leu Val Phe Cys
1205 1210 1215

Gln Val Cys Cys Asp Pro Phe His Pro Phe Cys Leu Glu Glu Ala
1220 1225 1230

Glu Arg Pro Leu Pro Gln His His Asp Thr Trp Cys Cys Arg Arg
1235 1240 1245

Cys Lys Phe Cys His Val Cys Gly Arg Lys Gly Arg Gly Ser Lys
1250 1255 1260

His Leu Leu Glu Cys Glu Arg Cys Arg His Ala Tyr His Pro Ala
1265 1270 1275

Cys Leu Gly Pro Ser Tyr Pro Thr Arg Ala Thr Arg Lys Arg Arg
1280 1285 1290

His Trp Ile Cys Ser Ala Cys Val Arg Cys Lys Ser Cys Gly Ala
1295 1300 1305

Thr Pro Gly Lys Asn Trp Asp Val Glu Trp Ser Gly Asp Tyr Ser
1310 1315 1320

Leu Cys Pro Arg Cys Thr Gln Leu Tyr Glu Lys Gly Asn Tyr Cys
1325 1330 1335

Pro Ile Cys Thr Arg Cys Tyr Glu Asp Asn Asp Tyr Glu Ser Lys
1340 1345 1350

Met Met Gln Cys Ala Gln Cys Asp His Trp Val His Ala Lys Cys
1355 1360 1365

Glu Gly Leu Ser Asp Glu Asp Tyr Glu Ile Leu Ser Gly Leu Pro
1370 1375 1380

Asp Ser Val Leu Tyr Thr Cys Gly Pro Cys Ala Gly Ala Ala Gln
1385 1390 1395

Pro Arg Trp Arg Glu Ala Leu Ser Gly Ala Leu Gln Gly Gly Leu
1400 1405 1410

Arg Gln Val Leu Gln Gly Leu Leu Ser Ser Lys Val Val Gly Pro
1415 1420 1425

Leu Leu Leu Cys Thr Gln Cys Gly Pro Asp Gly Lys Gln Leu His
1430 1435 1440

Pro Gly Pro Cys Gly Leu Gln Ala Val Ser Gln Arg Phe Glu Asp
1445 1450 1455

Gly His Tyr Lys Ser Val His Ser Phe Met Glu Asp Met Val Gly
1460 1465 1470

Ile Leu Met Arg His Ser Glu Glu Gly Glu Thr Pro Asp Arg Arg
1475 1480 1485

Ala Gly Gly Gln Met Lys Gly Leu Leu Leu Lys Leu Leu Glu Ser
1490 1495 1500

Ala Phe Gly Trp Phe Asp Ala His Asp Pro Lys Tyr Trp Arg Arg
1505 1510 1515

Ser Thr Arg Leu Pro Asn Gly Val Leu Pro Asn Ala Val Leu Pro
1520 1525 1530

Pro Ser Leu Asp His Val Tyr Ala Gln Trp Arg Gln Gln Glu Pro
1535 1540 1545

Glu Thr Pro Glu Ser Gly Gln Pro Pro Gly Asp Pro Ser Ala Ala
1550 1555 1560

Phe Gln Gly Lys Asp Pro Ala Ala Phe Ser His Leu Glu Asp Pro
1565 1570 1575

Arg Gln Cys Ala Leu Cys Leu Lys Tyr Gly Asp Ala Asp Ser Lys
1580 1585 1590

Glu Ala Gly Arg Leu Leu Tyr Ile Gly Gln Asn Glu Trp Thr His
1595 1600 1605

Val Asn Cys Ala Ile Trp Ser Ala Glu Val Phe Glu Glu Asn Asp
1610 1615 1620

Gly Ser Leu Lys Asn Val His Ala Ala Val Ala Arg Gly Arg Gln
1625 1630 1635

Met Arg Cys Glu Leu Cys Leu Lys Pro Gly Ala Thr Val Gly Cys
1640 1645 1650

Cys Leu Ser Ser Cys Leu Ser Asn Phe His Phe Met Cys Ala Arg
1655 1660 1665

Ala Ser Tyr Cys Ile Phe Gln Asp Asp Lys Lys Val Phe Cys Gln
1670 1675 1680

Lys His Thr Asp Leu Leu Asp Gly Lys Glu Ile Val Asn Pro Asp
1685 1690 1695

Gly Phe Asp Val Leu Arg Arg Val Tyr Val Asp Phe Glu Gly Ile
1700 1705 1710

Asn Phe Lys Arg Lys Phe Leu Thr Gly Leu Glu Pro Asp Ala Ile
1715 1720 1725

Asn Val Leu Ile Gly Ser Ile Arg Ile Asp Ser Leu Gly Thr Leu
1730 1735 1740

Ser Asp Leu Ser Asp Cys Glu Gly Arg Leu Phe Pro Ile Gly Tyr
1745 1750 1755

Gln Cys Ser Arg Leu Tyr Trp Ser Thr Val Asp Ala Arg Arg Arg
1760 1765 1770

Cys Trp Tyr Arg Cys Arg Ile Leu Glu Tyr Arg Pro Trp Gly Pro
1775 1780 1785

Arg Glu Glu Pro Ala His Leu Glu Ala Ala Glu Glu Asn Gln Thr
1790 1795 1800

Ile Val His Ser Pro Ala Pro Ser Ser Glu Pro Pro Gly Gly Glu
1805 1810 1815

Asp Pro Pro Leu Asp Thr Asp Val Leu Val Pro Gly Ala Pro Glu
1820 1825 1830

Arg His Ser Pro Ile Gln Asn Leu Asp Pro Pro Leu Arg Pro Asp
1835 1840 1845

Ser Gly Ser Ala Pro Pro Pro Ala Pro Arg Ser Phe Ser Gly Ala
1850 1855 1860

Arg Ile Lys Val Pro Asn Tyr Ser Pro Ser Arg Arg Pro Leu Gly
1865 1870 1875

Gly Val Ser Phe Gly Pro Leu Pro Ser Pro Gly Ser Pro Ser Ser
1880 1885 1890

Leu Thr His His Ile Pro Thr Val Gly Asp Pro Asp Phe Pro Ala
1895 1900 1905

Pro Pro Arg Arg Ser Arg Arg Pro Ser Pro Leu Ala Pro Arg Pro
1910 1915 1920

Pro Pro Ser Arg Trp Ala Ser Pro Pro Leu Lys Thr Ser Pro Gln
1925 1930 1935

Leu Arg Val Pro Pro Pro Thr Ser Val Val Thr Ala Leu Thr Pro
1940 1945 1950

Thr Ser Gly Glu Leu Ala Pro Pro Gly Pro Ala Pro Ser Pro Pro
1955 1960 1965

Pro Pro Glu Asp Leu Gly Pro Asp Phe Glu Asp Met Glu Val Val
1970 1975 1980

Ser Gly Leu Ser Ala Ala Asp Leu Asp Phe Ala Ala Ser Leu Leu
1985 1990 1995

Gly Thr Glu Pro Phe Gln Glu Glu Ile Val Ala Ala Gly Ala Met
2000 2005 2010

Gly Ser Ser His Gly Gly Pro Gly Asp Ser Ser Glu Glu Glu Ser
2015 2020 2025

Ser Pro Thr Ser Arg Tyr Ile His Phe Pro Val Thr Val Val Ser
2030 2035 2040

Ala Pro Gly Leu Ala Pro Ser Ala Thr Pro Gly Ala Pro Arg Ile
2045 2050 2055

Glu Gln Leu Asp Gly Val Asp Asp Gly Thr Asp Ser Glu Ala Glu
2060 2065 2070

Ala Val Gln Gln Pro Arg Gly Gln Gly Thr Pro Pro Ser Gly Pro
2075 2080 2085

Gly Val Val Arg Ala Gly Val Leu Gly Ala Ala Gly Asp Arg Ala
2090 2095 2100

Arg Pro Pro Glu Asp Leu Pro Ser Glu Ile Val Asp Phe Val Leu
2105 2110 2115

Lys Asn Leu Gly Gly Pro Gly Asp Gly Gly Ala Gly Pro Arg Glu
2120 2125 2130

Glu Ser Leu Pro Pro Ala Pro Pro Leu Ala Asn Gly Ser Gln Pro
2135 2140 2145

Ser Gln Gly Leu Thr Ala Ser Pro Ala Asp Pro Thr Arg Thr Phe
2150 2155 2160

Ala Trp Leu Pro Gly Ala Pro Gly Val Arg Val Leu Ser Leu Gly
2165 2170 2175

Pro Ala Pro Glu Pro Pro Lys Pro Ala Thr Ser Lys Ile Ile Leu
2180 2185 2190

Val Asn Lys Leu Gly Gln Val Phe Val Lys Met Ala Gly Glu Gly
2195 2200 2205

Glu Pro Val Pro Pro Pro Val Lys Gln Pro Pro Leu Pro Pro Thr
2210 2215 2220

Ile Ser Pro Thr Ala Pro Thr Ser Trp Thr Leu Pro Pro Gly Pro
2225 2230 2235

Leu Leu Gly Val Leu Pro Val Val Gly Val Val Arg Pro Ala Pro
2240 2245 2250

Pro Pro Pro Pro Pro Pro Leu Thr Leu Val Leu Ser Ser Gly Pro
2255 2260 2265

Ala Ser Pro Pro Arg Gln Ala Ile Arg Val Lys Arg Val Ser Thr
2270 2275 2280

Phe Ser Gly Arg Ser Pro Pro Ala Pro Pro Pro Tyr Lys Ala Pro
2285 2290 2295

Arg Leu Asp Glu Asp Gly Glu Ala Ser Glu Asp Thr Pro Gln Val
2300 2305 2310

Pro Gly Leu Gly Ser Gly Gly Phe Ser Arg Val Arg Met Lys Thr
2315 2320 2325

Pro Thr Val Arg Gly Val Leu Asp Leu Asp Arg Pro Gly Glu Pro
2330 2335 2340

Ala Gly Glu Glu Ser Pro Gly Pro Leu Gln Glu Arg Ser Pro Leu
2345 2350 2355

Leu Pro Leu Pro Glu Asp Gly Pro Pro Gln Val Pro Asp Gly Pro
2360 2365 2370

Pro Asp Leu Leu Leu Glu Ser Gln Trp His His Tyr Ser Gly Glu
2375 2380 2385

Ala Ser Ser Ser Glu Glu Glu Pro Pro Ser Pro Asp Asp Lys Glu
2390 2395 2400

Asn Gln Ala Pro Lys Arg Thr Gly Pro His Leu Arg Phe Glu Ile
2405 2410 2415

Ser Ser Glu Asp Gly Phe Ser Val Glu Ala Glu Ser Leu Glu Gly
2420 2425 2430

Ala Trp Arg Thr Leu Ile Glu Lys Val Gln Glu Ala Arg Gly His
2435 2440 2445

Ala Arg Leu Arg His Leu Ser Phe Ser Gly Met Ser Gly Ala Arg
2450 2455 2460

Leu Leu Gly Ile His His Asp Ala Val Ile Phe Leu Ala Glu Gln
2465 2470 2475

Leu Pro Gly Ala Gln Arg Cys Gln His Tyr Lys Phe Arg Tyr His
2480 2485 2490

Gln Gln Gly Glu Gly Gln Glu Glu Pro Pro Leu Asn Pro His Gly
2495 2500 2505

Ala Ala Arg Ala Glu Val Tyr Leu Arg Lys Cys Thr Phe Asp Met
2510 2515 2520

Phe Asn Phe Leu Ala Ser Gln His Arg Val Leu Pro Glu Gly Ala
2525 2530 2535

Thr Cys Asp Glu Glu Glu Asp Glu Val Gln Leu Arg Ser Thr Arg
2540 2545 2550

Arg Ala Thr Ser Leu Glu Leu Pro Met Ala Met Arg Phe Arg His
2555 2560 2565

Leu Lys Lys Thr Ser Lys Glu Ala Val Gly Val Tyr Arg Ser Ala
2570 2575 2580

Ile His Gly Arg Gly Leu Phe Cys Lys Arg Asn Ile Asp Ala Gly
2585 2590 2595

Glu Met Val Ile Glu Tyr Ser Gly Ile Val Ile Arg Ser Val Leu
2600 2605 2610

Thr Asp Lys Arg Glu Lys Phe Tyr Asp Gly Lys Gly Ile Gly Cys
2615 2620 2625

Tyr Met Phe Arg Met Asp Asp Phe Asp Val Val Asp Ala Thr Met
2630 2635 2640

His Gly Asn Ala Ala Arg Phe Ile Asn His Ser Cys Glu Pro Asn
2645 2650 2655

Cys Phe Ser Arg Val Ile His Val Glu Gly Gln Lys His Ile Val
2660 2665 2670

Ile Phe Ala Leu Arg Arg Ile Leu Arg Gly Glu Glu Leu Thr Tyr
2675 2680 2685

Asp Tyr Lys Phe Pro Ile Glu Asp Ala Ser Asn Lys Leu Pro Cys
2690 2695 2700

Asn Cys Gly Ala Lys Arg Cys Arg Arg Phe Leu Asn
2705 2710 2715

<210> 107
<211> 605
<212> PRT
<213> Homo sapiens

<400> 107

Met Gln Cys Cys Gly Leu Val His Arg Arg Arg Val Arg Val Ser Tyr
1 5 10 15

Gly Ser Ala Asp Ser Tyr Thr Ser Arg Pro Ser Asp Ser Asp Val Ser
20 25 30

Leu Glu Glu Asp Arg Glu Ala Val Arg Arg Glu Ala Glu Arg Gln Ala
35 40 45

Gln Ala Gln Leu Glu Lys Ala Lys Thr Lys Pro Val Ala Phe Ala Val
50 55 60

Arg Thr Asn Val Ser Tyr Ser Ala Ala His Glu Asp Asp Val Pro Val
65 70 75 80

Pro Gly Met Ala Ile Ser Phe Glu Ala Lys Asp Phe Leu His Val Lys
85 90 95

Glu Lys Phe Asn Asn Asp Trp Trp Ile Gly Arg Leu Val Lys Glu Gly
100 105 110

Cys Glu Ile Gly Phe Ile Pro Ser Pro Val Lys Leu Glu Asn Met Arg
115 120 125

Leu Gln His Glu Gln Arg Ala Lys Gln Gly Lys Phe Tyr Ser Ser Lys
130 135 140

Ser Gly Gly Asn Ser Ser Ser Ser Leu Gly Asp Ile Val Pro Ser Ser
145 150 155 160

Arg Lys Ser Thr Pro Pro Ser Ser Ala Ile Asp Ile Asp Ala Thr Gly
165 170 175

Leu Asp Ala Glu Glu Asn Asp Ile Pro Ala Asn His Arg Ser Pro Lys
180 185 190

Pro Ser Ala Asn Ser Val Thr Ser Pro His Ser Lys Glu Lys Arg Met
195 200 205

Pro Phe Phe Lys Lys Thr Glu His Thr Pro Pro Tyr Asp Val Val Pro
210 215 220

Ser Met Arg Pro Val Val Leu Val Gly Pro Ser Leu Lys Gly Tyr Glu
225 230 235 240

Val Thr Asp Met Met Gln Lys Ala Leu Phe Asp Phe Leu Lys His Arg
245 250 255

Phe Glu Gly Arg Ile Ser Ile Thr Arg Val Thr Ala Asp Ile Ser Leu
260 265 270

Ala Lys Arg Ser Val Leu Asn Asn Pro Ser Lys His Ala Ile Ile Glu
275 280 285

Arg Ser Asn Thr Arg Ser Ser Leu Ala Glu Val Gln Ser Glu Ile Glu
290 295 300

Arg Ile Phe Glu Leu Ala Arg Thr Leu Gln Leu Val Val Leu Asp Ala
305 310 315 320

Asp Thr Ile Asn His Pro Ala Gln Leu Ser Lys Thr Ser Leu Ala Pro
325 330 335

Ile Ile Val Tyr Val Lys Ile Ser Ser Pro Lys Val Leu Gln Arg Leu
340 345 350

Ile Lys Ser Arg Gly Lys Ser Gln Ala Lys His Leu Asn Val Gln Met
355 360 365

Val Ala Ala Asp Lys Leu Ala Gln Cys Pro Pro Glu Leu Phe Asp Val
370 375 380

Ile Leu Asp Glu Asn Gln Leu Glu Asp Ala Cys Glu His Leu Ala Asp
385 390 395 400

Tyr Leu Glu Ala Tyr Trp Lys Ala Thr His Pro Pro Ser Ser Ser Leu
405 410 415

Pro Asn Pro Leu Leu Ser Arg Thr Leu Ala Thr Ser Ser Leu Pro Leu
420 425 430

Ser Pro Thr Leu Ala Ser Asn Ser Gln Gly Ser Gln Gly Asp Gln Arg
435 440 445

Thr Asp Arg Ser Ala Pro Ile Arg Ser Ala Ser Gln Ala Glu Glu Glu
450 455 460

Pro Ser Val Glu Pro Val Lys Lys Ser Gln His Arg Ser Ser Ser Ser
465 470 475 480

Ala Pro His His Asn His Arg Ser Gly Thr Ser Arg Gly Leu Ser Arg
485 490 495

Gln Glu Thr Phe Asp Ser Glu Thr Gln Glu Ser Arg Asp Ser Ala Tyr
500 505 510

Val Glu Pro Lys Glu Asp Tyr Ser His Asp His Val Asp His Tyr Ala
515 520 525

Ser His Arg Asp His Asn His Arg Asp Glu Thr His Gly Ser Ser Asp
530 535 540

His Arg His Arg Glu Ser Arg His Arg Ser Arg Asp Val Asp Arg Glu
545 550 555 560

Gln Asp His Asn Glu Cys Asn Lys Gln Arg Ser Arg His Lys Ser Lys
565 570 575

Asp Arg Tyr Cys Glu Lys Asp Gly Glu Val Ile Ser Lys Lys Arg Asn
580 585 590

Glu Ala Gly Glu Trp Asn Arg Asp Val Tyr Ile Pro Gln
595 600 605

<210> 108
<211> 562
<212> PRT
<213> Homo sapiens

<400> 108

Met Ser Arg Val His Gly Met His Pro Lys Glu Thr Thr Arg Gln Leu
1               5                   10                  15

Ser Leu Ala Val Lys Asp Gly Leu Ile Val Glu Thr Leu Thr Val Gly
            20                  25                  30

Cys Lys Gly Ser Lys Ala Gly Ile Glu Gln Glu Gly Tyr Trp Leu Pro
        35                  40                  45

Gly Asp Glu Ile Asp Trp Glu Thr Glu Asn His Asp Trp Tyr Cys Phe
    50                  55                  60

Glu Cys His Leu Pro Gly Glu Val Leu Ile Cys Asp Leu Cys Phe Arg
65                  70                  75                  80

Val Tyr His Ser Lys Cys Leu Ser Asp Glu Phe Arg Leu Arg Asp Ser
                85                  90                  95

Ser Ser Pro Trp Gln Cys Pro Val Cys Arg Ser Ile Lys Lys Lys Asn
            100                 105                 110

Thr Asn Lys Gln Glu Met Gly Thr Tyr Leu Arg Phe Ile Val Ser Arg
        115                 120                 125

Met Lys Glu Arg Ala Ile Asp Leu Asn Lys Lys Gly Lys Asp Asn Lys
    130                 135                 140

His Pro Met Tyr Arg Arg Leu Val His Ser Ala Val Asp Val Pro Thr
145                 150                 155                 160

Ile Gln Glu Lys Val Asn Glu Gly Lys Tyr Arg Ser Tyr Glu Glu Phe
                165                 170                 175

Lys Ala Asp Ala Gln Leu Leu Leu His Asn Thr Val Ile Phe Tyr Gly
            180                 185                 190

Ala Asp Ser Glu Gln Ala Asp Ile Ala Arg Met Leu Tyr Lys Asp Thr
        195                 200                 205

Cys His Glu Leu Asp Glu Leu Gln Leu Cys Lys Asn Cys Phe Tyr Leu
    210                 215                 220

Ser Asn Ala Arg Pro Asp Asn Trp Phe Cys Tyr Pro Cys Ile Pro Asn
225                 230                 235                 240

His Glu Leu Val Trp Ala Lys Met Lys Gly Phe Gly Phe Trp Pro Ala
                245                 250                 255

Lys Val Met Gln Lys Glu Asp Asn Gln Val Asp Val Arg Phe Phe Gly
            260                 265                 270

His His His Gln Arg Ala Trp Ile Pro Ser Glu Asn Ile Gln Asp Ile
275 280 285

Thr Val Asn Ile His Arg Leu His Val Lys Arg Ser Met Gly Trp Lys
290 295 300

Lys Ala Cys Asp Glu Leu Glu Leu His Gln Arg Phe Leu Arg Glu Gly
305 310 315 320

Arg Phe Trp Lys Ser Lys Asn Glu Asp Arg Gly Glu Glu Glu Ala Glu
325 330 335

Ser Ser Ile Ser Ser Thr Ser Asn Glu Gln Leu Lys Val Thr Gln Glu
340 345 350

Pro Arg Ala Lys Lys Gly Arg Arg Asn Gln Ser Val Glu Pro Lys Lys
355 360 365

Glu Glu Pro Glu Pro Glu Thr Glu Ala Val Ser Ser Ser Gln Glu Ile
370 375 380

Pro Thr Met Pro Gln Pro Ile Glu Lys Val Ser Val Ser Thr Gln Thr
385 390 395 400

Lys Lys Leu Ser Ala Ser Ser Pro Arg Met Leu His Arg Ser Thr Gln
405 410 415

Thr Thr Asn Asp Gly Val Cys Gln Ser Met Cys His Asp Lys Tyr Thr
420 425 430

Lys Ile Phe Asn Asp Phe Lys Asp Arg Met Lys Ser Asp His Lys Arg
435 440 445

Glu Thr Glu Arg Val Val Arg Glu Ala Leu Glu Lys Leu Arg Ser Glu
450 455 460

Met Glu Glu Glu Lys Arg Gln Ala Val Asn Lys Ala Val Ala Asn Met
465 470 475 480

Gln Gly Glu Met Asp Arg Lys Cys Lys Gln Val Lys Glu Lys Cys Lys
485 490 495

Glu Glu Phe Val Glu Glu Ile Lys Lys Leu Ala Thr Gln His Lys Gln
500 505 510

Leu Ile Ser Gln Thr Lys Lys Lys Gln Trp Cys Tyr Asn Cys Glu Glu
515 520 525

Glu Ala Met Tyr His Cys Cys Trp Asn Thr Ser Tyr Cys Ser Ile Lys
530 535 540

Cys Gln Gln Glu His Trp His Ala Glu His Lys Arg Thr Cys Arg Arg
545 550 555 560

Lys Arg

<210> 109
<211> 2145
<212> PRT
<213> Homo sapiens

<400> 109

Met Ser Asp Arg Ser Gly Pro Thr Ala Lys Gly Lys Asp Gly Lys Lys
1 5 10 15

Tyr Ser Ser Leu Asn Leu Phe Asp Thr Tyr Lys Gly Lys Ser Leu Glu
20 25 30

Ile Gln Lys Pro Ala Val Ala Pro Arg His Gly Leu Gln Ser Leu Gly
35 40 45

Lys Val Ala Ile Ala Arg Arg Met Pro Pro Pro Ala Asn Leu Pro Ser
50 55 60

Leu Lys Ala Glu Asn Lys Gly Asn Asp Pro Asn Val Ser Leu Val Pro
65 70 75 80

Lys Asp Gly Thr Gly Trp Ala Ser Lys Gln Glu Gln Ser Asp Pro Lys
85 90 95

Ser Ser Asp Ala Ser Thr Ala Gln Pro Pro Glu Ser Gln Pro Leu Pro
100 105 110

Ala Ser Gln Thr Pro Ala Ser Asn Gln Pro Lys Arg Pro Pro Ala Ala
115 120 125

Pro Glu Asn Thr Pro Leu Val Pro Ser Gly Val Lys Ser Trp Ala Gln
130 135 140

Ala Ser Val Thr His Gly Ala His Gly Asp Gly Gly Arg Ala Ser Ser
145 150 155 160

Leu Leu Ser Arg Phe Ser Arg Glu Glu Phe Pro Thr Leu Gln Ala Ala
165 170 175

Gly Asp Gln Asp Lys Ala Ala Lys Glu Arg Glu Ser Ala Glu Gln Ser
180 185 190

Ser Gly Pro Gly Pro Ser Leu Arg Pro Gln Asn Ser Thr Thr Trp Arg
195 200 205

Asp Gly Gly Gly Arg Gly Pro Asp Glu Leu Glu Gly Pro Asp Ser Lys
210 215 220

Leu His His Gly His Asp Pro Arg Gly Gly Leu Gln Pro Ser Gly Pro
225 230 235 240

Pro Gln Phe Pro Pro Tyr Arg Gly Met Met Pro Pro Phe Met Tyr Pro
245 250 255

Pro Tyr Leu Pro Phe Pro Pro Pro Tyr Gly Pro Gln Gly Pro Tyr Arg
260 265 270

Tyr Pro Thr Pro Asp Gly Pro Ser Arg Phe Pro Arg Val Ala Gly Pro
275 280 285

Arg Gly Ser Gly Pro Pro Met Arg Leu Val Glu Pro Val Gly Arg Pro
290 295 300

Ser Ile Leu Lys Glu Asp Asn Leu Lys Glu Phe Asp Gln Leu Asp Gln
305 310 315 320

Glu Asn Asp Asp Gly Trp Ala Gly Ala His Glu Glu Val Asp Tyr Thr
325 330 335

Glu Lys Leu Lys Phe Ser Asp Glu Glu Asp Gly Arg Asp Ser Asp Glu
340 345 350

Glu Gly Ala Glu Gly His Arg Asp Ser Gln Ser Ala Ser Gly Glu Glu
355 360 365

Arg Pro Pro Glu Ala Asp Gly Lys Lys Gly Asn Ser Pro Asn Ser Glu
370 375 380

Pro Pro Thr Pro Lys Thr Ala Trp Ala Glu Thr Ser Arg Pro Pro Glu
385 390 395 400

Thr Glu Pro Gly Pro Pro Ala Pro Lys Pro Pro Leu Pro Pro Gly Asp
405 410 415

Tyr Pro Asp Arg Gly Gly Pro Pro Cys Lys Pro Pro Ala Pro Glu Asp
420 425 430

Glu Asp Glu Ala Trp Arg Gln Arg Arg Lys Gln Ser Ser Ser Glu Ile
435 440 445

Ser Leu Ala Val Glu Arg Ala Arg Arg Arg Arg Glu Glu Glu Glu Arg
450 455 460

Arg Met Gln Glu Glu Arg Arg Ala Ala Cys Ala Glu Lys Leu Lys Arg
465 470 475 480

Leu Asp Glu Lys Phe Gly Ala Pro Asp Lys Arg Leu Lys Ala Glu Pro
485 490 495

Ala Ala Pro Pro Ala Ala Pro Ser Thr Pro Ala Pro Pro Pro Ala Val
500 505 510

Pro Lys Glu Leu Pro Ala Pro Pro Ala Pro Pro Pro Ala Ser Ala Pro
515 520 525

Thr Pro Glu Lys Glu Pro Glu Glu Pro Ala Gln Ala Pro Pro Ala Gln
530 535 540

Ser Thr Pro Thr Pro Gly Val Ala Ala Ala Pro Thr Leu Val Ser Gly
545 550 555 560

Gly Gly Ser Thr Ser Ser Thr Ser Ser Gly Ser Phe Glu Ala Ser Pro
565 570 575

Val Glu Pro Gln Leu Pro Ser Lys Glu Gly Pro Glu Pro Pro Glu Glu
580 585 590

Val Pro Pro Pro Thr Thr Pro Pro Val Pro Lys Val Glu Pro Lys Gly
595 600 605

Asp Gly Ile Gly Pro Thr Arg Gln Pro Pro Ser Gln Gly Leu Gly Tyr
610 615 620

Pro Lys Tyr Gln Lys Ser Leu Pro Pro Arg Phe Gln Arg Gln Gln Gln
625 630 635 640

Glu Gln Leu Leu Lys Gln Gln Gln Gln His Gln Trp Gln Gln His Gln
645 650 655

Gln Gly Ser Ala Pro Pro Thr Pro Val Pro Pro Ser Pro Pro Gln Pro
660 665 670

Val Thr Leu Gly Ala Val Pro Ala Pro Gln Ala Pro Pro Pro Pro Pro
675 680 685

Lys Ala Leu Tyr Pro Gly Ala Leu Gly Arg Pro Pro Pro Met Pro Pro
690 695 700

Met Asn Phe Asp Pro Arg Trp Met Met Ile Pro Pro Tyr Val Asp Pro
705 710 715 720

Arg Leu Leu Gln Gly Arg Pro Pro Leu Asp Phe Tyr Pro Pro Gly Val
725 730 735

His Pro Ser Gly Leu Val Pro Arg Glu Arg Ser Asp Ser Gly Gly Ser
740 745 750

Ser Ser Glu Pro Phe Asp Arg His Ala Pro Ala Met Leu Arg Glu Arg
755 760 765

Gly Thr Pro Pro Val Asp Pro Lys Leu Ala Trp Val Gly Asp Val Phe
770 775 780

Thr Ala Thr Pro Ala Glu Pro Arg Pro Leu Thr Ser Pro Leu Arg Gln
785 790 795 800

Ala Ala Asp Glu Asp Asp Lys Gly Met Arg Ser Glu Thr Pro Pro Val
805 810 815

Pro Pro Pro Pro Pro Tyr Leu Ala Ser Tyr Pro Gly Phe Pro Glu Asn
820 825 830

Gly Ala Pro Gly Pro Pro Ile Ser Arg Phe Pro Leu Glu Glu Pro Gly
835 840 845

Pro Arg Pro Leu Pro Trp Pro Pro Gly Ser Asp Glu Val Ala Lys Ile
850 855 860

Gln Thr Pro Pro Pro Lys Lys Glu Pro Pro Lys Glu Glu Thr Ala Gln
865 870 875 880

Leu Thr Gly Pro Glu Ala Gly Arg Lys Pro Ala Arg Gly Val Gly Ser
885 890 895

Gly Gly Gln Gly Pro Pro Pro Pro Arg Arg Glu Ser Arg Thr Glu Thr
900 905 910

Arg Trp Gly Pro Arg Pro Gly Ser Ser Arg Arg Gly Ile Pro Pro Glu
915 920 925

Glu Pro Gly Ala Pro Pro Arg Arg Ala Gly Pro Ile Lys Lys Pro Pro
930 935 940

Pro Pro Thr Lys Val Glu Glu Leu Pro Pro Lys Pro Leu Glu Gln Gly
945 950 955 960

Asp Glu Thr Pro Lys Pro Pro Lys Pro Asp Pro Leu Lys Ile Thr Lys
965 970 975

Gly Lys Leu Gly Gly Pro Lys Glu Thr Pro Pro Asn Gly Asn Leu Ser
980 985 990

Pro Ala Pro Arg Leu Arg Arg Asp Tyr Ser Tyr Glu Arg Val Gly Pro
995 1000 1005

Thr Ser Cys Arg Gly Arg Gly Arg Gly Glu Tyr Phe Ala Arg Gly
1010 1015 1020

Arg Gly Phe Arg Gly Thr Tyr Gly Gly Arg Gly Arg Gly Ala Arg
1025 1030 1035

Ser Arg Glu Phe Arg Ser Tyr Arg Glu Phe Arg Gly Asp Asp Gly
1040 1045 1050

Arg Gly Gly Gly Thr Gly Gly Pro Asn His Pro Pro Ala Pro Arg
1055 1060 1065

Gly Arg Thr Ala Ser Glu Thr Arg Ser Glu Gly Ser Glu Tyr Glu
1070 1075 1080

Glu Ile Pro Lys Arg Arg Arg Gln Arg Gly Ser Glu Thr Gly Ser
1085 1090 1095

Glu Thr His Glu Ser Asp Leu Ala Pro Ser Asp Lys Glu Ala Pro
1100 1105 1110

Thr Pro Lys Glu Gly Thr Leu Thr Gln Val Pro Leu Ala Pro Pro
1115 1120 1125

Pro Pro Gly Ala Pro Pro Ser Pro Ala Pro Ala Arg Phe Thr Ala
1130 1135 1140

Arg Gly Gly Arg Val Phe Thr Pro Arg Gly Val Pro Ser Arg Arg
1145 1150 1155

Gly Arg Gly Gly Gly Arg Pro Pro Pro Gln Val Cys Pro Gly Trp
1160 1165 1170

Ser Pro Pro Ala Lys Ser Leu Ala Pro Lys Lys Pro Pro Thr Gly
1175 1180 1185

Pro Leu Pro Pro Ser Lys Glu Pro Leu Lys Glu Lys Leu Ile Pro
1190 1195 1200

Gly Pro Leu Ser Pro Val Ala Arg Gly Gly Ser Asn Gly Gly Ser
1205 1210 1215

Asn Val Gly Met Glu Asp Gly Glu Arg Pro Arg Arg Arg Arg His
1220 1225 1230

Gly Arg Ala Gln Gln Gln Asp Lys Pro Pro Arg Phe Arg Arg Leu
1235 1240 1245

Lys Gln Glu Arg Glu Asn Ala Ala Arg Gly Ser Glu Gly Lys Pro
1250 1255 1260

Ser Leu Thr Leu Pro Ala Ser Ala Pro Ala Pro Glu Glu Ala Leu
1265 1270 1275

Thr Thr Val Thr Val Ala Pro Ala Pro Arg Arg Ala Ala Ala Lys
1280 1285 1290

Ser Pro Asp Leu Ser Asn Gln Asn Ser Asp Gln Ala Asn Glu Glu
1295 1300 1305

Trp Glu Thr Ala Ser Glu Ser Ser Asp Phe Thr Ser Glu Arg Arg
1310 1315 1320

Gly Asp Lys Glu Ala Pro Pro Pro Val Leu Leu Thr Pro Lys Ala
1325 1330 1335

Val Gly Thr Pro Gly Gly Gly Gly Gly Gly Ala Val Pro Gly Ile
1340 1345 1350

Ser Ala Met Ser Arg Gly Asp Leu Ser Gln Arg Ala Lys Asp Leu
1355 1360 1365

Ser Lys Arg Ser Phe Ser Ser Gln Arg Pro Gly Met Glu Arg Gln
1370 1375 1380

Asn Arg Arg Pro Gly Pro Gly Gly Lys Ala Gly Ser Ser Gly Ser
1385 1390 1395

Ser Ser Gly Gly Gly Gly Gly Gly Pro Gly Gly Arg Thr Gly Pro
1400 1405 1410

Gly Arg Gly Asp Lys Arg Ser Trp Pro Ser Pro Lys Asn Arg Ser
1415 1420 1425

Arg Pro Pro Glu Glu Arg Pro Pro Gly Leu Pro Leu Pro Pro Pro
1430 1435 1440

Pro Pro Ser Ser Ser Ala Val Phe Arg Leu Asp Gln Val Ile His
1445 1450 1455

Ser Asn Pro Ala Gly Ile Gln Gln Ala Leu Ala Gln Leu Ser Ser
1460 1465 1470

Arg Gln Gly Ser Val Thr Ala Pro Gly Gly His Pro Arg His Lys
1475 1480 1485

Pro Gly Pro Pro Gln Ala Pro Gln Gly Pro Ser Pro Arg Pro Pro
1490 1495 1500

Thr Arg Tyr Glu Pro Gln Arg Val Asn Ser Gly Leu Ser Ser Asp
1505 1510 1515

Pro His Phe Glu Glu Pro Gly Pro Met Val Arg Gly Val Gly Gly
1520 1525 1530

Thr Pro Arg Asp Ser Ala Gly Val Ser Pro Phe Pro Pro Lys Arg
1535 1540 1545

Arg Glu Arg Pro Pro Arg Lys Pro Glu Leu Leu Gln Glu Glu Ser
1550 1555 1560

Leu Pro Pro Pro His Ser Ser Gly Phe Leu Gly Ser Lys Pro Glu
1565 1570 1575

Gly Pro Gly Pro Gln Ala Glu Ser Arg Asp Thr Gly Thr Glu Ala
1580 1585 1590

Leu Thr Pro His Ile Trp Asn Arg Leu His Thr Ala Thr Ser Arg
1595 1600 1605

Lys Ser Tyr Arg Pro Ser Ser Met Glu Pro Trp Met Glu Pro Leu
1610 1615 1620

Ser Pro Phe Glu Asp Val Ala Gly Thr Glu Met Ser Gln Ser Asp
1625 1630 1635

Ser Gly Val Asp Leu Ser Gly Asp Ser Gln Val Ser Ser Gly Pro
1640 1645 1650

Cys Ser Gln Arg Ser Ser Pro Asp Gly Gly Leu Lys Gly Ala Ala
1655 1660 1665

Glu Gly Pro Pro Lys Arg Pro Gly Gly Ser Ser Pro Leu Asn Ala
1670 1675 1680

Val Pro Cys Glu Gly Pro Pro Gly Ser Glu Pro Pro Arg Arg Pro
1685 1690 1695

Pro Pro Ala Pro His Asp Gly Asp Arg Lys Glu Leu Pro Arg Glu
1700 1705 1710

Gln Pro Leu Pro Pro Gly Pro Ile Gly Thr Glu Arg Ser Gln His
1715 1720 1725

Thr Asp Arg Gly Thr Glu Pro Gly Pro Ile Arg Pro Ser His Arg
1730 1735 1740

Pro Gly Pro Pro Val Gln Phe Gly Thr Ser Asp Lys Asp Ser Asp
1745 1750 1755

Leu Arg Leu Val Val Gly Asp Ser Leu Lys Ala Glu Lys Glu Leu
1760 1765 1770

Thr Ala Ser Val Thr Glu Ala Ile Pro Val Ser Arg Asp Trp Glu
1775 1780 1785

Leu Leu Pro Ser Ala Ala Ala Ser Ala Glu Pro Gln Ser Lys Asn
1790 1795 1800

Leu Asp Ser Gly His Cys Val Pro Glu Pro Ser Ser Ser Gly Gln
1805 1810 1815

Arg Leu Tyr Pro Glu Val Phe Tyr Gly Ser Ala Gly Pro Ser Ser
1820 1825 1830

Ser Gln Ile Ser Gly Gly Ala Met Asp Ser Gln Leu His Pro Asn
1835 1840 1845

Ser Gly Gly Phe Arg Pro Gly Thr Pro Ser Leu His Pro Tyr Arg
1850 1855 1860

Ser Gln Pro Leu Tyr Leu Pro Pro Gly Pro Ala Pro Pro Ser Ala
1865 1870 1875

Leu Leu Ser Gly Val Ala Leu Lys Gly Gln Phe Leu Asp Phe Ser
1880 1885 1890

Thr Met Gln Ala Thr Glu Leu Gly Lys Leu Pro Ala Gly Gly Val
1895 1900 1905

Leu Tyr Pro Pro Pro Ser Phe Leu Tyr Ser Pro Ala Phe Cys Pro
1910 1915 1920

Ser Pro Leu Pro Asp Thr Ser Leu Leu Gln Val Arg Gln Asp Leu
1925 1930 1935

Pro Ser Pro Ser Asp Phe Tyr Ser Thr Pro Leu Gln Pro Gly Gly
1940 1945 1950

Gln Ser Gly Phe Leu Pro Ser Gly Ala Pro Ala Gln Gln Met Leu
1955 1960 1965

Leu Pro Met Val Asp Ser Gln Leu Pro Val Val Asn Phe Gly Ser
1970 1975 1980

Leu Pro Pro Ala Pro Pro Pro Ala Pro Pro Pro Leu Ser Leu Leu
1985 1990 1995

Pro Val Gly Pro Ala Leu Gln Pro Pro Ser Leu Ala Val Arg Pro
2000 2005 2010

Pro Pro Ala Pro Ala Thr Arg Val Leu Pro Ser Pro Ala Arg Pro
2015 2020 2025

Phe Pro Ala Ser Leu Gly Arg Ala Glu Leu His Pro Val Glu Leu
2030 2035 2040

Lys Pro Phe Gln Asp Tyr Gln Lys Leu Ser Ser Asn Leu Gly Gly
2045 2050 2055

Pro Gly Ser Ser Arg Thr Pro Pro Thr Gly Arg Ser Phe Ser Gly
2060 2065 2070

Leu Asn Ser Arg Leu Lys Ala Thr Pro Ser Thr Tyr Ser Gly Val
2075 2080 2085

Phe Arg Thr Gln Arg Val Asp Leu Tyr Gln Gln Ala Ser Pro Pro
2090 2095 2100

Asp Ala Leu Arg Trp Ile Pro Lys Pro Trp Glu Arg Thr Gly Leu
2105 2110 2115

Pro Pro Arg Glu Gly Pro Ser Arg Arg Ala Glu Glu Pro Gly Ser
2120 2125 2130

Arg Gly Asp Lys Glu Pro Gly Leu Pro Pro Pro Arg
2135 2140 2145

<210> 110
<211> 535
<212> PRT
<213> Homo sapiens

<400> 110

Met Ser Glu Gly Glu Ser Gln Thr Val Leu Ser Ser Gly Ser Asp Pro
1 5 10 15

Lys Val Glu Ser Ser Ser Ser Ala Pro Gly Leu Thr Ser Val Ser Pro
20 25 30

Pro Val Thr Ser Thr Thr Ser Ala Ala Ser Pro Glu Glu Glu Glu Glu
35 40 45

Ser Glu Asp Glu Ser Glu Ile Leu Glu Glu Ser Pro Cys Gly Arg Trp
50 55 60

Gln Lys Arg Arg Glu Glu Val Asn Gln Arg Asn Val Pro Gly Ile Asp
65 70 75 80

Ser Ala Tyr Leu Ala Met Asp Thr Glu Glu Gly Val Glu Val Val Trp
85 90 95

Asn Glu Val Gln Phe Ser Glu Arg Lys Asn Tyr Lys Leu Gln Glu Glu
100 105 110

Lys Val Arg Ala Val Phe Asp Asn Leu Ile Gln Leu Glu His Leu Asn
115 120 125

Ile Val Lys Phe His Lys Tyr Trp Ala Asp Ile Lys Glu Asn Lys Ala
130 135 140

Arg Val Ile Phe Ile Thr Glu Tyr Met Ser Ser Gly Ser Leu Lys Gln
145 150 155 160

Phe Leu Lys Lys Thr Lys Lys Asn His Lys Thr Met Asn Glu Lys Ala
165 170 175

Trp Lys Arg Trp Cys Thr Gln Ile Leu Ser Ala Leu Ser Tyr Leu His
180 185 190

Ser Cys Asp Pro Pro Ile Ile His Gly Asn Leu Thr Cys Asp Thr Ile
195 200 205

Phe Ile Gln His Asn Gly Leu Ile Lys Ile Gly Ser Val Ala Pro Asp
210 215 220

Thr Ile Asn Asn His Val Lys Thr Cys Arg Glu Glu Gln Lys Asn Leu
225 230 235 240

His Phe Phe Ala Pro Glu Tyr Gly Glu Val Thr Asn Val Thr Thr Ala
245 250 255

Val Asp Ile Tyr Ser Phe Gly Met Cys Ala Leu Glu Met Ala Val Leu
260 265 270

Glu Ile Gln Gly Asn Gly Glu Ser Ser Tyr Val Pro Gln Glu Ala Ile
275 280 285

Ser Ser Ala Ile Gln Leu Leu Glu Asp Pro Leu Gln Arg Glu Phe Ile
290 295 300

Gln Lys Cys Leu Gln Ser Glu Pro Ala Arg Arg Pro Thr Ala Arg Glu
305 310 315 320

Leu Leu Phe His Pro Ala Leu Phe Glu Val Pro Ser Leu Lys Leu Leu
325 330 335

Ala Ala His Cys Ile Val Gly His Gln His Met Ile Pro Glu Asn Ala
340 345 350

Leu Glu Glu Ile Thr Lys Asn Met Asp Thr Ser Ala Val Leu Ala Glu
355 360 365

Ile Pro Ala Gly Pro Gly Arg Glu Pro Val Gln Thr Leu Tyr Ser Gln
370 375 380

Ser Pro Ala Leu Glu Leu Asp Lys Phe Leu Glu Asp Val Arg Asn Gly
385 390 395 400

Ile Tyr Pro Leu Thr Ala Phe Gly Leu Pro Arg Pro Gln Gln Pro Gln
405 410 415

Gln Glu Glu Val Thr Ser Pro Val Val Pro Pro Ser Val Lys Thr Pro
420 425 430

Thr Pro Glu Pro Ala Glu Val Glu Thr Arg Lys Val Val Leu Met Gln
435 440 445

Cys Asn Ile Glu Ser Val Glu Glu Gly Val Lys His His Leu Thr Leu
450 455 460

Leu Leu Lys Leu Glu Asp Lys Leu Asn Arg His Leu Ser Cys Asp Leu
465 470 475 480

Met Pro Asn Glu Asn Ile Pro Glu Leu Ala Ala Glu Leu Val Gln Leu
485 490 495

Gly Phe Ile Ser Glu Ala Asp Gln Ser Arg Leu Thr Ser Leu Leu Glu
500 505 510

Glu Thr Leu Asn Lys Phe Asn Phe Ala Arg Asn Ser Thr Leu Asn Ser
515 520 525

Ala Ala Val Thr Val Ser Ser
530 535

<210> 111
<211> 1980
<212> PRT
<213> Homo sapiens

<400> 111

Met Asn Pro Thr Asn Pro Phe Ser Gly Gln Gln Pro Ser Ala Phe Ser
1 5 10 15

Ala Ser Ser Ser Asn Val Gly Thr Leu Pro Ser Lys Pro Pro Phe Arg
20 25 30

Phe Gly Gln Pro Ser Leu Phe Gly Gln Asn Ser Thr Leu Ser Gly Lys
35 40 45

Ser Ser Gly Phe Ser Gln Val Ser Ser Phe Pro Ala Ser Ser Gly Val
50 55 60

Ser His Ser Ser Ser Val Gln Thr Leu Gly Phe Thr Gln Thr Ser Ser
65 70 75 80

Val Gly Pro Phe Ser Gly Leu Glu His Thr Ser Thr Phe Val Ala Thr
85 90 95

Ser Gly Pro Ser Ser Ser Ser Val Leu Gly Asn Thr Gly Phe Ser Phe
100 105 110

Lys Ser Pro Thr Ser Val Gly Ala Phe Pro Ser Thr Ser Ala Phe Gly
115 120 125

Gln Glu Ala Gly Glu Ile Val Asn Ser Gly Phe Gly Lys Thr Glu Phe
130 135 140

Ser Phe Lys Pro Leu Glu Asn Ala Val Phe Lys Pro Ile Leu Gly Ala
145 150 155 160

Glu Ser Glu Pro Glu Lys Thr Gln Ser Gln Ile Ala Ser Gly Phe Phe
165 170 175

Thr Phe Ser His Pro Ile Ser Ser Ala Pro Gly Gly Leu Ala Pro Phe
180 185 190

Ser Phe Pro Gln Val Thr Ser Ser Ser Ala Thr Thr Ser Asn Phe Thr
195 200 205

Phe Ser Lys Pro Val Ser Ser Asn Asn Ser Leu Ser Ala Phe Thr Pro
210 215 220

Ala Leu Ser Asn Gln Asn Val Glu Glu Glu Lys Arg Gly Pro Lys Ser
225 230 235 240

Ile Phe Gly Ser Ser Asn Asn Ser Phe Ser Ser Phe Pro Val Ser Ser
245 250 255

Ala Val Leu Gly Glu Pro Phe Gln Ala Ser Lys Ala Gly Val Arg Gln
260 265 270

Gly Cys Glu Glu Ala Val Ser Gln Val Glu Pro Leu Pro Ser Leu Met
275 280 285

Lys Gly Leu Lys Arg Lys Glu Asp Gln Asp Arg Ser Pro Arg Arg His
290 295 300

Gly His Glu Pro Ala Glu Asp Ser Asp Pro Leu Ser Arg Gly Asp His
305 310 315 320

Pro Pro Asp Lys Arg Pro Val Arg Leu Asn Arg Pro Arg Gly Gly Thr
325 330 335

Leu Phe Gly Arg Thr Ile Gln Asp Val Phe Lys Ser Asn Lys Glu Val
340 345 350

Gly Arg Leu Gly Asn Lys Glu Ala Lys Lys Glu Thr Gly Phe Val Glu
355 360 365

Ser Ala Glu Ser Asp His Met Ala Ile Pro Gly Gly Asn Gln Ser Val
370 375 380

Leu Ala Pro Ser Arg Ile Pro Gly Val Asn Lys Glu Glu Glu Thr Glu
385 390 395 400

Ser Arg Glu Lys Lys Glu Asp Ser Leu Arg Gly Thr Pro Ala Arg Gln
405 410 415

Ser Asn Arg Ser Glu Ser Thr Asp Ser Leu Gly Gly Leu Ser Pro Ser
420 425 430

Glu Val Thr Ala Ile Gln Cys Lys Asn Ile Pro Asp Tyr Leu Asn Asp
435 440 445

Arg Thr Ile Leu Glu Asn His Phe Gly Lys Ile Ala Lys Val Gln Arg
450 455 460

Ile Phe Thr Arg Arg Ser Lys Lys Leu Ala Val Val His Phe Phe Asp
465 470 475 480

His Ala Ser Ala Ala Leu Ala Arg Lys Lys Gly Lys Ser Leu His Lys
485 490 495

Asp Met Ala Ile Phe Trp His Arg Lys Lys Ile Ser Pro Asn Lys Lys
500 505 510

Pro Phe Ser Leu Lys Glu Lys Lys Pro Gly Asp Gly Glu Val Ser Pro
515 520 525

Ser Thr Glu Asp Ala Pro Phe Gln His Ser Pro Leu Gly Lys Ala Ala
530 535 540

Gly Arg Thr Gly Ala Ser Ser Leu Leu Asn Lys Ser Ser Pro Val Lys
545 550 555 560

Lys Pro Ser Leu Leu Lys Ala His Gln Phe Glu Gly Asp Ser Phe Asp
565 570 575

Ser Ala Ser Glu Gly Ser Glu Gly Leu Gly Pro Cys Val Leu Ser Leu
580 585 590

Ser Thr Leu Ile Gly Thr Val Ala Glu Thr Ser Lys Glu Lys Tyr Arg
595 600 605

Leu Leu Asp Gln Arg Asp Arg Ile Met Arg Gln Ala Arg Val Lys Arg
610 615 620

Thr Asp Leu Asp Lys Ala Arg Thr Phe Val Gly Thr Cys Leu Asp Met
625 630 635 640

Cys Pro Glu Lys Glu Arg Tyr Met Arg Glu Thr Arg Ser Gln Leu Ser
645 650 655

Val Phe Glu Val Val Pro Gly Thr Asp Gln Val Asp His Ala Ala Ala
660 665 670

Val Lys Glu Tyr Ser Arg Ser Ser Ala Asp Gln Glu Glu Pro Leu Pro
675 680 685

His Glu Leu Arg Pro Leu Pro Val Leu Ser Arg Thr Met Asp Tyr Leu
690 695 700

Val Thr Gln Ile Met Asp Gln Lys Glu Gly Ser Leu Arg Asp Trp Tyr
705 710 715 720

Asp Phe Val Trp Asn Arg Thr Arg Gly Ile Arg Lys Asp Ile Thr Gln
725 730 735

Gln His Leu Cys Asp Pro Leu Thr Val Ser Leu Ile Glu Lys Cys Thr
740 745 750

Arg Phe His Ile His Cys Ala His Phe Met Cys Glu Glu Pro Met Ser
755 760 765

Ser Phe Asp Ala Lys Ile Asn Asn Glu Asn Met Thr Lys Cys Leu Gln
770 775 780

Ser Leu Lys Glu Met Tyr Gln Asp Leu Arg Asn Lys Gly Val Phe Cys
785 790 795 800

Ala Ser Glu Ala Glu Phe Gln Gly Tyr Asn Val Leu Leu Ser Leu Asn
805 810 815

Lys Gly Asp Ile Leu Arg Glu Val Gln Gln Phe His Pro Ala Val Arg
820 825 830

Asn Ser Ser Glu Val Lys Phe Ala Val Gln Ala Phe Ala Ala Leu Asn
835 840 845

Ser Asn Asn Phe Val Arg Phe Phe Lys Leu Val Gln Ser Ala Ser Tyr
850 855 860

Leu Asn Ala Cys Leu Leu His Cys Tyr Phe Ser Gln Ile Arg Lys Asp
865 870 875 880

Ala Leu Arg Ala Leu Asn Phe Ala Tyr Thr Val Ser Thr Gln Arg Ser
885 890 895

Thr Ile Phe Pro Leu Asp Gly Val Val Arg Met Leu Leu Phe Arg Asp
900 905 910

Cys Glu Glu Ala Thr Asp Phe Leu Thr Cys His Gly Leu Thr Val Ser
915 920 925

Asp Gly Cys Val Glu Leu Asn Arg Ser Ala Phe Leu Glu Pro Glu Gly
930 935 940

Leu Ser Lys Thr Arg Lys Ser Val Phe Ile Thr Arg Lys Leu Thr Val
945 950 955 960

Ser Val Gly Glu Ile Val Asn Gly Gly Pro Leu Pro Pro Val Pro Arg
965 970 975

His Thr Pro Val Cys Ser Phe Asn Ser Gln Asn Lys Tyr Ile Gly Glu
980 985 990

Ser Leu Ala Ala Glu Leu Pro Val Ser Thr Gln Arg Pro Gly Ser Asp
995 1000 1005

Thr Val Gly Gly Gly Arg Gly Glu Glu Cys Gly Val Glu Pro Asp
1010 1015 1020

Ala Pro Leu Ser Ser Leu Pro Gln Ser Leu Pro Ala Pro Ala Pro
1025 1030 1035

Ser Pro Val Pro Leu Pro Pro Val Leu Ala Leu Thr Pro Ser Val
1040 1045 1050

Ala Pro Ser Leu Phe Gln Leu Ser Val Gln Pro Glu Pro Pro Pro
1055 1060 1065

Pro Glu Pro Val Pro Met Tyr Ser Asp Glu Asp Leu Ala Gln Val
1070 1075 1080

Val Asp Glu Leu Ile Gln Glu Ala Leu Gln Arg Asp Cys Glu Glu
1085 1090 1095

Val Gly Ser Ala Gly Ala Ala Tyr Ala Ala Ala Ala Leu Gly Val
1100 1105 1110

Ser Asn Ala Ala Met Glu Asp Leu Leu Thr Ala Ala Thr Thr Gly
1115 1120 1125

Ile Leu Arg His Ile Ala Ala Glu Glu Val Ser Lys Glu Arg Glu
1130 1135 1140

Arg Arg Glu Gln Glu Arg Gln Arg Ala Glu Glu Glu Arg Leu Lys
1145 1150 1155

Gln Glu Arg Glu Leu Val Leu Ser Glu Leu Ser Gln Gly Leu Ala
1160 1165 1170

Val Glu Leu Met Glu Arg Val Met Met Glu Phe Val Arg Glu Thr
1175 1180 1185

Cys Ser Gln Glu Leu Lys Asn Ala Val Glu Thr Asp Gln Arg Val
1190 1195 1200

Arg Val Ala Arg Cys Cys Glu Asp Val Cys Ala His Leu Val Asp
1205 1210 1215

Leu Phe Leu Val Glu Glu Ile Phe Gln Thr Ala Lys Glu Thr Leu
1220 1225 1230

Gln Glu Leu Gln Cys Phe Cys Lys Tyr Leu Gln Arg Trp Arg Glu
1235 1240 1245

Ala Val Thr Ala Arg Lys Lys Leu Arg Arg Gln Met Arg Ala Phe
1250 1255 1260

Pro Ala Ala Pro Cys Cys Val Asp Val Ser Asp Arg Leu Arg Ala
1265 1270 1275

Leu Ala Pro Ser Ala Glu Cys Pro Ile Ala Glu Glu Asn Leu Ala
1280 1285 1290

Arg Gly Leu Leu Asp Leu Gly His Ala Gly Arg Leu Gly Ile Ser
1295 1300 1305

Cys Thr Arg Leu Arg Arg Leu Arg Asn Lys Thr Ala His Gln Met
1310 1315 1320

Lys Val Gln His Phe Tyr Gln Gln Leu Leu Ser Asp Val Ala Trp
1325 1330 1335

Ala Ser Leu Asp Leu Pro Ser Leu Val Ala Glu His Leu Pro Gly
1340 1345 1350

Arg Gln Glu His Val Phe Trp Lys Leu Val Leu Val Leu Pro Asp
1355 1360 1365

Val Glu Glu Gln Ser Pro Glu Ser Cys Gly Arg Ile Leu Ala Asn
1370 1375 1380

Trp Leu Lys Val Lys Phe Met Gly Asp Glu Gly Ser Val Asp Asp
1385 1390 1395

Thr Ser Ser Asp Ala Gly Gly Ile Gln Thr Leu Ser Leu Phe Asn
1400 1405 1410

Ser Leu Ser Ser Lys Gly Asp Gln Met Ile Ser Val Asn Val Cys
1415 1420 1425

Ile Lys Val Ala His Gly Ala Leu Ser Asp Gly Ala Ile Asp Ala
1430 1435 1440

Val Glu Thr Gln Lys Asp Leu Leu Gly Ala Ser Gly Leu Met Leu
1445 1450 1455

Leu Leu Pro Pro Lys Met Lys Ser Glu Asp Met Ala Glu Glu Asp
1460 1465 1470

Val Tyr Trp Leu Ser Ala Leu Leu Gln Leu Lys Gln Leu Leu Gln
1475 1480 1485

Ala Lys Pro Phe Gln Pro Ala Leu Pro Leu Val Val Leu Val Pro
1490 1495 1500

Ser Pro Gly Gly Asp Ala Val Glu Lys Glu Val Glu Asp Gly Leu
1505 1510 1515

Met Leu Gln Asp Leu Val Ser Ala Lys Leu Ile Ser Asp Tyr Thr
1520 1525 1530

Val Thr Glu Ile Pro Asp Thr Ile Asn Asp Leu Gln Gly Ser Thr
1535 1540 1545

Lys Val Leu Gln Ala Val Gln Trp Leu Val Ser His Cys Pro His
1550 1555 1560

Ser Leu Asp Leu Cys Cys Gln Thr Leu Ile Gln Tyr Val Glu Asp
1565 1570 1575

Gly Ile Gly His Glu Phe Ser Gly Arg Phe Phe His Asp Arg Arg
1580 1585 1590

Glu Arg Arg Leu Gly Gly Leu Ala Ser Gln Glu Pro Gly Ala Ile
1595 1600 1605

Ile Glu Leu Phe Asn Ser Val Leu Gln Phe Leu Ala Ser Val Val
1610 1615 1620

Ser Ser Glu Gln Leu Cys Asp Leu Ser Trp Pro Val Thr Glu Phe
1625 1630 1635

Ala Glu Ala Gly Gly Ser Arg Leu Leu Pro His Leu His Trp Asn
1640 1645 1650

Ala Pro Glu His Leu Ala Trp Leu Lys Gln Ala Val Leu Gly Phe
1655 1660 1665

Gln Leu Pro Gln Met Asp Leu Pro Pro Leu Gly Ala Pro Trp Leu
1670 1675 1680

Pro Val Cys Ser Met Val Val Gln Tyr Ala Ser Gln Ile Pro Ser
1685 1690 1695

Ser Arg Gln Thr Gln Pro Val Leu Gln Ser Gln Val Glu Asn Leu
1700 1705 1710

Leu His Arg Thr Tyr Cys Arg Trp Lys Ser Lys Ser Pro Ser Pro
1715 1720 1725

Val His Gly Ala Gly Pro Ser Val Met Glu Ile Pro Trp Asp Asp
1730 1735 1740

Leu Ile Ala Leu Cys Ile Asn His Lys Leu Arg Asp Trp Thr Pro
1745 1750 1755

Pro Arg Leu Pro Val Thr Ser Glu Ala Leu Ser Glu Asp Gly Gln
1760 1765 1770

Ile Cys Val Tyr Phe Phe Lys Asn Asp Leu Lys Lys Tyr Asp Val
1775 1780 1785

Pro Leu Ser Trp Glu Gln Ala Arg Leu Gln Thr Gln Lys Glu Leu
1790 1795 1800

Gln Leu Arg Glu Gly Arg Leu Ala Ile Lys Pro Phe His Pro Ser
1805 1810 1815

Ala Asn Asn Phe Pro Ile Pro Leu Leu His Met His Arg Asn Trp
1820 1825 1830

Lys Arg Ser Thr Glu Cys Ala Gln Glu Gly Arg Ile Pro Ser Thr
1835 1840 1845

Glu Asp Leu Met Arg Gly Ala Ser Ala Glu Glu Leu Leu Ala Gln
1850 1855 1860

Cys Leu Ser Ser Ser Leu Leu Leu Glu Lys Glu Glu Asn Lys Arg
1865 1870 1875

Phe Glu Asp Gln Leu Gln Gln Trp Leu Ser Glu Asp Ser Gly Ala
1880 1885 1890

Phe Thr Asp Leu Thr Ser Leu Pro Leu Tyr Leu Pro Gln Thr Leu
1895 1900 1905

Val Ser Leu Ser His Thr Ile Glu Pro Val Met Lys Thr Ser Val
1910 1915 1920

Thr Thr Ser Pro Gln Ser Asp Met Met Arg Glu Gln Leu Gln Leu
1925 1930 1935

Ser Glu Ala Thr Gly Thr Cys Leu Gly Glu Arg Leu Lys His Leu
1940 1945 1950

Glu Arg Leu Ile Arg Ser Ser Arg Glu Glu Glu Val Ala Ser Glu
1955 1960 1965

Leu His Leu Ser Ala Leu Leu Asp Met Val Asp Ile
1970 1975 1980

<210> 112
<211> 1669
<212> PRT
<213> Homo sapiens

<400> 112

Met Gly Pro Arg Leu Ser Val Trp Leu Leu Leu Leu Pro Ala Ala Leu
1 5 10 15

Leu Leu His Glu Glu His Ser Arg Ala Ala Ala Lys Gly Gly Cys Ala
20 25 30

Gly Ser Gly Cys Gly Lys Cys Asp Cys His Gly Val Lys Gly Gln Lys
35 40 45

Gly Glu Arg Gly Leu Pro Gly Leu Gln Gly Val Ile Gly Phe Pro Gly
50 55 60

Met Gln Gly Pro Glu Gly Pro Gln Gly Pro Pro Gly Gln Lys Gly Asp
65 70 75 80

Thr Gly Glu Pro Gly Leu Pro Gly Thr Lys Gly Thr Arg Gly Pro Pro
85 90 95

Gly Ala Ser Gly Tyr Pro Gly Asn Pro Gly Leu Pro Gly Ile Pro Gly
100 105 110

Gln Asp Gly Pro Pro Gly Pro Pro Gly Ile Pro Gly Cys Asn Gly Thr
115 120 125

Lys Gly Glu Arg Gly Pro Leu Gly Pro Pro Gly Leu Pro Gly Phe Ala
130 135 140

Gly Asn Pro Gly Pro Pro Gly Leu Pro Gly Met Lys Gly Asp Pro Gly
145 150 155 160

Glu Ile Leu Gly His Val Pro Gly Met Leu Leu Lys Gly Glu Arg Gly
165 170 175

Phe Pro Gly Ile Pro Gly Thr Pro Gly Pro Pro Gly Leu Pro Gly Leu
180 185 190

Gln Gly Pro Val Gly Pro Pro Gly Phe Thr Gly Pro Pro Gly Pro Pro
195 200 205

Gly Pro Pro Gly Pro Pro Gly Glu Lys Gly Gln Met Gly Leu Ser Phe
210 215 220

Gln Gly Pro Lys Gly Asp Lys Gly Asp Gln Gly Val Ser Gly Pro Pro
225 230 235 240

Gly Val Pro Gly Gln Ala Gln Val Gln Glu Lys Gly Asp Phe Ala Thr
245 250 255

Lys Gly Glu Lys Gly Gln Lys Gly Glu Pro Gly Phe Gln Gly Met Pro
260 265 270

Gly Val Gly Glu Lys Gly Glu Pro Gly Lys Pro Gly Pro Arg Gly Lys
275 280 285

Pro Gly Lys Asp Gly Asp Lys Gly Glu Lys Gly Ser Pro Gly Phe Pro
290 295 300

Gly Glu Pro Gly Tyr Pro Gly Leu Ile Gly Arg Gln Gly Pro Gln Gly
305 310 315 320

Glu Lys Gly Glu Ala Gly Pro Pro Gly Pro Pro Gly Ile Val Ile Gly
325 330 335

Thr Gly Pro Leu Gly Glu Lys Gly Glu Arg Gly Tyr Pro Gly Thr Pro
340 345 350

Gly Pro Arg Gly Glu Pro Gly Pro Lys Gly Phe Pro Gly Leu Pro Gly
355 360 365

Gln Pro Gly Pro Pro Gly Leu Pro Val Pro Gly Gln Ala Gly Ala Pro
370 375 380

Gly Phe Pro Gly Glu Arg Gly Glu Lys Gly Asp Arg Gly Phe Pro Gly
385 390 395 400

Thr Ser Leu Pro Gly Pro Ser Gly Arg Asp Gly Leu Pro Gly Pro Pro
405 410 415

Gly Ser Pro Gly Pro Pro Gly Gln Pro Gly Tyr Thr Asn Gly Ile Val
420 425 430

Glu Cys Gln Pro Gly Pro Pro Gly Asp Gln Gly Pro Pro Gly Ile Pro
435 440 445

Gly Gln Pro Gly Phe Ile Gly Glu Ile Gly Glu Lys Gly Gln Lys Gly
450 455 460

Glu Ser Cys Leu Ile Cys Asp Ile Asp Gly Tyr Arg Gly Pro Pro Gly
465 470 475 480

Pro Gln Gly Pro Pro Gly Glu Ile Gly Phe Pro Gly Gln Pro Gly Ala
485 490 495

Lys Gly Asp Arg Gly Leu Pro Gly Arg Asp Gly Val Ala Gly Val Pro
500 505 510

Gly Pro Gln Gly Thr Pro Gly Leu Ile Gly Gln Pro Gly Ala Lys Gly
515 520 525

Glu Pro Gly Glu Phe Tyr Phe Asp Leu Arg Leu Lys Gly Asp Lys Gly
530 535 540

Asp Pro Gly Phe Pro Gly Gln Pro Gly Met Pro Gly Arg Ala Gly Ser
545 550 555 560

Pro Gly Arg Asp Gly His Pro Gly Leu Pro Gly Pro Lys Gly Ser Pro
565 570 575

Gly Ser Val Gly Leu Lys Gly Glu Arg Gly Pro Pro Gly Gly Val Gly
580 585 590

Phe Pro Gly Ser Arg Gly Asp Thr Gly Pro Pro Gly Pro Pro Gly Tyr
595 600 605

Gly Pro Ala Gly Pro Ile Gly Asp Lys Gly Gln Ala Gly Phe Pro Gly
610 615 620

Gly Pro Gly Ser Pro Gly Leu Pro Gly Pro Lys Gly Glu Pro Gly Lys
625 630 635 640

Ile Val Pro Leu Pro Gly Pro Pro Gly Ala Glu Gly Leu Pro Gly Ser
645 650 655

Pro Gly Phe Pro Gly Pro Gln Gly Asp Arg Gly Phe Pro Gly Thr Pro
660 665 670

Gly Arg Pro Gly Leu Pro Gly Glu Lys Gly Ala Val Gly Gln Pro Gly
675 680 685

Ile Gly Phe Pro Gly Pro Pro Gly Pro Lys Gly Val Asp Gly Leu Pro
690 695 700

Gly Asp Met Gly Pro Pro Gly Thr Pro Gly Arg Pro Gly Phe Asn Gly
705 710 715 720

Leu Pro Gly Asn Pro Gly Val Gln Gly Gln Lys Gly Glu Pro Gly Val
725 730 735

Gly Leu Pro Gly Leu Lys Gly Leu Pro Gly Leu Pro Gly Ile Pro Gly
740 745 750

Thr Pro Gly Glu Lys Gly Ser Ile Gly Val Pro Gly Val Pro Gly Glu
755 760 765

His Gly Ala Ile Gly Pro Pro Gly Leu Gln Gly Ile Arg Gly Glu Pro
770 775 780

Gly Pro Pro Gly Leu Pro Gly Ser Val Gly Ser Pro Gly Val Pro Gly
785 790 795 800

Ile Gly Pro Pro Gly Ala Arg Gly Pro Pro Gly Gly Gln Gly Pro Pro
805 810 815

Gly Leu Ser Gly Pro Pro Gly Ile Lys Gly Glu Lys Gly Phe Pro Gly
820 825 830

Phe Pro Gly Leu Asp Met Pro Gly Pro Lys Gly Asp Lys Gly Ala Gln
835 840 845

Gly Leu Pro Gly Ile Thr Gly Gln Ser Gly Leu Pro Gly Leu Pro Gly
850 855 860

Gln Gln Gly Ala Pro Gly Ile Pro Gly Phe Pro Gly Ser Lys Gly Glu
865 870 875 880

Met Gly Val Met Gly Thr Pro Gly Gln Pro Gly Ser Pro Gly Pro Val
885 890 895

Gly Ala Pro Gly Leu Pro Gly Glu Lys Gly Asp His Gly Phe Pro Gly
900 905 910

Ser Ser Gly Pro Arg Gly Asp Pro Gly Leu Lys Gly Asp Lys Gly Asp
915 920 925

Val Gly Leu Pro Gly Lys Pro Gly Ser Met Asp Lys Val Asp Met Gly
930 935 940

Ser Met Lys Gly Gln Lys Gly Asp Gln Gly Glu Lys Gly Gln Ile Gly
945 950 955 960

Pro Ile Gly Glu Lys Gly Ser Arg Gly Asp Pro Gly Thr Pro Gly Val
965 970 975

Pro Gly Lys Asp Gly Gln Ala Gly Gln Pro Gly Gln Pro Gly Pro Lys
980 985 990

Gly Asp Pro Gly Ile Ser Gly Thr Pro Gly Ala Pro Gly Leu Pro Gly
995 1000 1005

Pro Lys Gly Ser Val Gly Gly Met Gly Leu Pro Gly Thr Pro Gly
1010 1015 1020

Glu Lys Gly Val Pro Gly Ile Pro Gly Pro Gln Gly Ser Pro Gly
1025 1030 1035

Leu Pro Gly Asp Lys Gly Ala Lys Gly Glu Lys Gly Gln Ala Gly
1040 1045 1050

Pro Pro Gly Ile Gly Ile Pro Gly Leu Arg Gly Glu Lys Gly Asp
1055 1060 1065

Gln Gly Ile Ala Gly Phe Pro Gly Ser Pro Gly Glu Lys Gly Glu
1070 1075 1080

Lys Gly Ser Ile Gly Ile Pro Gly Met Pro Gly Ser Pro Gly Leu
1085 1090 1095

Lys Gly Ser Pro Gly Ser Val Gly Tyr Pro Gly Ser Pro Gly Leu
1100 1105 1110

Pro Gly Glu Lys Gly Asp Lys Gly Leu Pro Gly Leu Asp Gly Ile
1115 1120 1125

Pro Gly Val Lys Gly Glu Ala Gly Leu Pro Gly Thr Pro Gly Pro
1130 1135 1140

Thr Gly Pro Ala Gly Gln Lys Gly Glu Pro Gly Ser Asp Gly Ile
1145 1150 1155

Pro Gly Ser Ala Gly Glu Lys Gly Glu Pro Gly Leu Pro Gly Arg
1160 1165 1170

Gly Phe Pro Gly Phe Pro Gly Ala Lys Gly Asp Lys Gly Ser Lys
1175 1180 1185

Gly Glu Val Gly Phe Pro Gly Leu Ala Gly Ser Pro Gly Ile Pro
1190 1195 1200

Gly Ser Lys Gly Glu Gln Gly Phe Met Gly Pro Pro Gly Pro Gln
1205 1210 1215

Gly Gln Pro Gly Leu Pro Gly Ser Pro Gly His Ala Thr Glu Gly
1220 1225 1230

Pro Lys Gly Asp Arg Gly Pro Gln Gly Gln Pro Gly Leu Pro Gly
1235 1240 1245

Leu Pro Gly Pro Met Gly Pro Pro Gly Leu Pro Gly Ile Asp Gly
1250 1255 1260

Val Lys Gly Asp Lys Gly Asn Pro Gly Trp Pro Gly Ala Pro Gly
1265 1270 1275

Val Pro Gly Pro Lys Gly Asp Pro Gly Phe Gln Gly Met Pro Gly
1280 1285 1290

Ile Gly Gly Ser Pro Gly Ile Thr Gly Ser Lys Gly Asp Met Gly
1295 1300 1305

Pro Pro Gly Val Pro Gly Phe Gln Gly Pro Lys Gly Leu Pro Gly
1310 1315 1320

Leu Gln Gly Ile Lys Gly Asp Gln Gly Asp Gln Gly Val Pro Gly
1325 1330 1335

Ala Lys Gly Leu Pro Gly Pro Pro Gly Pro Pro Gly Pro Tyr Asp
1340 1345 1350

Ile Ile Lys Gly Glu Pro Gly Leu Pro Gly Pro Glu Gly Pro Pro
1355 1360 1365

Gly Leu Lys Gly Leu Gln Gly Leu Pro Gly Pro Lys Gly Gln Gln
1370 1375 1380

Gly Val Thr Gly Leu Val Gly Ile Pro Gly Pro Pro Gly Ile Pro
1385 1390 1395

Gly Phe Asp Gly Ala Pro Gly Gln Lys Gly Glu Met Gly Pro Ala
1400 1405 1410

Gly Pro Thr Gly Pro Arg Gly Phe Pro Gly Pro Pro Gly Pro Asp
1415 1420 1425

Gly Leu Pro Gly Ser Met Gly Pro Pro Gly Thr Pro Ser Val Asp
1430 1435 1440

His Gly Phe Leu Val Thr Arg His Ser Gln Thr Ile Asp Asp Pro
1445 1450 1455

Gln Cys Pro Ser Gly Thr Lys Ile Leu Tyr His Gly Tyr Ser Leu
1460 1465 1470

Leu Tyr Val Gln Gly Asn Glu Arg Ala His Gly Gln Asp Leu Gly
1475 1480 1485

Thr Ala Gly Ser Cys Leu Arg Lys Phe Ser Thr Met Pro Phe Leu
1490 1495 1500

Phe Cys Asn Ile Asn Asn Val Cys Asn Phe Ala Ser Arg Asn Asp
1505 1510 1515

Tyr Ser Tyr Trp Leu Ser Thr Pro Glu Pro Met Pro Met Ser Met
1520 1525 1530

Ala Pro Ile Thr Gly Glu Asn Ile Arg Pro Phe Ile Ser Arg Cys
1535 1540 1545

Ala Val Cys Glu Ala Pro Ala Met Val Met Ala Val His Ser Gln
1550 1555 1560

Thr Ile Gln Ile Pro Pro Cys Pro Ser Gly Trp Ser Ser Leu Trp
1565 1570 1575

Ile Gly Tyr Ser Phe Val Met His Thr Ser Ala Gly Ala Glu Gly
1580 1585 1590

Ser Gly Gln Ala Leu Ala Ser Pro Gly Ser Cys Leu Glu Glu Phe
1595 1600 1605

Arg Ser Ala Pro Phe Ile Glu Cys His Gly Arg Gly Thr Cys Asn
1610 1615 1620

Tyr Tyr Ala Asn Ala Tyr Ser Phe Trp Leu Ala Thr Ile Glu Arg
1625 1630 1635

Ser Glu Met Phe Lys Lys Pro Thr Pro Ser Thr Leu Lys Ala Gly
1640 1645 1650

Glu Leu Arg Thr His Val Ser Arg Cys Gln Val Cys Met Arg Arg
1655 1660 1665

Thr

<210> 113
<211> 465
<212> PRT
<213> Homo sapiens

<400> 113

Met Val Ser Ser Gln Lys Leu Glu Lys Pro Ile Glu Met Gly Ser Ser
1 5 10 15

Glu Pro Leu Pro Ile Ala Asp Gly Asp Arg Arg Arg Lys Lys Lys Arg
20 25 30

Arg Gly Arg Ala Thr Asp Ser Leu Pro Gly Lys Phe Glu Asp Met Tyr
35 40 45

Lys Leu Thr Ser Glu Leu Leu Gly Glu Gly Ala Tyr Ala Lys Val Gln
50 55 60

Gly Ala Val Ser Leu Gln Asn Gly Lys Glu Tyr Ala Val Lys Ile Ile
65 70 75 80

Glu Lys Gln Ala Gly His Ser Arg Ser Arg Val Phe Arg Glu Val Glu
85 90 95

Thr Leu Tyr Gln Cys Gln Gly Asn Lys Asn Ile Leu Glu Leu Ile Glu
100 105 110

Phe Phe Glu Asp Asp Thr Arg Phe Tyr Leu Val Phe Glu Lys Leu Gln
115 120 125

Gly Gly Ser Ile Leu Ala His Ile Gln Lys Gln Lys His Phe Asn Glu
130 135 140

Arg Glu Ala Ser Arg Val Val Arg Asp Val Ala Ala Ala Leu Asp Phe
145 150 155 160

Leu His Thr Lys Asp Lys Val Ser Leu Cys His Leu Gly Trp Ser Ala
165 170 175

Met Ala Pro Ser Gly Leu Thr Ala Ala Pro Thr Ser Leu Gly Ser Ser
180 185 190

Asp Pro Pro Thr Ser Ala Ser Gln Val Ala Gly Thr Thr Gly Ile Ala
195 200 205

His Arg Asp Leu Lys Pro Glu Asn Ile Leu Cys Glu Ser Pro Glu Lys
210 215 220

Val Ser Pro Val Lys Ile Cys Asp Phe Asp Leu Gly Ser Gly Met Lys
225 230 235 240

Leu Asn Asn Ser Cys Thr Pro Ile Thr Thr Pro Glu Leu Thr Thr Pro
245 250 255

Cys Gly Ser Ala Glu Tyr Met Ala Pro Glu Val Val Glu Val Phe Thr
260 265 270

Asp Gln Ala Thr Phe Tyr Asp Lys Arg Cys Asp Leu Trp Ser Leu Gly
275 280 285

Val Val Leu Tyr Ile Met Leu Ser Gly Tyr Pro Pro Phe Val Gly His
290 295 300

Cys Gly Ala Asp Cys Gly Trp Asp Arg Gly Glu Val Cys Arg Val Cys
305 310 315 320

Gln Asn Lys Leu Phe Glu Ser Ile Gln Glu Gly Lys Tyr Glu Phe Pro
325 330 335

Asp Lys Asp Trp Ala His Ile Ser Ser Glu Ala Lys Asp Leu Ile Ser
340 345 350

Lys Leu Leu Val Arg Asp Ala Lys Gln Arg Leu Ser Ala Ala Gln Val
355 360 365

Leu Gln His Pro Trp Val Gln Gly Gln Ala Pro Glu Lys Gly Leu Pro
370 375 380

Thr Pro Gln Val Leu Gln Arg Asn Ser Ser Thr Met Asp Leu Thr Leu
385 390 395 400

Phe Ala Ala Glu Ala Ile Ala Leu Asn Arg Gln Leu Ser Gln His Glu
405 410 415

Glu Asn Glu Leu Ala Glu Glu Pro Glu Ala Leu Ala Asp Gly Leu Cys
420 425 430

Ser Met Lys Leu Ser Pro Pro Cys Lys Ser Arg Leu Ala Arg Arg Arg
435 440 445

Ala Leu Ala Gln Ala Gly Arg Gly Glu Asp Arg Ser Pro Pro Thr Ala
450 455 460

Leu
465

<210> 114
<211> 193
<212> PRT
<213> Homo sapiens

<400> 114

Met Ala Arg Ala Arg Gln Glu Gly Ser Ser Pro Glu Pro Val Glu Gly
1 5 10 15

Leu Ala Arg Asp Gly Pro Arg Pro Phe Pro Leu Gly Arg Leu Val Pro
20 25 30

Ser Ala Val Ser Cys Gly Leu Cys Glu Pro Gly Leu Ala Ala Ala Pro
35 40 45

Ala Ala Pro Thr Leu Leu Pro Ala Ala Tyr Leu Cys Ala Pro Thr Ala
50 55 60

Pro Pro Ala Val Thr Ala Ala Leu Gly Gly Ser Arg Trp Pro Gly Gly
65 70 75 80

Pro Arg Ser Arg Pro Arg Gly Pro Arg Pro Asp Gly Pro Gln Pro Ser
85 90 95

Leu Ser Leu Ala Glu Gln His Leu Glu Ser Pro Val Pro Ser Ala Pro
100 105 110

Gly Ala Leu Ala Gly Gly Pro Thr Gln Ala Ala Pro Gly Val Arg Gly
115 120 125

Glu Glu Glu Gln Trp Ala Arg Glu Ile Gly Ala Gln Leu Arg Arg Met
130 135 140

Ala Asp Asp Leu Asn Ala Gln Tyr Glu Arg Arg Arg Gln Glu Glu Gln
145 150 155 160

Gln Arg His Arg Pro Ser Pro Trp Arg Val Leu Tyr Asn Leu Ile Met
165 170 175

Gly Leu Leu Pro Leu Pro Arg Gly His Arg Ala Pro Glu Met Glu Pro
180 185 190

Asn

<210> 115
<211> 897
<212> PRT
<213> Homo sapiens

<400> 115

Met Glu Tyr Thr Lys Gln Leu Ile Leu Ser Cys Leu Leu Asn Ile Cys
1 5 10 15

Gln Lys Leu Ser Pro Asp Gly Gly Lys Ile Pro Lys Asp Ile Leu Asp
20 25 30

Glu Glu Lys Phe Asn Val Glu Leu Ile Val Gln Cys Ile Arg Leu Ser
35 40 45

Glu Met Pro Gln Thr His His His Ala Leu Leu Leu Leu Gly Thr Val
50 55 60

Ala Gly Ile Phe Pro Asp Lys Val Leu His Asn Ile Met Ser Ile Phe
65 70 75 80

Thr Phe Met Gly Ala Asn Val Met Arg Leu Asp Asp Thr Tyr Ser Phe
85 90 95

Gln Val Ile Asn Lys Thr Val Lys Met Val Ile Pro Ala Leu Ile Gln
100 105 110

Ser Asp Ser Gly Asp Ser Ile Glu Val Ser Arg Asn Val Glu Glu Ile
115 120 125

Val Val Lys Ile Ile Ser Val Phe Val Asp Ala Leu Pro His Val Pro
130 135 140

Glu His Arg Arg Leu Pro Ile Leu Val Gln Leu Val Asp Thr Leu Gly
145 150 155 160

Ala Glu Lys Phe Leu Trp Ile Leu Leu Ile Leu Leu Phe Glu Gln Tyr
165 170 175

Val Thr Lys Thr Val Leu Ala Ala Ala Tyr Gly Glu Lys Asp Ala Ile
180 185 190

Leu Glu Ala Asp Thr Glu Phe Trp Phe Ser Val Cys Cys Glu Phe Ser
195 200 205

Val Gln His Gln Ile Gln Ser Leu Met Asn Ile Leu Gln Tyr Leu Leu
210 215 220

Lys Leu Pro Glu Glu Lys Glu Glu Thr Ile Pro Lys Ala Val Ser Phe
225 230 235 240

Asn Lys Ser Glu Ser Gln Glu Glu Met Leu Gln Val Phe Asn Val Glu
245 250 255

Thr His Thr Ser Lys Gln Leu Arg His Phe Lys Phe Leu Ser Val Ser
260 265 270

Phe Met Ser Gln Leu Leu Ser Ser Asn Asp Phe Leu Lys Lys Val Val
275 280 285

Glu Ser Gly Gly Pro Glu Ile Leu Lys Gly Leu Glu Glu Arg Leu Leu
290 295 300

Glu Thr Val Leu Gly Tyr Ile Ser Ala Val Ala Gln Ser Met Glu Arg
305 310 315 320

Asn Ala Asp Lys Leu Thr Val Lys Phe Trp Arg Ala Leu Leu Ser Lys
325 330 335

Ala Tyr Asp Leu Leu Asp Lys Val Asn Ala Leu Leu Pro Thr Glu Thr
340 345 350

Phe Ile Pro Val Ile Arg Gly Leu Val Gly Asn Pro Leu Pro Ser Val
355 360 365

Arg Arg Lys Ala Leu Asp Leu Leu Asn Asn Lys Leu Gln Gln Asn Ile
370 375 380

Ser Trp Lys Lys Thr Ile Val Thr Arg Phe Leu Lys Leu Val Pro Asp
385 390 395 400

Leu Leu Ala Ile Val Gln Arg Lys Lys Lys Glu Gly Glu Glu Glu Gln
405 410 415

Ala Ile Asn Arg Gln Thr Ala Leu Tyr Thr Leu Lys Leu Leu Cys Lys
420 425 430

Asn Phe Gly Ala Glu Asn Pro Asp Pro Phe Val Pro Val Leu Ser Thr
435 440 445

Ala Val Lys Leu Ile Ala Pro Glu Arg Lys Glu Glu Lys Asn Val Leu
450 455 460

Gly Ser Ala Leu Leu Cys Ile Ala Glu Val Thr Ser Thr Leu Glu Ala
465 470 475 480

Leu Ala Ile Pro Gln Leu Pro Ser Leu Met Pro Ser Leu Leu Thr Thr
485 490 495

Met Lys Asn Thr Ser Glu Leu Val Ser Ser Glu Val Tyr Leu Leu Ser
500 505 510

Ala Leu Ala Ala Leu Gln Lys Val Val Glu Thr Leu Pro His Phe Ile
515 520 525

Ser Pro Tyr Leu Glu Gly Ile Leu Ser Gln Val Ile His Leu Glu Lys
530 535 540

Ile Thr Ser Glu Met Gly Ser Ala Ser Arg Ala Asn Ile Arg Leu Thr
545 550 555 560

Ser Leu Lys Lys Thr Leu Ala Thr Thr Leu Ala Pro Arg Val Leu Leu
565 570 575

Pro Ala Ile Lys Lys Thr Tyr Lys Gln Ile Glu Lys Asn Trp Lys Asn
580 585 590

His Met Gly Pro Phe Met Ser Ile Leu Gln Glu His Ile Gly Ala Met
595 600 605

Lys Lys Glu Glu Leu Thr Ser His Gln Ser Gln Leu Thr Ala Phe Phe
610 615 620

Leu Glu Ala Leu Asp Phe Arg Ala Gln His Ser Glu Asn Asp Leu Glu
625 630 635 640

Glu Val Gly Lys Thr Glu Asn Cys Ile Ile Asp Cys Leu Val Ala Met
645 650 655

Val Val Lys Leu Ser Glu Val Thr Phe Arg Pro Leu Phe Phe Lys Leu
660 665 670

Phe Asp Trp Ala Lys Thr Glu Asp Ala Pro Lys Asp Arg Leu Leu Thr
675 680 685

Phe Tyr Asn Leu Ala Asp Cys Ile Ala Glu Lys Leu Lys Gly Leu Phe
690 695 700

Thr Leu Phe Ala Gly His Leu Val Lys Pro Phe Ala Asp Thr Leu Asp
705 710 715 720

Gln Val Asn Ile Ser Lys Thr Asp Glu Ala Phe Phe Asp Ser Glu Asn
725 730 735

Asp Pro Glu Lys Cys Cys Leu Leu Leu Gln Phe Ile Leu Asn Cys Leu
740 745 750

Tyr Lys Ile Phe Leu Phe Asp Thr Gln His Phe Ile Ser Lys Glu Arg
755 760 765

Ala Gly Ala Leu Met Met Pro Leu Val Asp Gln Leu Val Asn Arg Leu
770 775 780

Gly Gly Glu Glu Lys Phe Gln Glu Arg Val Thr Lys His Leu Ile Pro
785 790 795 800

Cys Ile Ala Gln Phe Ser Val Ala Met Ala Asp Asp Ser Leu Trp Lys
805 810 815

Pro Leu Asn Tyr Gln Ile Leu Leu Lys Thr Arg Asp Ser Ser Pro Lys
820 825 830

Val Arg Phe Ala Ala Leu Ile Thr Val Leu Ala Leu Ala Glu Lys Leu
835 840 845

Lys Glu Asn Tyr Ile Val Leu Leu Pro Glu Ser Ile Pro Phe Leu Ala
850 855 860

Glu Leu Met Glu Asp Glu Cys Glu Glu Val Glu His Gln Cys Gln Lys
865 870 875 880

Thr Ile Gln Gln Leu Glu Thr Val Leu Gly Glu Pro Leu Gln Ser Tyr
885 890 895

Phe

<210> 116
<211> 201
<212> PRT
<213> Homo sapiens

<400> 116

Met Asp Leu Ser Leu Leu Trp Val Leu Leu Pro Leu Val Thr Met Ala
1 5 10 15

Trp Gly Gln Tyr Gly Asp Tyr Gly Tyr Pro Tyr Gln Gln Tyr His Asp
20 25 30

Tyr Ser Asp Asp Gly Trp Val Asn Leu Asn Arg Gln Gly Phe Ser Tyr
35 40 45

Gln Cys Pro Gln Gly Gln Val Ile Val Ala Val Arg Ser Ile Phe Ser
50 55 60

Lys Lys Glu Gly Ser Asp Arg Gln Trp Asn Tyr Ala Cys Met Pro Thr
65 70 75 80

Pro Gln Ser Leu Gly Glu Pro Thr Glu Cys Trp Trp Glu Glu Ile Asn
85 90 95

Arg Ala Gly Met Glu Trp Tyr Gln Thr Cys Ser Asn Asn Gly Leu Val
100 105 110

Ala Gly Phe Gln Ser Arg Tyr Phe Glu Ser Val Leu Asp Arg Glu Trp
115 120 125

Gln Phe Tyr Cys Cys Arg Tyr Ser Lys Arg Cys Pro Tyr Ser Cys Trp
130 135 140

Leu Thr Thr Glu Tyr Pro Gly His Tyr Gly Glu Glu Met Asp Met Ile
145 150 155 160

Ser Tyr Asn Tyr Asp Tyr Tyr Ile Arg Gly Ala Thr Thr Thr Phe Ser
165 170 175

Ala Val Glu Arg Asp Arg Gln Trp Lys Phe Ile Met Cys Arg Met Thr
180 185 190

Glu Tyr Asp Cys Glu Phe Ala Asn Val
195 200

<210> 117
<211> 265
<212> PRT
<213> Homo sapiens

<400> 117

Met Pro Met Arg His Arg Lys Lys Ala Ala Asp Lys Asn Leu Pro Cys
1 5 10 15

Arg Pro Leu Val Cys Ala Val Leu Asp Leu Met Val Glu Phe Ile Val
20 25 30

Thr His Met Met Lys Glu Phe Pro Met Asp Leu Tyr Ile Arg Cys Ile
35 40 45

Gln Val Val His Lys Leu Leu Cys Tyr Gln Lys Lys Cys Arg Val Arg
50 55 60

Leu His Tyr Thr Trp Arg Glu Leu Trp Ser Ala Leu Ile Asn Leu Leu
65 70 75 80

Lys Phe Leu Met Ser Asn Glu Thr Val Leu Leu Ala Lys His Asn Ile
85 90 95

Phe Thr Leu Ala Leu Met Ile Val Asn Leu Phe Asn Met Phe Ile Thr
100 105 110

Tyr Gly Asp Thr Phe Leu Pro Thr Pro Ser Ser Tyr Asp Glu Leu Tyr
115 120 125

Tyr Glu Ile Ile Arg Met His Gln Ser Phe Asp Asn Leu Tyr Ser Met
130 135 140

Val Leu Arg Leu Ser Thr Asn Ala Gly Gln Trp Lys Glu Ala Ala Ser
145 150 155 160

Lys Val Thr His Ala Leu Val Asn Ile Arg Ala Ile Ile Asn His Phe
165 170 175

Asn Pro Lys Ile Glu Ser Tyr Ala Ala Val Asn His Ile Ser Gln Leu
180 185 190

Ser Glu Glu Gln Val Leu Glu Val Val Arg Ala Asn Tyr Asp Thr Leu
195 200 205

Thr Leu Lys Leu Gln Asp Gly Leu Asp Gln Tyr Glu Arg Tyr Ser Glu
210 215 220

Gln His Lys Glu Ala Ala Phe Phe Lys Glu Leu Val Arg Ser Ile Ser
225 230 235 240

Thr Asn Val Arg Arg Asn Leu Val Phe His Thr Leu Ser Gln Glu Val
245 250 255

Leu Leu Lys Glu Phe Ser Thr Ile Ser
260 265

<210> 118
<211> 278
<212> PRT
<213> Homo sapiens

<400> 118

Met Lys Leu Phe Gln Arg Ser Thr Pro Ala Ile Thr Leu Glu Ser Pro
1 5 10 15

Asp Ile Lys Tyr Pro Leu Arg Leu Ile Asp Arg Glu Ile Ile Ser His
20 25 30

Asp Thr Arg Arg Phe Arg Phe Ala Leu Pro Ser Pro Gln His Ile Leu
35 40 45

Gly Leu Pro Val Gly Gln His Ile Tyr Leu Ser Ala Arg Ile Asp Gly
50 55 60

Asn Leu Val Val Arg Pro Tyr Thr Pro Ile Ser Ser Asp Asp Asp Lys
65 70 75 80

Gly Phe Val Asp Leu Val Ile Lys Val Tyr Phe Lys Asp Thr His Pro
85 90 95

Lys Phe Pro Ala Gly Gly Lys Met Ser Gln Tyr Leu Glu Ser Met Gln
100 105 110

Ile Gly Asp Thr Ile Glu Phe Arg Gly Pro Ser Gly Leu Leu Val Tyr
115 120 125

Gln Gly Lys Gly Lys Phe Ala Ile Arg Pro Asp Lys Lys Ser Asn Pro
130 135 140

Ile Ile Arg Thr Val Lys Ser Val Gly Met Ile Ala Gly Gly Thr Gly
145 150 155 160

Ile Thr Pro Met Leu Gln Val Ile Arg Ala Ile Met Lys Asp Pro Asp
165 170 175

Asp His Thr Val Cys His Leu Leu Phe Ala Asn Gln Thr Glu Lys Asp
180 185 190

Ile Leu Leu Arg Pro Glu Leu Glu Glu Leu Arg Asn Lys His Ser Ala
195 200 205

Arg Phe Lys Leu Trp Tyr Thr Leu Asp Arg Ala Pro Glu Ala Trp Asp
210 215 220

Tyr Gly Gln Gly Phe Val Asn Glu Glu Met Ile Arg Asp His Leu Pro
225 230 235 240

Pro Pro Glu Glu Glu Pro Leu Val Leu Met Cys Gly Pro Pro Pro Met
245 250 255

Ile Gln Tyr Ala Cys Leu Pro Asn Leu Asp His Val Gly His Pro Thr
260 265 270

Glu Arg Cys Phe Val Phe
275

<210> 119
<211> 322
<212> PRT
<213> Homo sapi ens

<400> 119

Met Glu Gly Ile Ser Asn Phe Lys Thr Pro Ser Lys Leu Ser Glu Lys
1 5 10 15

Lys Lys Ser Val Leu Cys Ser Thr Pro Thr Ile Asn Ile Pro Ala Ser
20 25 30

Pro Phe Met Gln Lys Leu Gly Phe Gly Thr Gly Val Asn Val Tyr Leu
35 40 45

Met Lys Arg Ser Pro Arg Gly Leu Ser His Ser Pro Trp Ala Val Lys
50 55 60

Lys Ile Asn Pro Ile Cys Asn Asp His Tyr Arg Ser Val Tyr Gln Lys
65 70 75 80

Arg Leu Met Asp Glu Ala Lys Ile Leu Lys Ser Leu His His Pro Asn
85 90 95

Ile Val Gly Tyr Arg Ala Phe Thr Glu Ala Asn Asp Gly Ser Leu Cys
100 105 110

Leu Ala Met Glu Tyr Gly Gly Glu Lys Ser Leu Asn Asp Leu Ile Glu
115 120 125

Glu Arg Tyr Lys Ala Ser Gln Asp Pro Phe Pro Ala Ala Ile Ile Leu
130 135 140

Lys Val Ala Leu Asn Met Ala Arg Gly Leu Lys Tyr Leu His Gln Glu
145 150 155 160

Lys Lys Leu Leu His Gly Asp Ile Lys Ser Ser Asn Val Val Ile Lys
165 170 175

Gly Asp Phe Glu Thr Ile Lys Ile Cys Asp Val Gly Val Ser Leu Pro
180 185 190

Leu Asp Glu Asn Met Thr Val Thr Asp Pro Glu Ala Cys Tyr Ile Gly
195 200 205

Thr Glu Pro Trp Lys Pro Lys Glu Ala Val Glu Glu Asn Gly Val Ile
210 215 220

Thr Asp Lys Ala Asp Ile Phe Ala Phe Gly Leu Thr Leu Trp Glu Met
225 230 235 240

Met Thr Leu Ser Ile Pro His Ile Asn Leu Ser Asn Asp Asp Asp Asp
245 250 255

Glu Asp Lys Thr Phe Asp Glu Ser Asp Phe Asp Asp Glu Ala Tyr Tyr
260 265 270

Ala Ala Leu Gly Thr Arg Pro Pro Ile Asn Met Glu Glu Leu Asp Glu
275 280 285

Ser Tyr Gln Lys Val Ile Glu Leu Phe Ser Val Cys Thr Asn Glu Asp
290 295 300

Pro Lys Asp Arg Pro Ser Ala Ala His Ile Val Glu Ala Leu Glu Thr
305 310 315 320

Asp Val

<210> 120
<211> 878
<212> PRT
<213> Homo sapiens

<400> 120

Met Ala Thr Ala Pro Ser Tyr Pro Ala Gly Leu Pro Gly Ser Pro Gly
1 5 10 15

Pro Gly Ser Pro Pro Pro Pro Gly Gly Leu Glu Leu Gln Ser Pro Pro
20 25 30

Pro Leu Leu Pro Gln Ile Pro Ala Pro Gly Ser Gly Val Ser Phe His
35 40 45

Ile Gln Ile Gly Leu Thr Arg Glu Phe Val Leu Leu Pro Ala Ala Ser
50 55 60

Glu Leu Ala His Val Lys Gln Leu Ala Cys Ser Ile Val Asp Gln Lys
65 70 75 80

Phe Pro Glu Cys Gly Phe Tyr Gly Leu Tyr Asp Lys Ile Leu Leu Phe
85 90 95

Lys His Asp Pro Thr Ser Ala Asn Leu Leu Gln Leu Val Arg Ser Ser
100 105 110

Gly Asp Ile Gln Glu Gly Asp Leu Val Glu Val Val Leu Ser Ala Ser
115 120 125

Ala Thr Phe Glu Asp Phe Gln Ile Arg Pro His Ala Leu Thr Val His
130 135 140

Ser Tyr Arg Ala Pro Ala Phe Cys Asp His Cys Gly Glu Met Leu Phe
145 150 155 160

Gly Leu Val Arg Gln Gly Leu Lys Cys Asp Gly Cys Gly Leu Asn Tyr
165 170 175

His Lys Arg Cys Ala Phe Ser Ile Pro Asn Asn Cys Ser Gly Ala Arg
180 185 190

Lys Arg Arg Leu Ser Ser Thr Ser Leu Ala Ser Gly His Ser Val Arg
195 200 205

Leu Gly Thr Ser Glu Ser Leu Pro Cys Thr Ala Glu Glu Leu Ser Arg
210 215 220

Ser Thr Thr Glu Leu Leu Pro Arg Arg Pro Pro Ser Ser Ser Ser Ser
225 230 235 240

Ser Ser Ala Ser Ser Tyr Thr Gly Arg Pro Ile Glu Leu Asp Lys Met
245 250 255

Leu Leu Ser Lys Val Lys Val Pro His Thr Phe Leu Ile His Ser Tyr
260 265 270

Thr Arg Pro Thr Val Cys Gln Ala Cys Lys Lys Leu Leu Lys Gly Leu
275 280 285

Phe Arg Gln Gly Leu Gln Cys Lys Asp Cys Lys Phe Asn Cys His Lys
290 295 300

Arg Cys Ala Thr Arg Val Pro Asn Asp Cys Leu Gly Glu Ala Leu Ile
305 310 315 320

Asn Gly Asp Val Pro Met Glu Glu Ala Thr Asp Phe Ser Glu Ala Asp
325 330 335

Lys Ser Ala Leu Met Asp Glu Ser Glu Asp Ser Gly Val Ile Pro Gly
340 345 350

Ser His Ser Glu Asn Ala Leu His Ala Ser Glu Glu Glu Glu Gly Glu
355 360 365

Gly Gly Lys Ala Gln Ser Ser Leu Gly Tyr Ile Pro Leu Met Arg Val
370 375 380

Val Gln Ser Val Arg His Thr Thr Arg Lys Ser Ser Thr Thr Leu Arg
385 390 395 400

Glu Gly Trp Val Val His Tyr Ser Asn Lys Asp Thr Leu Arg Lys Arg
405 410 415

His Tyr Trp Arg Leu Asp Cys Lys Cys Ile Thr Leu Phe Gln Asn Asn
420 425 430

Thr Thr Asn Arg Tyr Tyr Lys Glu Ile Pro Leu Ser Glu Ile Leu Thr
435 440 445

Val Glu Ser Ala Gln Asn Phe Ser Leu Val Pro Pro Gly Thr Asn Pro
450 455 460

His Cys Phe Glu Ile Val Thr Ala Asn Ala Thr Tyr Phe Val Gly Glu
465 470 475 480

Met Pro Gly Gly Thr Pro Gly Gly Pro Ser Gly Gln Gly Ala Glu Ala
485 490 495

Ala Arg Gly Trp Glu Thr Ala Ile Arg Gln Ala Leu Met Pro Val Ile
500 505 510

Leu Gln Asp Ala Pro Ser Ala Pro Gly His Ala Pro His Arg Gln Ala
515 520 525

Ser Leu Ser Ile Ser Val Ser Asn Ser Gln Ile Gln Glu Asn Val Asp
530 535 540

Ile Ala Thr Val Tyr Gln Ile Phe Pro Asp Glu Val Leu Gly Ser Gly
545 550 555 560

Gln Phe Gly Val Val Tyr Gly Gly Lys His Arg Lys Thr Gly Arg Asp
565 570 575

Val Ala Val Lys Val Ile Asp Lys Leu Arg Phe Pro Thr Lys Gln Glu
580 585 590

Ser Gln Leu Arg Asn Glu Val Ala Ile Leu Gln Ser Leu Arg His Pro
595 600 605

Gly Ile Val Asn Leu Glu Cys Met Phe Glu Thr Pro Glu Lys Val Phe
610 615 620

Val Val Met Glu Lys Leu His Gly Asp Met Leu Glu Met Ile Leu Ser
625 630 635 640

Ser Glu Lys Gly Arg Leu Pro Glu Arg Leu Thr Lys Phe Leu Ile Thr
645 650 655

Gln Ile Leu Val Ala Leu Arg His Leu His Phe Lys Asn Ile Val His
660 665 670

Cys Asp Leu Lys Pro Glu Asn Val Leu Leu Ala Ser Ala Asp Pro Phe
675 680 685

Pro Gln Val Lys Leu Cys Asp Phe Gly Phe Ala Arg Ile Ile Gly Glu
690 695 700

Lys Ser Phe Arg Arg Ser Val Val Gly Thr Pro Ala Tyr Leu Ala Pro
705 710 715 720

Glu Val Leu Leu Asn Gln Gly Tyr Asn Arg Ser Leu Asp Met Trp Ser
725 730 735

Val Gly Val Ile Met Tyr Val Ser Leu Ser Gly Thr Phe Pro Phe Asn
740 745 750

Glu Asp Glu Asp Ile Asn Asp Gln Ile Gln Asn Ala Ala Phe Met Tyr
755 760 765

Pro Ala Ser Pro Trp Ser His Ile Ser Ala Gly Ala Ile Asp Leu Ile
770 775 780

Asn Asn Leu Leu Gln Val Lys Met Arg Lys Arg Tyr Ser Val Asp Lys
785 790 795 800

Ser Leu Ser His Pro Trp Leu Gln Glu Tyr Gln Thr Trp Leu Asp Leu
805 810 815

Arg Glu Leu Glu Gly Lys Met Gly Glu Arg Tyr Ile Thr His Glu Ser
820 825 830

Asp Asp Ala Arg Trp Glu Gln Phe Ala Ala Glu His Pro Leu Pro Gly
835 840 845

Ser Gly Leu Pro Thr Asp Arg Asp Leu Gly Gly Ala Cys Pro Pro Gln
850 855 860

Asp His Asp Met Gln Gly Leu Ala Glu Arg Ile Ser Val Leu
865 870 875

<210> 121
<211> 400
<212> PRT
<213> Homo sapiens

<400> 121

Met Thr Ser Tyr Ser Tyr Arg Gln Ser Ser Ala Thr Ser Ser Phe Gly
1 5 10 15

Gly Leu Gly Gly Gly Ser Val Arg Phe Gly Pro Gly Val Ala Phe Arg
20 25 30

Ala Pro Ser Ile His Gly Gly Ser Gly Gly Arg Gly Val Ser Val Ser
35 40 45

Ser Ala Arg Phe Val Ser Ser Ser Ser Ser Gly Ala Tyr Gly Gly Gly
50 55 60

Tyr Gly Gly Val Leu Thr Ala Ser Asp Gly Leu Leu Ala Gly Asn Glu
65 70 75 80

Lys Leu Thr Met Gln Asn Leu Asn Asp Arg Leu Ala Ser Tyr Leu Asp
85 90 95

Lys Val Arg Ala Leu Glu Ala Ala Asn Gly Glu Leu Glu Val Lys Ile
100 105 110

Arg Asp Trp Tyr Gln Lys Gln Gly Pro Gly Pro Ser Arg Asp Tyr Ser
115 120 125

His Tyr Tyr Thr Thr Ile Gln Asp Leu Arg Asp Lys Ile Leu Gly Ala
130 135 140

Thr Ile Glu Asn Ser Arg Ile Val Leu Gln Ile Asp Asn Ala Arg Leu
145 150 155 160

Ala Ala Asp Asp Phe Arg Thr Lys Phe Glu Thr Glu Gln Ala Leu Arg
165 170 175

Met Ser Val Glu Ala Asp Ile Asn Gly Leu Arg Arg Val Leu Asp Glu
180 185 190

Leu Thr Leu Ala Arg Thr Asp Leu Glu Met Gln Ile Glu Gly Leu Lys
195 200 205

Glu Glu Leu Ala Tyr Leu Lys Lys Asn His Glu Glu Glu Ile Ser Thr
210 215 220

Leu Arg Gly Gln Val Gly Gly Gln Val Ser Val Glu Val Asp Ser Ala
225 230 235 240

Pro Gly Thr Asp Leu Ala Lys Ile Leu Ser Asp Met Arg Ser Gln Tyr
245 250 255

Glu Val Met Ala Glu Gln Asn Arg Lys Asp Ala Glu Ala Trp Phe Thr
260 265 270

Ser Arg Thr Glu Glu Leu Asn Arg Glu Val Ala Gly His Thr Glu Gln
275 280 285

Leu Gln Met Ser Arg Ser Glu Val Thr Asp Leu Arg Arg Thr Leu Gln
290 295 300

Gly Leu Glu Ile Glu Leu Gln Ser Gln Leu Ser Met Lys Ala Ala Leu
305 310 315 320

Glu Asp Thr Leu Ala Glu Thr Glu Ala Arg Phe Gly Ala Gln Leu Ala
325 330 335

His Ile Gln Ala Leu Ile Ser Gly Ile Glu Ala Gln Leu Gly Asp Val
340 345 350

Arg Ala Asp Ser Glu Arg Gln Asn Gln Glu Tyr Gln Arg Leu Met Asp
355 360 365

Ile Lys Ser Arg Leu Glu Gln Glu Ile Ala Thr Tyr Arg Ser Leu Leu
370 375 380

Glu Gly Gln Glu Asp His Tyr Asn Asn Leu Ser Ala Ser Lys Val Leu
385 390 395 400

<210> 122
<211> 520
<212> PRT
<213> Homo sapiens

<400> 122

Met Arg Ser Pro Glu Gly Tyr Leu Arg Gly Asn Met Ser Glu Asn Glu
1 5 10 15

Glu Glu Glu Ile Ser Gln Gln Glu Gly Ser Gly Asp Tyr Glu Val Glu
20 25 30

Glu Ile Pro Phe Gly Leu Glu Pro Gln Ser Pro Gly Phe Glu Pro Gln
35 40 45

Ser Pro Glu Phe Glu Pro Gln Ser Pro Arg Phe Glu Pro Glu Ser Pro
50 55 60

Gly Phe Glu Ser Arg Ser Pro Gly Leu Val Pro Pro Ser Pro Glu Phe
65 70 75 80

Ala Pro Arg Ser Pro Glu Ser Asp Ser Gln Ser Pro Glu Phe Glu Ser
85 90 95

Gln Ser Pro Arg Tyr Glu Pro Gln Ser Pro Gly Tyr Glu Pro Arg Ser
100 105 110

Pro Gly Tyr Glu Pro Arg Ser Pro Gly Tyr Glu Ser Glu Ser Ser Arg
115 120 125

Tyr Glu Ser Gln Asn Thr Glu Leu Lys Thr Gln Ser Pro Glu Phe Glu
130 135 140

Ala Gln Ser Ser Lys Phe Gln Glu Gly Ala Glu Met Leu Leu Asn Pro
145 150 155 160

Asp Glu Lys Ser Pro Leu Asn Ile Ser Val Gly Val His Pro Leu Asp
165 170 175

Ser Phe Thr Gln Gly Phe Gly Glu Gln Pro Thr Gly Asp Leu Pro Ile
180 185 190

Gly Pro Pro Phe Glu Met Pro Thr Gly Ala Leu Leu Ser Thr Pro Gln
195 200 205

Phe Glu Met Leu Gln Asn Pro Leu Gly Leu Thr Gly Ala Leu Arg Gly
210 215 220

Pro Gly Arg Arg Gly Gly Arg Ala Arg Gly Gly Gln Gly Pro Arg Pro
225 230 235 240

Asn Ile Cys Gly Ile Cys Gly Lys Ser Phe Gly Arg Gly Ser Thr Leu
245 250 255

Ile Gln His Gln Arg Ile His Thr Gly Glu Lys Pro Tyr Lys Cys Glu
260 265 270

Val Cys Ser Lys Ala Phe Ser Gln Ser Ser Asp Leu Ile Lys His Gln
275 280 285

Arg Thr His Thr Gly Glu Arg Pro Tyr Lys Cys Pro Arg Cys Gly Lys
290 295 300

Ala Phe Ala Asp Ser Ser Tyr Leu Leu Arg His Gln Arg Thr His Ser
305 310 315 320

Gly Gln Lys Pro Tyr Lys Cys Pro His Cys Gly Lys Ala Phe Gly Asp
325 330 335

Ser Ser Tyr Leu Leu Arg His Gln Arg Thr His Ser His Glu Arg Pro
340 345 350

Tyr Ser Cys Thr Glu Cys Gly Lys Cys Tyr Ser Gln Asn Ser Ser Leu
355 360 365

Arg Ser His Gln Arg Val His Thr Gly Gln Arg Pro Phe Ser Cys Gly
370 375 380

Ile Cys Gly Lys Ser Phe Ser Gln Arg Ser Ala Leu Ile Pro His Ala
385 390 395 400

Arg Ser His Ala Arg Glu Lys Pro Phe Lys Cys Pro Glu Cys Gly Lys
405 410 415

Arg Phe Gly Gln Ser Ser Val Leu Ala Ile His Ala Arg Thr His Leu
420 425 430

Pro Gly Arg Thr Tyr Ser Cys Pro Asp Cys Gly Lys Thr Phe Asn Arg
435 440 445

Ser Ser Thr Leu Ile Gln His Gln Arg Ser His Thr Gly Glu Arg Pro
450 455 460

Tyr Arg Cys Ala Val Cys Gly Lys Gly Phe Cys Arg Ser Ser Thr Leu
465 470 475 480

Leu Gln His His Arg Val His Ser Gly Glu Arg Pro Tyr Lys Cys Asp
485 490 495

Asp Cys Gly Lys Ala Phe Ser Gln Ser Ser Asp Leu Ile Arg His Gln
500 505 510

Arg Thr His Ala Ala Gly Arg Arg
515 520

<210> 123
<211> 383
<212> PRT
<213> Homo sapiens

<400> 123

Met Glu Arg Arg Ala Arg Ser Ser Ser Arg Glu Ser Arg Gly Arg Gly
1 5 10 15

Gly Arg Thr Pro His Lys Glu Asn Lys Arg Ala Lys Ala Glu Arg Ser
20 25 30

Gly Gly Gly Arg Gly Arg Gln Glu Ala Gly Pro Glu Pro Ser Gly Ser
35 40 45

Gly Arg Ala Gly Thr Pro Gly Glu Pro Arg Ala Pro Ala Ala Thr Val
50 55 60

Val Asp Val Asp Glu Val Arg Gly Ser Gly Glu Glu Gly Thr Glu Val
65 70 75 80

Val Ala Leu Leu Glu Ser Glu Arg Pro Glu Glu Gly Thr Lys Ser Ser
85 90 95

Gly Leu Gly Ala Cys Glu Trp Leu Leu Val Leu Ile Ser Leu Leu Phe
100 105 110

Ile Ile Met Thr Phe Pro Phe Ser Ile Trp Phe Cys Val Lys Val Val
115 120 125

Gln Glu Tyr Glu Arg Val Ile Ile Phe Arg Leu Gly His Leu Leu Pro
130 135 140

Gly Arg Ala Lys Gly Pro Gly Leu Phe Phe Phe Leu Pro Cys Leu Asp
145 150 155 160

Thr Tyr His Lys Val Asp Leu Arg Leu Gln Thr Leu Glu Ile Pro Phe
165 170 175

His Glu Ile Val Thr Lys Asp Met Phe Ile Met Glu Ile Asp Ala Ile
180 185 190

Cys Tyr Tyr Arg Met Glu Asn Ala Ser Leu Leu Leu Ser Ser Leu Ala
195 200 205

His Val Ser Lys Ala Val Gln Phe Leu Val Gln Thr Thr Met Lys Arg
210 215 220

Leu Leu Ala His Arg Ser Leu Thr Glu Ile Leu Leu Glu Arg Lys Ser
225 230 235 240

Ile Ala Gln Asp Ala Lys Val Ala Leu Asp Ser Val Thr Cys Ile Trp
245 250 255

Gly Ile Lys Val Glu Arg Ile Glu Ile Lys Asp Val Arg Leu Pro Ala
260 265 270

Gly Leu Gln His Ser Leu Ala Val Glu Ala Glu Ala Gln Arg Gln Ala
275 280 285

Lys Val Arg Met Ile Ala Ala Glu Ala Glu Lys Ala Ala Ser Glu Ser
290 295 300

Leu Arg Met Ala Ala Glu Ile Leu Ser Gly Thr Pro Ala Ala Val Gln
305 310 315 320

Leu Arg Tyr Leu His Thr Leu Gln Ser Leu Ser Thr Glu Lys Pro Ser
325 330 335

Thr Val Val Leu Pro Leu Pro Phe Asp Leu Leu Asn Cys Leu Ser Ser
340 345 350

Pro Ser Asn Arg Thr Gln Gly Ser Leu Pro Phe Pro Ser Pro Ser Lys
355 360 365

Pro Val Glu Pro Leu Asn Pro Lys Lys Lys Asp Ser Pro Met Leu
370 375 380

<210> 124
<211> 128
<212> PRT
<213> Homo sapiens

<400> 124

Met Thr Ser Leu Phe Ala Gln Glu Ile Arg Leu Ser Lys Arg His Glu
1               5                   10                  15

Glu Ile Val Ser Gln Arg Leu Met Leu Leu Gln Gln Met Glu Asn Lys
            20                  25                  30

Leu Gly Asp Gln His Thr Glu Lys Ala Ser Gln Leu Gln Thr Val Glu
        35                  40                  45

Thr Ala Phe Lys Arg Asn Leu Ser Leu Leu Lys Asp Ile Glu Ala Ala
    50                  55                  60

Glu Lys Ser Leu Gln Thr Arg Ile His Pro Leu Pro Arg Pro Glu Val
65                  70                  75                  80

Val Ser Leu Glu Thr Arg Tyr Trp Ala Ser Val Glu Glu Tyr Ile Pro
                85                  90                  95

Lys Trp Glu Gln Phe Leu Leu Gly Arg Ala Pro Tyr Pro Phe Ala Val
            100                 105                 110

Glu Asn Gln Asn Glu Ala Glu Asn Thr Ile Gln Asn Glu Ala Gln Arg
        115                 120                 125

<210> 125
<211> 363
<212> PRT
<213> Homo sapiens

<400> 125

Met Lys Gly Asn Ser Thr Leu Ala Thr Thr Ser Lys Asn Ile Thr Ser
1               5                   10                  15

Gly Leu His Phe Gly Leu Val Asn Ile Ser Gly Asn Asn Glu Ser Thr
            20                  25                  30

Leu Asn Cys Ser Gln Lys Pro Ser Asp Lys His Leu Asp Ala Ile Pro
        35                  40                  45

Ile Leu Tyr Tyr Ile Ile Phe Val Ile Gly Phe Leu Val Asn Ile Val
    50                  55                  60

Val Val Thr Leu Phe Cys Cys Gln Lys Gly Pro Lys Lys Val Ser Ser
65                  70                  75                  80

Ile Tyr Ile Phe Asn Leu Ala Val Ala Asp Leu Leu Leu Leu Ala Thr
                85                  90                  95

Leu Pro Leu Trp Ala Thr Tyr Tyr Ser Tyr Arg Tyr Asp Trp Leu Phe
100 105 110

Gly Pro Val Met Cys Lys Val Phe Gly Ser Phe Leu Thr Leu Asn Met
115 120 125

Phe Ala Ser Ile Phe Phe Ile Thr Cys Met Ser Val Asp Arg Tyr Gln
130 135 140

Ser Val Ile Tyr Pro Phe Leu Ser Gln Arg Arg Asn Pro Trp Gln Ala
145 150 155 160

Ser Tyr Ile Val Pro Leu Val Trp Cys Met Ala Cys Leu Ser Ser Leu
165 170 175

Pro Thr Phe Tyr Phe Arg Asp Val Arg Thr Ile Glu Tyr Leu Gly Val
180 185 190

Asn Ala Cys Ile Met Ala Phe Pro Pro Glu Lys Tyr Ala Gln Trp Ser
195 200 205

Ala Gly Ile Ala Leu Met Lys Asn Ile Leu Gly Phe Ile Ile Pro Leu
210 215 220

Ile Phe Ile Ala Thr Cys Tyr Phe Gly Ile Arg Lys His Leu Leu Lys
225 230 235 240

Thr Asn Ser Tyr Gly Lys Asn Arg Ile Thr Arg Asp Gln Val Leu Lys
245 250 255

Met Ala Ala Ala Val Val Leu Ala Phe Ile Ile Cys Trp Leu Pro Phe
260 265 270

His Val Leu Thr Phe Leu Asp Ala Leu Ala Trp Met Gly Val Ile Asn
275 280 285

Ser Cys Glu Val Ile Ala Val Ile Asp Leu Ala Leu Pro Phe Ala Ile
290 295 300

Leu Leu Gly Phe Thr Asn Ser Cys Val Asn Pro Phe Leu Tyr Cys Phe
305 310 315 320

Val Gly Asn Arg Phe Gln Gln Lys Leu Arg Ser Val Phe Arg Val Pro
325 330 335

Ile Thr Trp Leu Gln Gly Lys Arg Glu Ser Met Ser Cys Arg Lys Ser
340 345 350

Ser Ser Leu Arg Glu Met Glu Thr Phe Val Ser
355 360

<210> 126

<211> 366
<212> PRT
<213> Homo sapiens

<400> 126

Met Asp Phe Leu Asn Ser Ser Asp Gln Asn Leu Thr Ser Glu Glu Leu
1 5 10 15

Leu Asn Arg Met Pro Ser Lys Ile Leu Val Ser Leu Thr Leu Ser Gly
20 25 30

Leu Ala Leu Met Thr Thr Thr Ile Asn Ser Leu Val Ile Ala Ala Ile
35 40 45

Ile Val Thr Arg Lys Leu His His Pro Ala Asn Tyr Leu Ile Cys Ser
50 55 60

Leu Ala Val Thr Asp Phe Leu Val Ala Val Leu Val Met Pro Phe Ser
65 70 75 80

Ile Val Tyr Ile Val Arg Glu Ser Trp Ile Met Gly Gln Val Val Cys
85 90 95

Asp Ile Trp Leu Ser Val Asp Ile Thr Cys Cys Thr Cys Ser Ile Leu
100 105 110

His Leu Ser Ala Ile Ala Leu Asp Arg Tyr Arg Ala Ile Thr Asp Ala
115 120 125

Val Glu Tyr Ala Arg Lys Arg Thr Pro Lys His Ala Gly Ile Met Ile
130 135 140

Thr Ile Val Trp Ile Ile Ser Val Phe Ile Ser Met Pro Pro Leu Phe
145 150 155 160

Trp Arg His Gln Gly Thr Ser Arg Asp Asp Glu Cys Ile Ile Lys His
165 170 175

Asp His Ile Val Ser Thr Ile Tyr Ser Thr Phe Gly Ala Phe Tyr Ile
180 185 190

Pro Leu Ala Leu Ile Leu Ile Leu Tyr Tyr Lys Ile Tyr Arg Ala Ala
195 200 205

Lys Thr Leu Tyr His Lys Arg Gln Ala Ser Arg Ile Ala Lys Glu Glu
210 215 220

Val Asn Gly Gln Val Leu Leu Glu Ser Gly Glu Lys Ser Thr Lys Ser
225 230 235 240

Val Ser Thr Ser Tyr Val Leu Glu Lys Ser Leu Ser Asp Pro Ser Thr
245 250 255

Asp Phe Asp Lys Ile His Ser Thr Val Arg Ser Leu Arg Ser Glu Phe
260 265 270

Lys His Glu Lys Ser Trp Arg Arg Gln Lys Ile Ser Gly Thr Arg Glu
275 280 285

Arg Lys Ala Ala Thr Thr Leu Gly Leu Ile Leu Gly Ala Phe Val Ile
290 295 300

Cys Trp Leu Pro Phe Phe Val Lys Glu Leu Val Val Asn Val Cys Asp
305 310 315 320

Lys Cys Lys Ile Ser Glu Glu Met Ser Asn Phe Leu Ala Trp Leu Gly
325 330 335

Tyr Leu Asn Ser Leu Ile Asn Pro Leu Ile Tyr Thr Ile Phe Asn Glu
340 345 350

Asp Phe Lys Lys Ala Phe Gln Lys Leu Val Arg Cys Arg Cys
355 360 365

<210> 127
<211> 747
<212> PRT
<213> Homo sapi ens

<400> 127

Met Ser Lys Leu Lys Ser Ser Glu Ser Val Arg Val Val Val Arg Cys
1 5 10 15

Arg Pro Met Asn Gly Lys Glu Lys Ala Ala Ser Tyr Asp Lys Val Val
20 25 30

Asp Val Asp Val Lys Leu Gly Gln Val Ser Val Lys Asn Pro Lys Gly
35 40 45

Thr Ala His Glu Met Pro Lys Thr Phe Thr Phe Asp Ala Val Tyr Asp
50 55 60

Trp Asn Ala Lys Gln Phe Glu Leu Tyr Asp Glu Thr Phe Arg Pro Leu
65 70 75 80

Val Asp Ser Val Leu Gln Gly Phe Asn Gly Thr Ile Phe Ala Tyr Gly
85 90 95

Gln Thr Gly Thr Gly Lys Thr Tyr Thr Met Glu Gly Ile Arg Gly Asp
100 105 110

Pro Glu Lys Arg Gly Val Ile Pro Asn Ser Phe Asp His Ile Phe Thr
115 120 125

His Ile Ser Arg Ser Gln Asn Gln Gln Tyr Leu Val Arg Ala Ser Tyr
130 135 140

Leu Glu Ile Tyr Gln Glu Glu Ile Arg Asp Leu Leu Ser Lys Asp Gln
145 150 155 160

Thr Lys Arg Leu Glu Leu Lys Glu Arg Pro Asp Thr Gly Val Tyr Val
165 170 175

Lys Asp Leu Ser Ser Phe Val Thr Lys Ser Val Lys Glu Ile Glu His
180 185 190

Val Met Asn Val Gly Asn Gln Asn Arg Ser Val Gly Ala Thr Asn Met
195 200 205

Asn Glu His Ser Ser Arg Ser His Ala Ile Phe Val Ile Thr Ile Glu
210 215 220

Cys Ser Glu Val Gly Leu Asp Gly Glu Asn His Ile Arg Val Gly Lys
225 230 235 240

Leu Asn Leu Val Asp Leu Ala Gly Ser Glu Arg Gln Ala Lys Thr Gly
245 250 255

Ala Gln Gly Glu Arg Leu Lys Glu Ala Thr Lys Ile Asn Leu Ser Leu
260 265 270

Ser Ala Leu Gly Asn Val Ile Ser Ala Leu Val Asp Gly Lys Ser Thr
275 280 285

His Ile Pro Tyr Arg Asp Ser Lys Leu Thr Arg Leu Leu Gln Asp Ser
290 295 300

Leu Gly Gly Asn Ala Lys Thr Val Met Val Ala Asn Val Gly Pro Ala
305 310 315 320

Ser Tyr Asn Val Glu Glu Thr Leu Thr Thr Leu Arg Tyr Ala Asn Arg
325 330 335

Ala Lys Asn Ile Lys Asn Lys Pro Arg Val Asn Glu Asp Pro Lys Asp
340 345 350

Ala Leu Leu Arg Glu Phe Gln Glu Glu Ile Ala Arg Leu Lys Ala Gln
355 360 365

Leu Glu Lys Arg Ser Ile Gly Arg Arg Lys Arg Arg Glu Lys Arg Arg
370 375 380

Glu Gly Gly Gly Ser Gly Gly Gly Gly Glu Glu Glu Glu Glu Glu Gly
385 390 395 400

Glu Glu Gly Glu Glu Glu Gly Asp Asp Lys Asp Asp Tyr Trp Arg Glu
405 410 415

Gln Gln Glu Lys Leu Glu Ile Glu Lys Arg Ala Ile Val Glu Asp His
420 425 430

Ser Leu Val Ala Glu Glu Lys Met Arg Leu Leu Lys Glu Lys Glu Lys
435 440 445

Lys Met Glu Asp Leu Arg Arg Glu Lys Asp Ala Ala Glu Met Leu Gly
450 455 460

Ala Lys Ile Lys Ala Met Glu Ser Lys Leu Leu Val Gly Gly Lys Asn
465 470 475 480

Ile Val Asp His Thr Asn Glu Gln Gln Lys Ile Leu Glu Gln Lys Arg
485 490 495

Gln Glu Ile Ala Glu Gln Lys Arg Arg Glu Arg Glu Ile Gln Gln Gln
500 505 510

Met Glu Ser Arg Asp Glu Glu Thr Leu Glu Leu Lys Glu Thr Tyr Ser
515 520 525

Ser Leu Gln Gln Glu Val Asp Ile Lys Thr Lys Lys Leu Lys Lys Leu
530 535 540

Phe Ser Lys Leu Gln Ala Val Lys Ala Glu Ile His Asp Leu Gln Glu
545 550 555 560

Glu His Ile Lys Glu Arg Gln Glu Leu Glu Gln Thr Gln Asn Glu Leu
565 570 575

Thr Arg Glu Leu Lys Leu Lys His Leu Ile Ile Glu Asn Phe Ile Pro
580 585 590

Leu Glu Glu Lys Ser Lys Ile Met Asn Arg Ala Phe Phe Asp Glu Glu
595 600 605

Glu Asp His Trp Lys Leu His Pro Ile Thr Arg Leu Glu Asn Gln Gln
610 615 620

Met Met Lys Arg Pro Val Ser Ala Val Gly Tyr Lys Arg Pro Leu Ser
625 630 635 640

Gln His Ala Arg Met Ser Met Met Ile Arg Pro Glu Ala Arg Tyr Arg
645 650 655

Ala Glu Asn Ile Val Leu Leu Glu Leu Asp Met Pro Ser Arg Thr Thr
660 665 670

Arg Asp Tyr Glu Gly Pro Ala Ile Ala Pro Lys Val Gln Ala Ala Leu
675 680 685

Asp Ala Ala Leu Gln Asp Glu Asp Glu Ile Gln Val Asp Ala Ser Ser
690 695 700

Phe Glu Ser Thr Ala Asn Lys Lys Ser Lys Ala Arg Pro Lys Ser Gly
705 710 715 720

Arg Lys Ser Gly Ser Ser Ser Ser Ser Ser Gly Thr Pro Ala Ser Gln
725 730 735

Leu Tyr Pro Gln Ser Arg Gly Leu Val Pro Lys
740 745

<210> 128
<211> 172
<212> PRT
<213> Homo sapiens

<400> 128

Met Lys Ala Thr Ile Ile Leu Leu Leu Leu Ala Gln Val Ser Trp Ala
1 5 10 15

Gly Pro Phe Gln Gln Arg Gly Leu Phe Asp Phe Met Leu Glu Asp Glu
20 25 30

Ala Ser Gly Ile Gly Pro Glu Val Pro Asp Asp Arg Asp Phe Glu Pro
35 40 45

Ser Leu Gly Pro Val Cys Pro Phe Arg Cys Gln Cys His Leu Arg Val
50 55 60

Val Gln Cys Ser Asp Leu Gly Leu Asp Lys Val Pro Lys Asp Leu Pro
65 70 75 80

Pro Asp Thr Thr Leu Leu Asp Leu Gln Asn Asn Lys Ile Thr Glu Ile
85 90 95

Lys Asp Gly Asp Phe Lys Asn Leu Lys Asn Leu His Val Val Tyr Leu
100 105 110

His Asn Asn Asn Ile Ser Val Val Gly Ser Ser Asp Phe Cys Pro Pro
115 120 125

Gly His Asn Thr Lys Lys Ala Ser Tyr Ser Gly Val Ser Leu Phe Ser
130 135 140

Asn Pro Val Gln Tyr Trp Glu Ile Gln Pro Ser Thr Phe Arg Cys Val
145 150 155 160

Tyr Val Arg Ser Ala Ile Gln Leu Gly Asn Tyr Lys
165 170

<210> 129
<211> 268
<212> PRT
<213> Homo sapiens

<400> 129

Met Glu Asp Phe Leu Leu Ser Asn Gly Tyr Gln Leu Gly Lys Thr Ile
1 5 10 15

Gly Glu Gly Thr Tyr Ser Lys Val Lys Glu Ala Phe Ser Lys Lys His
20 25 30

Gln Arg Lys Val Ala Ile Lys Val Ile Asp Lys Met Gly Gly Pro Glu
35 40 45

Glu Phe Ile Gln Arg Phe Leu Pro Arg Glu Leu Gln Ile Val Arg Thr
50 55 60

Leu Asp His Lys Asn Ile Ile Gln Val Tyr Glu Met Leu Glu Ser Ala
65 70 75 80

Asp Gly Lys Ile Cys Leu Val Met Glu Leu Ala Glu Gly Gly Asp Val
85 90 95

Phe Asp Cys Val Leu Asn Gly Gly Pro Leu Pro Glu Ser Arg Ala Lys
100 105 110

Ala Leu Phe Arg Gln Met Val Glu Ala Ile Arg Tyr Cys His Gly Cys
115 120 125

Gly Val Ala His Arg Asp Leu Lys Cys Glu Asn Ala Leu Leu Gln Gly
130 135 140

Phe Asn Leu Lys Leu Thr Asp Phe Gly Phe Ala Lys Val Leu Pro Lys
145 150 155 160

Ser His Arg Glu Leu Ser Gln Thr Phe Cys Gly Ser Thr Ala Tyr Ala
165 170 175

Ala Pro Glu Val Leu Gln Gly Ile Pro His Asp Ser Lys Lys Gly Asp
180 185 190

Val Trp Ser Met Gly Val Val Leu Tyr Val Met Leu Cys Ala Ser Leu
195 200 205

Pro Phe Asp Asp Thr Asp Ile Pro Lys Met Leu Trp Gln Gln Gln Lys
210 215 220

Gly Val Ser Phe Pro Thr His Leu Ser Ile Ser Ala Asp Cys Gln Asp
225 230 235 240

Leu Leu Lys Arg Leu Leu Glu Pro Asp Met Ile Leu Arg Pro Ser Ile
245 250 255

Glu Glu Val Ser Trp His Pro Trp Leu Ala Ser Thr
260 265

<210> 130
<211> 133
<212> PRT
<213> Homo sapiens

<400> 130

Met Arg Arg Ser Leu Arg Ala Gly Lys Arg Arg Gln Thr Ala Gly Arg
1 5 10 15

Lys Ser Lys Ser Pro Pro Lys Val Pro Ile Val Ile Gln Asp Asp Ser
20 25 30

Leu Pro Ala Gly Pro Pro Pro Gln Ile Arg Ile Leu Lys Arg Pro Thr
35 40 45

Ser Asn Gly Val Val Ser Ser Pro Asn Ser Thr Ser Arg Pro Thr Leu
50 55 60

Pro Val Lys Ser Leu Ala Gln Arg Glu Ala Glu Tyr Ala Glu Ala Arg
65 70 75 80

Lys Arg Ile Leu Gly Ser Ala Ser Pro Glu Glu Glu Gln Glu Lys Pro
85 90 95

Ile Leu Asp Arg Pro Thr Arg Ile Ser Gln Pro Glu Asp Ser Arg Gln
100 105 110

Pro Asn Asn Val Ile Arg Gln Pro Leu Gly Pro Asp Gly Ser Gln Gly
115 120 125

Phe Lys Gln Arg Arg
130

<210> 131
<211> 472
<212> PRT
<213> Homo sapiens

<400> 131

Met Lys Ser Ile Leu Asp Gly Leu Ala Asp Thr Thr Phe Arg Thr Ile
1 5 10 15

Thr Thr Asp Leu Leu Tyr Val Gly Ser Asn Asp Ile Gln Tyr Glu Asp
20 25 30

Ile Lys Gly Asp Met Ala Ser Lys Leu Gly Tyr Phe Pro Gln Lys Phe
35 40 45

Pro Leu Thr Ser Phe Arg Gly Ser Pro Phe Gln Glu Lys Met Thr Ala
50 55 60

Gly Asp Asn Pro Gln Leu Val Pro Ala Asp Gln Val Asn Ile Thr Glu
65 70 75 80

Phe Tyr Asn Lys Ser Leu Ser Ser Phe Lys Glu Asn Glu Glu Asn Ile
85 90 95

Gln Cys Gly Glu Asn Phe Met Asp Ile Glu Cys Phe Met Val Leu Asn
100 105 110

Pro Ser Gln Gln Leu Ala Ile Ala Val Leu Ser Leu Thr Leu Gly Thr
115 120 125

Phe Thr Val Leu Glu Asn Leu Leu Val Leu Cys Val Ile Leu His Ser
130 135 140

Arg Ser Leu Arg Cys Arg Pro Ser Tyr His Phe Ile Gly Ser Leu Ala
145 150 155 160

Val Ala Asp Leu Leu Gly Ser Val Ile Phe Val Tyr Ser Phe Ile Asp
165 170 175

Phe His Val Phe His Arg Lys Asp Ser Arg Asn Val Phe Leu Phe Lys
180 185 190

Leu Gly Gly Val Thr Ala Ser Phe Thr Ala Ser Val Gly Ser Leu Phe
195 200 205

Leu Thr Ala Ile Asp Arg Tyr Ile Ser Ile His Arg Pro Leu Ala Tyr
210 215 220

Lys Arg Ile Val Thr Arg Pro Lys Ala Val Val Ala Phe Cys Leu Met
225 230 235 240

Trp Thr Ile Ala Ile Val Ile Ala Val Leu Pro Leu Leu Gly Trp Asn
245 250 255

Cys Glu Lys Leu Gln Ser Val Cys Ser Asp Ile Phe Pro His Ile Asp
260 265 270

Glu Thr Tyr Leu Met Phe Trp Ile Gly Val Thr Ser Val Leu Leu Leu
275 280 285

Phe Ile Val Tyr Ala Tyr Met Tyr Ile Leu Trp Lys Ala His Ser His
290 295 300

Ala Val Arg Met Ile Gln Arg Gly Thr Gln Lys Ser Ile Ile Ile His
305 310 315 320

Thr Ser Glu Asp Gly Lys Val Gln Val Thr Arg Pro Asp Gln Ala Arg
325 330 335

Met Asp Ile Arg Leu Ala Lys Thr Leu Val Leu Ile Leu Val Val Leu
340 345 350

Ile Ile Cys Trp Gly Pro Leu Leu Ala Ile Met Val Tyr Asp Val Phe
355 360 365

Gly Lys Met Asn Lys Leu Ile Lys Thr Val Phe Ala Phe Cys Ser Met
370 375 380

Leu Cys Leu Leu Asn Ser Thr Val Asn Pro Ile Ile Tyr Ala Leu Arg
385 390 395 400

Ser Lys Asp Leu Arg His Ala Phe Arg Ser Met Phe Pro Ser Cys Glu
405 410 415

Gly Thr Ala Gln Pro Leu Asp Asn Ser Met Gly Asp Ser Asp Cys Leu
420 425 430

His Lys His Ala Asn Asn Ala Ala Ser Val His Arg Ala Ala Glu Ser
435 440 445

Cys Ile Lys Ser Thr Val Lys Ile Ala Lys Val Thr Met Ser Val Ser
450 455 460

Thr Asp Thr Ser Ala Glu Ala Leu
465 470

<210> 132
<211> 361
<212> PRT
<213> Homo sapiens

<400> 132

Met Ser Thr Ala Arg Glu Gln Pro Ile Phe Ser Thr Arg Ala His Val
1 5 10 15

Phe Gln Ile Asp Pro Ala Thr Lys Arg Asn Trp Ile Pro Ala Gly Lys
20 25 30

His Ala Leu Thr Val Ser Tyr Phe Tyr Asp Ala Thr Arg Asn Val Tyr
35 40 45

Arg Ile Ile Ser Ile Gly Gly Ala Lys Ala Ile Ile Asn Ser Thr Val
50 55 60

Thr Pro Asn Met Thr Phe Thr Lys Thr Ser Gln Lys Phe Gly Gln Trp
65 70 75 80

Ala Asp Ser Arg Ala Asn Thr Val Tyr Gly Leu Gly Phe Ala Ser Glu
85 90 95

Gln His Leu Thr Gln Phe Ala Glu Lys Phe Gln Glu Val Lys Glu Ala
100 105 110

Ala Arg Leu Ala Arg Glu Lys Ser Gln Asp Gly Gly Glu Leu Thr Ser
115 120 125

Pro Ala Leu Gly Leu Ala Ser His Gln Val Pro Pro Ser Pro Leu Val
130 135 140

Ser Ala Asn Gly Pro Gly Glu Glu Lys Leu Phe Arg Ser Gln Ser Ala
145 150 155 160

Asp Ala Pro Gly Pro Thr Glu Arg Glu Arg Leu Lys Lys Met Leu Ser
165 170 175

Glu Gly Ser Val Gly Glu Val Gln Trp Glu Ala Glu Phe Phe Ala Leu
180 185 190

Gln Asp Ser Asn Asn Lys Leu Ala Gly Ala Leu Arg Glu Ala Asn Ala
195 200 205

Ala Ala Ala Gln Trp Arg Gln Gln Leu Glu Ala Gln Arg Ala Glu Ala
210 215 220

Glu Arg Leu Arg Gln Arg Val Ala Glu Leu Glu Ala Gln Ala Ala Ser
225 230 235 240

Glu Val Thr Pro Thr Gly Glu Lys Glu Gly Leu Gly Gln Gly Gln Ser
245 250 255

Leu Glu Gln Leu Glu Ala Leu Val Gln Thr Lys Asp Gln Glu Ile Gln
260 265 270

Thr Leu Lys Ser Gln Thr Gly Gly Pro Arg Glu Ala Leu Glu Ala Ala
275 280 285

Glu Arg Glu Glu Thr Gln Gln Lys Val Gln Asp Leu Glu Thr Arg Asn
290 295 300

Ala Glu Leu Glu His Gln Leu Arg Ala Met Glu Arg Ser Leu Glu Glu
305 310 315 320

Ala Arg Ala Glu Arg Glu Arg Ala Arg Ala Glu Val Gly Arg Ala Ala
325 330 335

Gln Leu Leu Asp Val Arg Leu Phe Glu Leu Ser Glu Leu Arg Glu Gly
340 345 350

Leu Ala Arg Leu Ala Glu Ala Ala Pro
355 360

<210> 133
<211> 362
<212> PRT
<213> Homo sapiens

<400> 133

Met Gly Thr Glu Ala Thr Glu Gln Val Ser Trp Gly His Tyr Ser Gly
1 5 10 15

Asp Glu Glu Asp Ala Tyr Ser Ala Glu Pro Leu Pro Glu Leu Cys Tyr
20 25 30

Lys Ala Asp Val Gln Ala Phe Ser Arg Ala Phe Gln Pro Ser Val Ser
35 40 45

Leu Thr Val Ala Ala Leu Gly Leu Ala Gly Asn Gly Leu Val Leu Ala
50 55 60

Thr His Leu Ala Ala Arg Arg Ala Ala Arg Ser Pro Thr Ser Ala His
65 70 75 80

Leu Leu Gln Leu Ala Leu Ala Asp Leu Leu Leu Ala Leu Thr Leu Pro
85 90 95

Phe Ala Ala Ala Gly Ala Leu Gln Gly Trp Ser Leu Gly Ser Ala Thr
100 105 110

Cys Arg Thr Ile Ser Gly Leu Tyr Ser Ala Ser Phe His Ala Gly Phe
115 120 125

Leu Phe Leu Ala Cys Ile Ser Ala Asp Arg Tyr Val Ala Ile Ala Arg
130 135 140

Ala Leu Pro Ala Gly Pro Arg Pro Ser Thr Pro Gly Arg Ala His Leu
145 150 155 160

Val Ser Val Ile Val Trp Leu Leu Ser Leu Leu Leu Ala Leu Pro Ala
165 170 175

Leu Leu Phe Ser Gln Asp Gly Gln Arg Glu Gly Gln Arg Arg Cys Arg
180 185 190

Leu Ile Phe Pro Glu Gly Leu Thr Gln Thr Val Lys Gly Ala Ser Ala
195 200 205

Val Ala Gln Val Ala Leu Gly Phe Ala Leu Pro Leu Gly Val Met Val
210 215 220

Ala Cys Tyr Ala Leu Leu Gly Arg Thr Leu Leu Ala Ala Arg Gly Pro
225 230 235 240

Glu Arg Arg Arg Ala Leu Arg Val Val Val Ala Leu Val Ala Ala Phe
245 250 255

Val Val Leu Gln Leu Pro Tyr Ser Leu Ala Leu Leu Leu Asp Thr Ala
260 265 270

Asp Leu Leu Ala Ala Arg Glu Arg Ser Cys Pro Ala Ser Lys Arg Lys
275 280 285

Asp Val Ala Leu Leu Val Thr Ser Gly Leu Ala Leu Ala Arg Cys Gly
290 295 300

Leu Asn Pro Val Leu Tyr Ala Phe Leu Gly Leu Arg Phe Arg Gln Asp
305 310 315 320

Leu Arg Arg Leu Leu Arg Gly Gly Ser Ser Pro Ser Gly Pro Gln Pro
325 330 335

Arg Arg Gly Cys Pro Arg Arg Pro Arg Leu Ser Ser Cys Ser Ala Pro
340 345 350

Thr Glu Thr His Ser Leu Ser Trp Asp Asn
355 360

<210> 134
<211> 520
<212> PRT
<213> Homo sapiens

<400> 134

Met Arg Val Glu Ser Gly Ser Ala Gln Glu Arg Gly Ile Leu Leu Glu
1 5 10 15

Ser Leu Ser Thr Leu Leu Glu Lys Thr Thr Ala Ser His Glu Gly Arg
20 25 30

Ala Pro Gly Asn Arg Glu Leu Thr Asp Leu Leu Pro Pro Glu Val Cys
35 40 45

Ser Leu Leu Asn Pro Ala Ala Ile Tyr Ala Asn Asn Glu Ile Ser Leu
50 55 60

Arg Asp Val Glu Val Tyr Gly Phe Asp Tyr Asp Tyr Thr Leu Ala Gln
65 70 75 80

Tyr Ala Asp Ala Leu His Pro Glu Ile Phe Ser Thr Ala Arg Asp Ile
85 90 95

Leu Ile Glu His Tyr Lys Tyr Pro Glu Gly Ile Arg Lys Tyr Asp Tyr
100 105 110

Asn Pro Ser Phe Ala Ile Arg Gly Leu His Tyr Asp Ile Gln Lys Ser
115 120 125

Leu Leu Met Lys Ile Asp Ala Phe His Tyr Val Gln Leu Gly Thr Ala
130 135 140

Tyr Arg Gly Leu Gln Pro Val Pro Asp Glu Glu Val Ile Glu Leu Tyr
145 150 155 160

Gly Gly Thr Gln His Ile Pro Leu Tyr Gln Met Ser Gly Phe Tyr Gly
165 170 175

Lys Gly Pro Ser Ile Lys Gln Phe Met Asp Ile Phe Ser Leu Pro Glu
180 185 190

Met Ala Leu Leu Ser Cys Val Val Asp Tyr Phe Leu Gly His Ser Leu
195 200 205

Glu Phe Asp Gln Ala His Leu Tyr Lys Asp Val Thr Asp Ala Ile Arg
210 215 220

Asp Val His Val Lys Gly Leu Met Tyr Gln Trp Ile Glu Gln Asp Met
225 230 235 240

Glu Lys Tyr Ile Leu Arg Gly Asp Glu Thr Phe Ala Val Leu Ser Arg
245 250 255

Leu Val Ala His Gly Lys Gln Leu Phe Leu Ile Thr Asn Ser Pro Phe
260 265 270

Ser Phe Val Asp Lys Gly Met Arg His Met Val Gly Pro Asp Trp Arg
275 280 285

Gln Leu Phe Asp Val Val Ile Val Gln Ala Asp Lys Pro Ser Phe Phe
290 295 300

Thr Asp Arg Arg Lys Pro Phe Arg Lys Leu Asp Glu Lys Gly Ser Leu
305 310 315 320

Gln Trp Asp Arg Ile Thr Arg Leu Glu Lys Gly Lys Ile Tyr Arg Gln
325 330 335

Gly Asn Leu Phe Asp Phe Leu Arg Leu Thr Glu Trp Arg Gly Pro Arg
340 345 350

Val Leu Tyr Phe Gly Asp His Leu Tyr Ser Asp Leu Ala Asp Leu Met
355 360 365

Leu Arg His Gly Trp Arg Thr Gly Ala Ile Ile Pro Glu Leu Glu Arg
370 375 380

Glu Ile Arg Ile Ile Asn Thr Glu Gln Tyr Met His Ser Leu Thr Trp
385 390 395 400

Gln Gln Ala Leu Thr Gly Leu Leu Glu Arg Met Gln Thr Tyr Gln Asp
405 410 415

Ala Glu Ser Arg Gln Val Leu Ala Ala Trp Met Lys Glu Arg Gln Glu
420 425 430

Leu Arg Cys Ile Thr Lys Ala Leu Phe Asn Ala Gln Phe Gly Ser Ile
435 440 445

Phe Arg Thr Phe His Asn Pro Thr Tyr Phe Ser Arg Arg Leu Val Arg
450 455 460

Phe Ser Asp Leu Tyr Met Ala Ser Leu Ser Cys Leu Leu Asn Tyr Arg
465 470 475 480

Val Asp Phe Thr Phe Tyr Pro Arg Arg Thr Pro Leu Gln His Glu Ala
485 490 495

Pro Leu Trp Met Asp Gln Leu Cys Thr Gly Cys Met Lys Thr Pro Phe
500 505 510

Leu Gly Asp Met Ala His Ile Arg
515 520

<210> 135
<211> 322
<212> PRT
<213> Homo sapiens

<400> 135

Met Ala Arg Cys Phe Ser Leu Val Leu Leu Leu Thr Ser Ile Trp Thr
1 5 10 15

Thr Arg Leu Leu Val Gln Gly Ser Leu Arg Ala Glu Glu Leu Ser Ile
20 25 30

Gln Val Ser Cys Arg Ile Met Gly Ile Thr Leu Val Ser Lys Lys Ala
35 40 45

Asn Gln Gln Leu Asn Phe Thr Glu Ala Lys Glu Ala Cys Arg Leu Leu
50 55 60

Gly Leu Ser Leu Ala Gly Lys Asp Gln Val Glu Thr Ala Leu Lys Ala
65 70 75 80

Ser Phe Glu Thr Cys Ser Tyr Gly Trp Val Gly Asp Gly Phe Val Val
85 90 95

Ile Ser Arg Ile Ser Pro Asn Pro Lys Cys Gly Lys Asn Gly Val Gly
100 105 110

Val Leu Ile Trp Lys Val Pro Val Ser Arg Gln Phe Ala Ala Tyr Cys
115 120 125

Tyr Asn Ser Ser Asp Thr Trp Thr Asn Ser Cys Ile Pro Glu Ile Ile
130 135 140

Thr Thr Lys Asp Pro Ile Phe Asn Thr Gln Thr Ala Thr Gln Thr Thr
145 150 155 160

Glu Phe Ile Val Ser Asp Ser Thr Tyr Ser Val Ala Ser Pro Tyr Ser
165 170 175

Thr Ile Pro Ala Pro Thr Thr Thr Pro Pro Ala Pro Ala Ser Thr Ser
180 185 190

Ile Pro Arg Arg Lys Lys Leu Ile Cys Val Thr Glu Val Phe Met Glu
195 200 205

Thr Ser Thr Met Ser Thr Glu Thr Glu Pro Phe Val Glu Asn Lys Ala
210 215 220

Ala Phe Lys Asn Glu Ala Ala Gly Phe Gly Gly Val Pro Thr Ala Leu
225 230 235 240

Leu Val Leu Ala Leu Leu Phe Phe Gly Ala Ala Ala Gly Leu Gly Phe
245 250 255

Cys Tyr Val Lys Arg Tyr Val Lys Ala Phe Pro Phe Thr Asn Lys Asn
260 265 270

Gln Gln Lys Glu Met Ile Glu Thr Lys Val Val Lys Glu Glu Lys Ala
275 280 285

Asn Asp Ser Asn Pro Asn Glu Glu Ser Lys Lys Thr Asp Lys Asn Pro
290 295 300

Glu Glu Ser Lys Ser Pro Ser Lys Thr Thr Val Arg Cys Leu Glu Ala
305 310 315 320

Glu Val

<210> 136
<211> 1202
<212> PRT
<213> Homo sapiens

<400> 136

Met Gly Arg Glu Gln Asp Leu Ile Leu Ala Val Lys Asn Gly Asp Val
1 5 10 15

Thr Gly Val Gln Lys Leu Val Ala Lys Val Lys Ala Thr Lys Thr Lys
20 25 30

Leu Leu Gly Ser Thr Lys Arg Leu Asn Val Asn Tyr Gln Asp Ala Asp
35 40 45

Gly Phe Ser Ala Leu His His Ala Ala Leu Gly Gly Ser Leu Glu Leu
50 55 60

Ile Ala Leu Leu Leu Glu Ala Gln Ala Thr Val Asp Ile Lys Asp Ser
65 70 75 80

Asn Gly Met Arg Pro Leu His Tyr Ala Ala Trp Gln Gly Arg Leu Glu
85 90 95

Pro Val Arg Leu Leu Leu Arg Ala Ser Ala Ala Val Asn Ala Ala Ser
100 105 110

Leu Asp Gly Gln Ile Pro Leu His Leu Ala Ala Gln Tyr Gly His Tyr
115 120 125

Glu Val Ser Glu Met Leu Leu Gln His Gln Ser Asn Pro Cys Leu Val
130 135 140

Asn Lys Ala Lys Lys Thr Pro Leu Asp Leu Ala Cys Glu Phe Gly Arg
145 150 155 160

Leu Lys Val Ala Gln Leu Leu Leu Asn Ser His Leu Cys Val Ala Leu
165 170 175

Leu Glu Gly Glu Ala Lys Asp Pro Cys Asp Pro Asn Tyr Thr Thr Pro
180 185 190

Leu His Leu Ala Ala Lys Asn Gly His Arg Glu Val Ile Arg Gln Leu
195 200 205

Leu Arg Ala Gly Ile Glu Ile Asn Arg Gln Thr Lys Thr Gly Thr Ala
210 215 220

Leu His Glu Ala Ala Leu Tyr Gly Lys Thr Glu Val Val Arg Leu Leu
225 230 235 240

Leu Glu Gly Gly Val Asp Val Asn Ile Arg Asn Thr Tyr Asn Gln Thr
245 250 255

Ala Leu Asp Ile Val Asn Gln Phe Thr Thr Ser Gln Ala Ser Arg Glu
260 265 270

Ile Lys Gln Leu Leu Arg Glu Ala Ser Gly Ile Leu Lys Val Arg Ala
275 280 285

Leu Lys Asp Phe Trp Asn Leu His Asp Pro Thr Ala Leu Asn Val Arg
290 295 300

Ala Gly Asp Val Ile Thr Val Leu Glu Gln His Pro Asp Gly Arg Trp
305 310 315 320

Lys Gly His Ile His Glu Ser Gln Arg Gly Thr Asp Arg Ile Gly Tyr
325 330 335

Phe Pro Pro Gly Ile Val Glu Val Val Ser Lys Arg Val Gly Ile Pro
340 345 350

Ala Ala Arg Leu Pro Ser Ala Pro Thr Pro Leu Arg Pro Gly Phe Ser
355 360 365

Arg Thr Pro Gln Pro Pro Ala Glu Glu Pro Pro His Pro Leu Thr Tyr
370 375 380

Ser Gln Leu Pro Arg Val Gly Leu Ser Pro Asp Ser Pro Ala Gly Asp
385 390 395 400

Arg Asn Ser Val Gly Ser Glu Gly Ser Val Gly Ser Ile Arg Ser Ala
405 410 415

Gly Ser Gly Gln Ser Ser Glu Gly Thr Asn Gly His Gly Pro Gly Leu
420 425 430

Leu Ile Glu Asn Ala Gln Pro Leu Pro Ser Ala Gly Glu Asp Gln Val
435 440 445

Leu Pro Gly Leu His Pro Pro Ser Leu Ala Asp Asn Leu Ser His Arg
450 455 460

Pro Leu Ala Asn Cys Arg Ser Gly Glu Gln Ile Phe Thr Gln Asp Val
465 470 475 480

Arg Pro Glu Gln Leu Leu Glu Gly Lys Asp Ala Gln Ala Ile His Asn
485 490 495

Trp Leu Ser Glu Phe Gln Leu Glu Gly Tyr Thr Ala His Phe Leu Gln
500 505 510

Ala Gly Tyr Asp Val Pro Thr Ile Ser Arg Met Thr Pro Glu Asp Leu
515 520 525

Thr Ala Ile Gly Val Thr Lys Pro Gly His Arg Lys Lys Ile Ala Ser
530 535 540

Glu Ile Ala Gln Leu Ser Ile Ala Glu Trp Leu Pro Ser Tyr Ile Pro
545 550 555 560

Thr Asp Leu Leu Glu Trp Leu Cys Ala Leu Gly Leu Pro Gln Tyr His
565 570 575

Lys Gln Leu Val Ser Ser Gly Tyr Asp Ser Met Gly Leu Val Ala Asp
580 585 590

Leu Thr Trp Glu Glu Leu Gln Glu Ile Gly Val Asn Lys Leu Gly His
595 600 605

Gln Lys Lys Leu Met Leu Gly Val Lys Arg Leu Ala Glu Leu Arg Arg
610 615 620

Gly Leu Leu Gln Gly Glu Ala Leu Ser Glu Gly Gly Arg Arg Leu Ala
625 630 635 640

Lys Gly Pro Glu Leu Met Ala Ile Glu Gly Leu Glu Asn Gly Glu Gly
645 650 655

Pro Ala Thr Ala Gly Pro Arg Leu Leu Thr Phe Gln Gly Ser Glu Leu
660 665 670

Ser Pro Glu Leu Gln Ala Ala Met Ala Gly Gly Gly Pro Glu Pro Leu
675 680 685

Pro Leu Pro Pro Ala Arg Ser Pro Ser Gln Glu Ser Ile Gly Ala Arg
690 695 700

Ser Arg Gly Ser Gly His Ser Gln Glu Gln Pro Ala Pro Gln Pro Ser
705 710 715 720

Gly Gly Asp Pro Ser Pro Pro Gln Glu Arg Asn Leu Pro Glu Gly Thr
725 730 735

Glu Arg Pro Pro Lys Leu Cys Ser Ser Leu Pro Gly Gln Gly Pro Pro
740 745 750

Pro Tyr Val Phe Met Tyr Pro Gln Gly Ser Pro Ser Ser Pro Ala Pro
755 760 765

Gly Pro Pro Pro Gly Ala Pro Trp Ala Phe Ser Tyr Leu Ala Gly Pro
770 775 780

Pro Ala Thr Pro Pro Asp Pro Pro Arg Pro Lys Arg Arg Ser His Ser
785 790 795 800

Leu Ser Arg Pro Gly Pro Thr Glu Gly Asp Ala Glu Gly Glu Ala Glu
805 810 815

Gly Pro Val Gly Ser Thr Leu Gly Ser Tyr Ala Thr Leu Thr Arg Arg
820 825 830

Pro Gly Arg Ser Ala Leu Val Arg Thr Ser Pro Ser Val Thr Pro Thr
835 840 845

Pro Ala Arg Gly Thr Pro Arg Ser Gln Ser Phe Ala Leu Arg Ala Arg
850 855 860

Arg Lys Gly Pro Pro Pro Pro Pro Pro Lys Arg Leu Ser Ser Val Ser
865 870 875 880

Gly Pro Ser Pro Glu Pro Pro Pro Leu Asp Gly Ser Pro Gly Pro Lys
885 890 895

Glu Gly Ala Thr Gly Pro Arg Arg Arg Thr Leu Ser Glu Pro Ala Gly
900 905 910

Pro Ser Glu Pro Pro Gly Pro Pro Ala Pro Ala Gly Pro Ala Ser Asp
915 920 925

Thr Glu Glu Glu Glu Pro Gly Pro Glu Gly Thr Pro Pro Ser Arg Gly
930 935 940

Ser Ser Gly Glu Gly Leu Pro Phe Ala Glu Glu Gly Asn Leu Thr Ile
945 950 955 960

Lys Gln Arg Pro Lys Pro Ala Gly Pro Pro Pro Arg Glu Thr Pro Val
965 970 975

Pro Pro Gly Leu Asp Phe Asn Leu Thr Glu Ser Asp Thr Val Lys Arg
980 985 990

Arg Pro Lys Cys Arg Glu Arg Glu Pro Leu Gln Thr Ala Leu Leu Ala
995 1000 1005

Phe Gly Val Ala Ser Ala Thr Pro Gly Pro Ala Ala Pro Leu Pro
1010 1015 1020

Ser Pro Thr Pro Gly Glu Ser Pro Pro Ala Ser Ser Leu Pro Gln
1025 1030 1035

Pro Glu Pro Ser Ser Leu Pro Ala Gln Gly Val Pro Thr Pro Leu
1040 1045 1050

Ala Pro Ser Pro Ala Met Gln Pro Pro Val Pro Pro Cys Pro Gly
1055 1060 1065

Pro Gly Leu Glu Ser Ser Ala Ala Ser Arg Trp Asn Gly Glu Thr
1070 1075 1080

Glu Pro Pro Ala Ala Pro Ala Ala Leu Leu Lys Val Pro Gly Ala
1085 1090 1095

Gly Thr Ala Pro Lys Pro Val Ser Val Ala Cys Thr Gln Leu Ala
1100 1105 1110

Phe Ser Gly Pro Lys Leu Ala Pro Arg Leu Gly Pro Arg Pro Val
1115 1120 1125

Pro Pro Pro Arg Pro Glu Ser Thr Gly Thr Val Gly Pro Gly Gln
1130 1135 1140

Ala Gln Gln Arg Leu Glu Gln Thr Ser Ser Ser Leu Ala Ala Ala
1145 1150 1155

Leu Arg Ala Ala Glu Lys Ser Ile Gly Thr Lys Glu Gln Glu Gly
1160 1165 1170

Thr Pro Ser Ala Ser Thr Lys His Ile Leu Asp Asp Ile Ser Thr
1175 1180 1185

Met Phe Asp Ala Leu Ala Asp Gln Leu Asp Ala Met Leu Asp
1190 1195 1200

<210> 137
<211> 1496
<212> PRT
<213> Homo sapiens

<400> 137

Met Met Ala Asn Trp Ala Glu Ala Arg Pro Leu Leu Ile Leu Ile Val
1 5 10 15

Leu Leu Gly Gln Phe Val Ser Ile Lys Ala Gln Glu Glu Asp Glu Asp
20 25 30

Glu Gly Tyr Gly Glu Glu Ile Ala Cys Thr Gln Asn Gly Gln Met Tyr
35 40 45

Leu Asn Arg Asp Ile Trp Lys Pro Ala Pro Cys Gln Ile Cys Val Cys
50 55 60

Asp Asn Gly Ala Ile Leu Cys Asp Lys Ile Glu Cys Gln Asp Val Leu
65 70 75 80

Asp Cys Ala Asp Pro Val Thr Pro Pro Gly Glu Cys Cys Pro Val Cys
85 90 95

Ser Gln Thr Pro Gly Gly Gly Asn Thr Asn Phe Gly Arg Gly Arg Lys
100 105 110

Gly Gln Lys Gly Glu Pro Gly Leu Val Pro Val Val Thr Gly Ile Arg
115 120 125

Gly Arg Pro Gly Pro Ala Gly Pro Pro Gly Ser Gln Gly Pro Arg Gly
130 135 140

Glu Arg Gly Pro Lys Gly Arg Pro Gly Pro Arg Gly Pro Gln Gly Ile
145 150 155 160

Asp Gly Glu Pro Gly Val Pro Gly Gln Pro Gly Ala Pro Gly Pro Pro
165 170 175

Gly His Pro Ser His Pro Gly Pro Asp Gly Leu Ser Arg Pro Phe Ser
180 185 190

Ala Gln Met Ala Gly Leu Asp Glu Lys Ser Gly Leu Gly Ser Gln Val
195 200 205

Gly Leu Met Pro Gly Ser Val Gly Pro Val Gly Pro Arg Gly Pro Gln
210 215 220

Gly Leu Gln Gly Gln Gln Gly Gly Ala Gly Pro Thr Gly Pro Pro Gly
225 230 235 240

Glu Pro Gly Asp Pro Gly Pro Met Gly Pro Ile Gly Ser Arg Gly Pro
245 250 255

Glu Gly Pro Pro Gly Lys Pro Gly Glu Asp Gly Glu Pro Gly Arg Asn
260 265 270

Gly Asn Pro Gly Glu Val Gly Phe Ala Gly Ser Pro Gly Ala Arg Gly
275 280 285

Phe Pro Gly Ala Pro Gly Leu Pro Gly Leu Lys Gly His Arg Gly His
290 295 300

Lys Gly Leu Glu Gly Pro Lys Gly Glu Val Gly Ala Pro Gly Ser Lys
305 310 315 320

Gly Glu Ala Gly Pro Thr Gly Pro Met Gly Ala Met Gly Pro Leu Gly
325 330 335

Pro Arg Gly Met Pro Gly Glu Arg Gly Arg Leu Gly Pro Gln Gly Ala
340 345 350

Pro Gly Gln Arg Gly Ala His Gly Met Pro Gly Lys Pro Gly Pro Met
355 360 365

Gly Pro Leu Gly Ile Pro Gly Ser Ser Gly Phe Pro Gly Asn Pro Gly
370 375 380

Met Lys Gly Glu Ala Gly Pro Thr Gly Ala Arg Gly Pro Glu Gly Pro
385 390 395 400

Gln Gly Gln Arg Gly Glu Thr Gly Pro Pro Gly Pro Val Gly Ser Pro
405 410 415

Gly Leu Pro Gly Ala Ile Gly Thr Asp Gly Thr Pro Gly Pro Lys Gly
420 425 430

Pro Thr Gly Ser Pro Gly Thr Ser Gly Pro Pro Gly Ser Ala Gly Pro
435 440 445

Pro Gly Ser Pro Gly Pro Gln Gly Ser Thr Gly Pro Gln Gly Asn Ser
450 455 460

Gly Leu Pro Gly Asp Pro Gly Phe Lys Gly Glu Ala Gly Pro Lys Gly
465 470 475 480

Glu Pro Gly Pro His Gly Ile Gln Gly Pro Ile Gly Pro Pro Gly Glu
485 490 495

Glu Gly Lys Arg Gly Pro Arg Gly Asp Pro Gly Thr Leu Gly Pro Pro
500 505 510

Gly Pro Val Gly Glu Arg Gly Ala Pro Gly Asn Arg Gly Phe Pro Gly
515 520 525

Ser Asp Gly Leu Pro Gly Pro Lys Gly Ala Gln Gly Glu Arg Gly Pro
530 535 540

Val Gly Ser Ser Gly Pro Lys Gly Ser Gln Gly Asp Pro Gly Arg Pro
545 550 555 560

Gly Glu Pro Gly Leu Pro Gly Ala Arg Gly Leu Thr Gly Asn Pro Gly
565 570 575

Val Gln Gly Pro Glu Gly Lys Leu Gly Pro Leu Gly Ala Pro Gly Glu
580 585 590

Asp Gly Arg Pro Gly Pro Pro Gly Ser Ile Gly Ile Lys Gly Gln Pro
595 600 605

Gly Thr Met Gly Leu Pro Gly Pro Lys Gly Ser Asn Gly Asp Pro Gly
610 615 620

Lys Pro Gly Glu Ala Gly Asn Pro Gly Val Pro Gly Gln Arg Gly Ala
625 630 635 640

Pro Gly Lys Asp Gly Lys Val Gly Pro Tyr Gly Pro Pro Gly Pro Pro
645 650 655

Gly Leu Arg Gly Glu Arg Gly Glu Gln Gly Pro Pro Gly Pro Thr Gly
660 665 670

Phe Gln Gly His Pro Gly Pro Pro Gly Pro Pro Gly Glu Gly Gly Lys
675 680 685

Pro Gly Asp Gln Gly Val Pro Gly Gly Pro Gly Ala Val Gly Pro Leu
690 695 700

Gly Pro Arg Gly Glu Arg Gly Asn Pro Gly Glu Arg Gly Glu Pro Gly
705 710 715 720

Ile Thr Gly Leu Pro Gly Glu Lys Gly Met Ala Gly Gly His Gly Pro
725 730 735

Asp Gly Pro Lys Gly Ser Pro Gly Pro Ser Gly Thr Pro Gly Asp Thr
740 745 750

Gly Pro Pro Gly Leu Gln Gly Met Pro Gly Glu Arg Gly Ile Ala Gly
755 760 765

Thr Pro Gly Pro Lys Gly Asp Arg Gly Gly Ile Gly Glu Lys Gly Ala
770 775 780

Glu Gly Thr Ala Gly Asn Asp Gly Ala Gly Gly Leu Pro Gly Pro Leu
785 790 795 800

Gly Pro Pro Gly Pro Ala Gly Leu Leu Gly Glu Lys Gly Glu Pro Gly
805 810 815

Pro Arg Gly Leu Val Gly Pro Pro Gly Ser Arg Gly Asn Pro Gly Ser
820 825 830

Arg Gly Glu Asn Gly Pro Thr Gly Ala Val Gly Phe Ala Gly Pro Gln
835 840 845

Gly Ser Asp Gly Gln Pro Gly Val Lys Gly Glu Pro Gly Glu Pro Gly
850 855 860

Gln Lys Gly Asp Ala Gly Ser Pro Gly Pro Gln Gly Leu Ala Gly Ser
865 870 875 880

Pro Gly Pro His Gly Pro Asn Gly Val Pro Gly Leu Lys Gly Gly Arg
885 890 895

Gly Thr Gln Gly Pro Pro Gly Ala Thr Gly Phe Pro Gly Ser Ala Gly
900 905 910

Arg Val Gly Pro Pro Gly Pro Ala Gly Ala Pro Gly Pro Ala Gly Pro
915 920 925

Leu Gly Glu Pro Gly Lys Glu Gly Pro Pro Gly Pro Arg Gly Asp Pro
930 935 940

Gly Ser His Gly Arg Val Gly Val Arg Gly Pro Ala Gly Pro Pro Gly
945 950 955 960

Gly Pro Gly Asp Lys Gly Asp Pro Gly Glu Asp Gly Gln Pro Gly Pro
965 970 975

Asp Gly Pro Pro Gly Pro Ala Gly Thr Thr Gly Gln Arg Gly Ile Val
980 985 990

Gly Met Pro Gly Gln Arg Gly Glu Arg Gly Met Pro Gly Leu Pro Gly
995 1000 1005

Pro Ala Gly Thr Pro Gly Lys Val Gly Pro Thr Gly Ala Thr Gly
1010 1015 1020

Asp Lys Gly Pro Pro Gly Pro Val Gly Pro Pro Gly Ser Asn Gly
1025 1030 1035

Pro Val Gly Glu Pro Gly Pro Glu Gly Pro Ala Gly Asn Asp Gly
1040 1045 1050

Thr Pro Gly Arg Asp Gly Ala Val Gly Glu Arg Gly Asp Arg Gly
1055 1060 1065

Asp Pro Gly Pro Ala Gly Leu Pro Gly Ser Gln Gly Ala Pro Gly
1070 1075 1080

Thr Pro Gly Pro Val Gly Ala Pro Gly Asp Ala Gly Gln Arg Gly
1085 1090 1095

Asp Pro Gly Ser Arg Gly Pro Ile Gly His Leu Gly Arg Ala Gly
1100 1105 1110

Lys Arg Gly Leu Pro Gly Pro Gln Gly Pro Arg Gly Asp Lys Gly
1115 1120 1125

Asp His Gly Asp Arg Gly Asp Arg Gly Gln Lys Gly His Arg Gly
1130 1135 1140

Phe Thr Gly Leu Gln Gly Leu Pro Gly Pro Pro Gly Pro Asn Gly
1145 1150 1155

Glu Gln Gly Ser Ala Gly Ile Pro Gly Pro Phe Gly Pro Arg Gly
1160 1165 1170

Pro Pro Gly Pro Val Gly Pro Ser Gly Lys Glu Gly Asn Pro Gly
1175 1180 1185

Pro Leu Gly Pro Leu Gly Pro Pro Gly Val Arg Gly Ser Val Gly
1190 1195 1200

Glu Ala Gly Pro Glu Gly Pro Pro Gly Glu Pro Gly Pro Pro Gly
1205 1210 1215

Pro Pro Gly Pro Pro Gly His Leu Thr Ala Ala Leu Gly Asp Ile
1220 1225 1230

Met Gly His Tyr Asp Glu Ser Met Pro Asp Pro Leu Pro Glu Phe
1235 1240 1245

Thr Glu Asp Gln Ala Ala Pro Asp Asp Lys Asn Lys Thr Asp Pro
1250 1255 1260

Gly Val His Ala Thr Leu Lys Ser Leu Ser Ser Gln Ile Glu Thr
1265 1270 1275

Met Arg Ser Pro Asp Gly Ser Lys Lys His Pro Ala Arg Thr Cys
1280 1285 1290

Asp Asp Leu Lys Leu Cys His Ser Ala Lys Gln Ser Gly Glu Tyr
1295 1300 1305

Trp Ile Asp Pro Asn Gln Gly Ser Val Glu Asp Ala Ile Lys Val
1310 1315 1320

Tyr Cys Asn Met Glu Thr Gly Glu Thr Cys Ile Ser Ala Asn Pro
1325 1330 1335

Ser Ser Val Pro Arg Lys Thr Trp Trp Ala Ser Lys Ser Pro Asp
1340 1345 1350

Asn Lys Pro Val Trp Tyr Gly Leu Asp Met Asn Arg Gly Ser Gln
1355 1360 1365

Phe Ala Tyr Gly Asp His Gln Ser Pro Asn Thr Ala Ile Thr Gln
1370 1375 1380

Met Thr Phe Leu Arg Leu Leu Ser Lys Glu Ala Ser Gln Asn Ile
1385 1390 1395

Thr Tyr Ile Cys Lys Asn Ser Val Gly Tyr Met Asp Asp Gln Ala
1400 1405 1410

Lys Asn Leu Lys Lys Ala Val Val Leu Lys Gly Ala Asn Asp Leu
1415 1420 1425

Asp Ile Lys Ala Glu Gly Asn Ile Arg Phe Arg Tyr Ile Val Leu
1430 1435 1440

Gln Asp Thr Cys Ser Lys Arg Asn Gly Asn Val Gly Lys Thr Val
1445 1450 1455

Phe Glu Tyr Arg Thr Gln Asn Val Ala Arg Leu Pro Ile Ile Asp
1460 1465 1470

Leu Ala Pro Val Asp Val Gly Gly Thr Asp Gln Glu Phe Gly Val
1475 1480 1485

Glu Ile Gly Pro Val Cys Phe Val
1490 1495

<210> 138
<211> 726
<212> PRT
<213> Homo sapiens

<400> 138

Met Ser Thr Ala Thr Thr Val Ala Pro Ala Gly Ile Pro Ala Thr Pro
1 5 10 15

Gly Pro Val Asn Pro Pro Pro Pro Glu Val Ser Asn Pro Ser Lys Pro
20 25 30

Gly Arg Lys Thr Asn Gln Leu Gln Tyr Met Gln Asn Val Val Val Lys
35 40 45

Thr Leu Trp Lys His Gln Phe Ala Trp Pro Phe Tyr Gln Pro Val Asp
50 55 60

Ala Ile Lys Leu Asn Leu Pro Asp Tyr His Lys Ile Ile Lys Asn Pro
65 70 75 80

Met Asp Met Gly Thr Ile Lys Lys Arg Leu Glu Asn Asn Tyr Tyr Trp
85 90 95

Ser Ala Ser Glu Cys Met Gln Asp Phe Asn Thr Met Phe Thr Asn Cys
100 105 110

Tyr Ile Tyr Asn Lys Pro Thr Asp Asp Ile Val Leu Met Ala Gln Ala
115 120 125

Leu Glu Lys Ile Phe Leu Gln Lys Val Ala Gln Met Pro Gln Glu Glu
130 135 140

Val Glu Leu Leu Pro Pro Ala Pro Lys Gly Lys Gly Arg Lys Pro Ala
145 150 155 160

Ala Gly Ala Gln Ser Ala Gly Thr Gln Gln Val Ala Ala Val Ser Ser
165 170 175

Val Ser Pro Ala Thr Pro Phe Gln Ser Val Pro Pro Thr Val Ser Gln
180 185 190

Thr Pro Val Ile Ala Ala Thr Pro Val Pro Thr Ile Thr Ala Asn Val
195 200 205

Thr Ser Val Pro Val Pro Pro Ala Ala Ala Pro Pro Pro Pro Ala Thr
210 215 220

Pro Ile Val Pro Val Val Pro Pro Thr Pro Pro Val Val Lys Lys Lys
225 230 235 240

Gly Val Lys Arg Lys Ala Asp Thr Thr Thr Pro Thr Thr Ser Ala Ile
245 250 255

Thr Ala Ser Arg Ser Glu Ser Pro Pro Pro Leu Ser Asp Pro Lys Gln
260 265 270

Ala Lys Val Val Ala Arg Arg Glu Ser Gly Gly Arg Pro Ile Lys Pro
275 280 285

Pro Lys Lys Asp Leu Glu Asp Gly Glu Val Pro Gln His Ala Gly Lys
290 295 300

Lys Gly Lys Leu Ser Glu His Leu Arg Tyr Cys Asp Ser Ile Leu Arg
305 310 315 320

Glu Met Leu Ser Lys Lys His Ala Ala Tyr Ala Trp Pro Phe Tyr Lys
325 330 335

Pro Val Asp Ala Glu Ala Leu Glu Leu His Asp Tyr His Asp Ile Ile
340 345 350

Lys His Pro Met Asp Leu Ser Thr Val Lys Arg Lys Met Asp Gly Arg
355 360 365

Glu Tyr Pro Asp Ala Gln Gly Phe Ala Ala Asp Val Arg Leu Met Phe
370 375 380

Ser Asn Cys Tyr Lys Tyr Asn Pro Pro Asp His Glu Val Val Ala Met
385 390 395 400

Ala Arg Lys Leu Gln Asp Val Phe Glu Met Arg Phe Ala Lys Met Pro
405 410 415

Asp Glu Pro Val Glu Ala Pro Ala Leu Pro Ala Pro Ala Ala Pro Met
420 425 430

Val Ser Lys Gly Ala Glu Ser Ser Arg Ser Ser Glu Glu Ser Ser Ser
435 440 445

Asp Ser Gly Ser Ser Asp Ser Glu Glu Glu Arg Ala Thr Arg Leu Ala
450 455 460

Glu Leu Gln Glu Gln Leu Lys Ala Val His Glu Gln Leu Ala Ala Leu
465 470 475 480

Ser Gln Ala Pro Val Asn Lys Pro Lys Lys Lys Lys Glu Lys Lys Glu
485 490 495

Lys Glu Lys Lys Lys Lys Asp Lys Glu Lys Glu Lys Glu Lys His Lys
500 505 510

Val Lys Ala Glu Glu Glu Lys Lys Ala Lys Val Ala Pro Pro Ala Lys
515 520 525

Gln Ala Gln Gln Lys Lys Ala Pro Ala Lys Lys Ala Asn Ser Thr Thr
530 535 540

Thr Ala Gly Arg Gln Leu Lys Lys Gly Gly Lys Gln Ala Ser Ala Ser
545 550 555 560

Tyr Asp Ser Glu Glu Glu Glu Glu Gly Leu Pro Met Ser Tyr Asp Glu
565 570 575

Lys Arg Gln Leu Ser Leu Asp Ile Asn Arg Leu Pro Gly Glu Lys Leu
580 585 590

Gly Arg Val Val His Ile Ile Gln Ser Arg Glu Pro Ser Leu Arg Asp
595 600 605

Ser Asn Pro Asp Glu Ile Glu Ile Asp Phe Glu Thr Leu Lys Pro Thr
610 615 620

Thr Leu Arg Glu Leu Glu Arg Tyr Val Lys Ser Cys Leu Gln Lys Lys
625 630 635 640

Gln Arg Lys Pro Phe Ser Ala Ser Gly Lys Lys Gln Ala Ala Lys Ser
645 650 655

Lys Glu Glu Leu Ala Gln Glu Lys Lys Lys Glu Leu Glu Lys Arg Leu
660 665 670

Gln Asp Val Ser Gly Gln Leu Ser Ser Ser Lys Lys Pro Ala Arg Lys
675 680 685

Glu Lys Pro Gly Ser Ala Pro Ser Gly Gly Pro Ser Arg Leu Ser Ser
690 695 700

Ser Ser Ser Ser Glu Ser Gly Ser Ser Ser Ser Ser Gly Ser Ser Ser
705 710 715 720

Asp Ser Ser Asp Ser Glu
725

<210> 139
<211> 840
<212> PRT
<213> Homo sapiens

<400> 139

Met Ala Ser Val Phe Arg Ser Glu Glu Met Cys Leu Ser Gln Leu Phe
1 5 10 15

Leu Gln Val Glu Ala Ala Tyr Cys Cys Val Ala Glu Leu Gly Glu Leu
20 25 30

Gly Leu Val Gln Phe Lys Asp Leu Asn Met Asn Val Asn Ser Phe Gln
35 40 45

Arg Lys Phe Val Asn Glu Val Arg Arg Cys Glu Ser Leu Glu Arg Ile
50 55 60

Leu Arg Phe Leu Glu Asp Glu Met Gln Asn Glu Ile Val Val Gln Leu
65 70 75 80

Leu Glu Lys Ser Pro Leu Thr Pro Leu Pro Arg Glu Met Ile Thr Leu
85 90 95

Glu Thr Val Leu Glu Lys Leu Glu Gly Glu Leu Gln Glu Ala Asn Gln
100 105 110

Asn Gln Gln Ala Leu Lys Gln Ser Phe Leu Glu Leu Thr Glu Leu Lys
115 120 125

Tyr Leu Leu Lys Lys Thr Gln Asp Phe Phe Glu Thr Glu Thr Asn Leu
130 135 140

Ala Asp Asp Phe Phe Thr Glu Asp Thr Ser Gly Leu Leu Glu Leu Lys
145 150 155 160

Ala Val Pro Ala Tyr Met Thr Gly Lys Leu Gly Phe Ile Ala Gly Val
165 170 175

Ile Asn Arg Glu Arg Met Ala Ser Phe Glu Arg Leu Leu Trp Arg Ile
180 185 190

Cys Arg Gly Asn Val Tyr Leu Lys Phe Ser Glu Met Asp Ala Pro Leu
195 200 205

```
Glu Asp Pro Val Thr Lys Glu Glu Ile Gln Lys Asn Ile Phe Ile Ile
    210                 215                 220

Phe Tyr Gln Gly Glu Gln Leu Arg Gln Lys Ile Lys Lys Ile Cys Asp
225                 230                 235                 240

Gly Phe Arg Ala Thr Val Tyr Pro Cys Pro Glu Pro Ala Val Glu Arg
                245                 250                 255

Arg Glu Met Leu Glu Ser Val Asn Val Arg Leu Glu Asp Leu Ile Thr
            260                 265                 270

Val Ile Thr Gln Thr Glu Ser His Arg Gln Arg Leu Leu Gln Glu Ala
        275                 280                 285

Ala Ala Asn Trp His Ser Trp Leu Ile Lys Val Gln Lys Met Lys Ala
    290                 295                 300

Val Tyr His Ile Leu Asn Met Cys Asn Ile Asp Val Thr Gln Gln Cys
305                 310                 315                 320

Val Ile Ala Glu Ile Trp Phe Pro Val Ala Asp Ala Thr Arg Ile Lys
                325                 330                 335

Arg Ala Leu Glu Gln Gly Met Glu Leu Ser Gly Ser Ser Met Ala Pro
            340                 345                 350

Ile Met Thr Thr Val Gln Ser Lys Thr Ala Pro Pro Thr Phe Asn Arg
        355                 360                 365

Thr Asn Lys Phe Thr Ala Gly Phe Gln Asn Ile Val Asp Ala Tyr Gly
    370                 375                 380

Val Gly Ser Tyr Arg Glu Ile Asn Pro Ala Pro Tyr Thr Ile Ile Thr
385                 390                 395                 400

Phe Pro Phe Leu Phe Ala Val Met Phe Gly Asp Cys Gly His Gly Thr
                405                 410                 415

Val Met Leu Leu Ala Ala Leu Trp Met Ile Leu Asn Glu Arg Arg Leu
            420                 425                 430

Leu Ser Gln Lys Thr Asp Asn Glu Ile Trp Asn Thr Phe Phe His Gly
        435                 440                 445

Arg Tyr Leu Ile Leu Leu Met Gly Ile Phe Ser Ile Tyr Thr Gly Leu
    450                 455                 460

Ile Tyr Asn Asp Cys Phe Ser Lys Ser Leu Asn Ile Phe Gly Ser Ser
465                 470                 475                 480
```

Trp Ser Val Gln Pro Met Phe Arg Asn Gly Thr Trp Asn Thr His Val
485 490 495

Met Glu Glu Ser Leu Tyr Leu Gln Leu Asp Pro Ala Ile Pro Gly Val
500 505 510

Tyr Phe Gly Asn Pro Tyr Pro Phe Gly Ile Asp Pro Ile Trp Asn Leu
515 520 525

Ala Ser Asn Lys Leu Thr Phe Leu Asn Ser Tyr Lys Met Lys Met Ser
530 535 540

Val Ile Leu Gly Ile Val Gln Met Val Phe Gly Val Ile Leu Ser Leu
545 550 555 560

Phe Asn His Ile Tyr Phe Arg Arg Thr Leu Asn Ile Ile Leu Gln Phe
565 570 575

Ile Pro Glu Met Ile Phe Ile Leu Cys Leu Phe Gly Tyr Leu Val Phe
580 585 590

Met Ile Ile Phe Lys Trp Cys Cys Phe Asp Val His Val Ser Gln His
595 600 605

Ala Pro Ser Ile Leu Ile His Phe Ile Asn Met Phe Leu Phe Asn Tyr
610 615 620

Ser Asp Ser Ser Asn Ala Pro Leu Tyr Lys His Gln Gln Glu Val Gln
625 630 635 640

Ser Phe Phe Val Val Met Ala Leu Ile Ser Val Pro Trp Met Leu Leu
645 650 655

Ile Lys Pro Phe Ile Leu Arg Ala Ser His Arg Lys Ser Gln Leu Gln
660 665 670

Ala Ser Arg Ile Gln Glu Asp Ala Thr Glu Asn Ile Glu Gly Asp Ser
675 680 685

Ser Ser Pro Ser Ser Arg Ser Gly Gln Arg Thr Ser Ala Asp Thr His
690 695 700

Gly Ala Leu Asp Asp His Gly Glu Glu Phe Asn Phe Gly Asp Val Phe
705 710 715 720

Val His Gln Ala Ile His Thr Ile Glu Tyr Cys Leu Gly Cys Ile Ser
725 730 735

Asn Thr Ala Ser Tyr Leu Arg Leu Trp Ala Leu Ser Leu Ala His Ala
740 745 750

Gln Leu Ser Glu Val Leu Trp Thr Met Val Met Asn Ser Gly Leu Gln
755 760 765

Thr Arg Gly Trp Gly Gly Ile Val Gly Val Phe Ile Ile Phe Ala Val
770 775 780

Phe Ala Val Leu Thr Val Ala Ile Leu Leu Ile Met Glu Gly Leu Ser
785 790 795 800

Ala Phe Leu His Ala Leu Arg Leu His Trp Val Glu Phe Gln Asn Lys
805 810 815

Phe Tyr Val Gly Asp Gly Tyr Lys Phe Ser Pro Phe Ser Phe Lys His
820 825 830

Ile Leu Asp Gly Thr Ala Glu Glu
835 840

<210> 140
<211> 826
<212> PRT
<213> Homo sapiens

<400> 140

Pro Ser Gly Leu Pro Leu Leu Pro Val Leu Phe Ala Leu Gly Gly Leu
1 5 10 15

Leu Leu Leu Ser Asn Ala Ser Cys Val Gly Gly Val Leu Trp Gln Arg
20 25 30

Arg Leu Arg Arg Leu Ala Glu Ala Leu Asn Phe Pro Pro His Leu His
35 40 45

Pro Gly Arg Ser Glu Glu Asp Arg Val Arg Asn Glu Tyr Glu Glu Ser
50 55 60

Gln Trp Thr Gly Glu Arg Asp Thr Gln Ser Ser Thr Val Ser Thr Thr
65 70 75 80

Glu Ala Glu Pro Tyr Tyr Arg Ser Leu Arg Asp Phe Ser Pro Gln Leu
85 90 95

Pro Pro Thr Gln Glu Glu Val Ser Tyr Ser Arg Gly Phe Thr Gly Glu
100 105 110

Asp Glu Asp Met Ala Phe Pro Gly His Leu Tyr Asp Glu Val Glu Arg
115 120 125

Thr Tyr Pro Pro Ser Gly Ala Trp Gly Pro Leu Tyr Asp Glu Val Gln
130 135 140

Met Gly Pro Trp Asp Leu His Trp Pro Glu Asp Thr Tyr Gln Asp Pro
145 150 155 160

Arg Gly Ile Tyr Asp Gln Val Ala Gly Asp Leu Asp Thr Leu Glu Pro
165 170 175

Asp Ser Leu Pro Phe Glu Leu Arg Gly His Leu Val Trp Gly Phe Asn
180 185 190

His Val Ser Gln Ala Gly Leu Lys Leu Leu Ala Ser Ser Asp Pro Pro
195 200 205

Ala Ser Ala Ser Gln Ser Ala Glu Ile Thr Glu Ser His Ser Val Val
210 215 220

Gln Val Gly Val Gln Trp Arg Tyr Phe Gly Ser Leu His Pro Leu Pro
225 230 235 240

Pro Gly Ser Arg Asp Ser Leu Ala Ser Ala Ser Arg Ile Ala Gly Ile
245 250 255

Thr Ala Pro Trp Glu Ala Glu Val Ser Arg Ser Pro Gln Gly Thr Gln
260 265 270

Asp Ser Pro Val Thr Arg Ser Gly Pro Pro Ser Arg Gly Trp Gln Ser
275 280 285

Leu Ser Phe Asp Gly Gly Ala Phe His Leu Lys Gly Thr Gly Glu Leu
290 295 300

Thr Arg Ala Leu Leu Val Leu Arg Leu Cys Ala Trp Pro Pro Leu Val
305 310 315 320

Thr His Gly Leu Leu Leu Gln Ala Trp Ser Arg Arg Leu Leu Gly Ser
325 330 335

Arg Leu Ser Gly Ala Phe Leu Arg Ala Ser Val Tyr Gly Gln Phe Val
340 345 350

Ala Gly Glu Thr Ala Glu Glu Val Lys Gly Cys Val Gln Gln Leu Arg
355 360 365

Thr Leu Ser Leu Arg Pro Leu Leu Ala Val Pro Thr Glu Glu Glu Pro
370 375 380

Asp Ser Ala Ala Lys Arg Met Arg Leu His His Val Gly Gln Ala Gly
385 390 395 400

Leu Glu Leu Leu Thr Pro Ala Ala Ser Gly Ser Val Ala Gln Ala Gly
405 410 415

Val Gln Trp Arg Gln Ser Ser Asp Arg Gly Gly Gly Asn Gln Ala Ala
420 425 430

Ala Ser Arg Ser Ser Leu Leu Gln Glu Ala Ala Phe Ser Pro Pro Cys
435 440 445

Gly Arg Leu Gln Leu Pro Ala Gln Pro Ala Ser Arg His Gly Ala Arg
450 455 460

Gly Arg Gly Ser Met Lys Ala Lys Ser Leu Thr Ser Arg His Leu Leu
465 470 475 480

Ala Ser Gln Gly Gln Glu Thr Ile Ile Lys Thr Lys Val Arg Ile Pro
485 490 495

Ala Leu Trp Lys Ala Glu Pro Gly Gln His Ser Lys Thr Pro Ser Gln
500 505 510

Gln Asn Lys Ser Gln Tyr Val Thr Thr Leu Trp Glu Ala Asp Val Gly
515 520 525

Arg Ser Leu Glu Asn Leu Gln Val Ser Cys Leu Asn Ala Glu Gln Asn
530 535 540

Gln His Leu Arg Ala Ser Leu Ser Arg Leu His Arg Val Thr Pro Pro
545 550 555 560

Ala Gly Thr Ser Thr Ser Gly Pro Pro Ser Ala Ala Cys Ile Gly Trp
565 570 575

His Ser Arg Leu His Arg Val Ala Gln Tyr Ala Arg Ala Gln His Val
580 585 590

Arg Leu Leu Val Asp Ala Glu Tyr Thr Ser Leu Asn Pro Ala Leu Ser
595 600 605

Leu Leu Val Ala Ala Leu Ala Val Arg Trp Asn Ser Pro Gly Glu Gly
610 615 620

Gly Pro Trp Val Trp Asn Thr Tyr Gln Ala Cys Leu Lys Asp Thr Phe
625 630 635 640

Glu Arg Leu Gly Arg Asp Ala Glu Ala Ala His Arg Ala Gly Leu Ala
645 650 655

Phe Gly Val Lys Leu Val Arg Gly Ala Tyr Leu Asp Lys Glu Arg Ala
660 665 670

Val Ala Gln Leu His Gly Met Glu Asp Pro Thr Gln Pro Asp Tyr Glu
675 680 685

Ala Thr Ser Glu Leu Asn Arg Ala Ser Pro Phe Ser Tyr Ser Arg Cys
690 695 700

Leu Glu Leu Met Leu Thr His Val Ala Arg His Gly Pro Met Cys His
705 710 715 720

Leu Met Val Ala Ser His Asn Glu Glu Ser Val Arg Gln Ala Thr Lys
725 730 735

Arg Met Trp Glu Leu Gly Ile Pro Leu Asp Gly Thr Val Cys Phe Gly
740 745 750

Gln Leu Leu Gly Met Cys Asp His Val Ser Leu Ala Leu Gly Gln Ala
755 760 765

Gly Tyr Val Val Tyr Lys Ser Ile Pro Tyr Gly Ser Leu Glu Glu Val
770 775 780

Ile Pro Tyr Leu Ile Arg Arg Ala Gln Glu Asn Arg Ser Val Leu Gln
785 790 795 800

Gly Ala Arg Arg Glu Gln Glu Leu Leu Ser Gln Glu Leu Trp Arg Arg
805 810 815

Leu Leu Pro Gly Cys Arg Arg Ile Pro His
820 825

<210> 141
<211> 987
<212> PRT
<213> Homo sapiens

<400> 141

Met Ala Leu Arg Arg Leu Gly Ala Ala Leu Leu Leu Leu Pro Leu Leu
1 5 10 15

Ala Ala Val Glu Glu Thr Leu Met Asp Ser Thr Thr Ala Thr Ala Glu
20 25 30

Leu Gly Trp Met Val His Pro Pro Ser Gly Trp Glu Glu Val Ser Gly
35 40 45

Tyr Asp Glu Asn Met Asn Thr Ile Arg Thr Tyr Gln Val Cys Asn Val
50 55 60

Phe Glu Ser Ser Gln Asn Asn Trp Leu Arg Thr Lys Phe Ile Arg Arg
65 70 75 80

Arg Gly Ala His Arg Ile His Val Glu Met Lys Phe Ser Val Arg Asp
85 90 95

Cys Ser Ser Ile Pro Ser Val Pro Gly Ser Cys Lys Glu Thr Phe Asn
100 105 110

Leu Tyr Tyr Tyr Glu Ala Asp Phe Asp Ser Ala Thr Lys Thr Phe Pro
115 120 125

Asn Trp Met Glu Asn Pro Trp Val Lys Val Asp Thr Ile Ala Ala Asp
130 135 140

Glu Ser Phe Ser Gln Val Asp Leu Gly Gly Arg Val Met Lys Ile Asn
145 150 155 160

Thr Glu Val Arg Ser Phe Gly Pro Val Ser Arg Ser Gly Phe Tyr Leu
165 170 175

Ala Phe Gln Asp Tyr Gly Gly Cys Met Ser Leu Ile Ala Val Arg Val
180 185 190

Phe Tyr Arg Lys Cys Pro Arg Ile Ile Gln Asn Gly Ala Ile Phe Gln
195 200 205

Glu Thr Leu Ser Gly Ala Glu Ser Thr Ser Leu Val Ala Ala Arg Gly
210 215 220

Ser Cys Ile Ala Asn Ala Glu Glu Val Asp Val Pro Ile Lys Leu Tyr
225 230 235 240

Cys Asn Gly Asp Gly Glu Trp Leu Val Pro Ile Gly Arg Cys Met Cys
245 250 255

Lys Ala Gly Phe Glu Ala Val Glu Asn Gly Thr Val Cys Arg Gly Cys
260 265 270

Pro Ser Gly Thr Phe Lys Ala Asn Gln Gly Asp Glu Ala Cys Thr His
275 280 285

Cys Pro Ile Asn Ser Arg Thr Thr Ser Glu Gly Ala Thr Asn Cys Val
290 295 300

Cys Arg Asn Gly Tyr Tyr Arg Ala Asp Leu Asp Pro Leu Asp Met Pro
305 310 315 320

Cys Thr Thr Ile Pro Ser Ala Pro Gln Ala Val Ile Ser Ser Val Asn
325 330 335

Glu Thr Ser Leu Met Leu Glu Trp Thr Pro Pro Arg Asp Ser Gly Gly
340 345 350

Arg Glu Asp Leu Val Tyr Asn Ile Ile Cys Lys Ser Cys Gly Ser Gly
355 360 365

Arg Gly Ala Cys Thr Arg Cys Gly Asp Asn Val Gln Tyr Ala Pro Arg
370 375 380

Gln Leu Gly Leu Thr Glu Pro Arg Ile Tyr Ile Ser Asp Leu Leu Ala
385 390 395 400

His Thr Gln Tyr Thr Phe Glu Ile Gln Ala Val Asn Gly Val Thr Asp
405 410 415

Gln Ser Pro Phe Ser Pro Gln Phe Ala Ser Val Asn Ile Thr Thr Asn
420 425 430

Gln Ala Ala Pro Ser Ala Val Ser Ile Met His Gln Val Ser Arg Thr
435 440 445

Val Asp Ser Ile Thr Leu Ser Trp Ser Gln Pro Asp Gln Pro Asn Gly
450 455 460

Val Ile Leu Asp Tyr Glu Leu Gln Tyr Tyr Glu Lys Glu Leu Ser Glu
465 470 475 480

Tyr Asn Ala Thr Ala Ile Lys Ser Pro Thr Asn Thr Val Thr Val Gln
485 490 495

Gly Leu Lys Ala Gly Ala Ile Tyr Val Phe Gln Val Arg Ala Arg Thr
500 505 510

Val Ala Gly Tyr Gly Arg Tyr Ser Gly Lys Met Tyr Phe Gln Thr Met
515 520 525

Thr Glu Ala Glu Tyr Gln Thr Ser Ile Gln Glu Lys Leu Pro Leu Ile
530 535 540

Ile Gly Ser Ser Ala Ala Gly Leu Val Phe Leu Ile Ala Val Val Val
545 550 555 560

Ile Ala Ile Val Cys Asn Arg Arg Arg Gly Phe Glu Arg Ala Asp Ser
565 570 575

Glu Tyr Thr Asp Lys Leu Gln His Tyr Thr Ser Gly His Met Thr Pro
580 585 590

Gly Met Lys Ile Tyr Ile Asp Pro Phe Thr Tyr Glu Asp Pro Asn Glu
595 600 605

Ala Val Arg Glu Phe Ala Lys Glu Ile Asp Ile Ser Cys Val Lys Ile
610 615 620

Glu Gln Val Ile Gly Ala Gly Glu Phe Gly Glu Val Cys Ser Gly His
625 630 635 640

Leu Lys Leu Pro Gly Lys Arg Glu Ile Phe Val Ala Ile Lys Thr Leu
645 650 655

Lys Ser Gly Tyr Thr Glu Lys Gln Arg Arg Asp Phe Leu Ser Glu Ala
660 665 670

Ser Ile Met Gly Gln Phe Asp His Pro Asn Val Ile His Leu Glu Gly
675 680 685

Val Val Thr Lys Ser Thr Pro Val Met Ile Ile Thr Glu Phe Met Glu
690 695 700

Asn Gly Ser Leu Asp Ser Phe Leu Arg Gln Asn Asp Gly Gln Phe Thr
705 710 715 720

Val Ile Gln Leu Val Gly Met Leu Arg Gly Ile Ala Ala Gly Met Lys
725 730 735

Tyr Leu Ala Asp Met Asn Tyr Val His Arg Asp Leu Ala Ala Arg Asn
740 745 750

Ile Leu Val Asn Ser Asn Leu Val Cys Lys Val Ser Asp Phe Gly Leu
755 760 765

Ser Arg Phe Leu Glu Asp Asp Thr Ser Asp Pro Thr Tyr Thr Ser Ala
770 775 780

Leu Gly Gly Lys Ile Pro Ile Arg Trp Thr Ala Pro Glu Ala Ile Gln
785 790 795 800

Tyr Arg Lys Phe Thr Ser Ala Ser Asp Val Trp Ser Tyr Gly Ile Val
805 810 815

Met Trp Glu Val Met Ser Tyr Gly Glu Arg Pro Tyr Trp Asp Met Thr
820 825 830

Asn Gln Asp Val Ile Asn Ala Ile Glu Gln Asp Tyr Arg Leu Pro Pro
835 840 845

Pro Met Asp Cys Pro Ser Ala Leu His Gln Leu Met Leu Asp Cys Trp
850 855 860

Gln Lys Asp Arg Asn His Arg Pro Lys Phe Gly Gln Ile Val Asn Thr
865 870 875 880

Leu Asp Lys Met Ile Arg Asn Pro Asn Ser Leu Lys Ala Met Ala Pro
885 890 895

Leu Ser Ser Gly Ile Asn Leu Pro Leu Leu Asp Arg Thr Ile Pro Asp
900 905 910

Tyr Thr Ser Phe Asn Thr Val Asp Glu Trp Leu Glu Ala Ile Lys Met
915 920 925

Gly Gln Tyr Lys Glu Ser Phe Ala Asn Ala Gly Phe Thr Ser Phe Asp
930 935 940

Val Val Ser Gln Met Met Met Glu Asp Ile Leu Arg Val Gly Val Thr
945 950 955 960

Leu Ala Gly His Gln Lys Lys Ile Leu Asn Ser Ile Gln Val Met Arg
965 970 975

Ala Gln Met Asn Gln Ile Gln Ser Val Glu Val
980 985

<210> 142
<211> 379
<212> PRT
<213> Homo sapiens

<400> 142

Met Val Phe Phe Thr Cys Asn Ala Cys Gly Glu Ser Val Lys Lys Ile
1 5 10 15

Gln Val Glu Lys His Val Ser Val Cys Arg Asn Cys Glu Cys Leu Ser
20 25 30

Cys Ile Asp Cys Gly Lys Asp Phe Trp Gly Asp Asp Tyr Lys Asn His
35 40 45

Val Lys Cys Ile Ser Glu Asp Gln Lys Tyr Gly Gly Lys Gly Tyr Glu
50 55 60

Gly Lys Thr His Lys Gly Asp Ile Lys Gln Gln Ala Trp Ile Gln Lys
65 70 75 80

Ile Ser Glu Leu Ile Lys Arg Pro Asn Val Ser Pro Lys Val Arg Glu
85 90 95

Leu Leu Glu Gln Ile Ser Ala Phe Asp Asn Val Pro Arg Lys Lys Ala
100 105 110

Lys Phe Gln Asn Trp Met Lys Asn Ser Leu Lys Val His Asn Glu Ser
115 120 125

Ile Leu Asp Gln Val Trp Asn Ile Phe Ser Glu Ala Ser Asn Ser Glu
130 135 140

Pro Val Asn Lys Glu Gln Asp Gln Arg Pro Leu His Pro Val Ala Asn
145 150 155 160

Pro His Ala Glu Ile Ser Thr Lys Val Pro Ala Ser Lys Val Lys Asp
165 170 175

Ala Val Glu Gln Gln Gly Glu Val Lys Lys Asn Lys Arg Glu Arg Lys
180 185 190

Glu Glu Arg Gln Lys Lys Arg Lys Arg Glu Lys Lys Glu Leu Lys Leu
195 200 205

Glu Asn His Gln Glu Asn Ser Arg Asn Gln Lys Pro Lys Lys Arg Lys
210 215 220

Lys Gly Gln Glu Ala Asp Leu Glu Ala Gly Gly Glu Glu Val Pro Glu
225 230 235 240

Ala Asn Gly Ser Ala Gly Lys Arg Ser Lys Lys Lys Lys Gln Arg Lys
245 250 255

Asp Ser Ala Ser Glu Glu Glu Ala Arg Val Gly Ala Gly Lys Arg Lys
260 265 270

Arg Arg His Ser Glu Val Glu Thr Asp Ser Lys Lys Lys Lys Met Lys
275 280 285

Leu Pro Glu His Pro Glu Gly Gly Glu Pro Glu Asp Asp Glu Ala Pro
290 295 300

Ala Lys Gly Lys Phe Asn Trp Lys Gly Thr Ile Lys Ala Ile Leu Lys
305 310 315 320

Gln Ala Pro Asp Asn Glu Ile Thr Ile Lys Lys Leu Arg Lys Lys Val
325 330 335

Leu Ala Gln Tyr Tyr Thr Val Thr Asp Glu His His Arg Ser Glu Glu
340 345 350

Glu Leu Leu Val Ile Phe Asn Lys Lys Ile Ser Lys Asn Pro Thr Phe
355 360 365

Lys Leu Leu Lys Asp Lys Val Lys Leu Val Lys
370 375

<210> 143

<211> 86
<212> PRT
<213> Homo sapiens

<400> 143

Met Ala Glu Asp Met Glu Thr Lys Ile Lys Asn Tyr Lys Thr Ala Pro
1 5 10 15

Phe Asp Ser Arg Phe Pro Asn Gln Asn Gln Thr Arg Asn Cys Trp Gln
20 25 30

Asn Tyr Leu Asp Phe His Arg Cys Gln Lys Ala Met Thr Ala Lys Gly
35 40 45

Gly Asp Ile Ser Val Cys Glu Trp Tyr Gln Arg Val Tyr Gln Ser Leu
50 55 60

Cys Pro Thr Ser Trp Val Thr Asp Trp Asp Glu Gln Arg Ala Glu Gly
65 70 75 80

Thr Phe Pro Gly Lys Ile
85

<210> 144
<211> 166
<212> PRT
<213> Homo sapiens

<400> 144

Met Leu Leu Ile Leu Leu Ser Val Ala Leu Leu Ala Phe Ser Ser Ala
1 5 10 15

Gln Asp Leu Asn Glu Asp Val Ser Gln Glu Asp Val Pro Leu Val Ile
20 25 30

Ser Asp Gly Gly Asp Ser Glu Gln Phe Ile Asp Glu Glu Arg Gln Gly
35 40 45

Pro Pro Leu Gly Gly Gln Gln Ser Gln Pro Ser Ala Gly Asp Gly Asn
50 55 60

Gln Asp Asp Gly Pro Gln Gln Gly Pro Pro Gln Gln Gly Gly Gln Gln
65 70 75 80

Gln Gln Gly Pro Pro Pro Pro Gln Gly Lys Pro Gln Gly Pro Pro Gln
85 90 95

Gln Gly Gly His Pro Pro Pro Pro Gln Gly Arg Pro Gln Gly Pro Pro
100 105 110

Gln Gln Gly Gly His Pro Arg Pro Pro Arg Gly Arg Pro Gln Gly Pro
115 120 125

Pro Gln Gln Gly Gly His Gln Gln Gly Pro Pro Pro Pro Pro Pro Gly
130 135 140

Lys Pro Gln Gly Pro Pro Pro Gln Gly Gly Arg Pro Gln Gly Pro Pro
145 150 155 160

Gln Gly Gln Ser Pro Gln
165

<210> 145
<211> 262
<212> PRT
<213> Homo sapiens

<400> 145

Met Asp Pro Arg Leu Ser Thr Val Arg Gln Thr Cys Cys Cys Phe Asn
1 5 10 15

Val Arg Ile Ala Thr Thr Ala Leu Ala Ile Tyr His Val Ile Met Ser
20 25 30

Val Leu Leu Phe Ile Glu His Ser Val Glu Val Ala His Gly Lys Ala
35 40 45

Ser Cys Lys Leu Ser Gln Met Gly Tyr Leu Arg Ile Ala Asp Leu Ile
50 55 60

Ser Ser Phe Leu Leu Ile Thr Met Leu Phe Ile Ile Ser Leu Ser Leu
65 70 75 80

Leu Ile Gly Val Val Lys Asn Arg Glu Lys Tyr Leu Leu Pro Phe Leu
85 90 95

Ser Leu Gln Ile Met Asp Tyr Leu Leu Cys Leu Leu Thr Leu Leu Gly
100 105 110

Ser Tyr Ile Glu Leu Pro Ala Tyr Leu Lys Leu Ala Ser Arg Ser Arg
115 120 125

Ala Ser Ser Ser Lys Phe Pro Leu Met Thr Leu Gln Leu Leu Asp Phe
130 135 140

Cys Leu Ser Ile Leu Thr Leu Cys Ser Ser Tyr Met Glu Val Pro Thr
145 150 155 160

Tyr Leu Asn Phe Lys Ser Met Asn His Met Asn Tyr Leu Pro Ser Gln
165 170 175

Glu Asp Met Pro His Asn Gln Phe Ile Lys Met Met Ile Ile Phe Ser
180 185 190

Ile Ala Phe Ile Thr Val Leu Ile Phe Lys Val Tyr Met Phe Lys Cys
195 200 205

Val Trp Arg Cys Tyr Arg Leu Ile Lys Cys Met Asn Ser Val Glu Glu
210 215 220

Lys Arg Asn Ser Lys Met Leu Gln Lys Val Val Leu Pro Ser Tyr Glu
225 230 235 240

Glu Ala Leu Ser Leu Pro Ser Lys Thr Pro Glu Gly Gly Pro Ala Pro
245 250 255

Pro Pro Tyr Ser Glu Val
260

<210> 146
<211> 772
<212> PRT
<213> Homo sapiens

<400> 146

Met Gly Asp Glu Asp Asp Asp Glu Ser Cys Ala Val Glu Leu Arg Ile
1 5 10 15

Thr Glu Ala Asn Leu Thr Gly His Glu Glu Lys Val Ser Val Glu Asn
20 25 30

Phe Glu Leu Leu Lys Val Leu Gly Thr Gly Ala Tyr Gly Lys Val Phe
35 40 45

Leu Val Arg Lys Ala Gly Gly His Asp Ala Gly Lys Leu Tyr Ala Met
50 55 60

Lys Val Leu Arg Lys Ala Ala Leu Val Gln Arg Ala Lys Thr Gln Glu
65 70 75 80

His Thr Arg Thr Glu Arg Ser Val Leu Glu Leu Val Arg Gln Ala Pro
85 90 95

Phe Leu Val Thr Leu His Tyr Ala Phe Gln Thr Asp Ala Lys Leu His
100 105 110

Leu Ile Leu Asp Tyr Val Ser Gly Gly Glu Met Phe Thr His Leu Tyr
115 120 125

Gln Arg Gln Tyr Phe Lys Glu Ala Glu Val Arg Val Tyr Gly Gly Glu
130 135 140

Ile Val Leu Ala Leu Glu His Leu His Lys Leu Gly Ile Ile Tyr Arg
145 150 155 160

Asp Leu Lys Leu Glu Asn Val Leu Leu Asp Ser Glu Gly His Ile Val
165 170 175

Leu Thr Asp Phe Gly Leu Ser Lys Glu Phe Leu Thr Glu Glu Lys Glu
180 185 190

Arg Thr Phe Ser Phe Cys Gly Thr Ile Glu Tyr Met Ala Pro Glu Ile
195 200 205

Ile Arg Ser Lys Thr Gly His Gly Lys Ala Val Asp Trp Trp Ser Leu
210 215 220

Gly Ile Leu Leu Phe Glu Leu Leu Thr Gly Ala Ser Pro Phe Thr Leu
225 230 235 240

Glu Gly Glu Arg Asn Thr Gln Ala Glu Val Ser Arg Arg Ile Leu Lys
245 250 255

Cys Ser Pro Pro Phe Pro Pro Arg Ile Gly Pro Val Ala Gln Asp Leu
260 265 270

Leu Gln Arg Leu Leu Cys Lys Asp Pro Lys Lys Arg Leu Gly Ala Gly
275 280 285

Pro Gln Gly Ala Gln Glu Val Arg Asn His Pro Phe Phe Gln Gly Leu
290 295 300

Asp Trp Val Ala Leu Ala Ala Arg Lys Ile Pro Ala Pro Phe Arg Pro
305 310 315 320

Gln Ile Arg Ser Glu Leu Asp Val Gly Asn Phe Ala Glu Glu Phe Thr
325 330 335

Arg Leu Glu Pro Val Tyr Ser Pro Pro Gly Ser Pro Pro Pro Gly Asp
340 345 350

Pro Arg Ile Phe Gln Gly Tyr Ser Phe Val Ala Pro Ser Ile Leu Phe
355 360 365

Asp His Asn Asn Ala Val Met Thr Asp Gly Leu Glu Ala Pro Gly Ala
370 375 380

Gly Asp Arg Pro Gly Arg Ala Ala Val Ala Arg Ser Ala Met Met Gln
385 390 395 400

Asp Ser Pro Phe Phe Gln Gln Tyr Glu Leu Asp Leu Arg Glu Pro Ala
405 410 415

Leu Gly Gln Gly Ser Phe Ser Val Cys Arg Arg Cys Arg Gln Arg Gln
420 425 430

Ser Gly Gln Glu Phe Ala Val Lys Ile Leu Ser Arg Arg Leu Glu Ala
435 440 445

Asn Thr Gln Arg Glu Val Ala Ala Leu Arg Leu Cys Gln Ser His Pro
450 455 460

Asn Val Val Asn Leu His Glu Val His His Asp Gln Leu His Thr Tyr
465 470 475 480

Leu Val Leu Glu Leu Leu Arg Gly Gly Glu Leu Leu Glu His Ile Arg
485 490 495

Lys Lys Arg His Phe Ser Glu Ser Glu Ala Ser Gln Ile Leu Arg Ser
500 505 510

Leu Val Ser Ala Val Ser Phe Met His Glu Glu Ala Gly Val Val His
515 520 525

Arg Asp Leu Lys Pro Glu Asn Ile Leu Tyr Ala Asp Asp Thr Pro Gly
530 535 540

Ala Pro Val Lys Ile Ile Asp Phe Gly Phe Ala Arg Leu Arg Pro Gln
545 550 555 560

Ser Pro Gly Val Pro Met Gln Thr Pro Cys Phe Thr Leu Gln Tyr Ala
565 570 575

Ala Pro Glu Leu Leu Ala Gln Gln Gly Tyr Asp Glu Ser Cys Asp Leu
580 585 590

Trp Ser Leu Gly Val Ile Leu Tyr Met Met Leu Ser Gly Gln Val Pro
595 600 605

Phe Gln Gly Ala Ser Gly Gln Gly Gly Gln Ser Gln Ala Ala Glu Ile
610 615 620

Met Cys Lys Ile Arg Glu Gly Arg Phe Ser Leu Asp Gly Glu Ala Trp
625 630 635 640

Gln Gly Val Ser Glu Glu Ala Lys Glu Leu Val Arg Gly Leu Leu Thr
645 650 655

Val Asp Pro Ala Lys Arg Leu Lys Leu Glu Gly Leu Arg Gly Ser Ser
660 665 670

Trp Leu Gln Asp Gly Ser Ala Arg Ser Ser Pro Pro Leu Arg Thr Pro
675 680 685

Asp Val Leu Glu Ser Ser Gly Pro Ala Val Arg Ser Gly Leu Asn Ala
690 695 700

Thr Phe Met Ala Phe Asn Arg Gly Lys Arg Glu Gly Phe Phe Leu Lys
705 710 715 720

Ser Val Glu Asn Ala Pro Leu Ala Lys Arg Arg Lys Gln Lys Leu Arg
725 730 735

Ser Ala Thr Ala Ser Arg Arg Gly Ser Pro Ala Pro Ala Asn Pro Gly
740 745 750

Arg Ala Pro Val Ala Ser Lys Gly Ala Pro Arg Arg Ala Asn Gly Pro
755 760 765

Leu Pro Pro Ser
770

<210> 147
<211> 1684
<212> PRT
<213> Homo sapiens

<400> 147

Met Glu Asp Leu Val Gln Asp Gly Val Ala Ser Pro Ala Thr Pro Gly
1 5 10 15

Thr Gly Lys Ser Lys Leu Glu Thr Leu Pro Lys Glu Asp Leu Ile Lys
20 25 30

Phe Ala Lys Lys Gln Met Met Leu Ile Gln Lys Ala Lys Ser Arg Cys
35 40 45

Thr Glu Leu Glu Lys Glu Ile Glu Glu Leu Arg Ser Lys Pro Val Thr
50 55 60

Glu Gly Thr Gly Asp Ile Ile Lys Ala Leu Thr Glu Arg Leu Asp Ala
65 70 75 80

Leu Leu Leu Glu Lys Ala Glu Thr Glu Gln Gln Cys Leu Ser Leu Lys
85 90 95

Lys Glu Asn Ile Lys Met Lys Gln Glu Val Glu Asp Ser Val Thr Lys
100 105 110

Met Gly Asp Ala His Lys Glu Leu Glu Gln Ser His Ile Asn Tyr Val
115 120 125

Lys Glu Ile Glu Asn Leu Lys Asn Glu Leu Met Ala Val Arg Ser Lys
130 135 140

Tyr Ser Glu Asp Lys Ala Asn Leu Gln Lys Gln Leu Glu Glu Ala Met
145 150 155 160

Asn Thr Gln Leu Glu Leu Ser Glu Gln Leu Lys Phe Gln Asn Asn Ser
165 170 175

Glu Asp Asn Val Lys Lys Leu Gln Glu Glu Ile Glu Lys Ile Arg Pro
180 185 190

Gly Phe Glu Glu Gln Ile Leu Tyr Leu Gln Lys Gln Leu Asp Ala Thr
195 200 205

Thr Asp Glu Lys Lys Glu Thr Val Thr Gln Leu Gln Asn Ile Ile Glu
210 215 220

Ala Asn Ser Gln His Tyr Gln Lys Asn Ile Asn Ser Leu Gln Glu Glu
225 230 235 240

Leu Leu Gln Leu Lys Ala Ile His Gln Glu Glu Val Lys Glu Leu Met
245 250 255

Cys Gln Ile Glu Ala Ser Ala Lys Glu His Glu Ala Glu Ile Asn Lys
260 265 270

Leu Asn Glu Leu Lys Glu Asn Leu Val Lys Gln Cys Glu Ala Ser Glu
275 280 285

Lys Asn Ile Gln Lys Lys Tyr Glu Cys Glu Leu Glu Asn Leu Arg Lys
290 295 300

Ala Thr Ser Asn Ala Asn Gln Asp Asn Gln Ile Cys Ser Ile Leu Leu
305 310 315 320

Gln Glu Asn Thr Phe Val Glu Gln Val Val Asn Glu Lys Val Lys His
325 330 335

Leu Glu Asp Thr Leu Lys Glu Leu Glu Ser Gln His Ser Ile Leu Lys
340 345 350

Asp Glu Val Thr Tyr Met Asn Asn Leu Lys Leu Lys Leu Glu Met Asp
355 360 365

Ala Gln His Ile Lys Asp Glu Phe Phe His Glu Arg Glu Asp Leu Glu
370 375 380

Phe Lys Ile Asn Glu Leu Leu Leu Ala Lys Glu Glu Gln Gly Cys Val
385 390 395 400

Ile Glu Lys Leu Lys Ser Glu Leu Ala Gly Leu Asn Lys Gln Phe Cys
405 410 415

Tyr Thr Val Glu Gln His Asn Arg Glu Val Gln Ser Leu Lys Glu Gln
420 425 430

His Gln Lys Glu Ile Ser Glu Leu Asn Glu Thr Phe Leu Ser Asp Ser
435 440 445

Glu Lys Glu Lys Leu Thr Leu Met Phe Glu Ile Gln Gly Leu Lys Glu
450 455 460

Gln Cys Glu Asn Leu Gln Gln Glu Lys Gln Glu Ala Ile Leu Asn Tyr
465 470 475 480

Glu Ser Leu Arg Glu Ile Met Glu Ile Leu Gln Thr Glu Leu Gly Glu
485 490 495

Ser Ala Gly Lys Ile Ser Gln Glu Phe Glu Ser Met Lys Gln Gln Gln
500 505 510

Ala Ser Asp Val His Glu Leu Gln Gln Lys Leu Arg Thr Ala Phe Thr
515 520 525

Glu Lys Asp Ala Leu Leu Glu Thr Val Asn Arg Leu Gln Gly Glu Asn
530 535 540

Glu Lys Leu Leu Ser Gln Gln Glu Leu Val Pro Glu Leu Glu Asn Thr
545 550 555 560

Ile Lys Asn Leu Gln Glu Lys Asn Gly Val Tyr Leu Leu Ser Leu Ser
565 570 575

Gln Arg Asp Thr Met Leu Lys Glu Leu Glu Gly Lys Ile Asn Ser Leu
580 585 590

Thr Glu Glu Lys Asp Asp Phe Ile Asn Lys Leu Lys Asn Ser His Glu
595 600 605

Glu Met Asp Asn Phe His Lys Lys Cys Glu Arg Glu Glu Arg Leu Ile
610 615 620

Leu Glu Leu Gly Lys Lys Val Glu Gln Thr Ile Gln Tyr Asn Ser Glu
625 630 635 640

Leu Glu Gln Lys Val Asn Glu Leu Thr Gly Gly Leu Glu Glu Thr Leu
645 650 655

Lys Glu Lys Asp Gln Asn Asp Gln Lys Leu Glu Lys Leu Met Val Gln
660 665 670

Met Lys Val Leu Ser Glu Asp Lys Glu Val Leu Ser Ala Glu Val Lys
675 680 685

Ser Leu Tyr Glu Glu Asn Asn Lys Leu Ser Ser Glu Lys Lys Gln Leu
690 695 700

Ser Arg Asp Leu Glu Val Phe Leu Ser Gln Lys Glu Asp Val Ile Leu
705 710 715 720

Lys Glu His Ile Thr Gln Leu Glu Lys Lys Leu Gln Leu Met Val Glu
725 730 735

Glu Gln Asp Asn Leu Asn Lys Leu Leu Glu Asn Glu Gln Val Gln Lys
740 745 750

Leu Phe Val Lys Thr Gln Leu Tyr Gly Phe Leu Lys Glu Met Gly Ser
755 760 765

Glu Val Ser Glu Asp Ser Glu Glu Lys Asp Val Val Asn Val Leu Gln
770 775 780

Ala Val Gly Glu Ser Leu Ala Lys Ile Asn Glu Glu Lys Cys Asn Leu
785 790 795 800

Ala Phe Gln Arg Asp Glu Lys Val Leu Glu Leu Glu Lys Glu Ile Lys
805 810 815

Cys Leu Gln Glu Glu Ser Val Val Gln Cys Glu Glu Leu Lys Ser Leu
820 825 830

Leu Arg Asp Tyr Glu Gln Glu Lys Val Leu Leu Arg Lys Glu Leu Glu
835 840 845

Glu Ile Gln Ser Glu Lys Glu Ala Leu Gln Ser Asp Leu Leu Glu Met
850 855 860

Lys Asn Ala Asn Glu Lys Thr Arg Leu Glu Asn Gln Asn Leu Leu Ile
865 870 875 880

Gln Val Glu Glu Val Ser Gln Thr Cys Ser Lys Ser Glu Ile His Asn
885 890 895

Glu Lys Glu Lys Cys Phe Ile Lys Glu His Glu Asn Leu Lys Pro Leu
900 905 910

Leu Glu Gln Lys Glu Leu Arg Asp Arg Arg Ala Glu Leu Ile Leu Leu
915 920 925

Lys Asp Ser Leu Ala Lys Ser Pro Ser Val Lys Asn Asp Pro Leu Ser
930 935 940

Ser Val Lys Glu Leu Glu Glu Lys Ile Glu Asn Leu Glu Lys Glu Cys
945 950 955 960

Lys Glu Lys Glu Glu Lys Ile Asn Lys Ile Lys Leu Val Ala Val Lys
965 970 975

Ala Lys Lys Glu Leu Asp Ser Ser Arg Lys Glu Thr Gln Thr Val Lys
980 985 990

Glu Glu Leu Glu Ser Leu Arg Ser Glu Lys Asp Gln Leu Ser Ala Ser
995 1000 1005

Met Arg Asp Leu Ile Gln Gly Ala Glu Ser Tyr Lys Asn Leu Leu
1010 1015 1020

Leu Glu Tyr Glu Lys Gln Ser Glu Gln Leu Asp Val Glu Lys Glu
1025 1030 1035

Arg Ala Asn Asn Phe Glu His Arg Ile Glu Asp Leu Thr Arg Gln
1040 1045 1050

Leu Arg Asn Ser Thr Leu Gln Cys Glu Thr Ile Asn Ser Asp Asn
1055 1060 1065

Glu Asp Leu Leu Ala Arg Ile Glu Thr Leu Gln Ser Asn Ala Lys
1070 1075 1080

Leu Leu Glu Val Gln Ile Leu Glu Val Gln Arg Ala Lys Ala Met
1085 1090 1095

Val Asp Lys Glu Leu Glu Ala Glu Lys Leu Gln Lys Glu Gln Lys
1100 1105 1110

Ile Lys Glu His Ala Thr Thr Val Asn Glu Leu Glu Glu Leu Gln
1115 1120 1125

Val Gln Leu Gln Lys Glu Lys Lys Gln Leu Gln Lys Thr Met Gln
1130 1135 1140

Glu Leu Glu Leu Val Lys Lys Asp Ala Gln Gln Thr Thr Leu Met
1145 1150 1155

Asn Met Glu Ile Ala Asp Tyr Glu Arg Leu Met Lys Glu Leu Asn
1160 1165 1170

Gln Lys Leu Thr Asn Lys Asn Asn Lys Ile Glu Asp Leu Glu Gln
1175 1180 1185

Glu Ile Lys Ile Gln Lys Gln Lys Gln Glu Thr Leu Gln Glu Glu
1190 1195 1200

Ile Thr Ser Leu Gln Ser Ser Val Gln Gln Tyr Glu Glu Lys Asn
1205 1210 1215

Thr Lys Ile Lys Gln Leu Leu Val Lys Thr Lys Lys Glu Leu Ala
1220 1225 1230

Asp Ser Lys Gln Ala Glu Thr Asp His Leu Ile Leu Gln Ala Ser
1235 1240 1245

Leu Lys Gly Glu Leu Glu Ala Ser Gln Gln Gln Val Glu Val Tyr
1250 1255 1260

Lys Ile Gln Leu Ala Glu Ile Thr Ser Glu Lys His Lys Ile His
1265 1270 1275

Glu His Leu Lys Thr Ser Ala Glu Gln His Gln Arg Thr Leu Ser
1280 1285 1290

Ala Tyr Gln Gln Arg Val Thr Ala Leu Gln Glu Glu Cys Arg Ala
1295 1300 1305

Ala Lys Ala Glu Gln Ala Thr Val Thr Ser Glu Phe Glu Ser Tyr
1310 1315 1320

Lys Val Arg Val His Asn Val Leu Lys Gln Gln Lys Asn Lys Ser
1325 1330 1335

Met Ser Gln Ala Glu Thr Glu Gly Ala Lys Gln Glu Arg Glu His
1340 1345 1350

Leu Glu Met Leu Ile Asp Gln Leu Lys Ile Lys Leu Gln Asp Ser
1355 1360 1365

Gln Asn Asn Leu Gln Ile Asn Val Ser Glu Leu Gln Thr Leu Gln
1370 1375 1380

Ser Glu His Asp Thr Leu Leu Glu Arg His Asn Lys Met Leu Gln
1385 1390 1395

Glu Thr Val Ser Lys Glu Ala Glu Leu Arg Glu Lys Leu Cys Ser
1400 1405 1410

Ile Gln Ser Glu Asn Met Met Met Lys Ser Glu His Thr Gln Thr
1415 1420 1425

Val Ser Gln Leu Thr Ser Gln Asn Glu Val Leu Arg Asn Ser Phe
1430 1435 1440

Arg Asp Gln Val Arg His Leu Gln Glu Glu His Arg Lys Thr Val
1445 1450 1455

Glu Thr Leu Gln Gln Gln Leu Ser Lys Met Glu Ala Gln Leu Phe
1460 1465 1470

Gln Leu Lys Asn Glu Pro Thr Thr Arg Ser Pro Val Ser Ser Gln
1475 1480 1485

Gln Ser Leu Lys Asn Leu Arg Glu Arg Arg Asn Thr Asp Leu Pro
1490 1495 1500

Leu Leu Asp Met His Thr Val Thr Arg Glu Glu Gly Glu Gly Met
1505 1510 1515

Glu Thr Thr Asp Thr Glu Ser Val Ser Ser Ala Ser Thr Tyr Thr
1520 1525 1530

Gln Ser Leu Glu Gln Leu Leu Asn Ser Pro Glu Thr Lys Leu Glu
1535 1540 1545

Pro Pro Leu Trp His Ala Glu Phe Thr Lys Glu Glu Leu Val Gln
1550 1555 1560

Lys Leu Ser Ser Thr Thr Lys Ser Ala Asp His Leu Asn Gly Leu
1565 1570 1575

Leu Arg Glu Thr Glu Ala Thr Asn Ala Ile Leu Met Glu Gln Ile
1580 1585 1590

Lys Leu Leu Lys Ser Glu Ile Arg Arg Leu Glu Arg Asn Gln Glu
1595 1600 1605

Arg Glu Lys Ser Ala Ala Asn Leu Glu Tyr Leu Lys Asn Val Leu
1610 1615 1620

Leu Gln Phe Ile Phe Leu Lys Pro Gly Ser Glu Arg Glu Arg Leu
1625 1630 1635

Leu Pro Val Ile Asn Thr Met Leu Gln Leu Ser Pro Glu Glu Lys
1640 1645 1650

Gly Lys Leu Ala Ala Val Ala Gln Gly Glu Glu Glu Asn Ala Ser
1655 1660 1665

Arg Ser Ser Gly Trp Ala Ser Tyr Leu His Ser Trp Ser Gly Leu
1670 1675 1680

Arg

<210> 148
<211> 288
<212> PRT
<213> Homo sapiens

<400> 148

Met Val Gly Val Gly Gly Gly Asp Val Glu Asp Val Thr Pro Arg Pro
1 5 10 15

Gly Gly Cys Gln Ile Ser Gly Arg Ala Ala Arg Gly Cys Asn Gly Ile
20 25 30

Pro Gly Ala Ala Ala Trp Glu Ala Ala Leu Pro Arg Arg Arg Pro Arg
35 40 45

Arg His Pro Ser Val Asn Pro Arg Ser Arg Ala Ala Gly Ser Pro Arg
50 55 60

Thr Arg Gly Arg Arg Thr Glu Glu Arg Pro Ser Gly Ser Arg Leu Gly
65 70 75 80

Asp Arg Gly Arg Gly Arg Ala Leu Pro Gly Gly Arg Leu Gly Gly Arg
85 90 95

Gly Arg Gly Arg Ala Pro Glu Arg Val Gly Gly Arg Gly Arg Gly Arg
100 105 110

Gly Thr Ala Ala Pro Arg Ala Ala Pro Ala Ala Arg Gly Ser Arg Pro
115 120 125

Gly Pro Ala Gly Thr Met Ala Ala Gly Ser Ile Thr Thr Leu Pro Ala
130 135 140

Leu Pro Glu Asp Gly Gly Ser Gly Ala Phe Pro Pro Gly His Phe Lys
145 150 155 160

Asp Pro Lys Arg Leu Tyr Cys Lys Asn Gly Gly Phe Phe Leu Arg Ile
165 170 175

His Pro Asp Gly Arg Val Asp Gly Val Arg Glu Lys Ser Asp Pro His
180 185 190

Ile Lys Leu Gln Leu Gln Ala Glu Glu Arg Gly Val Val Ser Ile Lys
195 200 205

Gly Val Cys Ala Asn Arg Tyr Leu Ala Met Lys Glu Asp Gly Arg Leu
210 215 220

Leu Ala Ser Lys Cys Val Thr Asp Glu Cys Phe Phe Phe Glu Arg Leu
225 230 235 240

Glu Ser Asn Asn Tyr Asn Thr Tyr Arg Ser Arg Lys Tyr Thr Ser Trp
245 250 255

Tyr Val Ala Leu Lys Arg Thr Gly Gln Tyr Lys Leu Gly Ser Lys Thr
260 265 270

Gly Pro Gly Gln Lys Ala Ile Leu Phe Leu Pro Met Ser Ala Lys Ser
275 280 285

<210> 149
<211> 582
<212> PRT
<213> Homo sapiens

<400> 149

Met Ser Pro Ala Pro Arg Pro Pro Arg Cys Leu Leu Leu Pro Leu Leu
1 5 10 15

Thr Leu Gly Thr Ala Leu Ala Ser Leu Gly Ser Ala Gln Ser Ser Ser
20 25 30

Phe Ser Pro Glu Ala Trp Leu Gln Gln Tyr Gly Tyr Leu Pro Pro Gly
35 40 45

Asp Leu Arg Thr His Thr Gln Arg Ser Pro Gln Ser Leu Ser Ala Ala
50 55 60

Ile Ala Ala Met Gln Lys Phe Tyr Gly Leu Gln Val Thr Gly Lys Ala
65 70 75 80

Asp Ala Asp Thr Met Lys Ala Met Arg Arg Pro Arg Cys Gly Val Pro
85 90 95

Asp Lys Phe Gly Ala Glu Ile Lys Ala Asn Val Arg Arg Lys Arg Tyr
100 105 110

Ala Ile Gln Gly Leu Lys Trp Gln His Asn Glu Ile Thr Phe Cys Ile
115 120 125

Gln Asn Tyr Thr Pro Lys Val Gly Glu Tyr Ala Thr Tyr Glu Ala Ile
130 135 140

Arg Lys Ala Phe Arg Val Trp Glu Ser Ala Thr Pro Leu Arg Phe Arg
145 150 155 160

Glu Val Pro Tyr Ala Tyr Ile Arg Glu Gly His Glu Lys Gln Ala Asp
165 170 175

Ile Met Ile Phe Phe Ala Glu Gly Phe His Gly Asp Ser Thr Pro Phe
180 185 190

Asp Gly Glu Gly Gly Phe Leu Ala His Ala Tyr Phe Pro Gly Pro Asn
195 200 205

Ile Gly Gly Asp Thr His Phe Asp Ser Ala Glu Pro Trp Thr Val Arg
210 215 220

Asn Glu Asp Leu Asn Gly Asn Asp Ile Phe Leu Val Ala Val His Glu
225 230 235 240

Leu Gly His Ala Leu Gly Leu Glu His Ser Ser Asp Pro Ser Ala Ile
245 250 255

Met Ala Pro Phe Tyr Gln Trp Met Asp Thr Glu Asn Phe Val Leu Pro
260 265 270

Asp Asp Asp Arg Arg Gly Ile Gln Gln Leu Tyr Gly Gly Glu Ser Gly
275 280 285

Phe Pro Thr Lys Met Pro Pro Gln Pro Arg Thr Thr Ser Arg Pro Ser
290 295 300

Val Pro Asp Lys Pro Lys Asn Pro Thr Tyr Gly Pro Asn Ile Cys Asp
305 310 315 320

Gly Asn Phe Asp Thr Val Ala Met Leu Arg Gly Glu Met Phe Val Phe
325 330 335

Lys Glu Arg Trp Phe Trp Arg Val Arg Asn Asn Gln Val Met Asp Gly
340 345 350

Tyr Pro Met Pro Ile Gly Gln Phe Trp Arg Gly Leu Pro Ala Ser Ile
355 360 365

Asn Thr Ala Tyr Glu Arg Lys Asp Gly Lys Phe Val Phe Phe Lys Gly
370 375 380

Asp Lys His Trp Val Phe Asp Glu Ala Ser Leu Glu Pro Gly Tyr Pro
385 390 395 400

Lys His Ile Lys Glu Leu Gly Arg Gly Leu Pro Thr Asp Lys Ile Asp
405 410 415

Ala Ala Leu Phe Trp Met Pro Asn Gly Lys Thr Tyr Phe Phe Arg Gly
420 425 430

Asn Lys Tyr Tyr Arg Phe Asn Glu Glu Leu Arg Ala Val Asp Ser Glu
435 440 445

Tyr Pro Lys Asn Ile Lys Val Trp Glu Gly Ile Pro Glu Ser Pro Arg
450 455 460

Gly Ser Phe Met Gly Ser Asp Glu Val Phe Thr Tyr Phe Tyr Lys Gly
465 470 475 480

Asn Lys Tyr Trp Lys Phe Asn Asn Gln Lys Leu Lys Val Glu Pro Gly
485 490 495

Tyr Pro Lys Ser Ala Leu Arg Asp Trp Met Gly Cys Pro Ser Gly Gly
500 505 510

Arg Pro Asp Glu Gly Thr Glu Glu Glu Thr Glu Val Ile Ile Ile Glu
515 520 525

Val Asp Glu Glu Gly Gly Gly Ala Val Ser Ala Ala Ala Val Val Leu
530 535 540

Pro Val Leu Leu Leu Leu Leu Val Leu Ala Val Gly Leu Ala Val Phe
545 550 555 560

Phe Phe Arg Arg His Gly Thr Pro Arg Arg Leu Leu Tyr Cys Gln Arg
565 570 575

Ser Leu Leu Asp Lys Val
580

<210> 150
<211> 1255
<212> PRT
<213> Homo sapiens

<400> 150

Met Glu Leu Ala Ala Leu Cys Arg Trp Gly Leu Leu Leu Ala Leu Leu
1 5 10 15

Pro Pro Gly Ala Ala Ser Thr Gln Val Cys Thr Gly Thr Asp Met Lys
20 25 30

Leu Arg Leu Pro Ala Ser Pro Glu Thr His Leu Asp Met Leu Arg His
35 40 45

Leu Tyr Gln Gly Cys Gln Val Val Gln Gly Asn Leu Glu Leu Thr Tyr
50 55 60

Leu Pro Thr Asn Ala Ser Leu Ser Phe Leu Gln Asp Ile Gln Glu Val
65 70 75 80

Gln Gly Tyr Val Leu Ile Ala His Asn Gln Val Arg Gln Val Pro Leu
85 90 95

Gln Arg Leu Arg Ile Val Arg Gly Thr Gln Leu Phe Glu Asp Asn Tyr
100 105 110

Ala Leu Ala Val Leu Asp Asn Gly Asp Pro Leu Asn Asn Thr Thr Pro
115 120 125

Val Thr Gly Ala Ser Pro Gly Gly Leu Arg Glu Leu Gln Leu Arg Ser
130 135 140

Leu Thr Glu Ile Leu Lys Gly Gly Val Leu Ile Gln Arg Asn Pro Gln
145 150 155 160

Leu Cys Tyr Gln Asp Thr Ile Leu Trp Lys Asp Ile Phe His Lys Asn
165 170 175

Asn Gln Leu Ala Leu Thr Leu Ile Asp Thr Asn Arg Ser Arg Ala Cys
180 185 190

His Pro Cys Ser Pro Met Cys Lys Gly Ser Arg Cys Trp Gly Glu Ser
195 200 205

Ser Glu Asp Cys Gln Ser Leu Thr Arg Thr Val Cys Ala Gly Gly Cys
210 215 220

Ala Arg Cys Lys Gly Pro Leu Pro Thr Asp Cys Cys His Glu Gln Cys
225 230 235 240

Ala Ala Gly Cys Thr Gly Pro Lys His Ser Asp Cys Leu Ala Cys Leu
245 250 255

His Phe Asn His Ser Gly Ile Cys Glu Leu His Cys Pro Ala Leu Val
260 265 270

Thr Tyr Asn Thr Asp Thr Phe Glu Ser Met Pro Asn Pro Glu Gly Arg
275 280 285

Tyr Thr Phe Gly Ala Ser Cys Val Thr Ala Cys Pro Tyr Asn Tyr Leu
290 295 300

Ser Thr Asp Val Gly Ser Cys Thr Leu Val Cys Pro Leu His Asn Gln
305 310 315 320

Glu Val Thr Ala Glu Asp Gly Thr Gln Arg Cys Glu Lys Cys Ser Lys
325 330 335

Pro Cys Ala Arg Val Cys Tyr Gly Leu Gly Met Glu His Leu Arg Glu
340 345 350

Val Arg Ala Val Thr Ser Ala Asn Ile Gln Glu Phe Ala Gly Cys Lys
355 360 365

Lys Ile Phe Gly Ser Leu Ala Phe Leu Pro Glu Ser Phe Asp Gly Asp
370 375 380

Pro Ala Ser Asn Thr Ala Pro Leu Gln Pro Glu Gln Leu Gln Val Phe
385 390 395 400

Glu Thr Leu Glu Glu Ile Thr Gly Tyr Leu Tyr Ile Ser Ala Trp Pro
405 410 415

Asp Ser Leu Pro Asp Leu Ser Val Phe Gln Asn Leu Gln Val Ile Arg
420 425 430

Gly Arg Ile Leu His Asn Gly Ala Tyr Ser Leu Thr Leu Gln Gly Leu
435 440 445

Gly Ile Ser Trp Leu Gly Leu Arg Ser Leu Arg Glu Leu Gly Ser Gly
450 455 460

Leu Ala Leu Ile His His Asn Thr His Leu Cys Phe Val His Thr Val
465 470 475 480

Pro Trp Asp Gln Leu Phe Arg Asn Pro His Gln Ala Leu Leu His Thr
485 490 495

Ala Asn Arg Pro Glu Asp Glu Cys Val Gly Glu Gly Leu Ala Cys His
500 505 510

Gln Leu Cys Ala Arg Gly His Cys Trp Gly Pro Gly Pro Thr Gln Cys
515 520 525

Val Asn Cys Ser Gln Phe Leu Arg Gly Gln Glu Cys Val Glu Glu Cys
530 535 540

Arg Val Leu Gln Gly Leu Pro Arg Glu Tyr Val Asn Ala Arg His Cys
545 550 555 560

Leu Pro Cys His Pro Glu Cys Gln Pro Gln Asn Gly Ser Val Thr Cys
565 570 575

Phe Gly Pro Glu Ala Asp Gln Cys Val Ala Cys Ala His Tyr Lys Asp
580 585 590

Pro Pro Phe Cys Val Ala Arg Cys Pro Ser Gly Val Lys Pro Asp Leu
595 600 605

Ser Tyr Met Pro Ile Trp Lys Phe Pro Asp Glu Glu Gly Ala Cys Gln
610 615 620

Pro Cys Pro Ile Asn Cys Thr His Ser Cys Val Asp Leu Asp Asp Lys
625 630 635 640

Gly Cys Pro Ala Glu Gln Arg Ala Ser Pro Leu Thr Ser Ile Ile Ser
645 650 655

Ala Val Val Gly Ile Leu Leu Val Val Val Leu Gly Val Val Phe Gly
660 665 670

Ile Leu Ile Lys Arg Arg Gln Gln Lys Ile Arg Lys Tyr Thr Met Arg
675 680 685

Arg Leu Leu Gln Glu Thr Glu Leu Val Glu Pro Leu Thr Pro Ser Gly
690 695 700

Ala Met Pro Asn Gln Ala Gln Met Arg Ile Leu Lys Glu Thr Glu Leu
705 710 715 720

Arg Lys Val Lys Val Leu Gly Ser Gly Ala Phe Gly Thr Val Tyr Lys
725 730 735

Gly Ile Trp Ile Pro Asp Gly Glu Asn Val Lys Ile Pro Val Ala Ile
740 745 750

Lys Val Leu Arg Glu Asn Thr Ser Pro Lys Ala Asn Lys Glu Ile Leu
755 760 765

Asp Glu Ala Tyr Val Met Ala Gly Val Gly Ser Pro Tyr Val Ser Arg
770 775 780

Leu Leu Gly Ile Cys Leu Thr Ser Thr Val Gln Leu Val Thr Gln Leu
785 790 795 800

Met Pro Tyr Gly Cys Leu Leu Asp His Val Arg Glu Asn Arg Gly Arg
805 810 815

Leu Gly Ser Gln Asp Leu Leu Asn Trp Cys Met Gln Ile Ala Lys Gly
820 825 830

Met Ser Tyr Leu Glu Asp Val Arg Leu Val His Arg Asp Leu Ala Ala
835 840 845

Arg Asn Val Leu Val Lys Ser Pro Asn His Val Lys Ile Thr Asp Phe
850 855 860

Gly Leu Ala Arg Leu Leu Asp Ile Asp Glu Thr Glu Tyr His Ala Asp
865 870 875 880

Gly Gly Lys Val Pro Ile Lys Trp Met Ala Leu Glu Ser Ile Leu Arg
885 890 895

Arg Arg Phe Thr His Gln Ser Asp Val Trp Ser Tyr Gly Val Thr Val
900 905 910

Trp Glu Leu Met Thr Phe Gly Ala Lys Pro Tyr Asp Gly Ile Pro Ala
915 920 925

Arg Glu Ile Pro Asp Leu Leu Glu Lys Gly Glu Arg Leu Pro Gln Pro
930 935 940

Pro Ile Cys Thr Ile Asp Val Tyr Met Ile Met Val Lys Cys Trp Met
945 950 955 960

Ile Asp Ser Glu Cys Arg Pro Arg Phe Arg Glu Leu Val Ser Glu Phe
965 970 975

Ser Arg Met Ala Arg Asp Pro Gln Arg Phe Val Val Ile Gln Asn Glu
980 985 990

Asp Leu Gly Pro Ala Ser Pro Leu Asp Ser Thr Phe Tyr Arg Ser Leu
995 1000 1005

Leu Glu Asp Asp Asp Met Gly Asp Leu Val Asp Ala Glu Glu Tyr
1010 1015 1020

Leu Val Pro Gln Gln Gly Phe Phe Cys Pro Asp Pro Ala Pro Gly
1025 1030 1035

Ala Gly Gly Met Val His His Arg His Arg Ser Ser Ser Thr Arg
1040 1045 1050

Ser Gly Gly Gly Asp Leu Thr Leu Gly Leu Glu Pro Ser Glu Glu
1055 1060 1065

Glu Ala Pro Arg Ser Pro Leu Ala Pro Ser Glu Gly Ala Gly Ser
1070 1075 1080

Asp Val Phe Asp Gly Asp Leu Gly Met Gly Ala Ala Lys Gly Leu
1085 1090 1095

Gln Ser Leu Pro Thr His Asp Pro Ser Pro Leu Gln Arg Tyr Ser
1100 1105 1110

Glu Asp Pro Thr Val Pro Leu Pro Ser Glu Thr Asp Gly Tyr Val
1115 1120 1125

Ala Pro Leu Thr Cys Ser Pro Gln Pro Glu Tyr Val Asn Gln Pro
1130 1135 1140

Asp Val Arg Pro Gln Pro Pro Ser Pro Arg Glu Gly Pro Leu Pro
1145 1150 1155

Ala Ala Arg Pro Ala Gly Ala Thr Leu Glu Arg Pro Lys Thr Leu
1160 1165 1170

Ser Pro Gly Lys Asn Gly Val Val Lys Asp Val Phe Ala Phe Gly
1175 1180 1185

Gly Ala Val Glu Asn Pro Glu Tyr Leu Thr Pro Gln Gly Gly Ala
1190 1195 1200

Ala Pro Gln Pro His Pro Pro Pro Ala Phe Ser Pro Ala Phe Asp
1205 1210 1215

Asn Leu Tyr Tyr Trp Asp Gln Asp Pro Pro Glu Arg Gly Ala Pro
1220 1225 1230

Pro Ser Thr Phe Lys Gly Thr Pro Thr Ala Glu Asn Pro Glu Tyr
1235 1240 1245

Leu Gly Leu Asp Val Pro Val
1250 1255

<210> 151
<211> 552
<212> PRT
<213> Homo sapiens

<400> 151

Met Ala Glu Lys Gln Lys His Asp Gly Arg Val Lys Ile Gly His Tyr
1 5 10 15

Val Leu Gly Asp Thr Leu Gly Val Gly Thr Phe Gly Lys Val Lys Ile
20 25 30

Gly Glu His Gln Leu Thr Gly His Lys Val Ala Val Lys Ile Leu Asn
35 40 45

Arg Gln Lys Ile Arg Ser Leu Asp Val Val Gly Lys Ile Lys Arg Glu
50 55 60

Ile Gln Asn Leu Lys Leu Phe Arg His Pro His Ile Ile Lys Leu Tyr
65 70 75 80

Gln Val Ile Ser Thr Pro Thr Asp Phe Phe Met Val Met Glu Tyr Val
85 90 95

Ser Gly Gly Glu Leu Phe Asp Tyr Ile Cys Lys His Gly Arg Val Glu
100 105 110

Glu Met Glu Ala Arg Arg Leu Phe Gln Gln Ile Leu Ser Ala Val Asp
115 120 125

Tyr Cys His Arg His Met Val Val His Arg Asp Leu Lys Pro Glu Asn
130 135 140

Val Leu Leu Asp Ala His Met Asn Ala Lys Ile Ala Asp Phe Gly Leu
145 150 155 160

Ser Asn Met Met Ser Asp Gly Glu Phe Leu Arg Thr Ser Cys Gly Ser
165 170 175

Pro Asn Tyr Ala Ala Pro Glu Val Ile Ser Gly Arg Leu Tyr Ala Gly
180 185 190

Pro Glu Val Asp Ile Trp Ser Cys Gly Val Ile Leu Tyr Ala Leu Leu
195 200 205

Cys Gly Thr Leu Pro Phe Asp Asp Glu His Val Pro Thr Leu Phe Lys
210 215 220

Lys Ile Arg Gly Gly Val Phe Tyr Ile Pro Glu Tyr Leu Asn Arg Ser
225 230 235 240

Val Ala Thr Leu Leu Met His Met Leu Gln Val Asp Pro Leu Lys Arg
245 250 255

Ala Thr Ile Lys Asp Ile Arg Glu His Glu Trp Phe Lys Gln Asp Leu
260 265 270

Pro Ser Tyr Leu Phe Pro Glu Asp Pro Ser Tyr Asp Ala Asn Val Ile
275 280 285

Asp Asp Glu Ala Val Lys Glu Val Cys Glu Lys Phe Glu Cys Thr Glu
290 295 300

Ser Glu Val Met Asn Ser Leu Tyr Ser Gly Asp Pro Gln Asp Gln Leu
305 310 315 320

Ala Val Ala Tyr His Leu Ile Ile Asp Asn Arg Arg Ile Met Asn Gln
325 330 335

Ala Ser Glu Phe Tyr Leu Ala Ser Ser Pro Pro Ser Gly Ser Phe Met
340 345 350

Asp Asp Ser Ala Met His Ile Pro Pro Gly Leu Lys Pro His Pro Glu
355 360 365

Arg Met Pro Pro Leu Ile Ala Asp Ser Pro Lys Ala Arg Cys Pro Leu
370 375 380

Asp Ala Leu Asn Thr Thr Lys Pro Lys Ser Leu Ala Val Lys Lys Ala
385 390 395 400

Lys Trp His Leu Gly Ile Arg Ser Gln Ser Lys Pro Tyr Asp Ile Met
405 410 415

Ala Glu Val Tyr Arg Ala Met Lys Gln Leu Asp Phe Glu Trp Lys Val
420 425 430

Val Asn Ala Tyr His Leu Arg Val Arg Arg Lys Asn Pro Val Thr Gly
435 440 445

Asn Tyr Val Lys Met Ser Leu Gln Leu Tyr Leu Val Asp Asn Arg Ser
450 455 460

Tyr Leu Leu Asp Phe Lys Ser Ile Asp Asp Glu Val Val Glu Gln Arg
465 470 475 480

Ser Gly Ser Ser Thr Pro Gln Arg Ser Cys Ser Ala Ala Gly Leu His
485 490 495

Arg Pro Arg Ser Ser Phe Asp Ser Thr Thr Ala Glu Ser His Ser Leu
500 505 510

Ser Gly Ser Leu Thr Gly Ser Leu Thr Gly Ser Thr Leu Ser Ser Val
515 520 525

Ser Pro Arg Leu Gly Ser His Thr Met Asp Phe Phe Glu Met Cys Ala
530 535 540

Ser Leu Ile Thr Thr Leu Ala Arg
545 550

<210> 152
<211> 837
<212> PRT
<213> Homo sapiens

<400> 152

Met Phe Leu Trp Leu Phe Leu Ile Leu Ser Ala Leu Ile Ser Ser Thr
1 5 10 15

Asn Ala Asp Ser Asp Ile Ser Val Glu Ile Cys Asn Val Cys Ser Cys
20 25 30

Val Ser Val Glu Asn Val Leu Tyr Val Asn Cys Glu Lys Val Ser Val
35 40 45

Tyr Arg Pro Asn Gln Leu Lys Pro Pro Trp Ser Asn Phe Tyr His Leu
50 55 60

Asn Phe Gln Asn Asn Phe Leu Asn Ile Leu Tyr Pro Asn Thr Phe Leu
65 70 75 80

Asn Phe Ser His Ala Val Ser Leu His Leu Gly Asn Asn Lys Leu Gln
85 90 95

Asn Ile Glu Gly Gly Ala Phe Leu Gly Leu Ser Ala Leu Lys Gln Leu
100 105 110

His Leu Asn Asn Asn Glu Leu Lys Ile Leu Arg Ala Asp Thr Phe Leu
115 120 125

Gly Ile Glu Asn Leu Glu Tyr Leu Gln Ala Asp Tyr Asn Leu Ile Lys
130 135 140

Tyr Ile Glu Arg Gly Ala Phe Asn Lys Leu His Lys Leu Lys Val Leu
145 150 155 160

Ile Leu Asn Asp Asn Leu Ile Ser Phe Leu Pro Asp Asn Ile Phe Arg
165 170 175

Phe Ala Ser Leu Thr His Leu Asp Ile Arg Gly Asn Arg Ile Gln Lys
180 185 190

Leu Pro Tyr Ile Gly Val Leu Glu His Ile Gly Arg Val Val Glu Leu
195 200 205

Gln Leu Glu Asp Asn Pro Trp Asn Cys Ser Cys Asp Leu Leu Pro Leu
210 215 220

Lys Ala Trp Leu Glu Asn Met Pro Tyr Asn Ile Tyr Ile Gly Glu Ala
225 230 235 240

Ile Cys Glu Thr Pro Ser Asp Leu Tyr Gly Arg Leu Leu Lys Glu Thr
245 250 255

Asn Lys Gln Glu Leu Cys Pro Met Gly Thr Gly Ser Asp Phe Asp Val
260 265 270

Arg Ile Leu Pro Pro Ser Gln Leu Glu Asn Gly Tyr Thr Thr Pro Asn
275 280 285

Gly His Thr Thr Gln Thr Ser Leu His Arg Leu Val Thr Lys Pro Pro
290 295 300

Lys Thr Thr Asn Pro Ser Lys Ile Ser Gly Ile Val Ala Gly Lys Ala
305 310 315 320

Leu Ser Asn Arg Asn Leu Ser Gln Ile Val Ser Tyr Gln Thr Arg Val
325 330 335

Pro Pro Leu Thr Pro Cys Pro Ala Pro Cys Phe Cys Lys Thr His Pro
340 345 350

Ser Asp Leu Gly Leu Ser Val Asn Cys Gln Glu Lys Asn Ile Gln Ser
355 360 365

Met Ser Glu Leu Ile Pro Lys Pro Leu Asn Ala Lys Lys Leu His Val
370 375 380

Asn Gly Asn Ser Ile Lys Asp Val Asp Val Ser Asp Phe Thr Asp Phe
385 390 395 400

Glu Gly Leu Asp Leu Leu His Leu Gly Ser Asn Gln Ile Thr Val Ile
405 410 415

Lys Gly Asp Val Phe His Asn Leu Thr Asn Leu Arg Arg Leu Tyr Leu
420 425 430

Asn Gly Asn Gln Ile Glu Arg Leu Tyr Pro Glu Ile Phe Ser Gly Leu
435 440 445

His Asn Leu Gln Tyr Leu Tyr Leu Glu Tyr Asn Leu Ile Lys Glu Ile
450 455 460

Ser Ala Gly Thr Phe Asp Ser Met Pro Asn Leu Gln Leu Leu Tyr Leu
465 470 475 480

Asn Asn Asn Leu Leu Lys Ser Leu Pro Val Tyr Ile Phe Ser Gly Ala
485 490 495

Pro Leu Ala Arg Leu Asn Leu Arg Asn Asn Lys Phe Met Tyr Leu Pro
500 505 510

Val Ser Gly Val Leu Asp Gln Leu Gln Ser Leu Thr Gln Ile Asp Leu
515 520 525

Glu Gly Asn Pro Trp Asp Cys Thr Cys Asp Leu Val Ala Leu Lys Leu
530 535 540

Trp Val Glu Lys Leu Ser Asp Gly Ile Val Val Lys Glu Leu Lys Cys
545 550 555 560

Glu Thr Pro Val Gln Phe Ala Asn Ile Glu Leu Lys Ser Leu Lys Asn
565 570 575

Glu Ile Leu Cys Pro Lys Leu Leu Asn Lys Pro Ser Ala Pro Phe Thr
580 585 590

Ser Pro Ala Pro Ala Ile Thr Phe Thr Thr Pro Leu Gly Pro Ile Arg
595 600 605

Ser Pro Pro Gly Gly Pro Val Pro Leu Ser Ile Leu Ile Leu Ser Ile
610 615 620

Leu Val Val Leu Ile Leu Thr Val Phe Val Ala Phe Cys Leu Leu Val
625 630 635 640

Phe Val Leu Arg Arg Asn Lys Lys Pro Thr Val Lys His Glu Gly Leu
645 650 655

Gly Asn Pro Asp Cys Gly Ser Met Gln Leu Gln Leu Arg Lys His Asp
660 665 670

His Lys Thr Asn Lys Lys Asp Gly Leu Ser Thr Glu Ala Phe Ile Pro
675 680 685

Gln Thr Ile Glu Gln Met Ser Lys Ser His Thr Cys Gly Leu Lys Glu
690 695 700

Ser Glu Thr Gly Phe Met Phe Ser Asp Pro Pro Gly Gln Lys Val Val
705 710 715 720

Met Arg Asn Val Ala Asp Lys Glu Lys Asp Leu Leu His Val Asp Thr
725 730 735

Arg Lys Arg Leu Ser Thr Ile Asp Glu Leu Asp Glu Leu Phe Pro Ser
740 745 750

Arg Asp Ser Asn Val Phe Ile Gln Asn Phe Leu Glu Ser Lys Lys Glu
755 760 765

Tyr Asn Ser Ile Gly Val Ser Gly Phe Glu Ile Arg Tyr Pro Glu Lys
770 775 780

Gln Pro Asp Lys Lys Ser Lys Lys Ser Leu Ile Gly Gly Asn His Ser
785 790 795 800

Lys Ile Val Val Glu Gln Arg Lys Ser Glu Tyr Phe Glu Leu Lys Ala
805 810 815

Lys Leu Gln Ser Ser Pro Asp Tyr Leu Gln Val Leu Glu Glu Gln Thr
820 825 830

Ala Leu Asn Lys Ile
835

<210> 153
<211> 379
<212> PRT
<213> Homo sapiens

<400> 153

Met Pro Arg Ser Ser Arg Ser Pro Gly Asp Pro Gly Ala Leu Leu Glu
1 5 10 15

Asp Val Ala His Asn Pro Arg Pro Arg Arg Ile Ala Gln Arg Gly Arg
20 25 30

Asn Thr Ser Arg Met Ala Glu Asp Thr Ser Pro Asn Met Asn Asp Asn
35 40 45

Ile Leu Leu Pro Val Arg Asn Asn Asp Gln Ala Leu Gly Leu Thr Gln
50 55 60

Cys Met Leu Gly Cys Val Ser Trp Phe Thr Cys Phe Ala Cys Ser Leu
65 70 75 80

Arg Thr Gln Ala Gln Gln Val Leu Phe Asn Thr Cys Arg Cys Lys Leu
85 90 95

Leu Cys Gln Lys Leu Met Glu Lys Thr Gly Ile Leu Leu Leu Cys Ala
100 105 110

Phe Gly Phe Trp Met Phe Ser Ile His Leu Pro Ser Lys Met Lys Val
115 120 125

Trp Gln Asp Asp Ser Ile Asn Gly Pro Leu Gln Ser Leu Arg Leu Tyr
130 135 140

Gln Glu Lys Val Arg His His Ser Gly Glu Ile Gln Asp Leu Arg Gly
145 150 155 160

Ser Met Asn Gln Leu Ile Ala Lys Leu Gln Glu Met Glu Ala Met Ser
165 170 175

Asp Glu Gln Lys Met Ala Gln Lys Ile Met Lys Met Ile His Gly Asp
180 185 190

Tyr Ile Glu Lys Pro Asp Phe Ala Leu Lys Ser Ile Gly Ala Ser Ile
195 200 205

Asp Phe Glu His Thr Ser Val Thr Tyr Asn His Glu Lys Ala His Ser
210 215 220

Tyr Trp Asn Trp Ile Gln Leu Trp Asn Tyr Ala Gln Pro Pro Asp Val
225 230 235 240

Ile Leu Glu Pro Asn Val Thr Pro Gly Asn Cys Trp Ala Phe Glu Gly
245 250 255

Asp Arg Gly Gln Val Thr Ile Gln Leu Ala Gln Lys Val Tyr Leu Ser
260 265 270

Asn Leu Thr Leu Gln His Ile Pro Lys Thr Ile Ser Leu Ser Gly Ser
275 280 285

Leu Asp Thr Ala Pro Lys Asp Phe Val Ile Tyr Gly Met Glu Gly Ser
290 295 300

Pro Lys Glu Glu Val Phe Leu Gly Ala Phe Gln Phe Gln Pro Glu Asn
305 310 315 320

Ile Ile Gln Met Phe Pro Leu Gln Asn Gln Pro Ala Arg Ala Phe Ser
325 330 335

Ala Val Lys Val Lys Ile Ser Ser Asn Trp Gly Asn Pro Gly Phe Thr
340 345 350

Cys Leu Tyr Arg Val Arg Val His Gly Ser Val Ala Pro Pro Arg Glu
355 360 365

Gln Pro His Gln Asn Pro Tyr Pro Lys Arg Asp
370 375

<210> 154
<211> 601
<212> PRT
<213> Homo sapiens

<400> 154

Met His Pro Leu Gln Cys Val Leu Gln Val Gln Arg Ser Leu Gly Trp
1 5 10 15

Gly Pro Leu Ala Ser Val Ser Trp Leu Ser Leu Arg Met Cys Arg Ala
20 25 30

His Ser Ser Leu Ser Ser Thr Met Cys Pro Ser Pro Glu Arg Gln Glu
35 40 45

Asp Gly Ala Arg Lys Asp Phe Ser Ser Arg Leu Ala Ala Gly Pro Thr
50 55 60

Phe Gln His Phe Leu Lys Ser Ala Ser Ala Pro Gln Glu Lys Leu Ser
65 70 75 80

Ser Glu Val Glu Asp Pro Pro Pro Tyr Leu Met Met Asp Glu Leu Leu
85 90 95

Gly Arg Gln Arg Lys Val Tyr Leu Glu Thr Tyr Gly Cys Gln Met Asn
100 105 110

Val Asn Asp Thr Glu Ile Ala Trp Ser Ile Leu Gln Lys Ser Gly Tyr
115 120 125

Leu Arg Thr Ser Asn Leu Gln Glu Ala Asp Val Ile Leu Leu Val Thr
130 135 140

Cys Ser Ile Arg Glu Lys Ala Glu Gln Thr Ile Trp Asn Arg Leu His
145 150 155 160

Gln Leu Lys Ala Leu Lys Thr Arg Arg Pro Arg Ser Arg Val Pro Leu
165 170 175

Arg Ile Gly Ile Leu Gly Cys Met Ala Glu Arg Leu Lys Glu Glu Ile
180 185 190

Leu Asn Arg Glu Lys Met Val Asp Ile Leu Ala Gly Pro Asp Ala Tyr
195 200 205

Arg Asp Leu Pro Arg Leu Leu Ala Val Ala Glu Ser Gly Gln Gln Ala
210 215 220

Ala Asn Val Leu Leu Ser Leu Asp Glu Thr Tyr Ala Asp Val Met Pro
225 230 235 240

Val Gln Thr Ser Ala Ser Ala Thr Ser Ala Phe Val Ser Ile Met Arg
245 250 255

Gly Cys Asp Asn Met Cys Ser Tyr Cys Ile Val Pro Phe Thr Arg Gly
260 265 270

Arg Glu Arg Ser Arg Pro Ile Ala Ser Ile Leu Glu Glu Val Lys Lys
275 280 285

```
Leu Ser Glu Gln Val Phe Leu Pro Pro Arg Pro Pro Lys Val Leu Gly
    290                 295                 300

Leu Gln Gly Leu Lys Glu Val Thr Leu Leu Gly Gln Asn Val Asn Ser
305                 310                 315                 320

Phe Arg Asp Asn Ser Glu Val Gln Phe Asn Ser Ala Val Pro Thr Asn
                325                 330                 335

Leu Ser Arg Gly Phe Thr Thr Asn Tyr Lys Thr Lys Gln Gly Gly Leu
            340                 345                 350

Arg Phe Ala His Leu Leu Asp Gln Val Ser Arg Val Asp Pro Glu Met
        355                 360                 365

Arg Ile Arg Phe Thr Ser Pro His Pro Lys Asp Phe Pro Asp Glu Val
    370                 375                 380

Leu Gln Leu Ile His Glu Arg Asp Asn Ile Cys Lys Gln Ile His Leu
385                 390                 395                 400

Pro Ala Gln Ser Gly Ser Ser Arg Val Leu Glu Ala Met Arg Arg Gly
                405                 410                 415

Tyr Ser Arg Glu Ala Tyr Val Glu Leu Val His His Ile Arg Glu Ser
            420                 425                 430

Ile Pro Gly Val Ser Leu Ser Ser Asp Phe Ile Ala Gly Phe Cys Gly
        435                 440                 445

Glu Thr Glu Glu Asp His Val Gln Thr Val Ser Leu Leu Arg Glu Val
    450                 455                 460

Gln Tyr Asn Met Gly Phe Leu Phe Ala Tyr Ser Met Arg Gln Lys Thr
465                 470                 475                 480

Arg Ala Tyr His Arg Leu Lys Asp Asp Val Pro Glu Glu Val Lys Leu
                485                 490                 495

Arg Arg Leu Glu Glu Leu Ile Thr Ile Phe Arg Glu Glu Ala Thr Lys
            500                 505                 510

Ala Asn Gln Thr Ser Val Gly Cys Thr Gln Leu Val Leu Val Glu Gly
        515                 520                 525

Leu Ser Lys Arg Ser Ala Thr Asp Leu Cys Gly Arg Asn Asp Gly Asn
    530                 535                 540

Leu Lys Val Ile Phe Pro Asp Ala Glu Met Glu Asp Val Asn Asn Pro
545                 550                 555                 560
```

Gly Leu Arg Val Arg Ala Gln Pro Gly Asp Tyr Val Leu Val Lys Ile
565 570 575

Thr Ser Ala Ser Ser Gln Thr Leu Arg Gly His Val Leu Cys Arg Thr
580 585 590

Thr Leu Arg Asp Ser Ser Ala Tyr Cys
595 600

<210> 155
<211> 356
<212> PRT
<213> Homo sapiens

<400> 155

Met Ser Pro Cys Gly Arg Ala Arg Arg Gln Thr Ser Arg Gly Ala Met
1 5 10 15

Ala Val Leu Ala Trp Lys Phe Pro Arg Thr Arg Leu Pro Met Gly Ala
20 25 30

Ser Ala Leu Cys Val Val Val Leu Cys Trp Leu Tyr Ile Phe Pro Val
35 40 45

Tyr Arg Leu Pro Asn Glu Lys Glu Ile Val Gln Gly Val Leu Gln Gln
50 55 60

Gly Thr Ala Trp Arg Arg Asn Gln Thr Ala Ala Arg Ala Phe Arg Lys
65 70 75 80

Gln Met Glu Asp Cys Cys Asp Pro Ala His Leu Phe Ala Met Thr Lys
85 90 95

Met Asn Ser Pro Met Gly Lys Ser Met Trp Tyr Asp Gly Glu Phe Leu
100 105 110

Tyr Ser Phe Thr Ile Asp Asn Ser Thr Tyr Ser Leu Phe Pro Gln Ala
115 120 125

Thr Pro Phe Gln Leu Pro Leu Lys Lys Cys Ala Val Val Gly Asn Gly
130 135 140

Gly Ile Leu Lys Lys Ser Gly Cys Gly Arg Gln Ile Asp Glu Ala Asn
145 150 155 160

Phe Val Met Arg Cys Asn Leu Pro Pro Leu Ser Ser Glu Tyr Thr Lys
165 170 175

Asp Val Gly Ser Lys Ser Gln Leu Val Thr Ala Asn Pro Ser Ile Ile
180 185 190

Arg Gln Arg Phe Gln Asn Leu Leu Trp Ser Arg Lys Thr Phe Val Asp
195 200 205

Asn Met Lys Ile Tyr Asn His Ser Tyr Ile Tyr Met Pro Ala Phe Ser
210 215 220

Met Lys Thr Gly Thr Glu Pro Ser Leu Arg Val Tyr Tyr Thr Leu Ser
225 230 235 240

Asp Val Gly Ala Asn Gln Thr Val Leu Phe Ala Asn Pro Asn Phe Leu
245 250 255

Arg Ser Ile Gly Lys Phe Trp Lys Ser Arg Gly Ile His Ala Lys Arg
260 265 270

Leu Ser Thr Gly Leu Phe Leu Val Ser Ala Ala Leu Gly Leu Cys Glu
275 280 285

Glu Val Ala Ile Tyr Gly Phe Trp Pro Phe Ser Val Asn Met His Glu
290 295 300

Gln Pro Ile Ser His His Tyr Tyr Asp Asn Val Leu Pro Phe Ser Gly
305 310 315 320

Phe His Ala Met Pro Glu Glu Phe Leu Gln Leu Trp Tyr Leu His Lys
325 330 335

Ile Gly Ala Leu Arg Met Gln Leu Asp Pro Cys Glu Asp Thr Ser Leu
340 345 350

Gln Pro Thr Ser
355

<210> 156
<211> 404
<212> PRT
<213> Homo sapiens

<400> 156

Met Ser His Ile Gln Ile Pro Pro Gly Leu Thr Glu Leu Leu Gln Gly
1 5 10 15

Tyr Thr Val Glu Val Leu Arg Gln Gln Pro Pro Asp Leu Val Glu Phe
20 25 30

Ala Val Glu Tyr Phe Thr Arg Leu Arg Glu Ala Arg Ala Pro Ala Ser
35 40 45

Val Leu Pro Ala Ala Thr Pro Arg Gln Ser Leu Gly His Pro Pro Pro
50 55 60

Glu Pro Gly Pro Asp Arg Val Ala Asp Ala Lys Gly Asp Ser Glu Ser
65 70 75 80

Glu Glu Asp Glu Asp Leu Glu Val Pro Val Pro Ser Arg Phe Asn Arg
85 90 95

Arg Val Ser Val Cys Ala Glu Thr Tyr Asn Pro Asp Glu Glu Glu Glu
100 105 110

Asp Thr Asp Pro Arg Val Ile His Pro Lys Thr Asp Glu Gln Arg Cys
115 120 125

Arg Leu Gln Glu Ala Cys Lys Asp Ile Leu Leu Phe Lys Asn Leu Asp
130 135 140

Gln Glu Gln Leu Ser Gln Val Leu Asp Ala Met Phe Glu Arg Ile Val
145 150 155 160

Lys Ala Asp Glu His Val Ile Asp Gln Gly Asp Asp Gly Asp Asn Phe
165 170 175

Tyr Val Ile Glu Arg Gly Thr Tyr Asp Ile Leu Val Thr Lys Asp Asn
180 185 190

Gln Thr Arg Ser Val Gly Gln Tyr Asp Asn Arg Gly Ser Phe Gly Glu
195 200 205

Leu Ala Leu Met Tyr Asn Thr Pro Arg Ala Ala Thr Ile Val Ala Thr
210 215 220

Ser Glu Gly Ser Leu Trp Gly Leu Asp Arg Val Thr Phe Arg Arg Ile
225 230 235 240

Ile Val Lys Asn Asn Ala Lys Lys Arg Lys Met Phe Glu Ser Phe Ile
245 250 255

Glu Ser Val Pro Leu Leu Lys Ser Leu Glu Val Ser Glu Arg Met Lys
260 265 270

Ile Val Asp Val Ile Gly Glu Lys Ile Tyr Lys Asp Gly Glu Arg Ile
275 280 285

Ile Thr Gln Gly Glu Lys Ala Asp Ser Phe Tyr Ile Ile Glu Ser Gly
290 295 300

Glu Val Ser Ile Leu Ile Arg Ser Arg Thr Lys Ser Asn Lys Asp Gly
305 310 315 320

Gly Asn Gln Glu Val Glu Ile Ala Arg Cys His Lys Gly Gln Tyr Phe
325 330 335

Gly Glu Leu Ala Leu Val Thr Asn Lys Pro Arg Ala Ala Ser Ala Tyr
340 345 350

Ala Val Gly Asp Val Lys Cys Leu Val Met Asp Val Gln Ala Phe Glu
355 360 365

Arg Leu Leu Gly Pro Cys Met Asp Ile Met Lys Arg Asn Ile Ser His
370 375 380

Tyr Glu Glu Gln Leu Val Lys Met Phe Gly Ser Ser Val Asp Leu Gly
385 390 395 400

Asn Leu Gly Gln

<210> 157
<211> 505
<212> PRT
<213> Homo sapiens

<400> 157

Met Ser Gly Ala Ser Ser Ser Glu Gln Asn Asn Asn Ser Tyr Glu Thr
1 5 10 15

Lys Thr Pro Asn Leu Arg Met Ser Glu Lys Lys Cys Ser Trp Ala Ser
20 25 30

Tyr Met Thr Asn Ser Pro Thr Leu Ile Val Met Ile Gly Leu Pro Ala
35 40 45

Arg Gly Lys Thr Tyr Val Ser Lys Lys Leu Thr Arg Tyr Leu Asn Trp
50 55 60

Ile Gly Val Pro Thr Lys Val Phe Asn Leu Gly Val Tyr Arg Arg Glu
65 70 75 80

Ala Val Lys Ser Tyr Lys Ser Tyr Asp Phe Phe Arg His Asp Asn Glu
85 90 95

Glu Ala Met Lys Ile Arg Lys Gln Cys Ala Leu Val Ala Leu Glu Asp
100 105 110

Val Lys Ala Tyr Leu Thr Glu Glu Asn Gly Gln Ile Ala Val Phe Asp
115 120 125

Ala Thr Asn Thr Thr Arg Glu Arg Arg Asp Met Ile Leu Asn Phe Ala
130 135 140

Glu Gln Asn Ser Phe Lys Val Phe Phe Val Glu Ser Val Cys Asp Asp
145 150 155 160

Pro Asp Val Ile Ala Ala Asn Ile Leu Glu Val Lys Val Ser Ser Pro
165 170 175

Asp Tyr Pro Glu Arg Asn Arg Glu Asn Val Met Glu Asp Phe Leu Lys
180 185 190

Arg Ile Glu Cys Tyr Lys Val Thr Tyr Arg Pro Leu Asp Pro Asp Asn
195 200 205

Tyr Asp Lys Asp Leu Ser Phe Ile Lys Val Ile Asn Val Gly Gln Arg
210 215 220

Phe Leu Val Asn Arg Val Gln Asp Tyr Ile Gln Ser Lys Ile Val Tyr
225 230 235 240

Tyr Leu Met Asn Ile His Val Gln Pro Arg Thr Ile Tyr Leu Cys Arg
245 250 255

His Gly Glu Ser Glu Phe Asn Leu Leu Gly Lys Ile Gly Gly Asp Ser
260 265 270

Gly Leu Ser Val Arg Gly Lys Gln Phe Ala Gln Ala Leu Arg Lys Phe
275 280 285

Leu Glu Glu Gln Glu Ile Thr Asp Leu Lys Val Trp Thr Ser Gln Leu
290 295 300

Lys Arg Thr Ile Gln Thr Ala Glu Ser Leu Gly Val Pro Tyr Glu Gln
305 310 315 320

Trp Lys Ile Leu Asn Glu Ile Asp Ala Gly Val Cys Glu Glu Met Thr
325 330 335

Tyr Ala Glu Ile Glu Lys Arg Tyr Pro Glu Glu Phe Ala Leu Arg Asp
340 345 350

Gln Glu Lys Tyr Leu Tyr Arg Tyr Pro Gly Gly Glu Ser Tyr Gln Asp
355 360 365

Leu Val Gln Arg Leu Glu Pro Val Ile Met Glu Leu Glu Arg Gln Gly
370 375 380

Asn Val Leu Val Ile Ser His Gln Ala Val Met Arg Cys Leu Leu Ala
385 390 395 400

Tyr Phe Leu Asp Lys Gly Ala Asp Glu Leu Pro Tyr Leu Arg Cys Pro
405 410 415

Leu His Thr Ile Phe Lys Leu Thr Pro Val Ala Tyr Gly Cys Lys Val
420 425 430

Glu Thr Ile Lys Leu Asn Val Glu Ala Val Asn Thr His Arg Asp Lys
435 440 445

Pro Thr Asn Asn Phe Pro Lys Asn Gln Thr Pro Val Arg Met Arg Arg
450 455 460

Asn Ser Phe Thr Pro Leu Ser Ser Ser Asn Thr Ile Arg Arg Pro Arg
465 470 475 480

Asn Tyr Ser Val Gly Ser Arg Pro Leu Lys Pro Leu Ser Pro Leu Arg
485 490 495

Ala Gln Asp Met Gln Glu Gly Ala Asp
500 505

<210> 158
<211> 543
<212> PRT
<213> Homo sapiens

<400> 158

Met Met Pro Thr Pro Val Ile Leu Leu Lys Glu Gly Thr Asp Ser Ser
1 5 10 15

Gln Gly Ile Pro Gln Leu Val Ser Asn Ile Ser Ala Cys Gln Val Ile
20 25 30

Ala Glu Ala Val Arg Thr Thr Leu Gly Pro Arg Gly Met Asp Lys Leu
35 40 45

Ile Val Asp Gly Arg Gly Lys Ala Thr Ile Ser Asn Asp Gly Ala Thr
50 55 60

Ile Leu Lys Leu Leu Asp Val Val His Pro Ala Ala Lys Thr Leu Val
65 70 75 80

Asp Ile Ala Lys Ser Gln Asp Ala Glu Val Gly Asp Gly Thr Thr Ser
85 90 95

Val Thr Leu Leu Ala Ala Glu Phe Leu Lys Gln Val Lys Pro Tyr Val
100 105 110

Glu Glu Gly Leu His Pro Gln Ile Ile Ile Arg Ala Phe Arg Thr Ala
115 120 125

Thr Gln Leu Ala Val Asn Lys Ile Lys Glu Ile Ala Val Thr Val Lys
130 135 140

Lys Ala Asp Lys Val Glu Gln Arg Lys Leu Leu Glu Lys Cys Ala Met
145 150 155 160

Thr Ala Leu Ser Ser Lys Leu Ile Ser Gln Gln Lys Ala Phe Phe Ala
165 170 175

Lys Met Val Val Asp Ala Val Met Met Leu Asp Asp Leu Leu Gln Leu
180 185 190

Lys Met Ile Gly Ile Lys Lys Val Gln Gly Gly Ala Leu Glu Asp Ser
195 200 205

Gln Leu Val Ala Gly Val Ala Phe Lys Lys Thr Phe Ser Tyr Ala Gly
210 215 220

Phe Glu Met Gln Pro Lys Lys Tyr His Asn Pro Lys Ile Ala Leu Leu
225 230 235 240

Asn Val Glu Leu Glu Leu Lys Ala Glu Lys Asp Asn Ala Glu Ile Arg
245 250 255

Val His Thr Val Glu Asp Tyr Gln Ala Ile Val Asp Ala Glu Trp Asn
260 265 270

Ile Leu Tyr Asp Lys Leu Glu Lys Ile His His Ser Gly Ala Lys Val
275 280 285

Val Leu Ser Lys Leu Pro Ile Gly Asp Val Ala Thr Gln Tyr Phe Ala
290 295 300

Asp Arg Asp Met Phe Cys Ala Gly Arg Val Pro Glu Glu Asp Leu Lys
305 310 315 320

Arg Thr Met Met Ala Cys Gly Gly Ser Ile Gln Thr Ser Val Asn Ala
325 330 335

Leu Ser Ala Asp Val Leu Gly Arg Cys Gln Val Phe Glu Glu Thr Gln
340 345 350

Ile Gly Gly Glu Arg Tyr Asn Phe Phe Thr Gly Cys Pro Lys Ala Lys
355 360 365

Thr Cys Thr Phe Ile Leu Arg Gly Gly Ala Glu Gln Phe Met Glu Glu
370 375 380

Thr Glu Arg Ser Leu His Asp Ala Ile Met Ile Val Arg Arg Ala Ile
385 390 395 400

Lys Asn Asp Ser Val Val Ala Gly Gly Gly Ala Ile Glu Met Glu Leu
405 410 415

Ser Lys Tyr Leu Arg Asp Tyr Ser Arg Thr Ile Pro Gly Lys Gln Gln
420 425 430

Leu Leu Ile Gly Ala Tyr Ala Lys Ala Leu Glu Ile Ile Pro Arg Gln
435 440 445

Leu Cys Asp Asn Ala Gly Phe Asp Ala Thr Asn Ile Leu Asn Lys Leu
450 455 460

Arg Ala Arg His Ala Gln Gly Gly Thr Trp Tyr Gly Val Asp Ile Asn
465 470 475 480

Asn Glu Asp Ile Ala Asp Asn Phe Glu Ala Phe Val Trp Glu Pro Ala
485 490 495

Met Val Arg Ile Asn Ala Leu Thr Ala Ala Ser Glu Ala Ala Cys Leu
500 505 510

Ile Val Ser Val Asp Glu Thr Ile Lys Asn Pro Arg Ser Thr Val Asp
515 520 525

Ala Pro Thr Ala Ala Gly Arg Gly Arg Gly Arg Gly Arg Pro His
530 535 540

<210> 159
<211> 359
<212> PRT
<213> Homo sapiens

<400> 159

Met Thr Leu Glu Ser Met Met Ala Cys Cys Leu Ser Asp Glu Val Lys
1 5 10 15

Glu Ser Lys Arg Ile Asn Ala Glu Ile Glu Lys Gln Leu Arg Arg Asp
20 25 30

Lys Arg Asp Ala Arg Arg Glu Leu Lys Leu Leu Leu Leu Gly Thr Gly
35 40 45

Glu Ser Gly Lys Ser Thr Phe Ile Lys Gln Met Arg Ile Ile His Gly
50 55 60

Ala Gly Tyr Ser Glu Glu Asp Lys Arg Gly Phe Thr Lys Leu Val Tyr
65 70 75 80

Gln Asn Ile Phe Thr Ala Met Gln Ala Met Ile Arg Ala Met Glu Thr
85 90 95

Leu Lys Ile Leu Tyr Lys Tyr Glu Gln Asn Lys Ala Asn Ala Leu Leu
100 105 110

Ile Arg Glu Val Asp Val Glu Lys Val Thr Thr Phe Glu His Gln Tyr
115 120 125

Val Ser Ala Ile Lys Thr Leu Trp Glu Asp Pro Gly Ile Gln Glu Cys
130 135 140

Tyr Asp Arg Arg Arg Glu Tyr Gln Leu Ser Asp Ser Ala Lys Tyr Tyr
145 150 155 160

Leu Thr Asp Val Asp Arg Ile Ala Thr Leu Gly Tyr Leu Pro Thr Gln
165 170 175

Gln Asp Val Leu Arg Val Arg Val Pro Thr Thr Gly Ile Ile Glu Tyr
180 185 190

Pro Phe Asp Leu Glu Asn Ile Ile Phe Arg Met Val Asp Val Gly Gly
195 200 205

Gln Arg Ser Glu Arg Arg Lys Trp Ile His Cys Phe Glu Asn Val Thr
210 215 220

Ser Ile Met Phe Leu Val Ala Leu Ser Glu Tyr Asp Gln Val Leu Val
225 230 235 240

Glu Ser Asp Asn Glu Asn Arg Met Glu Glu Ser Lys Ala Leu Phe Arg
245 250 255

Thr Ile Ile Thr Tyr Pro Trp Phe Gln Asn Ser Ser Val Ile Leu Phe
260 265 270

Leu Asn Lys Lys Asp Leu Leu Glu Asp Lys Ile Leu Tyr Ser His Leu
275 280 285

Val Asp Tyr Phe Pro Glu Phe Asp Gly Pro Gln Arg Asp Ala Gln Ala
290 295 300

Ala Arg Glu Phe Ile Leu Lys Met Phe Val Asp Leu Asn Pro Asp Ser
305 310 315 320

Asp Lys Ile Ile Tyr Ser His Phe Thr Cys Ala Thr Asp Thr Glu Asn
325 330 335

Ile Arg Phe Val Phe Ala Ala Val Lys Asp Thr Ile Leu Gln Leu Asn
340 345 350

Leu Lys Glu Tyr Asn Leu Val
355

<210> 160
<211> 704
<212> PRT
<213> Homo sapiens

<400> 160

Met Ala Asn Glu Asn His Gly Ser Pro Arg Glu Glu Ala Ser Leu Leu
1 5 10 15

Ser His Ser Pro Gly Thr Ser Asn Gln Ser Gln Pro Cys Ser Pro Lys
20 25 30

Pro Ile Arg Leu Val Gln Asp Leu Pro Glu Glu Leu Val His Ala Gly
35 40 45

Trp Glu Lys Cys Trp Ser Arg Arg Glu Asn Arg Pro Tyr Tyr Phe Asn
50 55 60

Arg Phe Thr Asn Gln Ser Leu Trp Glu Met Pro Val Leu Gly Gln His
65 70 75 80

Asp Val Ile Ser Asp Pro Leu Gly Leu Asn Ala Thr Pro Leu Pro Gln
85 90 95

Asp Ser Ser Leu Val Glu Thr Pro Pro Ala Glu Asn Lys Pro Arg Lys
100 105 110

Arg Gln Leu Ser Glu Glu Gln Pro Ser Gly Asn Gly Val Lys Lys Pro
115 120 125

Lys Ile Glu Ile Pro Val Thr Pro Thr Gly Gln Ser Val Pro Ser Ser
130 135 140

Pro Ser Ile Pro Gly Thr Pro Thr Leu Lys Met Trp Gly Thr Ser Pro
145 150 155 160

Glu Asp Lys Gln Gln Ala Ala Leu Leu Arg Pro Thr Glu Val Tyr Trp
165 170 175

Asp Leu Asp Ile Gln Thr Asn Ala Val Ile Lys His Arg Gly Pro Ser
180 185 190

Glu Val Leu Pro Pro His Pro Glu Val Glu Leu Leu Arg Ser Gln Leu
195 200 205

Ile Leu Lys Leu Arg Gln His Tyr Arg Glu Leu Cys Gln Gln Arg Glu
210 215 220

Gly Ile Glu Pro Pro Arg Glu Ser Phe Asn Arg Trp Met Leu Glu Arg
225 230 235 240

Lys Val Val Asp Lys Gly Ser Asp Pro Leu Leu Pro Ser Asn Cys Glu
245 250 255

Pro Val Val Ser Pro Ser Met Phe Arg Glu Ile Met Asn Asp Ile Pro
260 265 270

Ile Arg Leu Ser Arg Ile Lys Phe Arg Glu Glu Ala Lys Arg Leu Leu
275 280 285

Phe Lys Tyr Ala Glu Ala Ala Arg Arg Leu Ile Glu Ser Arg Ser Ala
290 295 300

Ser Pro Asp Ser Arg Lys Val Val Lys Trp Asn Val Glu Asp Thr Phe
305 310 315 320

Ser Trp Leu Arg Lys Asp His Ser Ala Ser Lys Glu Asp Tyr Met Asp
325 330 335

Arg Leu Glu His Leu Arg Arg Gln Cys Gly Pro His Val Ser Ala Ala
340 345 350

Ala Lys Asp Ser Val Glu Gly Ile Cys Ser Lys Ile Tyr His Ile Ser
355 360 365

Leu Glu Tyr Val Lys Arg Ile Arg Glu Lys His Leu Ala Ile Leu Lys
370 375 380

Glu Asn Asn Ile Ser Glu Glu Val Glu Ala Pro Glu Val Glu Pro Arg
385 390 395 400

Leu Val Tyr Cys Tyr Pro Val Arg Leu Ala Val Ser Ala Pro Pro Met
405 410 415

Pro Ser Val Glu Met His Met Glu Asn Asn Val Val Cys Ile Arg Tyr
420 425 430

Lys Gly Glu Met Val Lys Val Ser Arg Asn Tyr Phe Ser Lys Leu Trp
435 440 445

Leu Leu Tyr Arg Tyr Ser Cys Ile Asp Asp Ser Ala Phe Glu Arg Phe
450 455 460

Leu Pro Arg Val Trp Cys Leu Leu Arg Arg Tyr Gln Met Met Phe Gly
465 470 475 480

Val Gly Leu Tyr Glu Gly Thr Gly Leu Gln Gly Ser Leu Pro Val His
485 490 495

Val Phe Glu Ala Leu His Arg Leu Phe Gly Val Ser Phe Glu Cys Phe
500 505 510

Ala Ser Pro Leu Asn Cys Tyr Phe Arg Gln Tyr Cys Ser Ala Phe Pro
515 520 525

Asp Thr Asp Gly Tyr Phe Gly Ser Arg Gly Pro Cys Leu Asp Phe Ala
530 535 540

Pro Leu Ser Gly Ser Phe Glu Ala Asn Pro Pro Phe Cys Glu Glu Leu
545 550 555 560

Met Asp Ala Met Val Ser His Phe Glu Arg Leu Leu Glu Ser Ser Pro
565 570 575

Glu Pro Leu Ser Phe Ile Val Phe Ile Pro Glu Trp Arg Glu Pro Pro
580 585 590

Thr Pro Ala Leu Thr Arg Met Glu Gln Ser Arg Phe Lys Arg His Gln
595 600 605

Leu Ile Leu Pro Ala Phe Glu His Glu Tyr Arg Ser Gly Ser Gln His
610 615 620

Ile Cys Lys Lys Glu Glu Met His Tyr Lys Ala Val His Asn Thr Ala
625 630 635 640

Val Leu Phe Leu Gln Asn Asp Pro Gly Phe Ala Lys Trp Ala Pro Thr
645 650 655

Pro Glu Arg Leu Gln Glu Leu Ser Ala Ala Tyr Arg Gln Ser Gly Arg
660 665 670

Ser His Ser Ser Gly Ser Ser Ser Ser Ser Ser Ser Glu Ala Lys Asp
675 680 685

Arg Asp Ser Gly Arg Glu Gln Gly Pro Ser Arg Glu Pro His Pro Thr
690 695 700

<210> 161
<211> 573
<212> PRT
<213> Homo sapiens

<400> 161

Met Leu Gly Ser Leu Gly Leu Trp Ala Leu Leu Pro Thr Ala Val Glu
1               5                   10                  15

Ala Pro Pro Asn Arg Arg Thr Cys Val Phe Phe Glu Ala Pro Gly Val
            20                  25                  30

Arg Gly Ser Thr Lys Thr Leu Gly Glu Leu Leu Asp Thr Gly Thr Glu
        35                  40                  45

Leu Pro Arg Ala Ile Arg Cys Leu Tyr Ser Arg Cys Cys Phe Gly Ile
    50                  55                  60

Trp Asn Leu Thr Gln Asp Arg Ala Gln Val Glu Met Gln Gly Cys Arg
65                  70                  75                  80

Asp Ser Asp Glu Pro Gly Cys Glu Ser Leu His Cys Asp Pro Ser Pro
                85                  90                  95

Arg Ala His Pro Ser Pro Gly Ser Thr Leu Phe Thr Cys Ser Cys Gly
            100                 105                 110

Thr Asp Phe Cys Asn Ala Asn Tyr Ser His Leu Pro Pro Pro Gly Ser
        115                 120                 125

Pro Gly Thr Pro Gly Ser Gln Gly Pro Gln Ala Ala Pro Gly Glu Ser
    130                 135                 140

Ile Trp Met Ala Leu Val Leu Leu Gly Leu Phe Leu Leu Leu Leu Leu
145                 150                 155                 160

Leu Leu Gly Ser Ile Ile Leu Ala Leu Leu Gln Arg Lys Asn Tyr Arg
                165                 170                 175

Val Arg Gly Glu Pro Val Pro Glu Pro Arg Pro Asp Ser Gly Arg Asp
            180                 185                 190

Trp Ser Val Glu Leu Gln Glu Leu Pro Glu Leu Cys Phe Ser Gln Val
        195                 200                 205

Ile Arg Glu Gly Gly His Ala Val Val Trp Ala Gly Gln Leu Gln Gly
    210                 215                 220

Lys Leu Val Ala Ile Lys Ala Phe Pro Pro Arg Ser Val Ala Gln Phe
225                 230                 235                 240

Gln Ala Glu Arg Ala Leu Tyr Glu Leu Pro Gly Leu Gln His Asp His
                245                 250                 255

Ile Val Arg Phe Ile Thr Ala Ser Arg Gly Gly Pro Gly Arg Leu Leu

260 265 270

Ser Gly Pro Leu Leu Val Leu Glu Leu His Pro Lys Gly Ser Leu Cys
275 280 285

His Tyr Leu Thr Gln Tyr Thr Ser Asp Trp Gly Ser Ser Leu Arg Met
290 295 300

Ala Leu Ser Leu Ala Gln Gly Leu Ala Phe Leu His Glu Glu Arg Trp
305 310 315 320

Gln Asn Gly Gln Tyr Lys Pro Gly Ile Ala His Arg Asp Leu Ser Ser
325 330 335

Gln Asn Val Leu Ile Arg Glu Asp Gly Ser Cys Ala Ile Gly Asp Leu
340 345 350

Gly Leu Ala Leu Val Leu Pro Gly Leu Thr Gln Pro Pro Ala Trp Thr
355 360 365

Pro Thr Gln Pro Gln Gly Pro Ala Ala Ile Met Glu Ala Gly Thr Gln
370 375 380

Arg Tyr Met Ala Pro Glu Leu Leu Asp Lys Thr Leu Asp Leu Gln Asp
385 390 395 400

Trp Gly Met Ala Leu Arg Arg Ala Asp Ile Tyr Ser Leu Ala Leu Leu
405 410 415

Leu Trp Glu Ile Leu Ser Arg Cys Pro Asp Leu Arg Pro Asp Ser Ser
420 425 430

Pro Pro Pro Phe Gln Leu Ala Tyr Glu Ala Glu Leu Gly Asn Thr Pro
435 440 445

Thr Ser Asp Glu Leu Trp Ala Leu Ala Val Gln Glu Arg Arg Arg Pro
450 455 460

Tyr Ile Pro Ser Thr Trp Arg Cys Phe Ala Thr Asp Pro Asp Gly Leu
465 470 475 480

Arg Glu Leu Leu Glu Asp Cys Trp Asp Ala Asp Pro Glu Ala Arg Leu
485 490 495

Thr Ala Glu Cys Val Gln Gln Arg Leu Ala Ala Leu Ala His Pro Gln
500 505 510

Glu Ser His Pro Phe Pro Glu Ser Cys Pro Arg Gly Cys Pro Pro Leu
515 520 525

Cys Pro Glu Asp Cys Thr Ser Ile Pro Ala Pro Thr Ile Leu Pro Cys

530 535 540

Arg Pro Gln Arg Ser Ala Cys His Phe Ser Val Gln Gln Gly Pro Cys
545 550 555 560

Ser Arg Asn Pro Gln Pro Ala Cys Thr Leu Ser Pro Val
565 570

<210> 162
<211> 402
<212> PRT
<213> Homo sapi ens

<400> 162

Met Gly Leu Gly Gln Gly Trp Gly Trp Glu Ala Ser Cys Phe Ala Cys
1 5 10 15

Leu Ile Arg Ser Cys Cys Gln Val Val Thr Phe Thr Phe Pro Phe Gly
20 25 30

Phe Gln Gly Ile Ser Gln Arg Leu Glu Asn Val Ser Gly Tyr Tyr Ala
35 40 45

Asp Ala Arg Leu Glu Val Gly Ser Thr Gln Leu Arg Thr Ala Gly Ser
50 55 60

Cys Ser His Ser Phe Lys Arg Ser Phe Leu Glu Lys Lys Arg Phe Thr
65 70 75 80

Glu Glu Ala Thr Lys Tyr Phe Arg Glu Arg Val Ser Pro Val His Leu
85 90 95

Gln Ile Leu Leu Thr Asn Asn Glu Ala Trp Lys Arg Phe Val Thr Ala
100 105 110

Ala Glu Leu Pro Arg Asp Glu Ala Asp Ala Leu Tyr Glu Ala Leu Lys
115 120 125

Lys Leu Arg Thr Tyr Ala Ala Ile Glu Asp Glu Tyr Val Gln Gln Lys
130 135 140

Asp Glu Gln Phe Arg Glu Trp Phe Leu Lys Glu Phe Pro Gln Val Lys
145 150 155 160

Arg Lys Ile Gln Glu Ser Ile Glu Lys Leu Arg Ala Leu Ala Asn Gly
165 170 175

Ile Glu Glu Val His Arg Gly Cys Thr Ile Ser Asn Val Val Ser Ser
180 185 190

Ser Thr Gly Ala Ala Ser Gly Ile Met Ser Leu Ala Gly Leu Val Leu
195 200 205

Ala Pro Phe Thr Ala Gly Thr Ser Leu Ala Leu Thr Ala Ala Gly Val
210 215 220

Gly Leu Gly Ala Ala Ser Ala Val Thr Gly Ile Thr Thr Ser Ile Val
225 230 235 240

Glu His Ser Tyr Thr Ser Ser Ala Glu Ala Glu Ala Ser Arg Leu Thr
245 250 255

Ala Thr Ser Ile Asp Arg Leu Lys Val Phe Lys Glu Val Met Arg Asp
260 265 270

Ile Thr Pro Asn Leu Leu Ser Leu Leu Asn Asn Tyr Tyr Glu Ala Thr
275 280 285

Gln Thr Ile Gly Ser Glu Ile Arg Ala Ile Arg Gln Ala Arg Ala Arg
290 295 300

Ala Arg Leu Pro Val Thr Thr Trp Arg Ile Ser Ala Gly Ser Gly Gly
305 310 315 320

Gln Ala Glu Arg Thr Ile Ala Gly Thr Thr Arg Ala Val Ser Arg Gly
325 330 335

Ala Arg Ile Leu Ser Ala Thr Thr Ser Gly Ile Phe Leu Ala Leu Asp
340 345 350

Val Val Asn Leu Val Tyr Glu Ser Lys His Leu His Glu Gly Ala Lys
355 360 365

Ser Ala Ser Ala Glu Glu Leu Arg Arg Gln Ala Gln Glu Leu Glu Glu
370 375 380

Asn Leu Met Glu Leu Thr Gln Ile Tyr Gln Arg Leu Asn Pro Cys His
385 390 395 400

Thr His

<210> 163
<211> 326
<212> PRT
<213> Homo sapiens

<400> 163

Met Ala Leu Ser Gly Ser Thr Pro Ala Pro Cys Trp Glu Glu Asp Glu
1 5 10 15

Cys Leu Asp Tyr Tyr Gly Met Leu Ser Leu His Arg Met Phe Glu Val
20 25 30

Val Gly Gly Gln Leu Thr Glu Cys Glu Leu Glu Leu Leu Ala Phe Leu
35 40 45

Leu Asp Glu Ala Pro Gly Ala Ala Gly Gly Leu Ala Arg Ala Arg Ser
50 55 60

Gly Leu Glu Leu Leu Leu Glu Leu Glu Arg Arg Gly Gln Cys Asp Glu
65 70 75 80

Ser Asn Leu Arg Leu Leu Gly Gln Leu Leu Arg Val Leu Ala Arg His
85 90 95

Asp Leu Leu Pro His Leu Ala Arg Lys Arg Arg Arg Pro Val Ser Pro
100 105 110

Glu Arg Tyr Ser Tyr Gly Thr Ser Ser Ser Ser Lys Arg Thr Glu Gly
115 120 125

Ser Cys Arg Arg Arg Arg Gln Ser Ser Ser Ser Ala Asn Ser Gln Gln
130 135 140

Gly Gln Trp Glu Thr Gly Ser Pro Pro Thr Lys Arg Gln Arg Arg Ser
145 150 155 160

Arg Gly Arg Pro Ser Gly Gly Ala Arg Arg Arg Arg Arg Gly Ala Pro
165 170 175

Ala Ala Pro Gln Gln Gln Ser Glu Pro Ala Arg Pro Ser Ser Glu Gly
180 185 190

Lys Val Thr Cys Asp Ile Arg Leu Arg Val Arg Ala Glu Tyr Cys Glu
195 200 205

His Gly Pro Ala Leu Glu Gln Gly Val Ala Ser Arg Arg Pro Gln Ala
210 215 220

Leu Ala Arg Gln Leu Asp Val Phe Gly Gln Ala Thr Ala Val Leu Arg
225 230 235 240

Ser Arg Asp Leu Gly Ser Val Val Cys Asp Ile Lys Phe Ser Glu Leu
245 250 255

Ser Tyr Leu Asp Ala Phe Trp Gly Asp Tyr Leu Ser Gly Ala Leu Leu
260 265 270

Gln Ala Leu Arg Gly Val Phe Leu Thr Glu Ala Leu Arg Glu Ala Val
275 280 285

Gly Arg Glu Ala Val Arg Leu Leu Val Ser Val Asp Glu Ala Asp Tyr
290 295 300

Glu Ala Gly Arg Arg Arg Leu Leu Leu Met Glu Glu Glu Gly Gly Arg
305 310 315 320

Arg Pro Thr Glu Ala Ser
325

<210> 164
<211> 556
<212> PRT
<213> Homo sapiens

<400> 164

Met Glu Leu Cys Gly Leu Gly Leu Pro Arg Pro Pro Met Leu Leu Ala
1 5 10 15

Leu Leu Leu Ala Thr Leu Leu Ala Ala Met Leu Ala Leu Leu Thr Gln
20 25 30

Val Ala Leu Val Val Gln Val Ala Glu Ala Ala Arg Ala Pro Ser Val
35 40 45

Ser Ala Lys Pro Gly Pro Ala Leu Trp Pro Leu Pro Leu Ser Val Lys
50 55 60

Met Thr Pro Asn Leu Leu His Leu Ala Pro Glu Asn Phe Tyr Ile Ser
65 70 75 80

His Ser Pro Asn Ser Thr Ala Gly Pro Ser Cys Thr Leu Leu Glu Glu
85 90 95

Ala Phe Arg Arg Tyr His Gly Tyr Ile Phe Gly Phe Tyr Lys Trp His
100 105 110

His Glu Pro Ala Glu Phe Gln Ala Lys Thr Gln Val Gln Gln Leu Leu
115 120 125

Val Ser Ile Thr Leu Gln Ser Glu Cys Asp Ala Phe Pro Asn Ile Ser
130 135 140

Ser Asp Glu Ser Tyr Thr Leu Leu Val Lys Glu Pro Val Ala Val Leu
145 150 155 160

Lys Ala Asn Arg Val Trp Gly Ala Leu Arg Gly Leu Glu Thr Phe Ser
165 170 175

Gln Leu Val Tyr Gln Asp Ser Tyr Gly Thr Phe Thr Ile Asn Glu Ser
180 185 190

Thr Ile Ile Asp Ser Pro Arg Phe Ser His Arg Gly Ile Leu Ile Asp
195 200 205

Thr Ser Arg His Tyr Leu Pro Val Lys Ile Ile Leu Lys Thr Leu Asp
210 215 220

Ala Met Ala Phe Asn Lys Phe Asn Val Leu His Trp His Ile Val Asp
225 230 235 240

Asp Gln Ser Phe Pro Tyr Gln Ser Ile Thr Phe Pro Glu Leu Ser Asn
245 250 255

Lys Gly Ser Tyr Ser Leu Ser His Val Tyr Thr Pro Asn Asp Val Arg
260 265 270

Met Val Ile Glu Tyr Ala Arg Leu Arg Gly Ile Arg Val Leu Pro Glu
275 280 285

Phe Asp Thr Pro Gly His Thr Leu Ser Trp Gly Lys Gly Gln Lys Asp
290 295 300

Leu Leu Thr Pro Cys Tyr Ser Arg Gln Asn Lys Leu Asp Ser Phe Gly
305 310 315 320

Pro Ile Asn Pro Thr Leu Asn Thr Thr Tyr Ser Phe Leu Thr Thr Phe
325 330 335

Phe Lys Glu Ile Ser Glu Val Phe Pro Asp Gln Phe Ile His Leu Gly
340 345 350

Gly Asp Glu Val Glu Phe Lys Cys Trp Glu Ser Asn Pro Lys Ile Gln
355 360 365

Asp Phe Met Arg Gln Lys Gly Phe Gly Thr Asp Phe Lys Lys Leu Glu
370 375 380

Ser Phe Tyr Ile Gln Lys Val Leu Asp Ile Ile Ala Thr Ile Asn Lys
385 390 395 400

Gly Ser Ile Val Trp Gln Glu Val Phe Asp Asp Lys Ala Lys Leu Ala
405 410 415

Pro Gly Thr Ile Val Glu Val Trp Lys Asp Ser Ala Tyr Pro Glu Glu
420 425 430

Leu Ser Arg Val Thr Ala Ser Gly Phe Pro Val Ile Leu Ser Ala Pro
435 440 445

Trp Tyr Leu Asp Leu Ile Ser Tyr Gly Gln Asp Trp Arg Lys Tyr Tyr
450 455 460

Lys Val Glu Pro Leu Asp Phe Gly Gly Thr Gln Lys Gln Lys Gln Leu
465 470 475 480

Phe Ile Gly Gly Glu Ala Cys Leu Trp Gly Glu Tyr Val Asp Ala Thr
485 490 495

Asn Leu Thr Pro Arg Leu Trp Pro Arg Ala Ser Ala Val Gly Glu Arg
500 505 510

Leu Trp Ser Ser Lys Asp Val Arg Asp Met Asp Asp Ala Tyr Asp Arg
515 520 525

Leu Thr Arg His Arg Cys Arg Met Val Glu Arg Gly Ile Ala Ala Gln
530 535 540

Pro Leu Tyr Ala Gly Tyr Cys Asn His Glu Asn Met
545 550 555

<210> 165
<211> 463
<212> PRT
<213> Homo sapiens

<400> 165

Met Glu Thr Glu Gln Pro Glu Glu Thr Phe Pro Asn Thr Glu Thr Asn
1               5                   10                  15

Gly Glu Phe Gly Lys Arg Pro Ala Glu Asp Met Glu Glu Glu Gln Ala
            20                  25                  30

Phe Lys Arg Ser Arg Asn Thr Asp Glu Met Val Glu Leu Arg Ile Leu
        35                  40                  45

Leu Gln Ser Lys Asn Ala Gly Ala Val Ile Gly Lys Gly Gly Lys Asn
    50                  55                  60

Ile Lys Ala Leu Arg Thr Asp Tyr Asn Ala Ser Val Ser Val Pro Asp
65                  70                  75                  80

Ser Ser Gly Pro Glu Arg Ile Leu Ser Ile Ser Ala Asp Ile Glu Thr
                85                  90                  95

Ile Gly Glu Ile Leu Lys Lys Ile Ile Pro Thr Leu Glu Glu Gly Leu
            100                 105                 110

Gln Leu Pro Ser Pro Thr Ala Thr Ser Gln Leu Pro Leu Glu Ser Asp
        115                 120                 125

Ala Val Glu Cys Leu Asn Tyr Gln His Tyr Lys Gly Ser Asp Phe Asp
    130                 135                 140

Cys Glu Leu Arg Leu Leu Ile His Gln Ser Leu Ala Gly Gly Ile Ile
145                 150                 155                 160

Gly Val Lys Gly Ala Lys Ile Lys Glu Leu Arg Glu Asn Thr Gln Thr

165 170 175

Thr Ile Lys Leu Phe Gln Glu Cys Cys Pro His Ser Thr Asp Arg Val
180 185 190

Val Leu Ile Gly Gly Lys Pro Asp Arg Val Val Glu Cys Ile Lys Ile
195 200 205

Ile Leu Asp Leu Ile Ser Glu Ser Pro Ile Lys Gly Arg Ala Gln Pro
210 215 220

Tyr Asp Pro Asn Phe Tyr Asp Glu Thr Tyr Asp Tyr Gly Gly Phe Thr
225 230 235 240

Met Met Phe Asp Asp Arg Arg Gly Arg Pro Val Gly Phe Pro Met Arg
245 250 255

Gly Arg Gly Gly Phe Asp Arg Met Pro Pro Gly Arg Gly Gly Arg Pro
260 265 270

Met Pro Pro Ser Arg Arg Asp Tyr Asp Asp Met Ser Pro Arg Arg Gly
275 280 285

Pro Pro Pro Pro Pro Pro Gly Arg Gly Gly Arg Gly Gly Ser Arg Ala
290 295 300

Arg Asn Leu Pro Leu Pro Pro Pro Pro Pro Pro Arg Gly Gly Asp Leu
305 310 315 320

Met Ala Tyr Asp Arg Arg Gly Arg Pro Gly Asp Arg Tyr Asp Gly Met
325 330 335

Val Gly Phe Ser Ala Asp Glu Thr Trp Asp Ser Ala Ile Asp Thr Trp
340 345 350

Ser Pro Ser Glu Trp Gln Met Ala Tyr Glu Pro Gln Gly Gly Ser Gly
355 360 365

Tyr Asp Tyr Ser Tyr Ala Gly Gly Arg Gly Ser Tyr Gly Asp Leu Gly
370 375 380

Gly Pro Ile Ile Thr Thr Gln Val Thr Ile Pro Lys Asp Leu Ala Gly
385 390 395 400

Ser Ile Ile Gly Lys Gly Gly Gln Arg Ile Lys Gln Ile Arg His Glu
405 410 415

Ser Gly Ala Ser Ile Lys Ile Asp Glu Pro Leu Glu Gly Ser Glu Asp
420 425 430

Arg Ile Ile Thr Ile Thr Gly Thr Gln Asp Gln Ile Gln Asn Ala Gln

435 440 445

Tyr Leu Leu Gln Asn Ser Val Lys Gln Tyr Ser Gly Lys Phe Phe
450 455 460

<210> 166
<211> 538
<212> PRT
<213> Homo sapi ens

<400> 166

Met Thr Thr Pro Gly Lys Glu Asn Phe Arg Leu Lys Ser Tyr Lys Asn
1               5                   10                  15

Lys Ser Leu Asn Pro Asp Glu Met Arg Arg Arg Arg Glu Glu Glu Gly
            20                  25                  30

Leu Gln Leu Arg Lys Gln Lys Arg Glu Glu Gln Leu Phe Lys Arg Arg
        35                  40                  45

Asn Val Ala Thr Ala Glu Glu Glu Thr Glu Glu Glu Val Met Ser Asp
    50                  55                  60

Gly Gly Phe His Glu Ala Gln Ile Asn Asn Met Glu Met Ala Pro Gly
65                  70                  75                  80

Gly Val Ile Thr Ser Asp Met Ile Glu Met Ile Phe Ser Lys Ser Pro
                85                  90                  95

Glu Gln Gln Leu Ser Ala Thr Gln Lys Phe Arg Lys Leu Leu Ser Lys
            100                 105                 110

Glu Pro Asn Pro Pro Ile Asp Glu Val Ile Ser Thr Pro Gly Val Val
        115                 120                 125

Ala Arg Phe Val Glu Phe Leu Lys Arg Lys Glu Asn Cys Thr Leu Gln
    130                 135                 140

Phe Glu Ser Ala Trp Val Leu Thr Asn Ile Ala Ser Gly Asn Ser Leu
145                 150                 155                 160

Gln Thr Arg Ile Val Ile Gln Ala Gly Ala Val Pro Ile Phe Ile Glu
                165                 170                 175

Leu Leu Ser Ser Glu Phe Glu Asp Val Gln Glu Gln Ala Val Trp Ala
            180                 185                 190

Leu Gly Asn Ile Ala Gly Asp Ser Thr Met Cys Arg Asp Tyr Val Leu
        195                 200                 205

Asp Cys Asn Ile Leu Pro Pro Leu Leu Gln Leu Phe Ser Lys Gln Asn
    210                 215                 220

Arg Leu Thr Met Thr Arg Asn Ala Val Trp Ala Leu Ser Asn Leu Cys
225 230 235 240

Arg Gly Lys Ser Pro Pro Pro Glu Phe Ala Lys Val Ser Pro Cys Leu
245 250 255

Asn Val Leu Ser Trp Leu Leu Phe Val Ser Asp Thr Asp Val Leu Ala
260 265 270

Asp Ala Cys Trp Ala Leu Ser Tyr Leu Ser Asp Gly Pro Asn Asp Lys
275 280 285

Ile Gln Ala Val Ile Asp Ala Gly Val Cys Arg Arg Leu Val Glu Leu
290 295 300

Leu Met His Asn Asp Tyr Lys Val Val Ser Pro Ala Leu Arg Ala Val
305 310 315 320

Gly Asn Ile Val Thr Gly Asp Asp Ile Gln Thr Gln Val Ile Leu Asn
325 330 335

Cys Ser Ala Leu Gln Ser Leu Leu His Leu Leu Ser Ser Pro Lys Glu
340 345 350

Ser Ile Lys Lys Glu Ala Cys Trp Thr Ile Ser Asn Ile Thr Ala Gly
355 360 365

Asn Arg Ala Gln Ile Gln Thr Val Ile Asp Ala Asn Ile Phe Pro Ala
370 375 380

Leu Ile Ser Ile Leu Gln Thr Ala Glu Phe Arg Thr Arg Lys Glu Ala
385 390 395 400

Ala Trp Ala Ile Thr Asn Ala Thr Ser Gly Gly Ser Ala Glu Gln Ile
405 410 415

Lys Tyr Leu Val Glu Leu Gly Cys Ile Lys Pro Leu Cys Asp Leu Leu
420 425 430

Thr Val Met Asp Ser Lys Ile Val Gln Val Ala Leu Asn Gly Leu Glu
435 440 445

Asn Ile Leu Arg Leu Gly Glu Gln Glu Ala Lys Arg Asn Gly Thr Gly
450 455 460

Ile Asn Pro Tyr Cys Ala Leu Ile Glu Glu Ala Tyr Gly Leu Asp Lys
465 470 475 480

Ile Glu Phe Leu Gln Ser His Glu Asn Gln Glu Ile Tyr Gln Lys Ala
485 490 495

Phe Asp Leu Ile Glu His Tyr Phe Gly Thr Glu Asp Glu Asp Ser Ser
500 505 510

Ile Ala Pro Gln Val Asp Leu Asn Gln Gln Gln Tyr Ile Phe Gln Gln
515 520 525

Cys Glu Ala Pro Met Glu Gly Phe Gln Leu
530 535

<210> 167
<211> 261
<212> PRT
<213> Homo sapiens

<400> 167

Met Asn Pro Asn Cys Ala Arg Cys Gly Lys Ile Val Tyr Pro Thr Glu
1 5 10 15

Lys Val Asn Cys Leu Asp Lys Phe Trp His Lys Ala Cys Phe His Cys
20 25 30

Glu Thr Cys Lys Met Thr Leu Asn Met Lys Asn Tyr Lys Gly Tyr Glu
35 40 45

Lys Lys Pro Tyr Cys Asn Ala His Tyr Pro Lys Gln Ser Phe Thr Met
50 55 60

Val Ala Asp Thr Pro Glu Asn Leu Arg Leu Lys Gln Gln Ser Glu Leu
65 70 75 80

Gln Ser Gln Val Arg Tyr Lys Glu Glu Phe Glu Lys Asn Lys Gly Lys
85 90 95

Gly Phe Ser Val Val Ala Asp Thr Pro Glu Leu Gln Arg Ile Lys Lys
100 105 110

Thr Gln Asp Gln Ile Ser Asn Ile Lys Tyr His Glu Glu Phe Glu Lys
115 120 125

Ser Arg Met Gly Pro Ser Gly Gly Glu Gly Met Glu Pro Glu Arg Arg
130 135 140

Asp Ser Gln Asp Gly Ser Ser Tyr Arg Arg Pro Leu Glu Gln Gln Gln
145 150 155 160

Pro His His Ile Pro Thr Ser Ala Pro Val Tyr Gln Gln Pro Gln Gln
165 170 175

Gln Pro Val Ala Gln Ser Tyr Gly Gly Tyr Lys Glu Pro Ala Ala Pro
180 185 190

Val Ser Ile Gln Arg Ser Ala Pro Gly Gly Gly Gly Lys Arg Tyr Arg
195 200 205

Ala Val Tyr Asp Tyr Ser Ala Ala Asp Glu Asp Glu Val Ser Phe Gln
210 215 220

Asp Gly Asp Thr Ile Val Asn Val Gln Gln Ile Asp Asp Gly Trp Met
225 230 235 240

Tyr Gly Thr Val Glu Arg Thr Gly Asp Thr Gly Met Leu Pro Ala Asn
245 250 255

Tyr Val Glu Ala Ile
260

<210> 168
<211> 372
<212> PRT
<213> Homo sapiens

<400> 168

Met Ser Leu Arg Cys Gly Asp Ala Ala Arg Thr Leu Gly Pro Arg Val
1 5 10 15

Phe Gly Arg Tyr Phe Cys Ser Pro Val Arg Pro Leu Ser Ser Leu Pro
20 25 30

Asp Lys Lys Lys Glu Leu Leu Gln Asn Gly Pro Asp Leu Gln Asp Phe
35 40 45

Val Ser Gly Asp Leu Ala Asp Arg Ser Thr Trp Asp Glu Tyr Lys Gly
50 55 60

Asn Leu Lys Arg Gln Lys Gly Glu Arg Leu Arg Leu Pro Pro Trp Leu
65 70 75 80

Lys Thr Glu Ile Pro Met Gly Lys Asn Tyr Asn Lys Leu Lys Asn Thr
85 90 95

Leu Arg Asn Leu Asn Leu His Thr Val Cys Glu Glu Ala Arg Cys Pro
100 105 110

Asn Ile Gly Glu Cys Trp Gly Gly Gly Glu Tyr Ala Thr Ala Thr Ala
115 120 125

Thr Ile Met Leu Met Gly Asp Thr Cys Thr Arg Gly Cys Arg Phe Cys
130 135 140

Ser Val Lys Thr Ala Arg Asn Pro Pro Pro Leu Asp Ala Ser Glu Pro
145 150 155 160

Tyr Asn Thr Ala Lys Ala Ile Ala Glu Trp Gly Leu Asp Tyr Val Val
165 170 175

Leu Thr Ser Val Asp Arg Asp Asp Met Pro Asp Gly Gly Ala Glu His
180 185 190

Ile Ala Lys Thr Val Ser Tyr Leu Lys Glu Arg Asn Pro Lys Ile Leu
195 200 205

Val Glu Cys Leu Thr Pro Asp Phe Arg Gly Asp Leu Lys Ala Ile Glu
210 215 220

Lys Val Ala Leu Ser Gly Leu Asp Val Tyr Ala His Asn Val Glu Thr
225 230 235 240

Val Pro Glu Leu Gln Ser Lys Val Arg Asp Pro Arg Val Asn Phe Asp
245 250 255

Gln Ser Leu Arg Val Leu Lys His Ala Lys Lys Val Gln Pro Asp Val
260 265 270

Ile Ser Lys Thr Ser Ile Met Leu Gly Leu Gly Glu Asn Asp Glu Gln
275 280 285

Val Tyr Ala Thr Met Lys Ala Leu Arg Glu Ala Asp Val Asp Cys Leu
290 295 300

Thr Leu Gly Gln Tyr Met Gln Pro Thr Arg Arg His Leu Lys Val Glu
305 310 315 320

Glu Tyr Ile Thr Pro Glu Lys Phe Lys Tyr Trp Glu Lys Val Gly Asn
325 330 335

Glu Leu Gly Phe His Tyr Thr Ala Ser Gly Pro Leu Val Arg Ser Ser
340 345 350

Tyr Lys Ala Gly Glu Phe Phe Leu Lys Asn Leu Val Ala Lys Arg Lys
355 360 365

Thr Lys Asp Leu
370

<210> 169
<211> 643
<212> PRT
<213> Homo sapiens

<400> 169

Met Leu Leu Pro Cys His Trp Val Leu Asp Ala Thr Phe Ser Asp Gly
1 5 10 15

Ser Leu Gly Gln Trp Val Lys Asn Thr Cys Ala Thr Tyr Ala Leu Ser

20 25 30

Pro Val Val Leu Pro Pro Gln Pro Gln Pro Arg Lys Lys Ala Thr Asp
35 40 45

Lys Asp Tyr Ser Ala Phe His Leu Gly His Leu Arg Glu Val Arg Leu
50 55 60

Phe Leu Arg Gly Gly Thr Ser Asp Gln Arg Met Asp Ser Leu Val Leu
65 70 75 80

Cys Pro Thr Tyr Phe Lys Leu Trp Arg Thr Leu Ser Gly Ser Pro Gly
85 90 95

Leu Gln Leu Ser Asp Leu His Phe Gly Ser Gln Pro Glu Gly Lys Phe
100 105 110

Ser Leu Arg Arg Ala Val Ser Val Lys Gln Arg Glu Glu Pro Gln Asp
115 120 125

Trp Pro Leu Asn Glu Lys Arg Thr Leu Trp Lys Asp Ser Asp Leu Pro
130 135 140

Thr Trp Arg Arg Gly Thr Gly Tyr Thr Leu Ser Leu Pro Ala Val Ser
145 150 155 160

Pro Gly Lys Arg Leu Trp Gly Glu Lys Ala Gly Ser Leu Pro Glu Ser
165 170 175

Glu Pro Leu Phe Thr Tyr Thr Leu Asp Glu Lys Val Asp Lys Leu Val
180 185 190

Gln Phe Leu Leu Leu Lys Tyr Gln Ala Lys Glu Pro Leu Thr Arg Ala
195 200 205

Glu Met Gln Met Asn Val Ile Asn Thr Tyr Thr Gly Tyr Phe Pro Met
210 215 220

Ile Phe Arg Lys Ala Arg Glu Phe Ile Glu Ile Leu Phe Gly Ile Ser
225 230 235 240

Leu Thr Glu Val Asp Pro Asp His Phe Tyr Val Phe Val Asn Thr Leu
245 250 255

Asp Leu Thr Cys Glu Gly Ser Leu Ser Asp Glu Gln Gly Met Pro Gln
260 265 270

Asn Arg Leu Leu Ile Leu Ile Leu Ser Val Ile Phe Ile Lys Gly Asn
275 280 285

Cys Ala Ser Glu Glu Val Ile Trp Glu Val Leu Asn Ala Ile Gly Pro

290 295 300

Trp Ser Ala Leu Ala Gly Phe Ala Asp Val Leu Ser Arg Leu Ala Leu
305 310 315 320

Trp Glu Ser Glu Gly Pro Glu Ala Phe Cys Glu Glu Ser Gly Leu Arg
325 330 335

Ser Ala Glu Gly Ser Val Leu Asp Leu Ala Asn Pro Gln Gly Leu Ala
340 345 350

Gly His Arg Gln Glu Asp Gly Arg Arg Gly Leu Thr Glu Ala Ser Pro
355 360 365

Gln Gln Lys Lys Gly Gly Glu Asp Glu Asp Met Pro Ala Ala Gly Met
370 375 380

Pro Pro Leu Pro Gln Ser Pro Pro Glu Ile Pro Pro Gln Gly Pro Pro
385 390 395 400

Lys Ile Ser Pro Gln Gly Pro Pro Gln Ser Pro Pro Gln Ser Pro Leu
405 410 415

Asp Ser Cys Ser Ser Pro Leu Leu Trp Thr Arg Leu Asp Glu Glu Ser
420 425 430

Ser Ser Glu Glu Glu Asp Thr Ala Thr Trp His Ala Leu Pro Glu Ser
435 440 445

Glu Ser Leu Pro Arg Tyr Ala Leu Asp Glu Lys Val Ala Glu Leu Val
450 455 460

Gln Phe Leu Leu Leu Lys Tyr Gln Thr Lys Glu Pro Val Thr Lys Ala
465 470 475 480

Glu Met Leu Thr Thr Val Ile Lys Lys Tyr Lys Asp Tyr Phe Pro Met
485 490 495

Ile Phe Gly Lys Ala His Glu Phe Ile Glu Leu Ile Phe Gly Ile Ala
500 505 510

Leu Thr Asp Met Asp Pro Asp Asn His Ser Tyr Phe Phe Glu Asp Thr
515 520 525

Leu Asp Leu Thr Tyr Glu Gly Ser Leu Ile Asp Asp Gln Gly Met Pro
530 535 540

Lys Asn Cys Leu Leu Ile Leu Ile Leu Ser Met Ile Phe Ile Lys Gly
545 550 555 560

Ser Cys Val Pro Glu Glu Val Ile Trp Glu Val Leu Ser Ala Ile Gly

565 570 575

Pro Ile Gln Arg Pro Ala Arg Glu Val Leu Glu Phe Leu Ser Lys Leu
580 585 590

Ser Ser Ile Ile Pro Ser Ala Phe Pro Ser Trp Tyr Met Asp Ala Leu
595 600 605

Lys Asp Met Glu Asp Arg Ala Gln Ala Ile Ile Asp Thr Thr Asp Asp
610 615 620

Ala Thr Ala Met Ala Ser Ala Ser Pro Ser Val Met Ser Thr Asn Phe
625 630 635 640

Cys Pro Glu

<210> 170
<211> 902
<212> PRT
<213> Homo sapi ens

<400> 170

Met Gly Ala Ala Ser Cys Glu Asp Glu Glu Leu Glu Phe Lys Leu Val
1 5 10 15

Phe Gly Glu Glu Lys Glu Ala Pro Pro Leu Gly Ala Gly Gly Leu Gly
20 25 30

Glu Glu Leu Asp Ser Glu Asp Ala Pro Pro Cys Cys Arg Leu Ala Leu
35 40 45

Gly Glu Pro Pro Pro Tyr Gly Ala Ala Pro Ile Gly Ile Pro Arg Pro
50 55 60

Pro Pro Pro Arg Pro Gly Met His Ser Pro Pro Pro Arg Pro Ala Pro
65 70 75 80

Ser Pro Gly Thr Trp Glu Ser Gln Pro Ala Arg Ser Val Arg Leu Gly
85 90 95

Gly Pro Gly Gly Gly Ala Gly Gly Ala Gly Gly Gly Arg Val Leu Glu
100 105 110

Cys Pro Ser Ile Arg Ile Thr Ser Ile Ser Pro Thr Pro Glu Pro Pro
115 120 125

Ala Ala Leu Glu Asp Asn Pro Asp Ala Trp Gly Asp Gly Ser Pro Arg
130 135 140

Asp Tyr Pro Pro Pro Glu Gly Phe Gly Gly Tyr Arg Glu Ala Gly Ala
145 150 155 160

Gln Gly Gly Gly Ala Phe Phe Ser Pro Ser Pro Gly Ser Ser Ser Leu
165 170 175

Ser Ser Trp Ser Phe Phe Ser Asp Ala Ser Asp Glu Ala Ala Leu Tyr
180 185 190

Ala Ala Cys Asp Glu Val Glu Ser Glu Leu Asn Glu Ala Ala Ser Arg
195 200 205

Phe Gly Leu Gly Ser Pro Leu Pro Ser Pro Arg Ala Ser Pro Arg Pro
210 215 220

Trp Thr Pro Glu Asp Pro Trp Ser Leu Tyr Gly Pro Ser Pro Gly Gly
225 230 235 240

Arg Gly Pro Glu Asp Ser Trp Leu Leu Leu Ser Ala Pro Gly Pro Thr
245 250 255

Pro Ala Ser Pro Arg Pro Ala Ser Pro Cys Gly Lys Arg Arg Tyr Ser
260 265 270

Ser Ser Gly Thr Pro Ser Ser Ala Ser Pro Ala Leu Ser Arg Arg Gly
275 280 285

Ser Leu Gly Glu Glu Gly Ser Glu Pro Pro Pro Pro Pro Pro Leu Pro
290 295 300

Leu Ala Arg Asp Pro Gly Ser Pro Gly Pro Phe Asp Tyr Val Gly Ala
305 310 315 320

Pro Pro Ala Glu Ser Ile Pro Gln Lys Thr Arg Arg Thr Ser Ser Glu
325 330 335

Gln Ala Val Ala Leu Pro Arg Ser Glu Glu Pro Ala Ser Cys Asn Gly
340 345 350

Lys Leu Pro Leu Gly Ala Glu Glu Ser Val Ala Pro Pro Gly Gly Ser
355 360 365

Arg Lys Glu Val Ala Gly Met Asp Tyr Leu Ala Val Pro Ser Pro Leu
370 375 380

Ala Trp Ser Lys Ala Arg Ile Gly Gly His Ser Pro Ile Phe Arg Thr
385 390 395 400

Ser Ala Leu Pro Pro Leu Asp Trp Pro Leu Pro Ser Gln Tyr Glu Gln
405 410 415

Leu Glu Leu Arg Ile Glu Val Gln Pro Arg Ala His His Arg Ala His
420 425 430

Tyr Glu Thr Glu Gly Ser Arg Gly Ala Val Lys Ala Ala Pro Gly Gly
435 440 445

His Pro Val Val Lys Leu Leu Gly Tyr Ser Glu Lys Pro Leu Thr Leu
450 455 460

Gln Met Phe Ile Gly Thr Ala Asp Glu Arg Asn Leu Arg Pro His Ala
465 470 475 480

Phe Tyr Gln Val His Arg Ile Thr Gly Lys Met Val Ala Thr Ala Ser
485 490 495

Tyr Glu Ala Val Val Ser Gly Thr Lys Val Leu Glu Met Thr Leu Leu
500 505 510

Pro Glu Asn Asn Met Ala Ala Asn Ile Asp Cys Ala Gly Ile Leu Lys
515 520 525

Leu Arg Asn Ser Asp Ile Glu Leu Arg Lys Gly Glu Thr Asp Ile Gly
530 535 540

Arg Lys Asn Thr Arg Val Arg Leu Val Phe Arg Val His Val Pro Gln
545 550 555 560

Gly Gly Gly Lys Val Val Ser Val Gln Ala Ala Ser Val Pro Ile Glu
565 570 575

Cys Ser Gln Arg Ser Ala Gln Glu Leu Pro Gln Val Glu Ala Tyr Ser
580 585 590

Pro Ser Ala Cys Ser Val Arg Gly Gly Glu Glu Leu Val Leu Thr Gly
595 600 605

Ser Asn Phe Leu Pro Asp Ser Lys Val Val Phe Ile Glu Arg Gly Pro
610 615 620

Asp Gly Lys Leu Gln Trp Glu Glu Glu Ala Thr Val Asn Arg Leu Gln
625 630 635 640

Ser Asn Glu Val Thr Leu Thr Leu Thr Val Pro Glu Tyr Ser Asn Lys
645 650 655

Arg Val Ser Arg Pro Val Gln Val Tyr Phe Tyr Val Ser Asn Gly Arg
660 665 670

Arg Lys Arg Ser Pro Thr Gln Ser Phe Arg Phe Leu Pro Val Ile Cys
675 680 685

Lys Glu Glu Pro Leu Pro Asp Ser Ser Leu Arg Gly Phe Pro Ser Ala
690 695 700

Ser Ala Thr Pro Phe Gly Thr Asp Met Asp Phe Ser Pro Pro Arg Pro
705 710 715 720

Pro Tyr Pro Ser Tyr Pro His Glu Asp Pro Ala Cys Glu Thr Pro Tyr
725 730 735

Leu Ser Glu Gly Phe Gly Tyr Gly Met Pro Pro Leu Tyr Pro Gln Thr
740 745 750

Gly Pro Pro Pro Ser Tyr Arg Pro Gly Leu Arg Met Phe Pro Glu Thr
755 760 765

Arg Gly Thr Thr Gly Cys Ala Gln Pro Pro Ala Val Ser Phe Leu Pro
770 775 780

Arg Pro Phe Pro Ser Asp Pro Tyr Gly Gly Arg Gly Ser Ser Phe Pro
785 790 795 800

Leu Gly Leu Pro Phe Ser Pro Pro Ala Pro Phe Arg Pro Pro Pro Leu
805 810 815

Pro Ala Ser Pro Pro Leu Glu Gly Pro Phe Pro Ser Gln Ser Asp Val
820 825 830

His Pro Leu Pro Ala Glu Gly Tyr Asn Lys Val Gly Pro Gly Tyr Gly
835 840 845

Pro Gly Glu Gly Ala Pro Glu Gln Glu Lys Ser Arg Gly Gly Tyr Ser
850 855 860

Ser Gly Phe Arg Asp Ser Val Pro Ile Gln Gly Ile Thr Leu Glu Glu
865 870 875 880

Val Ser Glu Ile Ile Gly Arg Asp Leu Ser Gly Phe Pro Ala Pro Pro
885 890 895

Gly Glu Glu Pro Pro Ala
900

<210> 171
<211> 478
<212> PRT
<213> Homo sapiens

<400> 171

Met Ser Pro Pro Leu Leu Lys Leu Gly Ala Val Leu Ser Thr Met Ala
1 5 10 15

Met Ile Ser Asn Trp Met Ser Gln Thr Leu Pro Ser Leu Val Gly Leu
20 25 30

Asn Thr Thr Arg Leu Ser Thr Pro Asp Thr Leu Thr Gln Ile Ser Pro
35 40 45

Lys Glu Gly Trp Gln Val Tyr Ser Ser Ala Gln Asp Pro Asp Gly Arg
50 55 60

Cys Ile Cys Thr Val Val Ala Pro Glu Gln Asn Leu Cys Ser Arg Asp
65 70 75 80

Ala Lys Ser Arg Gln Leu Arg Gln Leu Leu Glu Lys Val Gln Asn Met
85 90 95

Ser Gln Ser Ile Glu Val Leu Asn Leu Arg Thr Gln Arg Asp Phe Gln
100 105 110

Tyr Val Leu Lys Met Glu Thr Gln Met Lys Gly Leu Lys Ala Lys Phe
115 120 125

Arg Gln Ile Glu Asp Asp Arg Lys Thr Leu Met Thr Lys His Phe Gln
130 135 140

Glu Leu Lys Glu Lys Met Asp Glu Leu Leu Pro Leu Ile Pro Val Leu
145 150 155 160

Glu Gln Tyr Lys Thr Asp Ala Lys Leu Ile Thr Gln Phe Lys Glu Glu
165 170 175

Ile Arg Asn Leu Ser Ala Val Leu Thr Gly Ile Gln Glu Glu Ile Gly
180 185 190

Ala Tyr Asp Tyr Glu Glu Leu His Gln Arg Val Leu Ser Leu Glu Thr
195 200 205

Arg Leu Arg Asp Cys Met Lys Lys Leu Thr Cys Gly Lys Leu Met Lys
210 215 220

Ile Thr Gly Pro Val Thr Val Lys Thr Ser Gly Thr Arg Phe Gly Ala
225 230 235 240

Trp Met Thr Asp Pro Leu Ala Ser Glu Lys Asn Asn Arg Val Trp Tyr
245 250 255

Met Asp Ser Tyr Thr Asn Asn Lys Ile Val Arg Glu Tyr Lys Ser Ile
260 265 270

Ala Asp Phe Val Ser Gly Ala Glu Ser Arg Thr Tyr Asn Leu Pro Phe
275 280 285

Lys Trp Ala Gly Thr Asn His Val Val Tyr Asn Gly Ser Leu Tyr Phe
290 295 300

Asn Lys Tyr Gln Ser Asn Ile Ile Ile Lys Tyr Ser Phe Asp Met Gly
305 310 315 320

Arg Val Leu Ala Gln Arg Ser Leu Glu Tyr Ala Gly Phe His Asn Val
325 330 335

Tyr Pro Tyr Thr Trp Gly Gly Phe Ser Asp Ile Asp Leu Met Ala Asp
340 345 350

Glu Ile Gly Leu Trp Ala Val Tyr Ala Thr Asn Gln Asn Ala Gly Asn
355 360 365

Ile Val Ile Ser Gln Leu Asn Gln Asp Thr Leu Glu Val Met Lys Ser
370 375 380

Trp Ser Thr Gly Tyr Pro Lys Arg Ser Ala Gly Glu Ser Phe Met Ile
385 390 395 400

Cys Gly Thr Leu Tyr Val Thr Asn Ser His Leu Thr Gly Ala Lys Val
405 410 415

Tyr Tyr Ser Tyr Ser Thr Lys Thr Ser Thr Tyr Glu Tyr Thr Asp Ile
420 425 430

Pro Phe His Asn Gln Tyr Phe His Ile Ser Met Leu Asp Tyr Asn Ala
435 440 445

Arg Asp Arg Ala Leu Tyr Ala Trp Asn Asn Gly His Gln Val Leu Phe
450 455 460

Asn Val Thr Leu Phe His Ile Ile Lys Thr Glu Asp Asp Thr
465 470 475

<210> 172
<211> 316
<212> PRT
<213> Homo sapiens

<400> 172

Met Ala Glu Val Lys Val Lys Val Gln Pro Pro Asp Ala Asp Pro Val
1 5 10 15

Glu Ile Glu Asn Arg Ile Ile Glu Leu Cys His Gln Phe Pro His Gly
20 25 30

Ile Thr Asp Gln Val Ile Gln Asn Glu Met Pro His Ile Glu Ala Gln
35 40 45

Gln Arg Ala Val Ala Ile Asn Arg Leu Leu Ser Met Gly Gln Leu Asp
50 55 60

Leu Leu Arg Ser Asn Thr Gly Leu Leu Tyr Arg Ile Lys Asp Ser Gln
65 70 75 80

Asn Ala Gly Lys Met Lys Gly Ser Asp Asn Gln Glu Lys Leu Val Tyr
85 90 95

Gln Ile Ile Glu Asp Ala Gly Asn Lys Gly Ile Trp Ser Arg Asp Ile
100 105 110

Arg Tyr Lys Ser Asn Leu Pro Leu Thr Glu Ile Asn Lys Ile Leu Lys
115 120 125

Asn Leu Glu Ser Lys Lys Leu Ile Lys Ala Val Lys Ser Val Ala Ala
130 135 140

Ser Lys Lys Lys Val Tyr Met Leu Tyr Asn Leu Gln Pro Asp Arg Ser
145 150 155 160

Val Thr Gly Gly Ala Trp Tyr Ser Asp Gln Asp Phe Glu Ser Glu Phe
165 170 175

Val Glu Val Leu Asn Gln Gln Cys Phe Lys Phe Leu Gln Ser Lys Ala
180 185 190

Glu Thr Ala Arg Glu Ser Lys Gln Asn Pro Met Ile Gln Arg Asn Ser
195 200 205

Ser Phe Ala Ser Ser His Glu Val Trp Lys Tyr Ile Cys Glu Leu Gly
210 215 220

Ile Ser Lys Val Glu Leu Ser Met Glu Asp Ile Glu Thr Ile Leu Asn
225 230 235 240

Thr Leu Ile Tyr Asp Gly Lys Val Glu Met Thr Ile Ile Ala Ala Lys
245 250 255

Glu Gly Thr Val Gly Ser Val Asp Gly His Met Lys Leu Tyr Arg Ala
260 265 270

Val Asn Pro Ile Ile Pro Pro Thr Gly Leu Val Arg Ala Pro Cys Gly
275 280 285

Leu Cys Pro Val Phe Asp Asp Cys His Glu Gly Gly Glu Ile Ser Pro
290 295 300

Ser Asn Cys Ile Tyr Met Thr Glu Trp Leu Glu Phe
305 310 315

<210> 173
<211> 524
<212> PRT
<213> Homo sapiens

<400> 173

Met Ala Ala Pro Glu Pro Ala Arg Ala Ala Pro Pro Pro Pro Pro Pro
1 5 10 15

Pro Pro Pro Pro Pro Gly Ala Asp Arg Val Val Lys Ala Val Pro Phe
20 25 30

Pro Pro Thr His Arg Leu Thr Ser Glu Glu Val Phe Asp Leu Asp Gly
35 40 45

Ile Pro Arg Val Asp Val Leu Lys Asn His Leu Val Lys Glu Gly Arg
50 55 60

Val Asp Glu Glu Ile Ala Leu Arg Ile Ile Asn Glu Gly Ala Ala Ile
65 70 75 80

Leu Arg Arg Glu Lys Thr Met Ile Glu Val Glu Ala Pro Ile Thr Val
85 90 95

Cys Gly Asp Ile His Gly Gln Phe Phe Asp Leu Met Lys Leu Phe Glu
100 105 110

Val Gly Gly Ser Pro Ala Asn Thr Arg Tyr Leu Phe Leu Gly Asp Tyr
115 120 125

Val Asp Arg Gly Tyr Phe Ser Ile Glu Cys Val Leu Tyr Leu Trp Val
130 135 140

Leu Lys Ile Leu Tyr Pro Ser Thr Leu Phe Leu Leu Arg Gly Asn His
145 150 155 160

Glu Cys Arg His Leu Thr Glu Tyr Phe Thr Phe Lys Gln Glu Cys Lys
165 170 175

Ile Lys Tyr Ser Glu Arg Val Tyr Glu Ala Cys Met Glu Ala Phe Asp
180 185 190

Ser Leu Pro Leu Ala Ala Leu Leu Asn Gln Gln Phe Leu Cys Val His
195 200 205

Gly Gly Leu Ser Pro Glu Ile His Thr Leu Asp Asp Ile Arg Arg Leu
210 215 220

Asp Arg Phe Lys Glu Pro Pro Ala Phe Gly Pro Met Cys Asp Leu Leu
225 230 235 240

Trp Ser Asp Pro Ser Glu Asp Phe Gly Asn Glu Lys Ser Gln Glu His
245 250 255

Phe Ser His Asn Thr Val Arg Gly Cys Ser Tyr Phe Tyr Asn Tyr Pro

260 265 270

Ala Val Cys Glu Phe Leu Gln Asn Asn Asn Leu Leu Ser Ile Ile Arg
275 280 285

Ala His Glu Ala Gln Asp Ala Gly Tyr Arg Met Tyr Arg Lys Ser Gln
290 295 300

Thr Thr Gly Phe Pro Ser Leu Ile Thr Ile Phe Ser Ala Pro Asn Tyr
305 310 315 320

Leu Asp Val Tyr Asn Asn Lys Ala Ala Val Leu Lys Tyr Glu Asn Asn
325 330 335

Val Met Asn Ile Arg Gln Phe Asn Cys Ser Pro His Pro Tyr Trp Leu
340 345 350

Pro Asn Phe Met Asp Val Phe Thr Trp Ser Leu Pro Phe Val Gly Glu
355 360 365

Lys Val Thr Glu Met Leu Val Asn Val Leu Ser Ile Cys Ser Asp Asp
370 375 380

Glu Leu Met Thr Glu Gly Glu Asp Gln Phe Asp Gly Ser Ala Ala Ala
385 390 395 400

Arg Lys Glu Ile Ile Arg Asn Lys Ile Arg Ala Ile Gly Lys Met Ala
405 410 415

Arg Val Phe Ser Val Leu Arg Glu Glu Ser Glu Ser Val Leu Thr Leu
420 425 430

Lys Gly Leu Thr Pro Thr Gly Met Leu Pro Ser Gly Val Leu Ala Gly
435 440 445

Gly Arg Gln Thr Leu Gln Ser Ala Thr Val Glu Ala Ile Glu Ala Glu
450 455 460

Lys Ala Ile Arg Gly Phe Ser Pro Pro His Arg Ile Cys Ser Phe Glu
465 470 475 480

Glu Ala Lys Gly Leu Asp Arg Ile Asn Glu Arg Met Pro Pro Arg Lys
485 490 495

Asp Ala Val Gln Gln Asp Gly Phe Asn Ser Leu Asn Thr Ala His Ala
500 505 510

Thr Glu Asn His Gly Thr Gly Asn His Thr Ala Gln
515 520

<210> 174
<211> 639

<212> PRT
<213> Homo sapiens

<400> 174

Met Ala Thr Gly Ala Asn Ala Thr Pro Leu Asp Phe Pro Ser Lys Lys
1 5 10 15

Arg Lys Arg Ser Arg Trp Asn Gln Asp Thr Met Glu Gln Lys Thr Val
20 25 30

Ile Pro Gly Met Pro Thr Val Ile Pro Pro Gly Leu Thr Arg Glu Gln
35 40 45

Glu Arg Ala Tyr Ile Val Gln Leu Gln Ile Glu Asp Leu Thr Arg Lys
50 55 60

Leu Arg Thr Gly Asp Leu Gly Ile Pro Pro Asn Pro Glu Asp Arg Ser
65 70 75 80

Pro Ser Pro Glu Pro Ile Tyr Asn Ser Glu Gly Lys Arg Leu Asn Thr
85 90 95

Arg Glu Phe Arg Thr Arg Lys Lys Leu Glu Glu Glu Arg His Asn Leu
100 105 110

Ile Thr Glu Met Val Ala Leu Asn Pro Asp Phe Lys Pro Pro Ala Asp
115 120 125

Tyr Lys Pro Pro Ala Thr Arg Val Ser Asp Lys Val Met Ile Pro Gln
130 135 140

Asp Glu Tyr Pro Glu Ile Asn Phe Val Gly Leu Leu Ile Gly Pro Arg
145 150 155 160

Gly Asn Thr Leu Lys Asn Ile Glu Lys Glu Cys Asn Ala Lys Ile Met
165 170 175

Ile Arg Gly Lys Gly Ser Val Lys Glu Gly Lys Val Gly Arg Lys Asp
180 185 190

Gly Gln Met Leu Pro Gly Glu Asp Glu Pro Leu His Ala Leu Val Thr
195 200 205

Ala Asn Thr Met Glu Asn Val Lys Lys Ala Val Glu Gln Ile Arg Asn
210 215 220

Ile Leu Lys Gln Gly Ile Glu Thr Pro Glu Asp Gln Asn Asp Leu Arg
225 230 235 240

Lys Met Gln Leu Arg Glu Leu Ala Arg Leu Asn Gly Thr Leu Arg Glu
245 250 255

Asp Asp Asn Arg Ile Leu Arg Pro Trp Gln Ser Ser Glu Thr Arg Ser
260 265 270

Ile Thr Asn Thr Thr Val Cys Thr Lys Cys Gly Gly Ala Gly His Ile
275 280 285

Ala Ser Asp Cys Lys Phe Gln Arg Pro Gly Asp Pro Gln Ser Ala Gln
290 295 300

Asp Lys Ala Arg Met Asp Lys Glu Tyr Leu Ser Leu Met Ala Glu Leu
305 310 315 320

Gly Glu Ala Pro Val Pro Ala Ser Val Gly Ser Thr Ser Gly Pro Ala
325 330 335

Thr Thr Pro Leu Ala Ser Ala Pro Arg Pro Ala Ala Pro Ala Asn Asn
340 345 350

Pro Pro Pro Pro Ser Leu Met Ser Thr Thr Gln Ser Arg Pro Pro Trp
355 360 365

Met Asn Ser Gly Pro Ser Glu Ser Arg Pro Tyr His Gly Met His Gly
370 375 380

Gly Gly Pro Gly Gly Pro Gly Gly Gly Pro His Ser Phe Pro His Pro
385 390 395 400

Leu Pro Ser Leu Thr Gly Gly His Gly Gly His Pro Met Gln His Asn
405 410 415

Pro Asn Gly Pro Pro Pro Pro Trp Met Gln Pro Pro Pro Pro Pro Met
420 425 430

Asn Gln Gly Pro His Pro Pro Gly His His Gly Pro Pro Pro Met Asp
435 440 445

Gln Tyr Leu Gly Ser Thr Pro Val Gly Ser Gly Val Tyr Arg Leu His
450 455 460

Gln Gly Lys Gly Met Met Pro Pro Pro Pro Met Gly Met Met Pro Pro
465 470 475 480

Pro Pro Pro Pro Pro Ser Gly Gln Pro Pro Pro Pro Pro Ser Gly Pro
485 490 495

Leu Pro Pro Trp Gln Gln Gln Gln Gln Gln Pro Pro Pro Pro Pro Pro
500 505 510

Pro Ser Ser Ser Met Ala Ser Ser Thr Pro Leu Pro Trp Gln Gln Asn
515 520 525

Thr Thr Thr Thr Thr Thr Ser Ala Gly Thr Gly Ser Ile Pro Pro Trp
530 535 540

Gln Gln Gln Gln Ala Ala Ala Ala Ala Ser Pro Gly Ala Pro Gln Met
545 550 555 560

Gln Gly Asn Pro Thr Met Val Pro Leu Pro Pro Gly Val Gln Pro Pro
565 570 575

Leu Pro Pro Gly Ala Pro Pro Pro Pro Pro Pro Pro Pro Pro Gly Ser
580 585 590

Ala Gly Met Met Tyr Ala Pro Pro Pro Pro Pro Pro Pro Pro Met Asp
595 600 605

Pro Ser Asn Phe Val Thr Met Met Gly Met Gly Val Ala Gly Met Pro
610 615 620

Pro Phe Gly Met Pro Pro Ala Pro Pro Pro Pro Pro Pro Gln Asn
625 630 635

<210> 175
<211> 661
<212> PRT
<213> Homo sapiens

<400> 175

Met Asp Leu Gln Gln Ser Thr Thr Ile Thr Ser Leu Glu Lys Trp Cys
1 5 10 15

Leu Asp Glu Ser Leu Ser Gly Cys Arg Arg His Tyr Ser Val Lys Lys
20 25 30

Lys Leu Lys Leu Ile Arg Val Leu Gly Leu Phe Met Gly Leu Val Ala
35 40 45

Ile Ser Thr Val Ser Phe Ser Ile Ser Ala Phe Ser Glu Thr Asp Thr
50 55 60

Gln Ser Thr Gly Glu Ala Ser Val Val Ser Gly Pro Arg Val Ala Gln
65 70 75 80

Gly Tyr His Gln Arg Thr Leu Leu Asp Leu Asn Asp Lys Ile Leu Asp
85 90 95

Tyr Thr Pro Gln Pro Pro Leu Ser Lys Glu Gly Glu Ser Glu Asn Ser
100 105 110

Thr Asp His Ala Gln Gly Asp Tyr Pro Lys Asp Ile Phe Ser Leu Glu
115 120 125

Glu Arg Arg Lys Gly Ala Ile Ile Leu His Val Ile Gly Met Ile Tyr
130 135 140

Met Phe Ile Ala Leu Ala Ile Val Cys Asp Glu Phe Phe Val Pro Ser
145 150 155 160

Leu Thr Val Ile Thr Glu Lys Leu Gly Ile Ser Asp Asp Val Ala Gly
165 170 175

Ala Thr Phe Met Ala Ala Gly Gly Ser Ala Pro Glu Leu Phe Thr Ser
180 185 190

Leu Ile Gly Val Phe Ile Ala His Ser Asn Val Gly Ile Gly Thr Ile
195 200 205

Val Gly Ser Ala Val Phe Asn Ile Leu Phe Val Ile Gly Met Cys Ala
210 215 220

Leu Phe Ser Arg Glu Ile Leu Asn Leu Thr Trp Trp Pro Leu Phe Arg
225 230 235 240

Asp Val Ser Phe Tyr Ile Val Asp Leu Ile Met Leu Ile Ile Phe Phe
245 250 255

Leu Asp Asn Val Ile Met Trp Trp Glu Ser Leu Leu Leu Leu Thr Ala
260 265 270

Tyr Phe Cys Tyr Val Val Phe Met Lys Phe Asn Val Gln Val Glu Lys
275 280 285

Trp Val Lys Gln Met Ile Asn Arg Asn Lys Val Val Lys Val Thr Ala
290 295 300

Pro Glu Ala Gln Ala Lys Pro Ser Ala Ala Arg Asp Lys Asp Glu Pro
305 310 315 320

Thr Leu Pro Ala Lys Pro Arg Leu Gln Arg Gly Gly Ser Ser Ala Ser
325 330 335

Leu His Asn Ser Leu Met Arg Asn Ser Ile Phe Gln Leu Met Ile His
340 345 350

Thr Leu Asp Pro Leu Ala Glu Glu Leu Gly Ser Tyr Gly Lys Leu Lys
355 360 365

Tyr Tyr Asp Thr Met Thr Glu Glu Gly Arg Phe Arg Glu Lys Ala Ser
370 375 380

Ile Leu His Lys Ile Ala Lys Lys Lys Cys His Val Asp Glu Asn Glu
385 390 395 400

Arg Gln Asn Gly Ala Ala Asn His Val Glu Lys Ile Glu Leu Pro Asn
405 410 415

Ser Thr Ser Thr Asp Val Glu Met Thr Pro Ser Ser Asp Ala Ser Glu
420 425 430

Pro Val Gln Asn Gly Asn Leu Ser His Asn Ile Glu Gly Ala Glu Ala
435 440 445

Gln Thr Ala Asp Glu Glu Glu Asp Gln Pro Leu Ser Leu Ala Trp Pro
450 455 460

Ser Glu Thr Arg Lys Gln Val Thr Phe Leu Ile Val Phe Pro Ile Val
465 470 475 480

Phe Pro Leu Trp Ile Thr Leu Pro Asp Val Arg Lys Pro Ser Ser Arg
485 490 495

Lys Phe Phe Pro Ile Thr Phe Phe Gly Ser Ile Thr Trp Ile Ala Val
500 505 510

Phe Ser Tyr Leu Met Val Trp Trp Ala His Gln Val Gly Glu Thr Ile
515 520 525

Gly Ile Ser Glu Glu Ile Met Gly Leu Thr Ile Leu Ala Ala Gly Thr
530 535 540

Ser Ile Pro Asp Leu Ile Thr Ser Val Ile Val Ala Arg Lys Gly Leu
545 550 555 560

Gly Asp Met Ala Val Ser Ser Ser Val Gly Ser Asn Ile Phe Asp Ile
565 570 575

Thr Val Gly Leu Pro Leu Pro Trp Leu Leu Tyr Thr Val Ile His Arg
580 585 590

Phe Gln Pro Val Ala Val Ser Ser Asn Gly Leu Phe Cys Ala Ile Val
595 600 605

Leu Leu Phe Ile Met Leu Leu Phe Val Ile Leu Ser Ile Ala Leu Cys
610 615 620

Lys Trp Arg Met Asn Lys Ile Leu Gly Phe Ile Met Phe Gly Leu Tyr
625 630 635 640

Phe Val Phe Leu Val Val Ser Val Leu Leu Glu Asp Arg Ile Leu Thr
645 650 655

Cys Pro Val Ser Ile
660

<210> 176

<211> 780
<212> PRT
<213> Homo sapiens

<400> 176

Met Ala Ala Pro Gly Gly Arg Ser Glu Pro Pro Gln Leu Pro Glu Tyr
1 5 10 15

Ser Cys Ser Tyr Met Val Ser Arg Pro Val Tyr Ser Glu Leu Ala Phe
20 25 30

Gln Gln Gln His Glu Arg Arg Leu Gln Glu Arg Lys Thr Leu Arg Glu
35 40 45

Ser Leu Ala Lys Cys Cys Ser Cys Ser Arg Lys Arg Ala Phe Gly Val
50 55 60

Leu Lys Thr Leu Val Pro Ile Leu Glu Trp Leu Pro Lys Tyr Arg Val
65 70 75 80

Lys Glu Trp Leu Leu Ser Asp Val Ile Ser Gly Val Ser Thr Gly Leu
85 90 95

Val Ala Thr Leu Gln Gly Met Ala Tyr Ala Leu Leu Ala Ala Val Pro
100 105 110

Val Gly Tyr Gly Leu Tyr Ser Ala Phe Phe Pro Ile Leu Thr Tyr Phe
115 120 125

Ile Phe Gly Thr Ser Arg His Ile Ser Val Gly Pro Phe Pro Val Val
130 135 140

Ser Leu Met Val Gly Ser Val Val Leu Ser Met Ala Pro Asp Glu His
145 150 155 160

Phe Leu Val Ser Ser Ser Asn Gly Thr Val Leu Asn Thr Thr Met Ile
165 170 175

Asp Thr Ala Ala Arg Asp Thr Ala Arg Val Leu Ile Ala Ser Ala Leu
180 185 190

Thr Leu Leu Val Gly Ile Ile Gln Leu Ile Phe Gly Gly Leu Gln Ile
195 200 205

Gly Phe Ile Val Arg Tyr Leu Ala Asp Pro Leu Val Gly Gly Phe Thr
210 215 220

Thr Ala Ala Ala Phe Gln Val Leu Val Ser Gln Leu Lys Ile Val Leu
225 230 235 240

Asn Val Ser Thr Lys Asn Tyr Asn Gly Val Leu Ser Ile Ile Tyr Thr
245 250 255

Leu Val Glu Ile Phe Gln Asn Ile Gly Asp Thr Asn Leu Ala Asp Phe
260 265 270

Thr Ala Gly Leu Leu Thr Ile Val Val Cys Met Ala Val Lys Glu Leu
275 280 285

Asn Asp Arg Phe Arg His Lys Ile Pro Val Pro Ile Pro Ile Glu Val
290 295 300

Ile Val Thr Ile Ile Ala Thr Ala Ile Ser Tyr Gly Ala Asn Leu Glu
305 310 315 320

Lys Asn Tyr Asn Ala Gly Ile Val Lys Ser Ile Pro Arg Gly Phe Leu
325 330 335

Pro Pro Glu Leu Pro Pro Val Ser Leu Phe Ser Glu Met Leu Ala Ala
340 345 350

Ser Phe Ser Ile Ala Val Val Ala Tyr Ala Ile Ala Val Ser Val Gly
355 360 365

Lys Val Tyr Ala Thr Lys Tyr Asp Tyr Thr Ile Asp Gly Asn Gln Glu
370 375 380

Phe Ile Ala Phe Gly Ile Ser Asn Ile Phe Ser Gly Phe Phe Ser Cys
385 390 395 400

Phe Val Ala Thr Thr Ala Leu Ser Arg Thr Ala Val Gln Glu Ser Thr
405 410 415

Gly Gly Lys Thr Gln Val Ala Gly Ile Ile Ser Ala Ala Ile Val Met
420 425 430

Ile Ala Ile Leu Ala Leu Gly Lys Leu Leu Glu Pro Leu Gln Lys Ser
435 440 445

Val Leu Ala Ala Val Val Ile Ala Asn Leu Lys Gly Met Phe Met Gln
450 455 460

Leu Cys Asp Ile Pro Arg Leu Trp Arg Gln Asn Lys Ile Asp Ala Val
465 470 475 480

Ile Trp Val Phe Thr Cys Ile Val Ser Ile Ile Leu Gly Leu Asp Leu
485 490 495

Gly Leu Leu Ala Gly Leu Ile Phe Gly Leu Leu Thr Val Val Leu Arg
500 505 510

Val Gln Phe Pro Ser Trp Asn Gly Leu Gly Ser Ile Pro Ser Thr Asp
515 520 525

Ile Tyr Lys Ser Thr Lys Asn Tyr Lys Asn Ile Glu Glu Pro Gln Gly
530 535 540

Val Lys Ile Leu Arg Phe Ser Ser Pro Ile Phe Tyr Gly Asn Val Asp
545 550 555 560

Gly Phe Lys Lys Cys Ile Lys Ser Thr Val Gly Phe Asp Ala Ile Arg
565 570 575

Val Tyr Asn Lys Arg Leu Lys Ala Leu Arg Lys Ile Gln Lys Leu Ile
580 585 590

Lys Ser Gly Gln Leu Arg Ala Thr Lys Asn Gly Ile Ile Ser Asp Ala
595 600 605

Val Ser Thr Asn Asn Ala Phe Glu Pro Asp Glu Asp Ile Glu Asp Leu
610 615 620

Glu Glu Leu Asp Ile Pro Thr Lys Glu Ile Glu Ile Gln Val Asp Trp
625 630 635 640

Asn Ser Glu Leu Pro Val Lys Val Asn Val Pro Lys Val Pro Ile His
645 650 655

Ser Leu Val Leu Asp Cys Gly Ala Ile Ser Phe Leu Asp Val Val Gly
660 665 670

Val Arg Ser Leu Arg Val Ile Val Lys Glu Phe Gln Arg Ile Asp Val
675 680 685

Asn Val Tyr Phe Ala Ser Leu Gln Asp Tyr Val Ile Glu Lys Leu Glu
690 695 700

Gln Cys Gly Phe Phe Asp Asp Asn Ile Arg Lys Asp Thr Phe Phe Leu
705 710 715 720

Thr Val His Asp Ala Ile Leu Tyr Leu Gln Asn Gln Val Lys Ser Gln
725 730 735

Glu Gly Gln Gly Ser Ile Leu Glu Thr Ile Thr Leu Ile Gln Asp Cys
740 745 750

Lys Asp Thr Leu Glu Leu Ile Glu Thr Glu Leu Thr Glu Glu Glu Leu
755 760 765

Asp Val Gln Asp Glu Ala Met Arg Thr Leu Ala Ser
770 775 780

<210> 177

<211> 670
<212> PRT
<213> Homo sapi ens

<400> 177

Met Gly Glu Thr Glu Lys Arg Ile Glu Thr His Arg Ile Arg Cys Leu
1 5 10 15

Ser Lys Leu Lys Met Phe Leu Leu Ala Ile Thr Cys Ala Phe Val Ser
20 25 30

Lys Thr Leu Ser Gly Ser Tyr Met Asn Ser Met Leu Thr Gln Ile Glu
35 40 45

Arg Gln Phe Asn Ile Pro Thr Ser Leu Val Gly Phe Ile Asn Gly Ser
50 55 60

Phe Glu Ile Gly Asn Leu Leu Leu Ile Ile Phe Val Ser Tyr Phe Gly
65 70 75 80

Thr Lys Leu His Arg Pro Ile Met Ile Gly Ile Gly Cys Val Val Met
85 90 95

Gly Leu Gly Cys Phe Leu Lys Ser Leu Pro His Phe Leu Met Asn Gln
100 105 110

Tyr Glu Tyr Glu Ser Thr Val Ser Val Ser Gly Asn Leu Ser Ser Asn
115 120 125

Ser Phe Leu Cys Met Glu Asn Gly Thr Gln Ile Leu Arg Pro Thr Gln
130 135 140

Asp Pro Ser Glu Cys Thr Lys Glu Val Lys Ser Leu Met Trp Val Tyr
145 150 155 160

Val Leu Val Gly Asn Ile Val Arg Gly Met Gly Glu Thr Pro Ile Leu
165 170 175

Pro Leu Gly Ile Ser Tyr Ile Glu Asp Phe Ala Lys Phe Glu Asn Ser
180 185 190

Pro Leu Tyr Ile Gly Leu Val Glu Thr Gly Ala Ile Ile Gly Pro Leu
195 200 205

Ile Gly Leu Leu Leu Ala Ser Phe Cys Ala Asn Val Tyr Val Asp Thr
210 215 220

Gly Phe Val Asn Thr Asp Asp Leu Ile Ile Thr Pro Thr Asp Thr Arg
225 230 235 240

Trp Val Gly Ala Trp Trp Phe Gly Phe Leu Ile Cys Ala Gly Val Asn

245 250 255

Val Leu Thr Ala Ile Pro Phe Phe Phe Leu Pro Asn Thr Leu Pro Lys
260 265 270

Glu Gly Leu Glu Thr Asn Ala Asp Ile Ile Lys Asn Glu Asn Glu Asp
275 280 285

Lys Gln Lys Glu Glu Val Lys Lys Glu Lys Tyr Gly Ile Thr Lys Asp
290 295 300

Phe Leu Pro Phe Met Lys Ser Leu Ser Cys Asn Pro Ile Tyr Met Leu
305 310 315 320

Phe Ile Leu Val Ser Val Ile Gln Phe Asn Ala Phe Val Asn Met Ile
325 330 335

Ser Phe Met Pro Lys Tyr Leu Glu Gln Gln Tyr Gly Ile Ser Ser Ser
340 345 350

Asp Ala Ile Phe Leu Met Gly Ile Tyr Asn Leu Pro Pro Ile Cys Ile
355 360 365

Gly Tyr Ile Ile Gly Gly Leu Ile Met Lys Lys Phe Lys Ile Thr Val
370 375 380

Lys Gln Ala Ala His Ile Gly Cys Trp Leu Ser Leu Leu Glu Tyr Leu
385 390 395 400

Leu Tyr Phe Leu Ser Phe Leu Met Thr Cys Glu Asn Ser Ser Val Val
405 410 415

Gly Ile Asn Thr Ser Tyr Glu Gly Ile Pro Gln Asp Leu Tyr Val Glu
420 425 430

Asn Asp Ile Phe Ala Asp Cys Asn Val Asp Cys Asn Cys Pro Ser Lys
435 440 445

Ile Trp Asp Pro Val Cys Gly Asn Asn Gly Leu Ser Tyr Leu Ser Ala
450 455 460

Cys Leu Ala Gly Cys Glu Thr Ser Ile Gly Thr Gly Ile Asn Met Val
465 470 475 480

Phe Gln Asn Cys Ser Cys Ile Gln Thr Ser Gly Asn Ser Ser Ala Val
485 490 495

Leu Gly Leu Cys Asp Lys Gly Pro Asp Cys Ser Leu Met Leu Gln Tyr
500 505 510

Phe Leu Ile Leu Ser Ala Met Ser Ser Phe Ile Tyr Ser Leu Ala Ala

515 520 525

Ile Pro Gly Tyr Met Val Leu Leu Arg Cys Met Lys Ser Glu Glu Lys
530 535 540

Ser Leu Gly Val Gly Leu His Thr Phe Cys Thr Arg Val Phe Ala Gly
545 550 555 560

Ile Pro Ala Pro Ile Tyr Phe Gly Ala Leu Met Asp Ser Thr Cys Leu
565 570 575

His Trp Gly Thr Leu Lys Cys Gly Glu Ser Gly Ala Cys Arg Ile Tyr
580 585 590

Asp Ser Thr Thr Phe Arg Tyr Ile Tyr Leu Gly Leu Pro Ala Ala Leu
595 600 605

Arg Gly Ser Ser Phe Val Pro Ala Leu Ile Ile Leu Ile Leu Leu Arg
610 615 620

Lys Cys His Leu Pro Gly Glu Asn Ala Ser Ser Gly Thr Glu Leu Ile
625 630 635 640

Glu Thr Lys Val Lys Gly Lys Glu Asn Glu Cys Lys Asp Ile Tyr Gln
645 650 655

Lys Ser Thr Val Leu Lys Asp Asp Glu Leu Lys Thr Lys Leu
660 665 670

<210> 178
<211> 284
<212> PRT
<213> Homo sapiens

<400> 178

Met Asp Ala Ile Lys Lys Lys Met Gln Met Leu Lys Leu Asp Lys Glu
1 5 10 15

Asn Ala Ile Asp Arg Ala Glu Gln Ala Glu Ala Asp Lys Lys Gln Ala
20 25 30

Glu Asp Arg Cys Lys Gln Leu Glu Glu Glu Gln Gln Ala Leu Gln Lys
35 40 45

Lys Leu Lys Gly Thr Glu Asp Glu Val Glu Lys Tyr Ser Glu Ser Val
50 55 60

Lys Glu Ala Gln Glu Lys Leu Glu Gln Ala Glu Lys Lys Ala Thr Asp
65 70 75 80

Ala Glu Ala Asp Val Ala Ser Leu Asn Arg Arg Ile Gln Leu Val Glu
85 90 95

Glu Glu Leu Asp Arg Ala Gln Glu Arg Leu Ala Thr Ala Leu Gln Lys
100 105 110

Leu Glu Glu Ala Glu Lys Ala Ala Asp Glu Ser Glu Arg Gly Met Lys
115 120 125

Val Ile Glu Asn Arg Ala Met Lys Asp Glu Glu Lys Met Glu Leu Gln
130 135 140

Glu Met Gln Leu Lys Glu Ala Lys His Ile Ala Glu Asp Ser Asp Arg
145 150 155 160

Lys Tyr Glu Glu Val Ala Arg Lys Leu Val Ile Leu Glu Gly Glu Leu
165 170 175

Glu Arg Ser Glu Glu Arg Ala Glu Val Ala Glu Ser Lys Cys Gly Asp
180 185 190

Leu Glu Glu Glu Leu Lys Ile Val Thr Asn Asn Leu Lys Ser Leu Glu
195 200 205

Ala Gln Ala Asp Lys Tyr Ser Thr Lys Glu Asp Lys Tyr Glu Glu Glu
210 215 220

Ile Lys Leu Leu Glu Glu Lys Leu Lys Glu Ala Glu Thr Arg Ala Glu
225 230 235 240

Phe Ala Glu Arg Ser Val Ala Lys Leu Glu Lys Thr Ile Asp Asp Leu
245 250 255

Glu Asp Glu Val Tyr Ala Gln Lys Met Lys Tyr Lys Ala Ile Ser Glu
260 265 270

Glu Leu Asp Asn Ala Leu Asn Asp Ile Thr Ser Leu
275 280

<210> 179
<211> 329
<212> PRT
<213> Homo sapiens

<400> 179

Met Thr Gly Asn Ala Gly Glu Trp Cys Leu Met Glu Ser Asp Pro Gly
1 5 10 15

Val Phe Thr Glu Leu Ile Lys Gly Phe Gly Cys Arg Gly Ala Gln Val
20 25 30

Glu Glu Ile Trp Ser Leu Glu Pro Glu Asn Phe Glu Lys Leu Lys Pro
35 40 45

Val His Gly Leu Ile Phe Leu Phe Lys Trp Gln Pro Gly Glu Glu Pro
50 55 60

Ala Gly Ser Val Val Gln Asp Ser Arg Leu Asp Thr Ile Phe Phe Ala
65 70 75 80

Lys Gln Val Ile Asn Asn Ala Cys Ala Thr Gln Ala Ile Val Ser Val
85 90 95

Leu Leu Asn Cys Thr His Gln Asp Val His Leu Gly Glu Thr Leu Ser
100 105 110

Glu Phe Lys Glu Phe Ser Gln Ser Phe Asp Ala Ala Met Lys Gly Leu
115 120 125

Ala Leu Ser Asn Ser Asp Val Ile Arg Gln Val His Asn Ser Phe Ala
130 135 140

Arg Gln Gln Met Phe Glu Phe Asp Thr Lys Thr Ser Ala Lys Glu Glu
145 150 155 160

Asp Ala Phe His Phe Val Ser Tyr Val Pro Val Asn Gly Arg Leu Tyr
165 170 175

Glu Leu Asp Gly Leu Arg Glu Gly Pro Ile Asp Leu Gly Ala Cys Asn
180 185 190

Gln Asp Asp Trp Phe Ser Ala Val Arg Pro Val Ile Glu Lys Arg Ile
195 200 205

Gln Lys Tyr Ser Glu Gly Glu Ile Arg Phe Asn Leu Met Ala Ile Val
210 215 220

Ser Asp Arg Lys Met Ile Tyr Glu Gln Lys Ile Ala Glu Leu Gln Arg
225 230 235 240

Gln Leu Ala Glu Glu Glu Pro Met Asp Thr Asp Gln Gly Asn Ser Met
245 250 255

Leu Ser Ala Ile Gln Ser Glu Val Ala Lys Asn Gln Met Leu Ile Glu
260 265 270

Glu Glu Val Gln Lys Leu Lys Arg Tyr Lys Ile Glu Asn Ile Arg Arg
275 280 285

Lys His Asn Tyr Leu Pro Phe Ile Met Glu Leu Leu Lys Thr Leu Ala
290 295 300

Glu His Gln Gln Leu Ile Pro Leu Val Glu Lys Ala Lys Glu Lys Gln
305 310 315 320

Asn Ala Lys Lys Ala Gln Glu Thr Lys
325

<210> 180
<211> 541
<212> PRT
<213> Homo sapiens

<400> 180

Met Lys Ser Tyr Thr Pro Tyr Phe Ile Leu Leu Trp Ser Ala Val Gly
1 5 10 15

Ile Ala Lys Ala Ala Lys Ile Ile Ile Val Pro Pro Ile Met Phe Glu
20 25 30

Ser His Met Tyr Ile Phe Lys Thr Leu Ala Ser Ala Leu His Glu Arg
35 40 45

Gly His His Thr Val Phe Leu Leu Ser Glu Gly Arg Asp Ile Ala Pro
50 55 60

Ser Asn His Tyr Ser Leu Gln Arg Tyr Pro Gly Ile Phe Asn Ser Thr
65 70 75 80

Thr Ser Asp Ala Phe Leu Gln Ser Lys Met Arg Asn Ile Phe Ser Gly
85 90 95

Arg Leu Thr Ala Ile Glu Leu Phe Asp Ile Leu Asp His Tyr Thr Lys
100 105 110

Asn Cys Asp Leu Met Val Gly Asn His Ala Leu Ile Gln Gly Leu Lys
115 120 125

Lys Glu Lys Phe Asp Leu Leu Leu Val Asp Pro Asn Asp Met Cys Gly
130 135 140

Phe Val Ile Ala His Leu Leu Gly Val Lys Tyr Ala Val Phe Ser Thr
145 150 155 160

Gly Leu Trp Tyr Pro Ala Glu Val Gly Ala Pro Ala Pro Leu Ala Tyr
165 170 175

Val Pro Glu Phe Asn Ser Leu Leu Thr Asp Arg Met Asn Leu Leu Gln
180 185 190

Arg Met Lys Asn Thr Gly Val Tyr Leu Ile Ser Arg Leu Gly Val Ser
195 200 205

Phe Leu Val Leu Pro Lys Tyr Glu Arg Ile Met Gln Lys Tyr Asn Leu
210 215 220

```
Leu Pro Glu Lys Ser Met Tyr Asp Leu Val His Gly Ser Ser Leu Trp
225                 230                 235                 240

Met Leu Cys Thr Asp Val Ala Leu Glu Phe Pro Arg Pro Thr Leu Pro
                245                 250                 255

Asn Val Val Tyr Val Gly Gly Ile Leu Thr Lys Pro Ala Ser Pro Leu
            260                 265                 270

Pro Glu Asp Leu Gln Arg Trp Val Asn Gly Ala Asn Glu His Gly Phe
        275                 280                 285

Val Leu Val Ser Phe Gly Ala Gly Val Lys Tyr Leu Ser Glu Asp Ile
    290                 295                 300

Ala Asn Lys Leu Ala Gly Ala Leu Gly Arg Leu Pro Gln Lys Val Ile
305                 310                 315                 320

Trp Arg Phe Ser Gly Pro Lys Pro Lys Asn Leu Gly Asn Asn Thr Lys
                325                 330                 335

Leu Ile Glu Trp Leu Pro Gln Asn Asp Leu Leu Gly His Ser Lys Ile
            340                 345                 350

Lys Ala Phe Leu Ser His Gly Gly Leu Asn Ser Ile Phe Glu Thr Met
        355                 360                 365

Tyr His Gly Val Pro Val Val Gly Ile Pro Leu Phe Gly Asp His Tyr
    370                 375                 380

Asp Thr Met Thr Arg Val Gln Ala Lys Gly Met Gly Ile Leu Leu Glu
385                 390                 395                 400

Trp Lys Thr Val Thr Glu Lys Glu Leu Tyr Glu Ala Leu Val Lys Val
                405                 410                 415

Ile Asn Asn Pro Ser Tyr Arg Gln Arg Ala Gln Lys Leu Ser Glu Ile
            420                 425                 430

His Lys Asp Gln Pro Gly His Pro Val Asn Arg Thr Ile Tyr Trp Ile
        435                 440                 445

Asp Tyr Ile Ile Arg His Asn Gly Ala His His Leu Arg Ala Ala Val
    450                 455                 460

His Gln Ile Ser Phe Cys Gln Tyr Phe Leu Leu Asp Ile Ala Phe Val
465                 470                 475                 480

Leu Leu Leu Gly Ala Ala Leu Leu Tyr Phe Leu Leu Ser Trp Val Thr
                485                 490                 495
```

Lys Phe Ile Tyr Arg Lys Ile Lys Ser Leu Trp Ser Arg Asn Lys His
500 505 510

Ser Thr Val Asn Gly His Tyr His Asn Gly Ile Leu Asn Gly Lys Tyr
515 520 525

Lys Arg Asn Gly His Ile Lys His Glu Lys Lys Val Lys
530 535 540

<210> 181
<211> 457
<212> PRT
<213> Homo sapiens

<400> 181

Met Ser Gln Gly Ser Val Thr Phe Arg Asp Val Ala Ile Asp Phe Ser
1 5 10 15

Gln Glu Glu Trp Lys Trp Leu Gln Pro Ala Gln Arg Asp Leu Tyr Arg
20 25 30

Cys Val Met Leu Glu Asn Tyr Gly His Leu Val Ser Leu Gly Leu Ser
35 40 45

Ile Ser Lys Pro Asp Val Val Ser Leu Leu Glu Gln Gly Lys Glu Pro
50 55 60

Trp Leu Gly Lys Arg Glu Val Lys Arg Asp Leu Phe Ser Val Ser Glu
65 70 75 80

Ser Ser Gly Glu Ile Lys Asp Phe Ser Pro Lys Asn Val Ile Tyr Asp
85 90 95

Asp Ser Ser Gln Tyr Leu Ile Met Glu Arg Ile Leu Ser Gln Gly Pro
100 105 110

Val Tyr Ser Ser Phe Lys Gly Gly Trp Lys Cys Lys Asp His Thr Glu
115 120 125

Met Leu Gln Glu Asn Gln Gly Cys Ile Arg Lys Val Thr Val Ser His
130 135 140

Gln Glu Ala Leu Ala Gln His Met Asn Ile Ser Thr Val Glu Arg Pro
145 150 155 160

Tyr Gly Cys His Glu Cys Gly Lys Thr Phe Gly Arg Arg Phe Ser Leu
165 170 175

Val Leu His Gln Arg Thr His Thr Gly Glu Lys Pro Tyr Ala Cys Lys
180 185 190

Glu Cys Gly Lys Thr Phe Ser Gln Ile Ser Asn Leu Val Lys His Gln

195 200 205

Met Ile His Thr Gly Lys Lys Pro His Glu Cys Lys Asp Cys Asn Lys
210 215 220

Thr Phe Ser Tyr Leu Ser Phe Leu Ile Glu His Gln Arg Thr His Thr
225 230 235 240

Gly Glu Lys Pro Tyr Glu Cys Thr Glu Cys Gly Lys Ala Phe Ser Arg
245 250 255

Ala Ser Asn Leu Thr Arg His Gln Arg Ile His Ile Gly Lys Lys Gln
260 265 270

Tyr Ile Cys Arg Lys Cys Gly Lys Ala Phe Ser Ser Gly Ser Glu Leu
275 280 285

Ile Arg His Gln Ile Thr His Thr Gly Glu Lys Pro Tyr Glu Cys Ile
290 295 300

Glu Cys Gly Lys Ala Phe Arg Arg Phe Ser His Leu Thr Arg His Gln
305 310 315 320

Ser Ile His Thr Thr Lys Thr Pro Tyr Glu Cys Asn Glu Cys Arg Lys
325 330 335

Ala Leu Arg Cys His Ser Phe Leu Ile Lys His Gln Arg Ile His Ala
340 345 350

Gly Glu Lys Leu Tyr Glu Cys Asp Glu Cys Gly Lys Val Phe Thr Trp
355 360 365

His Ala Ser Leu Ile Gln His Thr Lys Ser His Thr Gly Glu Lys Pro
370 375 380

Tyr Ala Cys Ala Glu Cys Asp Lys Ala Phe Ser Arg Ser Phe Ser Leu
385 390 395 400

Ile Leu His Gln Arg Thr His Thr Gly Glu Lys Pro Tyr Val Cys Lys
405 410 415

Val Cys Asn Lys Ser Phe Ser Trp Ser Ser Asn Leu Ala Lys His Gln
420 425 430

Arg Thr His Thr Leu Asp Asn Pro Tyr Glu Tyr Glu Asn Ser Phe Asn
435 440 445

Tyr His Ser Phe Leu Thr Glu His Gln
450 455

<210> 182

<211> 653
<212> PRT
<213> Homo sapiens

<400> 182

Met Ala Ser Arg Leu Pro Thr Ala Trp Ser Cys Glu Pro Val Thr Phe
1 5 10 15

Glu Asp Val Thr Leu Gly Phe Thr Pro Glu Glu Trp Gly Leu Leu Asp
20 25 30

Leu Lys Gln Lys Ser Leu Tyr Arg Glu Val Met Leu Glu Asn Tyr Arg
35 40 45

Asn Leu Val Ser Val Glu His Gln Leu Ser Lys Pro Asp Val Val Ser
50 55 60

Gln Leu Glu Glu Ala Glu Asp Phe Trp Pro Val Glu Arg Gly Ile Pro
65 70 75 80

Gln Asp Thr Ile Pro Glu Tyr Pro Glu Leu Gln Leu Asp Pro Lys Leu
85 90 95

Asp Pro Leu Pro Ala Glu Ser Pro Leu Met Asn Ile Glu Val Val Glu
100 105 110

Val Leu Thr Leu Asn Gln Glu Val Ala Gly Pro Arg Asn Ala Gln Ile
115 120 125

Gln Ala Leu Tyr Ala Glu Asp Gly Ser Leu Ser Ala Asp Ala Pro Ser
130 135 140

Glu Gln Ile Gln Gln Gln Gly Lys His Pro Gly Asp Pro Glu Ala Ala
145 150 155 160

Arg Gln Arg Phe Arg Gln Phe Arg Tyr Lys Asp Met Thr Gly Pro Arg
165 170 175

Glu Ala Leu Asp Gln Leu Arg Glu Leu Cys His Gln Trp Leu Gln Pro
180 185 190

Lys Ala Arg Ser Lys Glu Gln Ile Leu Glu Leu Leu Val Leu Glu Gln
195 200 205

Phe Leu Gly Ala Leu Pro Val Lys Leu Arg Thr Trp Val Glu Ser Gln
210 215 220

His Pro Glu Asn Cys Gln Glu Val Val Ala Leu Val Glu Gly Val Thr
225 230 235 240

Trp Met Ser Glu Glu Glu Val Leu Pro Ala Gly Gln Pro Ala Glu Gly
245 250 255

Thr Thr Cys Cys Leu Glu Val Thr Ala Gln Gln Glu Glu Lys Gln Glu
260 265 270

Asp Ala Ala Ile Cys Pro Val Thr Val Leu Pro Glu Glu Pro Val Thr
275 280 285

Phe Gln Asp Val Ala Val Asp Phe Ser Arg Glu Glu Trp Gly Leu Leu
290 295 300

Gly Pro Thr Gln Arg Thr Glu Tyr Arg Asp Val Met Leu Glu Thr Phe
305 310 315 320

Gly His Leu Val Ser Val Gly Trp Glu Thr Thr Leu Glu Asn Lys Glu
325 330 335

Leu Ala Pro Asn Ser Asp Ile Pro Glu Glu Glu Pro Ala Pro Ser Leu
340 345 350

Lys Val Gln Glu Ser Ser Arg Asp Cys Ala Leu Ser Ser Thr Leu Glu
355 360 365

Asp Thr Leu Gln Gly Gly Val Gln Glu Val Gln Asp Thr Val Leu Lys
370 375 380

Gln Met Glu Ser Ala Gln Glu Lys Asp Leu Pro Gln Lys Lys His Phe
385 390 395 400

Asp Asn Arg Glu Ser Gln Ala Asn Ser Gly Ala Leu Asp Thr Asn Gln
405 410 415

Val Ser Leu Gln Lys Ile Asp Asn Pro Glu Ser Gln Ala Asn Ser Gly
420 425 430

Ala Leu Asp Thr Asn Gln Val Leu Leu His Lys Ile Pro Pro Arg Lys
435 440 445

Arg Leu Arg Lys Arg Asp Ser Gln Val Lys Ser Met Lys His Asn Ser
450 455 460

Arg Val Lys Ile His Gln Lys Ser Cys Glu Arg Gln Lys Ala Lys Glu
465 470 475 480

Gly Asn Gly Cys Arg Lys Thr Phe Ser Arg Ser Thr Lys Gln Ile Thr
485 490 495

Phe Ile Arg Ile His Lys Gly Ser Gln Val Cys Arg Cys Ser Glu Cys
500 505 510

Gly Lys Ile Phe Arg Asn Pro Arg Tyr Phe Ser Val His Lys Lys Ile
515 520 525

His Thr Gly Glu Arg Pro Tyr Val Cys Gln Asp Cys Gly Lys Gly Phe
530 535 540

Val Gln Ser Ser Ser Leu Thr Gln His Gln Arg Val His Ser Gly Glu
545 550 555 560

Arg Pro Phe Glu Cys Gln Glu Cys Gly Arg Thr Phe Asn Asp Arg Ser
565 570 575

Ala Ile Ser Gln His Leu Arg Thr His Thr Gly Ala Lys Pro Tyr Lys
580 585 590

Cys Gln Asp Cys Gly Lys Ala Phe Arg Gln Ser Ser His Leu Ile Arg
595 600 605

His Gln Arg Thr His Thr Gly Glu Arg Pro Tyr Ala Cys Asn Lys Cys
610 615 620

Gly Lys Ala Phe Thr Gln Ser Ser His Leu Ile Gly His Gln Arg Thr
625 630 635 640

His Asn Arg Thr Lys Arg Lys Lys Lys Gln Pro Thr Ser
645 650

<210> 183
<211> 715
<212> PRT
<213> Homo sapiens

<400> 183

Met Glu Thr Leu Glu Ser Glu Leu Thr Cys Pro Ile Cys Leu Glu Leu
1 5 10 15

Phe Glu Asp Pro Leu Leu Leu Pro Cys Ala His Ser Leu Cys Phe Ser
20 25 30

Cys Ala His Arg Ile Leu Val Ser Ser Cys Ser Ser Gly Glu Ser Ile
35 40 45

Glu Pro Ile Thr Ala Phe Gln Cys Pro Thr Cys Arg Tyr Val Ile Ser
50 55 60

Leu Asn His Arg Gly Leu Asp Gly Leu Lys Arg Asn Val Thr Leu Gln
65 70 75 80

Asn Ile Ile Asp Arg Phe Gln Lys Ala Ser Val Ser Gly Pro Asn Ser
85 90 95

Pro Ser Glu Ser Arg Arg Glu Arg Thr Tyr Arg Pro Thr Thr Ala Met
100 105 110

Ser Ser Glu Arg Ile Ala Cys Gln Phe Cys Glu Gln Asp Pro Pro Arg
115 120 125

Asp Ala Val Lys Thr Cys Ile Thr Cys Glu Val Ser Tyr Cys Asp Arg
130 135 140

Cys Leu Arg Ala Thr His Pro Asn Lys Lys Pro Phe Thr Ser His Arg
145 150 155 160

Leu Val Glu Pro Val Pro Asp Thr His Leu Arg Gly Ile Thr Cys Leu
165 170 175

Asp His Glu Asn Glu Lys Val Asn Met Tyr Cys Val Ser Asp Asp Gln
180 185 190

Leu Ile Cys Ala Leu Cys Lys Leu Val Gly Arg His Arg Asp His Gln
195 200 205

Val Ala Ser Leu Asn Asp Arg Phe Glu Lys Leu Lys Gln Thr Leu Glu
210 215 220

Met Asn Leu Thr Asn Leu Val Lys Arg Asn Ser Glu Leu Glu Asn Gln
225 230 235 240

Met Ala Lys Leu Ile Gln Ile Cys Gln Gln Val Glu Val Asn Thr Ala
245 250 255

Met His Glu Ala Lys Leu Met Glu Glu Cys Asp Glu Leu Val Glu Ile
260 265 270

Ile Gln Gln Arg Lys Gln Met Ile Ala Val Lys Ile Lys Glu Thr Lys
275 280 285

Val Met Lys Leu Arg Lys Leu Ala Gln Gln Val Ala Asn Cys Arg Gln
290 295 300

Cys Leu Glu Arg Ser Thr Val Leu Ile Asn Gln Ala Glu His Ile Leu
305 310 315 320

Lys Glu Asn Asp Gln Ala Arg Phe Leu Gln Ser Ala Lys Asn Ile Ala
325 330 335

Glu Arg Val Ala Met Ala Thr Ala Ser Ser Gln Val Leu Ile Pro Asp
340 345 350

Ile Asn Phe Asn Asp Ala Phe Glu Asn Phe Ala Leu Asp Phe Ser Arg
355 360 365

Glu Lys Lys Leu Leu Glu Gly Leu Asp Tyr Leu Thr Ala Pro Asn Pro
370 375 380

Pro Ser Ile Arg Glu Glu Leu Cys Thr Ala Ser His Asp Thr Ile Thr
385 390 395 400

Val His Trp Ile Ser Asp Asp Glu Phe Ser Ile Ser Ser Tyr Glu Leu
405 410 415

Gln Tyr Thr Ile Phe Thr Gly Gln Ala Asn Phe Ile Ser Lys Ser Trp
420 425 430

Cys Ser Trp Gly Leu Trp Pro Glu Ile Arg Lys Cys Lys Glu Ala Val
435 440 445

Ser Cys Ser Arg Leu Ala Gly Ala Pro Arg Gly Leu Tyr Asn Ser Val
450 455 460

Asp Ser Trp Met Ile Val Pro Asn Ile Lys Gln Asn His Tyr Thr Val
465 470 475 480

His Gly Leu Gln Ser Gly Thr Arg Tyr Ile Phe Ile Val Lys Ala Ile
485 490 495

Asn Gln Ala Gly Ser Arg Asn Ser Glu Pro Thr Arg Leu Lys Thr Asn
500 505 510

Ser Gln Pro Phe Lys Leu Asp Pro Lys Met Thr His Lys Lys Leu Lys
515 520 525

Ile Ser Asn Asp Gly Leu Gln Met Glu Lys Asp Glu Ser Ser Leu Lys
530 535 540

Lys Ser His Thr Pro Glu Arg Phe Ser Gly Thr Gly Cys Tyr Gly Ala
545 550 555 560

Ala Gly Asn Ile Phe Ile Asp Ser Gly Cys His Tyr Trp Glu Val Val
565 570 575

Met Gly Ser Ser Thr Trp Tyr Ala Ile Gly Ile Ala Tyr Lys Ser Ala
580 585 590

Pro Lys Asn Glu Trp Ile Gly Lys Asn Ala Ser Ser Trp Val Phe Ser
595 600 605

Arg Cys Asn Ser Asn Phe Val Val Arg His Asn Asn Lys Glu Met Leu
610 615 620

Val Asp Val Pro Pro His Leu Lys Arg Leu Gly Val Leu Leu Asp Tyr
625 630 635 640

Asp Asn Asn Met Leu Ser Phe Tyr Asp Pro Ala Asn Ser Leu His Leu
645 650 655

His Thr Phe Asp Val Thr Phe Ile Leu Pro Val Cys Pro Thr Phe Thr
660 665 670

Ile Trp Asn Lys Ser Leu Met Ile Leu Ser Gly Leu Pro Ala Pro Asp
675 680 685

Phe Ile Asp Tyr Pro Glu Arg Gln Glu Cys Asn Cys Arg Pro Gln Glu
690 695 700

Ser Pro Tyr Val Ser Gly Met Lys Thr Cys His
705 710 715

<210> 184
<211> 1076
<212> PRT
<213> Homo sapiens

<400> 184

Met Glu Pro Gly Ser Lys Ser Val Ser Arg Ser Asp Trp Gln Pro Glu
1 5 10 15

Pro His Gln Arg Pro Ile Thr Pro Leu Glu Pro Gly Pro Glu Lys Thr
20 25 30

Pro Ile Ala Gln Pro Glu Ser Lys Thr Leu Gln Gly Ser Asn Thr Gln
35 40 45

Gln Lys Pro Ala Ser Asn Gln Arg Pro Leu Thr Gln Gln Glu Thr Pro
50 55 60

Ala Gln His Asp Ala Glu Ser Gln Lys Glu Pro Arg Ala Gln Gln Lys
65 70 75 80

Ser Ala Ser Gln Glu Glu Phe Leu Ala Pro Gln Lys Pro Ala Pro Gln
85 90 95

Gln Ser Pro Tyr Ile Gln Arg Val Leu Leu Thr Gln Gln Glu Ala Ala
100 105 110

Ser Gln Gln Gly Pro Gly Leu Gly Lys Glu Ser Ile Thr Gln Gln Glu
115 120 125

Pro Ala Leu Arg Gln Arg His Val Ala Gln Pro Gly Pro Gly Pro Gly
130 135 140

Glu Pro Pro Pro Ala Gln Gln Glu Ala Glu Ser Thr Pro Ala Ala Gln
145 150 155 160

Ala Lys Pro Gly Ala Lys Arg Glu Pro Ser Ala Pro Thr Glu Ser Thr
165 170 175

Ser Gln Glu Thr Pro Glu Gln Ser Asp Lys Gln Thr Thr Pro Val Gln
180 185 190

Gly Ala Lys Ser Lys Gln Gly Ser Leu Thr Glu Leu Gly Phe Leu Thr
195 200 205

Lys Leu Gln Glu Leu Ser Ile Gln Arg Ser Ala Leu Glu Trp Lys Ala
210 215 220

Leu Ser Glu Trp Val Ala Asp Ser Glu Ser Glu Ser Asp Val Gly Ser
225 230 235 240

Ser Ser Asp Thr Asp Ser Pro Ala Thr Met Gly Gly Met Val Ala Gln
245 250 255

Gly Val Lys Leu Gly Phe Lys Gly Lys Ser Gly Tyr Lys Val Met Ser
260 265 270

Gly Tyr Ser Gly Thr Ser Pro His Glu Lys Thr Ser Ala Arg Asn His
275 280 285

Arg His Tyr Gln Asp Thr Ala Ser Arg Leu Ile His Asn Met Asp Leu
290 295 300

Arg Thr Met Thr Gln Ser Leu Val Thr Leu Ala Glu Asp Asn Ile Ala
305 310 315 320

Phe Phe Ser Ser Gln Gly Pro Gly Glu Thr Ala Gln Arg Leu Ser Gly
325 330 335

Val Phe Ala Gly Val Arg Glu Gln Ala Leu Gly Leu Glu Pro Ala Leu
340 345 350

Gly Arg Leu Leu Gly Val Ala His Leu Phe Asp Leu Asp Pro Glu Thr
355 360 365

Pro Ala Asn Gly Tyr Arg Ser Leu Val His Thr Ala Arg Cys Cys Leu
370 375 380

Ala His Leu Leu His Lys Ser Arg Tyr Val Ala Ser Asn Arg Arg Ser
385 390 395 400

Ile Phe Phe Arg Thr Ser His Asn Leu Ala Glu Leu Glu Ala Tyr Leu
405 410 415

Ala Ala Leu Thr Gln Leu Arg Ala Leu Val Tyr Tyr Ala Gln Arg Leu
420 425 430

Leu Val Thr Asn Arg Pro Gly Val Leu Phe Phe Glu Gly Asp Glu Gly
435 440 445

Leu Thr Ala Asp Phe Leu Arg Glu Tyr Val Thr Leu His Lys Gly Cys
450 455 460

Phe Tyr Gly Arg Cys Leu Gly Phe Gln Phe Thr Pro Ala Ile Arg Pro
465 470 475 480

Phe Leu Gln Thr Ile Ser Ile Gly Leu Val Ser Phe Gly Glu His Tyr
485 490 495

Lys Arg Asn Glu Thr Gly Leu Ser Val Ala Ala Ser Ser Leu Phe Thr
500 505 510

Ser Gly Arg Phe Ala Ile Asp Pro Glu Leu Arg Gly Ala Glu Phe Glu
515 520 525

Arg Ile Thr Gln Asn Leu Asp Val His Phe Trp Lys Ala Phe Trp Asn
530 535 540

Ile Thr Glu Met Glu Val Leu Ser Ser Leu Ala Asn Met Ala Ser Ala
545 550 555 560

Thr Val Arg Val Ser Arg Leu Leu Ser Leu Pro Pro Glu Ala Phe Glu
565 570 575

Met Pro Leu Thr Ala Asp Pro Thr Leu Thr Val Thr Ile Ser Pro Pro
580 585 590

Leu Ala His Thr Gly Pro Gly Pro Val Leu Val Arg Leu Ile Ser Tyr
595 600 605

Asp Leu Arg Glu Gly Gln Asp Ser Glu Glu Leu Ser Ser Leu Ile Lys
610 615 620

Ser Asn Gly Gln Arg Ser Leu Glu Leu Trp Pro Arg Pro Gln Gln Ala
625 630 635 640

Pro Arg Ser Arg Ser Leu Ile Val His Phe His Gly Gly Gly Phe Val
645 650 655

Ala Gln Thr Ser Arg Ser His Glu Pro Tyr Leu Lys Ser Trp Ala Gln
660 665 670

Glu Leu Gly Ala Pro Ile Ile Ser Ile Asp Tyr Ser Leu Ala Pro Glu
675 680 685

Ala Pro Phe Pro Arg Ala Leu Glu Glu Cys Phe Phe Ala Tyr Cys Trp
690 695 700

Ala Ile Lys His Cys Ala Leu Leu Gly Ser Thr Gly Glu Arg Ile Cys
705 710 715 720

Leu Ala Gly Asp Ser Ala Gly Gly Asn Leu Cys Phe Thr Val Ala Leu
725 730 735

Arg Ala Ala Ala Tyr Gly Val Arg Val Pro Asp Gly Ile Met Ala Ala
740 745 750

Tyr Pro Ala Thr Met Leu Gln Pro Ala Ala Ser Pro Ser Arg Leu Leu
755 760 765

Ser Leu Met Asp Pro Leu Leu Pro Leu Ser Val Leu Ser Lys Cys Val
770 775 780

Ser Ala Tyr Ala Gly Ala Lys Thr Glu Asp His Ser Asn Ser Asp Gln
785 790 795 800

Lys Ala Leu Gly Met Met Gly Leu Val Arg Arg Asp Thr Ala Leu Leu
805 810 815

Leu Arg Asp Phe Arg Leu Gly Ala Ser Ser Trp Leu Asn Ser Phe Leu
820 825 830

Glu Leu Ser Gly Arg Lys Ser Gln Lys Met Ser Glu Pro Ile Ala Glu
835 840 845

Pro Met Arg Arg Ser Val Ser Glu Ala Ala Leu Ala Gln Pro Gln Gly
850 855 860

Pro Leu Gly Thr Asp Ser Leu Lys Asn Leu Thr Leu Arg Asp Leu Ser
865 870 875 880

Leu Arg Gly Asn Ser Glu Thr Ser Ser Asp Thr Pro Glu Met Ser Leu
885 890 895

Ser Ala Glu Thr Leu Ser Pro Ser Thr Pro Ser Asp Val Asn Phe Leu
900 905 910

Leu Pro Pro Glu Asp Ala Gly Glu Glu Ala Glu Ala Lys Asn Glu Leu
915 920 925

Ser Pro Met Asp Arg Gly Leu Gly Val Arg Ala Ala Phe Pro Glu Gly
930 935 940

Phe His Pro Arg Arg Ser Ser Gln Gly Ala Thr Gln Met Pro Leu Tyr
945 950 955 960

Ser Ser Pro Ile Val Lys Asn Pro Phe Met Ser Pro Leu Leu Ala Pro
965 970 975

Asp Ser Met Leu Lys Ser Leu Pro Pro Val His Ile Val Ala Cys Ala
980 985 990

Leu Asp Pro Met Leu Asp Asp Ser Val Met Leu Ala Arg Arg Leu Arg
995 1000 1005

Asn Leu Gly Gln Pro Val Thr Leu Arg Val Val Glu Asp Leu Pro
1010 1015 1020

His Gly Phe Leu Thr Leu Ala Ala Leu Cys Arg Glu Thr Arg Gln
1025 1030 1035

Ala Ala Glu Leu Cys Val Glu Arg Ile Arg Leu Val Leu Thr Pro
1040 1045 1050

Pro Ala Gly Ala Gly Pro Ser Gly Glu Thr Gly Ala Ala Gly Val
1055 1060 1065

Asp Gly Gly Cys Gly Gly Arg His
1070 1075

<210> 185
<211> 1215
<212> PRT
<213> Homo sapiens

<400> 185

Met Thr Ser Thr Gly Gln Asp Ser Thr Thr Thr Arg Gln Arg Arg Ser
1 5 10 15

Arg Gln Asn Pro Gln Ser Pro Pro Gln Asp Ser Ser Val Thr Ser Lys
20 25 30

Arg Asn Ile Lys Lys Gly Ala Val Pro Arg Ser Ile Pro Asn Leu Ala
35 40 45

Glu Val Lys Lys Lys Gly Lys Met Lys Lys Leu Gly Gln Ala Met Glu
50 55 60

Glu Asp Leu Ile Val Gly Leu Gln Gly Met Asp Leu Asn Leu Glu Ala
65 70 75 80

Glu Ala Leu Ala Gly Thr Gly Leu Val Leu Asp Glu Gln Leu Asn Glu
85 90 95

Phe His Cys Leu Trp Asp Asp Ser Phe Pro Glu Gly Pro Glu Arg Leu
100 105 110

His Ala Ile Lys Glu Gln Leu Ile Gln Glu Gly Leu Leu Asp Arg Cys
115 120 125

Val Ser Phe Gln Ala Arg Phe Ala Glu Lys Glu Glu Leu Met Leu Val
130 135 140

His Ser Leu Glu Tyr Ile Asp Leu Met Glu Thr Thr Gln Tyr Met Asn

145 150 155 160

Glu Gly Glu Leu Arg Val Leu Ala Asp Thr Tyr Asp Ser Val Tyr Leu
165 170 175

His Pro Asn Ser Tyr Ser Cys Ala Cys Leu Ala Ser Gly Ser Val Leu
180 185 190

Arg Leu Val Asp Ala Val Leu Gly Ala Glu Ile Arg Asn Gly Met Ala
195 200 205

Ile Ile Arg Pro Pro Gly His His Ala Gln His Ser Leu Met Asp Gly
210 215 220

Tyr Cys Met Phe Asn His Val Ala Val Ala Ala Arg Tyr Ala Gln Gln
225 230 235 240

Lys His Arg Ile Arg Arg Val Leu Ile Val Asp Trp Asp Val His His
245 250 255

Gly Gln Gly Thr Gln Phe Thr Phe Asp Gln Asp Pro Ser Val Leu Tyr
260 265 270

Phe Ser Ile His Arg Tyr Glu Gln Gly Arg Phe Trp Pro His Leu Lys
275 280 285

Ala Ser Asn Trp Ser Thr Thr Gly Phe Gly Gln Gly Gln Gly Tyr Thr
290 295 300

Ile Asn Val Pro Trp Asn Gln Val Gly Met Arg Asp Ala Asp Tyr Ile
305 310 315 320

Ala Ala Phe Leu His Val Leu Leu Pro Val Ala Leu Glu Phe Gln Pro
325 330 335

Gln Leu Val Leu Val Ala Ala Gly Phe Asp Ala Leu Gln Gly Asp Pro
340 345 350

Lys Gly Glu Met Ala Ala Thr Pro Ala Gly Phe Ala Gln Leu Thr His
355 360 365

Leu Leu Met Gly Leu Ala Gly Gly Lys Leu Ile Leu Ser Leu Glu Gly
370 375 380

Gly Tyr Asn Leu Arg Ala Leu Ala Glu Gly Val Ser Ala Ser Leu His
385 390 395 400

Thr Leu Leu Gly Asp Pro Cys Pro Met Leu Glu Ser Pro Gly Ala Pro
405 410 415

Cys Arg Ser Ala Gln Ala Ser Val Ser Cys Ala Leu Glu Ala Leu Glu

420 425 430

Pro Phe Trp Glu Val Leu Val Arg Ser Thr Glu Thr Val Glu Arg Asp
435 440 445

Asn Met Glu Glu Asp Asn Val Glu Glu Ser Glu Glu Glu Gly Pro Trp
450 455 460

Glu Pro Pro Val Leu Pro Ile Leu Thr Trp Pro Val Leu Gln Ser Arg
465 470 475 480

Thr Gly Leu Val Tyr Asp Gln Asn Met Met Asn His Cys Asn Leu Trp
485 490 495

Asp Ser His His Pro Glu Val Pro Gln Arg Ile Leu Arg Ile Met Cys
500 505 510

Arg Leu Glu Glu Leu Gly Leu Ala Gly Arg Cys Leu Thr Leu Thr Pro
515 520 525

Arg Pro Ala Thr Glu Ala Glu Leu Leu Thr Cys His Ser Ala Glu Tyr
530 535 540

Val Gly His Leu Arg Ala Thr Glu Lys Met Lys Thr Arg Glu Leu His
545 550 555 560

Arg Glu Ser Ser Asn Phe Asp Ser Ile Tyr Ile Cys Pro Ser Thr Phe
565 570 575

Ala Cys Ala Gln Leu Ala Thr Gly Ala Ala Cys Arg Leu Val Glu Ala
580 585 590

Val Leu Ser Gly Glu Val Leu Asn Gly Ala Ala Val Val Arg Pro Pro
595 600 605

Gly His His Ala Glu Gln Asp Ala Ala Cys Gly Phe Cys Phe Phe Asn
610 615 620

Ser Val Ala Val Ala Ala Arg His Ala Gln Thr Ile Ser Gly His Ala
625 630 635 640

Leu Arg Ile Leu Ile Val Asp Trp Asp Val His His Gly Asn Gly Thr
645 650 655

Gln His Met Phe Glu Asp Asp Pro Ser Val Leu Tyr Val Ser Leu His
660 665 670

Arg Tyr Asp His Gly Thr Phe Phe Pro Met Gly Asp Glu Gly Ala Ser
675 680 685

Ser Gln Ile Gly Arg Ala Ala Gly Thr Gly Phe Thr Val Asn Val Ala

690 695 700

Trp Asn Gly Pro Arg Met Gly Asp Ala Asp Tyr Leu Ala Ala Trp His
705 710 715 720

Arg Leu Val Leu Pro Ile Ala Tyr Glu Phe Asn Pro Glu Leu Val Leu
725 730 735

Val Ser Ala Gly Phe Asp Ala Ala Arg Gly Asp Pro Leu Gly Gly Cys
740 745 750

Gln Val Ser Pro Glu Gly Tyr Ala His Leu Thr His Leu Leu Met Gly
755 760 765

Leu Ala Ser Gly Arg Ile Ile Leu Ile Leu Glu Gly Gly Tyr Asn Leu
770 775 780

Thr Ser Ile Ser Glu Ser Met Ala Ala Cys Thr Arg Ser Leu Leu Gly
785 790 795 800

Asp Pro Pro Pro Leu Leu Thr Leu Pro Arg Pro Pro Leu Ser Gly Ala
805 810 815

Leu Ala Ser Ile Thr Glu Thr Ile Gln Val His Arg Arg Tyr Trp Arg
820 825 830

Ser Leu Arg Val Met Lys Val Glu Asp Arg Glu Gly Pro Ser Ser Ser
835 840 845

Lys Leu Val Thr Lys Lys Ala Pro Gln Pro Ala Lys Pro Arg Leu Ala
850 855 860

Glu Arg Met Thr Thr Arg Glu Lys Lys Val Leu Glu Ala Gly Met Gly
865 870 875 880

Lys Val Thr Ser Ala Ser Phe Gly Glu Glu Ser Thr Pro Gly Gln Thr
885 890 895

Asn Ser Glu Thr Ala Val Val Ala Leu Thr Gln Asp Gln Pro Ser Glu
900 905 910

Ala Ala Thr Gly Gly Ala Thr Leu Ala Gln Thr Ile Ser Glu Ala Ala
915 920 925

Ile Gly Gly Ala Met Leu Gly Gln Thr Thr Ser Glu Glu Ala Val Gly
930 935 940

Gly Ala Thr Pro Asp Gln Thr Thr Ser Glu Glu Thr Val Gly Gly Ala
945 950 955 960

Ile Leu Asp Gln Thr Thr Ser Glu Asp Ala Val Gly Gly Ala Thr Leu

965 970 975

Gly Gln Thr Thr Ser Glu Glu Ala Val Gly Gly Ala Thr Leu Ala Gln
980 985 990

Thr Ile Ser Glu Ala Ala Met Glu Gly Ala Thr Leu Asp Gln Thr Thr
995 1000 1005

Ser Glu Glu Ala Pro Gly Gly Thr Glu Leu Ile Gln Thr Pro Leu
1010 1015 1020

Ala Ser Ser Thr Asp His Gln Thr Pro Pro Thr Ser Pro Val Gln
1025 1030 1035

Gly Thr Thr Pro Gln Ile Ser Pro Ser Thr Leu Ile Gly Ser Leu
1040 1045 1050

Arg Thr Leu Glu Leu Gly Ser Glu Ser Gln Gly Ala Ser Glu Ser
1055 1060 1065

Gln Ala Pro Gly Glu Glu Asn Leu Leu Gly Glu Ala Ala Gly Gly
1070 1075 1080

Gln Asp Met Ala Asp Ser Met Leu Met Gln Gly Ser Arg Gly Leu
1085 1090 1095

Thr Asp Gln Ala Ile Phe Tyr Ala Val Thr Pro Leu Pro Trp Cys
1100 1105 1110

Pro His Leu Val Ala Val Cys Pro Ile Pro Ala Ala Gly Leu Asp
1115 1120 1125

Val Thr Gln Pro Cys Gly Asp Cys Gly Thr Ile Gln Glu Asn Trp
1130 1135 1140

Val Cys Leu Ser Cys Tyr Gln Val Tyr Cys Gly Arg Tyr Ile Asn
1145 1150 1155

Gly His Met Leu Gln His His Gly Asn Ser Gly His Pro Leu Val
1160 1165 1170

Leu Ser Tyr Ile Asp Leu Ser Ala Trp Cys Tyr Tyr Cys Gln Ala
1175 1180 1185

Tyr Val His His Gln Ala Leu Leu Asp Val Lys Asn Ile Ala His
1190 1195 1200

Gln Asn Lys Phe Gly Glu Asp Met Pro His Pro His
1205 1210 1215

<210> 186
<211> 780

<212> PRT
<213> Homo sapiens

<400> 186

Met Ala Pro Tyr Ser Leu Leu Val Thr Arg Leu Gln Lys Ala Leu Gly
1 5 10 15

Val Arg Gln Tyr His Val Ala Ser Val Leu Cys Gln Arg Ala Lys Val
20 25 30

Ala Met Ser His Phe Glu Pro Asn Glu Tyr Ile His Tyr Asp Leu Leu
35 40 45

Glu Lys Asn Ile Asn Ile Val Arg Lys Arg Leu Asn Arg Pro Leu Thr
50 55 60

Leu Ser Glu Lys Ile Val Tyr Gly His Leu Asp Asp Pro Ala Ser Gln
65 70 75 80

Glu Ile Glu Arg Gly Lys Ser Tyr Leu Arg Leu Arg Pro Asp Arg Val
85 90 95

Ala Met Gln Asp Ala Thr Ala Gln Met Ala Met Leu Gln Phe Ile Ser
100 105 110

Ser Gly Leu Ser Lys Val Ala Val Pro Ser Thr Ile His Cys Asp His
115 120 125

Leu Ile Glu Ala Gln Val Gly Gly Glu Lys Asp Leu Arg Arg Ala Lys
130 135 140

Asp Ile Asn Gln Glu Val Tyr Asn Phe Leu Ala Thr Ala Gly Ala Lys
145 150 155 160

Tyr Gly Val Gly Phe Trp Lys Pro Gly Ser Gly Ile Ile His Gln Ile
165 170 175

Ile Leu Glu Asn Tyr Ala Tyr Pro Gly Val Leu Leu Ile Gly Thr Asp
180 185 190

Ser His Thr Pro Asn Gly Gly Gly Leu Gly Gly Ile Cys Ile Gly Val
195 200 205

Gly Gly Ala Asp Ala Val Asp Val Met Ala Gly Ile Pro Trp Glu Leu
210 215 220

Lys Cys Pro Lys Val Ile Gly Val Lys Leu Thr Gly Ser Leu Ser Gly
225 230 235 240

Trp Ser Ser Pro Lys Asp Val Ile Leu Lys Val Ala Gly Ile Leu Thr
245 250 255

Val Lys Gly Gly Thr Gly Ala Ile Val Glu Tyr His Gly Pro Gly Val
260 265 270

Asp Ser Ile Ser Cys Thr Gly Met Ala Thr Ile Cys Asn Met Gly Ala
275 280 285

Glu Ile Gly Ala Thr Thr Ser Val Phe Pro Tyr Asn His Arg Met Lys
290 295 300

Lys Tyr Leu Ser Lys Thr Gly Arg Glu Asp Ile Ala Asn Leu Ala Asp
305 310 315 320

Glu Phe Lys Asp His Leu Val Pro Asp Pro Gly Cys His Tyr Asp Gln
325 330 335

Leu Ile Glu Ile Asn Leu Ser Glu Leu Lys Pro His Ile Asn Gly Pro
340 345 350

Phe Thr Pro Asp Leu Ala His Pro Val Ala Glu Val Gly Lys Val Ala
355 360 365

Glu Lys Glu Gly Trp Pro Leu Asp Ile Arg Val Gly Leu Ile Gly Ser
370 375 380

Cys Thr Asn Ser Ser Tyr Glu Asp Met Gly Arg Ser Ala Ala Val Ala
385 390 395 400

Lys Gln Ala Leu Ala His Gly Leu Lys Cys Lys Ser Gln Phe Thr Ile
405 410 415

Thr Pro Gly Ser Glu Gln Ile Arg Ala Thr Ile Glu Arg Asp Gly Tyr
420 425 430

Ala Gln Ile Leu Arg Asp Leu Gly Gly Ile Val Leu Ala Asn Ala Cys
435 440 445

Gly Pro Cys Ile Gly Gln Trp Asp Arg Lys Asp Ile Lys Lys Gly Glu
450 455 460

Lys Asn Thr Ile Val Thr Ser Tyr Asn Arg Asn Phe Thr Gly Arg Asn
465 470 475 480

Asp Ala Asn Pro Glu Thr His Ala Phe Val Thr Ser Pro Glu Ile Val
485 490 495

Thr Ala Leu Ala Ile Ala Gly Thr Leu Lys Phe Asn Pro Glu Thr Asp
500 505 510

Tyr Leu Thr Gly Thr Asp Gly Lys Lys Phe Arg Leu Glu Ala Pro Asp
515 520 525

Ala Asp Glu Leu Pro Lys Gly Glu Phe Asp Pro Gly Gln Asp Thr Tyr
530 535 540

Gln His Pro Pro Lys Asp Ser Ser Gly Gln His Val Asp Val Ser Pro
545 550 555 560

Thr Ser Gln Arg Leu Gln Leu Leu Glu Pro Phe Asp Lys Trp Asp Gly
565 570 575

Lys Asp Leu Glu Asp Leu Gln Ile Leu Ile Lys Val Lys Gly Lys Cys
580 585 590

Thr Thr Asp His Ile Ser Ala Ala Gly Pro Trp Leu Lys Phe Arg Gly
595 600 605

His Leu Asp Asn Ile Ser Asn Asn Leu Leu Ile Gly Ala Ile Asn Ile
610 615 620

Glu Asn Gly Lys Ala Asn Ser Val Arg Asn Ala Val Thr Gln Glu Phe
625 630 635 640

Gly Pro Val Pro Asp Thr Ala Arg Tyr Tyr Lys Lys His Gly Ile Arg
645 650 655

Trp Val Val Ile Gly Asp Glu Asn Tyr Gly Glu Gly Ser Ser Arg Glu
660 665 670

His Ala Ala Leu Glu Pro Arg His Leu Gly Gly Arg Ala Ile Ile Thr
675 680 685

Lys Ser Phe Ala Arg Ile His Glu Thr Asn Leu Lys Lys Gln Gly Leu
690 695 700

Leu Pro Leu Thr Phe Ala Asp Pro Ala Asp Tyr Asn Lys Ile His Pro
705 710 715 720

Val Asp Lys Leu Thr Ile Gln Gly Leu Lys Asp Phe Thr Pro Gly Lys
725 730 735

Pro Leu Lys Cys Ile Ile Lys His Pro Asn Gly Thr Gln Glu Thr Ile
740 745 750

Leu Leu Asn His Thr Phe Asn Glu Thr Gln Ile Glu Trp Phe Arg Ala
755 760 765

Gly Ser Ala Leu Asn Arg Met Lys Glu Leu Gln Gln
770 775 780

<210> 187
<211> 650
<212> PRT
<213> Homo sapi ens

<400> 187

Met Gly Pro Ala Ser Pro Ala Ala Arg Gly Leu Ser Arg Arg Pro Gly
1 5 10 15

Gln Pro Pro Leu Pro Leu Leu Leu Pro Leu Leu Leu Leu Leu Leu Arg
20 25 30

Ala Gln Pro Ala Ile Gly Ser Leu Ala Gly Gly Ser Pro Gly Ala Ala
35 40 45

Glu Ala Pro Gly Ser Ala Gln Val Ala Gly Leu Cys Gly Arg Leu Thr
50 55 60

Leu His Arg Asp Leu Arg Thr Gly Arg Trp Glu Pro Asp Pro Gln Arg
65 70 75 80

Ser Arg Arg Cys Leu Arg Asp Pro Gln Arg Val Leu Glu Tyr Cys Arg
85 90 95

Gln Met Tyr Pro Glu Leu Gln Ile Ala Arg Val Glu Gln Ala Thr Gln
100 105 110

Ala Ile Pro Met Glu Arg Trp Cys Gly Gly Ser Arg Ser Gly Ser Cys
115 120 125

Ala His Pro His His Gln Val Val Pro Phe Arg Cys Leu Pro Gly Glu
130 135 140

Phe Val Ser Glu Ala Leu Leu Val Pro Glu Gly Cys Arg Phe Leu His
145 150 155 160

Gln Glu Arg Met Asp Gln Cys Glu Ser Ser Thr Arg Arg His Gln Glu
165 170 175

Ala Gln Glu Ala Cys Ser Ser Gln Gly Leu Ile Leu His Gly Ser Gly
180 185 190

Met Leu Leu Pro Cys Gly Ser Asp Arg Phe Arg Gly Val Glu Tyr Val
195 200 205

Cys Cys Pro Pro Pro Gly Thr Pro Asp Pro Ser Gly Thr Ala Val Gly
210 215 220

Asp Pro Ser Thr Arg Ser Trp Pro Pro Gly Ser Arg Val Glu Gly Ala
225 230 235 240

Glu Asp Glu Glu Glu Glu Glu Ser Phe Pro Gln Pro Val Asp Asp Tyr
245 250 255

Phe Val Glu Pro Pro Gln Ala Glu Glu Glu Glu Glu Thr Val Pro Pro
260 265 270

Pro Ser Ser His Thr Leu Ala Val Val Gly Lys Val Thr Pro Thr Pro
275 280 285

Arg Pro Thr Asp Gly Val Asp Ile Tyr Phe Gly Met Pro Gly Glu Ile
290 295 300

Ser Glu His Glu Gly Phe Leu Arg Ala Lys Met Asp Leu Glu Glu Arg
305 310 315 320

Arg Met Arg Gln Ile Asn Glu Val Met Arg Glu Trp Ala Met Ala Asp
325 330 335

Asn Gln Ser Lys Asn Leu Pro Lys Ala Asp Arg Gln Ala Leu Asn Glu
340 345 350

His Phe Gln Ser Ile Leu Gln Thr Leu Glu Glu Gln Val Ser Gly Glu
355 360 365

Arg Gln Arg Leu Val Glu Thr His Ala Thr Arg Val Ile Ala Leu Ile
370 375 380

Asn Asp Gln Arg Arg Ala Ala Leu Glu Gly Phe Leu Ala Ala Leu Gln
385 390 395 400

Ala Asp Pro Pro Gln Ala Glu Arg Val Leu Leu Ala Leu Arg Arg Tyr
405 410 415

Leu Arg Ala Glu Gln Lys Glu Gln Arg His Thr Leu Arg His Tyr Gln
420 425 430

His Val Ala Ala Val Asp Pro Glu Lys Ala Gln Gln Met Arg Phe Gln
435 440 445

Val His Thr His Leu Gln Val Ile Glu Glu Arg Val Asn Gln Ser Leu
450 455 460

Gly Leu Leu Asp Gln Asn Pro His Leu Ala Gln Glu Leu Arg Pro Gln
465 470 475 480

Ile Gln Glu Leu Leu His Ser Glu His Leu Gly Pro Ser Glu Leu Glu
485 490 495

Ala Pro Ala Pro Gly Gly Ser Ser Glu Asp Lys Gly Gly Leu Gln Pro
500 505 510

Pro Asp Ser Lys Asp Asp Thr Pro Met Thr Leu Pro Lys Gly Ser Thr
515 520 525

Glu Gln Asp Ala Ala Ser Pro Glu Lys Glu Lys Met Asn Pro Leu Glu
530 535 540

Gln Tyr Glu Arg Lys Val Asn Ala Ser Val Pro Arg Gly Phe Pro Phe
545 550 555 560

His Ser Ser Glu Ile Gln Arg Asp Glu Leu Ala Pro Ala Gly Thr Gly
565 570 575

Val Ser Arg Glu Ala Val Ser Gly Leu Leu Ile Met Gly Ala Gly Gly
580 585 590

Gly Ser Leu Ile Val Leu Ser Met Leu Leu Leu Arg Arg Lys Lys Pro
595 600 605

Tyr Gly Ala Ile Ser His Gly Val Val Glu Val Asp Pro Met Leu Thr
610 615 620

Leu Glu Glu Gln Gln Leu Arg Glu Leu Gln Arg His Gly Tyr Glu Asn
625 630 635 640

Pro Thr Tyr Arg Phe Leu Glu Glu Arg Pro
645 650

<210> 188
<211> 651
<212> PRT
<213> Homo sapiens

<400> 188

Met Asn Lys Leu Arg Gln Ser Phe Arg Arg Lys Lys Asp Val Tyr Val
1 5 10 15

Pro Glu Ala Ser Arg Pro His Gln Trp Gln Thr Asp Glu Glu Gly Val
20 25 30

Arg Thr Gly Lys Cys Ser Phe Pro Val Lys Tyr Leu Gly His Val Glu
35 40 45

Val Asp Glu Ser Arg Gly Met His Ile Cys Glu Asp Ala Val Lys Arg
50 55 60

Leu Lys Ala Glu Arg Lys Phe Phe Lys Gly Phe Phe Gly Lys Thr Gly
65 70 75 80

Lys Lys Ala Val Lys Ala Val Leu Trp Val Ser Ala Asp Gly Leu Arg
85 90 95

Val Val Asp Glu Lys Thr Lys Asp Leu Ile Val Asp Gln Thr Ile Glu
100 105 110

Lys Val Ser Phe Cys Ala Pro Asp Arg Asn Phe Asp Arg Ala Phe Ser
115 120 125

Tyr Ile Cys Arg Asp Gly Thr Thr Arg Arg Trp Ile Cys His Cys Phe
130 135 140

Met Ala Val Lys Asp Thr Gly Glu Arg Leu Ser His Ala Val Gly Cys
145 150 155 160

Ala Phe Ala Ala Cys Leu Glu Arg Lys Gln Lys Arg Glu Lys Glu Cys
165 170 175

Gly Val Thr Ala Thr Phe Asp Ala Ser Arg Thr Thr Phe Thr Arg Glu
180 185 190

Gly Ser Phe Arg Val Thr Thr Ala Thr Glu Gln Ala Glu Arg Glu Glu
195 200 205

Ile Met Lys Gln Met Gln Asp Ala Lys Lys Ala Glu Thr Asp Lys Ile
210 215 220

Val Val Gly Ser Ser Val Ala Pro Gly Asn Thr Ala Pro Ser Pro Ser
225 230 235 240

Ser Pro Thr Ser Pro Thr Ser Asp Ala Thr Thr Ser Leu Glu Met Asn
245 250 255

Asn Pro His Ala Ile Pro Arg Arg His Ala Pro Ile Glu Gln Leu Ala
260 265 270

Arg Gln Gly Ser Phe Arg Gly Phe Pro Ala Leu Ser Gln Lys Met Ser
275 280 285

Pro Phe Lys Arg Gln Leu Ser Leu Arg Ile Asn Glu Leu Pro Ser Thr
290 295 300

Met Gln Arg Lys Thr Asp Phe Pro Ile Lys Asn Ala Val Pro Glu Val
305 310 315 320

Glu Gly Glu Ala Glu Ser Ile Ser Ser Leu Cys Ser Gln Ile Thr Asn
325 330 335

Ala Phe Ser Thr Pro Glu Asp Pro Phe Ser Ser Ala Pro Met Thr Lys
340 345 350

Pro Val Thr Val Val Ala Pro Gln Ser Pro Thr Phe Gln Ala Asn Gly
355 360 365

Thr Asp Ser Ala Phe His Val Leu Ala Lys Pro Ala His Thr Ala Leu
370 375 380

Ala Pro Val Ala Met Pro Val Arg Glu Thr Asn Pro Trp Ala His Ala
385 390 395 400

Pro Asp Ala Ala Asn Lys Glu Ile Ala Ala Thr Cys Ser Gly Thr Glu
405 410 415

Trp Gly Gln Ser Ser Gly Ala Ala Ser Pro Gly Leu Phe Gln Ala Gly
420 425 430

His Arg Arg Thr Pro Ser Glu Ala Asp Arg Trp Leu Glu Glu Val Ser
435 440 445

Lys Ser Val Arg Ala Gln Gln Pro Gln Ala Ser Ala Ala Pro Leu Gln
450 455 460

Pro Val Leu Gln Pro Pro Pro Pro Thr Ala Ile Ser Gln Pro Ala Ser
465 470 475 480

Pro Phe Gln Gly Asn Ala Phe Leu Thr Ser Gln Pro Val Pro Val Gly
485 490 495

Val Val Pro Ala Leu Gln Pro Ala Phe Val Pro Ala Gln Ser Tyr Pro
500 505 510

Val Ala Asn Gly Met Pro Tyr Pro Ala Pro Asn Val Pro Val Val Gly
515 520 525

Ile Thr Pro Ser Gln Met Val Ala Asn Val Phe Gly Thr Ala Gly His
530 535 540

Pro Gln Ala Ala His Pro His Gln Ser Pro Ser Leu Val Arg Gln Gln
545 550 555 560

Thr Phe Pro His Tyr Glu Ala Ser Ser Ala Thr Thr Ser Pro Phe Phe
565 570 575

Lys Pro Pro Ala Gln His Leu Asn Gly Ser Ala Ala Phe Asn Gly Val
580 585 590

Asp Asp Gly Arg Leu Ala Ser Ala Asp Arg His Thr Glu Val Pro Thr
595 600 605

Gly Thr Cys Pro Val Asp Pro Phe Glu Ala Gln Trp Ala Ala Leu Glu
610 615 620

Asn Lys Ser Lys Gln Arg Thr Asn Pro Ser Pro Thr Asn Pro Phe Ser
625 630 635 640

Ser Asp Leu Gln Lys Thr Phe Glu Ile Glu Leu
645 650

<210> 189

<211> 803
<212> PRT
<213> Homo sapi ens

<400> 189

Met Asn Ser Ala Glu Gln Thr Val Thr Trp Leu Ile Thr Leu Gly Val
1 5 10 15

Leu Glu Ser Pro Lys Lys Thr Ile Ser Asp Pro Glu Gly Phe Leu Gln
20 25 30

Ala Ser Leu Lys Asp Gly Val Val Leu Cys Arg Leu Leu Glu Arg Leu
35 40 45

Leu Pro Gly Thr Ile Glu Lys Val Tyr Pro Glu Pro Arg Ser Glu Ser
50 55 60

Glu Cys Leu Ser Asn Ile Arg Glu Phe Leu Arg Gly Cys Gly Ala Ser
65 70 75 80

Leu Arg Leu Glu Leu Leu Phe Pro Pro Ser Gln Pro Pro Gln His Leu
85 90 95

Val Thr Thr Ile Leu Leu Ser Ala Ser Thr Phe Asp Ala Asn Asp Leu
100 105 110

Tyr Gln Gly Gln Asn Phe Asn Lys Val Leu Ser Ser Leu Val Thr Leu
115 120 125

Asn Lys Val Thr Ala Asp Ile Gly Leu Gly Ser Asp Ser Val Cys Ala
130 135 140

Arg Pro Ser Ser His Arg Ile Lys Ser Phe Asp Ser Leu Gly Ser Gln
145 150 155 160

Ser Leu His Thr Arg Thr Ser Lys Leu Phe Gln Gly Gln Tyr Arg Ser
165 170 175

Leu Asp Met Thr Asp Asn Ser Asn Asn Gln Leu Val Val Arg Ala Lys
180 185 190

Phe Asn Phe Gln Gln Thr Asn Glu Asp Glu Leu Ser Phe Ser Lys Gly
195 200 205

Asp Val Ile His Val Thr Arg Val Glu Glu Gly Gly Trp Trp Glu Gly
210 215 220

Thr Leu Asn Gly Arg Thr Gly Trp Phe Pro Ser Asn Tyr Val Arg Glu
225 230 235 240

Val Lys Ala Ser Glu Lys Pro Val Ser Pro Lys Ser Gly Thr Leu Lys

245 250 255

Ser Pro Pro Lys Gly Phe Asp Thr Thr Ala Ile Asn Lys Ser Tyr Tyr
260 265 270

Asn Val Val Leu Gln Asn Ile Leu Glu Thr Glu Asn Glu Tyr Ser Lys
275 280 285

Glu Leu Gln Thr Val Leu Ser Thr Tyr Leu Arg Pro Leu Gln Thr Ser
290 295 300

Glu Lys Leu Ser Ser Ala Asn Ile Ser Tyr Leu Met Gly Asn Leu Glu
305 310 315 320

Glu Ile Cys Ser Phe Gln Gln Met Leu Val Gln Ser Leu Glu Glu Cys
325 330 335

Thr Lys Leu Pro Glu Ala Gln Gln Arg Val Gly Gly Cys Phe Leu Asn
340 345 350

Leu Met Pro Gln Met Lys Thr Leu Tyr Leu Thr Tyr Cys Ala Asn His
355 360 365

Pro Ser Ala Val Asn Val Leu Thr Glu His Ser Glu Glu Leu Gly Glu
370 375 380

Phe Met Glu Thr Lys Gly Ala Ser Ser Pro Gly Ile Leu Val Leu Thr
385 390 395 400

Thr Gly Leu Ser Lys Pro Phe Met Arg Leu Asp Lys Tyr Pro Thr Leu
405 410 415

Leu Lys Glu Leu Glu Arg His Met Glu Asp Tyr His Thr Asp Arg Gln
420 425 430

Asp Ile Gln Lys Ser Met Ala Ala Phe Lys Asn Leu Ser Ala Gln Cys
435 440 445

Gln Glu Val Arg Lys Arg Lys Glu Leu Glu Leu Gln Ile Leu Thr Glu
450 455 460

Ala Ile Arg Asn Trp Glu Gly Asp Asp Ile Lys Thr Leu Gly Asn Val
465 470 475 480

Thr Tyr Met Ser Gln Val Leu Ile Gln Cys Ala Gly Ser Glu Glu Lys
485 490 495

Asn Glu Arg Tyr Leu Leu Leu Phe Pro Asn Val Leu Leu Met Leu Ser
500 505 510

Ala Ser Pro Arg Met Ser Gly Phe Ile Tyr Gln Gly Lys Leu Pro Thr

515 520 525

Thr Gly Met Thr Ile Thr Lys Leu Glu Asp Ser Glu Asn His Arg Asn
530 535 540

Ala Phe Glu Ile Ser Gly Ser Met Ile Glu Arg Ile Leu Val Ser Cys
545 550 555 560

Asn Asn Gln Gln Asp Leu Gln Glu Trp Val Glu His Leu Gln Lys Gln
565 570 575

Thr Lys Val Thr Ser Val Gly Asn Pro Thr Ile Lys Pro His Ser Val
580 585 590

Pro Ser His Thr Leu Pro Ser His Pro Val Thr Pro Ser Ser Lys His
595 600 605

Ala Asp Ser Lys Pro Ala Pro Leu Thr Pro Ala Tyr His Thr Leu Pro
610 615 620

His Pro Ser His His Gly Thr Pro His Thr Thr Ile Asn Trp Gly Pro
625 630 635 640

Leu Glu Pro Pro Lys Thr Pro Lys Pro Trp Ser Leu Ser Cys Leu Arg
645 650 655

Pro Ala Pro Pro Leu Arg Pro Ser Ala Ala Leu Cys Tyr Lys Glu Asp
660 665 670

Leu Ser Lys Ser Pro Lys Thr Met Lys Lys Leu Leu Pro Lys Arg Lys
675 680 685

Pro Glu Arg Lys Pro Ser Asp Glu Glu Phe Ala Ser Arg Lys Ser Thr
690 695 700

Ala Ala Leu Glu Glu Asp Ala Gln Ile Leu Lys Val Ile Glu Ala Tyr
705 710 715 720

Cys Thr Ser Ala Lys Thr Arg Gln Thr Leu Asn Ser Thr Trp Gln Gly
725 730 735

Thr Asp Leu Met His Asn His Val Leu Ala Asp Asp Asp Gln Pro Ser
740 745 750

Leu Asp Ser Leu Gly Arg Arg Ser Ser Leu Ser Arg Leu Glu Pro Ser
755 760 765

Asp Leu Ser Glu Asp Ser Asp Tyr Asp Ser Ile Trp Thr Ala His Ser
770 775 780

Tyr Arg Met Gly Ser Thr Ser Arg Lys Ser Cys Cys Ser Tyr Ile Ser

785 790 795 800

His Gln Asn

<210> 190
<211> 353
<212> PRT
<213> Homo sapiens

<400> 190

Met Ala Cys Cys Leu Ser Glu Glu Ala Lys Glu Ala Arg Arg Ile Asn
1 5 10 15

Asp Glu Ile Glu Arg Gln Leu Arg Arg Asp Lys Arg Asp Ala Arg Arg
20 25 30

Glu Leu Lys Leu Leu Leu Leu Gly Thr Gly Glu Ser Gly Lys Ser Thr
35 40 45

Phe Ile Lys Gln Met Arg Ile Ile His Gly Ser Gly Tyr Ser Asp Glu
50 55 60

Asp Lys Arg Gly Phe Thr Lys Leu Val Tyr Gln Asn Ile Phe Thr Ala
65 70 75 80

Met Gln Ala Met Ile Arg Ala Met Asp Thr Leu Lys Ile Pro Tyr Lys
85 90 95

Tyr Glu His Asn Lys Ala His Ala Gln Leu Val Arg Glu Val Asp Val
100 105 110

Glu Lys Val Ser Ala Phe Glu Asn Pro Tyr Val Asp Ala Ile Lys Ser
115 120 125

Leu Trp Asn Asp Pro Gly Ile Gln Glu Cys Tyr Asp Arg Arg Arg Glu
130 135 140

Tyr Gln Leu Ser Asp Ser Thr Lys Tyr Tyr Leu Asn Asp Leu Asp Arg
145 150 155 160

Val Ala Asp Pro Ala Tyr Leu Pro Thr Gln Gln Asp Val Leu Arg Val
165 170 175

Arg Val Pro Thr Thr Gly Ile Ile Glu Tyr Pro Phe Asp Leu Gln Ser
180 185 190

Val Ile Phe Arg Met Val Asp Val Gly Gly Gln Arg Ser Glu Arg Arg
195 200 205

Lys Trp Ile His Cys Phe Glu Asn Val Thr Ser Ile Met Phe Leu Val
210 215 220

Ala Leu Ser Glu Tyr Asp Gln Val Leu Val Glu Ser Asp Asn Glu Asn
225 230 235 240

Arg Met Glu Glu Ser Lys Ala Leu Phe Arg Thr Ile Ile Thr Tyr Pro
245 250 255

Trp Phe Gln Asn Ser Ser Val Ile Leu Phe Leu Asn Lys Lys Asp Leu
260 265 270

Leu Glu Glu Lys Ile Met Tyr Ser His Leu Val Asp Tyr Phe Pro Glu
275 280 285

Tyr Asp Gly Pro Gln Arg Asp Ala Gln Ala Ala Arg Glu Phe Ile Leu
290 295 300

Lys Met Phe Val Asp Leu Asn Pro Asp Ser Asp Lys Ile Ile Tyr Ser
305 310 315 320

His Phe Thr Cys Ala Thr Asp Thr Glu Asn Ile Arg Phe Val Phe Ala
325 330 335

Ala Val Lys Asp Thr Ile Leu Gln Leu Asn Leu Lys Glu Tyr Asn Leu
340 345 350

Val

<210> 191
<211> 394
<212> PRT
<213> Home sapi ens

<400> 191

Met Ala Leu Leu Asp Leu Ala Leu Glu Gly Met Ala Val Phe Gly Phe
1 5 10 15

Val Leu Phe Leu Val Leu Trp Leu Met His Phe Met Ala Ile Ile Tyr
20 25 30

Thr Arg Leu His Leu Asn Lys Lys Ala Thr Asp Lys Gln Pro Tyr Ser
35 40 45

Lys Leu Pro Gly Val Ser Leu Leu Lys Pro Leu Lys Gly Val Asp Pro
50 55 60

Asn Leu Ile Asn Asn Leu Glu Thr Phe Phe Glu Leu Asp Tyr Pro Lys
65 70 75 80

Tyr Glu Val Leu Leu Cys Val Gln Asp His Asp Asp Pro Ala Ile Asp
85 90 95

Val Cys Lys Lys Leu Leu Gly Lys Tyr Pro Asn Val Asp Ala Arg Leu
100 105 110

Phe Ile Gly Gly Lys Lys Val Gly Ile Asn Pro Lys Ile Asn Asn Leu
115 120 125

Met Pro Gly Tyr Glu Val Ala Lys Tyr Asp Leu Ile Trp Ile Cys Asp
130 135 140

Ser Gly Ile Arg Val Ile Pro Asp Thr Leu Thr Asp Met Val Asn Gln
145 150 155 160

Met Thr Glu Lys Val Gly Leu Val His Gly Leu Pro Tyr Val Ala Asp
165 170 175

Arg Gln Gly Phe Ala Ala Thr Leu Glu Gln Val Tyr Phe Gly Thr Ser
180 185 190

His Pro Arg Tyr Tyr Ile Ser Ala Asn Val Thr Gly Phe Lys Cys Val
195 200 205

Thr Gly Met Ser Cys Leu Met Arg Lys Asp Val Leu Asp Gln Ala Gly
210 215 220

Gly Leu Ile Ala Phe Ala Gln Tyr Ile Ala Glu Asp Tyr Phe Met Ala
225 230 235 240

Lys Ala Ile Ala Asp Arg Gly Trp Arg Phe Ala Met Ser Thr Gln Val
245 250 255

Ala Met Gln Asn Ser Gly Ser Tyr Ser Ile Ser Gln Phe Gln Ser Arg
260 265 270

Met Ile Arg Trp Thr Lys Leu Arg Ile Asn Met Leu Pro Ala Thr Ile
275 280 285

Ile Cys Glu Pro Ile Ser Glu Cys Phe Val Ala Ser Leu Ile Ile Gly
290 295 300

Trp Ala Ala His His Val Phe Arg Trp Asp Ile Met Val Phe Phe Met
305 310 315 320

Cys His Cys Leu Ala Trp Phe Ile Phe Asp Tyr Ile Gln Leu Arg Gly
325 330 335

Val Gln Gly Gly Thr Leu Cys Phe Ser Lys Leu Asp Tyr Ala Val Ala
340 345 350

Trp Phe Ile Arg Glu Ser Met Thr Ile Tyr Ile Phe Leu Ser Ala Leu
355 360 365

Trp Asp Pro Thr Ile Ser Trp Arg Thr Gly Arg Tyr Arg Leu Arg Cys
370 375 380

Gly Gly Thr Ala Glu Glu Ile Leu Asp Val
385 390

<210> 192
<211> 660
<212> PRT
<213> Homo sapiens

<400> 192

Met Glu Ala Leu Met Ala Arg Gly Ala Leu Thr Gly Pro Leu Arg Ala
1 5 10 15

Leu Cys Leu Leu Gly Cys Leu Leu Ser His Ala Ala Ala Ala Pro Ser
20 25 30

Pro Ile Ile Lys Phe Pro Gly Asp Val Ala Pro Lys Thr Asp Lys Glu
35 40 45

Leu Ala Val Gln Tyr Leu Asn Thr Phe Tyr Gly Cys Pro Lys Glu Ser
50 55 60

Cys Asn Leu Phe Val Leu Lys Asp Thr Leu Lys Lys Met Gln Lys Phe
65 70 75 80

Phe Gly Leu Pro Gln Thr Gly Asp Leu Asp Gln Asn Thr Ile Glu Thr
85 90 95

Met Arg Lys Pro Arg Cys Gly Asn Pro Asp Val Ala Asn Tyr Asn Phe
100 105 110

Phe Pro Arg Lys Pro Lys Trp Asp Lys Asn Gln Ile Thr Tyr Arg Ile
115 120 125

Ile Gly Tyr Thr Pro Asp Leu Asp Pro Glu Thr Val Asp Asp Ala Phe
130 135 140

Ala Arg Ala Phe Gln Val Trp Ser Asp Val Thr Pro Leu Arg Phe Ser
145 150 155 160

Arg Ile His Asp Gly Glu Ala Asp Ile Met Ile Asn Phe Gly Arg Trp
165 170 175

Glu His Gly Asp Gly Tyr Pro Phe Asp Gly Lys Asp Gly Leu Leu Ala
180 185 190

His Ala Phe Ala Pro Gly Thr Gly Val Gly Gly Asp Ser His Phe Asp
195 200 205

Asp Asp Glu Leu Trp Thr Leu Gly Glu Gly Gln Val Val Arg Val Lys
210 215 220

Tyr Gly Asn Ala Asp Gly Glu Tyr Cys Lys Phe Pro Phe Leu Phe Asn
225 230 235 240

Gly Lys Glu Tyr Asn Ser Cys Thr Asp Thr Gly Arg Ser Asp Gly Phe
245 250 255

Leu Trp Cys Ser Thr Thr Tyr Asn Phe Glu Lys Asp Gly Lys Tyr Gly
260 265 270

Phe Cys Pro His Glu Ala Leu Phe Thr Met Gly Gly Asn Ala Glu Gly
275 280 285

Gln Pro Cys Lys Phe Pro Phe Arg Phe Gln Gly Thr Ser Tyr Asp Ser
290 295 300

Cys Thr Thr Glu Gly Arg Thr Asp Gly Tyr Arg Trp Cys Gly Thr Thr
305 310 315 320

Glu Asp Tyr Asp Arg Asp Lys Lys Tyr Gly Phe Cys Pro Glu Thr Ala
325 330 335

Met Ser Thr Val Gly Gly Asn Ser Glu Gly Ala Pro Cys Val Phe Pro
340 345 350

Phe Thr Phe Leu Gly Asn Lys Tyr Glu Ser Cys Thr Ser Ala Gly Arg
355 360 365

Ser Asp Gly Lys Met Trp Cys Ala Thr Thr Ala Asn Tyr Asp Asp Asp
370 375 380

Arg Lys Trp Gly Phe Cys Pro Asp Gln Gly Tyr Ser Leu Phe Leu Val
385 390 395 400

Ala Ala His Glu Phe Gly His Ala Met Gly Leu Glu His Ser Gln Asp
405 410 415

Pro Gly Ala Leu Met Ala Pro Ile Tyr Thr Tyr Thr Lys Asn Phe Arg
420 425 430

Leu Ser Gln Asp Asp Ile Lys Gly Ile Gln Glu Leu Tyr Gly Ala Ser
435 440 445

Pro Asp Ile Asp Leu Gly Thr Gly Pro Thr Pro Thr Leu Gly Pro Val
450 455 460

Thr Pro Glu Ile Cys Lys Gln Asp Ile Val Phe Asp Gly Ile Ala Gln
465 470 475 480

Ile Arg Gly Glu Ile Phe Phe Phe Lys Asp Arg Phe Ile Trp Arg Thr
485 490 495

Val Thr Pro Arg Asp Lys Pro Met Gly Pro Leu Leu Val Ala Thr Phe
500 505 510

Trp Pro Glu Leu Pro Glu Lys Ile Asp Ala Val Tyr Glu Ala Pro Gln
515 520 525

Glu Glu Lys Ala Val Phe Phe Ala Gly Asn Glu Tyr Trp Ile Tyr Ser
530 535 540

Ala Ser Thr Leu Glu Arg Gly Tyr Pro Lys Pro Leu Thr Ser Leu Gly
545 550 555 560

Leu Pro Pro Asp Val Gln Arg Val Asp Ala Ala Phe Asn Trp Ser Lys
565 570 575

Asn Lys Lys Thr Tyr Ile Phe Ala Gly Asp Lys Phe Trp Arg Tyr Asn
580 585 590

Glu Val Lys Lys Lys Met Asp Pro Gly Phe Pro Lys Leu Ile Ala Asp
595 600 605

Ala Trp Asn Ala Ile Pro Asp Asn Leu Asp Ala Val Val Asp Leu Gln
610 615 620

Gly Gly Gly His Ser Tyr Phe Phe Lys Gly Ala Tyr Tyr Leu Lys Leu
625 630 635 640

Glu Asn Gln Ser Leu Lys Ser Val Lys Phe Gly Ser Ile Lys Ser Asp
645 650 655

Trp Leu Gly Cys
660

<210> 193
<211> 260
<212> PRT
<213> Homo sapiens

<400> 193

Met Ala Arg Pro His Pro Trp Trp Leu Cys Val Leu Gly Thr Leu Val
1 5 10 15

Gly Leu Ser Ala Thr Pro Ala Pro Lys Ser Cys Pro Glu Arg His Tyr
20 25 30

Trp Ala Gln Gly Lys Leu Cys Cys Gln Met Cys Glu Pro Gly Thr Phe
35 40 45

Leu Val Lys Asp Cys Asp Gln His Arg Lys Ala Ala Gln Cys Asp Pro
50 55 60

Cys Ile Pro Gly Val Ser Phe Ser Pro Asp His His Thr Arg Pro His
65 70 75 80

Cys Glu Ser Cys Arg His Cys Asn Ser Gly Leu Leu Val Arg Asn Cys
85 90 95

Thr Ile Thr Ala Asn Ala Glu Cys Ala Cys Arg Asn Gly Trp Gln Cys
100 105 110

Arg Asp Lys Glu Cys Thr Glu Cys Asp Pro Leu Pro Asn Pro Ser Leu
115 120 125

Thr Ala Arg Ser Ser Gln Ala Leu Ser Pro His Pro Gln Pro Thr His
130 135 140

Leu Pro Tyr Val Ser Glu Met Leu Glu Ala Arg Thr Ala Gly His Met
145 150 155 160

Gln Thr Leu Ala Asp Phe Arg Gln Leu Pro Ala Arg Thr Leu Ser Thr
165 170 175

His Trp Pro Pro Gln Arg Ser Leu Cys Ser Ser Asp Phe Ile Arg Ile
180 185 190

Leu Val Ile Phe Ser Gly Met Phe Leu Val Phe Thr Leu Ala Gly Ala
195 200 205

Leu Phe Leu His Gln Arg Arg Lys Tyr Arg Ser Asn Lys Gly Glu Ser
210 215 220

Pro Val Glu Pro Ala Glu Pro Cys Arg Tyr Ser Cys Pro Arg Glu Glu
225 230 235 240

Glu Gly Ser Thr Ile Pro Ile Gln Glu Asp Tyr Arg Lys Pro Glu Pro
245 250 255

Ala Cys Ser Pro
260

<210> 194
<211> 885
<212> PRT
<213> Homo sapi ens

<400> 194

Met Gly Cys Ala Pro Ser Ile His Val Ser Gln Ser Gly Val Ile Tyr

1 5 10 15

Cys Arg Asp Ser Asp Glu Ser Ser Ser Pro Arg Gln Thr Thr Ser Val
20 25 30

Ser Gln Gly Pro Ala Ala Pro Leu Pro Gly Leu Phe Val Gln Thr Asp
35 40 45

Ala Ala Asp Ala Ile Pro Pro Ser Arg Ala Ser Gly Pro Pro Ser Val
50 55 60

Ala Arg Val Arg Arg Ala Arg Thr Glu Leu Gly Ser Gly Ser Ser Ala
65 70 75 80

Gly Ser Ala Ala Pro Ala Ala Thr Thr Ser Arg Gly Arg Arg Arg His
85 90 95

Cys Cys Ser Ser Ala Glu Ala Glu Thr Gln Thr Cys Tyr Thr Ser Val
100 105 110

Lys Gln Val Ser Ser Ala Glu Val Arg Ile Gly Pro Met Arg Leu Thr
115 120 125

Gln Asp Pro Ile Gln Val Leu Leu Ile Phe Ala Lys Glu Asp Ser Gln
130 135 140

Ser Asp Gly Phe Trp Trp Ala Cys Asp Arg Ala Gly Tyr Arg Cys Asn
145 150 155 160

Ile Ala Arg Thr Pro Glu Ser Ala Leu Glu Cys Phe Leu Asp Lys His
165 170 175

His Glu Ile Ile Val Ile Asp His Arg Gln Thr Gln Asn Phe Asp Ala
180 185 190

Glu Ala Val Cys Arg Ser Ile Arg Ala Thr Asn Pro Ser Glu His Thr
195 200 205

Val Ile Leu Ala Val Val Ser Arg Val Ser Asp Asp His Glu Glu Ala
210 215 220

Ser Val Leu Pro Leu Leu His Ala Gly Phe Asn Arg Arg Phe Met Glu
225 230 235 240

Asn Ser Ser Ile Ile Ala Cys Tyr Asn Glu Leu Ile Gln Ile Glu His
245 250 255

Gly Glu Val Arg Ser Gln Phe Lys Leu Arg Ala Cys Asn Ser Val Phe
260 265 270

Thr Ala Leu Asp His Cys His Glu Ala Ile Glu Ile Thr Ser Asp Asp

275 280 285

His Val Ile Gln Tyr Val Asn Pro Ala Phe Glu Arg Met Met Gly Tyr
290 295 300

His Lys Gly Glu Leu Leu Gly Lys Glu Leu Ala Asp Leu Pro Lys Ser
305 310 315 320

Asp Lys Asn Arg Ala Asp Leu Leu Asp Thr Ile Asn Thr Cys Ile Lys
325 330 335

Lys Gly Lys Glu Trp Gln Gly Val Tyr Tyr Ala Arg Arg Lys Ser Gly
340 345 350

Asp Ser Ile Gln Gln His Val Lys Ile Thr Pro Val Ile Gly Gln Gly
355 360 365

Gly Lys Ile Arg His Phe Val Ser Leu Lys Lys Leu Cys Cys Thr Thr
370 375 380

Asp Asn Asn Lys Gln Ile His Lys Ile His Arg Asp Ser Gly Asp Asn
385 390 395 400

Ser Gln Thr Glu Pro His Ser Phe Arg Tyr Lys Asn Arg Arg Lys Glu
405 410 415

Ser Ile Asp Val Lys Ser Ile Ser Ser Arg Gly Ser Asp Ala Pro Ser
420 425 430

Leu Gln Asn Arg Arg Tyr Pro Ser Met Ala Arg Ile His Ser Met Thr
435 440 445

Ile Glu Ala Pro Ile Thr Lys Val Ile Asn Ile Ile Asn Ala Ala Gln
450 455 460

Glu Asn Ser Pro Val Thr Val Ala Glu Ala Leu Asp Arg Val Leu Glu
465 470 475 480

Ile Leu Arg Thr Thr Glu Leu Tyr Ser Pro Gln Leu Gly Thr Lys Asp
485 490 495

Glu Asp Pro His Thr Ser Asp Leu Val Gly Gly Leu Met Thr Asp Gly
500 505 510

Leu Arg Arg Leu Ser Gly Asn Glu Tyr Val Phe Thr Lys Asn Val His
515 520 525

Gln Ser His Ser His Leu Ala Met Pro Ile Thr Ile Asn Asp Val Pro
530 535 540

Pro Cys Ile Ser Gln Leu Leu Asp Asn Glu Glu Ser Trp Asp Phe Asn

545 550 555 560

Ile Phe Glu Leu Glu Ala Ile Thr His Lys Arg Pro Leu Val Tyr Leu
565 570 575

Gly Leu Lys Val Phe Ser Arg Phe Gly Val Cys Glu Phe Leu Asn Cys
580 585 590

Ser Glu Thr Thr Leu Arg Ala Trp Phe Gln Val Ile Glu Ala Asn Tyr
595 600 605

His Ser Ser Asn Ala Tyr His Asn Ser Thr His Ala Ala Asp Val Leu
610 615 620

His Ala Thr Ala Phe Phe Leu Gly Lys Glu Arg Val Lys Gly Ser Leu
625 630 635 640

Asp Gln Leu Asp Glu Val Ala Ala Leu Ile Ala Ala Thr Val His Asp
645 650 655

Val Asp His Pro Gly Arg Thr Asn Ser Phe Leu Cys Asn Ala Gly Ser
660 665 670

Glu Leu Ala Val Leu Tyr Asn Asp Thr Ala Val Leu Glu Ser His His
675 680 685

Thr Ala Leu Ala Phe Gln Leu Thr Val Lys Asp Thr Lys Cys Asn Ile
690 695 700

Phe Lys Asn Ile Asp Arg Asn His Tyr Arg Thr Leu Arg Gln Ala Ile
705 710 715 720

Ile Asp Met Val Leu Ala Thr Glu Met Thr Lys His Phe Glu His Val
725 730 735

Asn Lys Phe Val Asn Ser Ile Asn Lys Pro Met Ala Ala Glu Ile Glu
740 745 750

Gly Ser Asp Cys Glu Cys Asn Pro Ala Gly Lys Asn Phe Pro Glu Asn
755 760 765

Gln Ile Leu Ile Lys Arg Met Met Ile Lys Cys Ala Asp Val Ala Asn
770 775 780

Pro Cys Arg Pro Leu Asp Leu Cys Ile Glu Trp Ala Gly Arg Ile Ser
785 790 795 800

Glu Glu Tyr Phe Ala Gln Thr Asp Glu Glu Lys Arg Gln Gly Leu Pro
805 810 815

Val Val Met Pro Val Phe Asp Arg Asn Thr Cys Ser Ile Pro Lys Ser

820 825 830

Gln Ile Ser Phe Ile Asp Tyr Phe Ile Thr Asp Met Phe Asp Ala Trp
835 840 845

Asp Ala Phe Ala His Leu Pro Ala Leu Met Gln His Leu Ala Asp Asn
850 855 860

Tyr Lys His Trp Lys Thr Leu Asp Asp Leu Lys Cys Lys Ser Leu Arg
865 870 875 880

Leu Pro Ser Asp Ser
885

<210> 195
<211> 427
<212> PRT
<213> Homo sapiens

<400> 195

Met Phe Phe Thr Cys Gly Pro Asn Glu Ala Met Val Val Ser Gly Phe
1 5 10 15

Cys Arg Ser Pro Pro Val Met Val Ala Gly Gly Arg Val Phe Val Leu
20 25 30

Pro Cys Ile Gln Gln Ile Gln Arg Ile Ser Leu Asn Thr Leu Thr Leu
35 40 45

Asn Val Lys Ser Glu Lys Val Tyr Thr Arg His Gly Val Pro Ile Ser
50 55 60

Val Thr Gly Ile Ala Gln Val Lys Ile Gln Gly Gln Asn Lys Glu Met
65 70 75 80

Leu Ala Ala Ala Cys Gln Met Phe Leu Gly Lys Thr Glu Ala Glu Ile
85 90 95

Ala His Ile Ala Leu Glu Thr Leu Glu Gly His Gln Arg Ala Ile Met
100 105 110

Ala His Met Thr Val Glu Glu Ile Tyr Lys Asp Arg Gln Lys Phe Ser
115 120 125

Glu Gln Val Phe Lys Val Ala Ser Ser Asp Leu Val Asn Met Gly Ile
130 135 140

Ser Val Val Ser Tyr Thr Leu Lys Asp Ile His Asp Asp Gln Asp Tyr
145 150 155 160

Leu His Ser Leu Gly Lys Ala Arg Thr Ala Gln Val Gln Lys Asp Ala
165 170 175

Arg Ile Gly Glu Ala Glu Ala Lys Arg Asp Ala Gly Ile Arg Glu Ala
180 185 190

Lys Ala Lys Gln Glu Lys Val Ser Ala Gln Tyr Leu Ser Glu Ile Glu
195 200 205

Met Ala Lys Ala Gln Arg Asp Tyr Glu Leu Lys Lys Ala Ala Tyr Asp
210 215 220

Ile Glu Val Asn Thr Arg Arg Ala Gln Ala Asp Leu Ala Tyr Gln Leu
225 230 235 240

Gln Val Ala Lys Thr Lys Gln Gln Ile Glu Glu Gln Arg Val Gln Val
245 250 255

Gln Val Val Glu Arg Ala Gln Gln Val Ala Val Gln Glu Gln Glu Ile
260 265 270

Ala Arg Arg Glu Lys Glu Leu Glu Ala Arg Val Arg Lys Pro Ala Glu
275 280 285

Ala Glu Arg Tyr Lys Leu Glu Arg Leu Ala Glu Ala Glu Lys Ser Gln
290 295 300

Leu Ile Met Gln Ala Glu Ala Glu Ala Ala Ser Val Arg Met Arg Gly
305 310 315 320

Glu Ala Glu Ala Phe Ala Ile Gly Ala Arg Ala Arg Ala Glu Ala Glu
325 330 335

Gln Met Ala Lys Lys Ala Glu Ala Phe Gln Leu Tyr Gln Glu Ala Ala
340 345 350

Gln Leu Asp Met Leu Leu Glu Lys Leu Pro Gln Val Ala Glu Glu Ile
355 360 365

Ser Gly Pro Leu Thr Ser Ala Asn Lys Ile Thr Leu Val Ser Ser Gly
370 375 380

Ser Gly Thr Met Gly Ala Ala Lys Val Thr Gly Glu Val Leu Asp Ile
385 390 395 400

Leu Thr Arg Leu Pro Glu Ser Val Glu Arg Leu Thr Gly Val Ser Ile
405 410 415
0

Ser Gln Val Asn His Lys Pro Leu Arg Thr Ala
420 425

<210> 196
<211> 495
<212> PRT
<213> Homo sapiens

<400> 196

Met Pro Met Gly Ser Leu Gln Pro Leu Ala Thr Leu Tyr Leu Leu Gly
1               5                   10                  15

Met Leu Val Ala Ser Cys Leu Gly Arg Leu Ser Trp Tyr Asp Pro Asp
            20                  25                  30

Phe Gln Ala Arg Leu Thr Arg Ser Asn Ser Lys Cys Gln Gly Gln Leu
        35                  40                  45

Glu Val Tyr Leu Lys Asp Gly Trp His Met Val Cys Ser Gln Ser Trp
    50                  55                  60

Gly Arg Ser Ser Lys Gln Trp Glu Asp Pro Ser Gln Ala Ser Lys Val
65                  70                  75                  80

Cys Gln Arg Leu Asn Cys Gly Val Pro Leu Ser Leu Gly Pro Phe Leu
                85                  90                  95

Val Thr Tyr Thr Pro Gln Ser Ser Ile Ile Cys Tyr Gly Gln Leu Gly
            100                 105                 110

Ser Phe Ser Asn Cys Ser His Ser Arg Asn Asp Met Cys His Ser Leu
        115                 120                 125

Gly Leu Thr Cys Leu Glu Pro Gln Lys Thr Thr Pro Pro Thr Thr Arg
    130                 135                 140

Pro Pro Pro Thr Thr Thr Pro Glu Pro Thr Ala Pro Pro Arg Leu Gln
145                 150                 155                 160

Leu Val Ala Gln Ser Gly Gly Gln His Cys Ala Gly Val Val Glu Phe
                165                 170                 175

Tyr Ser Gly Ser Leu Gly Gly Thr Ile Ser Tyr Glu Ala Gln Asp Lys
            180                 185                 190

Thr Gln Asp Leu Glu Asn Phe Leu Cys Asn Asn Leu Gln Cys Gly Ser
        195                 200                 205

Phe Leu Lys His Leu Pro Glu Thr Glu Ala Gly Arg Ala Gln Asp Pro
    210                 215                 220

Gly Glu Pro Arg Glu His Gln Pro Leu Pro Ile Gln Trp Lys Ile Gln
225                 230                 235                 240

Asn Ser Ser Cys Thr Ser Leu Glu His Cys Phe Arg Lys Ile Lys Pro
                245                 250                 255

Gln Lys Ser Gly Arg Val Leu Ala Leu Leu Cys Ser Gly Phe Gln Pro
260 265 270

Lys Val Gln Ser Arg Leu Val Gly Gly Ser Ser Ile Cys Glu Gly Thr
275 280 285

Val Glu Val Arg Gln Gly Ala Gln Trp Ala Ala Leu Cys Asp Ser Ser
290 295 300

Ser Ala Arg Ser Ser Leu Arg Trp Glu Glu Val Cys Arg Glu Gln Gln
305 310 315 320

Cys Gly Ser Val Asn Ser Tyr Arg Val Leu Asp Ala Gly Asp Pro Thr
325 330 335

Ser Arg Gly Leu Phe Cys Pro His Gln Lys Leu Ser Gln Cys His Glu
340 345 350

Leu Trp Glu Arg Asn Ser Tyr Cys Lys Lys Val Phe Val Thr Cys Gln
355 360 365

Asp Pro Asn Pro Ala Gly Leu Ala Ala Gly Thr Val Ala Ser Ile Ile
370 375 380

Leu Ala Leu Val Leu Leu Val Val Leu Leu Val Val Cys Gly Pro Leu
385 390 395 400

Ala Tyr Lys Lys Leu Val Lys Lys Phe Arg Gln Lys Lys Gln Arg Gln
405 410 415

Trp Ile Gly Pro Thr Gly Met Asn Gln Asn Met Ser Phe His Arg Asn
420 425 430

His Thr Ala Thr Val Arg Ser His Ala Glu Asn Pro Thr Ala Ser His
435 440 445

Val Asp Asn Glu Tyr Ser Gln Pro Pro Arg Asn Ser Arg Leu Ser Ala
450 455 460

Tyr Pro Ala Leu Glu Gly Val Leu His Arg Ser Ser Met Gln Pro Asp
465 470 475 480

Asn Ser Ser Asp Ser Asp Tyr Asp Leu His Gly Ala Gln Arg Leu
485 490 495

<210> 197
<211> 540
<212> PRT
<213> Homo sapiens

<400> 197

Met Gly Thr Thr Ala Arg Ala Ala Leu Val Leu Thr Tyr Leu Ala Val
1 5 10 15

Ala Ser Ala Ala Ser Glu Gly Gly Phe Thr Ala Thr Gly Gln Arg Gln
20 25 30

Leu Arg Pro Glu His Phe Gln Glu Val Gly Tyr Ala Ala Pro Pro Ser
35 40 45

Pro Pro Leu Ser Arg Ser Leu Pro Met Asp His Pro Asp Ser Ser Gln
50 55 60

His Gly Pro Pro Phe Glu Gly Gln Ser Gln Val Gln Pro Pro Pro Ser
65 70 75 80

Gln Glu Ala Thr Pro Leu Gln Gln Glu Lys Leu Leu Pro Ala Gln Leu
85 90 95

Pro Ala Glu Lys Glu Val Gly Pro Pro Leu Pro Gln Glu Ala Val Pro
100 105 110

Leu Gln Lys Glu Leu Pro Ser Leu Gln His Pro Asn Glu Gln Lys Glu
115 120 125

Gly Thr Pro Ala Pro Phe Gly Asp Gln Ser His Pro Glu Pro Glu Ser
130 135 140

Trp Asn Ala Ala Gln His Cys Gln Gln Asp Arg Ser Gln Gly Gly Trp
145 150 155 160

Gly His Arg Leu Asp Gly Phe Pro Pro Gly Arg Pro Ser Pro Asp Asn
165 170 175

Leu Asn Gln Ile Cys Leu Pro Asn Arg Gln His Val Val Tyr Gly Pro
180 185 190

Trp Asn Leu Pro Gln Ser Ser Tyr Ser His Leu Thr Arg Gln Gly Glu
195 200 205

Thr Leu Asn Phe Leu Glu Ile Gly Tyr Ser Arg Cys Cys His Cys Arg
210 215 220

Ser His Thr Asn Arg Leu Glu Cys Ala Lys Leu Val Trp Glu Glu Ala
225 230 235 240

Met Ser Arg Phe Cys Glu Ala Glu Phe Ser Val Lys Thr Arg Pro His
245 250 255

Trp Cys Cys Thr Arg Gln Gly Glu Ala Arg Phe Ser Cys Phe Gln Glu
260 265 270

```
Glu Ala Pro Gln Pro His Tyr Gln Leu Arg Ala Cys Pro Ser His Gln
        275                 280                 285

Pro Asp Ile Ser Ser Gly Leu Glu Leu Pro Phe Pro Pro Gly Val Pro
    290                 295                 300

Thr Leu Asp Asn Ile Lys Asn Ile Cys His Leu Arg Arg Phe Arg Ser
305                 310                 315                 320

Val Pro Arg Asn Leu Pro Ala Thr Asp Pro Leu Gln Arg Glu Leu Leu
                325                 330                 335

Ala Leu Ile Gln Leu Glu Arg Glu Phe Gln Arg Cys Cys Arg Gln Gly
            340                 345                 350

Asn Asn His Thr Cys Thr Trp Lys Ala Trp Glu Asp Thr Leu Asp Lys
        355                 360                 365

Tyr Cys Asp Arg Glu Tyr Ala Val Lys Thr His His His Leu Cys Cys
    370                 375                 380

Arg His Pro Pro Ser Pro Thr Arg Asp Glu Cys Phe Ala Arg Arg Ala
385                 390                 395                 400

Pro Tyr Pro Asn Tyr Asp Arg Asp Ile Leu Thr Ile Asp Ile Gly Arg
                405                 410                 415

Val Thr Pro Asn Leu Met Gly His Leu Cys Gly Asn Gln Arg Val Leu
            420                 425                 430

Thr Lys His Lys His Ile Pro Gly Leu Ile His Asn Met Thr Ala Arg
        435                 440                 445

Cys Cys Asp Leu Pro Phe Pro Glu Gln Ala Cys Cys Ala Glu Glu Glu
    450                 455                 460

Lys Leu Thr Phe Ile Asn Asp Leu Cys Gly Pro Arg Arg Asn Ile Trp
465                 470                 475                 480

Arg Asp Pro Ala Leu Cys Cys Tyr Leu Ser Pro Gly Asp Glu Gln Val
                485                 490                 495

Asn Cys Phe Asn Ile Asn Tyr Leu Arg Asn Val Ala Leu Val Ser Gly
            500                 505                 510

Asp Thr Glu Asn Ala Lys Gly Gln Gly Glu Gln Gly Ser Thr Gly Gly
        515                 520                 525

Thr Asn Ile Ser Ser Thr Ser Glu Pro Lys Glu Glu
    530                 535                 540
```

**Description**

FIELD OF THE INVENTION

The present invention relates the use of to a composition for identifying (or diagnosing) a disease for predicting the prognosis for a subject and/or for predicting or detecting metastasis of kidney cancer, to a method for predicting or identifying the prognosis for a subject and/or metastasis of kidney cancer using the composition, and to the use of a kit for predicting the prognosis for a subject and/or metastasis of kidney cancer using the composition.

BACKGROUND OF THE INVENTION

The kidney is an important urinary organ that plays a key role in eliminating waste products from the body by filtering the blood and generating urine. The kidney is also important as an endocrine organ that produces hormones such as angiotensin, which controls the blood pressure, or erythropoietin, which is an erythropoietic factor.

Tumors that develop in the kidney are classified into: kidney cell cancer, which occurs in adults; Wilms' tumor, which occurs in children; and sarcoma, which is a rare form of tumor. Hereafter, malignant kidney cell cancer, which has the highest incidence, is referred to as "kidney cancer." In Japan, the frequency of kidney cancer development is approximately 2.5 people per 100,000 people, the male to female ratio thereof is 2 to 3:1, and men are more likely to develop kidney cancer. Among malignant urologic tumors, kidney cancer has the highest frequency after prostate cancer and bladder cancer.

As risk factors for kidney cancer, genetic factors are known. In general, smoking, excessive fat intake, and the like are known as risk factors. Also, such tumors frequently develop in long-term dialysis patients.

When the maximal diameter of the kidney tumor is not greater than 5 cm, the patients hardly have any subjective symptoms, and such tumors are often found via CT scanning at the time of physical examination. In the case of large-size tumors, hematuria, abdominal tumors, pain, or other symptoms are observed. As systemic symptoms, fever onset, weight loss, anemia, or other symptoms may occur, and erythrocytosis, elevated blood pressure, hypercalcemia, or other disease may be caused by endocrine factors. Also, spreading kidney cancer in the postcaval vein may occur in a varicose on the abdominal surface or in the testicular varicocele. Approximately 20% of kidney cancer cases are detected upon lung or bone metastasis. Kidney tumors are likely to spread in the vein and easily metastasize to other organs.

Examples of methods for detecting kidney cancer include echography, CT scanning, and angiography; however, specific biochemical markers are not yet known. Thus, examination with the use of equipment is necessary.

Also, kidney cancer can be further pathologically classified. Clear cell kidney cancer, which accounts for 90% of cases, is known to develop as a result of defects of the von-Hippel-Lindau (VHL) genes, which are cancer suppressive genes (Latif, F. et al., Science, 1993, vol. 260, pp. 1317-1320). VHL gene defects activate transcription of hypoxia-induced genes via disturbance of HIF-$\alpha$/VHF aggregation (Maxwell, P. et al., Nature, 1999, vol. 399, pp. 271-275) and also enhance the expression of genes such as VEGF and TGF$\beta$.

The main treatment for kidney cancer is surgical treatment. Regardless of the disease stage, partial or total removal of the kidney is the most common method when possible. Even if metastasis has already occurred, surgical removal of the kidney may be considered. In addition to surgical treatment, arterial embolization of the renal artery is possible. This method may be employed when surgical removal is unfeasible or before surgical removal of large tumors.

Because of the recent development of image diagnostic technologies, a very small size of kidney cancer has become detectable at an early stage. Through early-stage treatment, 90% (or more) of cancer patients can be treated. Since the treatment results for large tumors of 5 cm or greater or for metastatic tumors are poor (5-year survival rates for kidney cell cancer patients overall are approximately 50% to 60%), it is crucial to conduct high-throughput testing and diagnosis of the presence of tumors or tumor metastasis, to predict the prognosis, and to compose regimen or treatment plans.

As a method for detecting or diagnosing human kidney cancer, a method wherein differences in gene expression levels are employed is disclosed in WO 2005/024603. As proteins that are known to increase in human kidney cancer, PDE8B (WO 2004/042077), FLOT1 (WO 2004/048933), CD5 (Nagase, Y. et al., the Japanese Journal of Urology, 1991, vol. 82, pp. 1781-1789), and ECM1 (US Patent No. 6,303,765) are known, in addition to MMP2, which is considered to enhance cancer cell motility by degrading the extracellular matrix (Lein, M. et al., International Journal of Cancer, 2000, vol. 85, pp. 801-804), and TNFRSF7, whose expression level is known to increase in the case of renal disorder (Nakatsuji, T., Clinical and Experimental Medicine, 2003, vol. 2, pp. 192-196). Their specificities, however, are not relatively high, and a highly sensitive method for detecting kidney cancer based only on the expression levels of such proteins has not yet been put to clinical applications. Further, MCM3AP (JP Patent Publication (kohyo) No. 2005-520536 (A)), KRT19 (JP Patent Publication (kohyo) No. 2005-507997 (A)), SLK4 (WO 2002/06339), C5P1 (JP Patent Publication (kohyo) No. 2004-518402 (A)), FGF2 (Miyake, H. et al., 1996, Cancer Research, vol. 56, pp. 2440-2445), MMP14 (Kitagawa, Y. et al., 1999, Journal of Urology, vol. 162, pp. 905-909), ERBB2 (Freeman, M. R. et al., 1989, Cancer Research, vol.

49, pp. 6221-6225), and the like are deduced as tumor-associated markers for cancer, including kidney cancer.

DISCLOSURE OF THE INVENTION

Problems to be Solved by the Invention

As described above, gene expression markers for kidney cancer are well known; however, diagnostic markers capable of predicting metastasis and markers for predicting the prognosis are hardly known. Since existing markers have poor specificity and/or sensitivity and an effective method for detecting such markers from biological samples has not yet been established, such existing markers have not yet been clinically used in general. Thus, markers for kidney cancer with higher specificity and sensitivity have been desired.

If a plurality of effective markers for kidney cancer are discovered, remarkable progress can be expected in the treatment of kidney cancer or metastatic kidney cancer. In particular, many metastatic kidney cancer patients have poor prognoses. If the occurrence of metastasis is discovered at the time of diagnosis, more effective treatment can be provided to improve prognosis.

An object of the invention is to provide a composition, kit, or DNA chip for identifying a disease, which is useful for diagnosing kidney cancer, for diagnosing the metastasis of kidney cancer (or prognosis), and for treating kidney cancer.

Another object of the invention is to provide a method for detecting, identifying, or predicting the presence or metastasis of kidney cancer using the composition, kit, or DNA chip.

Means for Solving Problems

Markers may be searched for by a method wherein the prognosis for kidney cancer patients is observed and the gene expression levels or the protein expression levels in the kidney cancer cells from patients with good prognoses and in the kidney cancer cells from patients with poor prognoses or the amounts of metabolites of such cells are compared by some means; a method wherein the amounts of genes, proteins, or metabolites in body fluids and tissues containing the kidney cancer cells from patients with good prognoses or tissues containing the kidney cancer cells from patients with poor prognoses; or other methods.

In recent years, analysis of gene expression levels using DNA arrays has been commonly used as a method for searching for such markers. DNA arrays comprise probes having nucleotide sequences corresponding to several hundreds to several tens of thousands of genes immobilized thereon. By applying analyte samples to DNA arrays, the genes in the samples bind to probes, and the amount of binding is determined by some means. Thus, the amount of genes in the analyte samples can be determined. The genes corresponding to the probes to be immobilized on the DNA arrays can be freely selected, kidney cancer cells from patients with good prognoses and poor prognoses can be used as analyte samples, and the gene expression levels in the samples may be compared. Thus, genes that can become markers for predicting the metastasis of kidney cancer and/or the prognosis for kidney cancer patients can be deduced.

In order to overcome the aforementioned problem, we have analyzed, with the use of DNA arrays, the gene expression levels in the kidney cancer tissue from patients with good prognoses and in the kidney cancer tissue from patients with poor prognoses. Further, we have selected the genes that can discriminate the kidney cancer tissue from a patient with good prognosis from the kidney cancer tissue from a patient with poor prognosis, and/or that can determine the presence or absence of metastasis of kidney cancer by using the effect of the gene expression levels determined using DNA arrays on changes in survival rates for patients with the elapse of time after the surgery as an indication. This has led to the completion of the present invention.

<u>Summary of the Invention</u>

The present invention includes the following characteristics.

(1) Use of a composition in vitro for detecting, identifying, or predicting the metastasis of kidney cancer in a subject, or for predicting the prognosis of a subject with kidney cancer, the composition comprising one or more probes selected from the group of polynucleotides consisting of:

(a) a polynucleotide consisting of the nucleotide sequence as shown in SEQ ID NO: 1, mutants thereof having at least 95% identity with said nucleotide sequence, or fragments of said nucleotide sequence as shown in SEQ ID NO: 1 comprising at least 15 continuous nucleotides thereof;
(b) a polynucleotide comprising the nucleotide sequence as shown in SEQ ID NO: 1;
(c) a polynucleotide consisting of a nucleotide sequence complementary to the nucleotide sequence as shown in SEQ ID NO: 1, mutants thereof having at least 95% identity with said nucleotide sequence, or fragments of said nucleotide sequence as shown in SEQ ID NO: 1 comprising at least 15 continuous nucleotides thereof; and
(d) a polynucleotide comprising a nucleotide sequence complementary to the nucleotide sequence as shown in SEQ ID NO: 1.

(2) The use of the composition according to (1), wherein the composition further comprises at least one probe selected from the group of polynucleotides consisting of:

(a) polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(b) polynucleotides comprising nucleotide sequences as shown in SEQ ID NOS: 2 to 10, 12 to 20, and 22 to 47;
(c) polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof; and
(d) polynucleotides comprising nucleotide sequences complementary to nucleotide sequences as shown in SEQ ID NOS: 2 to 10, 12 to 20, and 22 to 47.

(3) The use of a composition according to (1) or (2), wherein the polynucleotide is DNA or RNA.

(4) The use of a composition according to any one of (1) to (3), wherein the fragments are each a polynucleotide comprising at least 60 continuous nucleotides.

(5). The use of a composition according to (1), wherein the fragments are each a polynucleotide comprising the nucleotide sequence as shown in SEQ ID NO: 51 in the nucleotide sequence as shown in SEQ ID NO: 1 or a polynucleotide comprising a nucleotide sequence complementary to the nucleotide sequence as shown in SEQ ID NO: 51 in the nucleotide sequence as shown in SEQ ID NO: 1.

(6). The use of a composition according to (2), wherein the fragments are each a polynucleotide comprising the nucleotide sequence as shown in any of SEQ ID NOS: 52 to 60, 62 to 70, and 72 to 97 in the nucleotide sequence as shown in any of SEQ ID NOS: 2 to 10, 12 to 20, and 22 to 47 or a polynucleotide comprising a nucleotide sequence complementary to the nucleotide sequence as shown in any of SEQ ID NOS: 52 to 60, 62 to 70, and 72 to 97 in the nucleotide sequence as shown in any of SEQ ID NOS: 2 to 10, 12 to 20, and 22 to 47.

(7). The use of a composition according to (2), wherein the fragments are each a polynucleotide comprising the nucleotide sequence as shown in any of SEQ ID NOS: 52 to 60, 62 to 70, and 72 to 97 or a nucleotide sequence complementary to the nucleotide sequence as shown in any of SEQ ID NOS: 52 to 60, 62 to 70, and 72 to 97.

(8). The use of a composition according to (1) or (2), which further comprises, as a probe or probes, one or more polynucleotides selected from the group consisting of: polynucleotides consisting of the nucleotide sequences as shown in SEQ ID NOS: 11, 21, and 48 to 50; polynucleotides consisting of complementary sequences of the nucleotide sequences as shown in SEQ ID NOS: 11,21, and 48 to 50; or fragments of any one of the nucleotide sequences as shown in SEQ ID NOS: 11, 21, and 48 to 50 comprising at least 15 continuous nucleotides thereof.

(9). The use of a composition according to (8), wherein the fragment is a polynucleotide comprising at least 60 continuous nucleotides.

(10). The use of a composition according to (8), wherein the fragment is a polynucleotide comprising the nucleotide sequence as shown in any of SEQ ID NOS: 61, 71, and 98 to 100 in the nucleotide sequence as shown in any of SEQ ID NOS: 11, 21, and 48 to 50 or a polynucleotide comprising a nucleotide sequence complementary to the nucleotide sequence as shown in any of SEQ ID NOS: 61, 71, and 98 to 100 in the nucleotide sequence as shown in any of SEQ ID NOS: 11, 21, and 48 to 50.

11. The use of a composition according to (8), wherein the fragment is a polynucleotide comprising the nucleotide sequence as shown in any of SEQ ID NOS: 61, 71, and 98 to 100 or a nucleotide sequence complementary to any of SEQ ID NOS: 61, 71, and 98 to 100.

(12). A kit for detecting, identifying, or predicting the metastasis of kidney cancer in a subject, or for predicting the prognosis of a subject with kidney cancer, in vitro, comprising one or more probes selected from the probes as defined in (1).

(13). The kit according to (12), which further comprises at least one probe selected from the group of polynucleotides consisting of:

(a') polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(b) polynucleotides comprising nucleotide sequences as shown in SEQ ID NOS: 2 to 10, 12 to 20, and 22 to 47;
(c) polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof; and
(d) polynucleotides consisting of nucleotide sequences complementary to nucleotide sequences as shown in SEQ ID NOS: 2 to 10, 12 to 20, and 22 to 47.

(14). The kit according to (12) or (13), which further comprises as a probe a polynucleotide selected from the group consisting of the nucleotide sequence as shown in any of SEQ ID NOS: 11, 21, and 48 to 50, a polynucleotide consisting of a complementary sequence thereof, or a fragment of any one of the nucleotide sequence as shown in any of SEQ ID NOS: 11, 21, and 48 to 50 or of a polynucleotide consisting of a complementary sequence of SEQ ID NOS: 11, 21, and 48 to 50 comprising at least 15 continuous nucleotides thereof.

(15). The kit according to (14), wherein the probe is a polynucleotide consisting of the nucleotide sequence as shown in any of SEQ ID NOS: 1 to 10, 11, 12 to 20, 21, 22 to 47, and 48 to 50, a polynucleotide consisting of a complementary sequence thereof, or a fragment of any one of the nucleotide sequence as shown in any of SEQ ID NOS: 1 to 10, 11, 12 to 20, 21, 22 to 47, and 48 to 50, or of a polynucleotide consisting of a complementary sequence of SEQ ID NOS: 1 to 10, 11, 12 to 20, 21, 22 to 47, and 48 to 50 comprising at least 15 continuous nucleotides thereof.

(16). The kit according to any one of (12) to (15), wherein the fragment is a polynucleotide comprising at least 60 continuous nucleotides.

(17). The kit according to (14) or (15), wherein the fragment is a polynucleotide comprising the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50 or a polynucleotide comprising a nucleotide sequence complementary to the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50.

(18). The kit according to (14) or (15), wherein the fragment is a polynucleotide comprising a nucleotide sequence as shown in any of SEQ ID NOS: 51 to 60, 61, 62 to 70, 71, 72 to 97, and 98 to 100 or a nucleotide sequence complementary to a nucleotide sequence as shown in any of SEQ ID NOS: 51 to 60, 61, 62 to 70, 71, 72 to 97, and 98 to 100.

(19). The kit according to any one of (12) to (18), wherein the probes are packaged in different containers, alone or in combination.

(20). A DNA chip for detecting, identifying, or predicting the metastasis of kidney cancer in a subject, or for predicting the prognosis of a subject with kidney cancer, in vitro, comprising one or more probes selected from the probes as defined in (1).

(21). The DNA chip according to (20), which further comprises at least one probe selected from the group of polynucleotides consisting of:

(a) polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(b) polynucleotides comprising nucleotide sequences as shown in SEQ ID NOS: 2 to 10, 12 to 20, and 22 to 47;
(c) polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(d) polynucleotides comprising nucleotide sequences complementary to nucleotide sequences as shown in SEQ ID NOS: 2 to 10, 12 to 20, and 22 to 47.

(22). The DNA chip according to (20) or (21), which further comprises as a probe a polynucleotide selected from the group consisting of the nucleotide sequence as shown in any of SEQ ID NOS: 11, 21, and 48 to 50, a mutant thereof having at least 95% identity with said nucleotide sequences, and/or a fragment of any of the nucleotide sequence as shown in any of SEQ ID NOS: 11, 21, and 48 to 50 comprising at least 15 continuous nucleotides thereof.

(23). The DNA chip according to (22), which comprises at least two or all of the polynucleotides comprising a nucleotide sequence as shown in any of SEQ ID NOS: 51 to 60, 61, 62 to 70, 71, 72 to 97, and 98 to 100 or complementary sequences thereto.

(24). A method for detecting, identifying, or predicting the metastasis of kidney cancer, or for predicting the prognosis of a subject with kidney cancer, in vitro, comprising using one or more probes selected from the probes as defined in any one of (1) to (11), to measure in vitro expression levels of the one or more kidney cancer-associated target nucleic acids in a biological sample from a subject.

(25). The method according to (24), wherein the measurement is carried out using a DNA chip.

(26). The method according to (24) or (25), wherein the metastasis of kidney cancer in a subject, or the prognosis of a subject with kidney cancer, is measured using changes compared with a control sample as an indication.

(27). The method according to any one of (24) to (26), wherein the biological sample is a kidney tissue or a kidney cell, blood, plasma, serum, or urine.

(28). A method for detecting, identifying, or predicting the metastasis of kidney cancer, or for predicting the prognosis of a subject with kidney cancer, in vitro, comprising measuring in vitro expression levels of one or more kidney cancer-associated target nucleic acids in a biological sample from a subject using the composition according to any one of (1) to (11), the kit according to any one of (12) to (19), or the DNA chip according to any one of (20) to (23).

(29). A method for predicting in vitro a patient's prognosis and/or metastasis of kidney cancer by comparing the expression levels of target nucleic acids in a biological sample of kidney cancer cells obtained from a subject with those of the target nucleic acids in kidney cancer cells obtained from a patient population with poor prognosis and/or those of the target nucleic acids in kidney cancer cells obtained from a patient population with good prognosis using one or more probes as defined in (1), or the composition of any one of (1) to (11), the kit of any one of (12) to (19), or the DNA chip of any one of (20) to (23) comprising one or more probes, wherein the target nucleic acids can be detected by the polynucleotides contained in the composition, kit, or DNA chip or a mutant or fragment of such polynucleotide, and, when the expression levels of the target nucleic acids in the subject are different from those in the patient population with good prognosis and/or those in the patient population with poor prognosis, the subject is identified to have a poor or good prognosis and/or the metastasis of kidney cancer is determined to have or have not occurred.

(30). The method according to (29), which involves the use of a DNA chip.

(31). The method according to (29) or (30), which comprises the steps of:

(1) measuring in vitro expression levels of target nucleic acids in a plurality of biological samples that are known to be kidney cancer cells obtained from a patient with a poor prognosis and/or kidney cancer cells obtained from a patient with a good prognosis using one or more probes as defined in (1), the composition of any one of (1) to (11), the kit of any one of (12) to (19), or the DNA chip of any one of (20) to (23) comprising the probe or probes;

(2) preparing a discriminant, a support vector machine, using, as training samples, the expression levels of the target nucleic acids as determined in step (1);

(3) measuring in vitro expression levels of the target nucleic acids in a biological sample obtained from the kidney cancer of the subject in the same manner as in step (1); and

(4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof, or fragments thereof, contained in the composition, kit, or DNA chip.

(32). Use of one or more probes selected from the probes as defined in (1) or (2), or of the composition of any one of (1) to (11), the kit of any one of (12) to (19), or the DNA chip of any one of (20) to (23) comprising the one or more probes, in detecting, identifying, or predicting in vitro the metastasis of kidney cancer in a subject, or in predicting in vitro the prognosis of a subject with kidney cancer, in a biological sample from the subject.

Definition

The terms as used herein comprise the definitions as set forth below.

The term "probe" as used herein refers to a nucleic acid, an antibody, or an equivalent thereof, which is for detecting, identifying, or predicting the presence or metastasis of kidney cancer, and which is capable of binding to a particular gene that is an kidney cancer-associated marker, or to a polypeptide encoded thereby.

The meanings of terms such as nucleotide, polynucleotide, amino acid, peptide, polypeptide, and protein, and their abbreviations are in accordance with common usage in the art.

The term "polynucleotide" as used herein refers to a nucleic acid including each of RNA and DNA. Such DNA includes cDNA, genomic DNA, and synthetic DNA. Such RNA includes total RNA, mRNA, rRNA, and synthetic RNA. The term "polynucleotide" is used interchangeably with the term "nucleic acid."

The term "cDNA" as used herein refers to a full-length DNA strand of a sequence complementary to RNA resulting from gene expression, or a DNA fragment consisting of a partial sequence thereof. cDNA can be synthesized via reverse transcriptase-polymerase chain reaction (RT-PCR) using RNA as a template and a poly T primer.

The term "gene" as used herein refers to not only double-stranded DNA but also single-stranded DNA such as a plus-strand (or a sense strand) or a complementary strand (or an antisense strand) constituting double-stranded DNA. It is not particularly limited by the length of such strand. Accordingly, the term "gene" refers to any of double-stranded DNA (including human genomic DNA), single-stranded DNA (plus-strand) (including cDNA), single-stranded DNA having a sequence complementary to the plus-strand (complementary strand), and a fragment thereof, unless otherwise specified. Such "gene" includes not only a "gene" represented by a specific nucleotide sequence (or a SEQ ID NO.) but also another "gene" encoding a protein, which has a biological function equivalent to that of a protein encoded by said gene, such as a homolog, a mutant such as a splice mutant, and a derivative. Specific examples of the "genes" encoding such homolog, mutant, or derivative include "genes" each having a nucleotide sequence which hybridizes to a sequence complementary to a specific nucleotide sequence as shown in any of SEQ ID NOS: 1 to 50 under stringent conditions as described below.

Examples of human-derived protein homologs or genes encoding the same include proteins or genes derived from other organism species corresponding to the human proteins or human genes encoding the same. Such protein homologs or gene homologs can be identified by HomoloGene (http://www.ncbi.nlm.nih.gov/homoloGene/). Specifically, a certain human amino acid or nucleotide sequence can be subjected to the BLAST programs (Karlin, S. et al., Proceedings of the National Academic Sciences, U.S.A., 1993, vol. 90, pp. 5873-5877, http://www.ncbi.nlm.nih.gov/BLAST/) to obtain the accession number of the corresponding sequence (i.e., the sequence exhibiting the highest score, E-value 0, and identity 100%). Examples of the known BLAST programs include BLASTN (gene) and BLASTX (protein). When searching for a gene, for example, the accession number obtained from the above-mentioned BLAST search is inputted into the UniGene (http://www.ncbi.nlm.nih.gov/UniGene/), and the obtained UniGeneClusterID (the number identified with "Hs.") is then inputted into the HomoloGene. From the list that shows the correlation of gene homologs between the genes of other organism species and the human genes, a gene of the other organism species can be selected as a gene homolog corresponding to the human gene represented by a given nucleotide sequence. In this procedure, the FASTA program (http://www.ddbj.nig.ac.jp/search/fasta-e.html) may be used instead of the BLAST program.

Functional regions of "genes" are not limited, and examples thereof include expression-control regions, coding regions, and exon or intron regions.

The term "transcription product" as used herein refers to messenger RNA (mRNA) which is synthesized from the DNA sequence of a gene as a template. Messenger RNA is synthesized by binding of RNA polymerase to a site called promoter, which is located upstream of the gene of interest, and subsequently by binding of ribonucleotides to the 3' end so as to be complementary to the nucleotide sequence of DNA. Such messenger RNA contains not only the gene of interest but also a full-length sequence spanning from a transcription initiation site to the terminus of a poly A sequence including expression control region, coding region, and exon or intron region.

The term "translation product" as used herein refers to a protein which is synthesized based on the information of messenger RNA synthesized via transcription regardless of modification such as splicing. During the translation process of messenger RNA, ribosome first binds to messenger RNA, and amino acids are then linked in accordance with the nucleotide sequence of messenger RNA, thereby leading to the synthesis of a protein.

The term "primer" as used herein refers to a continuous polynucleotide that specifically recognizes and amplifies RNA resulting from gene expression or a polynucleotide derived therefrom, and/or a polynucleotide complementary thereto.

The complementary polynucleotide (i.e., a complementary strand or reverse strand) refers to a polynucleotide that is basically complementary to the full-length sequence of a polynucleotide having a nucleotide sequence as shown in a given SEQ ID NO. or a partial sequence thereof (herein, conveniently referred to as a "plus strand"), on the basis of the base pairing such as A:T(U) or G:C. Such a complementary strand, however, is not limited to a sequence completely complementary to the nucleotide sequence of a plus strand of interest; that is, the complementary strand may have such a complementarity that it can hybridize to the plus strand under stringent conditions.

As used herein, the "stringent conditions" means such conditions that a probe can hybridize to a target sequence with a higher degree of detection when compared with its hybridization to other sequences (e.g., at least twice the background). Stringent conditions are dependent on the sequence of a target, varying depending on the environment where hybridization takes place. By controlling stringency of hybridization and/or washing conditions, a target sequence that is 100% complementary to the probe can be identified.

As used herein, the term "mutant" in case of a nucleic acid refers to a naturally-occurring mutant resulting from polymorphism, mutation, selective splicing during transcription, or the like, a homolog thereof, a mutant based on degeneracy of genetic cord, a mutant comprising a deletion, substitution, addition, or insertion of one or more nucleotides, preferably one or several nucleotides, in a nucleotide sequence as shown in any of SEQ ID NOS: 1 to 50 or a partial sequence thereof, a mutant having at least about 80%, at least about 85%, preferably at least about 90%, more preferably at least about 95%, at least about 97%, at least about 98%, or at least about 99% identity with said nucleotide sequence or said partial sequence thereof, or a nucleic acid mutant that hybridizes to a polynucleotide or oligonucleotide comprising said nucleotide sequence or said partial sequence thereof under the stringent conditions as defined above. On the other hand, a "mutant" in case of a protein or peptide refers to a mutant comprising a deletion, substitution, addition, or insertion of one or more amino acids, preferably one or several amino acids, in an amino acid sequence as shown in any of SEQ ID NOS: 101 to 197 or a partial sequence thereof, or a mutant having a % identity of at least about 80%, at least about 85%, at least about 90%, at least about 95%, at least about 97%, at least about 98%, or at least about 99% with said amino acid sequence or said partial sequence thereof.

The term "several" as used herein means an integer of about 10, 9, 8, 7, 6, 5, 4, 3, or 2.

As used herein, the "% identity" can be identified by using a protein or gene searching system such as BLAST or FASTA as mentioned above, with introducing a gap (Karlin, S. et al., 1993, Proceedings of the National Academic Sciences, U.S.A., vol. 90, pp. 5873-5877; Altschul, S. F. et al., 1990, Journal of Molecular Biology, vol. 215, pp. 403-410; Pearson, W. R. et al., 1988, Proceedings of the National Academic Sciences, U.S.A., vol. 85, pp. 2444-2448).

As used herein, the term "derivative" in case of a nucleic acid refers to a derivative labeled with fluorophore or the like, a derivative comprising a modified nucleotide (e.g., a nucleotide having a functional group such as halogen, alkyl (e.g., methyl), alkoxy (e.g., methoxy), thio, or carboxymethyl; or a nucleotide comprising, for example, reconstitution of a base, saturation of a double bond, deamination, or substitution of oxygen by sulfur), or the like. On the other hand, a "derivative" in case of a protein (including an antibody) refers to a derivative labeled with an enzyme, fluorophore, or radioisotope, or a chemically modified derivative, such as an acetylated, acylated, alkylated, phosphorylated, sulfated, or glycosylated derivative.

As used herein, the term "a composition for diagnosis (or detection or identification)" or "a composition for identifying the disease" refers to a composition that is directly or indirectly employed for predicting the prognosis for a kidney cancer patient and/or for diagnosing the development of kidney cancer, the degree of advancement, the presence or absence of metastasis of kidney cancer, or the degree of amelioration, or for screening for candidate substances useful for preventing, ameliorating, or treating kidney cancer. The composition comprises a nucleotide, an oligonucleotide, or a polynucleotide, which can specifically recognize and bind to a gene whose expression varies or fluctuates *in vivo*, in particularly kidney tissue, tissue associated with the development of the kidney cancer, and an antibody that can detect a protein as a translation product of the gene. Such nucleotide, oligonucleotide and polynucleotide can be effectively used as a probe for detecting the aforementioned gene that is expressed *in vivo*, in tissue, or in a cell, based on the aforementioned properties, or as a primer for amplifying the gene expressed *in vivo*. As used herein, the term "composition" refers to a composition comprising a single probe according to the present invention or a probe mixture comprising two or more different probes. Such composition can be in any form, including a solid matter (e.g., a freeze-dried product) or a solution (e.g., an aqueous solution or buffer). Also, such composition can comprise additives such as a stabilizer and a preservative as long as such additive would not influence the analysis, according to need.

As used herein, the term "biological tissue" to be detected or diagnosed refers to a sample (or specimen) obtained from a living body, in which the expression pattern of the target gene of the invention changes with the development of kidney cancer, or the amount of the target polypeptide of the present invention varies compared with a normal control sample. Examples of biological samples include kidney tissue, tissue obtained from peripheral lymph nodes, or another organ suspected of being at risk for metastasis, cells derived from such tissue, and body fluid such as blood.

The term "specifically bind(s)" as used herein means that an antibody or a fragment thereof forms an antigen-antibody complex with only the target polypeptide, or a mutant or fragment thereof, of the invention, but does not substantially form such a complex with other peptidic or polypeptidic substances. The term "substantially" as used herein means that formation of a nonspecific complex may occur, although its degree is small.

The term "epitope" as used herein refers to an antigenic or immunogenic partial amino acid region (or an antigenic determinant) of the target polypeptide of the invention or a mutant or fragment thereof. The epitope is generally composed of at least 5 amino acids, preferably at least 7 amino acids, and more preferably at least 10 amino acids.

The term "prognosis" as used herein refers to a probability of survival that is indicated by a survival curve plotted by the Kaplan-Meier method (e.g., BMJ, 1998, 317: 1572). The patients with "good prognosis" refers to patients who exhibit a high survival ratio with a significant difference of $p < 0.05$, which is obtained by classifying the kidney cancer patients in accordance with some sort of clinical information, the survival curves are plotted by the Kaplan-Meier method according to the classification, and the differences are determined by a statistical technique. Similarly, patients with "poor prognosis" refer to patients who exhibit a low survival ratio with a significant difference of $p < 0.05$, which is obtained by classifying the kidney cancer patients in accordance with some sort of clinical information, the survival curves are plotted by the Kaplan-Meier method according to the classification, and the differences are determined by a statistical technique. Since the presence or absence of cancer metastasis is a factor that influences the patient's prognosis, the prediction of the prognosis is partly correlated with the prediction of cancer metastasis.

The term "PABPN1 gene" or "PABPN1" as used herein includes a gene (or DNA) encoding the Poly(A)-Binding Protein, Nuclear 1 gene (or DNA) as shown in a given nucleotide sequence (i.e., SEQ ID NO: 1), a homolog thereof, a mutant thereof, a derivative thereof, or the like, unless it is defined by a SEQ ID NO. Specifically, it includes the PABPN1 gene (Ensembl Gene ID, ENSG00000100836) as shown in SEQ ID NO: 1 and homologs thereof derived from other organism species. The PABPN1 gene can be obtained by the method disclosed in, for example, Brais, B. et al., 1998, Nature genetics, vol. 18, pp. 164-167. The fact that variations in the PABPN1 gene expression can become an indication of metastasis of kidney cancer is not known.

The term "PINK1 gene" or "PINK1" as used herein includes a gene (or DNA) encoding the PTEN Induced Putative Kinase 1 gene (or DNA) as shown in a given nucleotide sequence (i.e., SEQ ID NO: 2), a homolog thereof, a mutant thereof, a derivative thereof, or the like, unless it is defined by a SEQ ID NO. Specifically, it includes the PINK1 gene (Ensembl Gene ID, ENSG00000180056) as shown in SEQ ID NO: 2 and homologs thereof derived from other organism species. The PINK1 gene can be obtained by the method disclosed in, for example, Unoki, M. et al., 2001, Oncogene, vol. 20, pp. 4457-4465. The fact that variations in the PINK1 gene expression can become an indication of metastasis of kidney cancer is not known.

The term "TFF2 gene" or "TFF2" as used herein includes a gene (or DNA) encoding the Trefoil Factor 2 gene (or DNA) as shown in a given nucleotide sequence (i.e., SEQ ID NO: 3), a homolog thereof, a mutant thereof, a derivative thereof, or the like, unless it is defined by a SEQ ID NO. Specifically, it includes the TFF2 gene (Ensembl Gene ID, ENSG00000160181) as shown in SEQ ID NO: 3 and homologs thereof derived from other organism species. The TFF2 gene can be obtained by the method disclosed in, for example, Tomasetto, C. et al., 1990, EMBO Journal, vol. 9, pp. 407-414. The fact that variations in the TFF2 gene expression can become an indication of metastasis of kidney cancer is not known.

The term "EIF3S9 gene" or "EIF3S9" as used herein includes a gene (or DNA) encoding the Eukaryotic Translation Initiation Factor 3 Subunit 9 gene (or DNA) as shown in a given nucleotide sequence (i.e., SEQ ID NO: 4), a homolog thereof, a mutant thereof, a derivative thereof, or the like, unless it is defined by a SEQ ID NO. Specifically, it includes the EIF3S9 gene (Ensembl Gene ID, ENSG00000106263) as shown in SEQ ID NO: 4 and homologs thereof derived from other organism species. The EIF3S9 gene can be obtained by the method disclosed in, for example, Methot, N. et al., 1997, Journal of Biological Chemistry, vol. 272, pp. 1110-1116. The fact that variations in the EIF3S9 gene expression can become an indication of metastasis of kidney cancer is not known.

The term "MAX gene" or "MAX" as used herein includes a gene (or DNA) encoding the MAX Protein gene (or DNA) as shown in a given nucleotide sequence (i.e., SEQ ID NO: 5), a homolog thereof, a mutant thereof, a derivative thereof, or the like, unless it is defined by a SEQ ID NO. Specifically, it includes the MAX gene (Ensembl Gene ID, ENSG00000125952) as shown in SEQ ID NO: 5 and homologs thereof derived from other organism species. The MAX gene can be obtained by the method disclosed in, for example, Wagner, A. et al., 1992, Proceedings of the National Academic Sciences, U.S.A., vol. 89, pp. 3111-3115. The fact that variations in the MAX gene expression can be an indication of metastasis of kidney cancer is not known.

The term "MLL4 gene" or "MLL4" as used herein includes a gene (or DNA) encoding the Trithorax Homolog 2 gene (or DNA) as shown in a given nucleotide sequence (i.e., SEQ ID NO: 6), a homolog thereof, a mutant thereof, a derivative thereof, or the like, unless it is defined by a SEQ ID NO. Specifically, it includes the MLL4 gene (Ensembl Gene ID, ENSG00000105663) as shown in SEQ ID NO: 6 and homologs thereof derived from other organism species. The MLL4 gene can be obtained by the method disclosed in, for example, Nagase, T. et al., 1997, DNA Research, vol. 4, pp. 141-150. The fact that variations in the MLL4 gene expression can become an indication of metastasis of kidney cancer is not known.

The term "CACNB2 gene" or "CACNB2" as used herein includes a gene (or DNA) encoding the Dihydropyridine-Sensitive L-Type, Calcium Channel Beta-2 Subunit gene (or DNA) as shown in a given nucleotide sequence (i.e., SEQ ID NO: 7), a homolog thereof, a mutant thereof, a derivative thereof, or the like, unless it is defined by a SEQ ID NO. Specifically, it includes the CACNB2 gene (Ensembl Gene ID, ENSG00000165995) as shown in SEQ ID NO: 7 and homologs thereof derived from other organism species. The CACNB2 gene can be obtained by the method disclosed in, for example, Rosenfeld, M. et al., 1993, Annals of Neurology., vol. 33, pp. 113-120. The fact that variations in the CACNB2 gene expression can become an indication of metastasis of kidney cancer is not known.

The term "ZMYND11 gene" or "ZMYND11" as used herein includes a gene (or DNA) encoding the Adenovirus 5 E1A-Binding Protein gene (or DNA) as shown in a given nucleotide sequence (i.e., SEQ ID NO: 8), a homolog thereof, a mutant thereof, a derivative thereof, or the like, unless it is defined by a SEQ ID NO. Specifically, it includes the ZMYND 11 gene (Ensembl Gene ID, ENSG00000015171) as shown in SEQ ID NO: 8 and homologs thereof derived from other organism species. The ZMYND11 gene can be obtained by the method disclosed in, for example, Hateboer, G. et al., 1995, EMBO Journal, vol. 14, pp. 3159-3169. The fact that variations in the ZMYND11 gene expression can become an indication of metastasis of kidney cancer is not known.

The term "BAT2 gene" or "BAT2" as used herein includes a gene (or DNA) encoding the Adenovirus 5 E1A-Binding Protein gene (or DNA) as shown in a given nucleotide sequence (i.e., SEQ ID NO: 9), a homolog thereof, a mutant thereof, a derivative thereof, or the like, unless it is defined by a SEQ ID NO. Specifically, it includes the BAT2 gene (Ensembl Gene ID, ENSG00000111960) as shown in SEQ ID NO: 9 and homologs thereof derived from other organism species. The BAT2 gene can be obtained by the method disclosed in, for example, Banerji, J. et al., 1990, Proceedings of the National Academic Sciences, U.S.A., vol. 87, pp. 2374-2378. The fact that variations in the BAT2 gene expression can become an indication of metastasis of kidney cancer is not known.

The term "NRBP gene" or "NRBP" as used herein includes a gene (or DNA) encoding the Nuclear Receptor Binding Protein gene (or DNA) as shown in a given nucleotide sequence (i.e., SEQ ID NO: 10), a homolog thereof, a mutant thereof, a derivative thereof, or the like, unless it is defined by a SEQ ID NO. Specifically, it includes the NRBP gene (Ensembl Gene ID, ENSG00000115216) as shown in SEQ ID NO: 10 and homologs thereof derived from other organism species. The NRBP gene can be obtained by the method disclosed in, for example, Hooper, J. D.. et al., 2000, Genomics, vol. 66, pp. 113-118. The fact that variations in the NRBP gene expression can become an indication of metastasis of kidney cancer is not known.

The term "MCM3AP gene" or "MCM3AP" as used herein includes a gene (or DNA) encoding the 80 KDa MCM3-Associated Protein gene (or DNA) as shown in a given nucleotide sequence (i.e., SEQ ID NO: 11), a homolog thereof, a mutant thereof, a derivative thereof, or the like, unless it is defined by a SEQ ID NO. Specifically, it includes the MCM3AP gene (Ensembl Gene ID, ENSG00000160294) as shown in SEQ ID NO: 11 and homologs thereof derived from other organism species. The MCM3AP gene can be obtained by the method disclosed in, for example, Takei, Y. et al., 1998, Journal of Biological Chemistry, vol. 273, pp. 22177-22180. The possibility that variations in the MCM3AP gene expression may become an indication of metastasis of kidney cancer is presumed in JP Patent Publication (kohyo) No. 2005-520536 (A), although it has not practically been verified.

The term "COL4A1 gene" or "COL4A1" as used herein includes a gene (or DNA) encoding the Collagen Alpha 1(IV) Chain gene (or DNA) as shown in a given nucleotide sequence (i.e., SEQ ID NO: 12), a homolog thereof, a mutant thereof, a derivative thereof, or the like, unless it is defined by a SEQ ID NO. Specifically, it includes the COL4A1 gene (Ensembl Gene ID, ENSG00000139825) as shown in SEQ ID NO: 12 and homologs thereof derived from other organism species. The COL4A1 gene can be obtained by the method disclosed in, for example, Solomon, E. et al., 1985, Proceedings of the National Academic Sciences, U.S.A., vol. 82, pp. 3330-3334. The fact that variations in the COL4A1 gene expression can become an indication of metastasis of kidney cancer is not known.

The term "MKNK1 gene" or "MKNK1" as used herein includes a gene (or DNA) encoding the MAP Kinase-Interacting Serine/Threonine Kinase 1 gene (or DNA) as shown in a given nucleotide sequence (i.e., SEQ ID NO: 13), a homolog thereof, a mutant thereof, a derivative thereof, or the like, unless it is defined by a SEQ ID NO. Specifically, it includes the MKNK1 gene (Ensembl Gene ID, ENSG00000079277) as shown in SEQ ID NO: 13 and homologs thereof derived from other organism species. The MKNK1 gene can be obtained by the method disclosed in, for example, Fukunaga, R. et al., 1997, EMBO Journal, vol. 16, pp. 1921-1933. The fact that variations in the MKNK1 gene expression can become an indication of metastasis of kidney cancer is not known.

The term "BBC3 gene" or "BBC3" as used herein includes a gene (or DNA) encoding the BCL2 Binding Component 3 gene (or DNA) as shown in a given nucleotide sequence (i.e., SEQ ID NO: 14), a homolog thereof, a mutant thereof, a derivative thereof, or the like, unless it is defined by a SEQ ID NO. Specifically, it includes the BBC3 gene (Ensembl Gene ID, ENSG00000105327) as shown in SEQ ID NO: 14 and homologs thereof derived from other organism species. The BBC3 gene can be obtained by the method disclosed in, for example, Yu, J. et al., 2001, Molecular Cell, vol. 7, pp. 673-682. The fact that variations in the BBC3 gene expression can become an indication of metastasis of kidney cancer is not known.

The term "FLJ10359 gene" or "FLJ10359" as used herein includes a gene (or DNA) encoding the Protein BAP28 gene (or DNA) as shown in a given nucleotide sequence (i.e., SEQ ID NO: 15), a homolog thereof, a mutant thereof, a derivative thereof, or the like, unless it is defined by a SEQ ID NO. Specifically, it includes the FLJ10359 gene (Ensembl Gene ID, ENSG00000119285) as shown in SEQ ID NO: 15 and homologs thereof derived from other organism species.

The term "DPT gene" or "DPT" as used herein includes a gene (or DNA) encoding the Dermatopontin gene (or DNA) as shown in a given nucleotide sequence (i.e., SEQ ID NO: 16), a homolog thereof, a mutant thereof, a derivative thereof, or the like, unless it is defined by a SEQ ID NO. Specifically, it includes the DPT gene (Ensembl Gene ID, ENSG00000143196) as shown in SEQ ID NO: 16 and homologs thereof derived from other organism species. The DPT gene can be obtained by the method disclosed in, for example, Superti-Fruga, A. et al., 1993, Genomics, vol. 17, pp. 463-467. The fact that variations in the DPT gene expression can become an indication of metastasis of kidney cancer is not known.

The term "C10orf76 gene" or "C10orf76" as used herein includes a gene (or DNA) encoding the gene (or DNA) as shown in a given nucleotide sequence (i.e., SEQ ID NO: 17), a homolog thereof, a mutant thereof, a derivative thereof, or the like, unless it is defined by a SEQ ID NO. Specifically, it includes the C10orf76 gene (Ensembl Gene ID, ENSG00000120029) as shown in SEQ ID NO: 17 and homologs thereof derived from other organism species.

The term "DIA1 gene" or "DIA1" as used herein includes a gene (or DNA) encoding the NADH-Cytochrome B5 Reductase gene (or DNA) as shown in a given nucleotide sequence (i.e., SEQ ID NO: 18), a homolog thereof, a mutant thereof, a derivative thereof, or the like, unless it is defined by a SEQ ID NO. Specifically, it includes the DIA1 gene (Ensembl Gene ID, ENSG00000100243) as shown in SEQ ID NO: 18 and homologs thereof derived from other organism species. The DIA1 gene can be obtained by the method disclosed in, for example, Du, M. et al., 1997, Biochemical Biophysical Research Communication, vol. 235, pp. 779-783. The fact that variations in the DIA1 gene expression can become an indication of metastasis of kidney cancer is not known.

The term "PBK gene" or "PBK" as used herein includes a gene (or DNA) encoding the T-LAK Cell-Originated Protein Kinase gene (or DNA) as shown in a given nucleotide sequence (i.e., SEQ ID NO: 19), a homolog thereof, a mutant thereof, a derivative thereof, or the like, unless it is defined by a SEQ ID NO. Specifically, it includes the PBK gene (Ensembl Gene ID, ENSG00000168078) as shown in SEQ ID NO: 19 and homologs thereof derived from other organism species. The PBK gene can be obtained by the method disclosed in, for example, Gaudet, S. et al., 2000, Proceedings of the National Academic Sciences, U.S.A., vol. 97, pp. 5167-5172. The fact that variations in the PBK gene expression can become an indication of metastasis of kidney cancer is not known.

The term "PRKD2 gene" or "PRKD2" as used herein includes a gene (or DNA) encoding the Protein Kinase C, D2 Type gene (or DNA) as shown in a given nucleotide sequence (i.e., SEQ ID NO: 20), a homolog thereof, a mutant thereof, a derivative thereof, or the like, unless it is defined by a SEQ ID NO. Specifically, it includes the PRKD2 gene (Ensembl Gene ID, ENSG00000105287) as shown in SEQ ID NO: 20 and homologs thereof derived from other organism species. The PRKD2 gene can be obtained by the method disclosed in, for example, Sturany, S. et al., 2001, Journal of Biological Chemistry, vol. 276, pp. 3310-3318. The fact that variations in the PRKD2 gene expression can become an indication of metastasis of kidney cancer is not known.

The term "KRT19 gene" or "KRT19" as used herein includes a gene (or DNA) encoding the Keratin, Type I Cytoskeletal 19 gene (or DNA) as shown in a given nucleotide sequence (i.e., SEQ ID NO: 21), a homolog thereof, a mutant thereof, a derivative thereof, or the like, unless it is defined by a SEQ ID NO. Specifically, it includes the KRT19 gene (Ensembl Gene ID, ENSG00000171345) as shown in SEQ ID NO: 21 and homologs thereof derived from other organism species. The KRT19 gene can be obtained by the method disclosed in, for example, Bader, B. et al., 1986, EMBO Journal, vol. 5, pp. 1865-1875. The fact that variations in the KRT19 gene expression can become an indication of kidney cancer is deduced in JP Patent Publication (kohyo) No. 2005-507997 (A), although it has not yet been verified.

The term "FLJ23436 gene" or "FLJ23436" as used herein includes a gene (or DNA) encoding the gene (or DNA) as shown in a given nucleotide sequence (i.e., SEQ ID NO: 22), a homolog thereof, a mutant thereof, a derivative thereof, or the like, unless it is defined by a SEQ ID NO. Specifically, it includes the FLJ23436 gene (Ensembl Gene ID, ENSG00000169957) as shown in SEQ ID NO: 22 and homologs thereof derived from other organism species. The FLJ23436 gene can be obtained by the method disclosed in, for example, Beausoleil, S. A. et al., 2004, Proceedings of the National Academic Sciences, U.S.A., vol. 101, pp. 12130-12135. The fact that variations in the FLJ23436 gene expression can become an indication of metastasis of kidney cancer is not known.

The term "NPHS2 gene" or "NPHS2" as used herein includes a gene (or DNA) encoding the Podocin gene (or DNA) as shown in a given nucleotide sequence (i.e., SEQ ID NO: 23), a homolog thereof, a mutant thereof, a derivative thereof, or the like, unless it is defined by a SEQ ID NO. Specifically, it includes the NPHS2 gene (Ensembl Gene ID, ENSG00000116218) as shown in SEQ ID NO: 23 and homologs thereof derived from other organism species. The NPHS2 gene can be obtained by the method disclosed in, for example, Kestila, M. et al., 1998, Molecular Cell, vol. 1, pp. 575-582. The fact that variations in the NPHS2 gene expression can become an indication of metastasis of kidney cancer is not known.

The term "C3orf14 gene" or "C3orf14" as used herein includes a gene (or DNA) encoding the HT021 gene (or DNA) as shown in a given nucleotide sequence (i.e., SEQ ID NO: 24), a homolog thereof, a mutant thereof, a derivative thereof, or the like, unless it is defined by a SEQ ID NO. Specifically, it includes the C3orf14 gene (Ensembl Gene ID, ENSG00000114405) as shown in SEQ ID NO: 24 and homologs thereof derived from other organism species.

The term "AGTR2 gene" or "AGTR2" as used herein includes a gene (or DNA) encoding the Type-2 Angiotensin II Receptor gene (or DNA) as shown in a given nucleotide sequence (i.e., SEQ ID NO: 25), a homolog thereof, a mutant thereof, a derivative thereof, or the like, unless it is defined by a SEQ ID NO. Specifically, it includes the AGTR2 gene (Ensembl Gene ID, ENSG00000180772) as shown in SEQ ID NO: 25 and homologs thereof derived from other organism species. The AGTR2 gene can be obtained by the method disclosed in, for example, Koike, G. et al., 1994, Biochemical Biophysical Research Communication, vol. 203, pp. 1842-1850. The fact that variations in the AGTR2 gene expression can become an indication of metastasis of kidney cancer is not known.

The term "HTR1F gene" or "HTR1F" as used herein includes a gene (or DNA) encoding the 5-Hydroxytryptamine IF Receptor gene (or DNA) as shown in a given nucleotide sequence (i.e., SEQ ID NO: 26), a homolog thereof, a mutant thereof, a derivative thereof, or the like, unless it is defined by a SEQ ID NO. Specifically, it includes the HTR1F gene (Ensembl Gene ID, ENSG00000179097) as shown in SEQ ID NO: 26 and homologs thereof derived from other organism species. The HTR1F gene can be obtained by the method disclosed in, for example, Lovenberg, T. W. et al., 1993, Proceedings of the National Academic Sciences, U.S.A., vol. 90, pp. 2184-2188. The fact that variations in the HTR1F gene expression can become an indication of metastasis of kidney cancer is not known.

The term "KIF3B gene" or "KIF3B" as used herein includes a gene (or DNA) encoding the Kinesin-Like Protein KIF3B gene (or DNA) as shown in a given nucleotide sequence (i.e., SEQ ID NO: 27), a homolog thereof, a mutant thereof, a derivative thereof, or the like, unless it is defined by a SEQ ID NO. Specifically, it includes the KIF3B gene (Ensembl Gene ID, ENSG00000101350) as shown in SEQ ID NO: 27 and homologs thereof derived from other organism species. The KIF3B gene can be obtained by the method disclosed in, for example, Nagase, T. et al., 1997, DNA Research, vol. 4, pp. 141-150. The fact that variations in the KIF3B gene expression can become an indication of metastasis of kidney cancer is not known.

The term "DCN gene" or "DCN" as used herein includes a gene (or DNA) encoding the Decorin gene (or DNA) as shown in a given nucleotide sequence (i.e., SEQ ID NO: 28), a homolog thereof, a mutant thereof, a derivative thereof, or the like, unless it is defined by a SEQ ID NO. Specifically, it includes the DCN gene (Ensembl Gene ID, ENSG00000011465) as shown in SEQ ID NO: 28 and homologs thereof derived from other organism species. The DCN gene can be obtained by the method disclosed in, for example, Danielson, K. G. et al., 1993, Genomics, vol. 15, pp. 146-160. The fact that variations in the DCN gene expression can become an indication of metastasis of kidney cancer is not known.

The term "STK22C gene" or "STK22C" as used herein includes a gene (or DNA) encoding the Testis-Specific Serine/Threonine Kinase 22C gene (or DNA) as shown in a given nucleotide sequence (i.e., SEQ ID NO: 29), a homolog thereof, a mutant thereof, a derivative thereof, or the like, unless it is defined by a SEQ ID NO. Specifically, it includes the STK22C gene (Ensembl Gene ID, ENSG00000162526) as shown in SEQ ID NO: 29 and homologs thereof derived from other organism species. The STK22C gene can be obtained by the method disclosed in, for example, Visconti, P. E. et al., 2001, Genomics, vol. 77, pp. 163-170. The fact that variations in the STK22C gene expression can become an indication of metastasis of kidney cancer is not known.

The term "DKFZp566C0424 gene" or "DKFZp566C0424" as used herein includes a gene (or DNA) encoding the Putative MAPK Activating Protein PM20 gene (or DNA) as shown in a given nucleotide sequence (i.e., SEQ ID NO: 30), a homolog thereof, a mutant thereof, a derivative thereof, or the like, unless it is defined by a SEQ ID NO. Specifically, it includes the DKFZp566C0424 gene (ENSG00000055070) as shown in SEQ ID NO: 30 and homologs thereof derived from other organism species. The DKFZp566C0424 gene can be obtained by the method disclosed in, for example, Visconti, P. E. et al., 2001, Genomics, vol. 77, pp. 163-170. The fact that variations in the DKFZp566C0424 gene expression can become an indication of metastasis of kidney cancer is not known.

The term "CNR1 gene" or "CNR1" as used herein includes a gene (or DNA) encoding the Cannabinoid Receptor 1 gene (or DNA) as shown in a given nucleotide sequence (i.e., SEQ ID NO: 31), a homolog thereof, a mutant thereof, a derivative thereof, or the like, unless it is defined by a SEQ ID NO. Specifically, it includes the CNR1 gene (Ensembl Gene ID, ENSG00000118432) as shown in SEQ ID NO: 31 and homologs thereof derived from other organism species. The CNR1 gene can be obtained by the method disclosed in, for example, Matsuda, L.A. et al., 1990, Nature, vol. 346, pp. 561-564. The fact that variations in the CNR1 gene expression can become an indication of metastasis of kidney cancer is not known.

The term "HOMER3 gene" or "HOMER3" as used herein includes a gene (or DNA) encoding the HOMER, Neuronal Immediate Early Gene, 3 gene (or DNA) as shown in a given nucleotide sequence (i.e., SEQ ID NO: 32), a homolog thereof, a mutant thereof, a derivative thereof, or the like, unless it is defined by a SEQ ID NO. Specifically, it includes the HOMER3 gene (Ensembl Gene ID, ENSG00000051128) as shown in SEQ ID NO: 32 and homologs thereof derived from other organism species. The HOMER3 gene can be obtained by the method disclosed in, for example, Xiao, B. et al., 2001, Neuron, vol. 21, pp. 707-716. The fact that variations in the HOMER3 gene expression can become an indication of metastasis of kidney cancer is not known.

The term "GPR2 gene" or "GPR2" as used herein includes a gene (or DNA) encoding the C-C Chemokine Receptor Type 10 gene (or DNA) as shown in a given nucleotide sequence (i.e., SEQ ID NO: 33), a homolog thereof, a mutant thereof, a derivative thereof, or the like, unless it is defined by a SEQ ID NO. Specifically, it includes the GPR2 gene (Ensembl Gene ID, ENSG00000177032) as shown in SEQ ID NO: 33 and homologs thereof derived from other organism species. The GPR2 gene can be obtained by the method disclosed in, for example, Marchese, A. et al., 1994, Genomics, vol. 23, pp. 609-618. The fact that variations in the GPR2 gene expression can become an indication of metastasis of kidney cancer is not known.

The term "FLJ 12442 gene" or "FLJ12442" as used herein includes a gene (or DNA) encoding the gene (or DNA) as shown in a given nucleotide sequence (i.e., SEQ ID NO: 34), a homolog thereof, a mutant thereof, a derivative thereof, or the like, unless it is defined by a SEQ ID NO. Specifically, it includes the FLJ12442 gene (Ensembl Gene ID, ENSG00000168268) as shown in SEQ ID NO: 34 and homologs thereof derived from other organism species. The FLJ12442 gene can be obtained by the method disclosed in, for example, Ota, T. et al., 2004, Nature Genetics, vol. 36, pp. 40-45. The fact that variations in the FLJ 12442 gene expression can become an indication of metastasis of kidney cancer is not known.

The term "XLKD1 gene" or "XLKD1 " as used herein includes a gene (or DNA) encoding the Extracellular Link Domain Containing 1 gene (or DNA) as shown in a given nucleotide sequence (i.e., SEQ ID NO: 35), a homolog thereof, a mutant thereof, a derivative thereof, or the like, unless it is defined by a SEQ ID NO. Specifically, it includes the XLKD1 gene (Ensembl Gene ID, ENSG00000133800) as shown in SEQ ID NO: 35 and homologs thereof derived from other organism species. The XLKD1 gene can be obtained by the method disclosed in, for example, Banerji, S. et al., 1999, Journal of Cellular Biology, vol. 144, pp. 1789-801. The fact that variations in the XLKD1 gene expression can become an indication of metastasis of kidney cancer is not known.

The term "CASKIN2 gene" or "CASKIN2" as used herein includes a gene (or DNA) encoding the CASK-Interacting Protein 2 gene (or DNA) as shown in a given nucleotide sequence (i.e., SEQ ID NO: 36), a homolog thereof, a mutant thereof, a derivative thereof, or the like, unless it is defined by a SEQ ID NO. Specifically, it includes the CASKIN2 gene (Ensembl Gene ID, ENSG00000177303) as shown in SEQ ID NO: 36 and homologs thereof derived from other organism species. The CASKIN2 gene can be obtained by the method disclosed in, for example, Strausberg, R. L. et al., 2002, Proceedings of the National Academic Sciences, U.S.A., vol. 99, pp. 16899-16903. The fact that variations in the CASKIN2 gene expression can become an indication of metastasis of kidney cancer is not known.

The term "COL5A2 gene" or "COL5A2" as used herein includes a gene (or DNA) encoding the Collagen Alpha 2(V) Chain Protein gene (or DNA) as shown in a given nucleotide sequence (i.e., SEQ ID NO: 37), a homolog thereof, a mutant thereof, a derivative thereof, or the like, unless it is defined by a SEQ ID NO. Specifically, it includes the COL5A2 gene (Ensembl Gene ID, ENSG00000179877) as shown in SEQ ID NO: 37 and homologs thereof derived from other organism species. The COL5A2 gene can be obtained by the method disclosed in, for example, Burgeson, R. E. et al., 2002, Proceedings of the National Academic Sciences, U.S.A., vol. 73, pp. 2579-2583. The fact that variations in the COL5A2 gene expression can become an indication of metastasis of kidney cancer is not known.

The term "BRD3 gene" or "BRD3" as used herein includes a gene (or DNA) encoding the Bromodomain-Containing Protein 3 gene (or DNA) as shown in a given nucleotide sequence (i.e., SEQ ID NO: 38), a homolog thereof, a mutant thereof, a derivative thereof, or the like, unless it is defined by a SEQ ID NO. Specifically, it includes the BRD3 gene (Ensembl Gene ID, ENSG00000169925) as shown in SEQ ID NO: 38 and homologs thereof derived from other organism species. The BRD3 gene can be obtained by the method disclosed in, for example, Nomura, N. L. et al., 1994, DNA Research, vol. 1, pp. 223-229. The fact that variations in the BRD3 gene expression can become an indication of metastasis of kidney cancer is not known.

The term "ATP6V0A4 gene" or "ATP6V0A4" as used herein includes a gene (or DNA) encoding the ATPase, H+ Transporting, Lysosomal V0 Subunit A ISOFORM 4 gene (or DNA) as shown in a given nucleotide sequence (i.e., SEQ ID NO: 39), a homolog thereof, a mutant thereof, a derivative thereof, or the like, unless it is defined by a SEQ ID NO. Specifically, it includes the ATP6V0A4gene (Ensembl Gene ID, ENSG00000105929) as shown in SEQ ID NO: 39 and homologs thereof derived from other organism species. The ATP6V0A4 gene can be obtained by the method disclosed in, for example, Smith, A. N. et al., 2000, Nature Genetics, vol. 26, pp. 71-75. The fact that variations in the ATP6V0A4 gene expression can become an indication of metastasis of kidney cancer is not known.

The term "PRODH2 gene" or "PRODH2" as used herein includes a gene (or DNA) encoding the Nephrin gene (or DNA) as shown in a given nucleotide sequence (i.e., SEQ ID NO: 40), a homolog thereof, a mutant thereof, a derivative thereof, or the like, unless it is defined by a SEQ ID NO. Specifically, it includes the PRODH2 gene (Ensembl Gene ID, ENSG00000161270) as shown in SEQ ID NO: 40 and homologs thereof derived from other organism species. The PRODH2 gene can be obtained by the method disclosed in, for example, Chakravarti, A. et al., 2002, Proceedings of the National Academic Sciences, U.S.A., vol. 99, pp. 4755-4756. The fact that variations in the PRODH2 gene expression can become an indication of metastasis of kidney cancer is not known.

The term "EPHB2 gene" or "EPHB2" as used herein includes a gene (or DNA) encoding the Ephrin Type-B Receptor 2 gene (or DNA) as shown in a given nucleotide sequence (i.e., SEQ ID NO: 41), a homolog thereof, a mutant thereof, a derivative thereof, or the like, unless it is defined by a SEQ ID NO. Specifically, it includes the EPHB2 gene (Ensembl Gene ID, ENSG00000133216) as shown in SEQ ID NO: 41 and homologs thereof derived from other organism species. The EPHB2 gene can be obtained by the method disclosed in, for example, Chan, J. et al., 1991, Oncogene, vol. 6, pp. 1057-1061. The fact that variations in the EPHB2 gene expression can become an indication of metastasis of kidney cancer is not known.

The term "LYAR gene" or "LYAR" as used herein includes a gene (or DNA) encoding the Hypothetical Protein FLJ20425 gene (or DNA) as shown in a given nucleotide sequence (i.e., SEQ ID NO: 42), a homolog thereof, a mutant thereof, a derivative thereof, or the like, unless it is defined by a SEQ ID NO. Specifically, it includes the LYAR gene (Ensembl Gene ID, ENSG00000145220) as shown in SEQ ID NO: 42 and homologs thereof derived from other organism species. The LYAR gene can be obtained by the method disclosed in, for example, Su, L. et al., 1993, Genes Development, vol. 7, pp. 735-748. The fact that variations in the LYAR gene expression can become an indication of metastasis of kidney cancer is not known.

The term "COX6B gene" or "COX6B" as used herein includes a gene (or DNA) encoding the Cytochrome C Oxidase Polypeptide VIB gene (or DNA) as shown in a given nucleotide sequence (i.e., SEQ ID NO: 43), a homolog thereof, a mutant thereof, a derivative thereof, or the like, unless it is defined by a SEQ ID NO. Specifically, it includes the COX6B gene (Ensembl Gene ID, ENSG00000126267) as shown in SEQ ID NO: 43 and homologs thereof derived from other organism species. The COX6B gene can be obtained by the method disclosed in, for example, Taanman, J-W. et al., 1989, Nucleic Acids Research, vol. 17, pp. 1766. The fact that variations in the COX6B gene expression can become an indication of metastasis of kidney cancer is not known.

The term "PRH1 gene" or "PRH1" as used herein includes a gene (or DNA) encoding the Salivary Acidic Proline-Rich Phosphoprotein 1/2 gene (or DNA) as shown in a given nucleotide sequence (i.e., SEQ ID NO: 44), a homolog thereof, a mutant thereof, a derivative thereof, or the like, unless it is defined by a SEQ ID NO. Specifically, it includes the PRH1 gene (Ensembl Gene ID, ENSG00000176621) as shown in SEQ ID NO: 44 and homologs thereof derived from other organism species. The PRH1 gene can be obtained by the method disclosed in, for example, Oppenheim, F. G. et al., 1971, Biochemistry, vol. 10, pp. 4233-4238. The fact that variations in the PRH1 gene expression can become an indication of metastasis of kidney cancer is not known.

The term "LAPTM5 gene" or "LAPTM5" as used herein includes a gene (or DNA) encoding the Lysosomal-Associated Multitransmembrane Protein gene (or DNA) as shown in a given nucleotide sequence (i.e., SEQ ID NO: 45), a homolog thereof, a mutant thereof, a derivative thereof, or the like, unless it is defined by a SEQ ID NO. Specifically, it includes the LAPTM5 gene (Ensembl Gene ID, ENSG00000162511) as shown in SEQ ID NO: 45 and homologs thereof derived from other organism species. The LAPTM5 gene can be obtained by the method disclosed in, for example, Adra, C. N. et al., 1996, Genomics, vol. 35, pp. 328-337. The fact that variations in the LAPTM5 gene expression can become an indication of metastasis of kidney cancer is not known; however, the possibility that LAPTM5 may become a marker for kidney cancer is presumed in WO 2005-24603.

The term "RPS6KA4 gene" or "RPS6KA4" as used herein includes a gene (or DNA) encoding the Ribosomal Protein S6 Kinase, 90KDA, Polypeptide 4 gene (or DNA) as shown in a given nucleotide sequence (i.e., SEQ ID NO: 46), a homolog thereof, a mutant thereof, a derivative thereof, or the like, unless it is defined by a SEQ ID NO. Specifically, it includes the RPS6KA4 gene (Ensembl Gene ID, ENSG00000162302) as shown in SEQ ID NO: 46 and homologs thereof derived from other organism species. The RPS6KA4 gene can be obtained by the method disclosed in, for example, Pierrat, B. et al., 1998, Journal of Biological Chemistry, vol. 273, pp. 29661-29671. The fact that variations in the RPS6KA4 gene expression can become an indication of metastasis of kidney cancer is not known.

The term "GCC2 gene" or "GCC2" as used herein includes a gene (or DNA) encoding the GRIP and Coiled-Coil Domain Containing 2 gene (or DNA) as shown in a given nucleotide sequence (i.e., SEQ ID NO: 47), a homolog thereof, a mutant thereof, a derivative thereof, or the like, unless it is defined by a SEQ ID NO. Specifically, it includes the GCC2 gene (Ensembl Gene ID, ENSG00000144055) as shown in SEQ ID NO: 47 and homologs thereof derived from other organism species. The GCC2 gene can be obtained by the method disclosed in, for example, Eichmuller, S. et al., 2001, Proceedings of the National Academic Sciences, U.S.A., vol. 98, pp. 629-634. The fact that variations in the GCC2 gene expression can become an indication of metastasis of kidney cancer is not known.

The term "FGF2 gene" or "FGF2" as used herein includes a gene (or DNA) encoding the Heparin-Binding Growth Factor 2 gene (or DNA) as shown in a given nucleotide sequence (i.e., SEQ ID NO: 48), a homolog thereof, a mutant thereof, a derivative thereof, or the like, unless it is defined by a SEQ ID NO. Specifically, it includes the FGF2 gene (Ensembl Gene ID, ENSG00000138685) as shown in SEQ ID NO: 48 and homologs thereof derived from other organism species. The FGF2 gene can be obtained by the method disclosed in, for example, Abraham, J. et al., 1986, Science, vol. 233, pp. 545-548. The fact that variations in the FGF2 gene expression can become an indication of metastasis of kidney cancer is known as disclosed in, for example, Miyake, H. et al., 1996, Cancer Research, vol. 56, pp. 2440-2445.

The term "MMP14 gene" or "MMP 14" as used herein includes a gene (or DNA) encoding the Matrix Metalloproteinase-14 gene (or DNA) as shown in a given nucleotide sequence (i.e., SEQ ID NO: 49), a homolog thereof, a mutant thereof, a derivative thereof, or the like, unless it is defined by a SEQ ID NO. Specifically, it includes the MMP14 gene (Ensembl Gene ID, ENSG00000157227) as shown in SEQ ID NO: 49 and homologs thereof derived from other organism species. The MMP14 gene can be obtained by the method disclosed in, for example, Sato, H. et al., 1994, Nature, vol. 370, pp. 61-65. The fact that variations in the MMP14 gene expression can become an indication of metastasis of kidney cancer is known as disclosed in, for example, Kitagawa, Y. et al., 1999, Journal of Urology, vol. 162, pp. 905-909

The term "ERBB2 gene" or "ERBB2" as used herein includes a gene (or DNA) encoding the Receptor Protein-Tyrosine Kinase ERBB-2 gene (or DNA) as shown in a given nucleotide sequence (i.e., SEQ ID NO: 50), a homolog thereof, a mutant thereof, a derivative thereof, or the like, unless it is defined by a SEQ ID NO. Specifically, it includes the ERBB2 gene (Ensembl Gene ID, ENSG00000141736) as shown in SEQ ID NO: 50 and homologs thereof derived from other organism species. The ERBB2 gene can be obtained by the method disclosed in, for example, Yang-Feng, T. L. et al., 1985, Citogenetic Cell Genetics, vol. 40, pp. 784. The fact that variations in the ERBB2 gene expression can become an indication of metastasis of kidney cancer is known as disclosed in, for example, Freeman, M. R. et al., 1989, Cancer Research, vol. 49, pp. 6221-6225.

Other genes or polypeptides described herein are adequately described in the following description.

## Effects of the Invention

The present invention provides a composition for diagnosing a disease, which is useful for diagnosing, detecting, identifying, or predicting the presence or metastasis of kidney cancer and for treating kidney cancer, and a method for diagnosing, detecting, identifying, or predicting the presence or metastasis of kidney cancer using said composition. Thus, the present invention provides remarkable effects by providing a rapid and simple method for detecting, identifying, or predicting the presence or metastasis of kidney cancer with high specificity and high efficiency of prediction.

Some of the markers for kidney cancer of the present invention are found in biological samples, such as blood taken from patients with kidney cancer. Since the above-mentioned markers are not or almost not found in healthy persons, the presence or amount of the markers can be used as the indication to easily detect kidney cancer in the blood, for example.

The present invention enables effective diagnosis for managing the prognosis for patients with kidney cancer by using the probes for detecting kidney cancer of the present invention in combination with the probes for detecting metastasis of kidney cancer of the present invention.

## BRIEF DESCRIPTION OF THE DRAWINGS

Fig. 1 shows the survival curves prepared by the Kaplan-Meier method concerning a group of patients in which metastasis to organs other than the kidney was not diagnosed at the time of surgery (M = 0) and concerning a group of patients in which metastasis was diagnosed (M = 1). The vertical axis indicates a survival rate, and the horizontal axis indicates years after surgery. The 5-year survival was 96% (M = 0) and 13% (M = 1), respectively.

Fig. 2 shows the probability of predicting patients with good prognoses when the polynucleotides shown in SEQ ID NOS: 1 to 19 and 48 corresponding to the genes indicated in Table 1 are used in combination as a DNA chip, and the probability of predicting patients with poor prognoses when the polynucleotides shown in SEQ ID NOS: 20 to 47, 49, and 50 corresponding to the genes are used in combination as a DNA chip. The vertical axis indicates the probability of predicting patients with good prognoses or poor prognoses, and the horizontal axis indicates the total number of genes required for predicting patients with good prognoses or poor prognoses. The solid line indicates the probability of predicting patients with good prognoses, and the broken line indicates the probability of predicting patients with poor prognoses.

## PREFERRED EMBODIMENTS OF THE INVENTION

Hereafter, the present invention is described in more detail.

### 1. Kidney cancer-associated markers

#### 1.1 Kidney cancer-associated target nucleic acids

Examples of target nucleic acids as markers associated with metastasis of kidney cancer for detecting, identifying, or predicting the presence or metastasis of kidney cancer, for predicting the prognosis for a kidney cancer patient, or for identifying the presence or absence of cells to which kidney cancer has metastasized using a composition, kit, or DNA chip include human genes each comprising a nucleotide sequence as shown in any of SEQ ID NOS: 1 to 50 (i.e., PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2, respectively), homologs thereof, transcription products or cDNAs thereof, mutants thereof, and derivatives thereof. The terms "gene," "homolog," "transcription product," "cDNA," "mutant," and "derivative" are as defined above. The preferred target nucleic acids are human genes, each of which comprises a nucleotide sequence as shown in any of SEQ ID NOS: 1 to 50, and transcription products or cDNAs thereof, preferably transcription products or cDNAs.

According to the present invention, the expression levels of said genes, a target of the prediction of the prognosis for kidney cancer patients and/or a target of the prediction of the metastasis of kidney cancer, significantly change (i.e., increase or decrease) in the kidney cancer tissues from the patients with poor prognoses, when compared with the kidney cancer tissues from patients with good prognoses. Table 1 shows the Ensemble Gene ID numbers and the GenBank accession numbers of the genes collectively. Genes shown in Table 1 are the genes that recognize kidney cancer tissue from a patient with good prognosis and the genes that recognize kidney cancer tissue from a patient with poor prognosis.

Table 1

| SEQ ID NO: | Ensemble Gene ID | GenBank Acc. No. | Genes | SEQ ID NO: | Ensemble Gene ID | GenBank Acc. No. | Genes |
|---|---|---|---|---|---|---|---|
| 1 | ENSG00000100836 | NM_004643 | PABPN1 | 26 | ENSG00000179097 | NM_000866 | HTR1F |
| 2 | ENSG00000180056 | NM_032409 | PINK1 | 27 | ENSG00000101350 | NM_004798 | KIF3B |
| 3 | ENSG00000160181 | NM_005423 | TFF2 | 28 | ENSG00000011465 | NM_133506 | DCN |
| 4 | ENSG00000106263 | NM_003751 | EIF3S9 | 29 | ENSG00000162526 | NM_052841 | STK22C |
| 5 | ENSG00000125952 | NM_002382 | MAX | 30 | ENSG00000055070 | BX640985 | DKFZp566C0424 |
| 6 | ENSG00000105663 | NM_014727 | MLL4 | 31 | ENSG00000118432 | NM_016083 | CNR1 |
| 7 | ENSG00000165995 | NM_000724 | CACNB2 | 32 | ENSG00000051128 | NM_004838 | HOMER3 |
| 8 | ENSG00000015171 | NM_006624 | ZMYND11 | 33 | ENSG00000177032 | NM_016602 | GPR2 |
| 9 | ENSG00000111960 | NM_004638 | BAT2 | 34 | ENSG00000168268 | NM_022908 | FLJ12442 |
| 10 | ENSG00000115216 | NM_013392 | NRBP | 35 | ENSG00000133800 | NM_006691 | XLKD1 |
| 11 | ENSG00000160294 | NM_003906 | MCM3AP | 36 | ENSG00000177303 | NM_020753 | CASKIN2 |
| 12 | ENSG00000139825 | NM_001845 | COL4A1 | 37 | ENSG00000179877 | NM_000393 | COL5A2 |
| 13 | ENSG00000079277 | NM_003684 | MKNK1 | 38 | ENSG00000169925 | NM_007371 | BRD3 |
| 14 | ENSG00000105327 | NM_014417 | BBC3 | 39 | ENSG00000105929 | NM_020632 | ATP6V0A4 |
| 15 | ENSG00000119285 | NM_018072 | FLJ10359 | 40 | ENSG00000161270 | NM_021232 | PRODH2 |
| 16 | ENSG00000143196 | NM_001937 | DPT | 41 | ENSG00000133216 | NM_004442 | EPHB2 |
| 17 | ENSG00000120029 | NM_024541 | C10orf76 | 42 | ENSG00000145220 | NM_017816 | LYAR |
| 18 | ENSG00000100243 | NM_007326 | DIA1 | 43 | ENSG00000126267 | NM_001863 | COX6B |
| 19 | ENSG00000168078 | NM_018492 | PBK | 44 | ENSG00000176621 | NM_006250 | PRH2 |
| 20 | ENSG00000105287 | NM_016457 | PRKD2 | 45 | ENSG00000162511 | NM_006762 | LAPTM5 |
| 21 | ENSG00000171345 | NM_002276 | KRT19 | 46 | ENSG00000162302 | NM_003942 | RPS6KA4 |
| 22 | ENSG00000169957 | NM_024671 | FLJ23436 | 47 | ENSG00000144055 | NM_181453, NM 014635 | GCC2 |
| 23 | ENSG00000116218 | NM_014625 | NPHS2 | 48 | ENSG00000138685 | NM_002006 | FGF2 |
| 24 | ENSG00000114405 | NM_020685 | C3orf14 | 49 | ENSG00000157227 | NM_004995 | MMP14 |
| 25 | ENSG00000180772 | NM_000686 | AGTR2 | 50 | ENSG00000141736 | NM_004448 | ERBB2 |

The 1st target nucleic acids are the PABPN1 gene, homologs thereof, transcription products or cDNAs thereof, mutants thereof, or derivatives thereof.

The 2nd target nucleic acids are the PINK1 gene, homologs thereof, transcription products or cDNAs thereof, mutants thereof, or derivatives thereof.

The 3rd target nucleic acids are the TFF2 gene, homologs thereof, transcription products or cDNAs thereof, mutants thereof, or derivatives thereof.

The 4th target nucleic acids are the EIF3S9 gene, homologs thereof, transcription products or cDNAs thereof, mutants thereof, or derivatives thereof.

The 5th target nucleic acids are the MAX gene, homologs thereof, transcription products or cDNAs thereof, mutants thereof, or derivatives thereof.

The 6th target nucleic acids are the MLL4 gene, homologs thereof, transcription products or cDNAs thereof, mutants thereof, or derivatives thereof.

The 7th target nucleic acids are the CACNB2 gene, homologs thereof, transcription products or cDNAs thereof, mutants thereof, or derivatives thereof.

The 8th target nucleic acids are the ZMYND11 gene, homologs thereof, transcription products or cDNAs thereof, mutants thereof, or derivatives thereof.

The 9th target nucleic acids are the BAT2 gene, homologs thereof, transcription products or cDNAs thereof, mutants thereof, or derivatives thereof.

The 10th target nucleic acids are the NRBP gene, homologs thereof, transcription products or cDNAs thereof, mutants thereof, or derivatives thereof.

The 11th target nucleic acids are the MCM3AP gene, homologs thereof, transcription products or cDNAs thereof, mutants thereof, or derivatives thereof.

The 12th target nucleic acids are the COL4A1 gene, homologs thereof, transcription products or cDNAs thereof, mutants thereof, or derivatives thereof.

The 13th target nucleic acids are the MKNK1 gene, homologs thereof, transcription products or cDNAs thereof, mutants thereof, or derivatives thereof.

The 14th target nucleic acids are the BBC3 gene, homologs thereof, transcription products or cDNAs thereof, mutants thereof, or derivatives thereof.

The 15th target nucleic acids are the FLJ10359 gene, homologs thereof, transcription products or cDNAs thereof, mutants thereof, or derivatives thereof.

The 16th target nucleic acids are the DPT gene, homologs thereof, transcription products or cDNAs thereof, mutants thereof, or derivatives thereof.

The 17th target nucleic acids are the C10orf76 gene, homologs thereof, transcription products or cDNAs thereof, mutants thereof, or derivatives thereof.

The 18th target nucleic acids are the DIA1 gene, homologs thereof, transcription products or cDNAs thereof, mutants thereof, or derivatives thereof.

The 19th target nucleic acids are the PBK gene, homologs thereof, transcription products or cDNAs thereof, mutants thereof, or derivatives thereof.

The 20th target nucleic acids are the PRKD2 gene, homologs thereof, transcription products or cDNAs thereof, mutants thereof, or derivatives thereof.

The 21st target nucleic acids are the KRT19 gene, homologs thereof, transcription products or cDNAs thereof, mutants thereof, or derivatives thereof.

The 22nd target nucleic acids are the FLJ23436 gene, homologs thereof, transcription products or cDNAs thereof, mutants thereof, or derivatives thereof.

The 23rd target nucleic acids are the NPHS2 gene, homologs thereof, transcription products or cDNAs thereof, mutants thereof, or derivatives thereof.

The 24th target nucleic acids are the C3orf14 gene, homologs thereof, transcription products or cDNAs thereof, mutants thereof, or derivatives thereof.

The 25th target nucleic acids are the AGTR2 gene, homologs thereof, transcription products or cDNAs thereof, mutants thereof, or derivatives thereof.

The 26th target nucleic acids are the HTR1F gene, homologs thereof, transcription products or cDNAs thereof, mutants thereof, or derivatives thereof.

The 27th target nucleic acids are the KIF3B gene, homologs thereof, transcription products or cDNAs thereof, mutants thereof, or derivatives thereof.

The 28th target nucleic acids are the DCN gene, homologs thereof, transcription products or cDNAs thereof, mutants thereof, or derivatives thereof.

The 29th target nucleic acids are the STK22C gene, homologs thereof, transcription products or cDNAs thereof, mutants thereof, or derivatives thereof.

The 30th target nucleic acids are the DKFZp566C0424 gene, homologs thereof, transcription products or cDNAs thereof, mutants thereof, or derivatives thereof.
The 31st target nucleic acids are the CNR1 gene, homologs thereof, transcription products or cDNAs thereof, mutants thereof, or derivatives thereof.
The 32nd target nucleic acids are the HOMER3 gene, homologs thereof, transcription products or cDNAs thereof, mutants thereof, or derivatives thereof.
The 33rd target nucleic acids are the GPR2 gene, homologs thereof, transcription products or cDNAs thereof, mutants thereof, or derivatives thereof.
The 34th target nucleic acids are the FLJ12442 gene, homologs thereof, transcription products or cDNAs thereof, mutants thereof, or derivatives thereof.
The 35th target nucleic acids are the XLKD1 gene, homologs thereof, transcription products or cDNAs thereof, mutants thereof, or derivatives thereof.
The 36th target nucleic acids are the CASKIN2 gene, homologs thereof, transcription products or cDNAs thereof, mutants thereof, or derivatives thereof.
The 37th target nucleic acids are the COL5A2 gene, homologs thereof, transcription products or cDNAs thereof, mutants thereof, or derivatives thereof.
The 38th target nucleic acids are the BRD3 gene, homologs thereof, transcription products or cDNAs thereof, mutants thereof, or derivatives thereof.
The 39th target nucleic acids are the ATP6V0A4 gene, homologs thereof, transcription products or cDNAs thereof, mutants thereof, or derivatives thereof.
The 40th target nucleic acids are the PRODH2 gene, homologs thereof, transcription products or cDNAs thereof, mutants thereof, or derivatives thereof.
The 41st target nucleic acids are the EPHB2 gene, homologs thereof, transcription products or cDNAs thereof, mutants thereof, or derivatives thereof.
The 42nd target nucleic acids are the LYAR gene, homologs thereof, transcription products or cDNAs thereof, mutants thereof, or derivatives thereof.
The 43rd target nucleic acids are the COX6B gene, homologs thereof, transcription products or cDNAs thereof, mutants thereof, or derivatives thereof.
The 44th target nucleic acids are the PRH1 gene, homologs thereof, transcription products or cDNAs thereof, mutants thereof, or derivatives thereof.
The 45th target nucleic acids are the LAPTM5 gene, homologs thereof, transcription products or cDNAs thereof, mutants thereof, or derivatives thereof.
The 46th target nucleic acids are the RPS6KA4 gene, homologs thereof, transcription products or cDNAs thereof, mutants thereof, or derivatives thereof.
The 47th target nucleic acids are the GCC2 gene, homologs thereof, transcription products or cDNAs thereof, mutants thereof, or derivatives thereof.
The 48th target nucleic acids are the FGF2 gene, homologs thereof, transcription products or cDNAs thereof, mutants thereof, or derivatives thereof.
The 49th target nucleic acids are the MMP14 gene, homologs thereof, transcription products or cDNAs thereof, mutants thereof, or derivatives thereof.
The 50th target nucleic acids are the ERBB2 gene, homologs thereof, transcription products or cDNAs thereof, mutants thereof, or derivatives thereof.

1.2 Kidney cancer-associated target polypeptide (1)

Examples of target polypeptides as markers associated with the kidney cancer for detecting, identifying, or predicting the presence or metastasis of kidney cancer, for predicting the prognosis for a kidney cancer patient, or for identifying the presence or absence of cells to which kidney cancer has metastasized using acomposition or kit include polypeptides encoded by PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2 genes, such as human polypeptides each comprising an amino acid sequence as shown in SEQ ID NOS: 101 to 150, homologs thereof, mutants thereof, or derivatives thereof. The terms "polypeptide," "homolog," "mutant," and "derivative" are as defined above. Examples of preferable target polypeptides are human polypeptides each comprising an amino acid sequence as shown in SEQ ID NOS: 101 to 110, 112 to 120, and 122 to 147.
According to the present invention, the expression levels of the polypeptides, which are a target of the prediction of the prognosis for kidney cancer patients and/or a target of the prediction of the metastasis of kidney cancer, significantly change (i.e., increase or decrease) in the kidney cancer tissues from the patients with poor prognoses, when compared with the kidney cancer tissues from patients with good prognoses, as with the expression levels of the corresponding genes and the transcription products thereof. Alternatively, the levels of such polypeptides in the blood significantly change (i.e., increase or decrease) in the kidney cancer patients with poor prognoses, when compared with the kidney cancer patients with good prognoses.

1.3 Kidney cancer-associated target polypeptide (2)

Other kidney cancer-associated target polypeptides as markers for detecting kidney cancer *in vitro* using the composition or kit are polypeptides comprising the amino acid sequences as shown in SEQ ID NOS: 151 to 197, preferably SEQ ID NOS: 151, 153, 155 to 160, or SEQ ID NOS: 161 to 190, mutants thereof, or fragments thereof.
Polypeptides as shown in SEQ ID NOS: 151 to 160 and 191 and in SEQ ID NOS: 161 to 190, and 192 to 197 are shown in Tables 2 and 3 with the gene names, protein numbers (GenBank names and accession numbers), and properties. The listed polypeptides are detected specifically in, for example, blood plasma of patients with kidney cancer, whereas they are not detected or are much lower than the detectable level in blood plasmas of healthy persons.

Table 2

| SEQ ID NO: | Gene name | Protein No. | Properties |
|---|---|---|---|
| 151 | AAK2 | P54646 | AMP kinase 2 |
| 152 | SLK4 | Q8IW52 | SLIT and NTRK-like protein 4 precursor |
| 153 | SP4L | Q8TC36 | Sperm-associated antigen 4-like protein (spermary and spermatogenesis-associated gene 4 protein) |
| 154 | C5P1 | Q96SZ6 | CDK5 regulatory subunit-associated protein 1 (CDK5 activator-binding protein C42) (CGI-05) |
| 155 | SI8A | Q92185 | $\alpha$-N-acetyl-neuraminide $\alpha$-2,8-sialyltransferase (EC 2.4.99.8) (ganglioside GD3 synthase) |
| 156 | KAP2 | P13861 | cAMP-dependent protein kinase type II-$\alpha$ regulatory chain |
| 157 | F262 | 060825 | 6-Phosphofructo-2-kinase/fructose-2,6-biphosphatase 2 (6PF-2-K/Fru-2,6-P2ASE heart isozyme) (PFK-2/FBPase-2)[6-phosphofructo-2-kinase (EC 2.7.1.105); containing fructose-2,6-biphosphatase (EC 3.1.3.46)] |
| 158 | TCPH | Q99832 | T-complex protein 1, eta subunit (TCP-1-eta) (CCT-eta) (HIV-1 Nef interacting protein) |
| 159 | GB11 | P29992 | Interacting protein-binding protein G(y), $\alpha$ subunit ($\alpha$-11) |
| 160 | CT67 | Q9H4Z3 | Protein C20orf67 |
| 191 | CEGT | Q16739 | Ceramide glucosyltransferase (EC 2.4.1.80) (glucosyltransferase) (GCS) (UDP-glucose) |

Table 3

| SEQ ID NO: | Gene name | Protein No. | Properties |
|---|---|---|---|
| 161 | AMHR2 | Q16671 | Anti-muellerian hormone type II receptor precursor (EC 2.7.1.37) (AMH type II receptor) (MIS type II receptor) (MISRII) (MRII) |
| 162 | APOL3 | 095236 | Apolipoprotein L3 (apolipoprotein L-III) (ApoL-III) (TNF-inducible protein CG12-1) |
| 163 | DEDD2 | Q8WXF8 | DNA-binding death effector domain-containing protein 2 (DED-containing protein FLAME-3) |

(continued)

| SEQ ID NO: | Gene name | Protein No. | Properties |
|---|---|---|---|
| 164 | HEXB | P07686 | β-Hexosaminidase P chain precursor (EC 3.2.1.52) (N-acetyl-β-glucosaminidase) |
| 165 | HNRPK | P61978 | Heterogeneous nuclear ribonucleoprotein K (hnRNP K) |
| 166 | KPNA1 | P52294 | Importin α-1 subunit (karyopherin α-1 subunit) (SRP1-β) (RAG protein 2) (nucleoprotein interactor 1) (NPI-1) |
| 167 | LASP1 | Q14847 | LIM and SH3 domain protein (LASP-1) (MLN 50) |
| 168 | LIAS | 043766 | Lipoic acid synthetase, mitochondrial precursor (Lip-syn) (lipoic acid synthetase) (HUSSY-01) |
| 169 | MAGEC3 | Q8TD91 | Melanoma-associated antigen C3 (MAGE-C3 antigen) |
| 170 | NFATC4 | Q14934 | Nuclear factor of activated T cells, cytoplam 4 (T cell transcription factor NFAT3) (NF-ATc4) (NF-AT3) |
| 171 | OLFM3 | Q96PB7 | Noelin 3 precursor (olfactomedin 3) (optimedin) (UNQ1924/PRO4399) |
| 172 | POLR3F | Q9H1D9 | DNA-dependent RNA polymerase III 39 kDa polypeptide (EC 2.7.7.6) |
| 173 | PPP3CB | NP_066955 | Serine/threonine protein phosphatase 2B catalyst subunit, P isoform (EC 3.1.3.16) |
| 174 | SF1 | Q15637 | Splicing factor 1 (zinc finger protein 162) (transcription factor ZFM1) |
| 175 | SLC24A2 | Q9UI40 | Sodium/potassium/calcium exchanger 2 precursor (Na(+)/K(+)/Ca(2+)-exchange protein 2) |
| 176 | SLC26A4 | 043511 | Pendrin (sodium-independent chlorine/iodine transporter) |
| 177 | SLCO1A2 | P46721 | Solute carrier organic anion transporter family member 1A2 |
| 178 | TPM2 | P07951 | Tropomyosin β chain (tropomyosin 2) (β-tropomyosin) |
| 179 | UCHL5 | Q9Y5K5 | Ubiquitin carboxyl terminal hydrolase isozyme L5 (EC 3.4.19.12) (UCH-L5) |
| 180 | UGT8 | Q16880 | 2-Hydroxyacyl sphingosine 1-β-galactosyl transferase precursor (EC 2.4.1.45) (UDP- galactosylceramide transferase) |
| 181 | ZNF140 | P52738 | Zinc finger protein 140 |
| 182 | ZNF274 | Q96GC6 | Zinc finger protein 274 (zinc finger protein SP2114) (zinc finger protein HFB101) |
| 183 | MID2 | Q9UJV3 | Midline 2 |
| 184 | LIPE | Q05469 | Lipase, hormone-sensitive |
| 185 | HDAC6 | Q9UBN7 | Histone deacetylase 6 |
| 186 | ACO2 | Q99798 | Aconitase 2, mitochondria |
| 187 | APLP1 | P51693 | Amyloid β (A4) precursor-like protein 1 |
| 188 | NUMB | P49757 | Numb homolog (Drosophila) |
| 189 | ARHGEF7 | Q14155 | Rho guanine nucleotide exchange factor (GEF)7 |
| 190 | GNAQ | P50148 | Guanine nucleotide-binding protein (G protein), q polypeptide |
| 191 | MMP2 | P08253 | 72 kDa type IV collagenase precursor (EC 3.4.24.24) (72 kDa gelatinase) |
| 192 | TNFRSF7 | P26842 | Tumor necrosis factor receptor superfamily member 7 precursor (CD27L receptor) |

(continued)

| SEQ ID NO: | Gene name | Protein No. | Properties |
|---|---|---|---|
| 193 | PDE8B | 095263 | High-affinity cAMP-specific and IBMX-nonsensitive 3',5'-cyclic hosphodiesterase 8B |
| 194 | FLOT1 | 075955 | Flotillin 1 |
| 195 | CD5 | P06127 | T-cell surface glycoprotein CD5 [precursor] |
| 196 | ECM1 | Q16610 | Extracellular matrix protein 1 |

According to the present invention, the levels of the target polypeptides for detecting kidney cancer in a biological sample such as blood are significantly or remarkably high in a subject with kidney cancer, when compared with healthy persons.

2. Probes for diagnosing kidney cancer

The probes for detecting, identifying, or predicting the presence and/or metastasis of kidney cancer or for predicting the subject's prognosis after surgery are selected from the probes of group I and/or group II:

group I: polynucleotides consisting of:

(a) a polynucleotide consisting of a nucleotide sequence as shown in any of SEQ ID NOS: 1 to 10, 12 to 20, and 22 to 47, a mutant thereof, or a fragment comprising at least 15 continuous nucleotides thereof,
(b) a polynucleotide comprising a nucleotide sequence as shown in any of SEQ ID NOS: 1 to 10, 12 to 20, and 22 to 47,
(c) a polynucleotide consisting of a nucleotide sequence complementary to a nucleotide sequence as shown in any of SEQ ID NOS: 1 to 10, 12 to 20, and 22 to 47, a mutant thereof, or a fragment comprising at least 15 continuous nucleotides thereof,
(d) a polynucleotide comprising a nucleotide sequence complementary to a nucleotide sequence as shown in any of SEQ ID NOS: 1 to 10, 12 to 20, and 22 to 47, and
(e) a polynucleotide hybridizing under stringent conditions to any of the polynucleotides (a) to (d), or a fragment comprising at least 15 continuous nucleotides thereof; and

group II: antibodies, fragments thereof or chemically modified derivatives thereof consisting of:

(f) an antibody specifically binding to at least one of a polypeptide encoded by a nucleotide sequence as shown in any of SEQ ID NOS: 1 to 10, 12 to 20, and 22 to 47 or a polypeptide having an amino acid sequence as shown in any of SEQ ID NOS: 101 to 110, 112 to 120, and 122 to 147, a mutant thereof, and a fragment thereof, a fragment of the antibody, or a chemically modified derivative of the antibody or fragment,
(g) an antibody specifically binding to at least one of a polypeptide having an amino acid sequence as shown in any of SEQ ID NOS: 151, 153, and 155 to 160, a mutant thereof, and a fragment thereof, a fragment of the antibody, or a chemically modified derivative of the antibody, and
(h) an antibody specifically binding to at least one of a polypeptide having an amino acid sequence as shown in any of SEQ ID NOS: 161 to 190, a mutant thereof, and a fragment thereof, a fragment of the antibody, or a chemically modified derivative of the antibody.

All of the above-described probes can bind to any of the kidney cancer-associated markers described in Sections 1.1 to 1.3 above, and they can be used to detect, identify or predict the presence or metastasis of kidney cancer. For example, any probe of group I and group II (f) enables detection, identification, or prediction of the presence or metastasis of kidney cancer. Also, any probe of group II (g) and (h) enables detection, identification, or prediction of the presence of kidney cancer.

The nucleic acid probe includes DNA or RNA, and the antibody probe includes, for example, a polyclonal antibody, a monoclonal antibody, a fragment thereof, a synthetic antibody, a recombinant antibody, a polyspecific antibody, and a single-chain antibody.

We have now found that the probes as described in group I and/or group II could be used for detecting, identifying, or predicting the presence and/or metastasis of kidney cancer for the first time. The probes which are according to the present invention are selected from the group of polynudeotides as defined in any of claims 1 to 11.

3. Composition for diagnosing kidney cancer and/or for predicting the prognosis or metastasis of kidney cancer

3.1 Nucleic acid composition

According to the present invention, the nucleic acid composition for detecting, identifying, or predicting the presence and metastasis of kidney cancer, for predicting the prognosis for a kidney cancer patient, and for identifying the presence or absence of to which kidney cancer has metastasized using the composition or kit of the present invention comprises one or more probes as described in Section 2 above. Such composition enables qualitative and/or quantitative measurements of the presence, expression level, or amount of human derived PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2 genes, homologs thereof, transcription products or cDNA thereof, mutants thereof, or derivatives thereof, as target nucleic acids for predicting the prognosis or metastasis of kidney cancer.

The expression levels of said target nucleic acids significantly change (i.e., increase or decrease) in the kidney cancer tissue from a patient with poor prognosis, when compared with the kidney cancer tissue from a patient with good prognosis. Accordingly, the composition of the present invention can be effectively used for measuring and comparing the expression levels of the target nucleic acids both in the kidney cancer tissue from a patient with good prognosis and in the kidney cancer tissue from a patient with poor prognosis.

The compositions usable include a combination of one or more polynucleotides selected from: polynucleotides comprising the nucleotide sequences as shown in SEQ ID NOS: 1 to 50 as observed in the body tissue of a patient with kidney cancer, and polynucleotides complementary thereto; polynucleotides hybridizing under stringent conditions to DNA consisting of nucleotide sequences complementary to said nucleotide sequences, and polynucleotides complementary thereto; and polynucleotides comprising at least 15 continuous nucleotides in the nucleotide sequences of said polynucleotides.

Specifically, the composition can comprise one or more polynucleotides or fragments thereof set forth below:

(1) polynucleotides each consisting of a nucleotide sequence as shown in any of SEQ ID NOS: 1 to 50, mutants thereof, or fragments comprising at least 15 continuous nucleotides thereof;
(2) polynucleotides each comprising a nucleotide sequence as shown in any of SEQ ID NOS: 1 to 50;
(3) polynucleotides each consisting of a nucleotide sequence as shown in any of SEQ ID NOS: 1 to 10, 12 to 20, and 22 to 47, mutants thereof, or fragments comprising at least 15 continuous nucleotides thereof;
(4) polynucleotides each comprising a nucleotide sequence as shown in any of SEQ ID NOS: 1 to 10, 12 to 20, and 22 to 47;
(5) polynucleotides each consisting of a nucleotide sequence complementary to a nucleotide sequence as shown in any of SEQ ID NOS: 1 to 10, 12 to 20, and 22 to 47, mutants thereof, or fragments comprising at least 15 continuous nucleotides thereof;
(6) polynucleotides each comprising a nucleotide sequence complementary to a nucleotide sequence as shown in any of SEQ ID NOS: 1 to 10, 12 to 20, and 22 to 47;
(7) polynucleotides each hybridizing under stringent conditions to DNA consisting of a nucleotide sequence complementary to a nucleotide sequence as shown in any of SEQ ID NOS: 1 to 10, 12 to 20, and 22 to 47, or fragments comprising at least 15 continuous nucleotides thereof;
(8) polynucleotides each hybridizing under stringent conditions to DNA consisting of a nucleotide sequence as shown in any of SEQ ID NOS: 1 to 10, 12 to 20, and 22 to 47 or fragments comprising at least 15 continuous nucleotides thereof;
(9) a polynucleotide consisting of the nucleotide sequence as shown in any of SEQ ID NOS: 1 to 11, 21, and 48 to 50, mutants thereof, or fragments comprising at least 15 continuous nucleotides thereof;
(10) polynucleotides comprising a nucleotide sequence as shown in any of SEQ ID NOS: 11, 21, and 48 to 50;
(11) polynucleotides consisting of a nucleotide sequence complementary to a nucleotide sequence as shown in SEQ ID NOS: 11, 21, and 48 to 50, mutants thereof, or fragments comprising at least 15 continuous nucleotides thereof;
(12) polynucleotides comprising a nucleotide sequence complementary to the nucleotide sequence as shown in any of SEQ ID NOS: 11, 21, and 48 to 50;
(13) polynucleotides each hybridizing under stringent conditions to DNA consisting of a nucleotide sequence complementary to the nucleotide sequence as shown in any of SEQ ID NOS: 11, 21, and 48 to 50 or fragments comprising at least 15 continuous nucleotides thereof; and

(14) polynucleotides each hybridizing under stringent conditions to DNA consisting of a nucleotide sequence as shown in any of SEQ ID NOS: 11, 21, and 48 to 50 or fragments comprising at least 15 continuous nucleotides thereof.

Fragments of the polynucleotide as described in (1) to (14) above can include, but are not limited to, nucleotide sequences of, for example, continuous 15 to all nucleotides, 15 to 5000 nucleotides, 15 to 4500 nucleotides, 15 to 4000 nucleotides, 15 to 3500 nucleotides, 15 to 3000 nucleotides, 15 to 2500 nucleotides, 15 to 2000 nucleotides, 15 to 1500 nucleotides, 15 to 1000 nucleotides, 15 to 900 nucleotides, 15 to 800 nucleotides, 15 to 700 nucleotides, 15 to 600 nucleotides, 15 to 500 nucleotides, 15 to 400 nucleotides, 15 to 300 nucleotides, 15 to 250 nucleotides, 15 to 200 nucleotides, 15 to 150 nucleotides, 15 to 140 nucleotides, 15 to 130 nucleotides, 15 to 120 nucleotides, 15 to 110 nucleotides, 15 to 100 nucleotides, 15 to 90 nucleotides, 15 to 80 nucleotides, 15 to 70 nucleotides, 15 to 60 nucleotides, 15 to 50 nucleotides, 15 to 40 nucleotides, 15 to 30 nucleotides or 15 to 25 nucleotides, 25 to all nucleotides, 25 to 1000 nucleotides, 25 to 900 nucleotides, 25 to 800 nucleotides, 25 to 700 nucleotides, 25 to 600 nucleotides, 25 to 500 nucleotides, 25 to 400 nucleotides, 25 to 300 nucleotides, 25 to 250 nucleotides, 25 to 200 nucleotides, 25 to 150 nucleotides, 25 to 140 nucleotides, 25 to 130 nucleotides, 25 to 120 nucleotides, 25 to 110 nucleotides, 25 to 100 nucleotides, 25 to 90 nucleotides, 25 to 80 nucleotides, 25 to 70 nucleotides, 25 to 60 nucleotides, 25 to 50 nucleotides or 25 to 40 nucleotides, 50 to all nucleotides, 50 to 1000 nucleotides, 50 to 900 nucleotides, 50 to 800 nucleotides, 50 to 700 nucleotides, 50 to 600 nucleotides, 50 to 500 nucleotides, 50 to 400 nucleotides, 50 to 300 nucleotides, 50 to 250 nucleotides, 50 to 200 nucleotides, 50 to 150 nucleotides, 50 to 140 nucleotides, 50 to 130 nucleotides, 50 to 120 nucleotides, 50 to 110 nucleotides, 50 to 100 nucleotides, 50 to 90 nucleotides, 50 to 80 nucleotides, 50 to 70 nucleotides or 50 to 60 nucleotides, 60 to all nucleotides, 60 to 1000 nucleotides, 60 to 900 nucleotides, 60 to 800 nucleotides, 60 to 700 nucleotides, 60 to 600 nucleotides, 60 to 500 nucleotides, 60 to 400 nucleotides, 60 to 300 nucleotides, 60 to 250 nucleotides, 60 to 200 nucleotides, 60 to 150 nucleotides, 60 to 140 nucleotides, 60 to 130 nucleotides, 60 to 120 nucleotides, 60 to 110 nucleotides, 60 to 100 nucleotides, 60 to 90 nucleotides, and 60 to 80 nucleotides or 60 to 70 nucleotides, and so on.

According to an embodiment, fragments of polynucleotides each comprising the nucleotide sequence as shown in any of SEQ ID NOS: 1 to 10, 12 to 20, and 22 to 47 preferably comprise a nucleotide sequence as shown in any of SEQ ID NOS: 51 to 60, 62 to 70, and 72 to 97, a complementary sequence thereof, or a partial sequence comprising at least 15 continuous nucleotides thereof.

The composition includes the following polynucleotide or polynucleotides, for example.

(1) a polynucleotide comprising at least 15 continuous nucleotides in each of the nucleotide sequence as shown in any of SEQ ID NOS: 1 to 10, 12 to 20, and 22 to 47 or complementary sequences thereof;

(2) a polynucleotide comprising at least 60 continuous nucleotides in each of the nucleotide sequence as shown in any of SEQ ID NOS: 1 to 10, 12 to 20, and 22 to 47 or complementary sequences thereof;

(3) a polynucleotide comprising at least 15 continuous nucleotides in the nucleotide sequence as shown in any of SEQ ID NOS: 11, 21, and 48 to 50 or a complementary sequence thereof;

(4) a polynucleotide comprising at least 60 continuous nucleotides in the nucleotide sequence as shown in any of SEQ ID NOS: 11, 21, and 48 to 50 or a complementary sequence thereof;

(5) a polynucleotide comprising a nucleotide sequence as shown in any of SEQ ID NOS: 51 to 60, 62 to 70, and 72 to 97 in the nucleotide sequence as shown in SEQ ID NOS: 1 to 10, 12 to 20, and 22 to 47, and comprising at least 60 continuous nucleotides;

(6) a polynucleotide comprising a sequence complementary to a nucleotide sequence as shown in any of SEQ ID NOS: 51 to 60, 62 to 70, and 72 to 97 in the sequences complementary to the nucleotide sequence as shown in any of SEQ ID NOS: 1 to 10, 12 to 20, and 22 to 47, and comprising at least 60 continuous nucleotides;

(7) a polynucleotide comprising the nucleotide sequence as shown in any of SEQ ID NOS: 61, 71, and 98 to 100 in the nucleotide sequence as shown in any of SEQ ID NOS: 11, 21, and 48 to 50, and comprising at least 60 continuous nucleotides; and

(8) a polynucleotide comprising a sequence complementary to the nucleotide sequence as shown in any of SEQ ID NOS: 61, 71, and 98 to 100 in the sequence complementary to the nucleotide sequence as shown in any of SEQ ID NOS: 11, 21, and 48 to 50, and comprising at least 60 continuous nucleotides.

The polynucleotides or fragments thereof as used may be DNA or RNA.

Polynucleotides in the compositions can be prepared by common techniques such as recombinant DNA technology, PCR, or a method of using an automatic DNA/RNA synthesizer. Polynucleotides which are according to the present invention are selected from the group of polynucleotides as defined in any of claims 1 to 11. Further, the compositions which are according to the present invention are the compositions as defined in any of claims 1 to 11.

Recombinant DNA technology or PCR can include the use of the techniques as disclosed in, for example, Ausubel. et al., Current Protocols in Molecular Biology, John Willey & Sons, US (1993); or Sambrook et al., Molecular Cloning: A Laboratory Manual, Cold Spring Harbor Laboratory Press, US (1989).

The human-derived PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2 genes are known, and the methods for obtaining the same are also known. Thus, these genes can be cloned in order to prepare polynucleotides as the compositions of the present invention.

Polynucleotides constituting the compositions of the present invention may be chemically synthesized using an automatic DNA synthesizer. Such synthesis is generally carried out by the phosphoramidite method, which enables the automatic synthesis of a single-stranded DNA of at most about 100 nucleotides. The automatic DNA synthesizer is commercially available from, for example, Polygen, ABI, or Applied BioSystems.

Alternatively, the polynucleotides of the present invention can be prepared by cDNA cloning. Total RNA is extracted from a tissue of a living body, such as kidney tissue, in which the target gene or genes of the present invention is/are expressed, the extracted total RNA is applied to the oligo dT cellulose column to obtain poly A(+) RNA, cDNA library is prepared therefrom by RT-PCR, and the target cDNA clones can be obtained from the resulting library by a screening method such as hybridization screening, expression screening, or antibody screening. If necessary, the cDNA clones may be amplified by PCR. Probes or primers can be selected and synthesized from any sequences comprising 15 to 100 continuous nucleotides in the nucleotide sequences as shown in SEQ ID NOS: 1 to 50. The cDNA cloning technique is described in, for example, Sambrook, J. & Russel, D., Molecular Cloning, A LABORATORY MANUAL, Cold Spring Harbor Laboratory Press, January 15, 2001, vol. 1: 7.42 to 7.45, vol. 2: 8.9 to 8.17.

3.2 Antibody composition

The compositions can comprise, as probe for diagnosing kidney cancer, the antibodies described in group II of Section 2 above, fragments thereof, or chemically-modified derivatives thereof. Such compositions are useful for detecting, identifying, or predicting *in vitro* the presence and/or metastasis of kidney cancer in a subject afflicted with kidney cancer. In the present invention, prediction of metastasis can lead to prediction of good or poor prognosis for the subject after surgery.

(A) The first example of the antibody composition is a composition comprising: one or more antibodies against polypeptides having the amino acid sequence as shown in any of SEQ ID NOS: 101 to 110, 112 to 120, and 122 to 147 of group II (f), fragments thereof, or chemically modified derivatives thereof.
The composition can further comprise one or more antibodies against a polypeptide having the amino acid sequence as shown in any of SEQ ID NOS: 111, 121, and 148 to 150, a mutant of the polypeptide, or a fragment of the polypeptide; a fragment of the antibody; or a chemically modified derivative of the antibody. Use of such antibodies in combination can improve the accuracy for predicting the prognosis.
In order to detect polypeptides encoded by the PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2 genes, as markers for predicting the prognosis or metastasis of kidney cancer, homologs thereof, mutants thereof, or derivatives thereof, at least 1, and preferably at least 2, antibodies against such polypeptides can be used in combination..
(B) The second example of the antibody composition is a composition comprising: one or more antibodies against polypeptides having the amino acid sequence as shown in any of SEQ ID NOS: 151 to 160 and 191 and SEQ ID NOS: 161 to 190, and 192 to 197 of group II (g) and (h), respectively, fragments thereof, or chemically modified derivatives thereof.

Such polypeptides are encoded by the genes shown in Tables 1, 2, and 3. The nucleotide sequences of such genes can be easily obtained based on the gene names shown in the tables by accessing the NCBI website.

The above-mentioned polypeptides can be obtained by the recombinant DNA technology. For example, the cDNA clones obtained in the above-described manner are incorporated into an expression vector, which is then transformed or transfected into prokaryotic or eukaryotic host cells, and the resulting prokaryotic or eukaryotic host cells are cultured. Thus, polypeptides of interest can be obtained from the cells or culture supernatants. Vectors and expression systems are commercially available from Novagen, Takara Shuzo, Daiichi Pure Chemicals, Qiagen, Stratagene, Promega, Roche Diagnostics, Invitrogen, Genetics Institute, or Amersham Bioscience.

Examples of host cells that can be used include prokaryotic cells such as bacteria (e.g., *E. coli* or *Bacillus subtilis*), yeast (e.g., *Saccharomyces cerevisiae*), insect cells (e.g., Sf cells), and mammalian cells (e.g., COS, CHO, and BHK cells).

Vectors can comprise, in addition to DNA encoding each of the aforementioned polypeptides, regulatory elements such as promoter, enhancer, polyadenylation signal, ribosome-binding site, replication origin, terminator, and selection marker. Moreover, in order to facilitate the purification of a polypeptide, a peptidic label may be added to the C- or N-terminus of the polypeptide to form a fusion polypeptide. Examples of representative peptidic labels include, but are not limited to, (histidine)$_{6-10}$ repeat, FLAG, myc peptide, and GFP polypeptide. The recombinant DNA techniques are described in Sambrook, J. & Russel, D. (*supra*).

When the polypeptides are produced without the addition of a peptidic label, the polypeptides can be purified by, for example, ultrafiltration, salting-out, gel filtration, or ion-exchange chromatography. In addition thereto, affinity chromatography, HPLC, hydrophobic chromatography, isoelectric chromatography or the like may be carried out in combination. When the protein has a peptidic label, such as histidine repeat, FLAG, myc, or GFP, affinity chromatography suitable for each peptidic label can be carried out in accordance with conventional techniques. Construction of such an expression vector that facilitates isolation or purification is preferable. When the expression vector is constructed so as to express in the form of the fusion polypeptide of a polypeptide with a peptidic label, and such vector is used to prepare the polypeptide by genetic engineering techniques, isolation or purification of the polypetide is easy.

The mutants of the above polypeptides are a mutant comprising a deletion, substitution, addition, or insertion of one or more amino acids, preferably one or several amino acids, in each of the amino acid sequences as shown in SEQ ID NOS: 101 to 197 or partial sequences thereof; or alternatively a mutant having an identity of about 80% or higher, about 85% or higher, preferably about 90% or higher, more preferably about 95% or higher, about 97% or higher, about 98% or higher, or about 99% or higher with said amino acid sequence or a partial sequence thereof, as defined above. Examples of such mutants include naturally-occurring mutants, such as a homolog of a mammalian species other than human, a mutant thereof based on human polymorphism or splicing mutation, or the like.

A fragment of the polypeptide or mutant thereof consists of at least 5, at least 7, at least 10, at least 15, preferably at least 20, at least 25, more preferably at least 30, at least 40, or at least 50 continuous amino acid residues in the amino acid sequence of the polypeptide, and has a single epitope or a plurality of epitomes. Such a fragment can immunospecifically bind to the antibody or a fragment thereof. The polypeptide may be cleaved or fragmented by an enzyme that is present in the body, such as protease or peptidase, thereby being present as fragments.

The thus-obtained antibody that recognizes the polypeptide can specifically bind to the polypeptide via an antigen-binding site of the antibody. Specifically, a polypeptide having an amino acid sequence as shown in any of SEQ ID NOS: 101 to 197, a fragment thereof, a mutant thereof, or a fusion polypeptide can be used as an immunogen to produce immunoreactive antibodies.

More specifically, the polypeptide, a fragment thereof, a mutant thereof, or a fusion polypeptide comprises an antigenic determinant or epitope that elicits antibody formation, which antigen determinant or epitope may have a linear structure or a higher-order (or disconnected) structure. Such antigen determinant or epitope can be identified by any epitope analysis known in the art, such as phage display or reverse immunogenetics.

Antibodies of any aspect are elicited by the polypeptides. If all, part, or an epitope of the polypeptide is isolated, a polyclonal or monoclonal antibody can be prepared in accordance with conventional techniques. An example of the method for preparing an antibody is described in Kennet et al. (ed.), Monoclonal Antibodies, Hybridomas: A New Dimension in Biological Analyses, Plenum Press, New York, 1980.

A polyclonal antibody can be prepared by immunizing an animal such as a bird (e.g., a chicken) or a mammalian (e.g., a rabbit, goat, horse, sheep, or mouse) with thepolypeptide. The antibody of interest can be purified from the blood of an immunized animal via an appropriate combination of techniques such as ammonium sulfate fractionation, ion-exchange chromatography, and affinity chromatography.

A monoclonal antibody can be obtained by the technique comprising producing hybridoma cell lines that produce monoclonal antibodies specific for each relevant polypeptide in a mouse in accordance with conventional techniques. One method for producing such hybridoma cell lines comprises immunizing an animal with an enzyme polypeptide, removing spleen cells from the immunized animal, fusing the spleen cells with a myeloma cell line to produce hybridoma cells therefrom, and identifying a hybridoma cell line that produces a monoclonal antibody binding to the enzyme of interest. A monoclonal antibody can be recovered by a conventional technique.

The antibody is not particularly limited, provided that such antibody can bind specifically to the target polypeptide or a fragment thereof. The antibody usable is a monoclonal or polyclonal antibody, preferably monoclonal antibody. Examples of other antibodies include a recombinant antibody, a synthetic enzyme, a polyspecific antibody (e.g., a bispecific antibody), a single-stranded antibody, and an antibody fragment. Specific examples of such antibodies include Fab, $F(ab')_2$, scFv, Fv, and dsFv. The globulin type of the antibody of the present invention is not particularly limited, as long as the antibody has the aforementioned properties. It may be any of IgG, IgM, IgA, IgE, and IgD.

Preparation of monoclonal antibody

(1) Immunization and collection of antibody-producing cell

The immunogen which is a target polypeptide is administered to a mammalian animal such as rat, mouse (e.g., the inbred mouse strain Balb/c), or rabbit. The dose of the immunogen is appropriately determined depending on, for example, the type of an animal to be immunized or the route of administration, and it is about 50 to 200 μg per animal. Immunization is primarily performed by injecting an immunogen subcutaneously or intraperitoneally. The intervals of immunization are not particularly limited. After the primary immunization, boost immunization is carried out 2 to 10 times, preferably 3 or 4 times, at the intervals of several days to several weeks, and preferably at the intervals of 1 to 4 weeks. After the primary immunization, the antibody titer of the blood serum of the immunized animal is repeatedly measured by, for example, enzyme-linked immuno sorbent assay (ELISA). When the antibody titer reached a plateau, the immunogen is injected intravenously or intraperitoneally to complete the final immunization. The antibody-producing cells are recovered 2 to 5 days, preferably 3 days, after the final immunization. Examples of antibody-producing cells include spleen cells, lymph node cells, and peripheral blood cells, preferably spleen cells or regional lymph node cells.

(2) Cell fusion

Hybridoma cell lines that produce monoclonal antibodies specific for target polypeptides are prepared. Such hybridomas can be produced and identified via conventional techniques. The method for producing such hybridoma cell lines comprises immunizing an animal with a protein, removing spleen cells from the immunized animal, fusing the spleen cells with a myeloma cell line, producing hybridoma cells therefrom, and identifying a hybridoma cell line that produces a monoclonal antibody binding to the protein of interest. Myeloma cell lines to be fused with antibody-producing cells can be commercially available established cell lines of animals such as mice. Preferably, cell lines to be used have drug selectivity; namely, they cannot survive in the HAT selection medium (containing hypoxanthine, aminopterin, and thymidine) in an unfused state, while they can survive only in a state fused with antibody-producing cells. The established cells are preferably derived from an animal of the same species with the animal to be immunized. A specific example of the myeloma cell line is the strain P3X63-Ag.8 (ATCC TIB9), which is a BALB/c mouse-derived hypoxanthine guanine phosphoribosyl-transferase (HGPRT) deficient cell line.

Subsequently, the myeloma cell lines are fused with the antibody-producing cells. Cell fusion is carried out in a serum-free medium for animal cell culture, such as DMEM or RPMI-1640 medium, by mixing the antibody-producing cells with the myeloma cell lines at about 1:1 to 20:1 in the presence of a cell fusion accelerator. As the cell fusion accelerator, polyethylene glycol having an average molecular weight of 1,500 to 4,000 daltons can be used at a concentration of about 10 to 80%, for example. Optionally, an auxiliary agent, such as dimethyl sulfoxide, can be used in combination in order to enhance the fusion efficiency. Further, the antibody-producing cells can be fused with the myeloma cell lines by using a commercially available cell fusion apparatus utilizing electric stimulus (e.g., electroporation).

(3) Selection and cloning of hybridomas

The hybridomas of interest are selected from the fused cells. To this end, the cell suspension is adequately diluted in, for example, a fetal bovine serum-containing RPMI-1640 medium, the suspension is aliquoted into each well of a microtiter plate at about two million cells/well, a selection medium to each well, and culture is thereafter carried out while appropriately exchanging the selection medium with the same fresh medium. The culture temperature is 20°C to 40°C, preferably about 37°C. When the myeloma cell is an HGPRT-deficient strain or thymidine kinase-deficient strain, a hybridoma of a cell having an ability to produce an antibody and a myeloma cell line can selectively be cultured and grown in the selection medium containing hypoxanthine, aminopterin, and thymidine (i.e., the HAT medium). As a result, cells grown about 14 days after the initiation of culture in the selection medium can be obtained as the hybridoma.

Subsequently, whether or not the culture supernatant of the grown hybridoma contains the antibody of interest is screened for. Screening of hybridomas can be carried out in accordance with conventional techniques, without particular limitation. For example, the culture supernatant in the well containing the grown hybridomas is partially sampled and then subjected to enzyme immuno assay (EIA) or ELISA or radio immuno assay (RIA). The fused cells are cloned using the limiting dilution method or the like, and monoclonal antibody-producing cells, i.e. hybridomas, are established in the end. The hybridoma is stable during the culture in a basic medium, such as RPMI-1640 or DMEM, as described below, and the hybridoma can produce and secrete a monoclonal antibody that reacts specifically with a target polypeptide.

(4) Recovery of antibody

Monoclonal antibody can be recovered by conventional techniques. Specifically, a monoclonal antibody can be collected from the established hybridoma by the conventional cell culture technique, the ascites development, or the like. According to the cell culture technique, hybridoma is cultured in an animal cell culture medium, such as 10% fetal bovine serum-containing RPMI-1640 medium, MEM medium, or a serum-free medium, under common culture conditions (e.g., 37°C, 5% $CO_2$) for 2 to 10 days, and the antibody is obtained from the culture supernatant. In the case of the ascites development, about 10 millions of myeloma-derived hybridomas cells are administered intraperitoneally to an animal of the same species as the mammal from which the myeloma cell is derived, so as to allow the hybridoma cells to grow in a large quantity. After one to two weeks, the ascites or blood serum is collected from said animal.

When the purification of an antibody is required in the above-described method for collecting the antibody, the conventional techniques, such as salting out by ammonium sulfate, ion-exchange chromatography, affinity chromatography, and gel filtration chromatography, may be appropriately selected or combined to obtain the purified monoclonal antibody.

Preparation of polyclonal antibody

When polyclonal antibodies are prepared, an animal such as rabbit is immunized in the same manner as described above, the antibody titer is measured 6 to 60 days after the final immunization by enzyme immunoassay (EIA or ELISA) or radio immunoassay (RIA), and blood is taken on the day the maximal antibody titer is measured, in order to obtain antiserum. Thereafter, the reactivity of the polyclonal antibodies in the antiserum is assayed by ELISA or the like.

Chemically modified derivative

The antibody or a fragment thereof may be a chemically modified derivative. Examples include derivatives labeled with an enzyme, fluorophore, or radioisotope, and chemically modified derivatives, such as an acetylated, acylated, alkylated, phosphorylated, sulfated, or glycosylated derivatives.

Examples of the labels for use in enzyme immunoassay include enzymes such as peroxidase (POD), alkaline phosphatase, β-galactosidase, urease, catalase, glucose oxidase, lactate dehydrogenase, amylase, and biotin-avidin complexes. Examples of the labels for use in fluorescence immunoassay include fluorescent substances or fluorophores, such as fluorescein isothiocyanate, tetramethylrhodamine isothiocyanate, substituted rhodamine isothiocyanate, dichlorotriazine isothiocyanate, Alexa, and AlexaFluoro. Examples of the labels for use in radio immunoassay include radioactive isotopes, such as tritium, iodine ($^{131}I$, $^{125}I$, $^{123}I$, and $^{121}I$), phosphorus ($^{32}P$), sulfur ($^{35}S$), and metals (e.g., $^{68}Ga$, $^{67}Ga$, $^{68}Ge$, $^{54}Mn$, $^{99}Mo$, $^{99}Tc$, and $^{133}Xe$). Examples of the labels for use in luminescent immunoassay include luminescent molecules, luminescent substances, or bioluminescent substances, such as an NADH-, FMNH2-, luciferase system, luminol-hydrogen peroxide-POD system, acridinium ester system, or dioxetane compound system.

Alternatively, an avidin-biotin system or streptavidin-biotin system can also be used optionally. In such a case, biotin may be bound to the antibodies or fragments thereof, for example. A label can be bound to an antibody by conventional techniques, such as the glutaraldehyde method, the maleimide method, the pyridyl disulfide method, or the periodic acid method in the case of enzyme immunoassay. In radioimmunoassay, the binding of label and antibody can be carried out in accordance with the conventional techniques, such as the chloramine-T method and Bolton-Hunter method.

4. Kit for diagnosis of kidney cancer and/or prediction of the prognosis or metastasis of kidney cancer

4.1 Nucleic acid kit

The present invention also provides a kit for detecting, identifying, or predicting *in vitro* the presence, metastasis, or prognosis of kidney cancer, comprising one or more polynucleotides described in Section 2 above, mutants thereof, and/or fragments thereof.

The kit can comprise nucleic acid probes selected from group I as shown below. Such probes may be packaged in suitable containers, alone or in combination.

The kit can comprise at least one of a polynucleotide comprising the nucleotide sequence as shown in any of SEQ ID NOS: 1 to 10, 12 to 20, and 22 to 47, a polynucleotide comprising a complementary sequence thereof, a polynucleotide hybridizing under stringent conditions to said polynucleotide, or a fragment thereof.

The kit can further comprise at least one of a polynucleotide comprising the nucleotide sequence as shown in any of SEQ ID NOS: 11, 21, and 48 to 50, a polynucleotide comprising a complementary sequence thereof, a polynucleotide hybridizing under stringent conditions to said polynucleotide, or a fragment thereof.

A fragment of the polynucleotide, which can be contained in the kit is, for example, at least one DNA selected from the following groups (1) to (5):

(1) DNA comprising at least 15 continuous nucleotides in the nucleotide sequence as shown in any of SEQ ID NOS: 1 to 10, 11, 12 to 20, 21, 22 to 47, and 48 to 50 or a complementary sequence thereof;
(2) DNA comprising at least 60 continuous nucleotides in the nucleotide sequence as shown in any of SEQ ID NOS: 1 to 10, 11, 12 to 20, 21, 22 to 47, and 48 to 50 or a complementary sequence thereof;
(3) DNA comprising the nucleotide sequence as shown in any of SEQ ID NOS: 51 to 60, 61, 62 to 70, 71, 72 to 97, and 98 to 100 or a complementary sequence thereof and comprising at least 60 continuous nucleotides in the nucleotide sequence as shown in any of SEQ ID NOS: 1 to 10, 11, 12 to 20, 21, 22 to 47, and 48 to 50 or a complementary sequence thereof;
(4) DNA consisting of the nucleotide sequence as shown in any of SEQ ID NOS: 51 to 60, 61, 62 to 70, 71, 72 to 97, and 98 to 100; or
(5) DNA comprising a nucleotide sequence complementary to the nucleotide sequence as shown in any of SEQ ID NOS: 51 to 60, 61, 62 to 70, 71, 72 to 97, and 98 to 100.

According to a preferable embodiment, the polynucleotide is a polynucleotide consisting of a nucleotide sequence as shown in any of SEQ ID NOS: 1 to 10, 12 to 20, and 22 to 47, a polynucleotide consisting of a complementary sequence thereof, a polynucleotide hybridizing under stringent conditions to said polynucleotide, or a fragment comprising at least 15 continuous nucleotides thereof.

According to another preferable embodiment, the kit can further comprise, in addition to the above polynucleotide, a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof.

According to a preferable embodiment, the fragment can be a polynucleotide comprising at least 15, preferably at least 60 continuous nucleotides.

According to another preferable embodiment, the fragment is a polynucleotide comprising a nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50, respectively, or a polynucleotide comprising a nucleotide sequence complementary thereto.

According to another preferable embodiment, the fragment is a polynucleotide comprising a nucleotide sequence as shown in any of SEQ ID NOS: 51 to 60, 61, 62 to 70, 71, 72 to 97, and 98 to 100.

According to another preferable embodiment, the fragment is a polynucleotide comprising a nucleotide sequence complementary to a nucleotide sequence as shown in any of SEQ ID NOS: 51 to 60, 61, 62 to 70, 71, 72 to 97, and 98 to 100.

According to another preferable embodiment, the fragment is a polynucleotide consisting of a nucleotide sequence as shown in any of SEQ ID NOS: 51 to 60, 61, 62 to 70, 71, 72 to 97, and 98 to 100.

Specific examples of the aforementioned combinations include: a polynucleotide comprising the nucleotide sequence as shown in SEQ ID NO: 1 or 2 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 3 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 4 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 5 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 6 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 7 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 8 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 9 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 10 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 11 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 12 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 13 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 14 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 15 or a complementary sequence thereof and/or a fragment thereof; a polynucleotide comprising a nucleotide sequence as shown in any of SEQ ID NOS: 1 to 16 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 17 or a complementary sequence thereof, and/or a fragment thereof; a polynucleotide comprising a nucleotide sequence as shown in any of SEQ ID NOS: 1 to 18 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 19 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 20 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 21 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 22 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 23 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 24 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 25 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 26 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 27 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 28 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 29 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 30 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 31 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 32 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 33 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 34 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 35 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 36 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 37 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 38 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 39 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 40 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 41 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 42 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 43 or a complementary sequence thereof, a polynucleotide hybridizing under stringent conditions to such polynucleotide, and/or a fragment thereof; a polynucleotide comprising a nucleotide sequence as shown in any of SEQ ID Nosy 1 to 44 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 45 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 46 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 47 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 48 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 49 or a complementary sequence thereof, a polynucleotide hybridizing under stringent conditions to such polynucleotide, and/or a fragment thereof; and a polynucleotide comprising a nucleotide sequence as shown in any of SEQ ID NOS: 1 to 50 or a complementary sequence thereof, a polynucleotide hybridizing under stringent conditions to such polynucleotide, and/or a fragment thereof.

According to a more preferable embodiment, the kit can comprise at least two to all polynucleotides comprising the nucleotide sequence as shown in any of SEQ ID NOS: 51 to 97 and 98 to 100 or a complementary sequence thereof.

A specific example of another combination is a polynucleotide comprising (or consisting of) the nucleotide sequence as shown in any of SEQ ID NOS: 1 to 19 and 48 or a complementary sequence thereof, and/or a fragment thereof.

A further specific example of another combination is a polynucleotide comprising (or consisting of) the nucleotide sequence as shown in any of SEQ ID NOS: 20 to 47, 49, and 50 or a complementary sequence thereof, and/or a fragment thereof.

In the present invention, the size of fragments of the polynucleotides is, for example, continuous 15 to all nucleotides, 15 to 5000 nucleotides, 15 to 4500 nucleotides, 15 to 4000 nucleotides, 15 to 3500 nucleotides, 15 to 3000 nucleotides, 15 to 2500 nucleotides, 15 to 2000 nucleotides, 15 to 1500 nucleotides, 15 to 1000 nucleotides, 15 to 900 nucleotides, 15 to 800 nucleotides, 15 to 700 nucleotides, 15 to 600 nucleotides, 15 to 500 nucleotides, 15 to 400 nucleotides, 15 to 300 nucleotides, 15 to 250 nucleotides, 15 to 200 nucleotides, 15 to 150 nucleotides, 15 to 140 nucleotides, 15 to 130 nucleotides, 15 to 120 nucleotides, 15 to 110 nucleotides, 15 to 100 nucleotides, 15 to 90 nucleotides, 15 to 80 nucleotides, 15 to 70 nucleotides, 15 to 60 nucleotides, 15 to 50 nucleotides, 15 to 40 nucleotides, 15 to 30 nucleotides or 15 to 25 nucleotides; 25 to all nucleotides, 25 to 1000 nucleotides, 25 to 900 nucleotides, 25 to 800 nucleotides, 25 to 700 nucleotides, 25 to 600 nucleotides, 25 to 500 nucleotides, 25 to 400 nucleotides, 25 to 300 nucleotides, 25 to 250 nucleotides, 25 to 200 nucleotides, 25 to 150 nucleotides, 25 to 140 nucleotides, 25 to 130 nucleotides, 25 to 120 nucleotides, 25 to 110 nucleotides, 25 to 100 nucleotides, 25 to 90 nucleotides, 25 to 80 nucleotides, 25 to 70 nucleotides, 25 to 60 nucleotides, 25 to 50 nucleotides or 25 to 40 nucleotides; 50 to all nucleotides, 50 to 1000 nucleotides, 50 to 900 nucleotides, 50 to 800 nucleotides, 50 to 700 nucleotides, 50 to 600 nucleotides, 50 to 500 nucleotides, 50 to 400 nucleotides, 50 to 300 nucleotides, 50 to 250 nucleotides, 50 to 200 nucleotides, 50 to 150 nucleotides, 50 to 140 nucleotides, 50 to 130 nucleotides, 50 to 120 nucleotides, 50 to 110 nucleotides, 50 to 100 nucleotides, 50 to 90 nucleotides, 50 to 80 nucleotides, 50 to 70 nucleotides or 50 to 60 nucleotides; 60 to all nucleotides, 60 to 1000 nucleotides, 60 to 900 nucleotides, 60 to 800 nucleotides, 60 to 700 nucleotides, 60 to 600 nucleotides, 60 to 500 nucleotides, 60 to 400 nucleotides, 60 to 300 nucleotides, 60 to 250 nucleotides, 60 to 200 nucleotides, 60 to 150 nucleotides, 60 to 140 nucleotides, 60 to 130 nucleotides, 60 to 120 nucleotides, 60 to 110 nucleotides, 60 to 100 nucleotides, 60 to 90 nucleotides, or 60 to 80 nucleotides or 60 to 70, in the nucleotide sequence of each polynucleotide.

The kit can comprise, in addition to the polynucleotides of the present invention, mutants thereof, or fragments thereof as described above, known or novel polynucleotides that enable detection of kidney cancer or prediction of metastasis or prognosis of kidney cancer. Polynucleotides, mutants thereof, or fragments thereof which are according to the present invention are selected from the group of polynucleotides as defined in any of claims 1 to 11. Further, the kit which is in accordance with the present invention is a kit as defined in any of claims 12 to 19.

4.2 Antibody kit

Also provided is a kit for detecting, identifying, or predicting *in vitro* the presence, metastasis, or prognosis of kidney cancer, comprising one or more of the antibodies of group II described in Section 2 above, fragments thereof, chemically modified derivatives thereof, the antibodies described in Section 3.2 above, fragments thereof, or chemically modified derivatives thereof.

The kit can comprise, as probes, antibodies from groups II (f), (g), and (h), fragments thereof, or chemically modified derivatives thereof. These probes can be packaged in suitable containers, alone or in combination.

Examples of probe combinations are as follows.

In order to detect polypeptides encoded by the PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6V0A4, PRODH2, EPHB2, LYAR, COX6B, PRH1, LAPTM5, RPS6KA4, GCC2, FGF2, MMP14, and ERBB2 genes as markers for predicting the prognosis of kidney cancer or as markers for metastasis of kidney cancer, homologs thereof, mutants thereof, or derivatives thereof, the first example comprises one or more, and preferably two or more antibodies against such polypeptides, mutants thereof, or fragments thereof.

Specifically, the probes comprised in the kit are: one or more antibodies that bind specifically to at least one of polypeptides each comprising the amino acid sequence as shown in any of SEQ ID NOS: 101 to 110, 112 to 120, and 122 to 147, mutants thereof, or fragments thereof; fragments thereof; or chemically modified derivatives.

The kit can further comprise an antibody against a polypeptide having the amino acid sequence as shown in any of SEQ ID NOS: 111, 121, and 148 to 150, a fragment thereof, or a chemically modified derivative thereof. Use of such antibodies in combination can improve the accuracy for predicting metastasis or prognosis after surgery.

The second example includes one or more antibodies against the polypeptides encoded comprising the amino acid sequences as shown in SEQ ID NOS: 151, 153, and 155 to 160 and in SEQ ID NOS: 161 to 190 of group II (g) and (h) as kidney cancer markers, fragments thereof, or chemically modified derivatives thereof.

The kit can further comprise an antibody against a polypeptide having the amino acid sequence as shown in any of SEQ ID NOS: 152, 154, and 191, and 192 to 197, a fragment thereof, or a chemically modified derivative thereof. Use of such antibodies in combination can improve the accuracy for detecting kidney cancer.

The antibodies comprised in the kit of the present invention can be present singly or in the form of a mixture. Alternatively, the antibodies may be bound onto a solid-phase carrier or may be in the free form. Further, the kit of the present invention can comprise a labeled secondary antibody, a carrier, a washing buffer, a sample diluent, a substrate for enzyme, a reaction terminator, a marker (target) polypeptide(s) as purified standard(s), instructions, and so on.

5. <u>DNA chip</u>

The present invention further provides a DNA chip for detecting kidney cancer or predicting the prognosis or metastasis of kidney cancer using the same polynucleotide(s) as the polynucleotide(s) comprised in the composition and/or the kit of the present invention as described in Sections 3 and 4 above, a mutant(s) thereof, or a fragment(s) thereof, alone or in combination, preferably in combination.

A substrate of the DNA chip is not particularly limited, provided that the substrate can comprise DNAs immobilized thereon. Examples of the substrate include a glass slide, a silicon chip, a polymer chip, and a nylon membrane. Such substrates may be subjected to surface treatment, for example, poly-L-lysine coating or introduction of a functional group such as an amino or carboxyl group.

DNA can be immobilized on a substrate by any common techniques without particular limitation. Examples of such techniques include a method wherein DNA is spotted using a high-density dispenser, called spotter or arrayer, a method of spraying DNA on a substrate using an apparatus (i.e., inkjet), which jets fine droplets from a nozzle by a piezoelectric element, and a method of synthesizing nucleotides successively on a substrate. When the high-density dispenser is used, for example, different gene solutions are first placed into each well of a multi-well plate, and the solutions are taken out of the plate using a pin (i.e., needle) and are successively spotted on the substrate. According to the inkjet technique, genes are jetted through a nozzle, and the genes are arrayed on the substrate at a high speed. In the DNA synthesis on the substrate, a nucleotide on the substrate is protected with a functional group, which is capable of leaving from the substrate by light, and light is selectively applied only to a nucleotide at a specific position by using a mask, thereby deprotecting the functional group. Thereafter, nucleotides are added to the reaction mixture, which nucleotides are coupled to the nucleotides on the substrate, and this step is repeated.

Polynucleotides to be immobilized are the polynucleotides of the present invention as described above.

Examples of polynucleotides can comprise one or more of the following polynucleotides or fragments thereof:

(1) polynucleotides each consisting of a nucleotide sequence as shown in any of SEQ ID NOS: 1 to 50, mutants thereof, or fragments comprising at least 15 continuous nucleotides thereof;
(2) polynucleotides each comprising a nucleotide sequence as shown in any of SEQ ID NOS: 1 to 50;
(3) polynucleotides each consisting of a nucleotide sequence as shown in any of SEQ ID NOS: 1 to 10, 12 to 20, and 22 to 47, mutants thereof, or fragments comprising at least 15 continuous nucleotides thereof;
(4) polynucleotides each comprising a nucleotide sequence as shown in any of SEQ ID NOS: 1 to 10, 12 to 20, and 22 to 47, mutants thereof, or fragments comprising at least 15 continuous nucleotides thereof;
(5) polynucleotides each consisting of a nucleotide sequence complementary to a nucleotide sequence as shown in any of SEQ ID NOS: 1 to 10, 12 to 20, and 22 to 47, mutants thereof, or fragments comprising at least 15 continuous nucleotides thereof;

(6) polynucleotides each comprising a nucleotide sequence complementary to a nucleotide sequence as shown in any of SEQ ID NOS: 1 to 10, 12 to 20, and 22 to 47;

(7) polynucleotide each hybridizing under stringent conditions to DNA consisting of a nucleotide sequence complementary to a nucleotide sequence as shown in any of SEQ ID NOS: 1 to 10, 12 to 20, and 22 to 47 or fragments comprising at least 15 continuous nucleotides thereof;

(8) polynucleotides each hybridizing under stringent conditions to DNA consisting of a nucleotide sequence as shown in any of SEQ ID NOS: 1 to 10, 12 to 20, and 22 to 47 or fragments comprising at least 15 continuous nucleotides thereof;

(9) a polynucleotide consisting of the nucleotide sequence as shown in any of SEQ ID NOS: 11, 21, and 48 to 50, mutants thereof, or fragments comprising at least 15 continuous nucleotides thereof;

(10) polynucleotides comprising a nucleotide sequence as shown in any of SEQ ID NOS: 11, 21, and 48 to 50, mutants thereof, or fragments comprising at least 15 continuous nucleotides thereof;

(11) polynucleotides consisting of a nucleotide sequence complementary to a nucleotide sequence as shown in SEQ ID NOS: 11, 21, and 48 to 50, mutants thereof, or fragments comprising at least 15 continuous nucleotides thereof;

(12) polynucleotides comprising a nucleotide sequence complementary to the nucleotide sequence as shown in any of SEQ ID NOS: 11, 21, and 48 to 50;

(13) polynucleotides each hybridizing under stringent conditions to DNA consisting of a nucleotide sequence complementary to a nucleotide sequence as shown in any of SEQ ID NOS: 11, 21, and 48 to 50 or fragments comprising at least 15 continuous nucleotides thereof;

(14) polynucleotides each hybridizing under stringent conditions to DNA consisting of a nucleotide sequence as shown in any of SEQ ID NOS: 11, 21, and 48 to 50 or fragments comprising at least 15 continuous nucleotides thereof;

(15) a polynucleotide comprising at least 15 continuous nucleotides in a nucleotide sequence as shown in any of SEQ ID NOS: 1 to 10, 12 to 20, and 22 to 47 or a complementary sequence thereof;

(16) a polynucleotide comprising at least 60 continuous nucleotides in a nucleotide sequence as shown in any of SEQ ID NOS: 1 to 10, 12 to 20, and 22 to 47 or a complementary sequence thereof;

(17) a polynucleotide comprising at least 15 continuous nucleotides in a nucleotide sequence as shown in any of SEQ ID NOS: 11, 21, and 48 to 50 or a complementary sequence thereof;

(18) a polynucleotide comprising at least 60 continuous nucleotides in a nucleotide sequence as shown in any of SEQ ID NOS: 11, 21, and 48 to 50 or a complementary sequence thereof;

(19) a polynucleotide comprising a nucleotide sequence as shown in any of SEQ ID NOS: 51 to 60, 62 to 70, and 72 to 97 and at least 60 continuous nucleotides in a nucleotide sequence as shown in any of SEQ ID NOS: 1 to 10, 12 to 20, and 22 to 47;

(20) a polynucleotide comprising a nucleotide sequence comprising a sequence complementary to a nucleotide sequence as shown in any of SEQ ID NOS: 51 to 60, 62 to 70, and 72 to 97 and at least 60 continuous nucleotides in a nucleotide sequence complementary to a nucleotide sequence as shown in any of SEQ ID NOS: 1 to 10, 12 to 20, and 22 to 47;

(21) a polynucleotide comprising a nucleotide sequence as shown in any of SEQ ID NOS: 61, 71, and 98 to 100 and at least 60 continuous nucleotides in a nucleotide sequence as shown in any of SEQ ID NOS: 11, 21, and 48 to 50; and

(22) a polynucleotide comprising a sequence complementary to a nucleotide sequence as shown in any of SEQ ID NOS: 61, 71, and 98 to 100 and at least 60 continuous nucleotides in a nucleotide sequence as shown in any of SEQ ID NOS: 11, 21, and 48 to 50.

According to a preferable embodiment, the DNA chip can include at least 2 or all polynucleotides each comprising a nucleotide sequence as shown in any of SEQ ID NOS: 51 to 60, 61, 62 to 70, 71, 72 to 97, and 98 to 100 or a complementary sequence thereof. Polynucleotides which are according to the present invention are selected from the group of polynucleotides as defined in any of claims 1 to 11. Further, the DNA chip which is according to the present invention is as defined in any of claims 20 to 23.

According to the present invention, the polynucleotides to be immobilized may be any of genomic DNA, cDNA, RNA, synthetic DNA, and synthetic RNA, or alternatively they may be single-stranded or double-stranded.

Examples of DNA chips that can detect and determine the expression levels of the target gene, RNA, or cDNA include the Gene Chip Human Genome U133 Plus 2.0 Array (Affymetrix), the Whole human genome oligo microarray (Agilent), the IntelliGene® HS Human Expression CHIP (Takara Bio), and a polymethylmethacrylate DNA chip substrate having a concave-convex structure (JP Patent Publication (kokai) No. 2004-264289 A).

DNA microarrays can be prepared by, for example, a method wherein probes that have been prepared in advance are immobilized on a solid-phase surface. In this method, polynucleotides into which functional groups have been introduced are synthesized, and oligonucleotides or polynucleotides are spot-deposited on the surface of a surface-treated solid-phase support, followed by covalent binding to the surface (e.g., J. B. Lamture et al., Nucleic. Acids. Research, 1994, vol. 22, pp. 2121-2125; Z. Guo et al., Nucleic. Acids. Research, 1994, vol. 22, pp. 5456-5465). In general, the polynucleotides are covalently bound to the surface-treated solid-phase support via a spacer or crosslinker. The method wherein fine pieces of polyacrylamide gel are aligned on the glass surface and synthetic polynucleotides are covalently bound thereto is also known (G. Yershov et al., Proceedings of the National Academic Sciences, U.S.A., 1996, vol. 94, p. 4913). As a further method, a microelectrode array is prepared on silica microarray, a reaction site is formed on the electrode by providing a permeable layer of streptavidin-containing agarose, this site is positively charged to immobilize the biotinylated polynucleotides thereon, and the charge at the site is regulated. This enables performance of hybridization at a high speed under stringent condition (R. G. Sosnowski et al., Proceedings of the National Academic Sciences, U.S.A., 1997, vol. 94, pp. 1119-1123).

6. <u>Method for detecting and identifying the presence of kidney cancer and/or for predicting the prognosis or metastasis of kidney cancer</u>

The present invention provides a method for predicting *in vitro* the subject's prognosis and/or the presence or absence of metastasis of kidney cancer comprising comparing the expression level of the target nucleic acids in a biological sample of kidney cancer cells from a subject with the expression level of the target nucleic acids in the kidney cancer cells from patients with poor prognoses and/or the expression level of the target nucleic acids in the kidney cancer cells from patients with good prognoses, by using the composition, kit, or DNA chip of the present invention, or combinations thereof, wherein the target nucleic acids can be detected by the polynucleotides in the composition, kit, or DNA chip, mutants thereof, or fragments thereof, and, when the expression level of the target nucleic acids in the subject is changed compared with that in the patients with good prognoses or poor prognoses and/or that in the patients with poor prognoses, the subject is determined to have poor or good prognosis, and/or the metastasis of kidney cancer is determined to have or have not occurred.

The present invention also provides use of the composition, kit, or DNA chip of the present invention for detecting *in vitro* the kidney cancer cells suspected of being at risk for metastasis in an analyte sample from a subject.

The above-described method of the present invention involves the use of the composition, kit, or DNA chip of the present invention comprising the polynucleotides of the present invention, mutants thereof, or fragments thereof alone or in any possible combination.

In the method for predicting, detecting, identifying, or (genetically) diagnosing the metastasis of kidney cancer and/or for predicting the prognosis for kidney cancer patients of the present invention, the polynucleotides comprised in the composition, kit, or DNA chip of the present invention, mutants thereof, or fragments thereof can be used as primers or detection probes (searchers). When used as primers, for example, primers comprising generally 15 to 50 nucleotides, preferably 15 to 30 nucleotides, and more preferably 18 to 25 nucleotides can be used. When used as detection probes, for example, polynucleotides comprising 15 to all nucleotides, preferably 25 to 1000 nucleotides, more preferably 25 to 100 nucleotides can be used. It should be understoodthat the number of nucleotides should not be limited to the specific ranges.

The polynucleotides, mutants thereof, or fragments thereof that are comprised in the composition or kit of the present invention can be used as primers or probes in accordance with the conventional techniques in known methods for specifically detecting a given gene, such as Northern blotting, RT-PCR, *in situ* hybridization, or Southern hybridization. As to samples to be tested (or analytes), the whole or part of the kidney tissue or the body tissue suspected of being at risk for having kidney cancer cells of a subject may be removed by biopsy or another means, or the samples may be removed from the body tissue excised by surgery, depending on types of detection methods. Further, total RNA prepared therefrom in accordance with the conventional techniques may be used, or various polynucleotides including cDNA or poly A(+) RNA prepared from the RNA may be used.

Alternatively, the expression levels of nucleic acids such as the gene, RNA, or cDNA of the present invention in the body tissue can be detected or quantified using a DNA chip (including a DNA microarray). In this case, the composition or kit of the present invention can be used as a DNA array probe (e.g., the Human Genome U133 Plus 2.0 Array (Affymetrix) uses a polynucleotide probe having 25 nucleotides). Such a DNA array may be hybridized to the labeled DNA or RNA, which is prepared from RNA removed from the body tissue, and a complex of the probe with the labeled DNA or RNA resulting from such hybridization may be detected using the labeled DNA or RNA as an indication to evaluate the presence or absence of the expression of the genes associated with kidney cancer metastasis or genes associated with prognosis for the cancer patient or the expression levels thereof in the body tissue. In the method of the present invention, a DNA chip is preferably used. This enables the simultaneous evaluation of the presence or absence of the expression of a plurality of genes, or the simultaneous evaluation of the expression levels of the genes, in a single biological sample.

The composition, kit, or DNA chip of the present invention is useful for predicting the prognosis for a kidney cancer patient and/or predicting, identifying, or detecting metastasis of kidney cancer (e.g., diagnosis of affection or degree of affection). Specifically, prognosis for a kidney cancer patient and/or metastasis of kidney cancer can be predicted using the composition, kit, or DNA chip in the following manner. That is, the body tissue of the subject having kidney cancer cells can be subjected to the assay of the expression levels of the genes that are detected with the use of such diagnostic composition. In this case, the term "differences in gene expression levels" refers to not only the presence or absence of the expression but also the case where differences in gene expression levels between the body tissue comprising kidney cancer cells from a patient with a good prognosis and the body tissue comprising kidney cancer cells from a patient with a poor prognosis are statistically significant (p value of < 0.05). For example, the expression of the PABPN1 gene is induced/decreased in kidney cancer cells from a patient with poor prognosis. Thus, its expression is increased/decreased in kidney cancer tissue from a subject with poor prognosis. If the differences between such expression level and the expression level in the normal tissue are significant, the subject is suspected of being at risk for kidney cancer metastasis and is also predicted to have poor prognosis.

A method for detecting kidney cancer (cells) using the composition, kit, or DNA chip of the present invention comprises: removing the whole or part of the body tissue from a subject via biopsy or recovering it from the body tissue excised by surgery; detecting the genes contained therein using a polynucleotide or polynucleotides selected from the polynucleotides of the present invention, mutants thereof, or fragments thereof; measuring the expression levels of said genes; and predicting metastasis of kidney cancer, diagnosing the presence or absence of metastasis of kidney cancer or a degree thereof, and/or predicting the prognosis for a kidney cancer patient. Also, the method for predicting metastasis of kidney cancer according to the present invention can detect, identify, or predict amelioration or the degree of amelioration of the disease when a therapeutic agent is administered to an kidney cancer bearing patient, for example.

The method of the present invention can comprise, for example, the following steps (a), (b), and (c) of:

(a) bringing a biological sample of a subject into contact with a polynucleotide or polynucleotides comprised in the composition, kit, or DNA chip of the present invention;
(b) measuring the expression level of the target nucleic acid(s) in the biological sample using the polynucleotide or polynucleotides as the probe; and
(c) predicting the prognosis for kidney cancer patients and/or identifying the presence or absence of kidney cancer (cells) suspected of being at risk for metastasis in the biological sample on the basis of the results obtained in step (b).

Examples of biological samples used in the method of the present invention include the body tissues of a subject, for example, samples prepared from kidney tissue and peripheral tissue thereof, tissue suspected of being at risk for having the metastasis of kidney cancer, and the like. Specifically, an RNA containing sample prepared from such tissue or a sample containing a polynucleotide prepared therefrom may be prepared by removing the whole or part of the body tissue from the subject via biopsy, or recovering the sample from the body tissue excised by surgery to prepare the sample therefrom in accordance with conventional techniques.

The term "subject" as used herein refers to a mammalian animal. Examples thereof include, but are not limited to, human, monkey, mouse, and rat, preferably human.

In the method of the present invention, the above-mentioned steps may be varied depending on types of biological samples used as analytes.

When RNA is used as the analyte, for example, detection of kidney cancer (cells) can comprise the following steps (a), (b), and (c) of:

(a) allowing RNA prepared from a biological sample of a subject or a complementary polynucleotide (cDNA) transcribed therefrom to bind to a polynucleotide comprised in the composition, kit, or DNA chip of the present invention;
(b) measuring the RNA prepared from the biological sample bound to the polynucleotide or a complementary polynucleotide transcribed from the RNA using the above polynucleotide as a probe; and
(c) identifying the presence or absence of kidney cancer (cells) from a patient with a poor prognosis based on the results obtained in step (b).

In order to detect, identify, or diagnose the metastatic kidney cancer (cells) by the method of the present invention, for example, various hybridization techniques can be employed. Examples of the hybridization techniques that can be employed include Northern blotting, quantitative Southern blotting, RT-PCR, DNA chip analysis, *in situ* hybridization, and Southern hybridization.

When Northern blotting is employed, the diagnostic composition of the present invention can be used as a probe to detect and assay the presence or absence of gene expression in RNA or the expression level thereof. Specifically, the diagnostic composition (specifically a complementary strand) of the present invention is labeled with a radioisotope (e.g., $^{32}P$, $^{33}P$, or $^{35}S$) or a fluorophore, the resultant is hybridized to the RNA obtained from a body tissue of a subject that has been transferred onto a nylon membrane or the like in accordance with any of the conventional techniques, the resulting double-strand of the diagnostic composition (i.e., DNA) and RNA can be measured by detecting a signal derived from a label (a radioisotope or fluorophore) of the diagnostic composition using a radio detector (e.g., BAS-1800 II, Fuji Photo Film, Japan) or a fluorescent detector (STORM 860, Amersham Bioscience).

When the quantitative RT-PCR is employed, the diagnostic composition of the present invention can be used as a primer to detect and assay the presence or absence of the gene expression in RNA or the expression level thereof. Specifically, cDNA is prepared from RNA of a body tissue of a subject in accordance with a conventional technique, a pair of primers prepared from the diagnostic composition of the present invention (i.e., a forward strand and a reverse strand, both bound to the cDNA) is hybridized to cDNA to perform PCR with the use of cDNA as a template in accordance with the conventional technique, thereby amplifying the target gene regions, and the resulting double-stranded DNA is detected. Double-stranded DNA can be detected by a method wherein PCR is carried out using a primer that has been labeled with a radioisotope or fluorophore in advance, a method wherein the PCR product is electrophoresed on agarose gel, and double-stranded DNA is detected by staining the same with ethidium bromide or the like, or a method wherein the resulting double-stranded DNA is transferred to a nylon membrane or the like in accordance with a conventional technique, and the resultant is subjected to hybridization to the labeled diagnostic composition as a probe to detect the substance of interest.

When the DNA array analysis is employed, a DNA chip comprising the diagnostic composition of the present invention as a DNA probe (single-stranded or double-stranded) bound to a substrate is used. A substrate comprising genes immobilized thereon is generally referred to as DNA chip or DNA array. Examples of the DNA array include a DNA macroarray and a DNA microarray. As used herein, the term "DNA chip" refers to such DNA arrays.

Hybridization conditions are not particularly limited. For example, hybridization is carried out in 3 to 4×SSC and 0.1% to 0.5% SDS at 30°C to 50°C for 1 to 24 hours, more preferably in 3.4×SSC and 0.3% SDS at 40°C to 45°C for 1 to 24 hours, followed by washing. Washing is continuously carried out, for example, with a solution containing 2×SSC and 0.1% SDS, with a solution of 1×SSC, and with a solution of 0.2×SSC at room temperature. The term "1×SSC" refers to an aqueous solution containing 150 mM sodium chloride and 15 mM sodium citrate (pH 7.2). Preferably, a complementary strand remains hybridized to the target (+) strand even if it is washed under such conditions. Specific examples of such complementary strand include a strand consisting of the nucleotide sequence completely complementary to the nucleotide sequence of the target (+) strand, and a strand consisting of a nucleotide sequence having at least 80% identity with said strand.

When PCR is carried out under stringent hybridization conditions using polynucleotide fragments obtained from the composition or kit of the present invention as primers, for example, a PCR buffer comprising 10 mM Tris-HCl (pH 8.3), 50 mM KCl, and 1 to 2 mM $MgCl_2$ is used, and the treatment is carried out at a temperature, Tm-(5 to 10°C) which is calculated from the primer sequence, for about 15 seconds to 1 minute. The Tm value can be calculated, for example, by the equation Tm = 2 × (the number of adenine residues + the number of thymine residues) + 4 × (the number of guanine residues + the number of cytosine residues).

Another example of the "stringent conditions" for hybridization is described in, for example, Sambrook, J. & Russel, D., Molecular Cloning, A LABORATORY MANUAL, Cold Spring Harbor Laboratory Press, January 15, 2001, vol. 1: 7.42 to 7.45, vol. 2: 8.9 to 8.17, and such conditions can be employed in the present invention.

The present invention also provides a method for predicting the prognosis for a kidney cancer patient by assaying the expression levels of target nucleic acids or genes in the biological sample from a subject using one or more probes of group I and/or group II, the composition, kit, or DNA chip of the present invention, or combinations thereof and using a discriminant, i.e., the support vector machine (SVM), using the gene expression levels in the kidney cancer tissue from a patient with good prognosis and the kidney cancer tissue from a patient with poor prognosis as the training samples, and/or a method for determining whether or not the biological sample contains metastatic kidney cancer cells.

The present invention further provides a method for detecting, identifying, or predicting the metastasis of kidney cancer, the method comprising the steps of:

(1) measuring *in vitro* expression levels of target nucleic acids in a plurality of biological samples that are known to be the tissue which contains kidney cancer cells obtained from a patient with a poor prognosis and/or the tissue which contains kidney cancer cells obtained from a patient with a good prognosis using a probe or probes as defined in Section 2 above, the composition, the kit, or the DNA chip comprising the probe or probes;
(2) preparing a discriminant, a support vector machine, using, as training samples, the expression levels of the target nucleic acids determined in step (1);
(3) measuring *in vitro* expression levels of the target nucleic acids in a biological sample obtained from the kidney cancer cells of the subject in the same manner as in step (1); and
(4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof, or fragments thereof contained in the composition, kit, or DNA chip.

Alternatively, the method of the present invention can comprise the following steps (a), (b), and (c) of:

(a) measuring the expression levels of the target genes in the biological samples that are known to be the tissue containing the kidney cancer cells from a patient with good prognosis or the tissue containing the kidney cancer cells from a patient with poor prognosis using the composition for diagnosis (detection), kit, or DNA chip of the present invention;
(b) preparing a discriminant (i.e., support vector machine) by assigning the expression levels determined in step (a) into the equations 1 to 5 below; and
(c) assaying the expression levels of the target genes in the biological sample from a subject using the composition for diagnosis (detection), kit, or DNA chip of the present invention, and assigning the determined values into the discriminant prepared in step (b), in order to predict the prognosis for a kidney cancer patient and/or determine whether or not the biological sample contains metastatic kidney cancer cells based on the obtained results.

SVM is a learning machine that was proposed in the framework of a statistical learning theory made to solve a two-class classification problem, by V. Vapnik of AT&T in 1995 (The Nature of Statistical Leaning Theory, Springer, 1995). SVM is a linear classifier but it can solve nonlinear problems in combination with the Kernel method as described below. Among many hyperplanes that classify training samples of different classes, the hyperplane that maximizes the minimum distance from the hyperplane to the training sample may be defined as the classification plane to classify a new test sample in the most accurate manner.

SVM can only solve linear problems. As a method for solving substantially nonlinear problems, a method wherein a feature vector is nonlinearly transformed into a higher-dimensional feature, and linear classification is then performed, is known. This becomes equivalent to the use of a nonlinear model in an original space. High-dimensional mapping, however, requires an enormous computational effort and reduces a generalization capability. According to SVM, the classification function depends exclusively on the inner product of the inputted pattern. Accordingly, if the inner product could be calculated, the optimal classification function could be constructed. The formula that represents the inner product of two elements in a nonlinearly mapped space only by the input in original spaces is referred to as the Kernel formula. An optimal classification function, i.e. a discriminant, can be formed only by the Kernel formula without computation of features in the actually mapped space while performing high-dimensional mapping (e.g., Hideki Asou et al., Toukei kagaku no furontia 6 (Frontier of statistical science 6), "Pataan ninshiki to gakushu no toukeigaku (Statistics of pattern recognition and learning)," pp. 107-138, Iwanami Shoten Publishers, Tokyo, Japan, date of publication, April 11, 2003).

Examples of the computation of a discriminant that can be used in the method of the present invention are shown below.

In order to identify SVM, the expression levels of the target gene in biological samples that are known to be a kidney cancer cell-containing tissue from a patient with good prognosis or kidney cancer tissue from a patient with poor prognosis is provided as training samples, and a constant of the classification function can be identified in the following manner.

The training sample $x_i$ is assumed to belong to either a group of kidney cancer cell-containing tissue from a patient with a good prognosis or kidney cancer tissue from a patient with a poor prognosis, which groups are classified into (+) or (-). When training samples can be linearly separated by the hyperplane, the classification function is, for example, as follows:

[equation 1]

$$f(x) = \sum_{i=1}^{n} w_i \cdot x_i + b$$

where w represents a weighting factor, b represents a bias constant, and x represents a sample variable.

This function, however, has a restriction:

[equation 2]

$$y_i\left(w^T x_i + b\right) \geq 1 - \xi_i$$

$$\xi_i \geq 0, i = 1, \cdots, n$$

where T represents an inner product, y represents a sample class, and ζ represents a slack variable. Thus, the Lagrange's method of unidentified multipliers may be used to regress to the following optimization problem using the Lagurange multiplier α.

[equation 3]

$$\max_{\alpha} \sum\nolimits_{i=1}^{n} \alpha_i - \frac{1}{2} \sum\nolimits_{i,j=1}^{n} \alpha_i \alpha_j y_i y_j x_i^T x_j$$

[equation 4]

$$0 \leq \alpha_i \leq C, \sum_{i=1}^{n} \alpha_i y_i = 0$$

where C represents a restriction parameter identified by an experiment.

If the above problem is dissolved, the following formula is consequently obtained.

[equation 5]

$$w = \sum_{i=1}^{n} \alpha_i y_i x_i$$

$$b = -\frac{1}{2}\left(w^T x_A + w^T x_B\right)$$

Thus, the nonambiguous classification function can be identified. By assigning x concerning a new biological sample (i.e., the gene expression level in a tissue, whether or not the tissue contains metastatic kidney cancer cells is not known) to this function, f(x) can be classified into (+) or (-), and the biological sample can be classified into the group of kidney cancer cell-containing tissue from a patient with a good prognosis or the group of kidney cancer cell-containing tissue from a patient with a good prognosis.

Thus, two groups of training samples are necessary in order to prepare a SVM discriminant for classifying unknown samples. According to the present invention, such training samples are, for example, a set of samples obtained from patients of "the expressed genes ($x_1$,$x_2$,..$x_i$,...$x_n$) obtained from the kidney cancer tissue of patients with good prognoses or poor prognoses" and a set of samples obtained from the patients of "the expressed genes ($x_1$,$x_2$,..$x_i$,...$x_n$) obtained from the kidney cancer tissue of patients with poor prognoses." The number (n) of the expressed genes concerning such sets varies depending on the design of the experiment. The expression levels of each gene yield significant difference, relatively small difference, or no difference between the two groups regardless of the type of experiment. In order to improve the accuracy of the SVM discriminant, distinctive differences are required between 2 groups of training samples. Thus, it is necessary to selectively extract and use genes that exhibit different expression levels between 2 groups from among gene sample sets.

Examples of methods for extracting genes that exhibit different expression levels between 2 groups include a t-test which is a parametric analysis for detecting different means and an U-test of Mann-Whitney which is a non-parametric analysis. As a method that utilizes survival analysis, the plots of the survival curve obtained by the Kaplan-Meier method are analyzed by the log-rank test or the Wilcoxon test. Also, a method wherein a survival curve is used as a regression model and analyzed by the Cox proportional hazards model is particularly useful to deduce whether or not a given variable is associated with survival. Specifically, the Cox proportional hazards model indicates how sufficiently a variety of variables describe the regression model concerning a plurality of survival curves plotted by the Kaplan-Meier method of the group of patients classified in accordance with a given category.

In the method, for example, any combination of one or more of the aforementioned polynucleotides as shown in any of SEQ ID NOS: 1 to 10, 12 to 20, and 22 to 47 and one or more of polynucleotides as shown in any of SEQ ID NOS: 11, 21, and 48 to 50 may be used. Also, the fact that the expression levels of the 50 types of target genes in tissue containing kidney cancer cells from a patient with a good prognosis are significantly different from those in tissue containing kidney cancer cells from a patient with poor prognosis, and that such expression levels vary in tissue containing kidney cancer cells from a patient with poor prognosis, are used as indications to determine the expression levels of the 50 types of genes. Thus, the prediction of the prognosis for a kidney cancer patient and/or the distinction of the metastasis of kidney cancer can be performed at the probability of 85% or higher, 89% or higher, 90% or higher, preferably 92% or higher, more preferably 93% or higher, and further preferably 94% or higher (Fig. 2).

Further provided is a method for predicting the prognosis of kidney cancer or for detecting, identifying, or predicting the metastasis of kidney cancer, comprising measuring *in vitro* the expression levels of the polypeptides or the blood levels (or existing amounts) of the polypeptides in tissue containing kidney cancer cells from a patient with a good prognosis and in tissue containing kidney cancer cells from a patient with a poor prognosis, by using one or more antibodies against respective polypeptides encoded by the aforementioned 50 types of genes (e.g., those as shown in SEQ ID NOS: 1 to 50) or fragments thereof (e.g., as shown in SEQ ID NOS: 51 to 100), such as polypeptides consisting of the respective amino acid sequences as shown in SEQ ID NOS: 101 to 150, or fragments thereof.

Also provided isa method for detecting, identifying, or predicting kidney cancer, comprising measuring *in vitro* the expression levels of the polypeptides between kidney cancer tissue and non-cancerous tissue or the blood levels of the polypeptides (or the existing amounts), by using one or more, for example, 2 or more, 3 ore more, or 5 or more to all antibodies against the respective polypeptides encoded by the aforementioned 47 genes (e.g., those as shown in SEQ ID NOS: 151 to 160, and 191 and those as shown in SEQ ID NOS: 161 to 190, and 192 to 197) or fragments thereof.

Specifically, the above mentioned measurement can be carried out by an immunological method.

Examples of immunological assay techniques include enzyme immunoassay (ELISA or EIA), fluorescence immunoassay, radio immunoassay (RIA), luminescent immunoassay, immunonephelometry, latex agglutination assay, latex turbidimetry, hemagglutination, particle agglutination, and Western blotting.

When the above method is carried out by an immunoassay technique using a label, the antibody may be immobilized, or a component in the sample may be immobilized to subject such substance to an immunological reaction.

Examples of solid-phase supports that can be used include insoluble supports in the form of beads, microplate, test tube, stick, or specimen (test strip) comprising a polystyrene, polycarbonate, polyvinyltoluene, polypropyrene, polyethylene, polyvinyl chloride, nylon, polymethacrylate, latex, gelatin, agarose, cellulose, sepharose, glass, metal, ceramic, or magnetic material.

The samples can be immobilized on the support in accordance with a conventional technique by binding the antibody of the present invention or a sample component to the solid-phase support by physical adsorption, chemical binding, or a combination thereof.

It is intended to easily detect the reaction between the antibody and the target polypeptide in the sample. To this end, the antibody is labeled to directly detect the reaction of interest. Alternatively, a labeled secondary antibody is used to indirectly detect the reaction. In the method of detection according to the present invention, the latter indirect detection technique (e.g., the sandwich technique) is preferably employed from the viewpoint of sensitivity.

Examples of label substances that can be used for enzyme immunoassay include enzymes such as peroxidase (POD), alkaline phosphatase, β-galactosidase, urease, catalase, glucose oxidase, lactate dehydrogenase, amylase, and a biotin-avidin complex. Examples of label substances that can be used for fluorescence immunoassay include fluorescent substances such as fluorescein isothiocyanate, tetramethylrhodamine isothiocyanate, substituted rhodamine isothiocyanate, dichlorotriazine isothiocyanate, Alexa, or AlexaFluoro and fluorophores. Examples of label substances that can be used for radio immunoassay include radioactive isotopes, such as tritium, iodine ($^{131}I$, $^{125}I$, $^{123}I$, and $^{121}I$), phosphorus ($^{32}P$ and $^{33}P$), sulfur ($^{35}S$), and metals (e.g., $^{68}Ga$, $^{67}Ga$, $^{68}Ge$, $^{54}Mn$, $^{99}Mo$, $^{99}Tc$, and $^{133}Xe$). Examples of label substances that can be used for luminescent immunoassay include luminescent molecules such as an NADH-, FMNH2-, luciferase system, luminol-hydrogen peroxide-POD system, acridinium ester system, or dioxetane compound system.

Also, an avidin-biotin system or streptavidin-biotin system may be used optionally. In such a case, the antibody of the invention or a fragment thereof may be bound, for example, to biotin.

A label can be bound to the antibody in case of enzyme immunoassay, for example, via a conventional technique, such as the glutaraldehyde method, the maleimide method, the pyridyl sulfide method, or the periodic acid method. Radio immunoassay can be carried out in accordance with a conventional technique, such as the chloramine-T method or Bolton-Hunter method. Such assay techniques can be carried out in accordance with conventional techniques (Current protocols in Protein Sciences, 1995, John Wiley & Sons Inc., Current protocols in Immunology, 2001, John Wiley & Sons Inc.). When the antibody is directly labeled, for example, a component in the sample is immobilized and brought into contact with the labeled antibody to form a complex of the marker polypeptide and the antibody. The unbound labeled antibody is separated by washing, and the amount of the target polypeptide in the sample can be determined based on the amount of the bound labeled antibody or the unbound labeled antibody.

When the labeled secondary antibody is used, for example, the antibody is allowed to react with the sample (the primary reaction), then with the labeled secondary antibody (the secondary reaction). The primary reaction and the secondary reaction may be carried out in the reverse order, concurrently, or separately. The primary and secondary reactions result in the formation of a complex of immobilized target polypeptide/the antibody / labeled secondary antibody or a complex of the immobilized antibody / target polypeptide/ labeled secondary antibody. The unbound labeled secondary antibody is separated by washing, and the amount of target polypeptide in the sample can be determined based on the amount of the bound labeled secondary antibody or of the unbound labeled secondary antibody.

In the enzyme immunoassay, specifically, the enzyme label is allowed to react with a substrate under optimal conditions, and the amount of the reaction product is assayed by an optical method or the like. In the fluorescence immunoassay, the fluorescent intensity from a fluorescent label is assayed. In the radio immunoassay, the radioactivity from radioactive label is assayed. In the luminescent immunoassay, the luminescent level from a luminescent reaction system is assayed.

In the method of the present invention, the generation of immune-complex aggregates in immunonephelometry, latex agglutination assay, latex turbidimetry, hemagglutination, particle agglutination, or the like is assayed by optically measuring the transmitted beam or scattered beam. When visually assayed, a solvent, such as a phosphate, glycine, Tris, or Good's buffer, can be used. Further, a reaction accelerator such as polyethylene glycol or an inhibitor of nonspecific reaction may be added to the reaction system.

The above-mentioned antibody or a fragment thereof includes, for example, a polyclonal antibody, a monoclonal antibody, a synthetic antibody, a recombinant antibody, a polyspecific antibody (including a bispecific antibody), a single chain antibody, an Fab fragment, and an $F(ab')_2$ fragment. The polyclonal antibody can be prepared as a specific antibody by a so-called absorption method, which comprises binding the antibody to an affinity column to which a purified polypeptide has been bound.

The measurement can comprise the steps of: bringing an antibody labeled with a common enzyme or fluorophore or a fragment thereof into contact with a tissue section or homogenized tissue or body fluid (such as blood, blood serum, blood plasma, or urine); and qualitatively or quantitatively measuring an antigen-antibody complex. Detection is carried out by, for example, a method wherein the presence and level of a target polypeptide are measured by immunoelectron microscopy, or a method wherein the level of a target polypeptide is assayed by a conventional method, such as ELISA or a fluorescent antibody method. Thus, kidney cancer can be detected. Further, when the expression level of a target polypeptide is decreased in tissue containing kidney cancer cells from a patient with good prognosis or in tissue containing kidney cancer cells from a patient with poor prognosis, or when the blood level of such polypeptides is significantly varied in the subject afflicted with kidney cancer with poor prognosis from the subject afflicted with kidney cancer with good prognosis, the subject can be identified to have poor prognosis and/or the metastasis of kidney cancer. In other words, when the expression level or amount of the existing target polypeptide is significantly varied from normal values, the subject is identified to have kidney cancer or have kidney cancer with poor prognosis and/or metastasis. The term "significantly" as used herein means that the identified values are statistically significant.

## EXAMPLES

The present invention will be described in more detail with reference to the examples set forth below.

<Example 1>

(1) Clinical and pathological findings concerning subjects

Informed consent was obtained from 31 Japanese patients with kidney cancer, and the kidney tissues were excised from them at the time of the surgical excision of kidney cancer. The excised tissue was visually and/or histopathologically inspected to identify the kidney cancer tissue, and the kidney cancer tissue was immediately frozen and stored in liquid nitrogen.

(2) Extraction of total RNA and preparation of cDNA

The tissue in the kidney cancer lesion of the kidney tissue obtained from an kidney cancer patient was used as a sample. Total RNA was prepared from the tissue using a Trizol reagent (Invitrogen) in accordance with the manufacturer's recommended protocol.

The thus obtained total RNA (1 μg) was subjected to reverse transcription using oligo (dT) primers in combination with random nonamers and using the CyScribe First-Strand cDNA Labeling Kit (GE Healthcare, Japan) in accordance with the manufacturer's recommended protocols. Cy3-dUTP (GE Healthcare) was added to total RNA obtained from the kidney cancer tissue, Cy5-dUTP (GE Healthcare) was added to reference total RNA (Stratagene), and cDNA was labeled at the time of reverse transcription in accordance with the manufacturer's recommended protocols. The labeled cDNA was purified using the QIA quick PCR purification Kit (QIAGEN) and then subjected to hybridization.

(3) Preparation of oligo DNA microarray

As the oligo DNA microarrays, the GeneChip® (Human Genome U133 A, Affymetrix) and the DNA chip prepared by the method as described herein were used.

A method for preparing a DNA chip is described below. In order to determine the type of oligo DNA to be loaded at first, genes were determined using the GeneChip® (Affymetrix). The GeneChip® was operated in accordance with the protocol of the Complete GeneChip® Instrument System. As a result of the analysis using the Complete GeneChip®, 8,961 types of genes in total, i.e., the genes whose expression patterns may vary due to kidney cancer and the control genes, were extracted.

Sequences comprising 60-70 residues at sites having high sequence specificity of the extracted 8,961 types of genes were selected and synthesized while avoiding sequence overlapping. The 8,961 types of 60 or 70-mer synthetic oligo DNAs comprising oligo DNAs as shown in SEQ ID NOS: 21 to 40 were separately dissolved in 4 x Solution I (Takara Bio, Japan) to a concentration of 30 $\mu$M. The resulting solutions were spotted on a DMSO-resistant coat glass for Matsunami DNA microarrays (an amino-modified oligo DNA-immobilized coat, type I; Matsunami Glass, Japan) using a spotter (GMS417 arrayer, Affymetrix) under a humidity environment of 50% to 60%.

(4) Hybridization

The labeled cDNA (1 $\mu$g) was dissolved in an antisense oligo cocktail (QIAGEN), the resulting solution was applied to the DNA chip covered by a Gap cover glass (Matsunami Glass), and hybridization was then carried out at 42°C for 16 hours. After hybridization, the DNA chip was washed successively with 2x SSC/0.1% SDS, 1x SSC, and 0.2x SSC.

(5) Determination of gene expression level

The DNA chip that had been subjected to hybridization in the above-described manner was scanned using the Agilent microarray scanner (Agilent) to obtain an image, and the fluorescent intensity was expressed numerically. The statistic procedures were carried out with reference to Speed, T., "Statistical analysis of gene expression microarray data," Chapman & Hall/CRC, and Causton, H. C. et al., "A beginner's guide Microarray gene expression data analysis," Blackwell publishing. Specifically, the data obtained by the image analysis following hybridization were converted into log values, which were then normalized by global normalization and were smoothed by LOWESS (locally weighted scatterplot smoother), and numerical correction was carried out by MAD scaling.

(6) Prediction scoring system

A variety of clinical information concerning all the patients was examined to perform survival analysis, and whether or not differences were observed in the years of life resulting from different clinical information was examined. For example, patients were divided into two groups: a group of patients who were diagnosed to have metastasis to organs other than the kidney at the time of surgery; and a group of patients who were not to diagnosed to have metastasis. The 2 groups were subjected to survival analysis, and the results of analysis were represented by charts by the Kaplan-Meier method (Fig. 1). In this case, significant difference ($p < 0.05$) was observed in the survival curve by the log-rank test between the group of patients who were diagnosed to have metastasis to organs other than the kidney at the time of surgery and the other group. This indicates that prognosis for the former group differs from that for the latter group and that the patients who were diagnosed to have metastasis would have relatively poor prognosis and the other patients would have good prognosis. Thus, influence of the prognosis for the group of patients classified in accordance with the occurrence of metastasis, i.e., the survival curve, on the model was further examined by the Cox proportional hazards model, concerning the expressed genes, in order to examine the significance of the degree of conformity between the gene expression level and the survival curve. As a result, 321 types of expressed genes that would be significantly advantageous for survival and 164 types of expressed genes that would be disadvantageous for survival were obtained with the use of the post-surgery survival curve as the indication (Table 1). Thus, these genes can be used to detect the prognosis for kidney cancer patients and/or the metastasis of kidney cancer.

These genes were used to prepare a discriminant using SVM loaded on the Genomic Profiler (Mitsui Knowledge Industry, Japan). All 31 samples were analyzed by this discriminant to predict the data. All specimens were subjected to analysis. The survivals 5 years after the surgery were used as an indication to compare the kidney cancer lesions from patients with good prognoses or poor prognoses and those from patients with poor prognoses, the data concerning the most expressed genes that would be advantageous for survival were analyzed, and gene expression assayed with the use of the polynucleotides as shown in SEQ ID NOS: 1 to 19 and 48 was examined. Thus, patients with good prognoses or poor prognoses were predicted at a probability of 96% or higher (Fig. 2).

Separately, all specimens were subjected to analysis. The survivals 5 years after the surgery were used as an indication to compare the kidney cancer lesions from patients with good prognoses or poor prognoses and those from patients with poor prognoses, the data concerning the expressed genes at high ranks that would be disadvantageous for survival were analyzed, and gene expression determined with the use of the polynucleotides as shown in SEQ ID NOS: 20 to 47, 49, and 50 was examined. Thus, patients with poor prognoses were predicted at a probability of 87% or higher (Fig. 2).

In addition to the probes used above, the other probes according to the present invention can also be used for predicting the prognosis for kidney cancer

In the above-described identification of prognosis, the SVM that identifies the kidney cancer tissue from a patient with a good prognosis and the SVM that identifies the kidney cancer tissue from a patient with a poor prognosis were simultaneously applied to the gene expression levels of an analyte tissue, and the analysis was thus performed.

When the two discriminants simultaneously yielded the result that the given analyte tissue was a kidney cancer tissue from a patient with good prognosis, or when the two discriminants yielded the result that the analyte was a kidney cancer tissue from a patient with poor prognosis, the accuracy of each diagnosis was found to be significantly higher than the accuracy attained by conventional diagnostic methods comprising the use of a single discriminant.

<Example 2>

(1) Identification of blood plasma proteins in healthy persons and patients with kidney cancer

EDTA-added blood plasma components were obtained from 5 Japanese patients with kidney cancer at an age of 50s to 70s before kidney cancer extirpation and 1 month after kidney cancer extirpation, i.e., at the healthy state.

The blood plasma was filtered through a filter (pore size 0.22 μm) to remove contaminants, and the protein concentration was adjusted to 50 mg/ml. The resulting blood plasma was further diluted in 25 mM ammonium bicarbonate solution (pH 8.0) to the concentration of 12.5 mg/ml, and molecular weight fractionation was then carried out using a hollow fiber filter (Toray, Japan). The fractionated blood plasma sample (total amount 1.8 ml, comprising 250 μg (max) of proteins) was divided into 7 fractions by reversed-phase chromatography (the ProteomeLab® PF2D System (Beckman Coulter)), lyophilized, and then redissolved in 100 μl of the 25 mM ammonium bicarbonate solution (pH 8.0). This sample was digested with trypsin (1/50 volumes of the protein) at 37°C for 2 to 3 hours for peptidization. Each peptide fraction was further fractionated into 4 fractions on the ion-exchange column (KYA Technologies, Japan).

Each fraction was further fractionated on the reversed-phase column (KYA Technologies), and the eluted peptides were measured using an online-linked mass spectrometer Q-TOF Ultima (Micromass) in a survey scan mode. The resulting data was analyzed using the protein identification software MASCOT (Matrix Science), to perform exhaustive identification of proteins. As a result, about 3,500 types of proteins, which exhibit a MASCOT score of 40 or higher (the number of identified peptides: two or more), were identified from the blood plasma components before surgery and after surgery.

(2) Comparison of the expression of blood plasma proteins of kidney cancer patient before surgery and after surgery

The blood plasma proteins of a kidney cancer patient identified in Example 2 (1) were compared between before surgery and after surgery. Proteins that were not expressed after surgery but were expressed before surgery in 4 or more of 6 patients were found. These proteins were found to be the polypeptides as shown in SEQ ID NOS: 151 to 160, and 191 shown in Table 2, and were found to be useful as the so-called kidney cancer markers, for detecting kidney cancer. Table 2 shows a frequency of the expression in each of the patients. Among the kidney cancer bearing patients before surgery, such expression was detected in 4 or more of 6 patients (indicated by "+") (Table 4).

Thus, the kidney cancer can be detected by measuring the presence or amount of at least one of the above-described polypeptides using, for example, antibodies specific thereto.

Table 4

| SEQ ID NO | Protein No. | Gene Name | Pat 1 | Pat 2 | Pat 3 | Pat 4 | Pat 5 | Pat 6 |
|---|---|---|---|---|---|---|---|---|
| 151 | P54646 | AAK2 | - | + | + | + | + | + |

(continued)

| SEQ ID NO | Protein No. | Gene Name | Pat 1 | Pat 2 | Pat 3 | Pat 4 | Pat 5 | Pat 6 |
|---|---|---|---|---|---|---|---|---|
| 152 | Q8IW52 | SLK4 | - | - | + | + | + | + |
| 153 | Q8TC36 | SP4L | - | + | - | + | + | + |
| 154 | Q96SZ6 | C5P1 | + | - | + | + | - | + |
| 155 | Q16739 | CEGT | + | + | + | + | - | - |
| 156 | Q92185 | SI8A | + | + | - | - | + | + |
| 157 | P13861 | KAP2 | + | + | - | + | - | + |
| 158 | 060825 | F262 | + | + | - | + | + | - |
| 159 | Q99832 | TCPH | - | + | + | - | + | + |
| 160 | P29992 | GB11 | + | - | - | + | + | + |
| 191 | Q9H4Z3 | CT67 | + | - | + | - | + | + |
| Pat: Patient | | | | | | | | |

<Example 3>

(1) Identification of blood plasma proteins in healthy persons and patients with kidney cancer

EDTA-added blood plasma components were obtained from 7 Japanese patients with kidney cancer at an age of 50s to 70s before kidney cancer extirpation and 1 month after kidney cancer extirpation, i.e., at the healthy state.

The blood plasma was filtered through a filter (pore size 0.22 μm) to remove contaminants, and the protein concentration was adjusted to 50 mg/ml. The resulting blood plasma was further diluted in 25 mM ammonium bicarbonate solution (pH 8.0) to the concentration of 12.5 mg/ml, and molecular weight fractionation was then carried out using a hollow fiber filter (Toray, Japan). The fractionated blood plasma sample (total amount 1.8 ml, comprising 250 μg (max) of proteins) was divided into 7 fractions by reversed-phase chromatography (the ProteomeLab® PF2D System (Beckman Coulter)), lyophilized, and then redissolved in 100 μl of the 25 mM ammonium bicarbonate solution (pH 8.0). This sample was digested with trypsin (1/50 volumes of the protein) at 37°C for 2 to 3 hours for peptidization. Each peptide fraction was further fractionated into 4 fractions on the ion-exchange column (KYA Technologies, Japan).

Each fraction was further fractionated on the reversed-phase column (KYA Technologies), and the eluted peptides were measured using an online-linked mass spectrometer Q-TOF Ultima (Micromass) in a survey scan mode. The resulting data was analyzed using the protein identification software MASCOT (Matrix Science), to perform exhaustive identification of proteins. As a result, about 3,500 types of proteins, which exhibit a MASCOT score of 40 or higher (the number of identified peptides: two or more), were identified from the blood plasma components before surgery and after surgery.

(2) Comparison of the expression of blood plasma proteins of kidney cancer patient before surgery and after surgery

The blood plasma proteins of a kidney cancer patient identified in Example 3 (1) were compared between before surgery and after surgery. Proteins that were not expressed after surgery but were expressed before surgery in 3 or more of 7 patients were discovered. These proteins were found to be the polypeptides as shown in SEQ ID NOS: 161 to 182, 192, and 193 shown in Table 1, and were found to be useful as the so-called kidney cancer markers, for detecting kidney cancer. Table 5 shows a frequency of the expression in each of the patients. Among the kidney cancer bearing patients before surgery, such expression was detected in 3 or more of 7 patients (indicated by "+").

Thus, the kidney cancer can be detected by measuring the presence or amount of at least one, and preferably at least 3 to 5, of the above-described polypeptides using, for example, antibodies specific thereto.

Table 5

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 161 | Q16671 | AMHR2 | - | - | + | + | - | + | - |
| 162 | 095236 | AP0L3 | + | - | + | - | - | + | - |

(continued)

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 163 | Q8WXF8 | DEDD2 | - | - | - | + | + | + | - |
| 164 | P07686 | HEXB | + | - | - | + | - | - | + |
| 165 | P61978 | HNRPK | - | - | + | + | + | - | - |
| 166 | P52294 | KPNA1 | - | - | + | + | - | - | + |
| 167 | . Q14847 | LASP1 | - | + | - | - | + | + | - |
| 168 | 043766 | LIAS | - | - | + |  | + | + | - |
| 169 | Q8TD91 | MAGEC3 | - | + | - | + | - | - | + |
| 170 | Q14934 | NFATC4 | + | + | + | - | - | - | - |
| 171 | Q96PB7 | 0LEM3 | + | - | - | + | - | - | + |
| 172 | Q9H1D9 | POLR3F | + | - | + | - | + | - | - |
| 173 | NP_066955 | PPP3CB | + | - | - | - | - | + | + |
| 174 | Q15637 | SF1 | + | + | - | - | - | - | + |
| 175 | Q9UI40 | SLC24A2 | + | + | + | - | - | - | - |
| 176 | 043511 | SLC26A4 | + | + | - | + | - | - | - |
| 177 | P46721 | SLC01A2 | - | + | - | + | + | - | + |
| 178 | P07951 | TPM2 | - | - | - | + | + | + | - |
| 179 | Q9Y5K5 | UCHL5 | - | + | + | - | + | - | - |
| 180 | Q16880 | UGT8 | + | + | - | - | + | - | - |
| 181 | P52738 | ZNF140 | + | - | + | - | - | - | + |
| 182 | Q96GC6 | ZNF274 | + | + | + | + | - | - | - |
| 192 | P08253 | MMP2 | - | + | - | + | + | + | - |
| 193 | P26842 | TNFRSF7 | + | + | - | + | - | + | - |
| Pat: Patient | | | | | | | | | |

(3) Comparison of the expression of blood plasma proteins of kidney cancer patient before surgery and after surgery

The blood plasma proteins of 7 kidney cancer patients identified in (1) above were compared between before surgery and after surgery. Kidney cancer-specific proteins that were not expressed after surgery but were expressed before surgery were discovered by comparison of the same subject. Table 6 shows proteins that were observed commonly in 5 out of 7 patients. These proteins were found to be the polypeptides as shown in SEQ ID NOS: 183 to 190 and 194 to 197 shown in Table 6, and were found to be useful as the so-called kidney cancer markers, for detecting kidney cancer.

Table 6

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 183 | Q9UJV3 | MID2 | + | + | + | + | - | - | + |
| 184 | Q05469 | LIPE | + | + | - | + | - | + | + |
| 185 | Q9UBN7 | HDAC6 | - | + | + | + | + | + | + |
| 186 | Q99798 | AC02 | + | + | + | - | + | - | + |
| 187 | P51693 | APLP1 | + | - | + | + | - | + | + |
| 188 | P49757 | NUMB | + | + | + | + | - | + | - |
| 189 | Q14155 | ARHGEF7 | + | - | - | + | + | + | + |

(continued)

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 190 | P50148 | GNAQ | + | - | - | + | + | + | + |
| 194 | 095263 | PDE8B | - | + | + | + | + | + | - |
| 195 | 075955 | FL0T1 | + | + | - | + | + | + | + |
| 196 | P06127 | CD5 | + | + | + | - | + | + | - |
| 197 | Q16610 | ECM1 | + | + | - | + | + | + | - |
| Pat: Patient | | | | | | | | | |

INDUSTRIAL APPLICABILITY

The present invention can provide a composition, kit, DNA chip, and method for detecting, diagnosing, and predicting metastasis of kidney cancer and/or for predicting the prognosis for kidney cancer patients, with high-specificity and high-sensitivity. Accordingly, the present invention is particularly useful in the pharmaceutical and medical industries.

## Claims

1. Use of a composition *in vitro* for detecting, identifying, or predicting the metastasis of kidney cancer in a subject, or for predicting the prognosis of a subject with kidney cancer, the composition comprising one or more probes selected from the group of polynucleotides consisting of:

   (a) a polynucleotide consisting of the nucleotide sequence as shown in SEQ ID NO: 1, mutants thereof having at least 95% identity with said nucleotide sequence, or fragments of said nucleotide sequence as shown in SEQ ID NO: 1 comprising at least 15 continuous nucleotides thereof;
   (b) a polynucleotide comprising the nucleotide sequence as shown in SEQ ID NO: 1;
   (c) a polynucleotide consisting of a nucleotide sequence complementary to the nucleotide sequence as shown in SEQ ID NO: 1, mutants thereof having at least 95% identity with said nucleotide sequence, or fragments of said nucleotide sequence as shown in SEQ ID NO: 1 comprising at least 15 continuous nucleotides thereof; and
   (d) a polynucleotide comprising a nucleotide sequence complementary to the nucleotide sequence as shown in SEQ ID NO: 1.

2. The use of the composition according to claim 1, wherein the composition further comprises at least one probe selected from the group of polynucleotides consisting of:

   (a') polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
   (b') polynucleotides comprising nucleotide sequences as shown in SEQ ID NOS: 2 to 10, 12 to 20, and 22 to 47;
   (c') polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof; and
   (d') polynucleotides comprising nucleotide sequences complementary to nucleotide sequences as shown in SEQ ID NOS: 2 to 10, 12 to 20, and 22 to 47.

3. The use of a composition according to claim 1 or 2, wherein the polynucleotide is DNA or RNA.

4. The use of a composition according to any one of claims 1 to 3, wherein the fragments are each a polynucleotide comprising at least 60 continuous nucleotides.

5. The use of a composition according to claim 1, wherein the fragments are each a polynucleotide comprising the nucleotide sequence as shown in SEQ ID NO: 51 in the nucleotide sequence as shown in SEQ ID NO: 1 or a polynucleotide comprising a nucleotide sequence complementary to the nucleotide sequence as shown in SEQ ID NO: 51 in the nucleotide sequence as shown in SEQ ID NO: 1.

6. The use of a composition according to claim 2, wherein the fragments are each a polynucleotide comprising the nucleotide sequence as shown in any of SEQ ID NOS: 52 to 60, 62 to 70, and 72 to 97 in the nucleotide sequence as shown in any of SEQ ID NOS: 2 to 10, 12 to 20, and 22 to 47 or a polynucleotide comprising a nucleotide sequence complementary to the nucleotide sequence as shown in any of SEQ ID NOS: 52 to 60, 62 to 70, and 72 to 97 in the nucleotide sequence as shown in any of SEQ ID NOS: 2 to 10, 12 to 20, and 22 to 47.

7. The use of a composition according to claim 2, wherein the fragments are each a polynucleotide comprising the nucleotide sequence as shown in any of SEQ ID NOS: 52 to 60, 62 to 70, and 72 to 97 or a nucleotide sequence complementary to the nucleotide sequence as shown in any of SEQ ID NOS: 52 to 60, 62 to 70, and 72 to 97.

8. The use of a composition according to claim 1 or 2, which further comprises, as a probe or probes, one or more polynucleotides selected from the group consisting of: polynucleotides consisting of the nucleotide sequences as shown in SEQ ID NOS: 11,21, and 48 to 50; polynucleotides consisting of complementary sequences of the nucleotide sequences as shown in SEQ ID NOS: 11, 21, and 48 to 50; or fragments of any one of the nucleotide sequences as shown in SEQ ID NOS: 11, 21, and 48 to 50 comprising at least 15 continuous nucleotides thereof.

9. The use of a composition according to claim 8, wherein the fragment is a polynucleotide comprising at least 60 continuous nucleotides.

**10.** The use of a composition according to claim 8, wherein the fragment is a polynucleotide comprising the nucleotide sequence as shown in any of SEQ ID NOS: 61, 71, and 98 to 100 in the nucleotide sequence as shown in any of SEQ ID NOS: 11, 21, and 48 to 50 or a polynucleotide comprising a nucleotide sequence complementary to the nucleotide sequence as shown in any of SEQ ID NOS: 61, 71, and 98 to 100 in the nucleotide sequence as shown in any of SEQ ID NOS: 11, 21, and 48 to 50.

**11.** The use of a composition according to claim 8, wherein the fragment is a polynucleotide comprising the nucleotide sequence as shown in any of SEQ ID NOS: 61, 71, and 98 to 100 or a nucleotide sequence complementary to any of SEQ ID NOS: 61, 71, and 98 to 100.

**12.** A kit for detecting, identifying, or predicting the metastasis of kidney cancer in a subject, or for predicting the prognosis of a subject with kidney cancer, *in vitro*, comprising one or more probes selected from the probes as defined in claim 1.

**13.** The kit according to claim 12, which further comprises at least one probe selected from the group of polynucleotides consisting of:

(a') polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(b') polynucleotides comprising nucleotide sequences as shown in SEQ ID NOS: 2 to 10, 12 to 20, and 22 to 47;
(c') polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof; and
(d') polynucleotides consisting of nucleotide sequences complementary to nucleotide sequences as shown in SEQ ID NOS: 2 to 10, 12 to 20, and 22 to 47.

**14.** The kit according to claim 12 or 13, which further comprises as a probe a polynucleotide selected from the group consisting of the nucleotide sequence as shown in any of SEQ ID NOS: 11, 21, and 48 to 50, a polynucleotide consisting of a complementary sequence thereof, or a fragment of any one of the nucleotide sequence as shown in any of SEQ ID NOS: 11, 21, and 48 to 50 or of a polynucleotide consisting of a complementary sequence of SEQ ID NOS: 11, 21, and 48 to 50 comprising at least 15 continuous nucleotides thereof.

**15.** The kit according to claim 14, wherein the probe is a polynucleotide consisting of the nucleotide sequence as shown in any of SEQ ID NOS: 1 to 10, 11, 12 to 20, 21, 22 to 47, and 48 to 50, a polynucleotide consisting of a complementary sequence thereof, or a fragment of any one of the nucleotide sequence as shown in any of SEQ ID NOS: 1 to 10, 11, 12 to 20, 21, 22 to 47, and 48 to 50, or of a polynucleotide consisting of a complementary sequence of SEQ ID NOS: 1 to 10, 11, 12 to 20, 21, 22 to 47, and 48 to 50 comprising at least 15 continuous nucleotides thereof.

**16.** The kit according to any one of claims 12 to 15, wherein the fragment is a polynucleotide comprising at least 60 continuous nucleotides.

**17.** The kit according to claim 14 or 15, wherein the fragment is a polynucleotide comprising the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50 or a polynucleotide comprising a nucleotide sequence complementary to the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50.

**18.** The kit according to claim 14 or 15, wherein the fragment is a polynucleotide comprising a nucleotide sequence as shown in any of SEQ ID NOS: 51 to 60, 61, 62 to 70, 71, 72 to 97, and 98 to 100 or a nucleotide sequence complementary to a nucleotide sequence as shown in any of SEQ ID NOS: 51 to 60, 61, 62 to 70, 71, 72-to 97, and 98 to 100.

**19.** The kit according to any one of claims 12 to 18, wherein the probes are packaged in different containers, alone or in combination.

**20.** A DNA chip for detecting, identifying, or predicting the metastasis of kidney cancer in a subject, or for predicting the prognosis of a subject with kidney cancer, *in vitro,* comprising one or more probes selected from the probes as defined in claim 1.

**21.** The DNA chip according to claim 20, which further comprises at least one probe selected from the group of polynucleotides consisting of:

(a') polynucleotides consisting of nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(b') polynucleotides comprising nucleotide sequences as shown in SEQ ID NOS: 2 to 10, 12 to 20, and 22 to 47;
(c') polynucleotides consisting of nucleotide sequences complementary to nucleotide sequences as shown in SEQ ID NOS: 2 to 10, 12 to 20, and 22 to 47, mutants thereof having at least 95% identity with said nucleotide sequences, or fragments of any one of the nucleotide sequences as shown in SEQ ID NOS: 2 to 10, 12 to 20, and 22 to 47 comprising at least 15 continuous nucleotides thereof;
(d') polynucleotides comprising nucleotide sequences complementary to nucleotide sequences as shown in SEQ ID NOS: 2 to 10, 12 to 20, and 22 to 47.

**22.** The DNA chip according to claim 20 or 21, which further comprises as a probe a polynucleotide selected from the group consisting of the nucleotide sequence as shown in any of SEQ ID NOS: 11, 21, and 48 to 50, a mutant thereof having at least 95% identity with said nucleotide sequences, and/or a fragment of any of the nucleotide sequence as shown in any of SEQ ID NOS: 11, 21, and 48 to 50 comprising at least 15 continuous nucleotides thereof.

**23.** The DNA chip according to claim 22, which comprises at least two or all of the polynucleotides comprising a nucleotide sequence as shown in any of SEQ ID NOS: 51 to 60, 61, 62 to 70, 71, 72 to 97, and 98 to 100 or complementary sequences thereto.

**24.** A method for detecting, identifying, or predicting the metastasis of kidney cancer, or for predicting the prognosis of a subject with kidney cancer, *in vitro,* comprising using one or more probes selected from the probes as defined in any one of claims 1 to 11, to measure *in vitro* expression levels of the one or more kidney cancer-associated target nucleic acids in a biological sample from a subject.

**25.** The method according to claim 24, wherein the measurement is carried out using a DNA chip.

**26.** The method according to claim 24 or 25, wherein the metastasis of kidney cancer in a subject, or the prognosis of a subject with kidney cancer, is measured using changes compared with a control sample as an indication.

**27.** The method according to any one of claims 24 to 26, wherein the biological sample is a kidney tissue or a kidney cell, blood, plasma, serum, or urine.

**28.** A method for detecting, identifying, or predicting the metastasis of kidney cancer, or for predicting the prognosis of a subject with kidney cancer, *in vitro*, comprising measuring *in vitro* expression levels of one or more kidney cancer-associated target nucleic acids in a biological sample from a subject using the composition according to any one of claims 1 to 11, the kit according to any one of claims 12 to 19, or the DNA chip according to any one of claims 20 to 23.

**29.** A method for predicting *in vitro* a patient's prognosis and/or metastasis of kidney cancer by comparing the expression levels of target nucleic acids in a biological sample of kidney cancer cells obtained from a subject with those of the target nucleic acids in kidney cancer cells obtained from a patient population with poor prognosis and/or those of the target nucleic acids in kidney cancer cells obtained from a patient population with good prognosis using one or more probes as defined in claim 1, or the composition of any one of claims 1 to 11, the kit of any one of claims 12 to 19, or the DNA chip of any one of claims 20 to 23 comprising one or more probes, wherein the target nucleic acids can be detected by the polynucleotides contained in the composition, kit, or DNA chip or a mutant or fragment of such polynucleotide, and, when the expression levels of the target nucleic acids in the subject are different from those in the patient population with good prognosis and/or those in the patient population with poor prognosis, the subject is identified to have a poor or good prognosis and/or the metastasis of kidney cancer is determined to have or have not occurred.

**30.** The method according to claim 29, which involves the use of a DNA chip.

**31.** The method according to claim 29 or 30, which comprises the steps of:

(1) measuring *in vitro* expression levels of target nucleic acids in a plurality of biological samples that are known to be kidney cancer cells obtained from a patient with a poor prognosis and/or kidney cancer cells obtained from a patient with a good prognosis using one or more probes as defined in claim 1, the composition of any one of claims 1 to 11, the kit of any one of claims 12 to 19, or the DNA chip of any one of claims 20 to 23 comprising the probe or probes;
(2) preparing a discriminant, a support vector machine, using, as training samples, the expression levels of the target nucleic acids as determined in step (1);
(3) measuring *in vitro* expression levels of the target nucleic acids in a biological sample obtained from the kidney cancer of the subject in the same manner as in step (1); and
(4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof, or fragments thereof, contained in the composition, kit, or DNA chip.

**32.** Use of one or more probes selected from the probes as defmed in claim 1 or 2, or of the composition of any one of claims 1 to 11, the kit of any one of claims 12 to 19, or the DNA chip of any one of claims 20 to 23 comprising the one or more probes, in detecting, identifying, or predicting *in vitro* the metastasis of kidney cancer in a subject, or in predicting *in vitro* the prognosis of a subject with kidney cancer, in a biological sample from the subject.

## Patentansprüche

**1.** Verwendung einer Zusammensetzung *in vitro* zum Detektieren, Identifizieren oder Metastasierungsvorhersagen von Nierenkrebs in einem Probanden, oder zum Vorhersagen der Prognose eines Probanden mit Nierenkrebs, wobei die Zusammensetzung eine oder mehrere Proben umfasst, ausgewählt aus der Gruppe von Polynukleotiden bestehend aus:

(a) Polynukleotid bestehend aus der Nukleotidsequenz wie in SEQ ID NO: 1 gezeigt, Mutanten davon mit einer Identität von mindestens 95% mit dieser Nukleotidsequenz, oder Fragmenten dieser Nukleotidsequenz wie in SEQ ID NO: 1 gezeigt, umfassend mindestens 15 kontinuierliche Nukleotide davon;
(b) Polynukleotid umfassend die Nukleotidsequenz wie in SEQ ID NO: 1 gezeigt;
(c) Polynukleotid bestehend aus einer Nukleotidsequenz komplementär zu der Nukleotidsequenz wie in SEQ ID NO: 1 gezeigt, Mutanten davon mit einer Identität von mindestens 95% mit dieser Nukleotidsequenz, oder Fragmenten dieser Nukleotidsequenz wie in SEQ ID NO: 1 gezeigt, umfassend mindestens 15 kontinuierliche Nukleotide davon; und
(d) Polynukleotid umfassend eine Nukleotidsequenz komplementär zu der Nukleotidsequenz wie in SEQ ID NO: 1 gezeigt.

**2.** Verwendung der Zusammensetzung gemäß Anspruch 1, wobei die Zusammensetzung ferner mindestens eine Sonde umfasst, ausgewählt aus der Gruppe von Polynukleotiden bestehend aus:

(a') Polynukleotiden bestehend aus Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendeiner dieser Nukleotidsequenzen wie in SEQ ID NOS: 2 bis 10, 12 bis 20 und 22 bis 47 gezeigt, umfassend mindestens 15 kontinuierliche Nukleotide davon;
(b') Polynukleotiden umfassend Nukleotidsequenzen wie in SEQ ID NOS: 2 bis 10, 12 bis 20 und 22 bis 47 gezeigt;
(c') Polynukleotiden bestehend aus Nukleotidsequenzen komplemenetär zu Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendeiner dieser Nukleotidsequenzen wie in SEQ ID NOS:

2 bis 10, 12 bis 20 und 22 bis 47 gezeigt, umfassend mindestens 15 kontinuierliche Nukleotide davon; und
(d') Polynukleotiden umfassend Nukleotidsequenzen komplementär zu Nukleotidsequenzen wie in SEQ ID NOS: 2 bis 10, 12 bis 20 und 22 bis 47 gezeigt.

**3.** Verwendung einer Zusammensetzung gemäß Anspruch 1 oder 2, wobei das Polynukleotid DNA oder RNA ist.

**4.** Verwendung der Zusammensetzung gemäß irgendeinem der Ansprüche 1 bis 3, wobei die Fragmente jeweils ein Polynukleotid umfassend mindestens 60 kontinuierliche Nukleotide sind.

**5.** Verwendung einer Zusammensetzung gemäß Anspruch 1, wobei die Fragmente jeweils ein Polynukleotid umfassend die Nukleotidsequenz wie in SEQ ID NO: 51 gezeigt sind, in der Nukleotidsequenz wie in SEQ ID NO: 1 gezeigt, oder ein Polynukleotid umfassend eine Nukleotidsequenz komplementär zu der Nukleotidsequenz wie in SEQ ID NO: 51 gezeigt, in der Nukleotidsequenz wie in SEQ ID NO: 1 gezeigt.

**6.** Verwendung einer Zusammensetzung gemäß Anspruch 2, wobei die Fragmente jeweils ein Polynukleotid umfassend die Nukleotidsequenz wie in irgendeiner der SEQ ID NOS: 52 bis 60, 62 bis 70 und 72 bis 97 gezeigt sind, in der Nukleotidsequenz wie in irgendeiner der SEQ ID NOS: 2 bis 10, 12 bis 20 und 22 bis 47 gezeigt, oder ein Polynukleotid umfassend eine Nukleotidsequenz komplementär zu der Nukleotidsequenz wie in irgendeiner der SEQ ID NOS: 52 bis 60, 62 bis 70 und 72 bis 97 gezeigt sind, in der Nukleotidsequenz wie in irgendeiner der SEQ ID NOS: 2 bis 10, 12 bis 20 und 22 bis 47 gezeigt.

**7.** Verwendung einer Zusammensetzung gemäß Anspruch 2, wobei die Fragmente jeweils ein Polynukleotid umfassend die Nukleotidsequenz wie in irgendeiner der SEQ ID NOS: 52 bis 60, 62 bis 70 und 72 bis 97 gezeigt sind, oder eine Nukleotidsequenz komplementär zu der Nukleotidsequenz wie in irgendeiner der SEQ ID NOS: 52 bis 60, 62 bis 70 und 72 bis 97 gezeigt.

**8.** Verwendung einer Zusammensetzung gemäß Anspruch 1 oder 2, welche ferner als eine Sonde oder als Sonden eine oder mehrere Polynukleotide umfasst, ausgewählt aus der Gruppe bestehend aus: Polynukleotiden bestehend aus den Nukleotidsequenzen wie in SEQ ID NOS: 11, 21 und 48 bis 50 gezeigt; Polynukleotiden bestehend aus Sequenzen, die komplementär sind zu den Nukleotidsequenzen wie in SEQ ID NOS: 11, 21 und 48 bis 50 gezeigt; oder Fragmenten von irgendeiner der Nukleotidsequenzen wie in SEQ ID NOS: 11, 21 und 48 bis 50 gezeigt, umfassend mindestens 15 kontinuierliche Nukleotide davon.

**9.** Verwendung einer Zusammensetzung gemäß Anspruch 8, wobei das Fragment ein Polynukleotid umfassend mindestens 60 kontinuierliche Nukleotide ist.

**10.** Verwendung einer Zusammensetzung gemäß Anspruch 8, wobei das Fragment ein Polynukleotid umfassend die Nukleotidsequenz wie in irgendeiner der SEQ ID NOS: 61, 71 und 98 bis 100 gezeigt ist, in der Nukleotidsequenz wie in irgendeiner der SEQ ID NOS: 11, 21 und 48 bis 50 gezeigt, oder ein Polynukleotid umfassend eine Nukleotidsequenz komplementär zu der Nukleotidsequenz wie in irgendeiner der SEQ ID NOS: 61, 71 und 98 bis 100 gezeigt, in der Nukleotidsequenz wie in irgendeiner der SEQ ID NOS: 11, 21 und 48 bis 50 gezeigt.

**11.** Verwendung einer Zusammensetzung gemäß Anspruch 8, wobei das Fragment ein Polynukleotid ist, umfassend die Nukleotidsequenz wie in irgendeiner der SEQ ID NOS: 61, 71 und 98 bis 100 gezeigt, oder eine Nukleotidsequenz komplementär zu irgendeiner der SEQ ID NOS: 61, 71 und 98 bis 100.

**12.** Kit zum Detektieren, Identifizieren oder Metastasierungsvorhersagen von Nierenkrebs in einem Probanden, oder zum Vorhersagen der Prognose eines Probanden mit Nierenkrebs *in vitro,* umfassend eine oder mehrere Sonden ausgewählt aus den Sonden wie in Anspruch 1 definiert.

**13.** Kit gemäß Anspruch 12, welcher ferner mindestens eine Sonde umfasst, ausgewählt aus der Gruppe von Polynukleotiden bestehend aus:

(a') Polynukleotiden bestehend aus Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendwelchen der Nukleotidsequenzen wie in SEQ ID NOS: 2 bis 10, 12 bis 20 und 22 bis 47 gezeigt, umfassend mindestens 15 kontinuierliche Nukleotide davon;
(b') Polynukleotiden umfassend Nukleotidsequenzen wie in SEQ ID NOS: 2 bis 10, 12 bis 20 und 22 bis 47 gezeigt;
(c') Polynukleotiden bestehend aus Nukleotidsequenzen komplemenetär zu Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendeiner der Nukleotidsequenzen wie in SEQ ID NOS: 2 bis 10, 12 bis 20 und 22 bis 47 gezeigt, umfassend mindestens 15 kontinuierliche Nukleotide davon; und
(d') Polynukleotiden bestehend aus Nukleotidsequenzen komplementär zu Nukleotidsequenzen wie in SEQ ID NOS: 2 bis 10, 12 bis 20 und 22 bis 47 gezeigt.

**14.** Kit gemäß Anspruch 12 oder 13, welcher ferner als eine Sonde ein Polynukleotid umfasst, ausgewählt aus der Gruppe bestehend aus der Nukleotidsequenz wie in irgendeiner der SEQ ID NOS: 11, 21 und 48 bis 50 gezeigt, ein Polynukleotid bestehend aus einer komplementären Sequenz davon, oder ein Fragment von irgendeiner der Nukleotidsequenz wie in irgendeiner der SEQ ID NOS: 11, 21 und 48 bis 50 gezeigt, oder von einem Polynukleotid bestehend aus einer komplementären Sequenz von SEQ ID NOS: 11, 21 und 48 bis 50 umfassend mindestens 15 kontinuierliche Nukleotide davon.

**15.** Kit gemäß Anspruch 14, wobei die Sonde ein Polynukleotid ist, bestehend aus der Nukleotidsequenz wie in irgendeiner der SEQ ID NOS: 1 bis 10, 11, 12 bis 20, 21, 22 bis 47 und 48 bis 50 gezeigt, ein Polynukleotid bestehend aus einer komplementären Sequenz davon, oder ein Fragment von irgendeiner der Nukleotidsequenz wie in irgendeiner der SEQ ID NOS: 1 bis 10, 11, 12 bis 20, 21, 22 bis 47 und 48 bis 50 gezeigt, oder von einem Polynukleotid bestehend aus einer komplementären Sequenz der SEQ ID NOS: 1 bis 10, 11, 12 bis 20, 21, 22 bis 47 und 48 bis 50, umfassend mindestens 15 kontinuierliche Nukleotide davon.

**16.** Kit gemäß irgendeinem der Ansprüche 12 bis 15, wobei das Fragment ein Polynukleotid umfassend mindestens 60 kontinuierliche Nukleotide ist.

**17.** Kit gemäß Anspruch 14 oder 15, wobei das Fragment ein Polynukleotid ist, umfassend die Nukleotidsequenz wie in irgendeiner der SEQ ID NOS: 51 bis 60, 61, 62 bis 70, 71, 72 bis 97 und 98 bis 100 gezeigt, und umfassend mindestens 60 kontinuierliche Nukleotide in der Nukleotidsequenz wie in irgendeiner der SEQ ID NOS: 1 bis 10, 11, 12 bis 20, 21, 22 bis 47 und 48 bis 50 gezeigt, oder ein Polynukleotid umfassend eine Nukleotidsequenz komplementär zu der Nukleotidsequenz wie in irgendeiner der SEQ ID NOS: 51 bis 60, 61, 62 bis 70, 71, 72 bis 97 und 98 bis 100 gezeigt, und umfassend mindestens 60 kontinuierliche Nukleotide in der Nukleotidsequenz wie in irgendeiner der SEQ ID NOS: 1 bis 10, 11, 12 bis 20, 21, 22 bis 47 und 48 bis 50 gezeigt.

**18.** Kit gemäß Anspruch 14 oder 15, wobei das Fragment ein Polynukleotid ist, umfassend eine Nukleotidsequenz wie in irgendeiner der SEQ ID NOS: 51 bis 60, 61, 62 bis 70, 71, 72 bis 97 und 98 bis 100 gezeigt, oder eine Nukleotidsequenz komplementär zu einer Nukleotidsequenz wie in irgendeiner der SEQ ID NOS: 51 bis 60, 61, 62 bis 70, 71, 72 bis 97 und 98 bis 100 gezeigt.

**19.** Kit gemäß irgendeinem der Ansprüche 12 bis 18, wobei die Sonden in unterschiedlichen Behältnissen, alleine oder in Kombination, gepackt sind.

**20.** DNA-Chip zum Detektieren, Identifizieren oder Metastasierungsvorhersagen von Nierenkrebs in einem Probanden, oder zum Vorhersagen der Prognose eines Probanden mit Nierenkrebs *in vitro,* umfassend eine oder mehrere Sonden ausgewählt von den Sonden wie in Anspruch 1 definiert.

**21.** DNA-Chip gemäß Anspruch 20, welcher ferner mindestens eine Sonde umfasst, ausgewählt aus der Gruppe von Polynukleotiden bestehend aus:

(a') Polynukleotiden bestehend aus Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendwelchen dieser Nukleotidsequenzen wie in SEQ ID NOS: 2 bis 10, 12 bis 20 und 22 bis 47 gezeigt, umfassend mindestens 15 kontinuierliche Nukleotide davon;
(b') Polynukleotiden umfassend Nukleotidsequenzen wie in SEQ ID NOS: 2 bis 10, 12 bis 20 und 22 bis 47 gezeigt;
(c') Polynukleotiden bestehend aus Nukleotidsequenzen, komplemenetär zu Nukleotidsequenzen wie in SEQ ID NOS: 2 bis 10, 12 bis 20 und 22 bis 47 gezeigt, Mutanten davon mit einer Identität von mindestens 95% mit diesen Nukleotidsequenzen, oder Fragmenten von irgendwelchen dieser Nukleotidsequenzen wie in SEQ ID NOS: 2 bis 10, 12 bis 20 und 22 bis 47 gezeigt, umfassend mindestens 15 kontinuierliche Nukleotide davon;

(d') Polynukleotiden umfassend Nukleotidsequenzen komplementär zu Nultleotidsequenzen wie in SEQ ID NOS: 2 bis 10, 12 bis 20 und 22 bis 47 gezeigt.

**22.** DNA-Chip gemäß Anspruch 20 oder 21, welcher ferner als eine Sonde ein Polynukleotid umfasst, ausgewählt aus der Gruppe bestehend aus der Nukleotidsequenz wie in irgendeiner der SEQ ID NOS: 11, 21 und 48 bis 50 gezeigt, einer Mutante davon mit mindestens 95% Identität mit diesen Nukleotidsequenzen, und/oder einem Fragment von irgendeiner der Nukleotidsequenz wie in irgendeiner der SEQ ID NOS: 11, 21 und 48 bis 50 gezeigt, umfassend mindestens 15 kontinuierliche Nukleotide davon.

**23.** DNA-Chip gemäß Anspruch 22, welcher ferner mindestens zwei oder alle der Polynukleotide umfassend eine Nukleotidsequenz wie in irgendeiner der SEQ ID NOS: 51 bis 60, 61, 62 bis 70, 71, 72 bis 97 und 98 bis 100 gezeigt, umfasst, oder komplementäre Sequenzen dazu.

**24.** Verfahren zum Detektieren, Identifizieren oder Metastasierungsvorhersagen von Nierenkrebs, oder zum Vorhersagen der Prognose eines Probanden mit Nierenkrebs *in vitro,* umfassend das Verwenden einer oder mehrerer Sonden ausgewählt aus den Sonden wie in irgendeinem der Ansprüche 1 bis 11 definiert, um *in vitro* Expressionslevel von der einen oder mehreren Nierenkrebs-assoziierten Zielnukleinsäure(n) in einer biologischen Probe eines Probanden zu bestimmen.

**25.** Verfahren gemäß Anspruch 24, wobei das Bestimmen unter Verwendung eines DNA-Chips ausgeführt wird.

**26.** Verfahren gemäß Anspruch 24 oder 25, wobei die Metastasierung von Nierenkrebs in einem Probanden, oder die Prognose eines Probanden mit Nierenkrebs, bestimmt wird unter Verwendung der Veränderungen im Vergleich mit einer Kontrollprobe als ein Indikator.

**27.** Verfahren gemäß irgendeinem der Ansprüche 24 bis 26, wobei die biologische Probe ein Nierengewebe oder eine Nierenzelle, Blut, Plasma, Serum oder Urin ist.

**28.** Verfahren zum Detektieren, Identifizieren oder Metastasierungsvorhersagen von Nierenkrebs, oder zum Vorhersagen der Prognose eines Probanden mit Nierenkrebs in *vitro,* umfassend das Messen der Expressionslevel *in vitro* von einer oder mehreren Nierenkrebs-assoziierten Zielnukleinsäure(n) in einer biologischen Probe eines Probanden unter Verwendung der Zusammensetzung gemäß irgend einem der Ansprüche 1 bis 11, des Kits gemäß irgendeinem der Ansprüche 12 bis 19 oder des DNA-Chips gemäß irgendeinem der Ansprüche 20 bis 23.

**29.** Verfahren zur Vorhersage *in vitro* einer Prognose eines Patienten und/oder Metastasierung von Nierenkrebs durch Vergleichen der Expressionslevel von Zielnukleinsäuren in einer biologischen Probe von Nierenkrebszellen erhalten aus einem Probanden, mit denen der Zielnukleinsäuren in Nierenkrebszellen erhalten aus einer Patientenpopulation mit schlechter Prognose und/oder denen der Zielnukleinsäuren in Nierenkrebszellen erhalten aus einer Patientenpopulation mit guter Prognose, unter Verwendung von einer oder mehreren Sonden wie in Anspruch 1 definiert, oder der Zusammensetzung von irgendeinem der Ansprüche 1 bis 11, des Kits gemäß irgendeinem der Ansprüche 12 bis 19, oder des DNA-Chips gemäß irgendeinem der Ansprüche 20 bis 23 umfassend eine oder mehrere Sonden, wobei die Zielnukleinsäuren detektiert werden können durch die Polynukleotide, die enthalten sind in der Zusammensetzung, dem Kit oder dem DNA-Chip oder einer Mutante oder einem Fragment von solchem Polynukleotid, und, wenn die Expressionslevel der Zielnukleinsäuren in dem Probanden unterschiedlich sind zu solchen in einer Patientenpopulation mit guter Prognose und/oder zu solchen in einer Patientenpopulation mit schlechter Prognose, wird der Proband identifiziert als eine schlechte oder gute Prognose besitzend und/oder es wird bestimmt, dass die Metastasierung von Nierenkrebs stattgefunden hat oder nicht.

**30.** Verfahren gemäß Anspruch 29, welches die Verwendung eines DNA-Chips involviert.

**31.** Verfahren gemäß Anspruch 29 oder 30, welches die Schritte umfasst:

(1) Messen *in vitro* der Expressionslevel von Zielnukleinsäuren in einer Vielzahl von biologischen Proben, von denen bekannt ist, dass sie Nierenkrebszellen sind, erhalten aus einem Patienten mit einer schlechten Prognose, und/oder Nierenkrebszellen erhalten aus einem Patienten mit einer guten Prognose, unter Verwendung von einer oder mehreren Sonden wie in Anspruch 1 definiert, der Zusammensetzung gemäß irgendeinem der Ansprüche 1 bis 11, des Kits gemäß irgendeinem der Ansprüche 12 bis 19, oder des DNA-Chips gemäß irgendeinem der Ansprüche 20 bis 23, umfassend die Sonde oder Sonden;

(2) Herstellen einer Diskriminante, einer Support-Vektor-Maschine, Verwenden, als Trainingsproben, die Expressionslevel der Zielnukleinsäuren wie in Schritt (1) bestimmt;
(3) Messen *in vitro* der Expressionslevel der Zielnukleinsäuren in einer biologischen Probe, erhalten aus dem Nierenkrebs eines Probanden auf die gleiche Art und Weise wie in Schritt (1); und
(4) Zuordnen der in Schritt (3) identifiziert Expressionslevel der Zielnukleinsäuren zu der in Schritt (2) hergestellten Diskriminante, und, basierend auf den Resultaten erhalten mit der Verwendung der Diskriminante, Vorhersagen der Prognose für einen Probanden und/oder Bestimmen, ob oder ob nicht Nierenkrebs eines Probanden metastatisch oder nicht-metastatisch ist, vorausgesetzt, dass die Zielnukleinsäuren von den Polynukleotiden, Mutanten davon, oder Fragmenten davon, welche in der Zusammensetzung, dem Kit oder dem DNA-Chip enthalten sind, detektiert werden können.

**32.** Verwendung von einer oder mehreren Sonden, ausgewählt aus den Sonden wie in Anspruch 1 oder 2 definiert, oder der Zusammensetzung gemäß irgendeinem der Ansprüche 1 bis 11, des Kits gemäß irgendeinem der Ansprüche 12 bis 19, oder des DNA-Chips gemäß irgendeinem der Ansprüche 20 bis 23, umfassend die eine oder mehrere Sonde(n), beim Detektieren, Identifizieren oder Metastasierungsvorhersagen *in vitro* von Nierenkrebs in einem Probanden, oder beim Vorhersagen in *vitro* der Prognose eines Probanden mit Nierenkrebs, in einer biologischen Probe von dem Probanden.

## Revendications

**1.** Utilisation d'une composition *in vitro* pour détecter, identifier, ou prédire la métastase du cancer du rein chez un sujet, ou pour prédire le pronostic d'un sujet atteint d'un cancer du rein, la composition comprenant une ou plusieurs sonde(s) choisie(s) dans le groupe de polynucléotides constitué :

(a) d'un polynucléotide constitué de la séquence nucléotidique telle que représentée dans la séquence SEQ ID NO : 1, de mutants de celui-ci ayant une identité d'au moins 95% avec ladite séquence nucléotidique, ou de fragments de ladite séquence nucléotidique telle que représentée dans la séquence SEQ ID NO : 1, comprenant au moins 15 nucléotides continus de celle-ci ;
(b) d'un polynucléotide comprenant la séquence nucléotidique telle que représentée dans la séquence SEQ ID NO : 1 ;
(c) d'un polynucléotide constitué d'une séquence nucléotidique complémentaire à la séquence nucléotidique telle que représentée dans la séquence SEQ ID NO : 1, de mutants de celui-ci ayant une identité d'au moins 95% avec ladite séquence nucléotidique, ou de fragments de ladite séquence nucléotidique telle que représentée dans la séquence SEQ ID NO : 1 comprenant au moins 15 nucléotides continus de celle-ci ; et
(d) d'un polynucléotide comprenant une séquence nucléotidique complémentaire à la séquence nucléotidique telle que représentée dans la séquence SEQ ID NO : 1.

**2.** Utilisation de la composition selon la revendication 1, dans laquelle la composition comprend en outre au moins une sonde choisie dans le groupe de polynucléotides constitué :

(a') de polynucléotides constitués de séquences nucléotidiques telles que représentées dans les séquences SEQ ID NO : 2 à 10, 12 à 20, et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ;
(b') de polynucléotides comprenant des séquences nucléotidiques telles que représentées dans les séquences SEQ ID NO : 2 à 10, 12 à 20, et 22 à 47 ;
(c') de polynucléotides constitués de séquences nucléotidiques complémentaires aux séquences nucléotidiques telles que représentées dans les séquences SEQ ID NO : 2 à 10, 12 à 20, et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ; et
(d') de polynucléotides comprenant des séquences nucléotidiques complémentaires aux séquences nucléotidiques telles que représentées dans les séquences SEQ ID NO : 2 à 10, 12 à 20 et 22 à 47.

**3.** Utilisation d'une composition selon la revendication 1 ou 2, dans laquelle le polynucléotide est un ADN ou un ARN.

**4.** Utilisation d'une composition selon l'une quelconque des revendications 1 à 3, dans laquelle les fragments sont chacun un polynucléotide comprenant au moins 60 nucléotides continus.

**5.** Utilisation d'une composition selon la revendication 1, dans laquelle les fragments sont chacun un polynucléotide comprenant la séquence nucléotidique telle que représentée dans la séquence SEQ ID NO : 51 dans la séquence nucléotidique telle que représentée dans la séquence SEQ ID NO : 1 ou un polynucléotide comprenant une séquence nucléotidique complémentaire à la séquence nucléotidique telle que représentée dans la séquence SEQ ID NO : 51 dans la séquence nucléotidique telle que représentée dans la séquence SEQ ID NO : 1.

**6.** Utilisation d'une composition selon la revendication 2, dans laquelle les fragments sont chacun un polynucléotide comprenant la séquence nucléotidique telle que représentée dans l'une des séquences SEQ ID NO : 52 à 60, 62 à 70, et 72 à 97 dans la séquence nucléotidique telle que représentée dans l'une des séquences SEQ ID NO : 2 à 10, 12 à 20, et 22 à 47 ou un polynucléotide comprenant une séquence nucléotidique complémentaire à la séquence nucléotidique telle que représentée dans l'une des séquences SEQ ID NO : 52 à 60, 62 à 70, et 72 à 97 dans la séquence nucléotidique telle que représentée dans l'une des séquences SEQ ID NO : 2 à 10, 12 à 20, et 22 à 47.

**7.** Utilisation d'une composition selon la revendication 2, dans laquelle les fragments sont chacun un polynucléotide comprenant la séquence nucléotidique telle que représentée dans l'une des séquences SEQ ID NO : 52 à 60, 62 à 70, et 72 à 97 ou une séquence nucléotidique complémentaire à la séquence nucléotidique telle que représentée dans l'une des séquences SEQ ID NO : 52 à 60, 62 à 70, et 72 à 97.

**8.** Utilisation d'une composition selon la revendication 1 ou 2, qui comprend en outre, en tant que sonde ou sondes, un ou plusieurs polynucléotide(s) choisi(s) dans le groupe constitué : de polynucléotides constitués des séquences nucléotidiques telles que représentées dans les séquences SEQ ID NO : 11, 21, et 48 à 50 ; de polynucléotides constitués de séquences complémentaires aux séquences nucléotidiques telles que représentées dans les séquences SEQ ID NO : 11, 21, et 48 à 50; ou des fragments de l'une quelconque des séquences nucléotidiques telles que représentées dans les séquences SEQ ID NO : 11, 21, et 48 à 50 comprenant au moins 15 nucléotides continus de celles-ci.

**9.** Utilisation d'une composition selon la revendication 8, dans laquelle le fragment est un polynucléotide comprenant au moins 60 nucléotides continus.

**10.** Utilisation d'une composition selon la revendication 8, dans laquelle le fragment est un polynucléotide comprenant la séquence nucléotidique telle que représentée dans l'une des séquences SEQ ID NO : 61, 71, et 98 à 100 dans la séquence nucléotidique telle que représentée dans l'une des séquences SEQ ID NO : 11, 21, et 48 à 50 ou un polynucléotide comprenant une séquence nucléotidique complémentaire à la séquence nucléotidique telle que représentée dans l'une des séquences SEQ ID NO : 61, 71, et 98 à 100 dans la séquence nucléotidique telle que représentée dans l'une des séquences SEQ ID NO : 11, 21, et 48 à 50.

**11.** Utilisation d'une composition selon la revendication 8, dans laquelle le fragment est un polynucléotide comprenant la séquence nucléotidique telle que représentée dans l'une des séquences SEQ ID NO : 61, 71, et 98 à 100 ou une séquence nucléotidique complémentaire à l'une des séquences SEQ ID NO : 61, 71, et 98 à 100.

**12.** Kit pour détecter, identifier, ou prédire la métastase du cancer du rein chez un sujet, ou pour prédire le pronostic d'un sujet atteint d'un cancer du rein, in *vitro,* comprenant une ou plusieurs sonde(s) choisie(s) parmi les sondes telles que définies dans la revendication 1.

**13.** Kit selon la revendication 12, qui comprend en outre au moins une sonde choisie dans le groupe de polynucléotides constitué :

(a') de polynucléotides constitués de séquences nucléotidiques telles que représentées dans les séquences SEQ ID NO : 2 à 10, 12 à 20 et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ;
(b') de polynucléotides comprenant des séquences nucléotidiques telles que représentées dans les séquences SEQ ID NO : 2 à 10, 12 à 20, et 22 à 47 ;
(c') de polynucléotides constitués de séquences nucléotidiques complémentaires aux séquences nucléotidiques telles que représentées dans les séquences SEQ ID NO : 2 à 10, 12 à 20 et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ; et
(d') de polynucléotides constitués de séquences nucléotidiques complémentaires aux séquences nucléotidiques telles que représentées dans les séquences SEQ ID NO : 2 à 10, 12 à 20 et 22 à 47.

**14.** Kit selon la revendication 12 ou 13, qui comprend en outre en tant que sonde un polynucléotide choisi dans le groupe constitué de la séquence nucléotidique telle que représentée dans l'une des séquences SEQ ID NO : 11, 21, et 48 à 50, un polynucléotide constitué d'une séquence complémentaire à celle-ci, ou d'un fragment de l'une quelconque de la séquence nucléotidique telle que représentée dans l'une des séquences SEQ ID NO : 11, 21, et 48 à 50 ou d'un polynucléotide constitué d'une séquence complémentaire des séquences SEQ ID NO : 11, 21, et 48 à 50 comprenant au moins 15 nucléotides continus de celles-ci.

**15.** Kit selon la revendication 14, dans lequel la sonde est un polynucléotide constitué de la séquence nucléotidique telle que représentée dans l'une des séquences SEQ ID NO : 1 à 10, 11, 12 à 20, 21, 22 à 47, et 48 à 50, un polynucléotide constitué d'une séquence complémentaire de celle-ci, ou un fragment de l'une quelconque de la séquence nucléotidique telle que représentée dans l'une des séquences SEQ ID NO : 1 à 10, 11, 12 à 20, 21, 22 à 47, et 48 à 50, ou d'un polynucléotide constitué d'une séquence complémentaire des séquences SEQ ID NO : 1 à 10, 11, 12 à 20, 21, 22 à 47, et 48 à 50 comprenant au moins 15 nucléotides continus de celles-ci.

**16.** Kit selon l'une quelconque des revendications 12 à 15, dans lequel le fragment est un polynucléotide comprenant au moins 60 nucléotides continus.

**17.** Kit selon la revendication 14 ou 15, dans lequel le fragment est un polynucléotide comprenant la séquence nucléotidique telle que représentée dans l'une des séquences SEQ ID NO : 51 à 60, 61, 62 à 70, 71, 72 à 97, et 98 à 100 et comprenant au moins 60 nucléotides continus dans la séquence nucléotidique telle que représentée dans l'une des séquences SEQ ID NO : 1 à 10, 11, 12 à 20, 21, 22 à 47, et 48 à 50 ou un polynucléotide comprenant une séquence nucléotidique complémentaire à la séquence nucléotidique telle que représentée dans l'une des séquences SEQ ID NO : 51 à 60, 61, 62 à 70, 71, 72 à 97 et 98 à 100 et comprenant au moins 60 nucléotides continus dans la séquence nucléotidique telle que représentée dans l'une des séquences SEQ ID NO : 1 à 10, 11, 12 à 20, 21, 22 à 47, et 48 à 50.

**18.** Kit selon la revendication 14 ou 15, dans lequel le fragment est un polynucléotide comprenant une séquence nucléotidique telle que représentée dans l'une des séquences SEQ ID NO : 51 à 60, 61, 62 à 70, 71, 72 à 97 et 98 à 100 ou une séquence nucléotidique complémentaire à une séquence nucléotidique telle que représentée dans l'une des séquences SEQ ID NO : 51 à 60, 61, 62 à 70, 71, 72 à 97, et 98 à 100.

**19.** Kit selon l'une quelconque des revendications 12 à 18, dans lequel les sondes sont conditionnées dans différents récipients, seules ou en combinaison.

**20.** Puce à ADN pour détecter, identifier, ou prédire la métastase du cancer du rein chez un sujet, ou pour prédire le pronostic d'un sujet atteint d'un cancer du rein, *in vitro,* comprenant une ou plusieurs sonde(s) choisie(s) parmi les sondes telles que définies dans la revendication 1 .

**21.** Puce à ADN selon la revendication 20, qui comprend en outre au moins une sonde choisie dans le groupe de polynucléotides constitué :

(a') de polynucléotides constitués de séquences nucléotidiques telles que représentées dans les séquences SEQ ID NO : 2 à 10, 12 à 20 et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ;
(b') de polynucléotides comprenant des séquences nucléotidiques telles que représentées dans les séquences SEQ ID NO : 2 à 10, 12 à 20, et 22 à 47 ;
(c') de polynucléotides constitués de séquences nucléotidiques complémentaires aux séquences nucléotidiques telles que représentées dans les séquences SEQ ID NO : 2 à 10, 12 à 20 et 22 à 47, de mutants de ceux-ci ayant une identité d'au moins 95% avec lesdites séquences nucléotidiques, ou de fragments de l'une quelconque des séquences nucléotidiques telles que représentées dans les séquences SEQ ID NO : 2 à 10, 12 à 20, et 22 à 47 comprenant au moins 15 nucléotides continus de celles-ci ;
(d') de polynucléotides comprenant des séquences nucléotidiques complémentaires à des séquences nucléotidiques telles que représentées dans les séquences SEQ ID NO : 2 à 10, 12 à 20 et 22 à 47.

**22.** Puce à ADN selon la revendication 20 ou 21, qui comprend en outre en tant que sonde un polynucléotide choisi dans le groupe constitué de la séquence nucléotidique telle que représentée dans l'une des séquences SEQ ID NO : 11, 21, et 48 à 50, un mutant de celui-ci ayant une identité d'au moins 95% avec lesdites séquences nucléotidiques, et/ou un fragment de l'une de la séquence nucléotidique telle que représentée dans l'une des séquences SEQ ID NO : 11, 21, et 48 à 50 comprenant au moins 15 nucléotides continus de celles-ci.

**23.** Puce à ADN selon la revendication 22, qui comprend au moins deux polynucléotides ou tous les polynucléotides comprenant une séquence nucléotidique telle que représentée dans l'une des séquences SEQ ID NO : 51 à 60, 61, 62 à 70, 71, 72 à 97, et 98 à 100 ou des séquences complémentaires à celles-ci.

**24.** Procédé pour détecter, identifier, ou prédire la métastase du cancer du rein, ou pour prédire le pronostic d'un sujet atteint d'un cancer du rein, *in vitro,* comprenant le fait d'utiliser une ou plusieurs sonde(s) choisie(s) parmi les sondes telles que définies dans l'une quelconque des revendications 1 à 11, pour mesurer des taux d'expression *in vitro* du ou des plusieurs acide(s) nucléique(s) cible(s) associé(s) au cancer du rein dans un échantillon biologique provenant d'un sujet.

**25.** Procédé selon la revendication 24, dans lequel la mesure est réalisée en utilisant une puce à ADN.

**26.** Procédé selon la revendication 24 ou 25, dans lequel la métastase du cancer du rein chez un sujet, ou le pronostic d'un sujet atteint d'un cancer du rein, est mesuré(e) en utilisant des changements par rapport à un échantillon témoin comme indication.

**27.** Procédé selon l'une quelconque des revendications 24 à 26, dans lequel l'échantillon biologique est un tissu rénal ou une cellule rénale, du sang, du plasma, du sérum, ou de l'urine.

**28.** Procédé pour détecter, identifier, ou prédire la métastase du cancer du rein, ou pour prédire le pronostic d'un sujet atteint d'un cancer du rein, *in vitro,* comprenant le fait de mesurer des taux d'expression *in vitro* d'un ou de plusieurs acide(s) nucléique(s) cible(s) associé(s) au cancer du rein dans un échantillon biologique provenant d'un sujet en utilisant la composition selon l'une quelconque des revendications 1 à 11, le kit selon l'une quelconque des revendications 12 à 19, ou la puce à ADN selon l'une quelconque des revendications 20 à 23.

**29.** Procédé pour prédire *in vitro* le pronostic et/ou la métastase du cancer du rein d'un patient en comparant les taux d'expression d'acides nucléiques cibles dans un échantillon biologique de cellules cancéreuses rénales obtenues à partir d'un sujet à ceux des acides nucléiques cibles dans des cellules cancéreuses rénales obtenues à partir d'une population de patients ayant un mauvais pronostic et/ou à ceux des acides nucléiques cibles dans des cellules cancéreuses rénales obtenues à partir d'une population de patients ayant un bon pronostic en utilisant une ou plusieurs sonde(s) telle(s) que définie(s) dans la revendication 1, ou la composition de l'une quelconque des revendications 1 à 11, le kit de l'une quelconque des revendications 12 à 19, ou la puce à ADN de l'une quelconque des revendications 20 à 23, comprenant une ou plusieurs sonde(s), où les acides nucléiques cibles peuvent être détectés par les polynucléotides contenus dans la composition, le kit, ou la puce à ADN ou un mutant ou fragment d'un tel polynucléotide, et, lorsque les taux d'expression des acides nucléiques cibles chez le sujet sont différents de ceux chez la population de patients ayant un bon pronostic et/ou de ceux chez la population de patients ayant un mauvais pronostic, le sujet est identifié comme ayant un mauvais ou un bon pronostic et/ou la métastase du cancer du rein est déterminée comme ayant ou n'ayant pas eu lieu.

**30.** Procédé selon la revendication 29, qui implique l'utilisation d'une puce à ADN.

**31.** Procédé selon la revendication 29 ou 30, qui comprend les étapes qui consistent :

(1) à mesurer des taux d'expression *in vitro* d'acides nucléiques cibles dans une pluralité d'échantillons biologiques qui sont connus comme étant des cellules cancéreuses rénales obtenues à partir d'un patient ayant un mauvais pronostic et/ou des cellules cancéreuses rénales obtenues à partir d'un patient ayant un bon pronostic en utilisant une ou plusieurs sonde(s) telle(s) que définie(s) dans la revendication 1, la composition de l'une quelconque des revendications 1 à 11, le kit de l'une quelconque des revendications 12 à 19, ou la puce à ADN de l'une quelconque des revendications 20 à 23, comprenant la sonde ou les sondes ;
(2) à préparer un discriminant, une machine à vecteurs de support, en utilisant comme échantillons d'apprentissage, les taux d'expression des acides nucléiques cibles tels que déterminés dans l'étape (1) ;
(3) à mesurer des taux d'expression *in vitro* des acides nucléiques cibles dans un échantillon biologique obtenu à partir du cancer du rein du sujet de la même manière que dans l'étape (1) ; et
(4) à attribuer les taux d'expression des acides nucléiques cibles identifiés dans l'étape (3) au discriminant préparé dans l'étape (2) et, sur la base des résultats obtenus avec l'utilisation du discriminant, à prédire le pronostic pour un sujet et/ou à déterminer si le cancer du rein du sujet est métastatique ou non métastatique ou non, à condition que les acides nucléiques cibles puissent être détectés par les polynucléotides, des mutants de ceux-ci, ou des fragments de ceux-ci, contenus dans la composition, le kit, ou la puce à ADN.

**32.** Utilisation d'une ou de plusieurs sonde(s) choisie(s) parmi les sondes telles que définies dans la revendication 1 ou 2, ou de la composition de l'une quelconque des revendications 1 à 11, du kit de l'une quelconque des revendications 12 à 19, ou de la puce à ADN de l'une quelconque des revendications 20 à 23, comprenant la ou les plusieurs sonde(s), dans la détection, l'identification, ou la prédiction *in vitro* de la métastase du cancer du rein chez un sujet, ou dans la prédiction *in vitro* du pronostic d'un sujet atteint d'un cancer du rein, dans un échantillon biologique provenant du sujet.

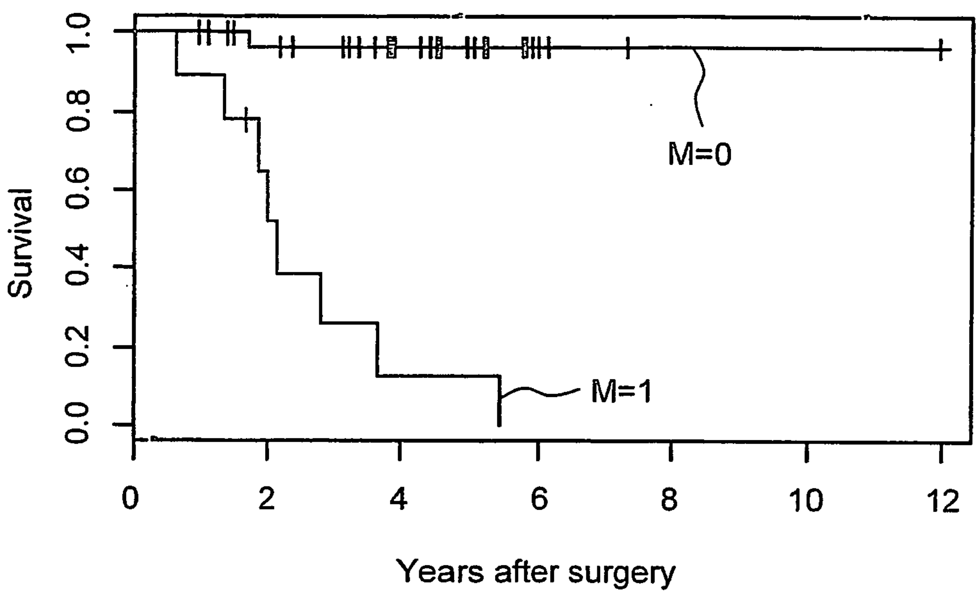
1.0
0.8
0.6
0.4
0.2
0.0
Survival
M=0
M=1
0
2
4
6
8
10
12
Years after surgery

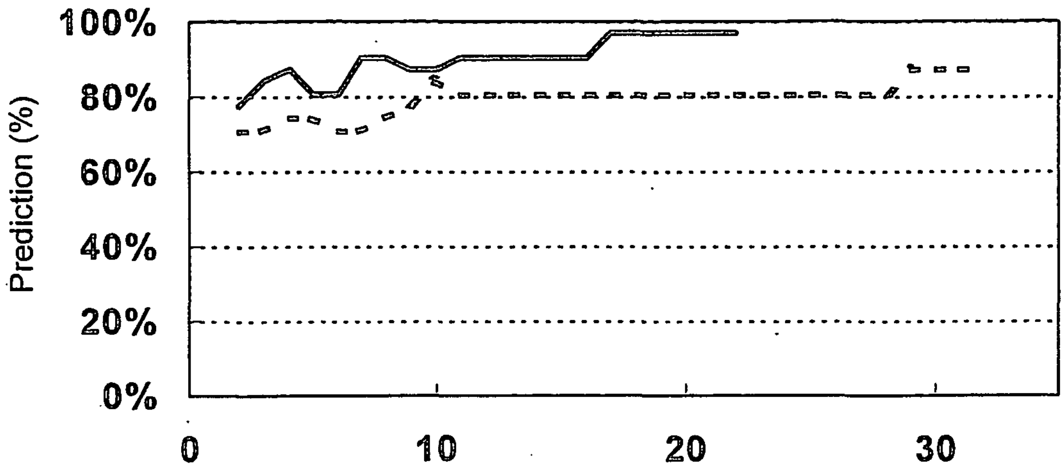


Prediction of prognosis for kidney cancer patient/number of genes necessary for detecting metastasis of kidney cancer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005024603 A
- WO 2004042077 A
- WO 2004048933 A
- US 6303765 B
- JP 2005520536 A
- JP 2005507997 A
- WO 200206339 A
- JP 2004518402 A
- WO 200524603 A
- JP 2004264289 A
- JP 2006317380 W
- JP 2005255499 A
- JP 2005318589 A


### Non-patent literature cited in the description


- **LATIF, F. et al.** *Science,* 1993, vol. 260, 1317-1320
- **MAXWELL, P. et al.** *Nature,* 1999, vol. 399, 271-275
- **NAGASE, Y. et al.** *Japanese Journal of Urology,* 1991, vol. 82, 1781-1789
- **LEIN, M. et al.** *International Journal of Cancer,* 2000, vol. 85, 801-804
- **NAKATSUJI, T.** *Clinical and Experimental Medicine,* 2003, vol. 2, 192-196
- **MIYAKE, H. et al.** *Cancer Research,* 1996, vol. 56, 2440-2445
- **KITAGAWA, Y. et al.** *Journal of Urology,* 1999, vol. 162, 905-909
- **FREEMAN, M. R. et al.** *Cancer Research,* 1989, vol. 49, 6221-6225
- **KARLIN, S. et al.** *Proceedings of the National Academic Sciences, U.S.A.,* 1993, vol. 90, 5873-5877, http://www.ncbi.nlm.nih.gov/BLAST
- **KARLIN, S. et al.** *Proceedings of the National Academic Sciences, U.S.A.,* 1993, vol. 90, 5873-5877
- **ALTSCHUL, S. F. et al.** *Journal of Molecular Biology,* 1990, vol. 215, 403-410
- **PEARSON, W. R. et al.** *Proceedings of the National Academic Sciences, U.S.A.,* 1988, vol. 85, 2444-2448
- *BMJ,* 1998, vol. 317, 1572
- **BRAIS, B. et al.** *Nature genetics,* 1998, vol. 18, 164-167
- **UNOKI, M. et al.** *Oncogene,* 2001, vol. 20, 4457-4465
- **TOMASETTO, C. et al.** *EMBO Journal,* 1990, vol. 9, 407-414
- **METHOT, N. et al.** *Journal of Biological Chemistry,* 1997, vol. 272, 1110-1116
- **WAGNER, A. et al.** *Proceedings of the National Academic Sciences, U.S.A.,* 1992, vol. 89, 3111-3115
- **NAGASE, T. et al.** *DNA Research,* 1997, vol. 4, 141-150
- **ROSENFELD, M. et al.** *Annals of Neurology.,* 1993, vol. 33, 113-120
- **HATEBOER, G. et al.** *EMBO Journal,* 1995, vol. 14, 3159-3169
- **BANERJI, J. et al.** *Proceedings of the National Academic Sciences, U.S.A.,* 1990, vol. 87, 2374-2378
- **HOOPER, J. D.. et al.** *Genomics,* 2000, vol. 66, 113-118
- **TAKEI, Y. et al.** *Journal of Biological Chemistry,* 1998, vol. 273, 22177-22180
- **SOLOMON, E. et al.** *Proceedings of the National Academic Sciences, U.S.A.,* 1985, vol. 82, 3330-3334
- **FUKUNAGA, R. et al.** *EMBO Journal,* 1997, vol. 16, 1921-1933
- **YU, J. et al.** *Molecular Cell,* 2001, vol. 7, 673-682
- **SUPERTI-FRUGA, A. et al.** *Genomics,* 1993, vol. 17, 463-467
- **DU, M. et al.** *Biochemical Biophysical Research Communication,* 1997, vol. 235, 779-783
- **GAUDET, S. et al.** *Proceedings of the National Academic Sciences, U.S.A.,* 2000, vol. 97, 5167-5172
- **STURANY, S. et al.** *Journal of Biological Chemistry,* 2001, vol. 276, 3310-3318
- **BADER, B. et al.** *EMBO Journal,* 1986, vol. 5, 1865-1875
- **BEAUSOLEIL, S. A. et al.** *Proceedings of the National Academic Sciences, U.S.A.,* 2004, vol. 101, 12130-12135

- **KESTILA, M. et al.** *Molecular Cell,* 1998, vol. 1, 575-582
- **KOIKE, G. et al.** *Biochemical Biophysical Research Communication,* 1994, vol. 203, 1842-1850
- **LOVENBERG, T. W. et al.** *Proceedings of the National Academic Sciences, U.S.A.,* 1993, vol. 90, 2184-2188
- **DANIELSON, K. G. et al.** *Genomics,* 1993, vol. 15, 146-160
- **VISCONTI, P. E. et al.** *Genomics,* 2001, vol. 77, 163-170
- **MATSUDA, L.A. et al.** *Nature,* 1990, vol. 346, 561-564
- **XIAO, B. et al.** *Neuron,* 2001, vol. 21, 707-716
- **MARCHESE, A. et al.** *Genomics,* 1994, vol. 23, 609-618
- **OTA, T. et al.** *Nature Genetics,* 2004, vol. 36, 40-45
- **BANERJI, S. et al.** *Journal of Cellular Biology,* 1999, vol. 144, 1789-801
- **STRAUSBERG, R. L. et al.** *Proceedings of the National Academic Sciences, U.S.A.,* 2000, vol. 99, 16899-16903
- **BURGESON, R. E. et al.** *Proceedings of the National Academic Sciences, U.S.A.,* 2002, vol. 73, 2579-2583
- **NOMURA, N. L. et al.** *DNA Research,* 1994, vol. 1, 223-229
- **SMITH, A. N. et al.** *Nature Genetics,* 2000, vol. 26, 71-75
- **CHAKRAVARTI, A. et al.** *Proceedings of the National Academic Sciences, U.S.A.,* 2002, vol. 99, 4755-4756
- **CHAN, J. et al.** *Oncogene,* 1991, vol. 6, 1057-1061
- **SU, L. et al.** *Genes Development,* 1993, vol. 7, 735-748
- **TAANMAN, J-W. et al.** *Nucleic Acids Research,* 1989, vol. 17, 1766
- **OPPENHEIM, F. G. et al.** *Biochemistry,* 1971, vol. 10, 4233-4238
- **ADRA, C. N. et al.** *Genomics,* 1996, vol. 35, 328-337
- **PIERRAT, B. et al.** *Journal of Biological Chemistry,* 1998, vol. 273, 29661-29671
- **EICHMULLER, S. et al.** *Proceedings of the National Academic Sciences, U.S.A.,* 2001, vol. 98, 629-634
- **ABRAHAM, J. et al.** *Science,* 1986, vol. 233, 545-548
- **SATO, H. et al.** *Nature,* 1994, vol. 370, 61-65
- **YANG-FENG, T. L. et al.** *Citogenetic Cell Genetics,* 1985, vol. 40, 784
- **AUSUBEL. et al.** Current Protocols in Molecular Biology. John Willey & Sons, US, 1993
- **SAMBROOK et al.** Molecular Cloning: A Laboratory Manual. Cold Spring Harbor Laboratory Press, 1989
- **SAMBROOK, J. ; RUSSEL, D.** Molecular Cloning, A LABORATORY MANUAL. Cold Spring Harbor Laboratory Press, 15 January 2001, vol. 1
- MOLECULAR CLONING, A LABORATORY MANUAL. vol. 2, 8.9-8.17
- Monoclonal Antibodies, Hybridomas: A New Dimension in Biological Analyses. Plenum Press, 1980
- **J. B. LAMTURE et al.** *Nucleic. Acids. Research,* 1994, vol. 22, 2121-2125
- **Z. GUO et al.** *Nucleic. Acids. Research,* 1994, vol. 22, 5456-5465
- **G. YERSHOV et al.** *Proceedings of the National Academic Sciences, U.S.A.,* 1996, vol. 94, 4913
- **R. G. SOSNOWSKI et al.** *Proceedings of the National Academic Sciences, U.S.A.,* 1997, vol. 94, 1119-1123
- **SAMBROOK, J. ; RUSSEL, D.** Molecular Cloning, A LABORATORY MANUAL. Cold Spring Harbor Laboratory Press, 15 January 2001, vol. 1, 7.42-7.45
- **V. VAPNIK.** The Nature of Statistical Leaning Theory. Springer, 1995
- Pataan ninshiki to gakushu no toukeigaku. **HIDEKI ASOU et al.** Toukei kagaku no furontia 6. Iwanami Shoten Publishers, 11 April 2003, 107-138
- Current protocols in Protein Sciences. John Wiley & Sons Inc, 1995
- Current protocols in Immunology. John Wiley & Sons Inc, 2001
- **SPEED, T.** Statistical analysis of gene expression microarray data. Chapman & Hall/CRC
- **CAUSTON, H. C. et al.** A beginner's guide Microarray gene expression data analysis. Blackwell publishing